# EUROPEAN PATENT APPLICATION

(11) **EP 1 666 469 A1**
(43) Date of publication of application: **07.06.2006**
(21) Application number: 04772932.2
(22) Date of filing: 03.09.2004
(51) Int. Cl.: C07D 231/22, C07D 231/26, C07D 231/36, C07D 233/96, C07D 277/34, C07D 277/36, C07D 401/06, C07D 401/10, C07D 401/12, C07D 403/06, C07D 409/12, C07D 413/06, A61K 31/4152, A61K 31/4155, A61K 31/4166, A61K 31/427, A61K 31/426, A61K 31/4439, A61K 31/4245, A61P 7/02, A61P 35/00

(54) **PLASMINOGEN ACTIVATOR INHIBITOR-1 INHIBITOR**

(30) Priority: 11.09.2003 JP 2003319191
(71) Applicant: Institute of Medicinal Molecular Design, Inc., Tokyo 113-0033 (JP)
(72) Inventor: MUTO, S., Inst. Of Medicinal Molecular Design, Inc, Bunkyo-ku, Tokyo 113-0033 (JP); KUBO, A., Inst. Of Medicinal Molecular Design, Inc, Bunkyo-ku, Tokyo 113-0033 (JP); ITAI, A., Inst. Of Medicinal Molecular Design, Inc, Bunkyo-ku, Tokyo 113-0033 (JP); SOTOME, T., Inst. Of Med. Molecular Design, Inc, Bunkyo-ku, Tokyo 113-0033 (JP); YAMAGUCHI, Y., Inst. Of Med. Molecular Design, Inc, Bunkyo-ku, Tokyo 113-0033 (JP)
(74) Representative: Sternagel, Fleischer, Godemeyer & Partner
(86) International application number: PCT/JP2004/013193
(87) International publication number: WO 2005/026127

(57) **Abstract**

A medicament having inhibitory activity against plasminogen activator inhibitor-1, which comprises as an active ingredient a compound represented by the following general formula (I) or a salt thereof: 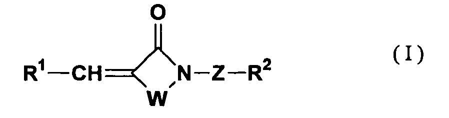 wherein R¹ and R² represents an aromatic group which may be substituted,
W represents a group selected from the following connecting group W-1: 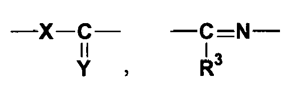 (wherein a bond at the left end binds to the carbon atom and a bond at the right end binds to the nitrogen atom,
X represents sulfur atom or NH,
Y represents oxygen atom or sulfur atom,
R³ represents a hydrocarbon group, hydroxy group, or carboxy group),
Z represents a single bond or a connecting group wherein a number of atoms in a main chain is 1 to 3.

## Description

### Field of Invention

The present invention relates to 5-membered heterocyclic derivatives which are useful as plasminogen activator inhibitor-1 (hereinafter referred to as PAI-1) inhibitors.

### Background Art

Blood coagulation system consists of a cascade reaction which comprises numbers of combinations of various kinds of proteases and precursors (substrates) thereof, and is regulated mainly depending upon blood endothelial cells. When the blood endothelial cells are disordered to collapse cascade regulation of the blood coagulation, a thrombotic tendency is increased to lead to stenosis or occlusion of blood vessels. The thrombus is a blood component which is coagulated intravascularly, and examples of the component thereof include fibrin, platelet, erythrocyte, leukocyte and the like.

Fibrinolytic system is a rather simple system as compared with the blood coagulation system. However, factors related to the fibrinolytic system are deeply related not only to an intravascular dissolution of thrombus but also to various reactions, which occur in tissues, such as movement or migration of cell; ovulation; cell proliferation; angiogenesis; reconstruction (remodeling) of tissue; inflammatory response and the like. The fibrinolytic system is driven by serine proteases. The plasminogen is converted into plasmin by a plasminogen activator (hereinafter referred to as PA); a tissue-type plasminogen activator (hereinafter referred to as tPA); or a urokinase-type plasminogen activator (hereinafter referred to as uPA), and the resulting plasmin degrades a fibrin thrombus and tissue protein. The fibrinolytic reaction is regulated and modulated by a plasminogen activator inhibitor-1 (PAI-1), a specific inhibitory protein against plasminogen activator existing in vivo. PAI-1 forms a complex with PA in a ratio of one to one to inhibit actions thereof. PAI-1 released from an activated platelet binds to fibrin to exist around the fibrin in a concentrated form, especially at the site of thrombogenesis, and inhibits an activity of tPA effectively. Furthermore, PAI-1 prompts hyperplasia of vascular wall to promote progress of cardiovascular lesion caused by inhibiting degradation of extracellular matrix by a protease. An activity of the fibrinolytic system is regulated by a balance between PA and PAI-1. Therefore, an increase or a decrease of production of PAI-1 in cells or a variation of an activity of PAI-1 molecule is reflected immediately in the activity of the fibrinolytic system in blood. Accordingly, a therapeutic effect for thrombotic diseases is expected by inhibiting PAI-1 activity followed by promoting the activation of PA.

PAI-1 binds to vitronectin which is a cell adhesion molecule to inhibit adhesion of cells to the extracellular matrix. Therefore, a therapeutic effect for diseases caused by movement or migration of cell is also expected. Furthermore, plasmin which is indirectly activated by an inhibition of PAI-1 relates to an activation of transforming growth factor which is a cell proliferation inhibitory cytokine or to an activation of collagenase. Therefore, a therapeutic effect for diseases caused by cell proliferation, angiogenesis, and remodeling of tissue is also expected.

It has been reported that an expression of PAI-1 is increased at the lesion of arteriosclerosis to increase risks of thrombotic diseases such as myocardial infarction, deep vein thrombosis, sepsis, disseminated intravascular coagulation and the like (see, "Proceeding of the National Academy of Sciences of the United States of America," (USA), 1992, Vol. 89, No. 15, p.6998-7002), and a PAI-1 transgenic mouse shows a thrombogenic tendency (refer to "Nature," (England), 1990, Vol. 346, No. 6279, p.74-76).

It has been reported that a mouse model of obesity shows a significantly higher blood level of PAI-1, and it has further been reported that PAI-1 is synthesized not only in endothelial tissues and hepatic tissues but also in adipose tissues, and an amount of synthesized PAI-1 is rapidly increased, especially in visceral fat (see, "Molecular Medicine," (USA), 1996, Vol. 2, No. 5, p.568-582). Furthermore, it has been reported that a PAI-1 gene knock out mouse model of obesity shows a decrease of body weight, and a lowering of blood levels of glucose and insulin (see, "The FASEB Journal," (USA), 2001, Vol. 15, No. 10, p.1840-1842), therefore, these results suggest a possibility that PAI-1 aggravates various symptoms caused by an accumulation of fats. It has been reported that PAI-1 exists specifically in cancerous tissues to be involved in regulation of physiological function of cancer cells, and a PAI-1 antibody inhibits metastasis of cancer in a cancer model (refer to "General & Diagnostic Pathology," (Germany), 1995, Vol. 141, No. 1, p.41-48). It has also been reported that, when a transplantation of malignant keratinocytes into PAI-1 knock out mouse is carried out, invasion of cancer and angiogenesis are inhibited (refer to "Nature Medicine," (USA), 1998, Vol. 4, No. 8, p.923-928).

Furthermore, it has been reported that PAI-1 is secreted from mast cell (see, "The Journal of Immunology," (USA), 2000, Vol. 165, No. 6, p.3154-3161), and an accumulation of extracellular matrix in an airway of a mouse model of asthma is alleviated by PAI-1 knockout (see, "Biochemical and Biophysical Research Communications," (USA), 2002, Vol. 294, No. 5, p.1155-1160).

An arterial lesion as an acute or a chronic rejection after cardiac or renal transplantation is thought to be caused by progressions such as progression of fibrogenesis of tissue, progression of thrombogenesis, progression of proliferation and remodeling of arterial endothelial cell. In experiments of murine cardiac transplantation, when the compound of compound No. 10 described in the present specification was administered, a graft survival was significantly prolonged (control group: 7.2±0.2 days; compound administered group: 9.0±0.7 days (p<0.05)) and an incidence of vascular intimal thickening was decreased to about one third (control group: 64.5±5.8%; compound administered group: 21.7±4.9%(p<0.05)) in comparison with control group. Accordingly, the compounds that can inhibit PAI-1 are considered to have inhibitory effects against acute rejections and arterial lesions after organ transplantation such as cardiac transplantation, renal transplantation or the like.

Therefore, compounds having specific inhibitory activity against PAI-1 are expected to be agents useful for diseases caused by thrombogenesis, fibrogenesis, accumulation of visceral fat, cell proliferation, angiogenesis, deposition and remodeling of extracellular matrix, and cell movement and migration.

An anthranilic acid derivative AR-H029953XX (see, "Biochemistry," (USA), 1998, Vol. 37, No. 5, p.1227-1234); diketopiperazine derivatives (see, the pamphlets of International Patent Publication WO95/32190 and International Patent Publication WO95/21832, and the specification of British Patent Application No. 2372740); indoleacetic acid derivatives (see, the pamphlet of International Patent Publication WO03/000253); aryloxyacetic acid derivatives (see, the pamphlet of International Patent Publication WO03/000258); naphthyloxyacetic acid derivatives (see, the pamphlet of International Patent Publication WO03/000649); naphthylbenzofuran derivatives (see, the pamphlet of International Patent Publication WO03/000671); naphthylindole derivatives (see, the pamphlet of International Patent Publication WO03/000684) and the like are known as the compounds having inhibitory activity against PAI-1.

As the thrombolytic agents clinically used at present, plasminogen activator enzymatic preparation such as tPA, uPA, streptokinase, single-stranded uPA, mutated tPA obtained by modification of the amino acid sequence of tPA and the like are known. However, these agents have serious problems in that route of administration of these agents is either intra-coronary arterial or intravenous injection, and no orally administrable preparations has been provided, these agents have a short half life in blood, and these agents increases a risk of bleeding at the sites other than the thrombotic site, for example, in brain, by an administration thereof.

### Disclosure of the Invention

An object of the present invention is to provide a low molecular compound which is useful for preventive and/or therapeutic treatment of diseases with stenosis or occlusion caused by thrombus.

Another object of the present invention is to provide an antithrombotic compound with few hemorrhagic diatheses by selective inhibition of PAI-1 which is highly expressed in local lesions and a following indirect activation of PA.

Further object of the present invention is to provide a medicament which is provided in the form of injections as well as in a form of preparation depending on a target disease and a purpose of application by using the low molecular compound which inhibits PAI-1.

Still further object of the present invention is to provide low molecular compounds which are useful for preventive and/or therapeutic treatment of other diseases caused by an expression of PAI-1, an enhancement of PAI-1 activity, or a lowering of plasmin activity, for example, diseases caused by fibrogenesis, accumulation of visceral fat, cell proliferation, angiogenesis, deposition or remodeling of extracellular matrix, and cell movement and migration.

The present invention provides novel 5-membered heterocyclic derivatives to solve those objects.

The inventors of the present invention conducted searches for low molecular compounds having inhibitory activity against PAI-1 by using computerized molecular design technology to solve the aforementioned objects. For a three-dimensional structure of an activated form of PAI-1 registered in PDB (Protein Data Bank), binding sites thereof and binding modes of a known PAI-1 inhibitor AR-H029953XX were estimated, and homology modeling of a tree-dimensional structure of PAI-1, which can be bound stably with AR-H029953XX, was carried out. For the obtained ligand-binding type structure of PAI-1, an automatic search program of a three-dimensional low molecular compound database, based on the three-dimensional structure of the protein, was carried out, and compounds potentially be PAI-1 inhibitors were selected by a virtual screening out of compounds registered in a database of compounds commercially available from Sigma-Aldrich, Aldrich, Maybridge, Specs, Bionet, Labotest, Lancaster, Tocros, Tokyo Kasei Kogyo, Wako Pure Chemical Industries and the like, and registered in the compounds that were synthesized originally by the inventors of the present invention. PAI-1 inhibitory activities of these compounds were confirmed by a comparison of a tPA activity in the co-presence of the test compound and PAI-1, with a tPA activity in the presence of PAI-1. As for the compounds confirmed to have PAI-1 inhibitory activity, binding mode thereof to PAI-1 and interactions were analyzed. On the basis of these results, the present invention was achieved by further carrying out syntheses of analogous compounds and tests for confirmation of activities thereof.

The present invention thus provides:
(1) a medicament having inhibitory activity against plasminogen activator inhibitor-1, which comprises as an active ingredient a substance selected from the group consisting of a compound represented by the following general formula (I) and a pharmacologically acceptable salt thereof, and a hydrate thereof and a solvate thereof: 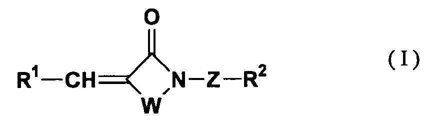 wherein R¹ represents an aromatic group which may be substituted,
   R² represents an aromatic group which may be substituted,
   W represents a group selected from the following connecting group W-1:
   [Connecting Group W-1] 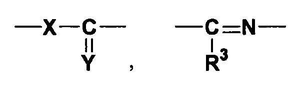 (wherein a bond at the left end binds to the carbon atom and a bond at the right end binds to the nitrogen atom,
   X represents sulfur atom or NH,
   Y represents oxygen atom or sulfur atom,
   R³ represents a hydrocarbon group which may be substituted, hydroxy group which may be substituted, or carboxy group which may be esterified),
   Z represents a single bond or a connecting group wherein a number of atoms in a main chain is 1 to 3 (said connecting group may be substituted).
   According to preferred embodiments of the present invention, provided are:
(2) the medicament according to the aforementioned (1), wherein R¹ is an aromatic group which may be substituted,
   R² is an aromatic group which may be substituted,
   W is a group represented by the following formula: 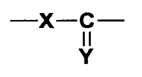 (wherein a bond at the left end binds to the carbon atom and a bond at the right end binds to the nitrogen atom,
   X represents sulfur atom or NH,
   Y represents oxygen atom or sulfur atom),
   Z is a single bond, methylene group, ethylene group, -CH₂CO- group, or -CH₂CONH-group;
(3) the medicament according to the aforementioned (2), wherein R¹ is an aromatic group which may be substituted,
   R² is an aromatic group which may be substituted,
   X is sulfur atom,
   Y is oxygen atom or sulfur atom,
   Z is methylene group;
(4) the medicament according to the aforementioned (3), wherein R¹ is a phenyl group which is substituted with one to three hydroxy groups (said phenyl group may further be substituted with one or more substituents other than the hydroxy group),
   R² is an aromatic group which may be substituted,
   X is sulfur atom,
   Y is oxygen atom or sulfur atom,
   Z is methylene group;
(5) the medicament according to the aforementioned (4), wherein R¹ is a phenyl group which is substituted with two or three hydroxy groups (said phenyl group may further be substituted with one or more substituents other than the hydroxy group),
   R² is a phenyl group which may be substituted,
   X is sulfur atom,
   Y is oxygen atom or sulfur atom,
   Z is methylene group;
(6) the medicament according to the aforementioned (5), wherein the compound represented by the general formula (I) is a compound selected from the following 14 compounds:
   - the compound wherein R¹ is 3,4,5-trihydroxyphenyl group, R² is 3,4-dichlorophenyl group, X is sulfur atom, Y is oxygen atom, Z is methylene group;
   - the compound wherein R¹ is 2,3-dihydroxyphenyl group, R² is 2,3-dichlorophenyl group, X is sulfur atom, Y is oxygen atom, Z is methylene group;
   - the compound wherein R¹ is 2,3-dihydroxyphenyl group, R² is 2,4-dichlorophenyl group, X is sulfur atom, Y is oxygen atom, Z is methylene group;
   - the compound wherein R¹ is 3,4-dihydroxyphenyl group, R² is 2,4-dichlorophenyl group, X is sulfur atom, Y is oxygen atom, Z is methylene group;
   - the compound wherein R¹ is 2,3-dihydroxyphenyl group, R² is 4-bromophenyl group, X is sulfur atom, Y is oxygen atom, Z is methylene group;
   - the compound wherein R¹ is 2,3-dihydroxyphenyl group, R² is 4-(trifluoromethyl)phenyl group, X is sulfur atom, Y is oxygen atom, Z is methylene group;
   - the compound wherein R¹ is 2,3-dihydroxyphenyl group, R² is 4-styrylphenyl group, X is sulfur atom, Y is oxygen atom, Z is methylene group;
   - the compound wherein R¹ is 3,4-dihydroxyphenyl group, R² is 3,4-dichlorophenyl group, X is sulfur atom, Y is sulfur atom, Z is methylene group;
   - the compound wherein R¹ is 2,3-dihydroxyphenyl group, R² is 4-[(3-phenoxyphenyl)acetylamino]phenyl group, X is sulfur atom, Y is oxygen atom, Z is methylene group;
   - the compound wherein R¹ is 2,3-dihydroxyphenyl group, R² is 4-(4-phenoxybenzoylamino)phenyl group, X is sulfur atom, Y is oxygen atom, Z is methylene group;
   - the compound wherein R¹ is 2,3-dihydroxyphenyl group, R² is 4-[3,5-bis(trifluoromethyl)phenylacetylamino]phenyl group, X is sulfur atom, Y is oxygen atom, Z is methylene group;
   - the compound wherein R¹ is 2,3-dihydroxyphenyl group, R² is 3-[(3-phenoxyphenyl)acetylamino]phenyl group, X is sulfur atom, Y is oxygen atom, Z is methylene group;
   - the compound wherein R¹ is 3,4-dihydroxy-5-nitrophenyl group, R² is 3,4-dichlorophenyl group, X is sulfur atom, Y is sulfur atom, Z is methylene group; and
   - the compound wherein R¹ is 3,4-dihydroxy-5-[3-(trifluoromethoxy)phenylcarbamoyl]phenyl group, R² is 3,4-dichlorophenyl group, X is sulfur atom, Y is sulfur atom, Z is methylene group;
(7) the medicament according to the aforementioned (3), wherein R¹ is a phenyl group which is substituted with a C₆-C₁₀ aryl-substituted carbamoyl group (said aryl group may be substituted, and said phenyl group may further be substituted with one or more substituents other than the C₆-C₁₀ aryl-substituted carbamoyl group), or a phenyl group which is substituted with a heteroaryl-substituted carbamoyl group (said heteroaryl group may be substituted, and said phenyl group may further be substituted with one or more substituents other than the heteroaryl-substituted carbamoyl group),
   R² is an aromatic group which may be substituted,
   X is sulfur atom,
   Y is oxygen atom or sulfur atom,
   Z is methylene group;
(8) the medicament according to the aforementioned (7), wherein R¹ is a phenyl group which is substituted with a C₆-C₁₀ aryl-substituted carbamoyl group in the 3-position (said aryl group may be substituted, and said phenyl group may further be substituted with one or more substituents other than the C₆-C₁₀ aryl-substituted carbamoyl group) or a phenyl group which is substituted with a heteroaryl-substituted carbamoyl group (said heteroaryl group may be substituted, and said phenyl group may further be substituted with one or more substituents other than the heteroaryl-substituted carbamoyl group), and a phenyl group which is substituted with hydroxy group in the 4-position (said phenyl group may be substituted in the 5-position),
   R² is a phenyl group which may be substituted,
   X is sulfur atom,
   Y is oxygen atom,
   Z is methylene group;
(9) the medicament according to the aforementioned (8), wherein the compound represented by the general formula (I) is a compound selected from the following 11 compounds:
   - the compound wherein R¹ is 3-methoxy-4-hydroxy-5-(3,4-dihydroxyphenylcarbamoyl)phenyl group, R² is 3,4-dichlorophenyl group, X is sulfur atom, Y is oxygen atom, Z is methylene group;
   - the compound wherein R¹ is 4-hydroxy-3-(3,4-dihydroxyphenylcarbamoyl)phenyl group, R² is 3,4-dichlorophenyl group, X is sulfur atom, Y is oxygen atom, Z is methylene group;
   - the compound wherein R¹ is 4-hydroxy-3-(3,4-dihydroxyphenylcarbamoyl)phenyl group, R² is 3,5-bis(trifluoromethyl)phenyl group, X is sulfur atom, Y is oxygen atom, Z is methylene group;
   - the compound wherein R¹ is 3-methyl-4-hydroxy-5-(3,4-dihydroxyphenylcarbamoyl)phenyl group, R² is 3,4-dichlorophenyl group, X is sulfur atom, Y is oxygen atom, Z is methylene group;
   - the compound wherein R¹ is 4-hydroxy-3-(3,4-dihydroxyphenylcarbamoyl)phenyl group, R² is 4-(trifluoromethyl)phenyl group, X is sulfur atom, Y is oxygen atom, Z is methylene group;
   - the compound wherein R¹ is 4-hydroxy-3-[2-chloro-5-(trifluoromethyl)phenylcarbamoyl]phenyl group, R² is 3,4-dichlorophenyl group, X is sulfur atom, Y is oxygen atom, Z is methylene group;
   - the compound wherein R¹ is 4-hydroxy-3-(3,4,5-trihydroxyphenylcarbamoyl)phenyl group, R² is 3,4-dichlorophenyl group, X is sulfur atom, Y is oxygen atom, Z is methylene group;
   - the compound wherein R¹ is 4-hydroxy-3-(2,3-dihydroxyphenylcarbamoyl)phenyl group, R² is 3,4-dichlorophenyl group, X is sulfur atom, Y is oxygen atom, Z is methylene group;
   - the compound wherein R¹ is 4-hydroxy-3-(2,5-dichlorophenylcarbamoyl)phenyl group, R² is 3,4-dichlorophenyl group, X is sulfur atom, Y is oxygen atom, Z is methylene group;
   - the compound wherein R¹ is 4-hydroxy-3-(2-chloro-5-nitrophenylcarbamoyl)phenyl group, R² is 3,4-dichlorophenyl group, X is sulfur atom, Y is oxygen atom, Z is methylene group; and
   - the compound wherein R¹ is 3,4-dihydroxy-5-[3-(trifluoromethyl)phenylcarbamoyl]phenyl group, R² is 3,4-dichlorophenyl group, X is sulfur atom, Y is oxygen atom, Z is methylene group;
(10) the medicament according to the aforementioned (3), wherein R¹ and R² are either the following (i) or (ii),
   X is sulfur atom,
   Y is oxygen atom or sulfur atom,
   Z is methylene group:
   (i) R¹ is a phenyl group which is substituted with carboxy group (said phenyl group may further be substituted with one or more substituents other than the carboxy group), R² is an aromatic group which may be substituted;
   (ii) R¹ is an aromatic group which may be substituted,
      R² is a phenyl group which is substituted with carboxy group (said phenyl group may further be substituted with one or more substituents other than the carboxy group);
(11) the medicament according to the aforementioned (10), wherein the compound represented by the general formula (I) is a compound selected from the following 2 compounds:
   - the compound wherein R¹ is 3-carboxyphenyl group, R² is 3,4-dichlorophenyl group, X is sulfur atom, Y is oxygen atom, Z is methylene group; and
   - the compound wherein R¹ is 3-carboxy-4-hydroxyphenyl group, R² is 4-methoxycarbonylphenyl group, X is sulfur atom, Y is oxygen atom, Z is methylene group;
(12) the medicament according to the aforementioned (2), wherein the compound represented by the general formula (I) is a compound selected from the group consisting of the compounds described in the present specification as Compound Nos. 1 to 93 and Compound Nos. 301 to 475.
(13) the medicament according to the aforementioned (1), wherein R¹ is an aromatic group which may be substituted,
   R² is an aromatic group which may be substituted,
   W is a group represented by the following formula: 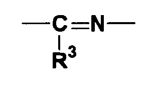 (wherein a bond at the left end binds to the carbon atom and a bond at the right end binds to the nitrogen atom,
   R³ represents a hydrocarbon group which may be substituted, hydroxy group which may be substituted, or carboxy group which may be esterified),
   Z is a single bond or methylene group;
(14) the medicament according to the aforementioned (13), wherein R¹ is an aromatic group which may be substituted,
   R² is an aromatic group which may be substituted,
   R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
   Z is a single bond;
(15) the medicament according to the aforementioned (14), wherein R¹ is a phenyl group which is substituted with one to three hydroxy groups (said phenyl group may further be substituted with one or more substituents other than the hydroxy group), R² is an aromatic group which may be substituted,
   R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
   Z is a single bond;
(16) the medicament according to the aforementioned (15), wherein R¹ is a phenyl group which is substituted with two or three hydroxy groups (said phenyl group may further be substituted with one or more substituents other than the hydroxy group), R² is a phenyl group which may be substituted,
   R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
   Z is a single bond;
(17) the medicament according to the aforementioned (16), wherein the compound represented by the general formula (I) is a compound selected from the following 6 compounds:
   - the compound wherein R¹ is 2,4,5-trihydroxyphenyl group, R² is 3,5-dichlorophenyl group, R³ is phenyl group, Z is a single bond;
   - the compound wherein R¹ is 2,4,5-trihydroxyphenyl group, R² is 3,4-dichlorophenyl group, R³ is isopropyl group, Z is a single bond;
   - the compound wherein R¹ is 2,4,5-trihydroxyphenyl group, R² is 4-nitrophenyl group, R³ is ethoxy group, Z is a single bond;
   - the compound wherein R¹ is 2,4,5-trihydroxyphenyl group, R² is 3,4-dichlorophenyl group, R³ is methyl group, Z is a single bond;
   - the compound wherein R¹ is 2,4,5-trihydroxyphenyl group, R² is 3,5-dichlorophenyl group, R³ is isopropyl group, Z is a single bond; and
   - the compound wherein R¹ is 2,4,5-trihydroxyphenyl group, R² is 3-chloro-4-fluorophenyl group, R³ is isopropyl group, Z is a single bond;
(18) the medicament according to the aforementioned (14), wherein R¹ is a phenyl group which is substituted with C₂-C₆ alkenyl group which may be substituted (said phenyl group may further be substituted with one or more substituents other than the C₂-C₆ alkenyl group),
   R² is an aromatic group which may be substituted,
   R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
   Z is a single bond;
(19) the medicament according to the aforementioned (18), wherein the compound represented by the general formula (I) is a compound selected from the following 2 compounds:
   - the compound wherein R¹ is 2-hydroxy-5-styrylphenyl group, R² is 4-nitrophenyl group, R³ is ethoxy group, Z is a single bond; and
   - the compound wherein R¹ is 4-styrylphenyl group, R² is 4-carboxyphenyl group, R³ is isopropyl group, Z is a single bond.
(20) the medicament according to the aforementioned (14), wherein R¹, R² and R³ are any one of the following (i) to (iii), Z is a single bond:
   (i) R¹ is a phenyl group which is substituted with carboxy group (said phenyl group may further be substituted with one or more substituents other than the carboxy group), R² is an aromatic group which may be substituted,
      R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group;
   (ii) R¹ is an aromatic group which may be substituted,
      R² is a phenyl group which is substituted with carboxy group (said phenyl group may further be substituted with one or more substituents other than the carboxy group),
      R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group;
   (iii) R¹ is an aromatic group which may be substituted,
      R² is an aromatic group which may be substituted,
      R³ is carboxy group;
(21) the medicament according to the aforementioned (20), wherein the compound represented by the general formula (I) is a following compound:
   - the compound wherein R¹ is 4-styrylphenyl group, R² is 4-carboxyphenyl group, R³ is isopropyl group, Z is a single bond; and
(22) the medicament according to the aforementioned (13), wherein the compound represented by the general formula (I) is a compound selected from the group consisting of the compounds described in the present specification as Compound Nos. 101 to 224 and Compound Nos. 501 to 567.

Furthermore, according to preferred embodiments of the aforementioned invention, provided are the aforementioned medicament for therapeutic and/or preventive treatment of diseases caused by an expression of PAI-1 or an enhancement of PAI-1 activity; the aforementioned medicament for preventive and/or therapeutic treatment of diseases caused by one or more abnormal conditions selected from the group consisting of thrombogenesis, fibrogenesis, accumulation of visceral fat, cell proliferation, angiogenesis, deposition or remodeling of extracellular matrix, and cell movement or migration.

From another aspect, the present invention provides use of the substance selected from the group consisting of the compound represented by the aforementioned general formula (I) and the pharmacologically acceptable salt thereof, and the hydrate thereof and the solvate thereof for manufacture of the aforementioned medicament; a PAI-1 inhibitor which comprises as an active ingredient a substance selected from the group consisting of the compound represented by the aforementioned general formula (I) and a pharmacologically acceptable salt thereof, and a hydrate thereof and a solvate thereof; a method for inhibiting PAI-1 in mammal including a human, which comprises the step of administering an effective amount of the substance selected from the group consisting of the compound represented by the aforementioned general formula (I) and the pharmacologically acceptable salt thereof, and the hydrate thereof and the solvate thereof to a mammal including a human; a method for therapeutic and/or preventive treatment of diseases caused by an expression of PAI-1 or an enhancement of PAI-1 activity in mammal including a human, which comprises the step of administering a therapeutically and/or preventively effective amount of the substance selected from the group consisting of the compound represented by the aforementioned general formula (I) and the pharmacologically acceptable salt thereof, and the hydrate thereof and the solvate thereof to a mammal including a human; and a method for preventive and/or therapeutic treatment of diseases caused by one or more abnormal conditions selected from the group consisting of thrombogenesis, fibrogenesis, accumulation of visceral fat, cell proliferation, angiogenesis, deposition or remodeling of extracellular matrix, and cell movement or migration in mammal including a human, which comprises the step of administering a therapeutically and/or preventively effective amount of the substance selected from the group consisting of the compound represented by the aforementioned general formula (I) and the pharmacologically acceptable salt thereof, and the hydrate thereof and the solvate thereof to a mammal including a human.

From further another aspect, the present invention provides:
(25) a compound represented by the general formula (II) or a salt thereof, or a hydrate thereof or a solvate thereof as a novel compound: 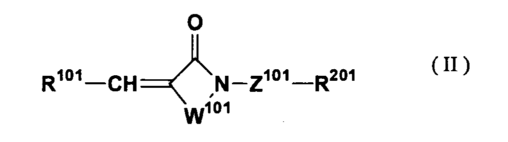 wherein R¹⁰¹ represents an aromatic group which may be substituted,
   R²⁰¹ represents an aromatic group which may be substituted,
   W¹⁰¹ represents a group selected from the following connecting group W-101-1: 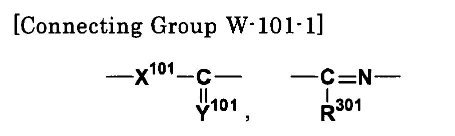 (wherein a bond at the left end binds to the carbon atom and a bond at the right end binds to the nitrogen atom,
   X¹⁰¹ represents sulfur atom or NH,
   Y¹⁰¹ represents oxygen atom or sulfur atom,
   R³⁰¹ represents a hydrocarbon group which may be substituted, hydroxy group which may be substituted, or carboxy group which may be esterified),
   Z¹⁰¹ represents a single bond or a connecting group wherein a number of atoms in a main chain is 1 to 3 (said connecting group may be substituted),
   provided that the following compounds are excluded:
   - the compound wherein R¹⁰¹ is 3,5-dibromo-2-hydroxyphenyl group, R²⁰¹ is phenyl group, W¹⁰¹ is -X¹⁰¹-C(=Y¹⁰¹)-, X¹⁰¹ is sulfur atom, Y¹⁰¹ is oxygen atom, Z¹⁰¹ is a single bond;
   - the compound wherein R¹⁰¹ is 5-(3-carboxyphenyl)furan-2-yl group, R²⁰¹ is 3-fluorophenyl group, W¹⁰¹ is -X¹⁰¹-C(=Y¹⁰¹)-, X¹⁰¹ is sulfur atom, Y¹⁰¹ is sulfur atom, Z¹⁰¹ is a single bond;
   - the compound wherein R¹⁰¹ is 3,5-dibromo-2-hydroxyphenyl group, R²⁰¹ is 4-chlorophenyl group, W¹⁰¹ is ·X¹⁰¹·C(=Y101)., X¹⁰¹ is NH, Y¹⁰¹ is sulfur atom, Z¹⁰¹ is a single bond;
   - the compound wherein R¹⁰¹ is 5-(3-carboxyphenyl)furan-2-yl group, R²⁰¹ is 4-methoxyphenyl group, W¹⁰¹ is ·X¹⁰¹·C(=Y¹⁰¹)-, X¹⁰¹ is sulfur atom, Y¹⁰¹ is sulfur atom, Z¹⁰¹ is methylene group;
   - the compound wherein R¹⁰¹ is 5-chloro-2-hydroxyphenyl group, R²⁰¹ is phenyl group, W¹⁰¹ is -X¹⁰¹-C(=Y¹⁰¹)-, X¹⁰¹ is sulfur atom, Y¹⁰¹ is sulfur atom, Z¹⁰¹ is a single bond;
   - the compound wherein R¹⁰¹ is 3,5-dibromo-2-hydroxyphenyl group, R²⁰¹ is phenyl group, W¹⁰¹ is -C(R³⁰¹)=N-, R³⁰¹ is hydroxy group, Z¹⁰¹ is a single bond;
   - the compound wherein R¹⁰¹ is 5-bromo-2-hydroxyphenyl group, R²⁰¹ is 3,4-dichlorophenyl group, W¹⁰¹ is -C(R³⁰¹)=N-, R³⁰¹ is methyl group, Z¹⁰¹ is a single bond;
   - the compound wherein R¹⁰¹ is 3-.ethoxy-4-{[(thiophen-2-yl)carbonyl]oxy}phenyl group, R²⁰¹ is 3-carboxyphenyl group, W¹⁰¹ is -C(R³⁰¹)=N-, R³⁰¹ is methyl group, Z¹⁰¹ is a single bond;
   - the compound wherein R¹⁰¹ is 4-carboxymethoxy-3-ethoxyphenyl group, R²⁰¹ is 3-(trifluoromethyl)phenyl group, W¹⁰¹ is -C(R³⁰¹)=N-, R³⁰¹ is methyl group, Z¹⁰¹ is a single bond;
   - the compound wherein R¹⁰¹ is 5-[3-(methoxycarbonyl)phenyl]furan-2-yl group, R²⁰¹ is 4-fluorophenyl group, W¹⁰¹ is -C(R³⁰¹)=N⁻, R³⁰¹ is hydroxy group, Z¹⁰¹ is a single bond;
   - the compound wherein R¹⁰¹ is 5-(4-carboxyphenyl)furan-2-yl group, R²⁰¹ is 4-chlorophenyl group, W¹⁰¹ is -C(R³⁰¹)=N-, R³⁰¹ is hydroxy group, Z¹⁰¹ is a single bond;
   - the compound wherein R¹⁰¹ is 5-(3,5-dichlorophenyl)furan-2-yl group, R²⁰¹ is 3-carboxyphenyl group, W¹⁰¹ is -C(R³⁰¹)=N⁻, R³⁰¹ is trifluoromethyl group, Z¹⁰¹ is a single bond;
   - the compound wherein R¹⁰¹ is 5-(5-carboxy-2-chlorophenyl)furan-2-yl group, R²⁰¹ is phenyl group, W¹⁰¹ is -C(R³⁰¹)=N-, R³⁰¹ is methyl group, Z¹⁰¹ is a single bond;
   - the compound wherein R¹⁰¹ is 4-bromophenyl group, R²⁰¹ is 3-carboxy-4-chlorophenyl group, W¹⁰¹ is -C(R³⁰¹)=N-, R³⁰¹ is trifluoromethyl group, Z¹⁰¹ is a single bond;
   - the compound wherein R¹⁰¹ is 5-(2-hydroxy-5-nitrophenyl)furan-2-yl group, R²⁰¹ is 3-chlorophenyl group, W¹⁰¹ is -C(R³⁰¹)=N-, R³⁰¹ is hydroxy group, Z¹⁰¹ is a single bond;
   - the compound wherein R¹⁰¹ is 4-bromophenyl group, R²⁰¹ is 3-carboxy-4-chlorophenyl group, W¹⁰¹ is -C(R³⁰¹)=N-, R³⁰¹ is methyl group, Z¹⁰¹ is a single bond;
   - the compound wherein R¹⁰¹ is 4-allyloxy-3-methoxyphenyl group, R²⁰¹ is 3-carboxy-4-chlorophenyl group, W¹⁰¹ is -C(R³⁰¹)-IV-, R³⁰¹ is methyl group, Z¹⁰¹ is a single bond;
   - the compound wherein R¹⁰¹ is 4-benzyloxy-3-methoxyphenyl group, R²⁰¹ is 3-carboxy-4-chlorophenyl group, W¹⁰¹ is -C(R³⁰¹)=N -, R³⁰¹ is methyl group, Z¹⁰¹ is a single bond;
   - the compound wherein R¹⁰¹ is 4-chlorophenyl group, R²⁰¹ is phenyl group, W¹⁰¹ is -C(R³⁰¹)=N-, R³⁰¹ is carboxy group, Z¹⁰¹ is a single bond;
   - the compound wherein R¹⁰¹ is phenyl group, R²⁰¹ is 3-carboxyphenyl group, W¹⁰¹ is -C(R³⁰¹)-N-, R³⁰¹ is methyl group, Z¹⁰¹ is a single bond;
   - the compound wherein R¹⁰¹ is 2-benzyloxy-5-bromophenyl group, R²⁰¹ is 3-carboxyphenyl group, W¹⁰¹ is -C(R³⁰¹)=N-, R³⁰¹ is methyl group, Z¹⁰¹ is a single bond;
   - the compound wherein R¹⁰¹ is 4-(benzylcarbamoyl)methoxy-3-bromo-5-methoxyphenyl group, R²⁰¹ is 3-carboxyphenyl group, W¹⁰¹ is -C(R³⁰¹)-N-, R³⁰¹ is methyl group, Z¹⁰¹ is a single bond;
   - the compound wherein R¹⁰¹ is phenyl group, R²⁰¹ is 3-carboxyphenyl group, W¹⁰¹ is -C(R³⁰¹)=N-, R³⁰¹ is phenyl group, Z¹⁰¹ is a single bond;
   - the compound wherein R¹⁰¹ is 5-(3-chlorophenyl)furan-2-yl group, R²⁰¹ is 4-sulfamoylphenyl group, W¹⁰¹ is -C(R³⁰¹)=N-, R³⁰¹ is methyl group, Z¹⁰¹ is a single bond;
   - the compound wherein R¹⁰¹ is 5-(3-chloro-4-methylphenyl)furan-2-yl group, R²⁰¹ is 3-carboxyphenyl group, W¹⁰¹ is -C(R³⁰¹)-N-, R³⁰¹ is methyl group, Z¹⁰¹ is a single bond;
   - the compound wherein R¹⁰¹ is 5-(2-chlorophenyl)furan-2-yl group, R²⁰¹ is 3-carboxy-4-chlorophenyl group, W¹⁰¹ is -C(R³⁰¹)=N-, R³⁰¹ is methyl group, Z¹⁰¹ is a single bond;
   - the compound wherein R¹⁰¹ is 5-[3-(methoxycarbonyl)phenyl]furan-2-yl group, R²⁰¹ is 3-carboxyphenyl group, W¹⁰¹ is -C(R³⁰¹)=N-, R³⁰¹ is methyl group, Z¹⁰¹ is a single bond;
   - the compound wherein R¹⁰¹ is 5-(3-chloro-2-methylphenyl)furan-2-yl group, R²⁰¹ is 3-carboxy-4-chlorophenyl group, W¹⁰¹ is -C(R³⁰¹)=N-, R³⁰¹ is methyl group, Z¹⁰¹ is a single bond;
   - the compound wherein R¹⁰¹ is 5-(3,5-dichlorophenyl)furan-2-yl group, R²⁰¹ is 3-carboxyphenyl group, W¹⁰¹ is -C(R³⁰¹)=N-, R³⁰¹ is methyl group, Z¹⁰¹ is a single bond;
   - the compound wherein R¹⁰¹ is 5-(3-fluorophenyl)furan-2-yl group, R²⁰¹ is 3-carboxy-4-chlorophenyl group, W¹⁰¹ is -C(R³⁰¹)=N-, R³⁰¹ is methyl group, Z¹⁰¹ is a single bond;
   - the compound wherein R¹⁰¹ is 5-(3-nitrophenyl)furan-2-yl group, R²⁰¹ is 3-carboxyphenyl group, W¹⁰¹ is -C(R³⁰¹)=N-, R³⁰¹ is methyl group, Z¹⁰¹ is a single bond; and
   - the compound wherein R¹⁰¹ is 5-(5-carboxy-2-chlorophenyl)furan-2-yl group, R²⁰¹ is 3-(trifluoromethyl)phenyl group, W¹⁰¹ is -C(R³⁰¹)=N-, R³⁰¹ is hydroxy group, Z¹⁰¹ is a single bond.

According to preferred embodiments of the present invention, provided are:
(26) the compound according to the aforementioned (25) or a salt thereof, or a hydrate thereof or a solvate thereof, wherein R¹⁰¹ is a phenyl group which is substituted with one to three hydroxy groups (said phenyl group may further be substituted with one or more substituents other than the hydroxy group),
   R²⁰¹ is an aromatic group which may be substituted,
   W¹⁰¹ is a group represented by the following formula: 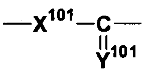 (wherein a bond at the left end binds to the carbon atom and a bond at the right end binds to the nitrogen atom,
   X¹⁰¹ represents sulfur atom,
   Y¹⁰¹ represents oxygen atom or sulfur atom),
   Z¹⁰¹ is methylene group;
(27) the compound according to the aforementioned (26) or a salt thereof, or a hydrate thereof or a solvate thereof, wherein R¹⁰¹ is a phenyl group which is substituted with two or three hydroxy groups (said phenyl group may further be substituted with one or more substituents other than the hydroxy group),
   R²⁰¹ is a phenyl group which may be substituted,
   X¹⁰¹ is sulfur atom,
   Y¹⁰¹ is oxygen atom or sulfur atom),
   Z¹⁰¹ is methylene group;
(28) the compound according to the aforementioned (27) or a salt thereof, or a hydrate thereof or a solvate thereof, wherein the compound represented by the general formula (II) is a compound selected from the following 14 compounds:
   the compound wherein R¹⁰¹ is 3,4,5-trihydroxyphenyl group, R²⁰¹ is 3,4-dichlorophenyl group, X¹⁰¹ is sulfur atom, Y¹⁰¹ is oxygen atom, Z¹⁰¹ is methylene group;
   • the compound wherein R¹⁰¹ is 2,3-dihydroxyphenyl group, R²⁰¹ is 2,3-dichlorophenyl group, X¹⁰¹ is sulfur atom, Y¹⁰¹ is oxygen atom, Z¹⁰¹ is methylene group;
   • the compound wherein R¹⁰¹ is 2,3-dihydroxyphenyl group, R²⁰¹ is 2,4-dichlorophenyl group, X¹⁰¹ is sulfur atom, Y¹⁰¹ is oxygen atom, Z¹⁰¹ is methylene group;
   • the compound wherein R¹⁰¹ is 3,4-dihydroxyphenyl group, R²⁰¹ is 2,4-dichlorophenyl group, X¹⁰¹ is sulfur atom, Y¹⁰¹ is oxygen atom, Z¹⁰¹ is methylene group;
   • the compound wherein R¹⁰¹ is 2,3-dihydroxyphenyl group, R²⁰¹ is 4-bromophenyl group, X¹⁰¹ is sulfur atom, Y¹⁰¹ is oxygen atom, Z¹⁰¹ is methylene group;
   • the compound wherein R¹⁰¹ is 2,3-dihydroxyphenyl group, R²⁰¹ is 4-(trifluoromethyl)phenyl group, X¹⁰¹ is sulfur atom, Y¹⁰¹ is oxygen atom, Z¹⁰¹ is methylene group;
   • the compound wherein R¹⁰¹ is 2,3-dihydroxyphenyl group, R²⁰¹ is 4-styrylphenyl group, X¹⁰¹ is sulfur atom, Y¹⁰¹ is oxygen atom, Z¹⁰¹ is methylene group;
   • the compound wherein R¹⁰¹ is 3,4-dihydroxyphenyl group, R²⁰¹ is 3,4-dichlorophenyl group, X¹⁰¹ is sulfur atom, Y¹⁰¹ is sulfur atom, Z¹⁰¹ is methylene group;
   • the compound wherein R¹⁰¹ is 2,3-dihydroxyphenyl group, R²⁰¹ is 4-[(3-phenoxyphenyl)acetylamino]phenyl group, X¹⁰¹ is sulfur atom, Y¹⁰¹ is oxygen atom, Z¹⁰¹ is methylene group;
   • the compound wherein R¹⁰¹ is 2,3-dihydroxyphenyl group, R²⁰¹ is 4-(4-phenoxybenzoylamino)phenyl group, X¹⁰¹ is sulfur atom, Y¹⁰¹ is oxygen atom, Z¹⁰¹ is methylene group;
   • the compound wherein R¹⁰¹ is 2,3-dihydroxyphenyl group, R²⁰¹ is 4-[3,5-bis(trifluoromethyl)phenylacetylamino]phenyl group, X¹⁰¹ is sulfur atom, Y¹⁰¹ is oxygen atom, Z¹⁰¹ is methylene group;
   • the compound wherein R¹⁰¹ is 2,3-dihydroxyphenyl group, R²⁰¹ is 3-[(3-phenoxyphenyl)acetylamino]phenyl group, X¹⁰¹ is sulfur atom, Y¹⁰¹ is oxygen atom, Z¹⁰¹ is methylene group;
   • the compound wherein R¹⁰¹ is 3,4-dihydroxy-5-nitrophenyl group, R²⁰¹ is 3,4-dichlorophenyl group, X¹⁰¹ is sulfur atom, Y¹⁰¹ is sulfur atom, Z¹⁰¹ is methylene group; and
   • the compound wherein R¹⁰¹ is 3,4-dihydroxy-5-[3-(trifluoromethoxy)phenylcarbamoyl]phenyl group, R²⁰¹ is 3,4-dichlorophenyl group, X¹⁰¹ is sulfur atom, Y¹⁰¹ is sulfur atom, Z¹⁰¹ is methylene group;
(29) the compound according to the aforementioned (25) or a salt thereof, or a hydrate thereof or a solvate thereof, wherein R¹⁰¹ is a phenyl group which is substituted with a C₆-C₁₀ aryl-substituted carbamoyl group (said aryl group may be substituted, and said phenyl group may further be substituted with one or more substituents other than the C₆-C₁₀ aryl-substituted carbamoyl group), or a phenyl group which is substituted with a heteroaryl-substituted carbamoyl group (said heteroaryl group may be substituted, and said phenyl group may further be substituted with one or more substituents other than the heteroaryl-substituted carbamoyl group),
   R²⁰¹ is an aromatic group which may be substituted,
   W¹⁰¹ is a group represented by the following formula: 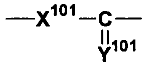 (wherein a bond at the left end binds to the carbon atom and a bond at the right end binds to the nitrogen atom,
   X¹⁰¹ represents sulfur atom,
   Y¹⁰¹ represents oxygen atom or sulfur atom),
   Z¹⁰¹ is methylene group;
(30) the compound according to the aforementioned (29) or a salt thereof, or a hydrate thereof or a solvate thereof, wherein R¹ is a phenyl group which is substituted with a C₆-C₁₀ aryl-substituted carbamoyl group in the 3-position (said aryl group may be substituted, and said phenyl group may further be substituted with one or more substituents other than the C₆-C₁₀ aryl-substituted carbamoyl group) or a phenyl group which is substituted with a heteroaryl-substituted carbamoyl group (said heteroaryl group may be substituted, and said phenyl group may further be substituted with one or more substituents other than the C₆-C₁₀ aryl-substituted carbamoyl group), and a phenyl group which is substituted with hydroxy group in the 4-position (said phenyl group may be substituted in the 5-position),
   R²⁰¹ is a phenyl group which may be substituted,
   X¹⁰¹ is sulfur atom,
   Y¹⁰¹ is oxygen atom,
   Z¹⁰¹ is methylene group;
(31) the compound according to the aforementioned (30) or a salt thereof, or a hydrate thereof or a solvate thereof, wherein the compound represented by the general formula (II) is a compound selected from the following 11 compounds:
   - the compound wherein R¹⁰¹ is 3-methoxy-4-hydroxy-5-(3,4-dihydroxyphenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
   - the compound wherein R¹⁰¹ is 4-hydroxy-3-(3,4-dihydroxyphenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
   - the compound wherein R¹⁰¹ is 4-hydroxy-3-(3,4-dihydroxyphenylcarbamoyl)phenyl group, R²⁰¹ is 3,5-bis(trifluoromethyl)phenyl group, X¹⁰¹ is sulfur atom, Y¹⁰¹ is oxygen atom, Z¹⁰¹ is methylene group;
   - the compound wherein R¹⁰¹ is 3-methyl-4-hydroxy-5-(3,4-dihydroxyphenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
   - the compound wherein R¹⁰¹ is 4-hydroxy-3-(3,4-dihydroxyphenylcarbamoyl)phenyl group, R²⁰¹ is 4-(trifluoromethyl)phenyl group, X¹⁰¹ is sulfur atom, Y¹⁰¹ is oxygen atom, Z¹⁰¹ is methylene group;
   - the compound wherein R¹⁰¹ is 4-hydroxy-3-[2-chloro-5-(trifluoromethyl)phenylcarbamoyl]phenyl group, R²⁰¹ is 3,4-dichlorophenyl group, X¹⁰¹ is sulfur atom, Y¹⁰¹ is oxygen atom, Z¹⁰¹ is methylene group;
   - the compound wherein R¹⁰¹ is 4-hydroxy-3-(3,4,5-trihydroxyphenylearbamoyl)phenyl group, R²⁰¹ is 3,4-dichlorophenyl group, X¹⁰¹ is sulfur atom, Y¹⁰¹ is oxygen atom, Z¹⁰¹ is methylene group;
   - the compound wherein R¹⁰¹ is 4-hydroxy-3-(2,3-dihydroxyphenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
   - the compound wherein R¹⁰¹ is 4-hydroxy-3-(2,5-dichlorophenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
   - the compound wherein R¹⁰¹ is 4-hydroxy-3-(2-chloro-5-nitrophenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group; and
   - the compound wherein R¹⁰¹ is 3,4-dihydroxy-5-[3-(trifluoromethyl)phenylcarbamoyl]phenyl group, R²⁰¹ is 3,4-dichlorophenyl group, X¹⁰¹ is sulfur atom, Y¹⁰¹ is oxygen atom, Z¹⁰¹ is methylene group;
(32) the compound according to the aforementioned (25) or a salt thereof, or a hydrate thereof or a solvate thereof, wherein R¹⁰¹ and R²⁰¹ are either the following (i) or (ii), X¹⁰¹ is sulfur atom,
   Y¹⁰¹ is oxygen atom or sulfur atom,
   Z¹⁰¹ is methylene group:
   (i) R¹⁰¹ is a phenyl group which is substituted with carboxy group (said phenyl group may further be substituted with one or more substituents other than the carboxy group), R²⁰¹ is an aromatic group which may be substituted;
   (ii) R¹⁰¹ is an aromatic group which may be substituted,
      R²⁰¹ is a phenyl group which is substituted with carboxy group (said phenyl group may further be substituted with one or more substituents other than the carboxy group);
(33) the compound according to the aforementioned (32) or a salt thereof, or a hydrate thereof or a solvate thereof, wherein the compound represented by the general formula (II) is a compound selected from the following 2 compounds:
   - the compound wherein R¹⁰¹ is 3-carboxyphenyl group, R²⁰¹ is 3,4-dichlorophenyl group, X¹⁰¹ is sulfur atom, Y¹⁰¹ is oxygen atom, Z¹⁰¹ is methylene group; and
   - the compound wherein R¹⁰¹ is 3-carboxy-4-hydroxyphenyl group, R²⁰¹ is 4-methoxycarbonylphenyl group, X¹⁰¹ is sulfur atom, Y¹⁰¹ is oxygen atom, Z¹⁰¹ is methylene group;
(34) the compound according to the aforementioned (25) or a salt thereof, or a hydrate thereof or a solvate thereof, wherein the compound represented by the general formula (II) is a compound selected from the group consisting of the compounds described in the present specification as Compound Nos. 2 to 61, Compound Nos. 66 to 93, and Compound Nos. 301 to 475;
(35) the compound according to the aforementioned (25) or a salt thereof, or a hydrate thereof or a solvate thereof, wherein R¹⁰¹ is a phenyl group which is substituted with one to three hydroxy groups (said phenyl group may further be substituted with one or more substituents other than the hydroxy group),
   R²⁰¹ is an aromatic group which may be substituted,
   W¹⁰¹ is a group represented by the following formula: 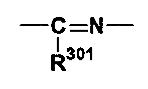 (wherein a bond at the left end binds to the carbon atom and a bond at the right end binds to the nitrogen atom,
   R³⁰¹ represents a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group),
   Z¹⁰¹ is a single bond;
(36) the compound according to the aforementioned (35) or a salt thereof, or a hydrate thereof or a solvate thereof, wherein R¹⁰¹ is a phenyl group which is substituted with two or three hydroxy groups (said phenyl group may further be substituted with one or more substituents other than the hydroxy group),
   R²⁰¹ is a phenyl group which may be substituted,
   R³⁰¹ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
   Z¹⁰¹ is a single bond;
(37) the compound according to the aforementioned (36) or a salt thereof, or a hydrate thereof or a solvate thereof, wherein the compound represented by the general formula (II) is a compound selected from the following 6 compounds:
   - the compound wherein R¹⁰¹ is 2,4,5-trihydroxyphenyl group, R²⁰¹ is 3,5-dichlorophenyl group, R³⁰¹ is phenyl group, Z¹⁰¹ is a single bond;
   - the compound wherein R¹⁰¹ is 2,4,5-trihydroxyphenyl group, R²⁰¹ is 3,4-dichlorophenyl group, R³⁰¹ is isopropyl group, Z¹⁰¹ is a single bond;
   - the compound wherein R¹⁰¹ is 2,4,5-trihydroxyphenyl group, R²⁰¹ is 4-nitrophenyl group, R³⁰¹ is ethoxy group, Z¹⁰¹ is a single bond;
   - the compound wherein R¹⁰¹ is 2,4,5-trihydroxyphenyl group, R²⁰¹ is 3,4-dichlorophenyl group, R³⁰¹ is methyl group, Z¹⁰¹ is a single bond;
   - the compound wherein R¹⁰¹ is 2,4,5-trihydroxyphenyl group, R²⁰¹ is 3,5-dichlorophenyl group, R³⁰¹ is isopropyl group, Z¹⁰¹ is a single bond; and
   - the compound wherein R¹⁰¹ is 2,4,5-trihydroxyphenyl group, R²⁰¹ is 3-chloro-4-fluorophenyl group, R³⁰¹ is isopropyl group, Z¹⁰¹ is a single bond;
(38) the compound according to the aforementioned (25) or a salt thereof, or a hydrate thereof or a solvate thereof, wherein R¹⁰¹ is a phenyl group which is substituted with C₂-C₆ alkenyl group which may be substituted (said phenyl group may further be substituted with one or more substituents other than the C₂-C₆ alkenyl group),
   R²⁰¹ is an aromatic group which may be substituted,
   W¹⁰¹ is a group represented by the following formula: 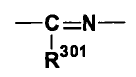 (wherein a bond at the left end binds to the carbon atom and a bond at the right end binds to the nitrogen atom,
   R³⁰¹ represents a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group),
   Z¹⁰¹ is a single bond;
(39) the compound according to the aforementioned (38) or a salt thereof, or a hydrate thereof or a solvate thereof, wherein the compound represented by the general formula (II) is a compound selected from the following 2 compounds:
   - the compound wherein R¹⁰¹ is 2-hydroxy-5-styrylphenyl group, R²⁰¹ is 4-nitrophenyl group, R³⁰¹ is ethoxy group, Z¹⁰¹ is a single bond; and
   - the compound wherein R¹⁰¹ is 4-styrylphenyl group, R²⁰¹ is 4-carboxyphenyl group, R³⁰¹ is isopropyl group, Z¹⁰¹ is a single bond;
(40) the compound according to the aforementioned (25) or a salt thereof, or a hydrate thereof or a solvate thereof, wherein R¹⁰¹ and R²⁰¹ are any one of the following (i) to (iii),
   W¹⁰¹ is a group represented by the following formula: 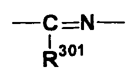 (wherein a bond at the left end binds to the carbon atom and a bond at the right end binds to the nitrogen atom),
   R³⁰¹ is any one of the following (i) to (iii),
   Z is a single bond:
   (i) R¹⁰¹ is a phenyl group which is substituted with carboxy group (said phenyl group may further be substituted with one or more substituents other than the carboxy group), R²⁰¹ is an aromatic group which may be substituted,
      R³⁰¹ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group;
   (ii) R¹⁰¹ is an aromatic group which may be substituted,
      R²⁰¹ is a phenyl group which is substituted with carboxy group (said phenyl group may further be substituted with one or more substituents other than the carboxy group), R³⁰¹ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group;
   (iii) R¹⁰¹ is an aromatic group which may be substituted,
      R²⁰¹ is an aromatic group which may be substituted,
      R³⁰¹ is carboxy group;
(41) the compound according to the aforementioned (40) or a salt thereof, or a hydrate thereof or a solvate thereof, wherein the compound represented by the general formula (II) is a following compound:
   - the compound wherein R¹⁰¹ is 4-styrylphenyl group, R²⁰¹ is 4-carboxyphenyl group, R³⁰¹ is isopropyl group, Z¹⁰¹ is a single bond; and
(42) the compound according to the aforementioned (25) or a salt thereof, or a hydrate thereof or a solvate thereof, wherein the compound represented by the general formula (II) is a compound selected from the group consisting of the compounds described in the present specification as Compound Nos. 104 to 158, Compound Nos. 166 to 167, Compound Nos. 176 to 185, Compound Nos. 194 to 219, Compound Nos. 221 to 224, and Compound Nos. 501 to 567.

From another aspect, the present invention provides a medicament which comprises as an active ingredient a substance selected from the group consisting of a compound represented by the aforementioned general formula (II) and a pharmacologically acceptable salt thereof, and a hydrate thereof and a solvate thereof use of the substance selected from the group consisting of the compound represented by the aforementioned general formula (II) and the pharmacologically acceptable salt thereof, and the hydrate thereof and the solvate thereof for manufacture of the aforementioned medicament; a medicament having inhibitory activity against plasminogen activator inhibitor-1, which comprises as an active ingredient a substance selected from the group consisting of a compound represented by the aforementioned general formula (II) and a pharmacologically acceptable salt thereof, and a hydrate thereof and a solvate thereof a PAI-1 inhibitor which comprises as an active ingredient a substance selected from the group consisting of the compound represented by the aforementioned general formula (II) and a pharmacologically acceptable salt thereof, and a hydrate thereof and a solvate thereof; a method for inhibiting PAI-1 in mammal including a human, which comprises the step of administering an effective amount of the substance selected from the group consisting of the compound represented by the aforementioned general formula (II) and the pharmacologically acceptable salt thereof, and the hydrate thereof and the solvate thereof to a mammal including a human; a method for therapeutic and/or preventive treatment of diseases caused by an expression of PAI-1 or an enhancement of PAI-1 activity in mammal including a human, which comprises the step of administering a therapeutically and/or preventively effective amount of the substance selected from the group consisting of the compound represented by the aforementioned general formula (II) and the pharmacologically acceptable salt thereof, and the hydrate thereof and the solvate thereof to a mammal including a human; and a method for preventive and/or therapeutic treatment of diseases caused by one or more abnormal conditions selected from the group consisting of thrombogenesis, fibrogenesis, accumulation of visceral fat, cell proliferation, angiogenesis, deposition or remodeling of extracellular matrix, and cell movement or migration in mammal including a human, which comprises the step of administering a therapeutically and/or preventively effective amount of the substance selected from the group consisting of the compound represented by the aforementioned general formula (II) and the pharmacologically acceptable salt thereof, and the hydrate thereof and the solvate thereof to a mammal including a human.

### Best Mode for Carrying out the Invention

The terms used in the specification have the following meanings.

In the specification, for example, the formula: -X-C(=Y)- means the following formula: 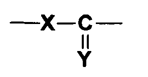 and the formula: -C(R³)=N- means the following formula.

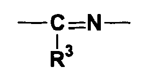

In the specification, any of fluorine atom, chlorine atom, bromine atom, or iodine atom may be used as the "halogen atom."

Examples of the "hydrocarbon group" include an alkyl group, an alkenyl group, an alkynyl group, an aryl group, an aralkyl group and the like.

The "alkyl group" or an alkyl moiety of the substituents (whose examples include, for example, an alkoxy group, an alkylsulfanyl group, a monoalkylamino group, a dialkylamino group, a halogenated alkyl group, an aralkyl group and the like), containing the alkyl moiety may be straight chain, branched chain, cyclic, or combination of these unless otherwise specifically referred to. The cyclic alkyl group may be a polycyclic alkyl group. As the alkyl group, a C₁-C₂₀ alkyl group, preferably, a C₁-C₆ alkyl group may be used. More specifically, examples of the alkyl group include, for example, methyl group, ethyl group, n-propyl group, isopropyl group, n-butyl group, isobutyl group, sec-butyl group, tert-butyl group, n-pentyl group, neopentyl group, isopentyl group, tert-pentyl group, n-hexyl group, n-heptyl group, n-octyl group, cyclopropyl group, cyclobutyl group, cyclopentyl group, cyclohexyl group, cyclopropylmethyl group, bornyl group, adamantyl group and the like, however, examples of the alkyl group are not limited to those exemplified above.

The "alkenyl group" or an alkenyl moiety of the substituents (whose examples include, for example, an alkenyloxy group and the like), containing the alkenyl moiety may be straight chain, branched chain, cyclic, or combination of these unless otherwise specifically referred to. The cyclic alkenyl group may be a polycyclic alkenyl group. Numbers of the double bonds existing in the alkenyl group are not particularly limited. When the alkenyl group includes two or more double bonds, they may either be conjugated or non-conjugated. As the alkenyl group, a C₂-C₂₀ alkenyl group, preferably, a C₂-C₆ alkenyl group may be used. More specifically, examples of the alkenyl group include, for example, vinyl group, allyl group, isopropenyl group, allenyl group and the like, however, examples of the alkenyl group are not limited to those exemplified above.

The "alkynyl group" or an alkynyl moiety of the substituents (whose examples include, for example, an alkynyloxy group and the like), containing the alkynyl moiety may be straight chain, branched chain, or combination of these unless otherwise specifically referred to. Numbers of the triple bonds existing in the alkynyl group are not particularly limited. The alkynyl group may include one or more double bonds. As the alkynyl group, a C₂-C₂₀ alkynyl group, preferably, a C₂-C₆ alkynyl group may be used. More specifically, examples of the alkynyl group include, for example, ethynyl group, propargyl group and the like, however, examples of the alkynyl group are not limited to those exemplified above.

As the "aryl group" or an aryl moiety of the substituents (whose examples include, for example, an aralkyl group, an aryloxy group, an arylsulfanyl group, an arylamino group and the like), containing the aryl moiety, an aromatic hydrocarbon (arene) residue, which comprises 6 to 14 carbon atoms, preferably, 6 to 10 carbon atoms may be used. The arene may either be monocyclic or fused polycyclic. More specifically, examples of the arene include, for example, benzene ring, naphthalene ring, anthracene ring, phenanthrene ring and the like. Preferred examples of the aryl group include phenyl group, 1-naphthyl group, 2-naphthyl group and the like. The aryl group can bind at any position on the ring.

As the "aralkyl group" or an aralkyl moiety of the substituents (whose examples include, for example, an aralkyloxy group and the like), containing the aralkyl moiety, a C₇-C₂₀ aralkyl group, preferably, a C₇-C₁₂ aralkyl group may be used. More specifically, examples of the aralkyl group include, for example, benzyl group, 1-naphthylmethyl group, 2-naphthylmethyl group, 1-phenethyl group, 2-phenethyl group and the like, however, examples of the aralkyl group are not limited to those exemplified above.

As the "heterocyclic group" or a heterocyclic moiety of the substituents (whose examples include, for example, a heterocyclic alkyl group, a heterocyclic oxy group and the like) containing the heterocyclic moiety, a 3- to 14-membered heterocyclic residue which comprises one or more hetero atoms such as nitrogen atom, oxygen atom, and sulfur atom may be used unless otherwise specifically referred to. In the specification, the "hetero atom" means atom other than the carbon atom, whose examples include nitrogen atom, oxygen atom, sulfur atom and the like unless otherwise specifically referred to. When the heterocyclic group comprises two or more hetero atoms, each of them may be the same or different. The hetero ring may either be monocyclic or fused polycyclic, and may be saturated, partly saturated, or aromatic. The "heteroaryl group" means a heterocyclic group whose heterocyclic moiety is aromatic, and the "non-aromatic heterocyclic group" means a heterocyclic group whose heterocyclic moiety is saturated or partly saturated. Examples of the heterocyclic group include, for example, isochromanyl group, chromanyl group, pyrrolidinyl group, pyrrolinyl group, imidazolidinyl group, imidazolinyl group, pyrazolidinyl group, pyrazolinyl group, piperidyl group, piperidino group, morpholinyl group, morpholino group, thiomorpholinyl group, thiomorpholino group, piperazinyl group, indolinyl group, isoindolinyl group, quinuclidinyl group, thienyl group, thianthrenyl group, furyl group, pyranyl group, isobenzofuranyl group, chromenyl group, xanthenyl group, phenoxathiinyl group, 2H-pyrrolyl group, pyrrolyl group, imidazolyl group, pyrazolyl group, isothiazolyl group, isoxazolyl group, pyridyl group, pyrazinyl group, pyrimidinyl group, pyridazinyl group, indolizinyl group, isoindolyl group, 3H-indolyl group, indolyl group, 1H-indazolyl group, purinyl group, quinolizinyl group, isoquinolyl group, quinolyl group, phthalazinyl group, naphthyridinyl group, quinoxalinyl group, quinazolinyl group, cinnolinyl group, pteridinyl group, 4aH-carbazolyl group, carbazolyl group, *β* -carbolinyl group, phenanthridinyl group, acridinyl group, perimidinyl group, phenanthrolinyl group, phenazinyl group, phenoxazinyl group, phenothiazinyl group, furazanyl group, phenoxazinyl group, hexamethyleneimino group, heptamethyleneimino group, oxazolyl group, thiazolyl group, triazolyl group, tetrazolyl group and the like, however, examples of the heterocyclic group are not limited to those exemplified above. The aryl group can bind at any position on the ring. The heterocyclic group can bind at any position on the ring. The "nitrogen-containing heterocyclic group" means a heterocyclic group which comprises at least one nitrogen atom.

Examples of the "aromatic group" include aryl group and heteroaryl group.

As the "acyl group" or an acyl moiety of the substituents (whose examples include, for example, an acyloxy group, an acylamino group and the like), containing the acyl moiety, examples include, for example, formyl group, an alkanoyl group (preferably, a C₂-C₇ alkanoyl group whose examples include, for example, acetyl group, propionyl group, butyryl group, isobutyryl group, valeryl group, isovaleryl group, pivaloyl group and the like), an alkenylcarbonyl group (preferably, a C₃-C₇ alkenylcarbonyl group whose examples include, for example, acryloyl group, methacryloyl group, crotonoyl group, isocrotonoyl group and the like), an alkynylcarbonyl group (preferably, a C₃-C₇ alkynylcarbonyl group whose examples include, for example, propioloyl group and the like), an aroyl group (preferably, a C₇-C₁₁ aroyl group whose examples include, for example, benzoyl group, 1-naphthoyl group, 2-naphthoyl group, and the like), an aralkylcarbonyl group (preferably, a C₈-C₁₃ aralkylcarbonyl group whose examples include, for example, benzylcarbonyl group and the like), a heterocyclic carbonyl group whose examples include, for example, piperidinocarbonyl group, morpholinocarbonyl group, furoyl group, thenoyl group, nicotinoyl group, isonicotinoyl group and the like), carboxy group, an alkoxycarbonyl group, an alkenyloxycarbonyl group (preferably, a C₃-C₇ alkenyloxycarbonyl group whose examples include, for example, allyloxycarbonyl group and the like), an alkynyloxycarbonyl group (preferably, a C₃-C₇ alkynyloxycarbonyl group whose examples include, for example, propargyloxycarbonyl group and the like), an aryloxycarbonyl group (preferably, a C₇-C₁₁ aryloxycarbonyl group whose examples include, for example, phenoxycarbonyl group and the like), an aralkyloxycarbonyl group (preferably, a C₈-C₁₃ aralkyloxycarbonyl group whose examples include, for example, benzyloxycarbonyl group and the like), a heterocyclic oxycarbonyl group whose examples include, for example, 4-piperidyloxycarbonyl group, 3-pyridyloxycarbonyl group and the like, carbamoyl group, an alkylcarbamoyl group (preferably, a C₂-C₇ alkylcarbamoyl group whose examples include, for example, methylcarbamoyl group and the like), a dialkylcarbamoyl group (preferably, a C₃-C₁₃ dialkylcarbamoyl group whose examples include, for example, dimethylcarbamoyl group and the like), an arylcarbamoyl group (preferably, a C₇-C₁₁ arylcarbamoyl group whose examples include, for example, phenylcarbamoyl group and the like), an aralkylcarbamoyl group (preferably, a C₈-C₁₃ aralkylcarbamoyl group whose examples include, for example, benzylcarbamoyl group and the like), sulfo group, an alkylsulfonyl group (preferably, a C₁-C₆ alkylsulfonyl group whose examples include, for example, mesyl group, propanesulfonyl group and the like), an alkenylsulfonyl group (preferably, a C₂-C₆ alkenylsulfonyl group whose examples include, for example, allylsulfonyl group and the like), an alkynylsulfonyl group (preferably, a C₂-C₆ alkynylsulfonyl group whose examples include, for example, propargylsulfonyl group and the like), an arylsulfonyl group (preferably, a C₆-C₁₀ arylsulfonyl group whose examples include, for example, benzenesulfonyl group, 1-naphthylsulfonyl group, 2-naphthylsulfonyl group and the like), an aralkylsulfonyl group (preferably, a C₇-C₁₂ aralkylsulfonyl group whose examples include, for example, benzylsulfonyl group and the like), a heterocyclic sulfonyl group whose examples include, for example, piperidinosulfonyl group, morpholinosulfonyl group, 8-quinolylsulfonyl group and the like, sulfamoyl group, an alkylsulfamonyl group (preferably, a C₁-C₆ alkylsulfamonyl group whose examples include, for example, methylsulfamoyl group and the like), a dialkylsulfamonyl group (preferably, a C₂-C₁₂ dialkylsulfamonyl group whose examples include, for example, dimethylsulfamoyl group and the like), an arylsulfamonyl group (preferably, a C₆-C₁₀ arylsulfamonyl group whose examples include, for example, phenylsulfamoyl group and the like), an aralkylsulfamonyl group (preferably, a C₇-C₁₂ alkylsulfamonyl group whose examples include, for example, benzylsulfamoyl group and the like), sulfeno group, sulfenamoyl group, phosphono group, a dialkylphosphono group (preferably, a C₂-C₁₂ dialkylphosphono group whose examples include, for example, dimethylphosphono group and the like), a diaralkylphosphono group (preferably, a C₁₄-C₂₂ diaralkylphosphono group whose examples include, for example, dibenzylphosphono group and the like), hydroxyphophonyl group, thioformyl group, dithocarboxy group, hydroxythiocarboxy group, sulfanylcarbonyl group, an alkylsulfanylthiocarbonyl group (preferably, a C₁-C₆ alkylsulfanylthiocarbonyl group whose examples include, for example, methylsulfanylthiocarbonyl group and the like), thiocarbamoyl group, glyoxyloyl group, oxalo group, an alkoxycarbonylcarbonyl group (preferably, a C₁-C₆ alkoxycarbonylcarbonyl group whose examples include, for example, methoxalyl group and the like), an alkylcarbonylcarbonyl group (preferably, a C₁-C₆ alkylcarbonylcarbonyl group whose examples include, for example, pyruvoyl group and the like) and the like, however, examples of the acyl group are not limited to those exemplified above.

As the "alkoxy group," a C₁-C₂₀ alkoxy group, preferably, a C₁-C₆ alkoxy group may be used. More specifically, examples of the alkoxy group include, for example, methoxy group, ethoxy group, n-propoxy group, isopropoxy group, n-butoxy group, isobutoxy group, sec-butoxy group, tert-butoxy group, n-pentyloxy group, neopentyloxy group, isopentyloxy group, tert-pentyloxy group, n-hexyloxy group, n-heptyloxy group, n-octyloxy group, cyclopropyloxy group, cyclobutyloxy group, cyclopentyloxy group, cyclohexyloxy group, cyclopropylmethoxy group and the like, however, examples of the alkoxy group are not limited to those exemplified above.

As the "alkoxycarbonyl group," a C₂-C₂₀ alkoxycarbonyl group, preferably, a C₂-C₇ alkoxycarbonyl group may be used. In the definition, the "C₂ alkoxycarbonyl group" means methoxycarbonyl group. More specifically, examples of the alkoxycarbonyl group include, for example, methoxycarbonyl group, ethoxycarbonyl group, n-propoxycarbonyl group, isopropoxycarbonyl group, n-butoxycarbonyl group, isobutoxycarbonyl group, sec-butoxycarbonyl group, tert-butoxycarbonyl group, n-pentyloxycarbonyl group, neopentyloxycarbonyl group, isopentyloxycarbonyl group, tert-pentyloxycarbonyl group, n-hexyloxycarbonyl group, n-heptyloxycarbonyl group, n-octyloxycarbonyl group, cyclopropyloxycarbonyl group, cyclobutyloxycarbonyl group, cyclopentyloxycarbonyl group, cyclohexyloxycarbonyl group, cyclopropylmethoxycarbonyl group and the like, however, examples of the alkoxycarbonyl group are not limited to those exemplified above.

Examples of the "carboxy group which may be esterified" include, for example, carboxy group, an alkoxycarbonyl group, an alkenyloxycarbonyl group, an alkynyloxycarbonyl group, an aryloxycarbonyl group, an aralkyloxycarbonyl group, a heterocyclic oxycarbonyl group and the like, however, examples of the "carboxy group which may be esterified" are not limited to those exemplified above.

In the specification, when a certain functional group is defined as "which may be substituted," the definition means that the functional group may have one or more substituents. Numbers, kinds, and positions of substituents are not particularly limited, and when two or more substituents exist, they may be the same or different.

Examples of the substituent include, for example, halogen atoms, oxo group, thioxo group, oxido group, nitro group, nitroso group, cyano group, isocyano group, cyanato group, thiocyanato group, isocyanato group, isothiocyanato group, hydroxy group, sulfanyl group, hydrocarbon group, heterocyclic group, acyl group, amino group, hydrazino group, hydrazono group, diazenyl group, ureido group, thioureido group, guanidino group, amidino group, azido group, imino group, hydroxyamino group, hydroxyimino group, aminooxy group, diazo group, semicarbazino group, semicarbazono group, allophanyl group, hydantoyl group, boryl group, silyl group, stannyl group, selanyl group, oxido group and the like, however, examples of the substituent are not limited to those exemplified above.

The substituents exemplified above may further be substituted with one or more other substituents. Examples of these substituent include a halogenated alkyl group (preferably, a halogenated C₁-C₆ alkyl group whose examples include, for example, trifluoromethyl group and the like), a hydroxyalkyl group, (preferably, a hydroxy C₁-C₆ alkyl group whose examples include, for example, hydroxymethyl group and the like), a carboxyalkyl group, (preferably, a carboxy C₁-C₆ alkyl group whose examples include, for example, carboxymethyl group and the like), a halogenated alkoxy group (preferably, a halogenated C₁-C₆ alkoxy group whose examples include, for example, trifluoromethoxy group and the like), a halogenated alkanoyl group (preferably, a halogenated C₂-C₇ alkanoyl group whose examples include, for example, trifluoroacetyl group and the like), a halogenated alkylsulfonyl group (preferably, a halogenated C₁-C₆ alkylsulfonyl group whose examples include, for example, trifluoromethanesulfonyl group and the like), an alkanoyloxy group (preferably, a C₂-C₇ alkanoyloxy group whose examples include, for example, acetoxy group and the like), an aroyloxy group (preferably, a C₇-C₁₁ aroyloxy group whose examples include, for example, benzoyloxy group and the like), an alkylamino group (preferably, a C₁-C₆ alkylamino group whose examples include, for example, methylamino group and the like), a dialkylamino group (preferably, a C₂-C₁₂ dialkylamino group whose examples include, for example, dimethylamino group and the like), an alkanoylamino group (preferably, a C₂-C₇ alkanoylamino group whose examples include, for example, acetylamino group and the like), an aroylamino group (preferably, a C₇-C₁₁ aroylamino group whose examples include, for example, benzoylamino group and the like), an alkoxycarbonylamino group (preferably, a C₂-C₇ alkoxycarbonylamino group whose examples include, for example, methoxycarbonylamino group, tert-butoxycarbonylamino group and the like), an aralkyloxycarbonylamino group (preferably, a C₈-C₁₃ aralkyloxycarbonylamino group whose examples include, for example, benzyloxycarbonylamino group and the like), an alkylsulfonylamino group (preferably, a C₁-C₆ alkylsulfonylamino group whose examples include, for example, mesylamino group and the like), an arylsulfonylamino group (preferably, a C₆-C₁₀ arylsulfonylamino group whose examples include, for example, benzenesulfonylamino group and the like) and the like. However, the substituents explained above are for exemplification, and examples of the substituent are not limited to those exemplified above.

Compounds represented by the general formulas (I) and (II) are explained in details below.

In the general formula (I), W represents a group selected from the following connecting group W-1.

### [Connecting Group W-1]

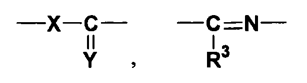

When W is -X-C(=Y)-, X represents sulfur atom or NH, and Y represents oxygen atom or sulfur atom. Preferably, X is sulfur atom. Preferably, Y is oxygen atom.

When W is -C(R³)=N-, R³ represents a hydrocarbon group which may be substituted, hydroxy group which may be substituted, or carboxy group which may be esterified.

Preferred examples of the hydrocarbon group represented by R³ include a C₁-C₆ alkyl group or a C₆-C₁₀ aryl group. More preferred examples include methyl group, ethyl group, isopropyl group, tert-butyl group, or phenyl group.

The hydrocarbon group represented by R³ may be substituted. Preferred examples of said substituent include a halogen atom; a C₁-C₆ alkoxy group (more preferably, methoxy group); hydroxy group; and a C₂-C₇ alkoxycarbonyl group (more preferably, ethoxycarbonyl group).

Preferred examples of the hydrocarbon group which may be substituted represented by R³ include a C₁-C₆ alkyl group (more preferably, methyl group, ethyl group, isopropyl group, and tert-butyl group); a C₆-C₁₀ aryl group (said aryl group may be substituted, and more preferred examples include phenyl group, 3,4,5-trimethoxyphenyl group, 4-hydroxyphenyl group, and 2-hydroxy-5-bromophenyl group); a halogenated C₁-C₆ alkyl group (more preferably, trifluoromethyl group); a C₂-C₇ alkoxycarbonyl-substituted C₁-C₆ alkyl group (more preferably, 2-(ethoxycarbonyl)ethyl group and 3-(ethoxycarbonyl)propyl group); and a C₁-C₆ alkoxy-substituted C₁-C₆ alkyl group (more preferably, methoxymethyl group). Further preferred examples include methyl group, isopropyl group, or phenyl group.

Preferred examples of the hydroxy group which may be substituted represented by R³ include hydroxy group or a C₁-C₆ alkoxy group. More preferred examples include hydroxy group or ethoxy group, and further preferred examples include ethoxy group.

Preferred examples of the carboxy group which may be esterified represented by R³ include carboxy group or a C₂-C₇ alkoxycarbonyl group. More preferred examples include carboxy group or ethoxycarbonyl group.

Preferably, W is -S-C(=O)- or -C(R³)=N-.

In the general formula (I), R¹ represents an aromatic group which may be substituted.

When W is -X-C(=Y)-, preferred examples of the aromatic group represented by R¹ include a C₆-C₁₀ aryl group or a 5- to 9-membered heteroaryl group. More preferred examples include phenyl group, furyl group, pyridyl group, benzo[2,1,3]oxadiazolyl group, or indolyl group, and most preferred examples include phenyl group.

The aromatic group represented by R¹ may be substituted. Preferred examples of said substituent include a halogen atom; nitro group; a C₁-C₆ alkyl group (more preferably, methyl group and isopropyl group); a C₆-C₁₀ aryl group (said aryl group may be substituted, and more preferred examples include 3-carboxyphenyl group, 3,5-dichlorophenyl group, phenyl group, 3,4-dimethoxyphenyl group, 3,4-dihydroxyphenyl group, 2,6-dimethoxyphenyl group, 4-methoxyphenyl group, 2,6-dihydroxyphenyl group, and 4-hydroxyphenyl group); hydroxy group; a C₁-C₆ alkoxy group (said alkoxy group may be substituted, and more preferred examples include methoxy group and methoxymethoxy group); a C₂-C₇ alkoxycarbonyl group (more preferably, methoxycarbonyl group and ethoxycarbonyl group); a C₁-C₃ alkylenedioxy group (more preferably, methylenedioxy group); a C₂-C₇ alkanoylamino group (more preferably, acetylamino group); amino group; a C₆-C₁₀ aryloxy group (more preferably, phenoxy group); a C₂-C₆ alkenyl group (more preferably, styryl group); a halogenated C₁-C₆ alkyl group (more preferably, trifluoromethyl group); a C₂-C₇ alkoxycarbonyloxy group (more preferably, ethoxycarbonyloxy group); carboxy group; a C₆-C₁₀ aryl-substituted carbamoyl group (said aryl group may be substituted, and more preferred examples include 3,4-dihydroxyphenylcarbamoyl group, 3,4-dimethoxyphenylcarbamoyl group, 4-methoxyphenylcarbamoyl group, 4-hydroxyphenylcarbamoyl group, 3-methoxyphenylcarbamoyl group, 3-hydroxyphenylcarbamoyl group, 3-trifluoromethyl-4-methoxyphenylcarbamoyl group, 3-methyl-4-hydroxyphenylcarbamoyl group, 4-carboxyphenylcarbamoyl group, 2,5-bis(trifluoromethyl)phenylcarbamoyl group, 3,5-bis(trifluoromethyl)phenylcarbamoyl group, 3-methoxy-4-(methoxycarbonyl)phenylcarbamoyl group, 3-(trifluoromethoxy)phenylcarbamoyl group, 3,4-dichlorophenylcarbamoyl group, 4-(methoxycarbonyl)phenylcarbamoyl group, 3-(methoxycarbonyl)phenylcarbamoyl group, 3-carboxyphenylcarbamoyl group, 2-(4-methoxyphenoxy)-5-(trifluoromethyl)phenylcarbamoyl group, 2-(4-hydroxyphenoxy)-5-(trifluoromethyl)phenylcarbamoyl group, 2-chloro-5-(trifluoromethyl)phenylcarbamoyl group, 3-nitrophenylcarbamoyl group, 3,4-methylenedioxyphenylcarbamoyl group, 3,4,5-trimethoxyphenylcarbamoyl group, 2,3-dimethoxyphenylcarbamoyl group, 3,4,5-trihydroxyphenylcarbamoyl group, 2,3-dihydroxyphenylcarbamoyl group, 3-hydroxy-4-(methoxycarbonyl)phenylcarbamoyl group, phenylcarbamoyl group, 2,5-dichlorophenylcarbamoyl group, 2-fluoro-5-(trifluoromethyl)phenylcarbamoyl 1 group, 2-methyl-5-methoxyphenylcarbamoyl group, 3-methoxy-5-(trifluoromethyl)phenylcarbamoyl group, 2-methoxy-5-methylphenylcarbamoyl group, 2-methoxycarbonyl-5-chlorophenylcarbamoyl group, 2-methoxy-5-chlorophenylcarbamoyl group, 2-methoxy-5-phenylphenylcarbamoyl group, 2-methyl-5-hydroxyphenylcarbamoyl group, 2-hydroxy-5-methyl phenylcarbamoyl group, 2-carboxy-5-chlorophenylcarbamoyl group, 3-hydroxy-5-(trifluoromethyl)phenylcarbamoyl group, (2,2-difluoro-1,3-benzodioxol-4-yl)carbamoyl group, 2,5-difluorophenylcarbamoyl group, 2-nitro-5-(trifluoromethyl)phenylcarbamoyl group, 2-methoxy-5-(tert-butyl)phenylcarbamoyl group, 2-methoxy-5-(trifluoromethyl)phenylcarbamoyl group, 2,5-dimethoxyphenylcarbamoyl group, 2-bromo-4-isopropylphenylcarbamoyl group, and 2-chloro-5-nitrophenylcarbamoyl group); a C₆-C₁₀ aroylamino group (said aroyl group may be substituted, and more preferred examples include 3,4-dihydroxybenzoylamino group, 4-(trifluoromethoxy)benzoylamino group, 4-methoxybenzoylamino group, 4-hydroxybenzoylamino group, and 3,4-dimethoxybenzoylamino group); a C₈-C₁₃ aralkyloxycarbonyl group (said aralkyl group may be substituted, and more preferred examples include 4-methoxybenzyloxycarbonyl group and benzyloxycarbonyl group); a heteroaryl-substituted carbamoyl group (said heteroaryl group may be substituted, and more preferred examples include (2-chloropyridin-5-yl)carbamoyl group, (2-methoxypyridin-5-yl)carbamoyl group, and (2-hydroxypyridin-5-yl)carbamoyl group); a C₇-C₁₂ aralkyl group (said aralkyl group may be substituted, and more preferred examples include 3,4-dibenzyloxybenzyl group, 3,4-dihydroxybenzyl group, 4-methoxycarbonylbenzyl group); and a C₆-C₁₀ aroyl group (said aroyl group may be substituted, and more preferred examples include 3,4-dimethoxybenzoyl group, 4-methoxycarbonylbenzoyl group). Further preferred examples include hydroxy group, a C₆-C₁₀ aryl-substituted carbamoyl group (said aryl group may be substituted), a heteroaryl-substituted carbamoyl group (said heteroaryl group may be substituted), carboxy group, nitro group, a C₁-C₆ alkyl group, or a C₁-C₆ alkoxy group (said alkoxy group may be substituted). It is preferable that the aromatic group represented by R¹ is substituted with one to three substituents independently selected from the aforementioned substituents.

Examples of the aromatic group which may be substituted represented by R¹ include, for example, a group selected from the following substituent group R-1a-1. [Substituent Group R-1a-1] 3,5-dibromo-2-hydroxyphenyl group, 2,3-dihydroxyphenyl group, 3,4-dihydroxyphenyl group, 2,4,5-trimethoxyphenyl group, 2-hydroxy-3-methylphenyl group, 3-hydroxyphenyl group, 3,4,5-trihydroxyphenyl group, 3-ethoxycarbonyl-4-hydroxyphenyl group, 2,3,4-trihydroxyphenyl group, 3,4-dihydroxy-5-methoxyphenyl group, 5-hydroxy-2-nitrophenyl group, 3,5-dihydroxyphenyl group, 2,4-dihydroxyphenyl group, 2-hydroxy-5-nitrophenyl group, 5-(3-carboxyphenyl)furan-2-yl group, 5-chloro-2-hydroxyphenyl group, 5-(3,5-dichlorophenyl)furan-2-yl group, 3,4-methylenedioxyphenyl group, 4-bromophenyl group, benzo[2,1,3]oxadiazol-5-yl group, 4-pyridyl group, 3,4-dichlorophenyl group, 4-acetamidophenyl group, 4-aminophenyl group, 4-hydroxy-3-nitrophenyl group, 3-amino-4-hydroxyphenyl group, 3-hydroxy-4-nitrophenyl group, 4-amino-3-hydroxyphenyl group, 4-phenoxyphenyl group, 3-phenoxyphenyl group, 2-hydroxy-5-styrylphenyl group, 3-hydroxy-4-methoxyphenyl group, 3-hydroxy-4,5-methylenedioxyphenyl group, 2-fluoro-3-hydroxyphenyl group; 3-hydroxy-4-fluorophenyl group, 3-methoxy-4-chlorophenyl group, 3-hydroxy-4-chlorophenyl group, 2-methoxy-3-hydroxyphenyl group, 3-isopropyl-4-methoxyphenyl group, 3-fluoro-4-methoxyphenyl group, 3-fluoro-4-hydroxyphenyl group, 3-trifluoromethyl-4-methoxyphenyl group, 3,4-bis(ethoxycarbonyloxy)phenyl group, 3,4,5-tris(ethoxycarbonyloxy)phenyl group, 3,4-dihydroxy-5-(methoxycarbonyl)phenyl group, 3,5-dihydroxy-4-(methoxycarbonyl)phenyl group, 3-methoxy-4-hydroxy-5-(methoxycarbonyl)phenyl group, 3-methoxy-4-hydroxy-5-carboxyphenyl group, 3-methoxy-4-hydroxy-5-nitrophenyl group, 3,4-dihydroxy-5-nitrophenyl group, 3,4,5-trimethoxyphenyl group, 3-methoxy-4-hydroxy-5-(3,4-dihydroxyphenylcarbamoyl)phenyl group, 3-carboxyphenyl group, 4-hydroxy-3-(3,4-dimethoxyphenylcarbamoyl)phenyl group, 4-hydroxy-3-(3,4-dihydroxyphenylcarbamoyl)phenyl group, 4-hydroxy-3-(4-methoxyphenylcarbamoyl)phenyl group, 4-hydroxy-3-(4-hydroxyphenylcarbamoyl)phenyl group, 4-hydroxy-3-(3-methoxyphenylcarbamoyl)phenyl group, 4-hydroxy-3-(3-hydroxyphenylcarbamoyl)phenyl group, 3-(3,4-dimethoxyphenylcarbamoyl)phenyl group, 3-carboxy-4-hydroxyphenyl group, 4-methoxy-3-(3,4-dihydroxybenzoylamino)phenyl group, 3-methoxy-4-hydroxy-5-(3-trifluoromethyl-4-methoxyphenylcarbamoyl)phenyl group, 3-methoxy-4-hydroxy-5-(3-methyl-4-hydroxyphenylcarbamoyl)phenyl group, 3-(4-methoxybenzyloxycarbonyl)-4-hydroxyphenyl group, 3-benzyloxycarbonyl-4-hydroxyphenyl group, 4-methoxy-3-[4-(trifluoromethoxy)benzoylamino]phenyl group, 4-hydroxy-3-[4-(trifluoromethoxy)benzoylamino]phenyl group, 3-[4-(trifluoromethoxy)benzoylamino]phenyl group, 3-(4-methoxybenzoylamino)phenyl group, 3-(4-hydroxybenzoylamino)phenyl group, 3-(3,4-dimethoxybenzoylamino)phenyl group, 3-(3,4-dihydroxybenzoylamino)phenyl group, 4-methoxy-3-(3,4-dimethoxybenzoylamino)phenyl group, 4-hydroxy-3-(3,4-dihydroxybenzoylamino)phenyl group, 4-methoxy-3-(4-methoxybenzoylamino)phenyl group, 4-hydroxy-3-(4-hydroxybenzoylamino)phenyl group, 3-(3,4-dihydroxyphenylcarbamoyl)phenyl group, 3-(4-carboxyphenylcarbamoyl)phenyl group, 3-methyl-4-hydroxy-5-carboxyphenyl group, 4-hydroxy-3-[(2-chloropyridin-5-yl)carbamoyl]phenyl group, 3-methyl-4-hydroxy-5-(3,4-dihydroxyphenylcarbamoyl)phenyl group, 4-hydroxy-3-[2,5-bis(trifluoromethyl)phenylcarbamoyl]phenyl group, 4-hydroxy-3-[3,5-bis(trifluoromethyl)phenylcarbamoyl]phenyl group, 4-hydroxy-3-[3-methoxy-4-(methoxycarbonyl)phenylcarbamoyl]phenyl group, 4-hydroxy-3-[(2-methoxypyridin-5-yl)carbamoyl]phenyl group, 4-hydroxy-3-[(2-hydroxypyridin-5-yl)carbamoyl]phenyl group, indol-3-yl group, 1-(3,4-dibenzyloxybenzyl)indol-3-yl group, 1-(3,4-dimethoxybenzoyl)indol-3-yl group, 1-(3,4-dihydroxybenzyl)indol-3-yl group, 4-hydroxy-3-[3-(trifluoromethoxy)phenylcarbamoyl]phenyl group, 4-hydroxy-3-(3,4-dichlorophenylcarbamoyl)phenyl group, 4-hydroxy-3-[4-(methoxycarbonyl)phenylcarbamoyl]phenyl group, 4-hydroxy-3-[3-(methoxycarbonyl)phenylcarbamoyl]phenyl group, 4-hydroxy-3-(4-carboxyphenylcarbamoyl)phenyl group, 4-hydroxy-3-(3-carboxyphenylcarbamoyl)phenyl group, 4-hydroxy-3-[2-(4-methoxyphenoxy)-5-(trifluoromethyl)phenylcarbamoyl]phenyl group, 4-hydroxy-3-[2-(4-hydroxyphenoxy)-5-(trifluoromethyl)phenylcarbamoyl]phenyl group, 4-hydroxy-3-[2-chloro-5-(trifluoromethyl)phenylcarbamoyl]phenyl group, 4-hydroxy-3-(3-nitrophenylcarbamoyl)phenyl group, 1-(4-methoxycarbonylbenzyl)indol-3-yl group, 3-phenyl-4-hydroxy-5-(benzyloxycarbonyl)phenyl group, 1-(4-methoxycarbonylbenzoyl)indol-3-yl group, 4-hydroxy-3-(3,4-methylenedioxyphenylcarbamoyl)phenyl group, 3-methoxy-4-hydroxy-5-(methoxycarbonyl)phenyl group, 3-phenyl-4-hydroxy-5-carboxyphenyl group, 3,5-bis(methoxymethoxy)-4-(methoxycarbonyl)phenyl group, 4-hydroxy-3-(3,4,5-trimethoxyphenylcarbamoyl)phenyl group, 4-hydroxy-3-(2,3-dimethoxyphenylcarbamoyl)phenyl group, 4-hydroxy-3-(3,4,5-trihydroxyphenylcarbamoyl)phenyl group, 4-hydroxy-3-(2,3-dihydroxyphenylcarbamoyl)phenyl group, 4-hydroxy-3-[3-hydroxy-4-(methoxycarbonyl)phenylcarbamoyl]phenyl group, 3-methoxy-4-hydroxy-5-[3,5-bis(trifluoromethyl)phenylcarbamoyl]phenyl group, 3-methoxy-4-hydroxy-5-[2-chloro-5-(trifluoromethyl)phenylcarbamoyl]phenyl group, 3-methoxy-4-hydroxy-5-(phenylcarbamoyl)phenyl group, 4-hydroxy-3-(2,5-dichlorophenylcarbamoyl)phenyl group, 4-hydroxy-3-[2-fluoro-5-(trifluoromethyl)phenylcarbamoyl]phenyl group, 4-methoxy-3-(3,4-dimethoxyphenyl)phenyl group, 4-methoxy-3-(3,4-dihydroxyphenyl)phenyl group, 4-hydroxy-3-(3,4-dihydroxyphenyl)phenyl group, 3,5-dihydroxy-4-carboxyphenyl group, 4-methoxy-3-(2,6-dimethoxyphenyl)phenyl group, 4-methoxy-3-(4-methoxyphenyl)phenyl group, 4-hydroxy-3-(2,6-dihydroxyphenyl)phenyl group, 4-hydroxy-3-(2-methyl-5-methoxyphenylcarbamoyl)phenyl group, 4-hydroxy-3-[3-methoxy-5-(trifluoromethyl)phenylcarbamoyl]phenyl group, 4-hydroxy-3-(2-methoxy-5-methylphenylcarbamoyl)phenyl group, 4-hydroxy-3-(2-methoxycarbonyl-5-chlorophenylcarbamoyl)phenyl group, 4-hydroxy-3-(2-methoxy-5-chlorophenylcarbamoyl)phenyl group, 4-hydroxy-3-(2-methoxy-5-phenylphenylcarbamoyl)phenyl group, 4-hydroxy-3-(2-methyl-5-hydroxyphenylcarbamoyl)phenyl group, 4-hydroxy-3-(2-hydroxy-5-methylphenylcarbamoyl)phenyl group, 4-hydroxy-3-(2-carboxy-5-chlorophenylcarbamoyl)phenyl group, 4-hydroxy-3-[3-hydroxy-5-(trifluoromethyl)phenylcarbamoyl]phenyl group, 4-hydroxy-3-[(2,2-difluoro-1,3-benzodioxol-4-yl)carbamoyl]phenyl group, 4-methoxy-3-(4-hydroxyphenyl)phenyl group, 4-hydroxy-3-(4-hydroxyphenyl)phenyl group, 3,4-dihydroxy-5-(benzyloxycarbonyl)phenyl group, 3,4-dihydroxy-5-carboxyphenyl group, 4-hydroxy-3-(2,5-difluorophenylcarbamoyl)phenyl group, 4-hydroxy-3-[2-nitro-5-(trifluoromethyl)phenylcarbamoyl]phenyl group, 4-hydroxy-3-[2-methoxy-5-(tert-butyl)phenylcarbamoyl]phenyl group, 4-hydroxy-3-[2-methoxy-5-(trifluoromethyl)phenylcarbamoyl]phenyl group, 3,4-dihydroxy-5-[2-chloro-5-(trifluoromethyl)phenylcarbamoyl]phenyl group, 3,4-dihydroxy-5-[3-(methoxycarbonyl)phenylcarbamoyl]phenyl group, 4-hydroxy-3-(2,5-dimethoxyphenylcarbamoyl)phenyl group, 4-hydroxy-3-(2-bromo-4-isopropylphenylcarbamoyl)phenyl group, 4-hydroxy-3-(2-chloro-5-nitrophenylcarbamoyl)phenyl group, 3,4-dihydroxy-5-(3,4-methylenedioxyphenylcarbamoyl)phenyl group, 3,4-dihydroxy-5-[3-(trifluoromethoxy)phenylcarbamoyl]phenyl group.

Preferred examples of the aromatic group which may be substituted represented by R¹ include a phenyl group which is substituted with one to three hydroxy groups (said phenyl group may further be substituted with one or more substituents other than the hydroxy group), and specific examples include a group selected from the following substituent group R-1a-2. More preferred examples include a phenyl group which is substituted with two or three hydroxy groups (said phenyl group may further be substituted with one or more substituents other than the hydroxy group), and specific examples include a group selected from the following substituent group R-1a-3. Further preferred examples include 2,3-dihydroxyphenyl group, 3,4-dihydroxyphenyl group, 3,4,5-trihydroxyphenyl group, 3,4-dihydroxy-5-nitrophenyl group, or 3,4-dihydroxy-5-[3-(trifluoromethoxy)phenylcarbamoyl]phenyl group.
[Substituent Group R-1a-2] 3,5-dibromo-2-hydroxyphenyl group, 2,3-dihydroxyphenyl group, 3,4-dihydroxyphenyl group, 2-hydroxy-3-methylphenyl group, 3-hydroxyphenyl group, 3,4,5-trihydroxyphenyl group, 3-ethoxycarbonyl-4-hydroxyphenyl group, 2,3,4-trihydroxyphenyl group, 3,4-dihydroxy-5-methoxyphenyl group, 5-hydroxy-2-nitrophenyl group, 3,5-dihydroxyphenyl group, 2,4-dihydroxyphenyl group, 2-hydroxy-5-nitrophenyl group, 5-chloro-2-hydroxyphenyl group, 4-hydroxy-3-nitrophenyl group, 3-amino-4-hydroxyphenyl group, 3-hydroxy-4-nitrophenyl group, 4-amino-3-hydroxyphenyl group, 2-hydroxy-5-styrylphenyl group, 3-hydroxy-4-methoxyphenyl group, 3-hydroxy-4,5-methylenedioxyphenyl group, 2-fluoro-3-hydroxyphenyl group, 3-hydroxy-4-fluorophenyl group, 3-hydroxy-4-chlorophenyl group, 2-methoxy-3-hydroxyphenyl group, 3-fluoro-4-hydroxyphenyl group, 3,4-dihydroxy-5-(methoxycarbonyl)phenyl group, 3,5-dihydroxy-4-(methoxycarbonyl)phenyl group, 3-methoxy-4-hydroxy-5-(methoxycarbonyl)phenyl group, 3-methoxy-4-hydroxy-5-carboxyphenyl group, 3-methoxy-4-hydroxy-5-nitrophenyl group, 3,4-dihydroxy-5-nitrophenyl group, 3-methoxy-4-hydroxy-5-(3,4-dihydroxyphenylcarbamoyl)phenyl group, 4-hydroxy-3-(3,4-dimethoxyphenylcarbamoyl)phenyl group, 4-hydroxy-3-(3,4-dihydroxyphenylcarbamoyl)phenyl group, 4-hydroxy-3-(4-methoxyphenylcarbamoyl)phenyl group, 4-hydroxy-3-(4-hydroxyphenylcarbamoyl)phenyl group, 4-hydroxy-3-(3-methoxyphenylcarbamoyl)phenyl group, 4-hydroxy-3-(3-hydroxyphenylcarbamoyl)phenyl group, 3-carboxy-4-hydroxyphenyl group, 3-methoxy-4-hydroxy-5-(3-trifluoromethyl-4-methoxyphenylcarbamoyl)phenyl group, 3-methoxy-4-hydroxy-5-(3-methyl-4-hydroxyphenylcarbamoyl)phenyl group, 3-(4-methoxybenzyloxycarbonyl)-4-hydroxyphenyl group, 3-benzyloxycarbonyl-4-hydroxyphenyl group, 4-hydroxy-3-[4-(trifluoromethoxy)benzoylamino]phenyl group, 4-hydroxy-3-(3,4-dihydroxybenzoylamino)phenyl group, 4-hydroxy-3-(4-hydroxybenzoylamino)phenyl group, 3-methyl-4-hydroxy-5-carboxyphenyl group, 4-hydroxy-3-[(2-chloropyridin-5-yl)carbamoyl]phenyl group, 3-methyl-4-hydroxy-5-(3,4-dihydroxyphenylcarbamoyl)phenyl group, 4-hydroxy-3-[2,5-bis(trifluoromethyl)phenylcarbamoyl]phenyl group, 4-hydroxy-3-[3,5-bis(trifluoromethyl)phenylcarbamoyl]phenyl group, 4-hydroxy-3-[3-methoxy-4-(methoxycarbonyl)phenylcarbamoyl]phenyl group, 4-hydroxy-3-[(2-methoxypyridin-5-yl)carbamoyl]phenyl group, 4-hydroxy-3-[(2-hydroxypyridin-5-yl)carbamoyl]phenyl group, 4-hydroxy-3-[3-(trifluoromethoxy)phenylcarbamoyl]phenyl group, 4-hydroxy-3-(3,4-dichlorophenylcarbamoyl)phenyl group, 4-hydroxy-3-[4-(methoxycarbonyl)phenylcarbamoyl]phenyl group, 4-hydroxy-3-[3-(methoxycarbonyl)phenylcarbamoyl]phenyl group, 4-hydroxy-3-(4-carboxyphenylcarbamoyl)phenyl group, 4-hydroxy-3-(3-carboxyphenylcarbamoyl)phenyl group, 4-hydroxy-3-[2-(4-methoxyphenoxy)-5-(trifluoromethyl)phenylcarbamoyl]phenyl group, 4-hydroxy-3-[2-(4-hydroxyphenoxy)-5-(trifluoromethyl)phenylcarbamoyl]phenyl group, 4-hydroxy-3-[2-chloro-5-(trifluoromethyl)phenylcarbamoyl]phenyl group, 4-hydroxy-3-(3-nitrophenylcarbamoyl)phenyl group, 3-phenyl-4-hydroxy-5-(benzyloxycarbonyl)phenyl group, 4-hydroxy-3-(3,4-methylenedioxyphenylcarbamoyl)phenyl group, 3-methoxy-4-hydroxy-5-(methoxycarbonyl)phenyl group, 3-phenyl-4-hydroxy-5-carboxyphenyl group, 4-hydroxy-3-(3,4,5-trimethoxyphenylcarbamoyl)phenyl group, 4-hydroxy-3-(2,3-dimethoxyphenylcarbamoyl)phenyl group, 4-hydroxy-3-(3,4,5-trihydroxyphenylcarbamoyl)phenyl group, 4-hydroxy-3-(2,3-dihydroxyphenylcarbamoyl)phenyl group, 4-hydroxy-3-[3-hydroxy-4-(methoxycarbonyl)phenylcarbamoyl]phenyl group, 3-methoxy-4-hydroxy-5-[3,5-bis(trifluoromethyl)phenylcarbamoyl]phenyl group, 3-methoxy-4-hydroxy-5-[2-chloro-5-(trifluoromethyl)phenylcarbamoyl]phenyl group, 3-methoxy-4-hydroxy-5-(phenylcarbamoyl)phenyl group, 4-hydroxy-3-(2,5-dichlorophenylcarbamoyl)phenyl group, 4-hydroxy-3-[2-fluoro-5-(trifluoromethyl)phenylcarbamoyl]phenyl group, 4-hydroxy-3-(3,4-dihydroxyphenyl)phenyl group, 3,5-dihydroxy-4-carboxyphenyl group, 4-hydroxy-3-(2,6-dihydroxyphenyl)phenyl group, 4-hydroxy-3-(2-methyl-5-methoxyphenylcarbamoyl)phenyl group, 4-hydroxy-3-[3-methoxy-5-(trifluoromethyl)phenylcarbamoyl]phenyl group, 4-hydroxy-3-(2-methoxy-5-methylphenylcarbamoyl)phenyl group, 4-hydroxy-3-(2-methoxycarbonyl-5-chlorophenylcarbamoyl)phenyl group, 4-hydroxy-3-(2-methoxy-5-chlorophenylcarbamoyl)phenyl group, 4-hydroxy-3-(2-methoxy-5-phenylphenylcarbamoyl)phenyl group, 4-hydroxy-3-(2-methyl-5-hydroxyphenylcarbamoyl)phenyl group, 4-hydroxy-3-(2-hydroxy-5-methylphenylcarbamoyl)phenyl group, 4-hydroxy-3-(2-carboxy-5-chlorophenylcarbamoyl)phenyl group, 4-hydroxy-3-[3-hydroxy-5-(trifluoromethyl)phenylcarbamoyl]phenyl group, 4-hydroxy-3-[(2,2-difluoro-1,3-benzodioxol-4-yl)carbamoyl]phenyl group, 4-hydroxy-3-(4-hydroxyphenyl)phenyl group, 3,4-dihydroxy-5-(benzyloxycarbonyl)phenyl group, 3,4-dihydroxy-5-carboxyphenyl 1 group, 4-hydroxy-3-(2,5-difluorophenylcarbamoyl)phenyl group, 4-hydroxy-3-[2-nitro-5-(trifluoromethyl)phenylcarbamoyl]phenyl group, 4-hydroxy-3-[2-methoxy-5-(tert-butyl)phenylcarbamoyl]phenyl group, 4-hydroxy-3-[2-methoxy-5-(trifluoromethyl)phenylcarbamoyl]phenyl group, 3,4-dihydroxy-5-[2-chloro-5-(trifluoromethyl)phenylcarbamoyl]phenyl group, 3,4-dihydroxy-5-[3-(methoxycarbonyl)phenylcarbamoyl]phenyl group, 4-hydroxy-3-(2,5-dimethoxyphenylcarbamoyl)phenyl group, 4-hydroxy-3-(2-bromo-4-isopropylphenylcarbamoyl)phenyl group, 4-hydroxy-3-(2-chloro-5-nitrophenylcarbamoyl)phenyl group, 3,4-dihydroxy-5-(3,4-methylenedioxyphenylcarbamoyl)phenyl group, 3,4-dihydroxy-5-[3-(trifluoromethoxy)phenylcarbamoyl]phenyl group.
[Substituent Group R-1a-3] 2,3-dihydroxyphenyl group, 3,4-dihydroxyphenyl group, 3,4,5-trihydroxyphenyl group, 2,3,4-trihydroxyphenyl group, 3,4-dihydroxy-5-methoxyphenyl group, 3,5-dihydroxyphenyl group, 2,4-dihydroxyphenyl group, 3,4-dihydroxy-5-(methoxycarbonyl)phenyl group, 3,5-dihydroxy-4-(methoxycarbonyl)phenyl group, 3,4-dihydroxy-5-nitrophenyl group, 3,5-dihydroxy-4-carboxyphenyl group, 3,4-dihydroxy-5-(benzyloxycarbonyl)phenyl group, 3,4-dihydroxy-5-carboxyphenyl group, 3,4-dihydroxy-5-[2-chloro-5-(trifluoromethyl)phenylcarbamoyl]phenyl group, 3,4-dihydroxy-5-[3-(methoxycarbonyl)phenylcarbamoyl]phenyl group, 3,4-dihydroxy-5-(3,4-methylenedioxyphenylcarbamoyl)phenyl group, 3,4-dihydroxy-5-[3-(trifluoromethoxy)phenylcarbamoyl]phenyl group.

Furthermore, preferred examples of the aromatic group which may be substituted represented by R¹ include a phenyl group which is substituted with a C₆-C₁₀ aryl-substituted carbamoyl group (said aryl group may be substituted, and said phenyl group may further be substituted with one or more substituents other than the C₆-C₁₀ aryl-substituted carbamoyl group), or a phenyl group which is substituted with a heteroaryl-substituted carbamoyl group (said heteroaryl group may be substituted, and said phenyl group may further be substituted with one or more substituents other than the heteroaryl-substituted carbamoyl group), and specific examples include a group selected from the following substituent group R-1a-4. More preferred examples include a phenyl group which is substituted with a C₆-C₁₀ aryl-substituted carbamoyl group (said aryl group may be substituted) or a heteroaryl-substituted carbamoyl group (said heteroaryl group may be substituted) in the 3-position, and substituted with hydroxy group in the 4-position (said phenyl group may be substituted in the 5-position), and specific examples include a group selected from the following substituent group R-la-5. Further preferred examples include 3-methoxy-4-hydroxy-5-(3,4-dihydroxyphenylcarbamoyl)phenyl group, 4-hydroxy-3-(3,4-dihydroxyphenylcarbamoyl)phenyl group, 4-hydroxy-3-[2-chloro-5-(trifluoromethyl)phenylcarbamoyl]phenyl group, 4-hydroxy-3-(2,3-dihydroxyphenylcarbamoyl)phenyl group, 3-methyl-4-hydroxy-5-(3,4-dihydroxyphenylcarbamoyl)phenyl group, 4-hydroxy-3-(3,4,5-trihydroxyphenylcarbamoyl)phenyl group, 4-hydroxy-3-(2,5-dichlorophenylcarbamoyl)phenyl group, 4-hydroxy-3-(2-chloro-5-nitrophenylcarbamoyl)phenyl group, or 3,4-dihydroxy-5-[3-(trifluoromethoxy)phenylcarbamoyl]phenyl group.
[Substituent Group R-1a-4]
3-methoxy-4-hydroxy-5-(3,4-dihydroxyphenylcarbamoyl)phenyl group, 4-hydroxy-3-(3,4-dimethoxyphenylcarbamoyl)phenyl group, 4-hydroxy-3-(3,4-dihydroxyphenylcarbamoyl)phenyl group, 4-hydroxy-3-(4-methoxyphenylcarbamoyl)phenyl group, 4-hydroxy-3-(4-hydroxyphenylcarbamoyl)phenyl group, 4-hydroxy-3-(3-methoxyphenylcarbamoyl)phenyl group, 4-hydroxy-3-(3-hydroxyphenylcarbamoyl)phenyl group, 3-(3,4-dimethoxyphenylcarbamoyl)phenyl group, 3-methoxy-4-hydroxy-5-(3-trifluoromethyl-4-methoxyphenylcarbamoyl)phenyl group, 3-methoxy-4-hydroxy-5-(3-methyl-4-hydroxyphenylcarbamoyl)phenyl group, 3-(3,4-dihydroxyphenylcarbamoyl)phenyl group, 3-(4-carboxyphenylcarbamoyl)phenyl group, 4-hydroxy-3-[(2-chloropyridin-5-yl)carbamoyl]phenyl group, 3-methyl-4-hydroxy-5-(3,4-dihydroxyphenylcarbamoyl)phenyl group, 4-hydroxy-3-[2,5-bis(trifluoromethyl)phenylcarbamoyl]phenyl group, 4-hydroxy-3-[3,5-bis(trifluoromethyl)phenylcarbamoyl]phenyl group, 4-hydroxy-3-[3-methoxy-4-(methoxycarbonyl)phenylcarbamoyl]phenyl group, 4-hydroxy-3-[(2-methoxypyridin-5-yl)carbamoyl]phenyl group, 4-hydroxy-3-[(2-hydroxypyridin-5-yl)carbamoyl]phenyl group, 4-hydroxy-3-[3-(trifluoromethoxy)phenylcarbamoyl]phenyl group, 4-hydroxy-3-(3,4-dichlorophenylcarbamoyl)phenyl group, 4-hydroxy-3-[4-(methoxycarbonyl)phenylcarbamoyl]phenyl group, 4-hydroxy-3-[3-(methoxycarbonyl)phenylcarbamoyl]phenyl group, 4-hydroxy-3-(4-carboxyphenylcarbamoyl)phenyl group, 4-hydroxy-3-(3-carboxyphenylcarbamoyl)phenyl group, 4-hydroxy-3-[2-(4-methoxyphenoxy)-5-(trifluoromethyl)phenylcarbamoyl]phenyl group, 4-hydroxy-3-[2-(4-hydroxyphenoxy)-5-(trifluoromethyl)phenylcarbamoyl]phenyl group, 4-hydroxy-3-[2-chloro-5-(trifluoromethyl)phenylcarbamoyl]phenyl group, 4-hydroxy-3-(3-nitrophenylcarbamoyl)phenyl group, 4-hydroxy-3-(3,4-methylenedioxyphenylcarbamoyl)phenyl group, 4-hydroxy-3-(3,4,5-trimethoxyphenylcarbamoyl)phenyl group, 4-hydroxy-3-(2,3-dimethoxyphenylcarbamoyl)phenyl group, 4-hydroxy-3-(3,4,5-trihydroxyphenylcarbamoyl)phenyl group, 4-hydroxy-3-(2,3-dihydroxyphenylcarbamoyl)phenyl group, 4-hydroxy-3-[3-hydroxy-4-(methoxycarbonyl)phenylcarbamoyl]phenyl group, 3-methoxy-4-hydroxy-5-[3,5-bis(trifluoromethyl)phenylcarbamoyl]phenyl group, 3-methoxy-4-hydroxy-5-[2-chloro-5-(trifluoromethyl)phenylcarbamoyl]phenyl group, 3-methoxy-4-hydroxy-5-(phenylcarbamoyl)phenyl group, 4-hydroxy-3-(2,5-dichlorophenylcarbamoyl)phenyl group, 4-hydroxy-3-[2-fluoro-5-(trifluoromethyl)phenylcarbamoyl]phenyl group, 4-hydroxy-3-(2-methyl-5-methoxyphenylcarbamoyl)phenyl group, 4-hydroxy-3-[3-methoxy-5-(trifluoromethyl)phenylcarbamoyl]phenyl group, 4-hydroxy-3-(2-methoxy-5-methylphenylcarbamoyl)phenyl group, 4-hydroxy-3-(2-methoxycarbonyl-5-chlorophenylcarbamoyl)phenyl group, 4-hydroxy-3-(2-methoxy-5-chlorophenylcarbamoyl)phenyl group, 4-hydroxy-3-(2-methoxy-5-phenylphenylcarbamoyl)phenyl group, 4-hydroxy-3-(2-methyl-5-hydroxyphenylcarbamoyl)phenyl group, 4-hydroxy-3-(2-hydroxy-5-methylphenylcarbamoyl)phenyl group, 4-hydroxy-3-(2-carboxy-5-chlorophenylcarbamoyl)phenyl group, 4-hydroxy-3-[3-hydroxy-5-(trifluoromethyl)phenylcarbamoyl]phenyl group, 4-hydroxy-3-[(2,2-difluoro-1,3-benzodioxol-4-yl)carbamoyl]phenyl group, 4-hydroxy-3-(2,5-difluorophenylcarbamoyl)phenyl group, 4-hydroxy-3-[2-nitro-5-(trifluoromethyl)phenylcarbamoyl]phenyl group, 4-hydroxy-3-[2-methoxy-5-(tert-butyl)phenylcarbamoyl]phenyl group, 4-hydroxy-3-[2-methoxy-5-(trifluoromethyl)phenylcarbamoyl]phenyl group, 3,4-dihydroxy-5-[2-chloro-5-(trifluoromethyl)phenylcarbamoyl]phenyl group, 3,4-dihydroxy-5-[3-(methoxycarbonyl)phenylcarbamoyl]phenyl group, 4-hydroxy-3-(2,5-dimethoxyphenylcarbamoyl)phenyl group, 4-hydroxy-3-(2-bromo-4-isopropylphenylcarbamoyl)phenyl group, 4-hydroxy-3-(2-chloro-5-nitrophenylcarbamoyl)phenyl group, 3,4-dihydroxy-5-(3,4-methylenedioxyphenylcarbamoyl)phenyl group, 3,4-dihydroxy-5-[3-(trifluoromethoxy)phenylcarbamoyl]phenyl group.
[Substituent Group R-1a-5]
3-methoxy-4-hydroxy-5-(3,4-dihydroxyphenylcarbamoyl)phenyl group, 4-hydroxy-3-(3,4-dimethoxyphenylcarbamoyl)phenyl group, 4-hydroxy-3-(3,4-dihydroxyphenylcarbamoyl)phenyl group, 4-hydroxy-3-(4-methoxyphenylcarbamoyl)phenyl group, 4-hydroxy-3-(4-hydroxyphenylcarbamoyl)phenyl group, 4-hydroxy-3-(3-methoxyphenylcarbamoyl)phenyl group, 4-hydroxy-3-(3-hydroxyphenylcarbamoyl)phenyl group, 3-methoxy-4-hydroxy-5-(3-trifluoromethyl-4-methoxyphenylcarbamoyl)phenyl group, 3-methoxy-4-hydroxy-5-(3-methyl-4-hydroxyphenylcarbamoyl)phenyl group, 4-hydroxy-3-[(2-chloropyridin-5-yl)carbamoyl]phenyl group, 3-methyl-4-hydroxy-5-(3,4-dihydroxyphenylcarbamoyl)phenyl group, 4-hydroxy-3-[2,5-bis(trifluoromethyl)phenylcarbamoyl]phenyl group, 4-hydroxy-3-[3,5-bis(trifluoromethyl)phenylcarbamoyl]phenyl group, 4-hydroxy-3-[3-methoxy-4-(methoxycarbonyl)phenylcarbamoyl]phenyl group, 4-hydroxy-3-[(2-methoxypyridin-5-yl)carbamoyl]phenyl group, 4-hydroxy-3-[(2-hydroxypyridin-5-yl)carbamoyl]phenyl group, 4-hydroxy-3-[3-(trifluoromethoxy)phenylcarbamoyl]phenyl group, 4-hydroxy-3-(3,4-dichlorophenylcarbamoyl)phenyl group, 4-hydroxy-3-[4-(methoxycarbonyl)phenylcarbamoyl]phenyl group, 4-hydroxy-3-[3-(methoxycarbonyl)phenylcarbamoyl]phenyl group, 4-hydroxy-3-(4-carboxyphenylcarbamoyl)phenyl group, 4-hydroxy-3-(3-carboxyphenylcarbamoyl)phenyl group, 4-hydroxy-3-[2-(4-methoxyphenoxy)-5-(trifluoromethyl)phenylcarbamoyl]phenyl group, 4-hydroxy-3-[2-(4-hydroxyphenoxy)-5-(trifluoromethyl)phenylcarbamoyl]phenyl group, 4-hydroxy-3-[2-chloro-5-(trifluoromethyl)phenylcarbamoyl]phenyl group, 4-hydroxy-3-(3-nitrophenylcarbamoyl)phenyl group, 4-hydroxy-3-(3,4-methylenedioxyphenylcarbamoyl)phenyl group, 4-hydroxy-3-(3,4,5-trimethoxyphenylcarbamoyl)phenyl group, 4-hydroxy-3-(2,3-dimethoxyphenylcarbamoyl)phenyl group, 4-hydroxy-3-(3,4,5-trihydroxyphenylcarbamoyl)phenyl group, 4-hydroxy-3-(2,3-dihydroxyphenylcarbamoyl)phenyl group, 4-hydroxy-3-[3-hydroxy-4-(methoxycarbonyl)phenylcarbamoyl]phenyl group, 3-methoxy-4-hydroxy-5-[3,5-bis(trifluoromethyl)phenylcarbamoyl]phenyl group, 3-methoxy-4-hydroxy-5-[2-chloro-5-(trifluoromethyl)phenylcarbamoyl]phenyl group, 3-methoxy-4-hydroxy-5-(phenylcarbamoyl)phenyl group, 4-hydroxy-3-(2,5-dichlorophenylcarbamoyl)phenyl group, 4-hydroxy-3-[2-fluoro-5-(trifluoromethyl)phenylcarbamoyl]phenyl group, 4-hydroxy-3-(2-methyl-5-methoxyphenylcarbamoyl)phenyl group, 4-hydroxy-3-[3-methoxy-5-(trifluoromethyl)phenylcarbamoyl]phenyl group, 4-hydroxy-3-(2-methoxy-5-methylphenylcarbamoyl)phenyl group, 4-hydroxy-3-(2-methoxycarbonyl-5-chlorophenylcarbamoyl)phenyl group, 4-hydroxy-3-(2-methoxy-5-chlorophenylcarbamoyl)phenyl group, 4-hydroxy-3-(2-methoxy-5-phenylphenylcarbamoyl)phenyl group, 4-hydroxy-3-(2-methyl-5-hydroxyphenylcarbamoyl)phenyl group, 4-hydroxy-3-(2-hydroxy-5-methylphenylcarbamoyl)phenyl group, 4-hydroxy-3-(2-carboxy-5-chlorophenylcarbamoyl)phenyl group, 4-hydroxy-3-[3-hydroxy-5-(trifluoromethyl)phenylcarbamoyl]phenyl group, 4-hydroxy-3-[(2,2-difluoro-1,3-benzodioxol-4-yl)carbamoyl]phenyl group, 4-hydroxy-3-(2,5-difluorophenylcarbamoyl)phenyl group, 4-hydroxy- 3- [2-nitro-5- (trifluoromethyl)phenylcarbamoyl]phenyl group, 4-hydroxy-3-[2-methoxy-5-(tert-butyl)phenylcarbamoyl]phenyl group, 4-hydroxy-3-[2-methoxy-5-(trifluoromethyl)phenylcarbamoyl]phenyl group, 3,4-dihydroxy-5-[2-chloro-5-(trifluoromethyl)phenylcarbamoyl]phenyl group, 3,4-dihydroxy-5-[3-(methoxycarbonyl)phenylcarbamoyl]phenyl group, 4-hydroxy-3-(2,5-dimethoxyphenylcarbamoyl)phenyl group, 4-hydroxy-3-(2-bromo-4-isopropylphenylcarbamoyl)phenyl group, 4-hydroxy-3-(2-chloro-5-nitrophenylcarbamoyl)phenyl group, 3,4-dihydroxy-5-(3,4-methylenedioxyphenylcarbamoyl)phenyl group, 3,4-dihydroxy-5-[3-(trifluoromethoxy)phenylcarbamoyl]phenyl group.

Furthermore, preferred examples of the aromatic group which may be substituted represented by R¹ include a phenyl group which is substituted with carboxy group (said phenyl group may further be substituted with one or more substituents other than the carboxy group). More preferred examples include a group selected from the following substituent group R-1a-6. Further preferred examples include 3-carboxyphenyl group or 3-carboxy-4-hydroxyphenyl group.
[Substituent Group R-1a-6] 3-methoxy-4-hydroxy-5-carboxyphenyl group, 3-carboxyphenyl group, 3-carboxy-4-hydroxyphenyl group, 3-methyl-4-hydroxy-5-carboxyphenyl group, 3-phenyl-4-hydroxy-5-carboxyphenyl group, 3,5-dihydroxy-4-carboxyphenyl group, 3,4-dihydroxy-5-carboxyphenyl group.

When W is -C(R³)=N-, preferred examples of the aromatic group represented by R¹ include a C₆-C₁₄ aryl group or a 5-membered heteroaryl group. More preferred examples include phenyl group, naphthyl group, anthryl group, furyl group, thienyl group, or imidazolyl group, and most preferred examples include phenyl group.

The aromatic group represented by R¹ may be substituted. Preferred examples of said substituent include a halogen atom; nitro group; a C₁-C₆ alkyl group (more preferably, methyl group and tert-butyl group); a C₆-C₁₀ aryl group (said aryl group may be substituted, and more preferred examples include 3-methoxycarbonylphenyl group, 3,5-dichlorophenyl group, 4-carboxyphenyl group, 2-chloro-5-carboxyphenyl group, 2-hydroxy-5-nitro group, 3,5-bis(trifluoromethyl)phenyl group, 3-chlorophenyl group, 3-chloro-4-methylphenyl group, 2-chlorophenyl group, 2-methyl-3-chlorophenyl group, 3-fluorophenyl group, 3-nitrophenyl group, and phenyl group); hydroxy group; a C₁-C₆ alkoxy group (said alkoxy group may be substituted, and more preferred examples include methoxy group, ethoxy group, carboxymethoxy group, and benzylcarbamoylmethoxy group); a C₂-C₆ alkenyloxy group (more preferably, allyloxy group); a heterocyclic carbonyloxy group (more preferably, thenoyloxy group); carboxy group; a C₁-C₆ alkoxycarbonyl group (more preferably, methoxycarbonyl group); a C₁-C₆ alkylsulfanyl group (said alkylsulfanyl group may be substituted, and more preferred examples include 2-dimethylaminoethylsulfanyl group); a C₁-C₄ alkylenedioxy group (more preferably, methylenedioxy group); a C₂-C₆ alkenyl group (said alkenyl group may be substituted, and more preferred examples include styryl group and 4-phenoxystyryl group); a C₆-C₁₀ aryloxy group (said aryloxy group may be substituted, and more preferred examples include phenoxy group, 4-bromophenoxy group, and 3,4-dimethoxyphenoxy group); a triC₁-C₆ alkylsilyl group (more preferably, tert-butyldimethylsilylgroup); and a C₂-C₁₂ dialkylamino group (more preferably, diethylamino group). Further preferred examples include hydroxy group, a C₂-C₆ alkenyl group (said alkenyl group may be substituted), or carboxy group. It is preferable that the aromatic group represented by R¹ is substituted with one to three substituents independently selected from the aforementioned substituents.

Examples of the aromatic group which may be substituted represented by R¹ include, for example, a group selected from the following substituent group R-1b-1. [Substituent Group R-1b-1] 3,5-dibromo-2-hydroxyphenyl group, 5-bromo-2-hydroxyphenyl group, 3-ethoxy-4-(thenoyloxy)phenyl group, 2,3-dihydroxyphenyl group, 3-carboxy-4-hydroxyphenyl group, 3,4-dihydroxyphenyl group, 2,4-dihydroxyphenyl group, 3-hydroxyphenyl group, 3,4,5-trihydroxyphenyl group, 3,4-dihydroxy-5-methoxyphenyl group, 2,4,5-trihydroxyphenyl group, 2,3,4-trihydroxyphenyl group, 4-carboxymethoxy-3-ethoxyphenyl group, 5-(3-methoxycarbonylphenyl)furan-2-yl group, 5-(4-carboxyphenyl)furan-2-yl group, 5-(3,5-dichlorophenyl)furan-2-yl group, 5-(5-carboxy-2-chlorophenyl)furan-2-yl group, 4-bromophenyl group, 5-(2-hydroxy-5-nitrophenyl)furan-2-yl group, 4-allyloxy-3-methoxyphenyl group, 4-benzyloxy-3-methoxyphenyl group, 4-chlorophenyl group, phenyl group, 2-benzyloxy-5-bromophenyl group, 4-benzylcarbamoylmethoxy-3-bromo-5-methoxyphenyl group, 5-[3,5-bis(trifluoromethyl)phenyl]furan-2-yl group, 5-(2-dimethylaminoethylsulfanyl)thiophen-2-yl group, 5-(3,5-dichlorophenyl)thiophen-2-yl group, 5-(3-chlorophenyl)furan-2-yl group, 5-(3-chloro-4-methylphenyl)furan-2-yl group, 5-(2-chlorophenyl)furan-2-yl group, 5-(3-chloro-2-methylphenyl)furan-2-yl group, 5-(3-fluorophenyl)furan-2-yl group, 5-(3-nitrophenyl)furan-2-yl group, 5-bromofuran-2-yl group, 3,4-methylenedioxyphenyl group, 3,4-dichlorophenyl group, 3-methoxycarbonylphenyl group, 4-chloro-3-nitrophenyl group, 3-carboxyphenyl group, 3-methoxy-4-methylphenyl group, 4-methoxycarbonylphenyl group, 2,4,5-trimethoxyphenyl group, 4-hydroxyphenyl group, 2-hydroxy-5-styrylphenyl group, 2-hydroxy-5-methoxyphenyl group, 2,5-dihydroxyphenyl group, 2-hydroxyphenyl group, 4-phenoxyphenyl group, 2-hydroxy-3-methoxy-5-bromophenyl group, 2,3-dihydroxy-5-bromophenyl group, 2-methoxy-5-styrylphenyl group, 4-styrylphenyl group, 3-methoxy-4-hydroxy-5-bromophenyl group, 3,4-dihydroxy-5-bromophenyl group, 3,4-dimethoxy-6-styrylphenyl group, 2-(tert-butyldimethylsilyloxy)-5-styrylphenyl group, 2-hydroxynaphthalen-1-yl-group, 9-anthryl group, 2-hydroxy-5-(tert-butyl)phenyl group, 2-hydroxy-3-methoxy-6-bromophenyl group, 2,3-dihydroxy-6-bromophenyl group, 2,6-dihydroxyphenyl group, 2-bromo-4,5-dihydroxyphenyl group, 2-nitro-5-hydroxyphenyl group, 3-methoxy-4-hydroxy-5-carboxyphenyl group, 2-hydroxy-5-diethylaminophenyl group, 3-methoxy-4-hydroxy-5-nitrophenyl group, 2-nitro-5-(4-bromophenoxy)phenyl group, 2-nitro-5-(3,4-dimethoxyphenoxy)phenyl group, 3,4-dichlorophenyl group, 2-hydroxy-5-(4-phenoxystyryl)phenyl group, biphenyl-4-yl group (4-phenylphenyl group), imidazol-4-yl group.

Preferred examples of the aromatic group which may be substituted represented by R¹ include a phenyl group which is substituted with one to three hydroxy groups (said phenyl group may further be substituted with one or more substituents other than the hydroxy group), and specific examples include a group selected from the following substituent group R-1b-2. More preferred examples include a phenyl group which is substituted with two or three hydroxy groups (said phenyl group may further be substituted with one or more substituents other than the hydroxy group), and specific examples include a group selected from the following substituent group R-1b-3. Further preferred examples include 2,4,5-trihydroxyphenyl group.
[Substituent Group R-1b-2] 3,5-dibromo-2-hydroxyphenyl group, 5-bromo-2-hydroxyphenyl group, 2,3-dihydroxyphenyl group, 3-carboxy-4-hydroxyphenyl group, 3,4-dihydroxyphenyl group, 2,4-dihydroxyphenyl group, 3-hydroxyphenyl group, 3,4,5-trihydroxyphenyl group, 3,4-dihydroxy-5-methoxyphenyl group, 2,4,5-trihydroxyphenyl group, 2,3,4-trihydroxyphenyl group, 4-hydroxyphenyl group, 2-hydroxy-5-styrylphenyl group, 2-hydroxy-5-methoxyphenyl group, 2,5-dihydroxyphenyl group, 2-hydroxyphenyl group, 2-hydroxy-3-methoxy-5-bromophenyl group, 2,3-dihydroxy-5-bromophenyl group, 3-methoxy-4-hydroxy-5-bromophenyl group, 3,4-dihydroxy-5-bromophenyl group, 2-hydroxy-5-(tert-butyl)phenyl group, 2-hydroxy-3-methoxy-6-bromophenyl group, 2,3-dihydroxy-6-bromophenyl group, 2,6-dihydroxyphenyl group, 2-bromo-4,5-dihydroxyphenyl group, 2-nitro-5-hydroxyphenyl group, 3-methoxy-4-hydroxy-5-carboxyphenyl group, 2-hydroxy-5-diethylaminophenyl group, 3-methoxy-4-hydroxy-5-nitrophenyl group, 2-hydroxy-5-(4-phenoxystyryl)phenyl group.
[Substituent Group R-1b-3] 2,3-dihydroxyphenyl group, 3,4-dihydroxyphenyl group, 2,4-dihydroxyphenyl group, 3,4,5-trihydroxyphenyl group, 3,4-dihydroxy-5-methoxyphenyl group, 2,4,5-trihydroxyphenyl group, 2,3,4-trihydroxyphenyl group, 2,5-dihydroxyphenyl group, 2,3-dihydroxy-5-bromophenyl group, 3,4-dihydroxy-5-bromophenyl group, 2,3-dihydroxy-6-bromophenyl group, 2,6-dihydroxyphenyl group, 2-bromo-4,5-dihydroxyphenyl group.

Furthermore, preferred examples of the aromatic group which may be substituted represented by R¹ include a phenyl group which is substituted with a C₂-C₆ alkenyl .group (said alkenyl group may be substituted, and said phenyl group may further be substituted with one or more substituents other than the C₂-C₆ alkenyl group), and specific examples include a group selected from the following substituent group R-1b-4. Further preferred examples include 2-hydroxy-5-styrylphenyl group or 4-styrylphenyl group.
[Substituent Group R-1b-4] 2-hydroxy-5-styrylphenyl group, 2-methoxy-5-styrylphenyl group, 4-styrylphenyl group, 3,4-dimethoxy-6-styrylphenyl group, 2-hydroxy-5-(4-phenoxystyryl)phenyl group.

Furthermore, preferred examples of the aromatic group which may be substituted represented by R¹ include a phenyl group which is substituted with carboxy group (said phenyl group may further be substituted with one or more substituents other than the carboxy group). More preferred examples include 3-carboxy-4-hydroxyphenyl group, 3-carboxyphenyl group, or 3-methoxy-4-hydroxy-5-carboxyphenyl group.

In the general formula (I), R² represents an aromatic group which may be substituted.

When W is -X-C(=Y)-, preferred examples of the aromatic group represented by R² include a C₆-C₁₀ aryl group or a 6-membered heteroaryl group. More preferred examples include phenyl group or pyridyl group, and most preferred examples include phenyl group.

The aromatic group represented by R² may be substituted. Preferred examples of said substituent include a halogen atom; nitro group; a C₁-C₆ alkyl group (more preferably, methyl group and tert-butyl group); a C₂-C₈ alkenyl group (said alkenyl group may be substituted, and more preferred examples include styryl group and 3,5-bis(trifluoromethyl)styryl group); a C₆-C₁₀ aryl group (more preferably, phenyl group); a halogenated C₁-C₆ alkyl group (more preferably, trifluoromethyl group); hydroxy group; a C₁-C₆ alkoxy group (more preferably, methoxy group); a C₆-C₁₀ aryloxy group (more preferably, phenoxy group); carboxy group; a C₂-C₇ alkoxycarbonyl group (more preferably, methoxycarbonyl group); a carbamoyl-substituted C₁-C₆ alkyl group (said carbamoyl group may be substituted, and more preferred examples include benzylcarbamoylmethyl group); a C₇-C₁₁ aroylamino group (said aroyl group may be substituted, and more preferred examples include benzoylamino group, 3,4-dichlorobenzoylamino group, and 4-phenoxybenzoylamino group); amino group; a C₆-C₁₀ aryl-substituted carbamoyl group (more preferably, phenylcarbamoyl group); a C₆-C₁₀ arylamino group (more preferably, phenylamino group); a C₂-C₇ alkanoylamino group (said alkanoyl group may be substituted, and more preferred examples include phenoxyacetylamino group); and a C₈-C₁₃ aralkylcarbonylamino group (said aralkyl group may be substituted, and more preferred examples include (3-phenoxyphenyl)acetylamino group, phenylacetylamino group, 3-phenylpropionylamino group, and 3,5-bis(trifluoromethyl)phenylacetylamino group). Further preferred examples include a halogen atom, a C₂-C₈ alkenyl group (said alkenyl group may be substituted), a C₇-C₁₁ aroylamino group (said aroyl group may be substituted), a halogenated C₁-C₆ alkyl group, a C₈-C₁₃ aralkylcarbonylamino group (said aralkyl group may be substituted), a C₂-C₇ alkoxycarbonyl group, or carboxy group. It is preferable that the aromatic group represented by R² is substituted with one to three substituents independently selected from the aforementioned substituents.

Examples of the aromatic group which may be substituted represented by R² include, for example, a group selected from the following substituent group R-2a-1. [Substituent Group R-2a-1] phenyl group, 3,5-bis(trifluoromethyl)phenyl group, 3,5-dimethoxyphenyl group, 3,4-dichlorophenyl group, 3,5-dichlorophenyl group, 3,5-di(tert-butyl)-4-hydroxyphenyl group, 2,4,5-trimethoxyphenyl group, 3-phenoxyphenyl group, 2,3-dichlorophenyl group, biphenyl-4-yl group (4-phenylphenyl group), 2,4-dichlorophenyl group, 3,5-difluorophenyl group, 3,5-dimethylphenyl group, 3-chlorophenyl group, 4-chlorophenyl group, 3-bromophenyl group, 4-bromophenyl group, 3-(trifluoromethyl)phenyl group, 4-(trifluoromethyl)phenyl group, 3,5-dibromophenyl group, 3-nitrophenyl group, 4-nitrophenyl group, 4-styrylphenyl group, 4-[3,5-bis(trifluoromethyl)styryl]phenyl group, 4-phenoxyphenyl group, 3-fluorophenyl group, 4-methoxyphenyl group, 4-chloro-3-methoxycarbonylphenyl group, 3-carboxy-4-chlorophenyl group, 2-hydroxyphenyl group, 2,5-dimethoxyphenyl group, 3,4-dimethoxyphenyl group, 3,4-dihydroxyphenyl group, 3,4,5-trihydroxyphenyl group, 4-benzylcarbamoylmethylphenyl group, 4-benzoylaminophenyl group, 4-aminophenyl group, 4-(3,4-dichlorobenzoylamino)phenyl group, 4-(phenylcarbamoyl)phenyl group, 4-(phenylamino)phenyl group, 5,6-dichloropyridin-3-yl group, 2,5-bis(trifluoromethyl)phenyl group, 4-(phenoxyacetylamino)phenyl group, 4-[(3-phenoxyphenyl)acetylamino]phenyl group, 4-(phenylacetylamino)phenyl group, 4-(3-phenylpropionylamino)phenyl group, 4-(4-phenoxybenzoylamino)phenyl group, 4-[3,5-bis(trifluoromethyl)phenylacetylamino]phenyl group, 3-(phenylamino)phenyl group, 3-[(3-phenoxyphenyl)acetylamino]phenyl group, 4-methoxycarbonylphenyl group, 4-carboxyphenyl group.

Preferred examples of the aromatic group which may be substituted represented by R² include a phenyl group which is substituted with a C₂-C₈ alkenyl group (said alkenyl group may be substituted, and said phenyl group may further be substituted with one or more substituents other than the C₂-C₈ alkenyl group), a phenyl group which is substituted with one or two halogen atoms (said phenyl group may further be substituted with one or more substituents other than the halogen atom), a phenyl group which is substituted with a C₇-C₁₁ aroylamino group (said aroyl group may be substituted, and said phenyl group may further be substituted with one or more substituents other than the C₇-C₁₁ aroylamino group), a phenyl group which is substituted with one or two halogenated C₁-C₆ alkyl groups (said phenyl group may further be substituted with one or more substituents other than the halogenated C₁-C₆ alkyl group), a phenyl group which is substituted with a C₈-C₁₃ aralkylcarbonylamino group (said aralkyl group may be substituted, and said phenyl group may further be substituted with one or more substituents other than the C₈-C₁₃ aralkylcarbonylamino group), or a phenyl group which is substituted with a C₂-C₇ alkoxycarbonyl group (said phenyl group may further be substituted with one or more substituents other than the C₂-C₇ alkoxycarbonyl group), and specific examples include a group selected from the following substituent group R-2a-2. More preferred examples include 4-styrylphenyl group, 2,4-dichlorophenyl group, 3,4-dichlorophenyl group, 4-(4-phenoxybenzoylamino)phenyl group, 2,3-dichlorophenyl group, 4-bromophenyl group, 4-(trifluoromethyl)phenyl group, 4-[(3-phenoxyphenyl)acetylamino]phenyl group, 4-[3,5-bis(trifluoromethyl)phenylacetylamino]phenyl group, 3-[(3-phenoxyphenyl)acetylamino]phenyl group, 3,5-bis(trifluoromethyl)phenyl group, or 4-methoxycarbonylphenyl group.
[Substituent Group R-2a-2] 3,4-dichlorophenyl group, 3,5-dichlorophenyl group, 2,3-dichlorophenyl group, 2,4-dichlorophenyl group, 3,5-difluorophenyl group, 3-chlorophenyl group, 4-chlorophenyl group, 3-bromophenyl group, 4-bromophenyl group, 3,5-dibromophenyl group, 4-styrylphenyl group, 4-[3,5-bis(trifluoromethyl)styryl]phenyl group, 3-fluorophenyl group, 4-chloro-3-methoxycarbonylphenyl group, 3-carboxy-4-chlorophenyl group, 4-benzoylaminophenyl group, 4-(3,4-dichlorobenzoylamino)phenyl group, 4-(4-phenoxybenzoylamino)phenyl group, 3,5-bis(trifluoromethyl)phenyl group, 3-(trifluoromethyl)phenyl group, 4-(trifluoromethyl)phenyl group, 2,5-bis(trifluoromethyl)phenyl group, 4-[(3-phenoxyphenyl)acetylamino]phenyl group, 4-(phenylacetylamino)phenyl group, 4-(3-phenylpropionylamino)phenyl group, 4-[3,5-bis(trifluoromethyl)phenylacetylamino]phenyl group, 3-[(3-phenoxyphenyl)acetylamino]phenyl group, 4-methoxycarbonylphenyl group.

Furthermore, preferred examples of the aromatic group which may be substituted represented by R² include a phenyl group which is substituted with carboxy group (said phenyl group may further be substituted with one or more substituents other than the carboxy group). More preferred examples include 3-carboxy-4-chlorophenyl group, or 4-carboxyphenyl group.

When W is -C(R³)=N-, preferred examples of the aromatic group represented by R² include a C₆-C₁₀ aryl group or a 6-membered heteroaryl group. More preferred examples include phenyl group or pyridyl group, and most preferred examples include phenyl group.

The aromatic group represented by R² may be substituted. Preferred examples of said substituent include a halogen atom; nitro group; a C₁-C₆ alkyl group (more preferably, methyl group); a halogenated C₁-C₆ alkyl group (more preferably, trifluoromethyl group); a C₁-C₆ alkoxy group (more preferably, methoxy group); carboxy group; a C₂-C₇ alkoxycarbonyl group (more preferably, methoxycarbonyl group); sulfamoyl group; and a C₂-C₆ alkenyl group (said alkenyl group may be substituted, and more preferred examples include 2-(pyridin-2-yl)vinyl group). Further preferred examples include a halogen atom, nitro group, or carboxy group. It is preferable that the aromatic group represented by R² is substituted with one or two substituents independently selected from the aforementioned substituents.

Examples of the aromatic group which may be substituted represented by R² include, for example, a group selected from the following substituent group R-2b-1. [Substituent Group R-2b-1] phenyl group, 3,4-dichlorophenyl group, 3-carboxyphenyl group, 3,5-dichlorophenyl group, 3-methoxycarbonylphenyl group, 3-(trifluoromethyl)phenyl group, 4-methylphenyl group, 4-nitrophenyl group, 3-chlorophenyl group, 3,5-bis(trifluoromethyl)phenyl group, 3-chloro-4-methylphenyl group, 2,3-dimethylphenyl group, 3-methylphenyl group, 3-methoxyphenyl group, 3-nitrophenyl group, 4-fluorophenyl group, 4-chlorophenyl group, 3-carboxy-4-chlorophenyl group, 3-ethoxycarbonylphenyl group, 4-sulfamoylphenyl group, 6-chloropyridin-3-yl group, 2,5-dichlorophenyl group, 4-carboxyphenyl group, 3-chloro-4-fluorophenyl group, 4-[2-(pyridin-2-yl)vinyl]phenyl group.

Preferred examples of the aromatic group which may be substituted represented by R² include a phenyl group which is substituted with nitro group (said phenyl group may further be substituted with one or more substituents other than the nitro group), or a phenyl group which is substituted with one or two halogen atoms (said phenyl group may further be substituted with one or more substituents other than the halogen atom), and specific examples include a group selected from the following substituent group R-2b-2. More preferred examples include 4-nitrophenyl group, 3,4-dichlorophenyl group, 3,5-dichlorophenyl group, or 3-chloro-4-fluorophenyl group.
[Substituent Group R-2b-2] 3,4-dichlorophenyl group, 3,5-dichlorophenyl group, 4-nitrophenyl group, 3-chlorophenyl group, 3-chloro-4-methylphenyl group, 3-nitrophenyl group, 4-fluorophenyl group, 4-chlorophenyl group, 3-carboxy-4-chlorophenyl group, 2,5-dichlorophenyl group, 3-chloro-4-fluorophenyl group.

Furthermore, preferred examples of the aromatic group which may be substituted represented by R² include a phenyl group which is substituted with carboxy group (said phenyl group may further be substituted with one or more substituents other than the carboxy group). More preferred examples include 3-carboxyphenyl group, 3-carboxy-4-chlorophenyl group, or 4-carboxyphenyl group. Further preferred examples include 4-carboxyphenyl group.

In the general formula (I), Z represents a single bond or a connecting group wherein a number of atoms in a main chain is 1 to 3 (said connecting group may be substituted). The "connecting group wherein a number of atoms of main chain is 1 to 3" represented by Z means a connecting group wherein 1 to 3 atoms in a main chain link together between the nitrogen atom and R² constituting the 5-membered hetero ring. The number of atoms of the main chain is counted so as to minimize the number of connecting atoms existing between said nitrogen atom and R², regardless of the presence or absence of hetero atom(s). The connecting group represented by Z may be substituted.

When W is -X-C(=Y)-, preferred examples of Z include a single bond, methylene group, ethylene group, -CH₂CO- group, or -CH₂CONH- group. More preferred examples include methylene group.

When W is -C(R³)=N-, preferred examples of Z include a single bond or methylene group. More preferred examples include a single bond.

When W is -X-C(=Y)-, the compound wherein R¹ is an aromatic group which may be substituted,
R² is an aromatic group which may be substituted,
X is sulfur atom,
Y is oxygen atom or sulfur atom,
Z is methylene group is preferred as the compound represented by the general formula (I).

The compounds which correspond to any one of the following (A), (B) and (C) are more preferred:
(A) the compound wherein R¹ is a phenyl group which is substituted with one to three hydroxy groups (said phenyl group may further be substituted with one or more substituents other than the hydroxy group),
   R² is an aromatic group which may be substituted,
   X is sulfur atom,
   Y is oxygen atom or sulfur atom,
   Z is methylene group;
(B) the compound wherein R¹ is a phenyl group which is substituted with a C₆-C₁₀ aryl-substituted carbamoyl group (said aryl group may be substituted, and said phenyl group may further be substituted with one or more substituents other than the C₆-C₁₀ aryl-substituted carbamoyl group), or a phenyl group which is substituted with a heteroaryl-substituted carbamoyl group (said heteroaryl group may be substituted, and said phenyl group may further be substituted with one or more substituents other than the heteroaryl-substituted carbamoyl group),
   R² is an aromatic group which may be substituted,
   X is sulfur atom,
   Y is oxygen atom or sulfur atom,
   Z is methylene group;
(C) the compound wherein R¹ and R² are either the following (i) or (ii),
   X is sulfur atom,
   Y is oxygen atom or sulfur atom,
   Z is methylene group:
   (i) R¹ is a phenyl group which is substituted with carboxy group (said phenyl group may further be substituted with one or more substituents other than the carboxy group), R² is an aromatic group which may be substituted;
   (ii) R¹ is an aromatic group which may be substituted,
      R² is a phenyl group which is substituted with carboxy group (said phenyl group may further be substituted with one or more substituents other than the carboxy group).

The compound wherein R¹ is a phenyl group which is substituted with two or three hydroxy groups (said phenyl group may further be substituted with one or more substituents other than the hydroxy group),
R² is a phenyl group which may be substituted,
X is sulfur atom,
Y is oxygen atom or sulfur atom,
Z is methylene group is preferred as the compound which corresponds to the aforementioned (A).

The compound selected from the compounds represented by the following compound numbers described in the specification is more preferred:
- Compound Nos. 3, 4, 8 to 27, 29 to 38, 40 to 54, 56 to 61, 70, 81 to 84, 89, 301 to 307,. 309, 311 to 321, 334, 335, 338, 339, 341, 342, 344, 347, 432, 446, 463, 464, 469, 470, 474, and 475.

The compound selected from the following 14 compounds is further preferred:
- the compound wherein R¹ is 3,4,5-trihydroxyphenyl group, R² is 3,4-dichlorophenyl group, X is sulfur atom, Y is oxygen atom, Z is methylene group;
- the compound wherein R¹ is 2,3-dihydroxyphenyl group, R² is 2,3-dichlorophenyl group, X is sulfur atom, Y is oxygen atom, Z is methylene group;
- the compound wherein R¹ is 2,3-dihydroxyphenyl group, R² is 2,4-dichlorophenyl group, X is sulfur atom, Y is oxygen atom, Z is methylene group;
- the compound wherein R¹ is 3,4-dihydroxyphenyl group, R² is 2,4-dichlorophenyl group, X is sulfur atom, Y is oxygen atom, Z is methylene group;
- the compound wherein R¹ is 2,3-dihydroxyphenyl group, R² is 4-bromophenyl group, X is sulfur atom, Y is oxygen atom, Z is methylene group;
- the compound wherein R¹ is 2,3-dihydroxyphenyl group, R² is 4-(trifluoromethyl)phenyl group, X is sulfur atom, Y is oxygen atom, Z is methylene group;
- the compound wherein R¹ is 2,3-dihydroxyphenyl group, R² is 4-styrylphenyl group, X is sulfur atom, Y is oxygen atom, Z is methylene group;
- the compound wherein R¹ is 3,4-dihydroxyphenyl group, R² is 3,4-dichlorophenyl group, X is sulfur atom, Y is sulfur atom, Z is methylene group;
- the compound wherein R¹ is 2,3-dihydroxyphenyl group, R² is 4-[(3-phenoxyphenyl)acetylamino]phenyl group, X is sulfur atom, Y is oxygen atom, Z is methylene group;
- the compound wherein R¹ is 2,3-dihydroxyphenyl group, R² is 4-(4-phenoxybenzoylamino)phenyl group, X is sulfur atom, Y is oxygen atom, Z is methylene group;
- the compound wherein R¹ is 2,3-dihydroxyphenyl group, R² is 4-[3,5-bis(trifluoromethyl)phenylacetylamino]phenyl group, X is sulfur atom, Y is oxygen atom, Z is methylene group;
- the compound wherein R¹ is 2,3-dihydroxyphenyl group, R² is 3-[(3-phenoxyphenyl)acetylamino]phenyl group, X is sulfur atom, Y is oxygen atom, Z is methylene group;
- the compound wherein R¹ is 3,4-dihydroxy-5-nitrophenyl group, R² is 3,4-dichlorophenyl group, X is sulfur atom, Y is sulfur atom, Z is methylene group; and
- the compound wherein R¹ is 3,4-dihydroxy-5-[3-(trifluoromethoxy)phenylcarbamoyl]phenyl group, R² is 3,4-dichlorophenyl group, X is sulfur atom, Y is sulfur atom, Z is methylene group.

The compound wherein R¹ is a phenyl group which is substituted with a C₆-C₁₀ aryl-substituted carbamoyl group in the 3-position (said aryl group may be substituted, and said phenyl group may further be substituted with one or more substituents other than the C₆·C₁₀ aryl-substituted carbamoyl group) or a phenyl group which is substituted with a heteroaryl-substituted carbamoyl group (said heteroaryl group may be substituted, and said phenyl group may further be substituted with one or more substituents other than the heteroaryl-substituted carbamoyl group), and a phenyl group which is substituted with hydroxy group in the 4-position (said phenyl group may be substituted in the 5-position),
R² is a phenyl group which may be substituted,
X is sulfur atom,
Y is oxygen atom,
Z is methylene group is preferred as the compound which corresponds to the aforementioned (B).

The compound selected from the compounds represented by the following compound numbers described in the specification is more preferred:
- Compound Nos. 349, 351 to 356, 360, 361, 371 to 378, 383 to 386, 391 to 408, 414 to 424, 428, 433 to 442, 450 to 460, and 465 to 475.

The compound selected from the following 11 compounds is further preferred:
- the compound wherein R¹ is 3-methoxy-4-hydroxy-5-(3,4-dihydroxyphenylcarbamoyl)phenyl group, R² is 3,4-dichlorophenyl group, X is sulfur atom, Y is oxygen atom, Z is methylene group;
- the compound wherein R¹ is 4-hydroxy-3-(3,4-dihydroxyphenylcarbamoyl)phenyl group, R² is 3,4-dichlorophenyl group, X is sulfur atom, Y is oxygen atom, Z is methylene group;
- the compound wherein R¹ is 4-hydroxy-3-(3,4-dihydroxyphenylcarbamoyl)phenyl group, R² is 3,5-bis(trifluoromethyl)phenyl group, X is sulfur atom, Y is oxygen atom, Z is methylene group;
- the compound wherein R¹ is 3-methyl-4-hydroxy-5-(3,4-dihydroxyphenylcarbamoyl)phenyl group, R² is 3,4-dichlorophenyl group, X is sulfur atom, Y is oxygen atom, Z is methylene group;
- the compound wherein R¹ is 4-hydroxy-3-(3,4-dihydroxyphenylcarbamoyl)phenyl group, R² is 4-(trifluoromethyl)phenyl group, X is sulfur atom, Y is oxygen atom, Z is methylene group;
- the compound wherein R¹ is 4-hydroxy-3-[2-chloro-5-(trifluoromethyl)phenylcarbamoyl]phenyl group, R² is 3,4-dichlorophenyl group, X is sulfur atom, Y is oxygen atom, Z is methylene group;
- the compound wherein R¹ is 4-hydroxy-3-(3,4,5-trihydroxyphenylcarbamoyl)phenyl group, R² is 3,4-dichlorophenyl group, X is sulfur atom, Y is oxygen atom, Z is methylene group;
- the compound wherein R¹ is 4-hydroxy-3-(2,3-dihydroxyphenylcarbamoyl)phenyl group, R² is 3,4-dichlorophenyl group, X is sulfur atom, Y is oxygen atom, Z is methylene group;
- the compound wherein R¹ is 4-hydroxy-3-(2,5-dichlorophenylcarbamoyl)phenyl group, R² is 3,4-dichlorophenyl group, X is sulfur atom, Y is oxygen atom, Z is methylene group;
- the compound wherein R¹ is 4-hydroxy-3-(2-chloro-5-nitrophenylcarbamoyl)phenyl group, R² is 3,4-dichlorophenyl group, X is sulfur atom, Y is oxygen atom, Z is methylene group; and
- the compound wherein R¹ is 3,4-dihydroxy-5-[3-(trifluoromethyl)phenylcarbamoyl]phenyl group, R² is 3,4-dichlorophenyl group, X is sulfur atom, Y is oxygen atom, Z is methylene group.

The compound selected from the compounds represented by the following compound numbers described in the specification is preferred as the compound which corresponds to the aforementioned (C):
- Compound Nos. 345, 350, 358, 390, 409, 418, 430, 446, and 464.

The compound selected from the following 2 compounds is more preferred:
- the compound wherein R¹ is 3-carboxyphenyl group, R² is 3,4-dichlorophenyl group, X is sulfur atom, Y is oxygen atom, Z is methylene group; and
- the compound wherein R¹ is 3-carboxy-4-hydroxyphenyl group, R² is 4-methoxycarbonylphenyl group, X is sulfur atom, Y is oxygen atom, Z is methylene group.

When W is -C(R³)=N-, the compound wherein R¹ is an aromatic group which may be substituted,
R² is an aromatic group which may be substituted,
R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
Z is a single bond is preferred as the compound represented by the general formula (I).

The compound which corresponds to any one of the following (D), (E) and (F) is more preferred:
(D) the compound wherein R¹ is a phenyl group which is substituted with one to three hydroxy groups (said phenyl group may further be substituted with one or more substituents other than the hydroxy group),
   R² is an aromatic group which may be substituted,
   R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
   Z is a single bond;
(E) the compound wherein R¹ is a phenyl group which is substituted with C₂-C₆ alkenyl group which may be substituted (said phenyl group may further be substituted with one or more substituents other than the C₂-C₆ alkenyl group),
   R² is an aromatic group which may be substituted,
   R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
   Z is a single bond;
(F) the compound wherein R¹, R² and R³ are any one of the following (i) to (iii), Z is a single bond:
   (i) R¹ is a phenyl group which is substituted with carboxy group (said phenyl group may further be substituted with one or more substituents other than the carboxy group), R² is an aromatic group which may be substituted,
      R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group;
   (ii) R¹ is an aromatic group which may be substituted,
      R² is a phenyl group which is substituted with carboxy group (said phenyl group may further be substituted with one or more substituents other than the carboxy group), R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group;
   (iii) R¹ is an aromatic group which may be substituted,
      R² is an aromatic group which may be substituted,
      R³ is carboxy group.

The compound wherein R¹ is a phenyl group which is substituted with two or three hydroxy groups (said phenyl group may further be substituted with one or more substituents other than the hydroxy group),
R² is a phenyl group which may be substituted,
R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
Z is a single bond is preferred as the compound which corresponds to the aforementioned (D).

The compound selected from the compounds represented by the following compound numbers described in the specification is more preferred:
- Compound Nos. 104, 107, 109 to 114, 116 to 120, 123 to 158, 221 to 224, 501 to 505, 514, 515, 519, 522, 529, 540, 545, 551 to 554, 556, and 557.

The compound selected from the following 6 compounds is further preferred:
- the compound wherein R¹ is 2,4,5-trihydroxyphenyl group, R² is 3,5-dichlorophenyl group, R³ is phenyl group, Z is a single bond;
- the compound wherein R¹ is 2,4,5-trihydroxyphenyl group, R² is 3,4-dichlorophenyl group, R³ is isopropyl group, Z is a single bond;
- the compound wherein R¹ is 2,4,5-trihydroxyphenyl group, R² is 4-nitrophenyl group, R³ is ethoxy group, Z is a single bond;
- the compound wherein R¹ is 2,4,5-trihydroxyphenyl group, R² is 3,4-dichlorophenyl group, R³ is methyl group, Z is a single bond;
- the compound wherein R¹ is 2,4,5-trihydroxyphenyl group, R² is 3,5-dichlorophenyl group, R³ is isopropyl group, Z is a single bond; and
- the compound wherein R¹ is 2,4,5-trihydroxyphenyl group, R² is 3-chloro-4-fluorophenyl group, R³ is isopropyl group, Z is a single bond.

The compound selected from the compounds represented by the following compound numbers described in the specification is preferred as the compound which corresponds to the aforementioned (E):
- Compound Nos. 517, 524, 525, 530 to 538, 541 to 544, 565, and 566.

The compound selected from the following 2 compounds is more preferred:
the compound wherein R¹ is 2-hydroxy-5-styrylphenyl group, R² is 4-nitrophenyl group, R³ is ethoxy group, Z is a single bond; and
• the compound wherein R¹ is 4-styrylphenyl group, R² is 4-carboxyphenyl group, R³ is isopropyl group, Z is a single bond.

The compound selected from the compounds represented by the following compound numbers described in the specification is preferred as the compound which corresponds to the aforementioned (F):
- Compound Nos. 103, 106, 153, 162, 164, 168 to 176, 179, 184, 187 to 193, 198, 211, 212, 215 to 218, 507, 534 to 536, 555, 559, 564, and 567.

The following compounds is more preferred:
- the compound wherein R¹ is 4-styrylphenyl group, R² is 4-carboxyphenyl group, R³ is isopropyl group, Z is a single bond.

In the general formula (II), W¹⁰¹ represents a group selected from the following connecting group W-101-1.

### [Connecting Group W101-1]

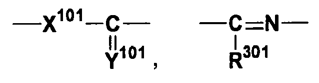

When W¹⁰¹ is -X¹⁰¹-C(=Y¹⁰¹)-, X¹⁰¹ represents sulfur atom or NH, and Y¹⁰¹ represents oxygen atom or sulfur atom. Preferably, X¹⁰¹ is sulfur atom. Preferably, Y¹⁰¹ is oxygen atom.

When W¹⁰¹ is -C(R³⁰¹)=N-, R³⁰¹ represents a hydrocarbon group which may be substituted, hydroxy group which may be substituted, or carboxy group which may be esterified.

Preferred examples of the hydrocarbon group represented by R³⁰¹ include a C₁-C₆ alkyl group or a C₆-C₁₀ aryl group. More preferred examples include methyl group, ethyl group, isopropyl group, tert-butyl group, or phenyl group.

The hydrocarbon group represented by R³⁰¹ may be substituted. Preferred examples of said substituent include a halogen atom; a C₁-C₆ alkoxy group (more preferably, methoxy group); hydroxy group; and a C₂-C₇ alkoxycarbonyl group (more preferably, ethoxycarbonyl group).

Preferred examples of the hydrocarbon group which may be substituted represented by R³⁰¹ include a C₁-C₆ alkyl group (more preferably, methyl group, ethyl group, isopropyl group, and tert-butyl group); a C₆-C₁₀ aryl group (said aryl group may be substituted, and more preferred examples include phenyl group, 3,4,5-trimethoxyphenyl group, 4-hydroxyphenyl group, and 2-hydroxy-5-bromophenyl group); a halogenated C₁-C₆ alkyl group (more preferably, trifluoromethyl group); a C₂-C₇ alkoxycarbonyl-substituted C₁-C₆ alkyl group (more preferably, 2-(ethoxycarbonyl)ethyl group and 3-(ethoxycarbonyl)propyl group); and a C₁-C₆ alkoxy-substituted C₁-C₆ alkyl group (more preferably, methoxymethyl group).
Further preferred examples include methyl group, isopropyl group, or phenyl group.

Preferred examples of the hydroxy group which may be substituted represented by R³⁰¹ include hydroxy group or a C₁-C₆ alkoxy group. More preferred examples include hydroxy group or ethoxy group, and further preferred examples include ethoxy group.

Preferred examples of the carboxy group which may be esterified represented by R³⁰¹ include carboxy group or a C₂-C₇ alkoxycarbonyl group. More preferred examples include carboxy group or ethoxycarbonyl group.

Preferably, W¹⁰¹ is -S-C(=O)- or -C(R³⁰¹)=N-.

In the general formula (II), R¹⁰¹ represents an aromatic group which may be substituted.

When W¹⁰¹ is -X¹⁰¹-C(=Y¹⁰¹)-, preferred examples of the aromatic group represented by R¹⁰¹ include a C₆-C₁₀ aryl group or a 5- to 9-membered heteroaryl group. More preferred examples include phenyl group, furyl group, pyridyl group, benzo[2,1,3]oxadiazolyl group, or indolyl group, and most preferred examples include phenyl group.

The aromatic group represented by R¹⁰¹ may be substituted. Preferred examples of said substituent include a halogen atom; nitro group; a C₁-C₆ alkyl group (more preferably, methyl group and isopropyl group); a C₆-C₁₀ aryl group (said aryl group may be substituted, and more preferred examples include 3-carboxyphenyl group, 3,5-dichlorophenyl group, phenyl group, 3,4-dimethoxyphenyl group, 3,4-dihydroxyphenyl group, 2,6-dimethoxyphenyl group, 4-methoxyphenyl group, 2,6-dihydroxyphenyl group, and 4-hydroxyphenyl group); hydroxy group; a C₁-C₆ alkoxy group (said alkoxy group may be substituted, and more preferred examples include methoxy group and methoxymethoxy group); a C₂-C₇ alkoxycarbonyl group (more preferably, methoxycarbonyl group and ethoxycarbonyl group); a C₁-C₃ alkylenedioxy group (more preferably, methylenedioxy group); a C₂-C₇ alkanoylamino group (more preferably, acetylamino group); amino group; a C₆-C₁₀ aryloxy group (more preferably, phenoxy group); a C₂-C₆ alkenyl group (more preferably, styryl group); a halogenated C₁-C₆ alkyl group (more preferably, trifluoromethyl group); a C₂-C₇ alkoxycarbonyloxy group (more preferably, ethoxycarbonyloxy group); carboxy group; a C₆-C₁₀ aryl-substituted carbamoyl group (said aryl group may be substituted, and more preferred examples include 3,4-dihydroxyphenylcarbamoyl group, 3,4-dimethoxyphenylcarbamoyl group, 4-methoxyphenylcarbamoyl group, 4-hydroxyphenylcarbamoyl group, 3-methoxyphenylcarbamoyl group, 3-hydroxyphenylcarbamoyl group, 3-trifluoromethyl-4-methoxyphenylcarbamoyl group, 3-methyl-4-hydroxyphenylcarbamoyl group, 4-carboxyphenylcarbamoyl group, 2,5-bis(trifluoromethyl)phenylcarbamoyl group, 3,5-bis(trifluoromethyl)phenylcarbamoyl group, 3-methoxy-4-(methoxycarbonyl)phenylcarbamoyl group, 3-(trifluoromethoxy)phenylcarbamoyl group, 3,4-dichlorophenylcarbamoyl group, 4-(methoxycarbonyl)phenylcarbamoyl group, 3-(methoxycarbonyl)phenylcarbamoyl group, 3-carboxyphenylcarbamoyl group, 2-(4-methoxyphenoxy)-5-(trifluoromethyl)phenylcarbamoyl group, 2-(4-hydroxyphenoxy)-5-(trifluoromethyl)phenylcarbamoyl group, 2-chloro-5-(trifluoromethyl)phenylcarbamoyl group, 3-nitrophenylcarbamoyl group, 3,4-methylenedioxyphenylcarbamoyl group, 3,4,5-trimethoxyphenylcarbamoyl group, 2,3-dimethoxyphenylcarbamoyl group, 3,4,5-trihydroxyphenylcarbamoyl group, 2,3-dihydroxyphenylcarbamoyl group, 3-hydroxy-4-(methoxycarbonyl)phenylcarbamoyl group, phenylcarbamoyl group, 2,5-dichlorophenylcarbamoyl group, 2-fluoro-5-(trifluoromethyl)phenylcarbamoyl group, 2-methyl-5-methoxyphenylcarbamoyl group, 3-methoxy-5-(trifluoromethyl)phenylcarbamoyl group, 2-methoxy-5-methylphenylcarbamoyl group, 2-methoxycarbonyl-5-chlorophenylcarbamoyl group, 2-methoxy-5-chlorophenylcarbamoyl group, 2-methoxy-5-phenylphenylcarbamoyl group, 2-methyl-5-hydroxyphenylcarbamoyl group, 2-hydroxy-5-methyl phenylcarbamoyl group, 2-carboxy-5-chlorophenylcarbamoyl group, 3-hydroxy-5-(trifluoromethyl)phenylcarbamoyl group, (2,2-difluoro-1,3-benzodioxol-4-yl)carbamoyl group, 2,5-difluorophenylcarbamoyl 1 group, 2-nitro-5-(trifluoromethyl)phenylcarbamoyl group, 2-methoxy-5-(tert-butyl)phenylcarbamoyl group, 2-methoxy-5-(trifluoromethyl)phenylcarbamoyl group, 2,5-dimethoxyphenylcarbamoyl group, 2-bromo-4-isopropylphenylcarbamoyl group, and 2-chloro-5-nitrophenylcarbamoyl group); a C₆-C₁₀ aroylamino group (said aroyl group may be substituted, and more preferred examples include 3,4-dihydroxybenzoylamino group, 4-(trifluoromethoxy)benzoylamino group, 4-methoxybenzoylamino group, 4-hydroxybenzoylamino group, and 3,4-dimethoxybenzoylamino group); a C₈-C₁₃ aralkyloxycarbonyl group (said aralkyl group may be substituted, and more preferred examples include 4-methoxybenzyloxycarbonyl group and benzyloxycarbonyl group); a heteroaryl-substituted carbamoyl group (said heteroaryl group may be substituted, and more preferred examples include (2-chloropyridin-5-yl)carbamoyl group, (2-methoxypyridin-5-yl)carbamoyl group, and (2-hydroxypyridin-5-yl)carbamoyl group); a C₇-C₁₂ aralkyl group (said aralkyl group may be substituted, and more preferred examples include 3,4-dibenzyloxybenzyl group, 3,4-dihydroxybenzyl group, 4-methoxycarbonylbenzyl group); and a C₆-C₁₀ aroyl group (said aroyl group may be substituted, and more preferred examples include 3,4-dimethoxybenzoyl group, 4-methoxycarbonylbenzoyl group). Further preferred examples include hydroxy group, a C₆-C₁₀ aryl-substituted carbamoyl group (said aryl group may be substituted), a heteroaryl-substituted carbamoyl group (said heteroaryl group may be substituted), carboxy group, nitro group, a C₁-C₆ alkyl group, or a C₁-C₆ alkoxy group (said alkoxy group may be substituted). It is preferable that the aromatic group represented by R¹⁰¹ is substituted with one to three substituents independently selected from the aforementioned substituents.

Examples of the aromatic group which may be substituted represented by R¹⁰¹ include, for example, a group selected from the following substituent group R-101a-1. [Substituent Group R-101a-1] 3,5-dibromo-2-hydroxyphenyl group, 2,3-dihydroxyphenyl group, 3,4-dihydroxyphenyl group, 2,4,5-trimethoxyphenyl group, 2-hydroxy-3-methylphenyl group, 3-hydroxyphenyl group, 3,4,5-trihydroxyphenyl group, 3-ethoxycarbonyl-4-hydroxyphenyl group, 2,3,4-trihydroxyphenyl group, 3,4-dihydroxy-5-methoxyphenyl group, 5-hydroxy-2-nitrophenyl group, 3,5-dihydroxyphenyl group, 2,4-dihydroxyphenyl group, 2-hydroxy-5-nitrophenyl group, 5-(3-carboxyphenyl)furan-2-yl group, 5-chloro-2-hydroxyphenyl group, 5-(3,5-dichlorophenyl)furan-2-yl group, 3,4-methylenedioxyphenyl group, 4-bromophenyl group, benzo[2,1,3]oxadiazol-5-yl group, 4-pyridyl group, 3,4-dichlorophenyl group, 4-acetamidophenyl group, 4-aminophenyl group, 4-hydroxy-3-nitrophenyl group, 3-amino-4-hydroxyphenyl group, 3-hydroxy-4-nitrophenyl group, 4-amino-3-hydroxyphenyl group, 4-phenoxyphenyl group, 3-phenoxyphenyl group, 2-hydroxy-5-styrylphenyl group, 3-hydroxy-4-methoxyphenyl group, 3-hydroxy-4,5-methylenedioxyphenyl group, 2-fluoro-3-hydroxyphenyl group, 3-hydroxy-4-fluorophenyl group, 3-methoxy-4-chlorophenyl group, 3-hydroxy-4-chlorophenyl group, 2-methoxy-3-hydroxyphenyl group, 3-isopropyl-4-methoxyphenyl group, 3-fluoro-4-methoxyphenyl group, 3-fluoro-4-hydroxyphenyl group, 3-trifluoromethyl-4-methoxyphenyl group, 3,4-bis(ethoxycarbonyloxy)phenyl group, 3,4,5-tris(ethoxycarbonyloxy)phenyl group, 3,4-dihydroxy-5-(methoxycarbonyl)phenyl group, 3,5-dihydroxy-4-(methoxycarbonyl)phenyl group, 3-methoxy-4-hydroxy-5-(methoxycarbonyl)phenyl group, 3-methoxy-4-hydroxy-5-carboxyphenyl group, 3-methoxy-4-hydroxy-5-nitrophenyl group, 3,4-dihydroxy-5-nitrophenyl group, 3,4,5-trimethoxyphenyl group, 3-methoxy-4-hydroxy-5-(3,4-dihydroxyphenylcarbamoyl)phenyl group, 3-carboxyphenyl group, 4-hydroxy-3-(3,4-dimethoxyphenylcarbamoyl)phenyl group, 4-hydroxy-3-(3,4-dihydroxyphenylcarbamoyl)phenyl group, 4-hydroxy-3-(4-methoxyphenylcarbamoyl)phenyl group, 4-hydroxy-3-(4-hydroxyphenylcarbamoyl)phenyl group, 4-hydroxy-3-(3-methoxyphenylcarbamoyl)phenyl group, 4-hydroxy-3-(3-hydroxyphenylcarbamoyl)phenyl group, 3-(3,4-dimethoxyphenylcarbamoyl)phenyl group, 3-carboxy-4-hydroxyphenyl group, 4-methoxy-3-(3,4-dihydroxybenzoylamino)phenyl group, 3-methoxy-4-hydroxy-5-(3-trifluoromethyl-4-methoxyphenylcarbamoyl)phenyl group, 3-methoxy-4-hydroxy-5-(3-methyl-4-hydroxyphenylcarbamoyl)phenyl group, 3-(4-methoxybenzyloxycarbonyl)-4-hydroxyphenyl group, 3-benzyloxycarbonyl-4-hydroxyphenyl group, 4-methoxy-3-[4-(trifluoromethoxy)benzoylamino]phenyl group, 4-hydroxy-3-[4-(trifluoromethoxy)benzoylamino]phenyl group, 3-[4-(trifluoromethoxy)benzoylamino]phenyl group, 3-(4-methoxybenzoylamino)phenyl group, 3-(4-hydroxybenzoylamino)phenyl group, 3-(3,4-dimethoxybenzoylamino)phenyl group, 3-(3,4-dihydroxybenzoylamino)phenyl group, 4-methoxy-3-(3,4-dimethoxybenzoylamino)phenyl group, 4-hydroxy-3-(3,4-dihydroxybenzoylamino)phenyl group, 4-methoxy-3-(4-methoxybenzoylamino)phenyl group, 4-hydroxy-3-(4-hydroxybenzoylamino)phenyl group, 3-(3,4-dihydroxyphenylcarbamoyl)phenyl group, 3-(4-carboxyphenylcarbamoyl)phenyl group, 3-methyl-4-hydroxy-5-carboxyphenyl group, 4-hydroxy-3-[(2-chloropyridin-5-yl)carbamoyl]phenyl group, 3-methyl-4-hydroxy-5-(3,4-dihydroxyphenylcarbamoyl)phenyl group, 4-hydroxy-3-[2,5-bis(trifluoromethyl)phenylcarbamoyl]phenyl group, 4-hydroxy-3-[3,5-bis(trifluoromethyl)phenylcarbamoyl]phenyl group, 4-hydroxy-3-[3-methoxy-4-(methoxycarbonyl)phenylcarbamoyl]phenyl group, 4-hydroxy-3-[(2-methoxypyridin-5-yl)carbamoyl]phenyl group, 4-hydroxy-3-[(2-hydroxypyridin-5-yl)carbamoyl]phenyl group, indol-3-yl group, 1-(3,4-dibenzyloxybenzyl)indol-3-yl group, 1-(3,4-dimethoxybenzoyl)indol-3-yl group, 1-(3,4-dihydroxybenzyl)indol-3-yl group, 4-hydroxy-3-[3-(trifluoromethoxy)phenylcarbamoyl]phenyl group, 4-hydroxy-3-(3,4-dichlorophenylcarbamoyl)phenyl group, 4-hydroxy-3-[4-(methoxycarbonyl)phenylcarbamoyl]phenyl group, 4-hydroxy-3-[3-(methoxycarbonyl)phenylcarbamoyl]phenyl group, 4-hydroxy-3-(4-carboxyphenylcarbamoyl)phenyl group, 4-hydroxy-3-(3-carboxyphenylcarbamoyl)phenyl group, 4-hydroxy-3-[2-(4-methoxyphenoxy)-5-(trifluoromethyl)phenylcarbamoyl]phenyl group, 4-hydroxy-3-[2-(4-hydroxyphenoxy)-5-(trifluoromethyl)phenylcarbamoyl]phenyl group, 4-hydroxy-3-[2-chloro-5-(trifluoromethyl)phenylcarbamoyl]phenyl group, 4-hydroxy-3-(3-nitrophenylcarbamoyl)phenyl group, 1-(4-methoxycarbonylbenzyl)indol-3-yl group, 3-phenyl-4-hydroxy-5-(benzyloxycarbonyl)phenyl group, 1-(4-methoxycarbonylbenzoyl)indol-3-yl group, 4-hydroxy-3-(3,4-methylenedioxyphenylcarbamoyl)phenyl group, 3-methoxy-4-hydroxy-5-(methoxycarbonyl)phenyl group, 3-phenyl-4-hydroxy-5-carboxyphenyl group, 3,5-bis(methoxymethoxy)-4-(methoxycarbonyl)phenyl group, 4-hydroxy-3-(3,4,5-trimethoxyphenylcarbamoyl)phenyl group, 4-hydroxy-3-(2,3-dimethoxyphenylcarbamoyl)phenyl group, 4-hydroxy-3-(3,4,5-trihydroxyphenylcarbamoyl)phenyl group, 4-hydroxy-3-(2,3-dihydroxyphenylcarbamoyl)phenyl group, 4-hydroxy-3-[3-hydroxy-4-(methoxycarbonyl)phenylcarbamoyl]phenyl group, 3-methoxy-4-hydroxy-5-[3,5-bis(trifluoromethyl)phenylcarbamoyl]phenyl group, 3-methoxy-4-hydroxy-5-[2-chloro-5-(trifluoromethyl)phenylcarbamoyl]phenyl group, 3-methoxy-4-hydroxy-5-(phenylcarbamoyl)phenyl group, 4-hydroxy-3-(2,5-dichlorophenylcarbamoyl)phenyl group, 4-hydroxy-3-[2-fluoro-5-(trifluoromethyl)phenylcarbamoyl]phenyl group, 4-methoxy-3-(3,4-dimethoxyphenyl)phenyl group, 4-methoxy-3-(3,4-dihydroxyphenyl)phenyl group, 4-hydroxy-3-(3,4-dihydroxyphenyl)phenyl group, 3,5-dihydroxy-4-carboxyphenyl group, 4-methoxy-3-(2,6-dimethoxyphenyl)phenyl group, 4-methoxy-3-(4-methoxyphenyl)phenyl group, 4-hydroxy-3-(2,6-dihydroxyphenyl)phenyl group, 4-hydroxy-3-(2-methyl-5-methoxyphenylcarbamoyl)phenyl group, 4-hydroxy-3-[3-methoxy-5-(trifluoromethyl)phenylcarbamoyl]phenyl group, 4-hydroxy-3-(2-methoxy-5-methylphenylcarbamoyl)phenyl group, 4-hydroxy-3-(2-methoxycarbonyl-5-chlorophenylcarbamoyl)phenyl group, 4-hydroxy-3-(2-methoxy-5-chlorophenylcarbamoyl)phenyl group, 4-hydroxy-3-(2-methoxy-5-phenylphenylcarbamoyl)phenyl group, 4-hydroxy-3-(2-methyl-5-hydroxyphenylcarbamoyl)phenyl group, 4-hydroxy-3-(2-hydroxy-5-methylphenylcarbamoyl)phenyl group, 4-hydroxy-3-(2-carboxy-5-chlorophenylcarbamoyl)phenyl group, 4-hydroxy-3-[3-hydroxy-5-(trifluoromethyl)phenylcarbamoyl]phenyl group, 4-hydroxy-3-[(2,2-difluoro-1,3-benzodioxol-4-yl)carbamoyl]phenyl group, 4-methoxy-3-(4-hydroxyphenyl)phenyl group, 4-hydroxy-3-(4-hydroxyphenyl)phenyl group, 3,4-dihydroxy-5-(benzyloxycarbonyl)phenyl group, 3,4-dihydroxy-5-carboxyphenyl group, 4-hydroxy-3-(2,5-difluorophenylcarbamoyl)phenyl group, 4-hydroxy-3-[2-nitro-5-(trifluoromethyl)phenylcarbamoyl]phenyl group, 4-hydroxy-3-[2-methoxy-5-(tert-butyl)phenylcarbamoyl]phenyl group, 4-hydroxy-3-[2-methoxy-5-(trifluoromethyl)phenylcarbamoyl]phenyl group, 3,4-dihydroxy-5-[2-chloro-5-(trifluoromethyl)phenylcarbamoyl]phenyl group, 3,4-dihydroxy-5-[3-(methoxycarbonyl)phenylcarbamoyl]phenyl group, 4-hydroxy-3-(2,5-dimethoxyphenylcarbamoyl)phenyl group, 4-hydroxy-3-(2-bromo-4-isopropylphenylcarbamoyl)phenyl group, 4-hydroxy-3-(2-chloro-5-nitrophenylcarbamoyl)phenyl group, 3,4-dihydroxy-5-(3,4-methylenedioxyphenylcarbamoyl)phenyl group, 3,4-dihydroxy-5-[3-(trifluoromethoxy)phenylcarbamoyl]phenyl group.

Preferred examples of the aromatic group which may be substituted represented by R¹⁰¹ include a phenyl group which is substituted with one to three hydroxy groups (said phenyl group may further be substituted with one or more substituents other than the hydroxy group), and specific examples include a group selected from the following substituent group R-101a-2. More preferred examples include a phenyl group which is substituted with two or three hydroxy groups (said phenyl group may further be substituted with one or more substituents other than the hydroxy group), and specific examples include a group selected from the following substituent group R-101a-3. Further preferred examples include 2,3-dihydroxyphenyl group, 3,4-dihydroxyphenyl group, 3,4,5-trihydroxyphenyl group, 3,4-dihydroxy-5-nitrophenyl group, or 3,4-dihydroxy-5-[3-(trifluoromethoxy)phenylcarbamoyl]phenyl group.
[Substituent Group R-101a-2] 3,5-dibromo-2-hydroxyphenyl group, 2,3-dihydroxyphenyl group, 3,4-dihydroxyphenyl group, 2-hydroxy-3-methylphenyl group, 3-hydroxyphenyl group, 3,4,5-trihydroxyphenyl group, 3-ethoxycarbonyl-4-hydroxyphenyl group, 2,3,4-trihydroxyphenyl group, 3,4-dihydroxy-5-methoxyphenyl group, 5-hydroxy-2-nitrophenyl group, 3,5-dihydroxyphenyl group, 2,4-dihydroxyphenyl group, 2-hydroxy-5-nitrophenyl group, 5-chloro-2-hydroxyphenyl group, 4-hydroxy-3-nitrophenyl group, 3-amino-4-hydroxyphenyl group, 3-hydroxy-4-nitrophenyl group, 4-amino-3-hydroxyphenyl group, 2-hydroxy-5-styrylphenyl group, 3-hydroxy-4-methoxyphenyl group, 3-hydroxy-4,5-methylenedioxyphenyl group, 2-fluoro-3-hydroxyphenyl group, 3-hydroxy-4-fluorophenyl group, 3-hydroxy-4-chlorophenyl group, 2-methoxy-3-hydroxyphenyl group, 3-fluoro-4-hydroxyphenyl group, 3,4-dihydroxy-5-(methoxycarbonyl)phenyl group, 3,5-dihydroxy-4-(methoxycarbonyl)phenyl group, 3-methoxy-4-hydroxy-5-(methoxycarbonyl)phenyl group, 3-methoxy-4-hydroxy-5-carboxyphenyl group, 3-methoxy-4-hydroxy-5-nitrophenyl group, 3,4-dihydroxy-5-nitrophenyl group, 3-methoxy-4-hydroxy-5-(3,4-dihydroxyphenylcarbamoyl)phenyl group, 4-hydroxy-3-(3,4-dimethoxyphenylcarbamoyl)phenyl group, 4-hydroxy-3-(3,4-dihydroxyphenylcarbamoyl)phenyl group, 4-hydroxy-3-(4-methoxyphenylcarbamoyl)phenyl group, 4-hydroxy-3-(4-hydroxyphenylcarbamoyl)phenyl group, 4-hydroxy-3-(3-methoxyphenylcarbamoyl)phenyl group, 4-hydroxy-3-(3-hydroxyphenylcarbamoyl)phenyl group, 3-carboxy-4-hydroxyphenyl group, 3-methoxy-4-hydroxy-5-(3-trifluoromethyl-4-methoxyphenylcarbamoyl)phenyl group, 3-methoxy-4-hydroxy-5-(3-methyl-4-hydroxyphenylcarbamoyl)phenyl group, 3-(4-methoxybenzyloxycarbonyl)-4-hydroxyphenyl group, 3-benzyloxycarbonyl-4-hydroxyphenyl group, 4-hydroxy-3-[4-(trifluoromethoxy)benzoylamino]phenyl group, 4-hydroxy-3-(3,4-dihydroxybenzoylamino)phenyl group, 4-hydroxy-3-(4-hydroxybenzoylamino)phenyl group, 3-methyl-4-hydroxy-5-carboxyphenyl group, 4-hydroxy-3-[(2-chloropyridin-5-yl)carbamoyl]phenyl group, 3-methyl-4-hydroxy-5-(3,4-dihydroxyphenylcarbamoyl)phenyl group, 4-hydroxy-3-[2,5-bis(trifluoromethyl)phenylcarbamoyl]phenyl group, 4-hydroxy-3-[3,5-bis(trifluoromethyl)phenylcarbamoyl]phenyl group, 4-hydroxy-3-[3-methoxy-4-(methoxycarbonyl)phenylcarbamoyl]phenyl group, 4-hydroxy-3-[(2-methoxypyridin-5-yl)carbamoyl]phenyl group, 4-hydroxy-3-[(2-hydroxypyridin-5-yl)carbamoyl]phenyl group, 4-hydroxy-3-[3-(trifluoromethoxy)phenylcarbamoyl]phenyl group, 4-hydroxy-3-(3,4-dichlorophenylcarbamoyl)phenyl group, 4-hydroxy-3-[4-(methoxycarbonyl)phenylcarbamoyl]phenyl group, 4-hydroxy-3-[3-(methoxycarbonyl)phenylcarbamoyl]phenyl group, 4-hydroxy-3-(4-carboxyphenylcarbamoyl)phenyl group, 4-hydroxy-3-(3-carboxyphenylcarbamoyl)phenyl group, 4-hydroxy-3-[2-(4-methoxyphenoxy)-5-(trifluoromethyl)phenylcarbamoyl]phenyl group, 4-hydroxy-3-[2-(4-hydroxyphenoxy)-5-(trifluoromethyl)phenylcarbamoyl]phenyl group, 4-hydroxy-3-[2-chloro-5-(trifluoromethyl)phenylcarbamoyl]phenyl group, 4-hydroxy-3-(3-nitrophenylcarbamoyl)phenyl group, 3-phenyl-4-hydroxy-5-(benzyloxycarbonyl)phenyl group, 4-hydroxy-3-(3,4-methylenedioxyphenylcarbamoyl)phenyl group, 3-methoxy-4-hydroxy-5-(methoxycarbonyl)phenyl group, 3-phenyl-4-hydroxy-5-carboxyphenyl group, 4-hydroxy-3-(3,4,5-trimethoxyphenylcarbamoyl)phenyl group, 4-hydroxy-3-(2,3-dimethoxyphenylcarbamoyl)phenyl group, 4-hydroxy-3-(3,4,5-trihydroxyphenylcarbamoyl)phenyl group, 4-hydroxy-3-(2,3-dihydroxyphenylcarbamoyl)phenyl group, 4-hydroxy-3-[3-hydroxy-4-(methoxycarbonyl)phenylcarbamoyl]phenyl group, 3-methoxy-4-hydroxy-5-[3,5-bis(trifluoromethyl)phenylcarbamoyl]phenyl group, 3-methoxy-4-hydroxy-5-[2-chloro-5-(trifluoromethyl)phenylcarbamoyl]phenyl group, 3-methoxy-4-hydroxy-5-(phenylcarbamoyl)phenyl group, 4-hydroxy-3-(2,5-dichlorophenylcarbamoyl)phenyl group, 4-hydroxy-3-[2-fluoro-5-(trifluoromethyl)phenylcarbamoyl]phenyl group, 4-hydroxy-3-(3,4-dihydroxyphenyl)phenyl group, 3,5-dihydroxy-4-carboxyphenyl group, 4-hydroxy-3-(2,6-dihydroxyphenyl)phenyl group, 4-hydroxy-3-(2-methyl-5-methoxyphenylcarbamoyl)phenyl group, 4-hydroxy-3-[3-methoxy-5-(trifluoromethyl)phenylcarbamoyl]phenyl group, 4-hydroxy-3-(2-methoxy-5-methylphenylcarbamoyl)phenyl group, 4-hydroxy-3-(2-methoxycarbonyl-5-chlorophenylcarbamoyl)phenyl group, 4-hydroxy-3-(2-methoxy-5-chlorophenylcarbamoyl)phenyl group, 4-hydroxy-3-(2-methoxy-5-phenylphenylcarbamoyl)phenyl group, 4-hydroxy-3-(2-methyl-5-hydroxyphenylcarbamoyl)phenyl group, 4-hydroxy-3-(2-hydroxy-5-methylphenylcarbamoyl)phenyl group, 4-hydroxy-3-(2-carboxy-5-chlorophenylcarbamoyl)phenyl group, 4-hydroxy-3-[3-hydroxy-5-(trifluoromethyl)phenylcarbamoyl]phenyl group, 4-hydroxy-3- [(2,2-difluoro-1,3-benzodioxol-4-yl)carbamoyl]phenyl group, 4-hydroxy-3-(4-hydroxyphenyl)phenyl group, 3,4-dihydroxy-5-(benzyloxycarbonyl)phenyl group, 3,4-dihydroxy-5-carboxyphenyl 1 group, 4-hydroxy-3-(2,5-difluorophenylcarbamoyl)phenyl group, 4-hydroxy-3-[2-nitro-5-(trifluoromethyl)phenylcarbamoyl]phenyl group, 4-hydroxy-3-[2-methoxy-5-(tert-butyl)phenylcarbamoyl]phenyl group, 4-hydroxy-3-[2-methoxy-5-(trifluoromethyl)phenylcarbamoyl]phenyl group, 3,4-dihydroxy-5-[2-chloro-5-(trifluoromethyl)phenylcarbamoyl]phenyl group, 3,4-dihydroxy-5-[3-(methoxycarbonyl)phenylcarbamoyl]phenyl group, 4-hydroxy-3-(2,5-dimethoxyphenylcarbamoyl)phenyl group, 4-hydroxy-3-(2-bromo-4-isopropylphenylcarbamoyl)phenyl group, 4-hydroxy-3-(2-chloro-5-nitrophenylcarbamoyl)phenyl group, 3,4-dihydroxy-5-(3,4-methylenedioxyphenylcarbamoyl)phenyl group, 3,4-dihydroxy-5-[3-(trifluoromethoxy)phenylcarbamoyl]phenyl group.
[Substituent Group R-101a-3] 2,3-dihydroxyphenyl group, 3,4-dihydroxyphenyl group, 3,4,5-trihydroxyphenyl group, 2,3,4-trihydroxyphenyl group, 3,4-dihydroxy-5-methoxyphenyl group, 3,5-dihydroxyphenyl group, 2,4-dihydroxyphenyl group, 3,4-dihydroxy-5-(methoxycarbonyl)phenyl group, 3,5-dihydroxy-4-(methoxycarbonyl)phenyl group, 3,4-dihydroxy-5-nitrophenyl group, 3,5-dihydroxy-4-carboxyphenyl group, 3,4-dihydroxy-5-(benzyloxycarbonyl)phenyl group, 3,4-dihydroxy-5-carboxyphenyl group, 3,4-dihydroxy-5-[2-chloro-5-(trifluoromethyl)phenylcarbamoyl]phenyl group, 3,4-dihydroxy-5-[3-(methoxycarbonyl)phenylcarbamoyl]phenyl group, 3,4-dihydroxy-5-(3,4-methylenedioxyphenylcarbamoyl)phenyl group, 3,4-dihydroxy-5-[3-(trifluoromethoxy)phenylcarbamoyl]phenyl group.

Furthermore, preferred examples of the aromatic group which may be substituted represented by R¹⁰¹ include a phenyl group which is substituted with a C₆-C₁₀ aryl-substituted carbamoyl group (said aryl group may be substituted, and said phenyl group may further be substituted with one or more substituents other than the C₆-C₁₀ aryl-substituted carbamoyl group), or a phenyl group which is substituted with a heteroaryl-substituted carbamoyl group (said heteroaryl group may be substituted, and said phenyl group may further be substituted with one or more substituents other than the heteroaryl-substituted carbamoyl group), and specific examples include a group selected from the following substituent group R-101a-4. More preferred examples include a phenyl group which is substituted with a C₆-C₁₀ aryl-substituted carbamoyl group (said aryl group may be substituted) or a heteroaryl-substituted carbamoyl group (said heteroaryl group may be substituted) in the 3-position, and substituted with hydroxy group in the 4-position (said phenyl group may be substituted in the 5-position), and specific examples include a group selected from the following substituent group R-101a-5. Further preferred examples include 3-methoxy-4-hydroxy-5-(3,4-dihydroxyphenylcarbamoyl)phenyl group, 4-hydroxy-3-(3,4-dihydroxyphenylcarbamoyl)phenyl group, 4-hydroxy-3-[2-chloro-5-(trifluoromethyl)phenylcarbamoyl]phenyl group, 4-hydroxy-3-(2,3-dihydroxyphenylcarbamoyl)phenyl group, 3-methyl-4-hydroxy-5-(3,4-dihydroxyphenylcarbamoyl)phenyl group, 4-hydroxy-3-(3,4,5-trihydroxyphenylcarbamoyl)phenyl group, 4-hydroxy-3-(2,5-dichlorophenylcarbamoyl)phenyl group, 4-hydroxy-3-(2-chloro-5-nitrophenylcarbamoyl)phenyl group, or 3,4-dihydroxy-5-[3-(trifluoromethoxy)phenylcarbamoyl]phenyl group.
[Substituent Group R-101a-4]
3-methoxy-4-hydroxy-5-(3,4-dihydroxyphenylcarbamoyl)phenyl group, 4-hydroxy-3-(3,4-dimethoxyphenylcarbamoyl)phenyl group, 4-hydroxy-3-(3,4-dihydroxyphenylcarbamoyl)phenyl group, 4-hydroxy-3-(4-methoxyphenylcarbamoyl)phenyl group, 4-hydroxy-3-(4-hydroxyphenylcarbamoyl)phenyl group, 4-hydroxy-3-(3-methoxyphenylcarbamoyl)phenyl group, 4-hydroxy-3-(3-hydroxyphenylcarbamoyl)phenyl group, 3-(3,4-dimethoxyphenylcarbamoyl)phenyl group, 3-methoxy-4-hydroxy-5-(3-trifluoromethyl-4-methoxyphenylcarbamoyl)phenyl group, 3-methoxy-4-hydroxy-5-(3-methyl-4-hydroxyphenylcarbamoyl)phenyl group, 3-(3,4-dihydroxyphenylcarbamoyl)phenyl group, 3-(4-carboxyphenylcarbamoyl)phenyl 1 group, 4-hydroxy-3-[(2-chloropyridin-5-yl)carbamoyl]phenyl group, 3-methyl-4-hydroxy-5-(3,4-dihydroxyphenylcarbamoyl)phenyl group, 4-hydroxy-3-[2,5-bis(trifluoromethyl)phenylcarbamoyl]phenyl group, 4-hydroxy-3-[3,5-bis(trifluoromethyl)phenylcarbamoyl]phenyl group, 4-hydroxy-3-[3-methoxy-4-(methoxycarbonyl)phenylcarbamoyl]phenyl group, 4-hydroxy-3-[(2-methoxypyridin-5-yl)carbamoyl]phenyl group, 4-hydroxy-3-[(2-hydroxypyridin-5-yl)carbamoyl]phenyl group, 4-hydroxy-3-[3-(trifluoromethoxy)phenylcarbamoyl]phenyl group, 4-hydroxy-3-(3,4-dichlorophenylcarbamoyl)phenyl group, 4-hydroxy-3-[4-(methoxycarbonyl)phenylcarbamoyl]phenyl group, 4-hydroxy-3-[3-(methoxycarbonyl)phenylcarbamoyl]phenyl group, 4-hydroxy-3-(4-carboxyphenylcarbamoyl)phenyl group, 4-hydroxy-3-(3-carboxyphenylcarbamoyl)phenyl group, 4-hydroxy-3-[2-(4-methoxyphenoxy)-5-(trifluoromethyl)phenylcarbamoyl]phenyl group, 4-hydroxy-3-[2-(4-hydroxyphenoxy)-5-(trifluoromethyl)phenylcarbamoyl]phenyl group, 4-hydroxy-3-[2-chloro-5-(trifluoromethyl)phenylcarbamoyl]phenyl group, 4-hydroxy-3-(3-nitrophenylcarbamoyl)phenyl group, 4-hydroxy-3-(3,4-methylenedioxyphenylcarbamoyl)phenyl group, 4-hydroxy-3-(3,4,5-trimethoxyphenylcarbamoyl)phenyl group, 4-hydroxy-3-(2,3-dimethoxyphenylcarbamoyl)phenyl group, 4-hydroxy-3-(3,4,5-trihydroxyphenylcarbamoyl)phenyl group, 4-hydroxy-3-(2,3-dihydroxyphenylcarbamoyl)phenyl group, 4-hydroxy-3-[3-hydroxy-4-(methoxycarbonyl)phenylcarbamoyl]phenyl group, 3-methoxy-4-hydroxy-5-[3,5-bis(trifluoromethyl)phenylcarbamoyl]phenyl group, 3-methoxy-4-hydroxy-5-[2-chloro-5-(trifluoromethyl)phenylcarbamoyl]phenyl group, 3-methoxy-4-hydroxy-5-(phenylcarbamoyl)phenyl-group, 4-hydroxy-3-(2,5-dichlorophenylcarbamoyl)phenyl group, 4-hydroxy-3-[2-fluoro-5-(trifluoromethyl)phenylcarbamoyl]phenyl group, 4-hydroxy-3-(2-methyl-5-methoxyphenylcarbamoyl)phenyl group, 4-hydroxy-3-[3-methoxy-5-(trifluoromethyl)phenylcarbamoyl]phenyl group, 4-hydroxy-3-(2-methoxy-5-methylphenylcarbamoyl)phenyl group, 4-hydroxy-3-(2-methoxycarbonyl-5-chlorophenylcarbamoyl)phenyl group, 4-hydroxy-3-(2-methoxy-5-chlorophenylcarbamoyl)phenyl group, 4-hydroxy-3-(2-methoxy-5-phenylphenylcarbamoyl)phenyl group, 4-hydroxy-3-(2-methyl-5-hydroxyphenylcarbamoyl)phenyl group, 4-hydroxy-3-(2-hydroxy-5-methylphenylcarbamoyl)phenyl group, 4-hydroxy-3-(2-carboxy-5-chlorophenylcarbamoyl)phenyl group, 4-hydroxy-3-[3-hydroxy-5-(trifluoromethyl)phenylcarbamoyl]phenyl group, 4-hydroxy-3- [(2,2-difluoro-1,3-benzodioxol-4-yl)carbamoyl]phenyl group, 4-hydroxy-3-(2,5-difluorophenylcarbamoyl)phenyl group, 4-hydroxy-3-[2-nitro-5-(trifluoromethyl)phenylcarbamoyl]phenyl group, 4-hydroxy-3-[2-methoxy-5-(tert-butyl)phenylcarbamoyl]phenyl group, 4-hydroxy-3-[2-methoxy-5-(trifluoromethyl)phenylcarbamoyl]phenyl group, 3,4-dihydroxy-5-[2-chloro-5-(trifluoromethyl)phenylcarbamoyl]phenyl group, 3,4-dihydroxy-5-[3-(methoxycarbonyl)phenylcarbamoyl]phenyl group, 4-hydroxy-3-(2,5-dimethoxyphenylcarbamoyl)phenyl group, 4-hydroxy-3-(2-bromo-4-isopropylphenylcarbamoyl)phenyl group, 4-hydroxy-3-(2-chloro-5-nitrophenylcarbamoyl)phenyl group, 3,4-dihydroxy-5-(3,4-methylenedioxyphenylcarbamoyl)phenyl group, 3,4-dihydroxy-5-[3-(trifluoromethoxy)phenylcarbamoyl]phenyl group.
[Substituent Group R-101a-5]
3-methoxy-4-hydroxy-5-(3,4-dihydroxyphenylcarbamoyl)phenyl group, 4-hydroxy-3-(3,4-dimethoxyphenylcarbamoyl)phenyl group, 4-hydroxy-3-(3,4-dihydroxyphenylcarbamoyl)phenyl group, 4-hydroxy-3-(4-methoxyphenylcarbamoyl)phenyl group, 4-hydroxy-3-(4-hydroxyphenylcarbamoyl)phenyl group, 4-hydroxy-3-(3-methoxyphenylcarbamoyl)phenyl group, 4-hydroxy-3-(3-hydroxyphenylcarbamoyl)phenyl group, 3-methoxy-4-hydroxy-5-(3-trifluoromethyl-4-methoxyphenylcarbamoyl)phenyl group, 3-methoxy-4-hydroxy-5-(3-methyl-4-hydroxyphenylcarbamoyl)phenyl group, 4-hydroxy-3-[(2-chloropyridin-5-yl)carbamoyl]phenyl group, 3-methyl-4-hydroxy-5-(3,4-dihydroxyphenylcarbamoyl)phenyl group, 4-hydroxy-3-[2,5-bis(trifluoromethyl)phenylcarbamoyl]phenyl group, 4-hydroxy-3-[3,5-bis(trifluoromethyl)phenylcarbamoyl]phenyl group, 4-hydroxy-3-[3-methoxy-4-(methoxycarbonyl)phenylcarbamoyl]phenyl group, 4-hydroxy-3-[(2-methoxypyridin-5-yl)carbamoyl]phenyl group, 4-hydroxy-3-[(2-hydroxypyridin-5-yl)carbamoyl]phenyl group, 4-hydroxy-3-[3-(trifluoromethoxy)phenylcarbamoyl]phenyl group, 4-hydroxy-3-(3,4-dichlorophenylcarbamoyl)phenyl group, 4-hydroxy-3-[4-(methoxycarbonyl)phenylcarbamoyl]phenyl group, 4-hydroxy-3-[3-(methoxycarbonyl)phenylcarbamoyl]phenyl group, 4-hydroxy-3-(4-carboxyphenylcarbamoyl)phenyl group, 4-hydroxy-3-(3-carboxyphenylcarbamoyl)phenyl group, 4-hydroxy-3-[2-(4-methoxyphenoxy)-5-(trifluoromethyl)phenylcarbamoyl]phenyl group, 4-hydroxy-3-[2-(4-hydroxyphenoxy)-5-(trifluoromethyl)phenylcarbamoyl]phenyl group, 4-hydroxy-3-[2-chloro-5-(trifluoromethyl)phenylcarbamoyl]phenyl group, 4-hydroxy-3-(3-nitrophenylcarbamoyl)phenyl group, 4-hydroxy-3-(3,4-methylenedioxyphenylcarbamoyl)phenyl group, 4-hydroxy-3-(3,4,5-trimethoxyphenylcarbamoyl)phenyl group, 4-hydroxy-3-(2,3-dimethoxyphenylcarbamoyl)phenyl group, 4-hydroxy-3-(3,4,5-trihydroxyphenylcarbamoyl)phenyl group, 4-hydroxy-3-(2,3-dihydroxyphenylcarbamoyl)phenyl group, 4-hydroxy-3-[3-hydroxy-4-(methoxycarbonyl)phenylcarbamoyl]phenyl group, 3-methoxy-4-hydroxy-5-[3,5-bis(trifluoromethyl)phenylcarbamoyl]phenyl group, 3-methoxy-4-hydroxy-5-[2-chloro-5-(trifluoromethyl)phenylcarbamoyl]phenyl group, 3-methoxy-4-hydroxy-5-(phenylcarbamoyl)phenyl group, 4-hydroxy-3-(2,5-dichlorophenylcarbamoyl)phenyl group, 4-hydroxy-3-[2-fluoro-5-(trifluoromethyl)phenylcarbamoyl]phenyl group, 4-hydroxy-3-(2-methyl-5-methoxyphenylcarbamoyl)phenyl group, 4-hydroxy-3-[3-methoxy-5-(trifluoromethyl)phenylcarbamoyl]phenyl group, 4-hydroxy-3-(2-methoxy-5-methylphenylcarbamoyl)phenyl group, 4-hydroxy-3-(2-methoxycarbonyl-5-chlorophenylcarbamoyl)phenyl group, 4-hydroxy-3-(2-methoxy-5-chlorophenylcarbamoyl)phenyl group, 4-hydroxy-3-(2-methoxy-5-phenylphenylcarbamoyl)phenyl group, 4-hydroxy-3-(2-methyl-5-hydroxyphenylcarbamoyl)phenyl group, 4-hydroxy-3-(2-hydroxy-5-methylphenylcarbamoyl)phenyl group, 4-hydroxy-3-(2-carboxy-5-chlorophenylcarbamoyl)phenyl group, 4-hydroxy-3-[3-hydroxy-5-(trifluoromethyl)phenylcarbamoyl]phenyl group, 4-hydroxy-3-[(2,2-difluoro-1,3-benzodioxol-4-yl)carbamoyl]phenyl group, 4-hydroxy-3-(2,5-difluorophenylcarbamoyl)phenyl group, 4-hydroxy-3-[2-nitro-5-(trifluoromethyl)phenylcarbamoyl]phenyl group, 4-hydroxy-3-[2-methoxy-5-(tert-butyl)phenylcarbamoyl]phenyl group, 4-hydroxy-3-[2-methoxy-5-(trifluoromethyl)phenylcarbamoyl]phenyl group, 3,4-dihydroxy-5-[2-chloro-5-(trifluoromethyl)phenylcarbamoyl]phenyl group, 3,4-dihydroxy-5-[3-(methoxycarbonyl)phenylcarbamoyl]phenyl group, 4-hydroxy-3-(2,5-dimethoxyphenylcarbamoyl)phenyl group, 4-hydroxy-3-(2-bromo-4-isopropylphenylcarbamoyl)phenyl group, 4-hydroxy-3-(2-chloro-5-nitrophenylcarbamoyl)phenyl group, 3,4-dihydroxy-5-(3,4-methylenedioxyphenylcarbamoyl)phenyl group, 3,4-dihydroxy-5-[3-(trifluoromethoxy)phenylcarbamoyl]phenyl group.

Furthermore, preferred examples of the aromatic group which may be substituted represented by R¹⁰¹ include a phenyl group which is substituted with carboxy group (said phenyl group may further be substituted with one or more substituents other than the carboxy group). More preferred examples include a group selected from the following substituent group R-101a-6. Further preferred examples include 3-carboxyphenyl group or 3-carboxy-4-hydroxyphenyl group.
[Substituent Group R-101a-6] 3-methoxy-4-hydroxy-5-carboxyphenyl group, 3-carboxyphenyl group, 3-carboxy-4-hydroxyphenyl group, 3-methyl-4-hydroxy-5-carboxyphenyl group, 3-phenyl-4-hydroxy-5-carboxyphenyl 1 group, 3,5-dihydroxy-4-carboxyphenyl group, 3,4-dihydroxy-5-carboxyphenyl group.

When W¹⁰¹ is -C(R³⁰¹)=N-, preferred examples of the aromatic group represented by R¹⁰¹ include a C₆-C₁₄ aryl group or a 5-membered heteroaryl group. More preferred examples include phenyl group, naphthyl group, anthryl group, furyl group, thienyl group, or imidazolyl group, and most preferred examples include phenyl group.

The aromatic group represented by R¹⁰¹ may be substituted. Preferred examples of said substituent include a halogen atom; nitro group; a C₁-C₆ alkyl group (more preferably, methyl group and tert-butyl group); a C₆-C₁₀ aryl group (said aryl group may be substituted, and more preferred examples include 3-methoxycarbonylphenyl group, 3,5-dichlorophenyl group, 4-carboxyphenyl group, 2-chloro-5-carboxyphenyl group, 2-hydroxy-5-nitro group, 3,5-bis(trifluoromethyl)phenyl group, 3-chlorophenyl group, 3-chloro-4-methylphenyl group, 2-chlorophenyl group, 2-methyl-3-chlorophenyl group, 3-fluorophenyl group, 3-nitrophenyl group, and phenyl group); hydroxy group; a C₁-C₆ alkoxy group (said alkoxy group may be substituted, and more preferred examples include methoxy group, ethoxy group, carboxymethoxy group, and benzylcarbamoylmethoxy group); a C₂-C₆ alkenyloxy group (more preferably, allyloxy group); a heterocyclic carbonyloxy group (more preferably, thenoyloxy group); carboxy group; a C₁-C₆ alkoxycarbonyl group (more preferably, methoxycarbonyl group); a C₁-C₆ alkylsulfanyl group (said alkylsulfanyl group may be substituted, and more preferred examples include 2-dimethylaminoethylsulfanyl group); a C₁-C₄ alkylenedioxy group (more preferably, methylenedioxy group); a C₂-C₆ alkenyl group (said alkenyl group may be substituted, and more preferred examples include styryl group and 4-phenoxystyryl group); a C₆-C₁₀ aryloxy group (said aryloxy group may be substituted, and more preferred examples include phenoxy group, 4-bromophenoxy group, and 3,4-dimethoxyphenoxy group); a tri-C₁-C₆ alkylsilyl group (more preferably, tert-butyldimethylsilylgroup); and a C₂-C₁₂ dialkylamino group (more preferably, diethylamino group). Further preferred examples include hydroxy group, a C₂-C₆ alkenyl group (said alkenyl group may be substituted), or carboxy group. It is preferable that the aromatic group represented by R¹⁰¹ is substituted with one to three substituents independently selected from the aforementioned substituents.

Examples of the aromatic group which may be substituted represented by R¹⁰¹ include, for example, a group selected from the following substituent group R-101b-1. [Substituent Group R-101b-1] 3,5-dibromo-2-hydroxyphenyl group, 5-bromo-2-hydroxyphenyl group, 3-ethoxy-4-(thenoyloxy)phenyl group, 2,3-dihydroxyphenyl group, 3-carboxy-4-hydroxyphenyl group, 3,4-dihydroxyphenyl group, 2,4-dihydroxyphenyl group, 3-hydroxyphenyl group, 3,4,5-trihydroxyphenyl group, 3,4-dihydroxy-5-methoxyphenyl group, 2,4,5-trihydroxyphenyl group, 2,3,4-trihydroxyphenyl group, 4-carboxymethoxy-3-ethoxyphenyl group, 5-(3-methoxycarbonylphenyl)furan-2-yl group, 5-(4-carboxyphenyl)furan-2-yl group, 5-(3,5-dichlorophenyl)furan-2-yl group, 5-(5-carboxy-2-chlorophenyl)furan-2-yl group, 4-bromophenyl group, 5-(2-hydroxy-5-nitrophenyl)furan-2-yl group, 4-allyloxy-3-methoxyphenyl group, 4-benzyloxy-3-methoxyphenyl group, 4-chlorophenyl group, phenyl group, 2-benzyloxy-5-bromophenyl group, 4-benzylcarbamoylmethoxy-3-bromo-5-methoxyphenyl group, 5-[3,5-bis(trifluoromethyl)phenyl]furan-2-yl group, 5-(2-dimethylaminoethylsulfanyl)thiophen-2-yl group, 5-(3,5-dichlorophenyl)thiophen-2-yl group, 5-(3-chlorophenyl)furan-2-yl group, 5-(3-chloro-4-methylphenyl)furan-2-yl group, 5-(2-chlorophenyl)furan-2-yl group, 5-(3-chloro-2-methylphenyl)furan-2-yl group, 5-(3-fluorophenyl)furan-2-yl group, 5-(3-nitrophenyl)furan-2-yl group, 5-bromofuran-2-yl group, 3,4-methylenedioxyphenyl group, 3,4-dichlorophenyl group, 3-methoxycarbonylphenyl group, 4-chloro-3-nitrophenyl group, 3-carboxyphenyl group, 3-methoxy-4-methylphenyl group, 4-methoxycarbonylphenyl group, 2,4,5-trimethoxyphenyl group, 4-hydroxyphenyl group, 2-hydroxy-5-styrylphenyl group, 2-hydroxy-5-methoxyphenyl group, 2,5-dihydroxyphenyl group, 2-hydroxyphenyl group, 4-phenoxyphenyl group, 2-hydroxy-3-methoxy-5-bromophenyl group, 2,3-dihydroxy-5-bromophenyl group, 2-methoxy-5-styrylphenyl group, 4-styrylphenyl group, 3-methoxy-4-hydroxy-5-bromophenyl group, 3,4-dihydroxy-5-bromophenyl group, 3,4-dimethoxy-6-styrylphenyl group, 2-(tert-butyldimethylsilyloxy)-5-styrylphenyl group, 2-hydroxynaphthalen-1-yl-group, 9-anthryl group, 2-hydroxy-5-(tert-butyl)phenyl group, 2-hydroxy-3-methoxy-6-bromophenyl group, 2,3-dihydroxy-6-bromophenyl group, 2,6-dihydroxyphenyl group, 2-bromo-4,5-dihydroxyphenyl group, 2-nitro-5-hydroxyphenyl group, 3-methoxy-4-hydroxy-5-carboxyphenyl group, 2-hydroxy-5-diethylaminophenyl group, 3-methoxy-4-hydroxy-5-nitrophenyl group, 2-nitro-5-(4-bromophenoxy)phenyl group, 2-nitro-5-(3,4-dimethoxyphenoxy)phenyl group, 3,4-dichlorophenyl group, 2-hydroxy-5-(4-phenoxystyryl)phenyl group, biphenyl-4-yl group (4-phenylphenyl group), imidazol-4-yl group.

Preferred examples of the aromatic group which may be substituted represented by R¹⁰¹ include a phenyl group which is substituted with one to three hydroxy groups (said phenyl group may further be substituted with one or more substituents other than the hydroxy group), and specific examples include a group selected from the following substituent group R-101b-2. More preferred examples include a phenyl group which is substituted with two or three hydroxy groups (said phenyl group may further be substituted with one or more substituents other than the hydroxy group), and specific examples include a group selected from the following substituent group R-101b-3. Further preferred examples include 2,4,5-trihydroxyphenyl group.
[Substituent Group R-101b-2] 3,5-dibromo-2-hydroxyphenyl group, 5-bromo-2-hydroxyphenyl group, 2,3-dihydroxyphenyl group, 3-carboxy-4-hydroxyphenyl group, 3,4-dihydroxyphenyl group, 2,4-dihydroxyphenyl group, 3-hydroxyphenyl group, 3,4,5-trihydroxyphenyl group, 3,4-dihydroxy-5-methoxyphenyl group, 2,4,5-trihydroxyphenyl group, 2,3,4-trihydroxyphenyl group, 4-hydroxyphenyl group, 2-hydroxy-5-styrylphenyl group, 2-hydroxy-5-methoxyphenyl group, 2,5-dihydroxyphenyl group, 2-hydroxyphenyl group, 2-hydroxy-3-methoxy-5-bromophenyl group, 2,3-dihydroxy-5-bromophenyl group, 3-methoxy-4-hydroxy-5-bromophenyl group, 3,4-dihydroxy-5-bromophenyl group, 2-hydroxy-5-(tert-butyl)phenyl group, 2-hydroxy-3-methoxy-6-bromophenyl group, 2,3-dihydroxy-6-bromophenyl group, 2,6-dihydroxyphenyl group, 2-bromo-4,5-dihydroxyphenyl group, 2-nitro-5-hydroxyphenyl group, 3-methoxy-4-hydroxy-5-carboxyphenyl group, 2-hydroxy-5-diethylaminophenyl group, 3-methoxy-4-hydroxy-5-nitrophenyl group, 2-hydroxy-5-(4-phenoxystyryl)phenyl group.
[Substituent Group R-101b-3] 2,3-dihydroxyphenyl group, 3,4-dihydroxyphenyl group, 2,4-dihydroxyphenyl group, 3,4,5-trihydroxyphenyl group, 3,4-dihydroxy-5-methoxyphenyl group, 2,4,5-trihydroxyphenyl group, 2,3,4-trihydroxyphenyl group, 2,5-dihydroxyphenyl group, 2,3-dihydroxy-5-bromophenyl group, 3,4-dihydroxy-5-bromophenyl group, 2,3-dihydroxy-6-bromophenyl group, 2,6-dihydroxyphenyl group, 2-bromo-4,5-dihydroxyphenyl group.

Furthermore, preferred examples of the aromatic group which may be substituted represented by R¹⁰¹ include a phenyl group which is substituted with a C₂-C₆ alkenyl group (said alkenyl group may be substituted, and said phenyl group may further be substituted with one or more substituents other than the C₂-C₆ alkenyl group), and specific examples include a group selected from the following substituent group R-101b-4. Further preferred examples include 2-hydroxy-5-styrylphenyl group or 4-styrylphenyl group.
[Substituent Group R-101b-4] 2-hydroxy-5-styrylphenyl group, 2-methoxy-5-styrylphenyl group, 4-styrylphenyl group, 3,4-dimethoxy-6-styrylphenyl group, 2-hydroxy-5-(4-phenoxystyryl)phenyl group.

Furthermore, preferred examples of the aromatic group which may be substituted represented by R¹⁰¹ include a phenyl group which is substituted with carboxy group (said phenyl group may further be substituted with one or more substituents other than the carboxy group). More preferred examples include 3-carboxy-4-hydroxyphenyl group, 3-carboxyphenyl group, or 3-methoxy-4-hydroxy-5-carboxyphenyl group.

In the general formula (II), R²⁰¹ represents an aromatic group which may be substituted.

When W¹⁰¹ is -X¹⁰¹-C(=Y¹⁰¹)-, preferred examples of the aromatic group represented by R²⁰¹ include a C₆-C₁₀ aryl group or a 6-membered heteroaryl group. More preferred examples include phenyl group or pyridyl group, and most preferred examples include phenyl group.

The aromatic group represented by R²⁰¹ may be substituted. Preferred examples of said substituent include a halogen atom; nitro group; a C₁-C₆ alkyl group (more preferably, methyl group and tert-butyl group); a C₂-C₈ alkenyl group (said alkenyl group may be substituted, and more preferred examples include styryl group and 3,5-bis(trifluoromethyl)styryl group); a C₆-C₁₀ aryl group (more preferably, phenyl group); a halogenated C₁-C₆ alkyl group (more preferably, trifluoromethyl group); hydroxy group; a C₁-C₆ alkoxy group (more preferably, methoxy group); a C₆-C₁₀ aryloxy group (more preferably, phenoxy group); carboxy group; a C₂-C₇ alkoxycarbonyl group (more preferably, methoxycarbonyl group); a carbamoyl-substituted C₁-C₆ alkyl group (said carbamoyl group may be substituted, and more preferred examples include benzylcarbamoylmethyl group); a C₇-C₁₁ aroylamino group (said aroyl group may be substituted, and more preferred examples include benzoylamino group, 3,4-dichlorobenzoylamino group, and 4-phenoxybenzoylamino group); amino group; a C₆-C₁₀ aryl-substituted carbamoyl group (more preferably, phenylcarbamoyl group); a C₆-C₁₀ arylamino group (more preferably, phenylamino group); a C₂-C₇ alkanoylamino group (said alkanoyl group may be substituted, and more preferred examples include phenoxyacetylamino group); and a C₈-C₁₃ aralkylcarbonylamino group (said aralkyl group may be substituted, and more preferred examples include (3-phenoxyphenyl)acetylamino group, phenylacetylamino group, 3-phenylpropionylamino group, and 3,5-bis(trifluoromethyl)phenylacetylamino group). Further preferred examples include a halogen atom, a C₂-C₈ alkenyl group (said alkenyl group may be substituted), a C₇-C₁₁ aroylamino group (said aroyl group may be substituted), a halogenated C₁-C₆ alkyl group, a C₈-C₁₃ aralkylcarbonylamino group (said aralkyl group may be substituted), a C₂-C₇ alkoxycarbonyl group, or carboxy group. It is preferable that the aromatic group represented by R²⁰¹ is substituted with one to three substituents independently selected from the aforementioned substituents.

Examples of the aromatic group which may be substituted represented by R²⁰¹ include, for example, a group selected from the following substituent group R-201a-1. [Substituent Group R-201a-1] phenyl group, 3,5-bis(trifluoromethyl)phenyl group, 3,5-dimethoxyphenyl group, 3,4-dichlorophenyl group, 3,5-dichlorophenyl group, 3,5-di(tert-butyl)-4-hydroxyphenyl group, 2,4,5-trimethoxyphenyl group, 3-phenoxyphenyl group, 2,3-dichlorophenyl group, biphenyl-4-yl group (4-phenylphenyl group), 2,4-dichlorophenyl group, 3,5-difluorophenyl group, 3,5-dimethylphenyl group, 3-chlorophenyl group, 4-chlorophenyl group, 3-bromophenyl group, 4-bromophenyl group, 3-(trifluoromethyl)phenyl group, 4-(trifluoromethyl)phenyl group, 3,5-dibromophenyl group, 3-nitrophenyl group, 4-nitrophenyl group, 4-styrylphenyl group, 4-[3,5-bis(trifluoromethyl)styryl]phenyl group, 4-phenoxyphenyl group, 3-fluorophenyl group, 4-methoxyphenyl group, 4-chloro-3-methoxycarbonylphenyl group, 3-carboxy-4-chlorophenyl group, 2-hydroxyphenyl group, 2,5-dimethoxyphenyl group, 3,4-dimethoxyphenyl group, 3,4-dihydroxyphenyl group, 3,4,5-trihydroxyphenyl group, 4-benzylcarbamoylmethylphenyl group, 4-benzoylaminophenyl group, 4-aminophenyl group, 4-(3,4-dichlorobenzoylamino)phenyl group, 4-(phenylcarbamoyl)phenyl group, 4-(phenylamino)phenyl group, 5,6-dichloropyridin-3-yl group, 2,5-bis(trifluoromethyl)phenyl group, 4-(phenoxyacetylamino)phenyl group, 4-[(3-phenoxyphenyl)acetylamino]phenyl group, 4-(phenylacetylamino)phenyl group, 4-(3-phenylpropionylamino)phenyl group, 4-(4-phenoxybenzoylamino)phenyl group, 4-[3,5-bis(trifluoromethyl)phenylacetylamino]phenyl group, 3-(phenylamino)phenyl group, 3-[(3-phenoxyphenyl)acetylamino]phenyl group, 4-methoxycarbonylphenyl group, 4-carboxyphenyl group.

Preferred examples of the aromatic group which may be substituted represented by R²⁰¹ include a phenyl group which is substituted with a C₂-C₈ alkenyl group (said alkenyl group may be substituted, and said phenyl group may further be substituted with one or more substituents other than the C₂-C₈ alkenyl group), a phenyl group which is substituted with one or two halogen atoms (said phenyl group may further be substituted with one or more substituents other than the halogen atom), a phenyl group which is substituted with a C₇-C₁₁ aroylamino group (said aroyl group may be substituted, and said phenyl group may further be substituted with one or more substituents other than the C₇-C₁₁ aroylamino group), a phenyl group which is substituted with one or two halogenated C₁-C₆ alkyl groups (said phenyl group may further be substituted with one or more substituents other than the halogenated C₁-C₆ alkyl group), a phenyl group which is substituted with a C₈-C₁₃ aralkylcarbonylamino group (said aralkyl group may be substituted, and said phenyl group may further be substituted with one or more substituents other than the C₈-C₁₃ aralkylcarbonylamino group), or a phenyl group which is substituted with a C₂-C₇ alkoxycarbonyl group (said phenyl group may further be substituted with one or more substituents other than the C₂-C₇ alkoxycarbonyl group), and specific examples include a group selected from the following substituent group R-201a-2. More preferred examples include 4-styrylphenyl group, 2,4-dichlorophenyl group, 3,4-dichlorophenyl group, 4-(4-phenoxybenzoylamino)phenyl group, 2,3-dichlorophenyl group, 4-bromophenyl group, 4-(trifluoromethyl)phenyl group, 4-[(3-phenoxyphenyl)acetylamino]phenyl group, 4-[3,5-bis(trifluoromethyl)phenylacetylamino]phenyl group, 3-[(3-phenoxyphenyl)acetylamino]phenyl group, 3,5-bis(trifluoromethyl)phenyl group, or 4-methoxycarbonylphenyl group.
[Substituent Group R-201a-2] 3,4-dichlorophenyl group, 3,5-dichlorophenyl group, 2,3-dichlorophenyl group, 2,4-dichlorophenyl group, 3,5-difluorophenyl group, 3-chlorophenyl group, 4-chlorophenyl group, 3-bromophenyl group, 4-bromophenyl group, 3,5-dibromophenyl group, 4-styrylphenyl group, 4-[3,5-bis(trifluoromethyl)styryl]phenyl group, 3-fluorophenyl group, 4-chloro-3-methoxycarbonylphenyl group, 3-carboxy-4-chlorophenyl group, 4-benzoylaminophenyl group, 4-(3,4-dichlorobenzoylamino)phenyl group, 4-(4-phenoxybenzoylamino)phenyl group, 3,5-bis(trifluoromethyl)phenyl group, 3-(trifluoromethyl)phenyl group, 4-(trifluoromethyl)phenyl group, 2,5-bis(trifluoromethyl)phenyl group, 4-[(3-phenoxyphenyl)acetylamino]phenyl group, 4-(phenylacetylamino)phenyl group, 4-(3-phenylpropionylamino)phenyl group, 4-[3,5-bis(trifluoromethyl)phenylacetylamino]phenyl group, 3-[(3-phenoxyphenyl)acetylamino]phenyl group, 4-methoxycarbonylphenyl group.

Furthermore, preferred examples of the aromatic group which may be substituted represented by R²⁰¹ include a phenyl group which is substituted with carboxy group (said phenyl group may further be substituted with one or more substituents other than the carboxy group). More preferred examples include 3-carboxy-4-chlorophenyl group, or 4-carboxyphenyl group.

When W¹⁰¹ is -C(R³⁰¹)=N-, preferred examples of the aromatic group represented by R²⁰¹ include a C₆-C₁₀ aryl group or a 6-membered heteroaryl group. More preferred examples include phenyl group or pyridyl group, and most preferred examples include phenyl group.

The aromatic group represented by R²⁰¹ may be substituted. Preferred examples of said substituent include a halogen atom; nitro group; a C₁-C₆ alkyl group (more preferably, methyl group); a halogenated C₁-C₆ alkyl group (more preferably, trifluoromethyl group); a C₁-C₆ alkoxy group (more preferably, methoxy group); carboxy group; a C₂-C₇ alkoxycarbonyl group (more preferably, methoxycarbonyl group); sulfamoyl group; and a C₂-C₆ alkenyl group (said alkenyl group may be substituted, and more preferred examples include 2-(pyridin-2-yl)vinyl group). Further preferred examples include a halogen atom, nitro group, or carboxy group. It is preferable that the aromatic group represented by R²⁰¹ is substituted with one or two substituents independently selected from the aforementioned substituents.

Examples of the aromatic group which may be substituted represented by R²⁰¹ include, for example, a group selected from the following substituent group R-201b-1. [Substituent Group R-201b-1] phenyl group, 3,4-dichlorophenyl group, 3-carboxyphenyl group, 3,5-dichlorophenyl group, 3-methoxycarbonylphenyl group, 3-(trifluoromethyl)phenyl group, 4-methylphenyl group, 4-nitrophenyl group, 3-chlorophenyl group, 3,5-bis(trifluoromethyl)phenyl group, 3-chloro-4-methylphenyl group, 2,3-dimethylphenyl group, 3-methylphenyl group, 3-methoxyphenyl group, 3-nitrophenyl group, 4-fluorophenyl group, 4-chlorophenyl group, 3-carboxy-4-chlorophenyl group, 3-ethoxycarbonylphenyl group, 4-sulfamoylphenyl group, 6-chloropyridin-3-yl group, 2,5-dichlorophenyl group, 4-carboxyphenyl group, 3-chloro-4-fluorophenyl group, 4-[2-(pyridin-2-yl)vinyl]phenyl group.

Preferred examples of the aromatic group which may be substituted represented by R²⁰¹ include a phenyl group which is substituted with nitro group (said phenyl group may further be substituted with one or more substituents other than the nitro group), or a phenyl group which is substituted with one or two halogen atoms (said phenyl group may further be substituted with one or more substituents other than the halogen atom), and specific examples include a group selected from the following substituent group R-201b-2. More preferred examples include 4-nitrophenyl group, 3,4-dichlorophenyl group, 3,5-dichlorophenyl group, or 3-chloro-4-fluorophenyl group.
[Substituent Group R-201b-2] 3,4-dichlorophenyl group, 3,5-dichlorophenyl group, 4-nitrophenyl group, 3-chlorophenyl group, 3-chloro-4-methylphenyl group, 3-nitrophenyl group, 4-fluorophenyl group, 4-chlorophenyl group, 3-carboxy-4-chlorophenyl group, 2,5-dichlorophenyl group, 3-chloro-4-fluorophenyl group.

Furthermore, preferred examples of the aromatic group which may be substituted represented by R²⁰¹ include a phenyl group which is substituted with carboxy group (said phenyl group may further be substituted with one or more substituents other than the carboxy group). More preferred examples include 3-carboxyphenyl group, 3-carboxy-4-chlorophenyl group, or 4-carboxyphenyl group. Further preferred examples include 4-carboxyphenyl group.

In the general formula (II), Z¹⁰¹ represents a single bond or a connecting group wherein a number of atoms in a main chain is 1 to 3 (said connecting group may be substituted). The "connecting group wherein a number of atoms of main chain is 1 to 3" represented by Z¹⁰¹ means connecting groups wherein 1 to 3 atoms in a main chain link together between the nitrogen atom and R²⁰¹ constituting the 5-membered hetero ring. The number of atoms of the main chain is counted so as to minimize the number of connecting atoms existing between said nitrogen atom and R²⁰¹, regardless of the presence or absence of hetero atom(s). The connecting group represented by Z¹⁰¹ may be substituted.

When W¹⁰¹ is -X¹⁰¹-C(=Y¹⁰¹)-, preferred examples of Z¹⁰¹ include a single bond, methylene group, ethylene group, -CH₂CO- group, or -CH₂CONH- group. More preferred examples include methylene group.

When W¹⁰¹ is -C(R³⁰¹)=N⁻, preferred examples of Z¹⁰¹ include a single bond or methylene group. More preferred examples include a single bond.

As the compound represented by the aforementioned general formula (II), the following compounds are excluded.
- the compound wherein R¹⁰¹ is 3,5-dibromo-2-hydroxyphenyl group, R²⁰¹ is phenyl group, W¹⁰¹ is -X¹⁰¹-C(=y¹⁰¹)-, X¹⁰¹ is sulfur atom, Y¹⁰¹ is oxygen atom, Z¹⁰¹ is a single bond;
- the compound wherein R¹⁰¹ is 5-(3-carboxyphenyl)furan-2-yl group, R²⁰¹ is 3-fluorophenyl group, W¹⁰¹ is -X¹⁰¹-C(=Y¹⁰¹)-, X¹⁰¹ is sulfur atom, Y¹⁰¹ is sulfur atom, Z¹⁰¹ is a single bond;
- the compound wherein R¹⁰¹ is 3,5-dibromo-2-hydroxyphenyl group, R²⁰¹ is 4-chlorophenyl group, W¹⁰¹ is -X¹⁰¹-C(=Y¹⁰¹)-, X¹⁰¹ is NH, Y¹⁰¹ is sulfur atom, Z¹⁰¹ is a single bond;
- the compound wherein R¹⁰¹ is 5-(3-carboxyphenyl)furan-2-yl group, R²⁰¹ is 4-methoxyphenyl group, W¹⁰¹ is -X¹⁰¹-C(=Y¹⁰¹)-, X¹⁰¹ is sulfur atom, Y¹⁰¹ is sulfur atom, Z¹⁰¹ is methylene group;
- the compound wherein R¹⁰¹ is 5-chloro-2-hydroxyphenyl group, R²⁰¹ is phenyl group, W¹⁰¹ is ⁻X¹⁰¹-C(=Y¹⁰¹)-, X¹⁰¹ is sulfur atom, Y¹⁰¹ is sulfur atom, Z¹⁰¹ is a single bond;
- the compound wherein R¹⁰¹ is 3,5-dibromo-2-hydroxyphenyl group, R²⁰¹ is phenyl group, W¹⁰¹ is -C(R³⁰¹)=N-, R³⁰¹ is hydroxy group, Z¹⁰¹ is a single bond;
- the compound wherein R¹⁰¹ is 5-bromo-2-hydroxyphenyl group, R²⁰¹ is 3,4-dichlorophenyl group, W¹⁰¹ is -C(R³⁰¹)=N-, R³⁰¹ is methyl group, Z¹⁰¹ is a single bond;
- the compound wherein R¹⁰¹ is 3-ethoxy-4-{[(thiophen-2-yl)carbonyl]oxy}phenyl group, R²⁰¹ is 3-carboxyphenyl group, W¹⁰¹ is -C(R³⁰¹)=N-, R³⁰¹ is methyl group, Z¹⁰¹ is a single bond;
- the compound wherein R¹⁰¹ is 4-carboxymethoxy-3-ethoxyphenyl group, R²⁰¹ is 3-(trifluoromethyl)phenyl group, W¹⁰¹ is -C(R³⁰¹)=N-, R³⁰¹ is methyl group, Z¹⁰¹ is a single bond;
- the compound wherein R¹⁰¹ is 5-[3-(methoxycarbonyl)phenyl]furan-2-yl group, R²⁰¹ is 4-fluorophenyl group, W¹⁰¹ is -C(R³⁰¹)=N-, R³⁰¹ is hydroxy group, Z¹⁰¹ is a single bond;
- the compound wherein R¹⁰¹ is 5-(4-carboxyphenyl)furan-2-yl group, R²⁰¹ is 4-chlorophenyl group, W¹⁰¹ is -C(R³⁰¹)=N-, R³⁰¹ is hydroxy group, Z¹⁰¹ is a single bond;
- the compound wherein R¹⁰¹ is 5-(3,5-dichlorophenyl)furan-2-yl group, R²⁰¹ is 3-carboxyphenyl group, W¹⁰¹ is -C(R³⁰¹)=N-, R³⁰¹ is trifluoromethyl group, Z¹⁰¹ is a single bond;
- the compound wherein R¹⁰¹ is 5-(5-carboxy-2-chlorophenyl)furan-2-yl group, R²⁰¹ is phenyl group, W¹⁰¹ is -C(R³⁰¹)=N-, R³⁰¹ is methyl group, Z¹⁰¹ is a single bond;
- the compound wherein R¹⁰¹ is 4-bromophenyl group, R²⁰¹ is 3-carboxy-4-chlorophenyl group, W¹⁰¹ is -C(R³⁰¹)=N-, R³⁰¹ is trifluoromethyl group, Z¹⁰¹ is a single bond;
- the compound wherein R¹⁰¹ is 5-(2-hydroxy-5-nitrophenyl)furan-2-yl group, R²⁰¹ is 3-chlorophenyl group, W¹⁰¹ is -C(R³⁰¹)=N-, R³⁰¹ is hydroxy group, Z¹⁰¹ is a single bond;
- the compound wherein R¹⁰¹ is 4-bromophenyl group, R²⁰¹ is 3-carboxy-4-chlorophenyl group, W¹⁰¹ is -C(R³⁰¹)=N-, R³⁰¹ is methyl group, Z¹⁰¹ is a single bond;
- the compound wherein R¹⁰¹ is 4-allyloxy-3-methoxyphenyl group, R²⁰¹ is 3-carboxy-4-chlorophenyl group, W¹⁰¹ is -C(R³⁰¹)=N-, R³⁰¹ is methyl group, Z¹⁰¹ is a single bond;
- the compound wherein R¹⁰¹ is 4-benzyloxy-3-methoxyphenyl group, R²⁰¹ is 3-carboxy-4-chlorophenyl group, W¹⁰¹ is -C(R³⁰¹)=N⁻, R³⁰¹ is methyl group, Z¹⁰¹ is a single bond;
- the compound wherein R¹⁰¹ is 4-chlorophenyl group, R²⁰¹ is phenyl group, W¹⁰¹ is -C(R³⁰¹)=N-, R³⁰¹ is carboxy group, Z¹⁰¹ is a single bond;
- the compound wherein R¹⁰¹ is phenyl group, R²⁰¹ is 3-carboxyphenyl group, W¹⁰¹ is -C(R³⁰¹)=N-, R³⁰¹ is methyl group, Z¹⁰¹ is a single bond;
- the compound wherein R¹⁰¹ is 2-benzyloxy-5-bromophenyl group, R²⁰¹ is 3-carboxyphenyl group, W¹⁰¹ is -C(R³⁰¹)=N-, R³⁰¹ is methyl group, Z¹⁰¹ is a single bond;
- the compound wherein R¹⁰¹ is 4-(benzylcarbamoyl)methoxy-3-bromo-5-methoxyphenyl group, R²⁰¹ is 3-carboxyphenyl group, W¹⁰¹ is -C(R³⁰¹)=N-, R³⁰¹ is methyl group, Z¹⁰¹ is a single bond;
- the compound wherein R¹⁰¹ is phenyl group, R²⁰¹ is 3-carboxyphenyl group, W¹⁰¹ is -C(R³⁰¹)=N-, R³⁰¹ is phenyl group, Z¹⁰¹ is a single bond;
- the compound wherein R¹⁰¹ is 5-(3-chlorophenyl)furan-2-yl group, R²⁰¹ is 4-sulfamoylphenyl group, W¹⁰¹ is -C(R³⁰¹)=N-, R³⁰¹ is methyl group, Z¹⁰¹ is a single bond;
- the compound wherein R¹⁰¹ is 5-(3-chloro-4-methylphenyl)furan-2-yl group, R²⁰¹ is 3-carboxyphenyl group, W¹⁰¹ is -C(R³⁰¹)=N-, R³⁰¹ is methyl group, Z¹⁰¹ is a single bond;
- the compound wherein R¹⁰¹ is 5-(2-chlorophenyl)furan-2-yl group, R²⁰¹ is 3-carboxy-4-chlorophenyl group, W¹⁰¹ is -C(R³⁰¹)=N-, R³⁰¹ is methyl group, Z¹⁰¹ is a single bond;
- the compound wherein R¹⁰¹ is 5-[3-(methoxycarbonyl)phenyl]furan-2-yl group, R²⁰¹ is 3-carboxyphenyl group, W¹⁰¹ is -C(R³⁰¹)=N-, R³⁰¹ is methyl group, Z¹⁰¹ is a single bond;
- the compound wherein R¹⁰¹ is 5-(3-chloro-2-methylphenyl)furan-2-yl group, R²⁰¹ is 3-carboxy-4-chlorophenyl group, W¹⁰¹ is -C(R³⁰¹)=N⁻, R³⁰¹ is methyl group, Z¹⁰¹ is a single bond;
- the compound wherein R¹⁰¹ is 5-(3,5-dichlorophenyl)furan-2-yl group, R²⁰¹ is 3-carboxyphenyl group, W¹⁰¹ is -C(R³⁰¹)=N-, R³⁰¹ is methyl group, Z¹⁰¹ is a single bond;
- the compound wherein R¹⁰¹ is 5-(3-fluorophenyl)furan-2-yl group, R²⁰¹ is 3-carboxy-4-chlorophenyl group, W¹⁰¹ is -C(R³⁰¹)=N-, R³⁰¹ is methyl group, Z¹⁰¹ is a single bond;
- the compound wherein R¹⁰¹ is 5-(3-nitrophenyl)furan-2-yl group, R²⁰¹ is 3-carboxyphenyl group, W¹⁰¹ is -C(R³⁰¹)=N-, R³⁰¹ is methyl group, Z¹⁰¹ is a single bond; and
- the compound wherein R¹⁰¹ is 5-(5-carboxy-2-chlorophenyl)furan-2-yl group, R²⁰¹ is 3-(trifluoromethyl)phenyl group, W¹⁰¹ is -C(R³⁰¹)=N-, R³⁰¹ is hydroxy group, Z¹⁰¹ is a single bond.

When W¹⁰¹ is -X¹⁰¹-C(=Y¹⁰¹)-, the compound which corresponds to any one of the following (a), (b) and (c) is preferred as the compound represented by the general formula (II).
(a) the compound wherein R¹⁰¹ is a phenyl group which is substituted with one to three hydroxy groups (said phenyl group may further be substituted with one or more substituents other than the hydroxy group),
   R²⁰¹ is an aromatic group which may be substituted,
   X¹⁰¹ is sulfur atom,
   Y¹⁰¹ is oxygen atom or sulfur atom,
   Z¹⁰¹ is methylene group;
(b) the compound wherein R¹⁰¹ is a phenyl group which is substituted with a C₆-C₁₀ aryl-substituted carbamoyl group (said aryl group may be substituted, and said phenyl group may further be substituted with one or more substituents other than the C₆-C₁₀ aryl-substituted carbamoyl group), or a phenyl group which is substituted with a heteroaryl-substituted carbamoyl group (said heteroaryl group may be substituted, and said phenyl group may further be substituted with one or more substituents other than the heteroaryl-substituted carbamoyl group),
   R²⁰¹ is an aromatic group which may be substituted,
   X¹⁰¹ is sulfur atom,
   Y¹⁰¹ is oxygen atom or sulfur atom,
   Z¹⁰¹ is methylene group;
(c) the compound wherein R¹⁰¹ and R²⁰¹ are either the following (i) or (ii),
   X¹⁰¹ is sulfur atom,
   Y¹⁰¹ is oxygen atom or sulfur atom,
   Z¹⁰¹ is methylene group:
   (i) R¹⁰¹ is a phenyl group which is substituted with carboxy group (said phenyl group may further be substituted with one or more substituents other than the carboxy group), R²⁰¹ is an aromatic group which may be substituted;
   (ii) R¹⁰¹ is an aromatic group which may be substituted,
      R²⁰¹ is a phenyl group which is substituted with carboxy group (said phenyl group may further be substituted with one or more substituents other than the carboxy group).

The compound wherein R¹⁰¹ is a phenyl group which is substituted with two or three hydroxy groups (said phenyl group may further be substituted with one or more substituents other than the hydroxy group),
R²⁰¹ is a phenyl group which may be substituted,
X¹⁰¹ is sulfur atom,
Y¹⁰¹ is oxygen atom or sulfur atom,
Z¹⁰¹ is methylene group is preferred as the compound which corresponds to the aforementioned (a).

The compound selected from the compounds represented by the following compound numbers described in the specification is more preferred:
- Compound Nos. 3, 4, 8 to 27, 29 to 38, 40 to 54, 56 to 61, 70, 81 to 84, 89, 301 to 307, 309, 311 to 321, 334, 335, 338, 339, 341, 342, 344, 347, 432, 446, 463, 464, 469, 470, 474, and 475.

The compound selected from the following 14 compounds is further preferred:
- the compound wherein R¹⁰¹ is 3,4,5-trihydroxyphenyl group, R²⁰¹ is 3,4-dichlorophenyl group, X¹⁰¹ is sulfur atom, Y¹⁰¹ is oxygen atom, Z¹⁰¹ is methylene group;
- the compound wherein R¹⁰¹ is 2,3-dihydroxyphenyl group, R²⁰¹ is 2,3-dichlorophenyl group, X¹⁰¹ is sulfur atom, Y¹⁰¹ is oxygen atom, Z¹⁰¹ is methylene group;
- the compound wherein R¹⁰¹ is 2,3-dihydroxyphenyl group, R²⁰¹ is 2,4-dichlorophenyl group, X¹⁰¹ is sulfur atom, Y¹⁰¹ is oxygen atom, Z¹⁰¹ is methylene group;
- the compound wherein R¹⁰¹ is 3,4-dihydroxyphenyl group, R²⁰¹ is 2,4-dichlorophenyl group, X¹⁰¹ is sulfur atom, Y¹⁰¹ is oxygen atom, Z¹⁰¹ is methylene group;
- the compound wherein R¹⁰¹ is 2,3-dihydroxyphenyl group, R²⁰¹ is 4-bromophenyl group, X¹⁰¹ is sulfur atom, Y¹⁰¹is oxygen atom, Z¹⁰¹ is methylene group;
- the compound wherein R¹⁰¹ is 2,3-dihydroxyphenyl group, R²⁰¹ is 4-(trifluoromethyl)phenyl group, X¹⁰¹ is sulfur atom, Y¹⁰¹ is oxygen atom, Z¹⁰¹ is methylene group;
- the compound wherein R¹⁰¹ is 2,3-dihydroxyphenyl group, R²⁰¹ is 4-styrylphenyl group, X¹⁰¹ is sulfur atom, Y¹⁰¹ is oxygen atom, Z¹⁰¹ is methylene group;
- the compound wherein R¹⁰¹ is 3,4-dihydroxyphenyl group, R²⁰¹ is 3,4-dichlorophenyl group, X¹⁰¹ is sulfur atom, Y¹⁰¹ is sulfur atom, Z¹⁰¹ is methylene group;
- the compound wherein R¹⁰¹ is 2,3-dihydroxyphenyl group, R²⁰¹ is 4-[(3-phenoxyphenyl)acetylamino]phenyl group, X¹⁰¹ is sulfur atom, Y¹⁰¹ is oxygen atom, Z¹⁰¹ is methylene group;
- the compound wherein R¹⁰¹ is 2,3-dihydroxyphenyl group, R²⁰¹ is 4-(4-phenoxybenzoylamino)phenyl group, X¹⁰¹ is sulfur atom, Y¹⁰¹ is oxygen atom, Z¹⁰¹ is methylene group;
- the compound wherein R¹⁰¹ is 2,3-dihydroxyphenyl group, R²⁰¹ is 4-[3,5-bis(trifluoromethyl)phenylacetylamino]phenyl group, X¹⁰¹ is sulfur atom, Y¹⁰¹ is oxygen atom, Z¹⁰¹ is methylene group;
- the compound wherein R¹⁰¹ is 2,3-dihydroxyphenyl group, R²⁰¹ is 3-[(3-phenoxyphenyl)acetylamino]phenyl group, X¹⁰¹ is sulfur atom, Y¹⁰¹ is oxygen atom, Z¹⁰¹ is methylene group;
- the compound wherein R¹⁰¹ is 3,4-dihydroxy-5-nitrophenyl group, R²⁰¹ is 3,4-dichlorophenyl group, X¹⁰¹ is sulfur atom, Y¹⁰¹ is sulfur atom, Z¹⁰¹ is methylene group; and
- the compound wherein R¹⁰¹ is 3,4-dihydroxy-5-[3-(trifluoromethoxy)phenylcarbamoyl]phenyl group, R²⁰¹ is 3,4-dichlorophenyl group, X¹⁰¹ is sulfur atom, Y¹⁰¹ is sulfur atom, Z¹⁰¹ is methylene group.

The compound wherein R¹⁰¹ is a phenyl group which is substituted with a C₆-C₁₀ aryl-substituted carbamoyl group in the 3-position (said aryl group may be substituted, and said phenyl group may further be substituted with one or more substituents other than the C₆-C₁₀ aryl-substituted carbamoyl group) or a phenyl group which is substituted with a heteroaryl-substituted carbamoyl group (said heteroaryl group may be substituted, and said phenyl group may further be substituted with one or more substituents other than the heteroaryl-substituted carbamoyl group), and a phenyl group which is substituted with hydroxy group in the 4-position (said phenyl group may be substituted in the 5-position),
R²⁰¹ is a phenyl group which may be substituted,
X¹⁰¹ is sulfur atom,
Y¹⁰¹ is oxygen atom,
Z¹⁰¹ is methylene group is preferred as the compound which corresponds to the aforementioned (b).

The compound selected from the compounds represented by the following compound numbers described in the specification is more preferred:
- Compound Nos. 349, 351 to 356, 360, 361, 371 to 378, 383 to 386, 391 to 408, 414 to 424, 428, 433 to 442, 450 to 460, and 465 to 475.

The compound selected from the following 11 compounds is further preferred:
- the compound wherein R¹⁰¹ is 3-methoxy-4-hydroxy-5-(3,4-dihydroxyphenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
- the compound wherein R¹⁰¹ is 4-hydroxy-3-(3,4-dihydroxyphenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
- the compound wherein R¹⁰¹ is 4-hydroxy-3-(3,4-dihydroxyphenylcarbamoyl)phenyl group, R²⁰¹ is 3,5-bis(trifluoromethyl)phenyl group, X¹⁰¹ is sulfur atom, Y¹⁰¹ is oxygen atom, Z¹⁰¹ is methylene group;
- the compound wherein R¹⁰¹ is 3-methyl-4-hydroxy-5-(3,4-dihydroxyphenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
- the compound wherein R¹⁰¹ is 4-hydroxy-3-(3,4-dihydroxyphenylcarbamoyl)phenyl group, R²⁰¹ is 4-(trifluoromethyl)phenyl group, X¹⁰¹ is sulfur atom, Y¹⁰¹ is oxygen atom, Z¹⁰¹ is methylene group;
- the compound wherein R¹⁰¹ is 4-hydroxy-3-[2-chloro-5-(trifluoromethyl)phenylcarbamoyl]phenyl group, R²⁰¹ is 3,4-dichlorophenyl group, X¹⁰¹ is sulfur atom, Y¹⁰¹ is oxygen atom, Z¹⁰¹ is methylene group;
- the compound wherein R¹⁰¹ is 4-hydroxy-3-(3,4,5-trihydroxyphenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
- the compound wherein R¹⁰¹ is 4-hydroxy-3-(2,3-dihydroxyphenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
- the compound wherein R¹⁰¹ is 4-hydroxy-3-(2,5-dichlorophenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
- the compound wherein R¹⁰¹ is 4-hydroxy-3-(2-chloro-5-nitrophenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group; and
- the compound wherein R¹⁰¹ is 3,4-dihydroxy-5-[3-(trifluoromethyl)phenylcarbamoyl]phenyl group, R²⁰¹ is 3,4-dichlorophenyl group, X¹⁰¹ is sulfur atom, Y¹⁰¹ is oxygen atom, Z¹⁰¹ is methylene group.

The compound selected from the compounds represented by the following compound numbers described in the specification is preferred as the compound which corresponds to the aforementioned (c):
- Compound Nos. 345, 350, 358, 390, 409, 418, 430, 446, and 464.

The compound selected from the following 2 compounds is more preferred:
- the compound wherein R¹⁰¹ is 3-carboxyphenyl group, R²⁰¹ is 3,4-dichlorophenyl group, X¹⁰¹ is sulfur atom, Y¹⁰¹ is oxygen atom, Z¹⁰¹ is methylene group; and
- the compound wherein R¹⁰¹ is 3-carboxy-4-hydroxyphenyl group, R²⁰¹ is 4-methoxycarbonylphenyl group, X¹⁰¹ is sulfur atom, Y¹⁰¹ is oxygen atom, Z¹⁰¹ is methylene group.

When W¹⁰¹ is -C(R³⁰¹)=N-, a compound which corresponds to any one of the following (d), (e) and (f) is preferred as the compound represented by the general formula (II).
(d) the compound wherein R¹⁰¹ is a phenyl group which is substituted with one to three hydroxy groups (said phenyl group may further be substituted with one or more substituents other than the hydroxy group),
   R²⁰¹ is an aromatic group which may be substituted,
   R³⁰¹ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
   Z¹⁰¹ is a single bond;
(e) the compound wherein R¹⁰¹ is a phenyl group which is substituted with C₂-C₆ alkenyl group which may be substituted (said phenyl group may further be substituted with one or more substituents other than the C₂-C₆ alkenyl group),
   R²⁰¹ is an aromatic group which may be substituted,
   R³⁰¹ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
   Z¹⁰¹ is a single bond;
(f) the compound wherein R¹⁰¹, R²⁰¹ and R³⁰¹ are any one of the following (i) to (iii), Z¹⁰¹ is a single bond:
   (i) R¹⁰¹ is a phenyl group which is substituted with carboxy group (said phenyl group may further be substituted with one or more substituents other than the carboxy group), R²⁰¹ is an aromatic group which may be substituted,
      R³⁰¹ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group;
   (ii) R¹⁰¹ is an aromatic group which may be substituted,
      R²⁰¹ is a phenyl group which is substituted with carboxy group (said phenyl group may further be substituted with one or more substituents other than the carboxy group), R³⁰¹ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group;
   (iii) R¹⁰¹ is an aromatic group which may be substituted,
      R²⁰¹ is an aromatic group which may be substituted,
      R³⁰¹ is carboxy group.

The compound wherein R¹⁰¹ is a phenyl group which is substituted with two or three hydroxy groups (said phenyl group may further be substituted with one or more substituents other than the hydroxy group),
R²⁰¹ is a phenyl group which may be substituted,
R³⁰¹ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
Z¹⁰¹ is a single bond is preferred as the compound which corresponds to the aforementioned (d).

The compound selected from the compounds represented by the following compound numbers described in the specification is more preferred:
- Compound Nos. 104, 107, 109 to 114, 116 to 120, 123 to 158, 221 to 224, 501 to 505, 514, 515, 519, 522, 529, 540, 545, 551 to 554, 556, and 557.

The compound selected from the following 6 compounds is further preferred:
- the compound wherein R¹⁰¹ is 2,4,5-trihydroxyphenyl group, R²⁰¹ is 3,5-dichlorophenyl group, R³⁰¹ is phenyl group, Z¹⁰¹ is a single bond;
- the compound wherein R¹⁰¹ is 2,4,5-trihydroxyphenyl group, R²⁰¹ is 3,4-dichlorophenyl group, R³⁰¹ is isopropyl group, Z¹⁰¹ is a single bond;
- the compound wherein R¹⁰¹ is 2,4,5-trihydroxyphenyl group, R²⁰¹ is 4-nitrophenyl group, R³⁰¹ is ethoxy group, Z¹⁰¹ is a single bond;
- the compound wherein R¹⁰¹ is 2,4,5-trihydroxyphenyl group, R²⁰¹ is 3,4-dichlorophenyl group, R³⁰¹ is methyl group, Z¹⁰¹ is a single bond;
- the compound wherein R¹⁰¹ is 2,4,5-trihydroxyphenyl group, R²⁰¹ is 3,5-dichlorophenyl group, R³⁰¹ is isopropyl group, Z¹⁰¹ is a single bond; and
- the compound wherein R¹⁰¹ is 2,4,5-trihydroxyphenyl group, R²⁰¹ is 3-chloro-4-fluorophenyl group, R³⁰¹ is isopropyl group, Z¹⁰¹ is a single bond.

The compound selected from the compounds represented by the following compound numbers described in the specification is preferred as the compound which corresponds to the aforementioned (e):
- Compound Nos. 517, 524, 525, 530 to 538, 541 to 544, 565, and 566.

The compound selected from the following 2 compounds is more preferred:
- the compound wherein R¹⁰¹ is 2-hydroxy-5-styrylphenyl group, R²⁰¹ is 4-nitrophenyl group, R³⁰¹ is ethoxy group, Z¹⁰¹ is a single bond; and
- the compound wherein R¹⁰¹ is 4-styrylphenyl group, R²⁰¹ is 4-carboxyphenyl group, R³⁰¹ is isopropyl group, Z¹⁰¹ is a single bond.

The compound selected from the compounds represented by the following compound numbers described in the specification is preferred as the compound which corresponds to the aforementioned (f):
- Compound Nos. 106, 153, 176, 179, 184, 198, 211, 212, 215 to 218, 507, 534 to 536, 555, 559, 564, and 567.

The following compounds is more preferred:
- the compound wherein R¹⁰¹ is 4-styrylphenyl group, R²⁰¹ is 4-carboxyphenyl group, R³⁰¹ is isopropyl group, Z¹⁰¹ is a single bond.

The compounds represented by the aforementioned general formula (I) or (II) may form salts. Examples of salts include, when acidic groups exist, salts which are formed with alkali metals and alkaline-earth metals such as lithium, sodium, potassium, magnesium, calcium; salts which are formed with amines such as ammonia, methylamine, dimethylamine, trimethylamine, dicyclohexylamine; or salts which are formed with basic amino acids such as lysine, arginine. Examples include, when basic groups exist, salts which are formed with mineral acids such as hydrochloric acid, hydrobromic acid, sulfuric acid, nitric acid, phosphoric acid; salts which are formed with organic acids such as methanesulfonic acid, benzenesulfonic acid, p-toluenesulfonic acid, acetic acid, propionic acid, tartaric acid, fumaric acid, maleic acid, malic acid, oxalic acid, succinic acid, citric acid, benzoic acid, mandelic acid, mandelic acid, cinnamic acid, lactic acid; or salts which are formed with acidic amino acids such as aspartic acid, glutamic acid.

As an active ingredient of the medicament according to the present invention, the compounds represented by the aforementioned general formula (I) or pharmacologically acceptable salts thereof, as well as hydrates thereof or solvates thereof can be used. A type of a solvent that forms the solvate is not particularly limited. Examples thereof include, for example, alcohols such as ethanol. The compounds represented by the aforementioned general formula (I) have double bond(s), the compounds may be in either E-configuration or Z-configuration, and any geometrical isomer in a pure form or any mixture thereof can be used as an active ingredient of the medicament of the present invention. The compounds represented by the aforementioned general formula (I) may have one or more asymmetric carbon atoms. As for the stereochemistry of asymmetric carbon atoms, each asymmetric carbon atom may independently in either R-configuration or S-configuration, and the compounds may exist as stereoisomers such as optical isomers and diastereoisomers. As an active ingredient of the medicament according to the present invention, any stereoisomers thereof in a pure form, any mixtures thereof, racemates thereof and the like may be used. The compounds represented by the aforementioned general formula (I) may exist as tautomers depending on a type of a substituent. As an active ingredient of the medicament according to the present invention, any tautomers may be used.

The scope of the present invention relating to the novel compounds represented by the aforementioned general formula (II) encompasses compounds in free form and salts thereof as well as hydrates thereof and solvates thereof. A type of a solvent that forms the solvate is not particularly limited. Examples thereof include, for example, alcohols such as ethanol. The compounds represented by the aforementioned general formula (II) have double bond(s), the compounds may be in either E-configuration or Z-configuration, and any geometrical isomer in a pure form or any mixture thereof fall within the scope of the present invention. The compounds represented by the aforementioned general formula (II) may have one or more asymmetric carbon atoms. As for the stereochemistry of asymmetric carbon atoms, each asymmetric carbon atom may independently in either R-configuration or S-configuration, and the compounds may exist as stereoisomers such as optical isomers and diastereoisomers. Any stereoisomers thereof in a pure form, any mixtures thereof, racemates thereof and the like fall within the scope of the present invention. The compounds represented by the aforementioned general formula (II) may exist as tautomers depending on a type of a substituent. Any tautomers fall within the scope of the present invention.

Examples of the compounds represented by the general formula (I) or (II) will be shown below. However, the compounds represented by the general formula (I) or (II) are not limited to these examples.

It is obvious that the compounds represented by the general formula (II) fall within the compounds represented by the general formula (I). Each of R¹, R², R³, W, X, Y and Z which is used in the following tables as partial structure of the general formula (I) can correspond to each of R¹⁰¹, R²⁰¹, R³⁰¹, W¹⁰¹, X¹⁰¹, Y¹⁰¹ and Z¹⁰¹ which is partial structure of the general formula (II).

The abbreviations used in the following tables have the following meanings. Me: methyl group; Et: ethyl group; i-Pr: isopropyl group; t-Bu: tert-butyl group; Allyl: allyl group; Ph: phenyl group; OMe: methoxy group; OEt: ethoxy group; OBn: benzyloxy group; CO₂Me: methoxycarbonyl group; CO₂Et: ethoxycarbonyl group; Ac: acetyl group; Bn: benzyl group; Arg: L-(+)-arginine.

In the following tables,
(1) the compound of Compound No. 334 represents a tri(L-(+)-arginine) salt of 5-(3,4,5-trihydroxybenzylidene)-3-(3,4-dichlorobenzyl)thiazolidine-2,4-dione;
(2) the compound of Compound No. 335 represents a di(L-(+)-arginine) salt of 5-(3,4-dihydroxybenzylidene)-3-(2,4-dichlorobenzyl)thiazolidine-2,4-dione;
(3) the compound of Compound No. 553 represents a tri(L-(+)-arginine) salt of 4-(2,4,5-trihydroxybenzylidene)-1-(3,4-dichlorobenzyl)-3-isopropyl-2-pyrazolin-5-one;
(4) the compound of Compound No. 554 represents a di(L-(+)-arginine) salt of 4-(2,4, 5-trihydroxybenzylidene)-1-(3,4-dichlorobenzyl)-3-isopropyl-2-pyrazolin-5-one.

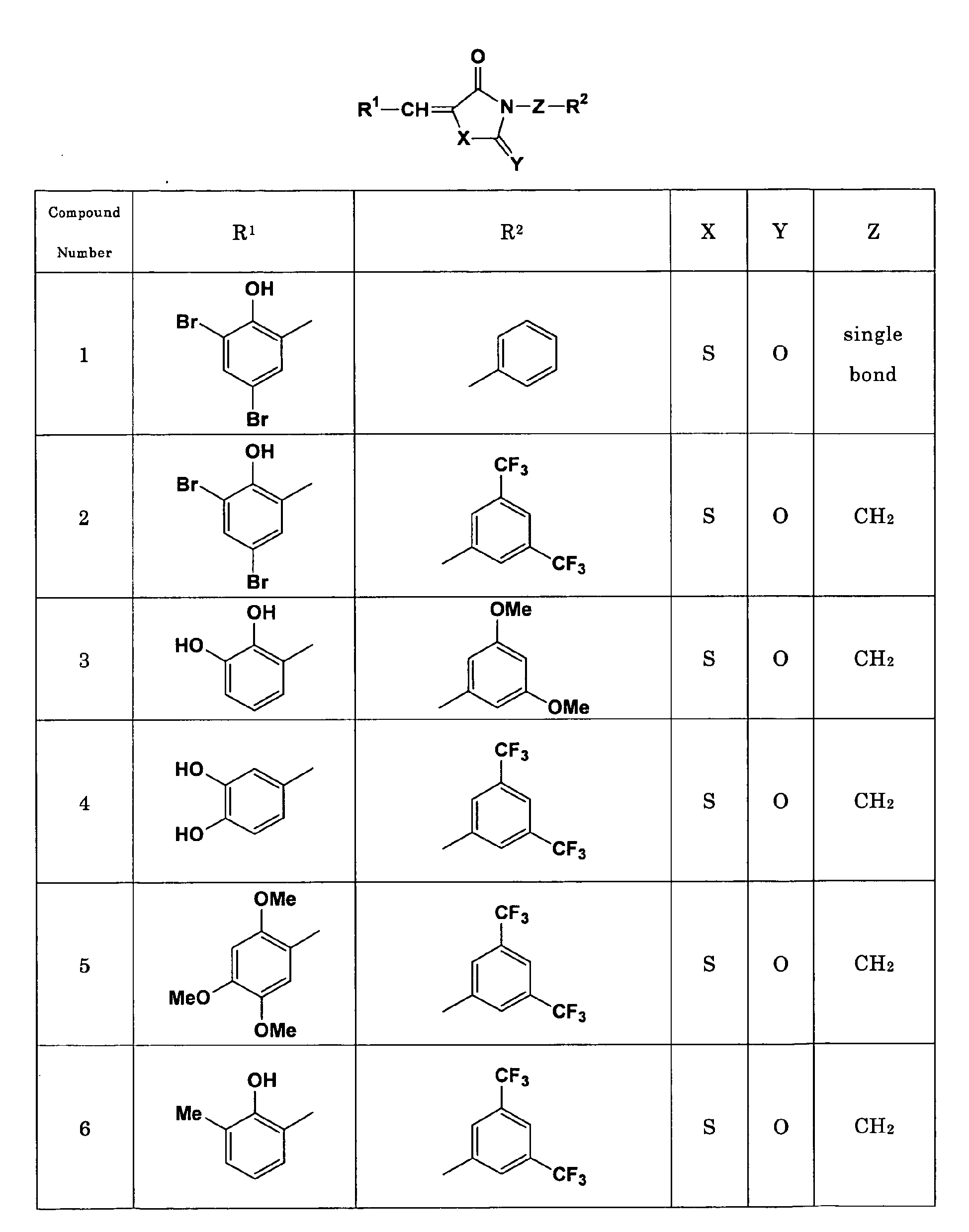

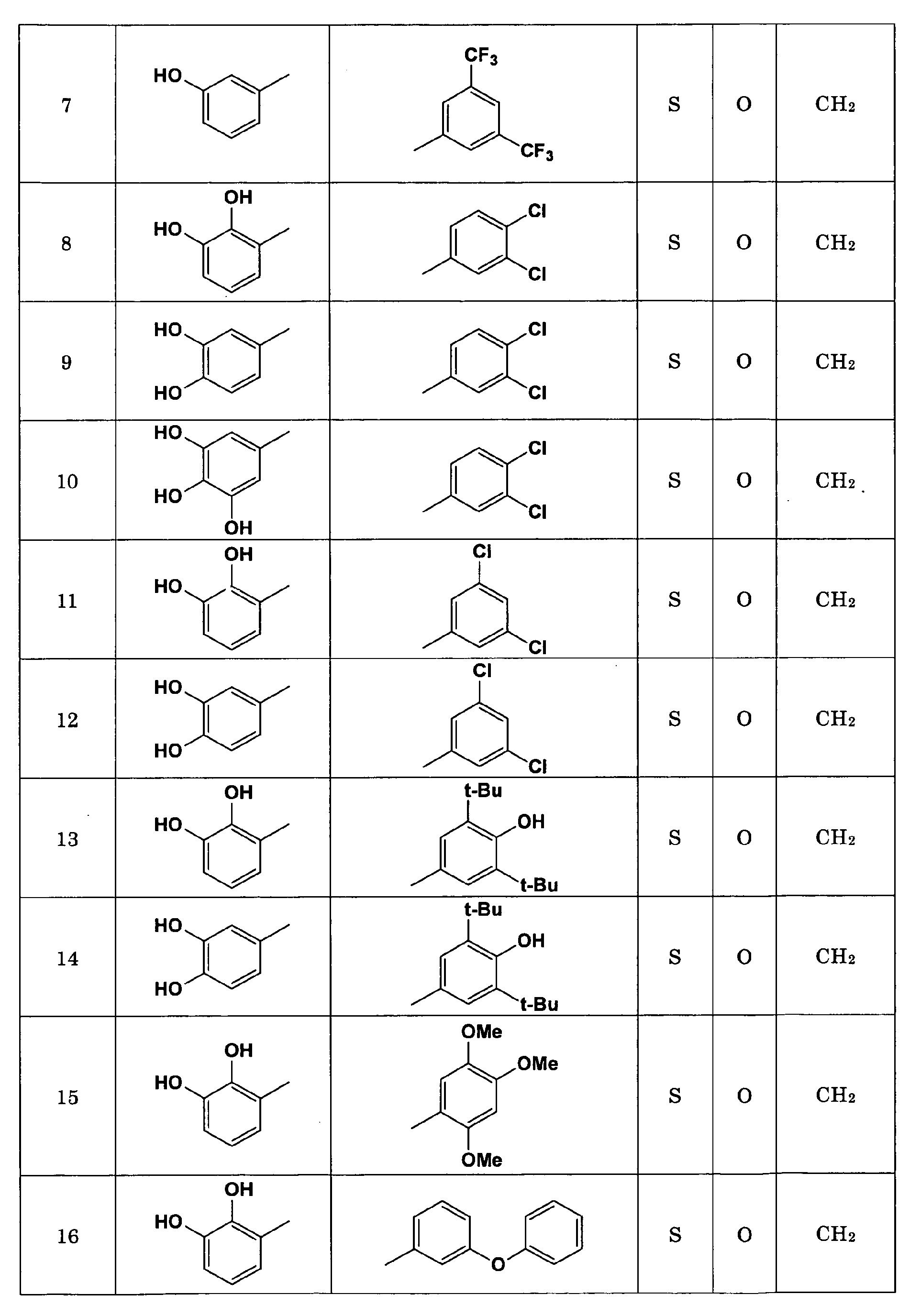

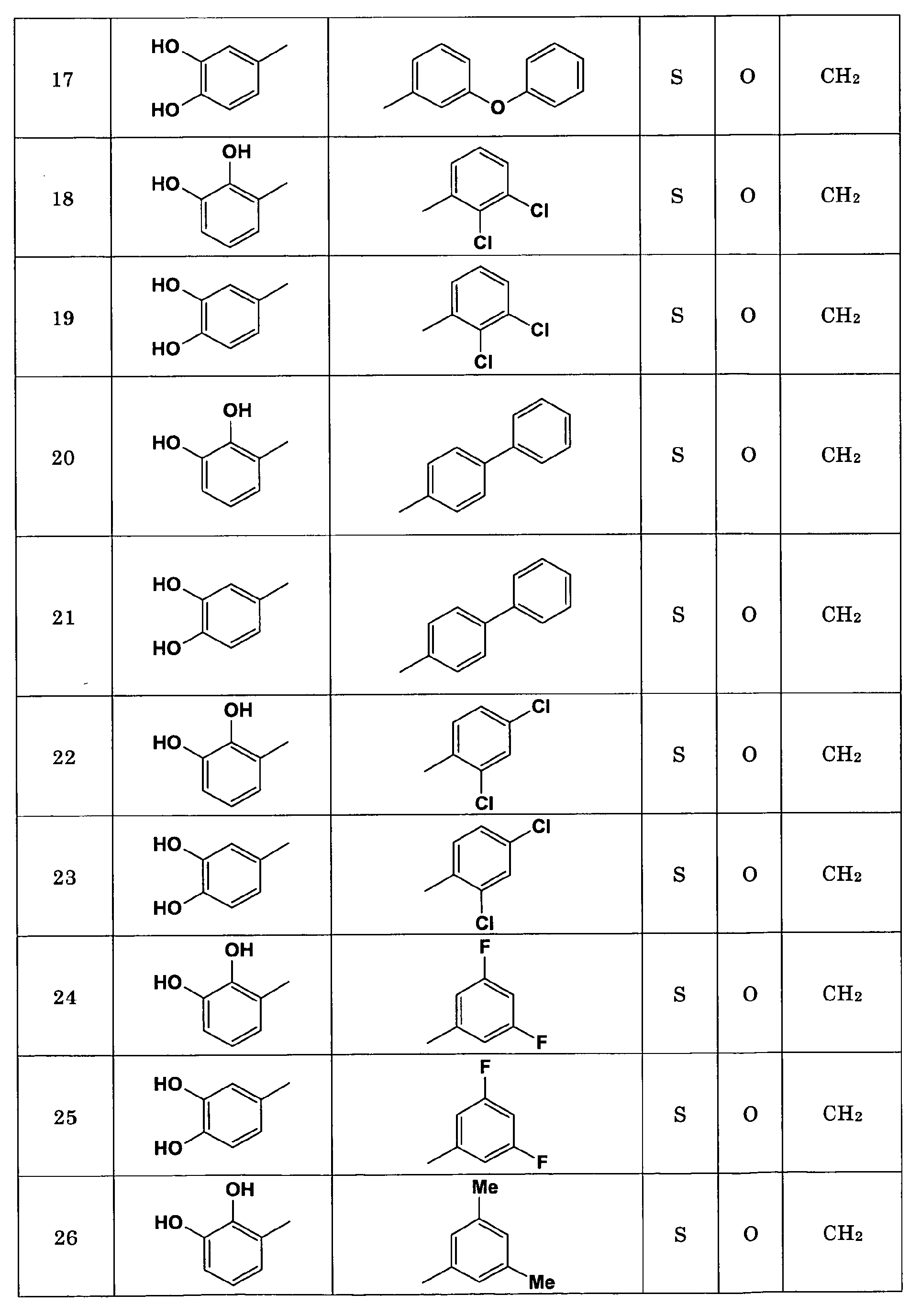

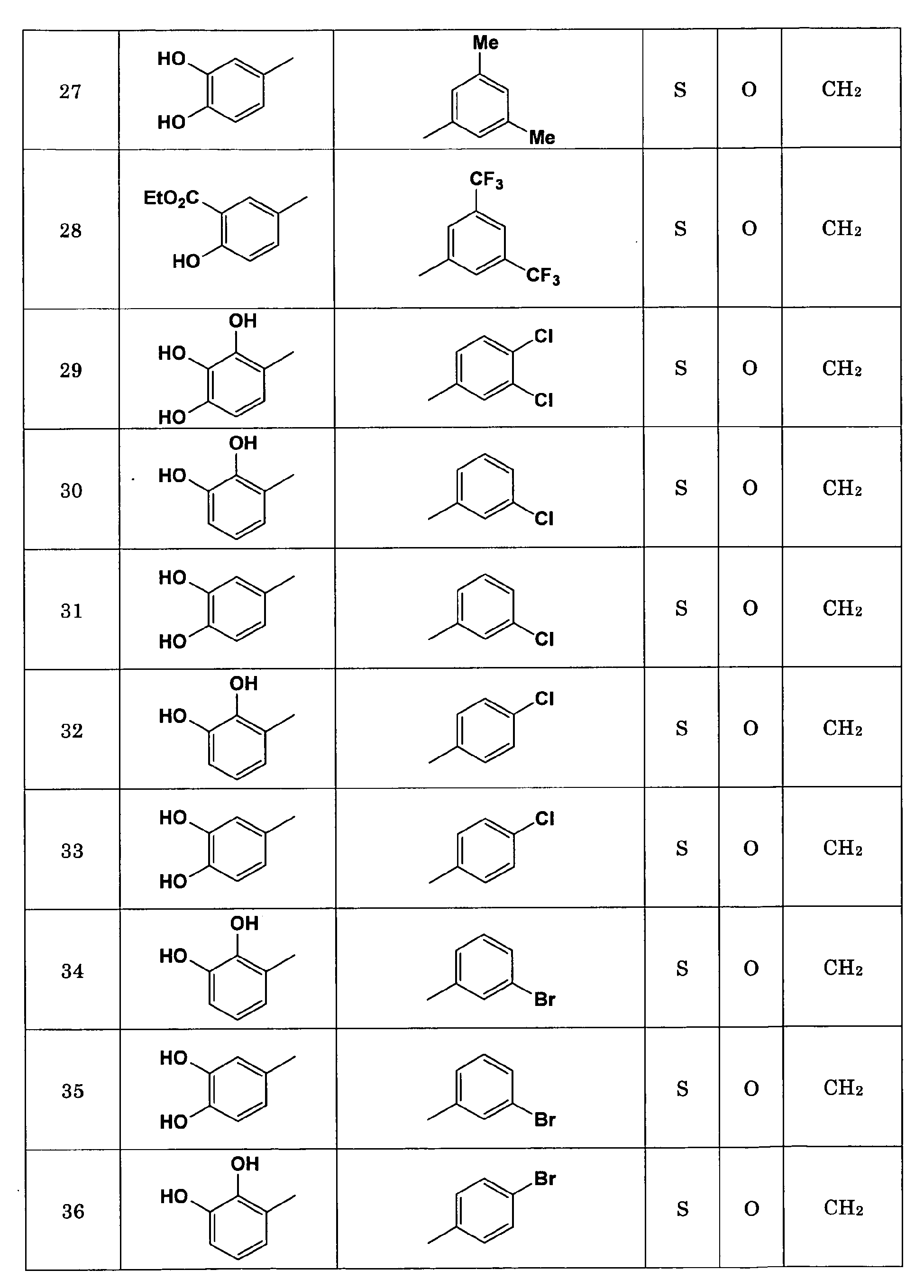

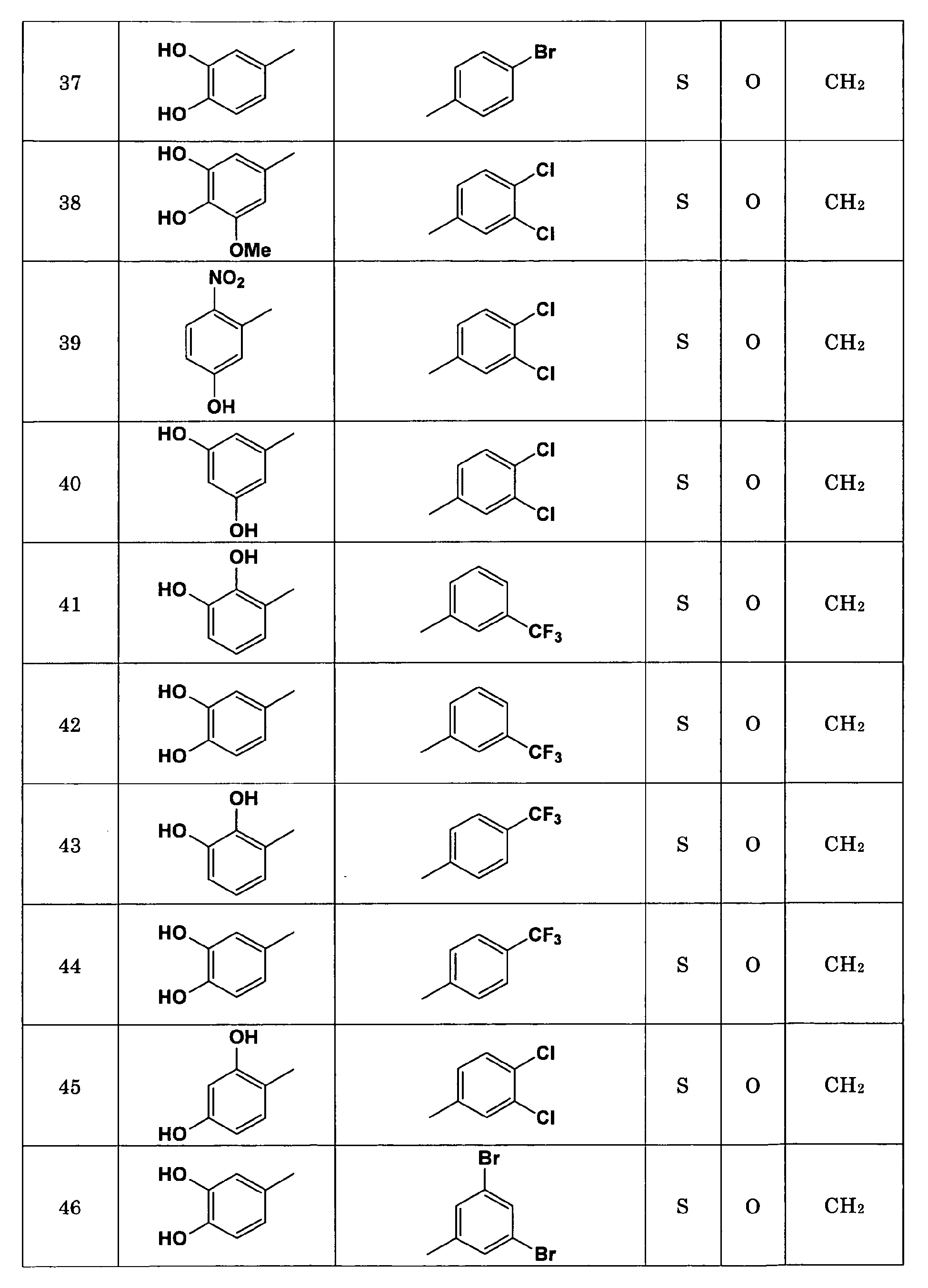

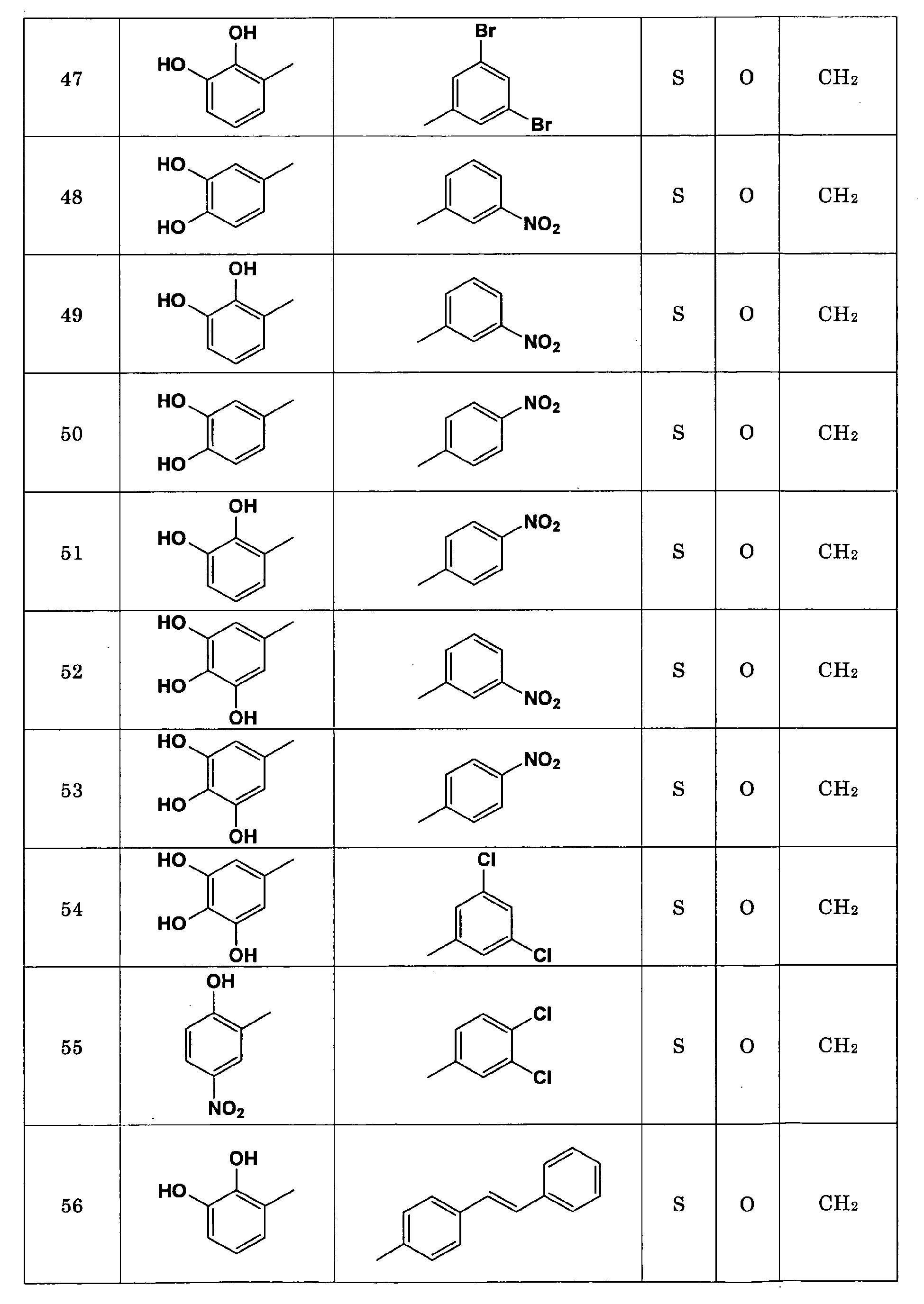

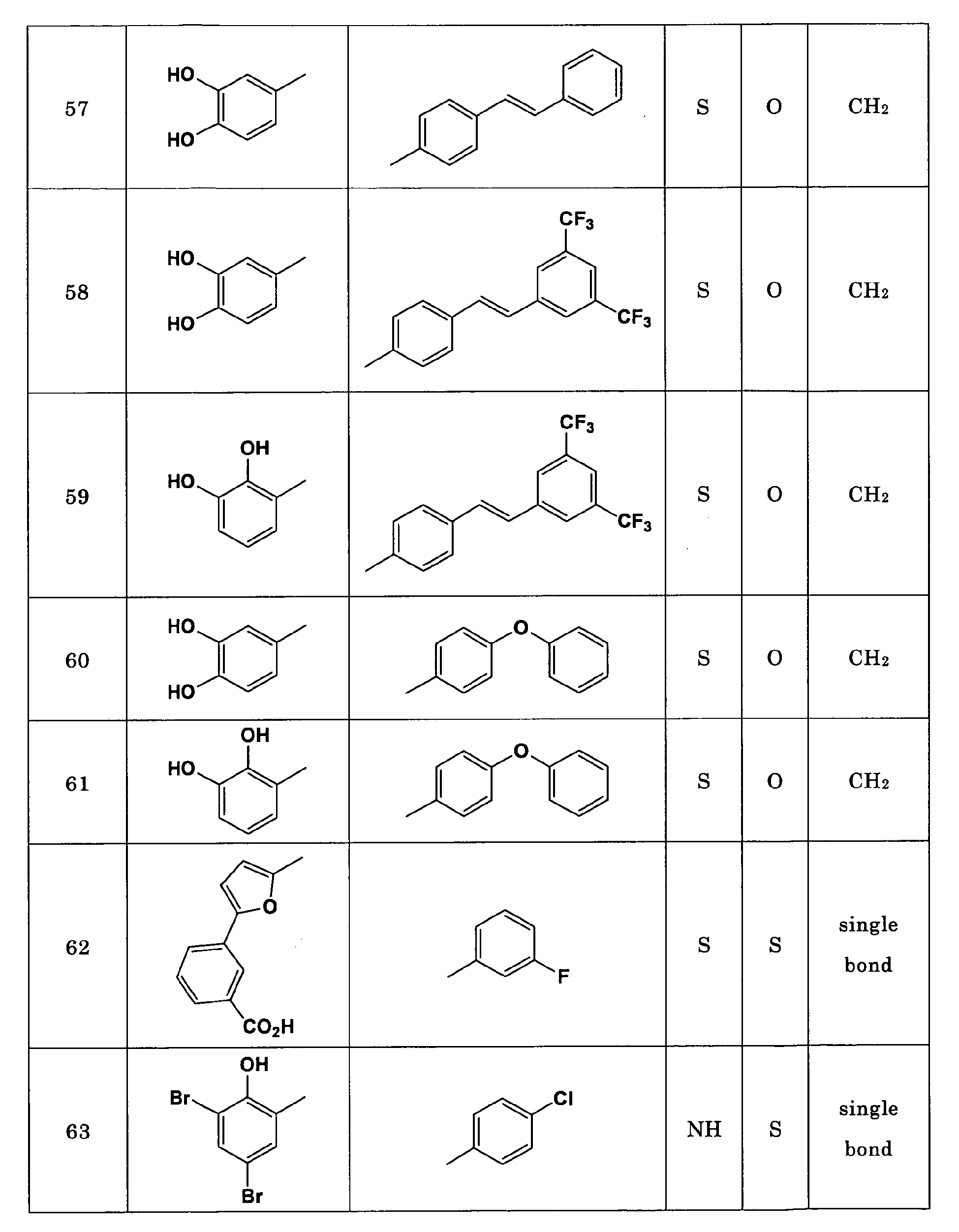

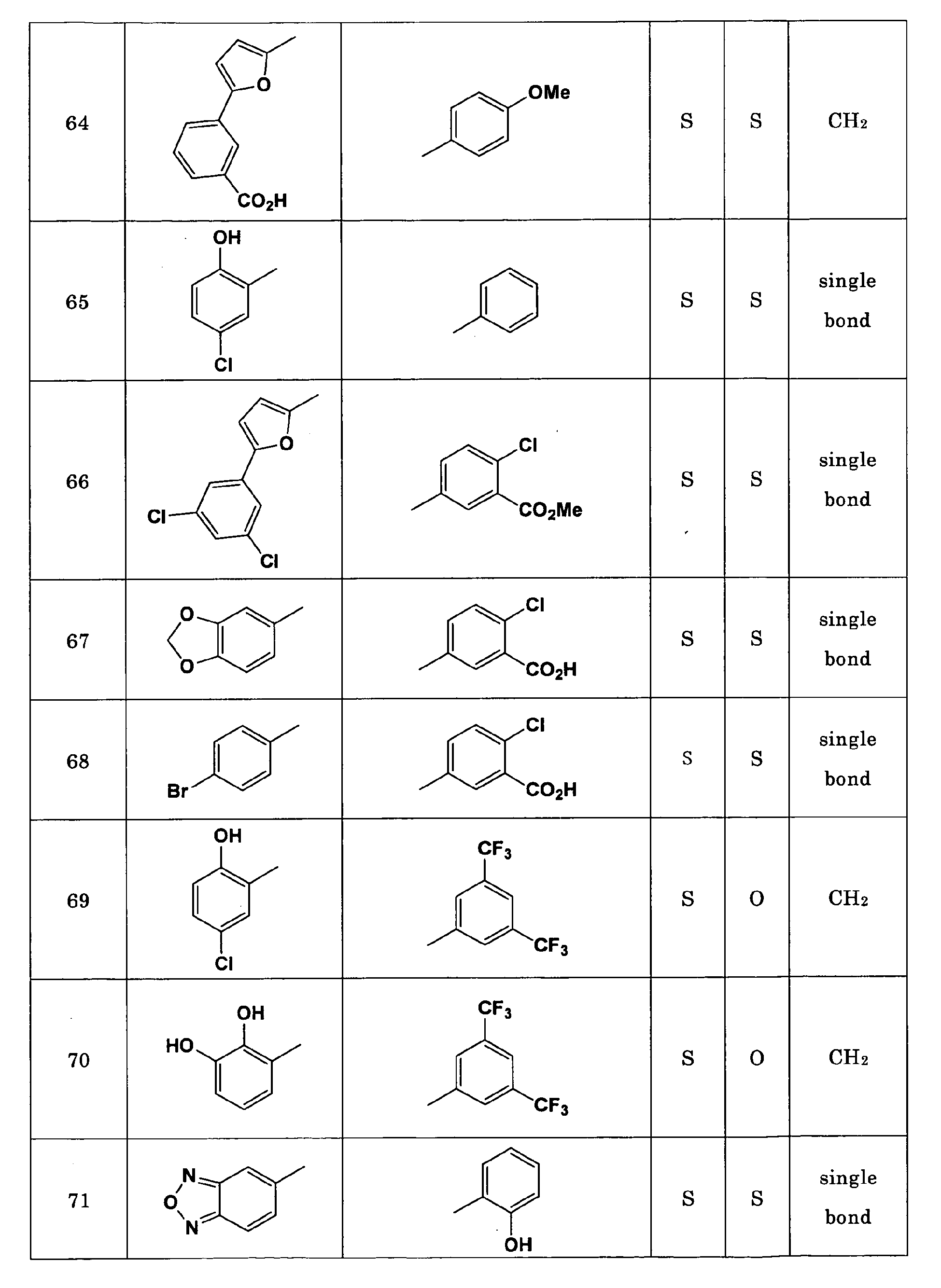

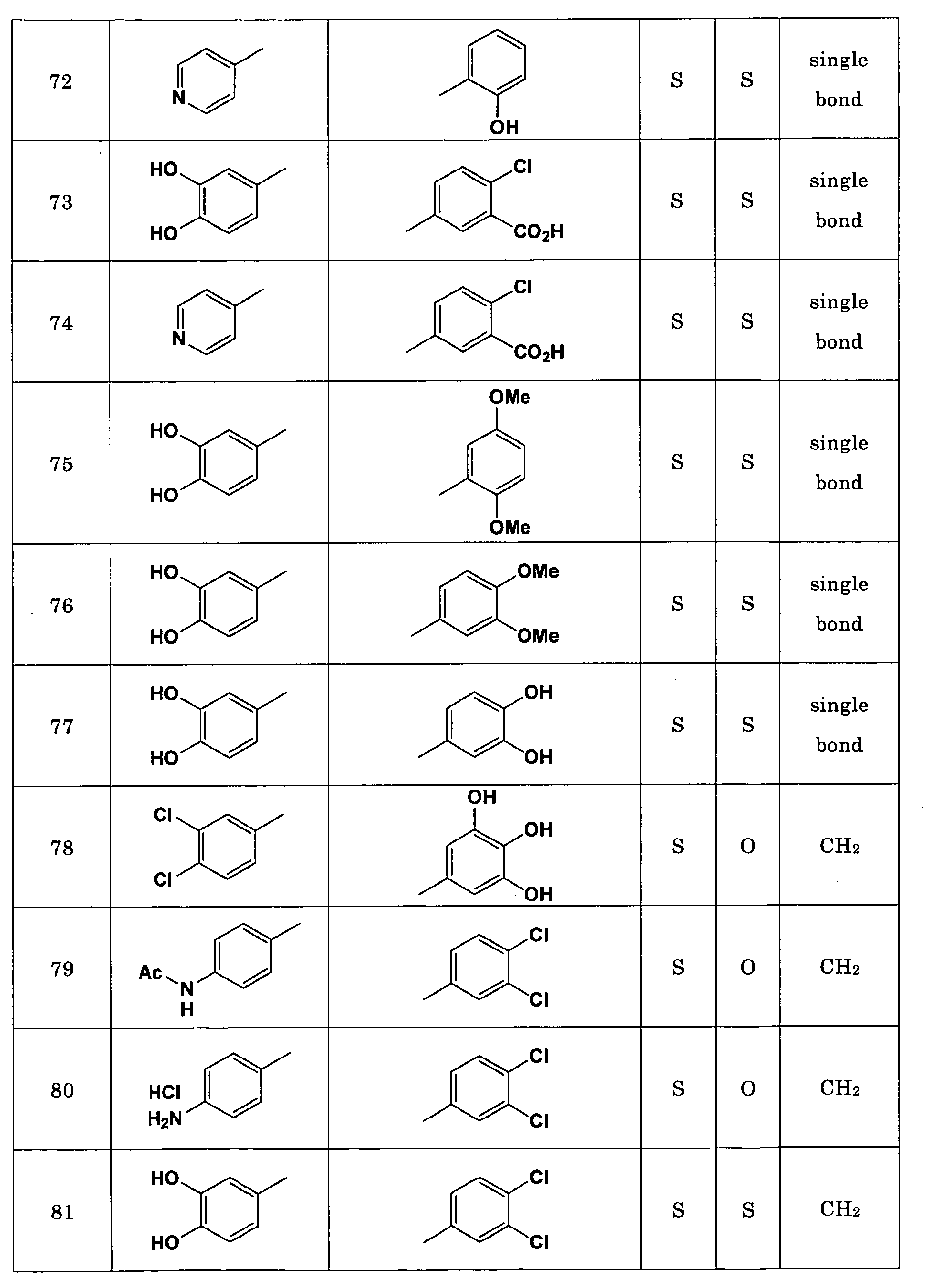

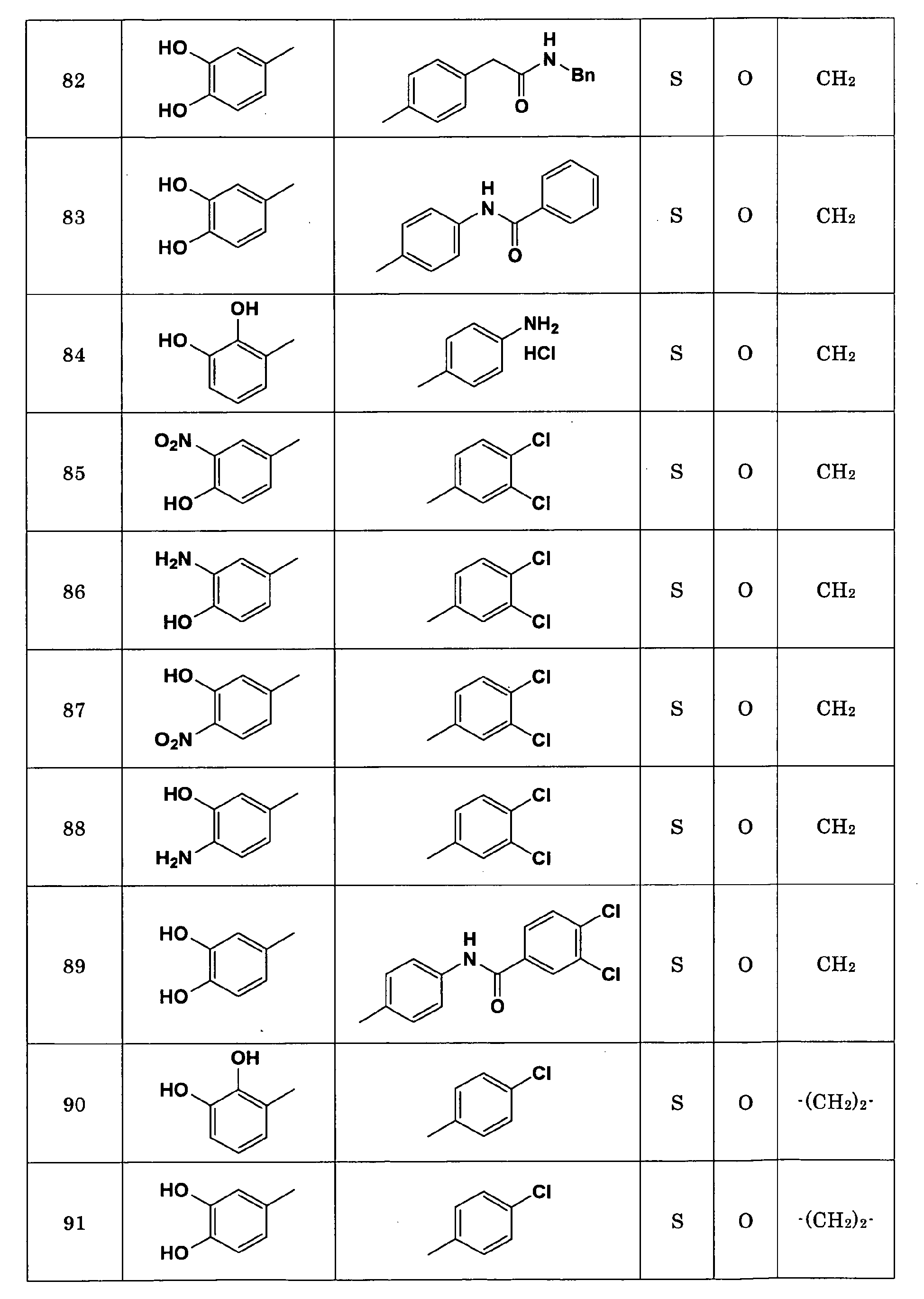

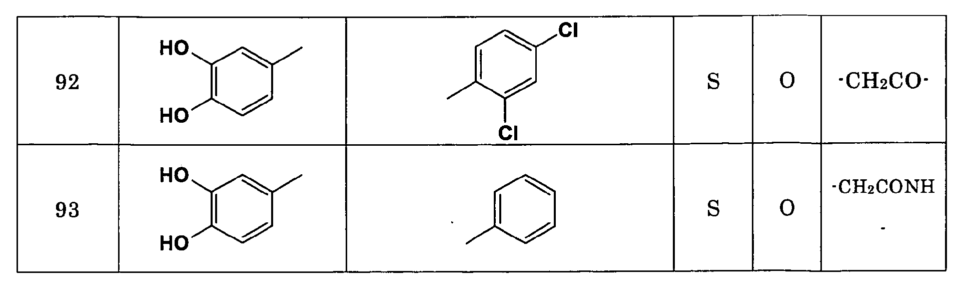

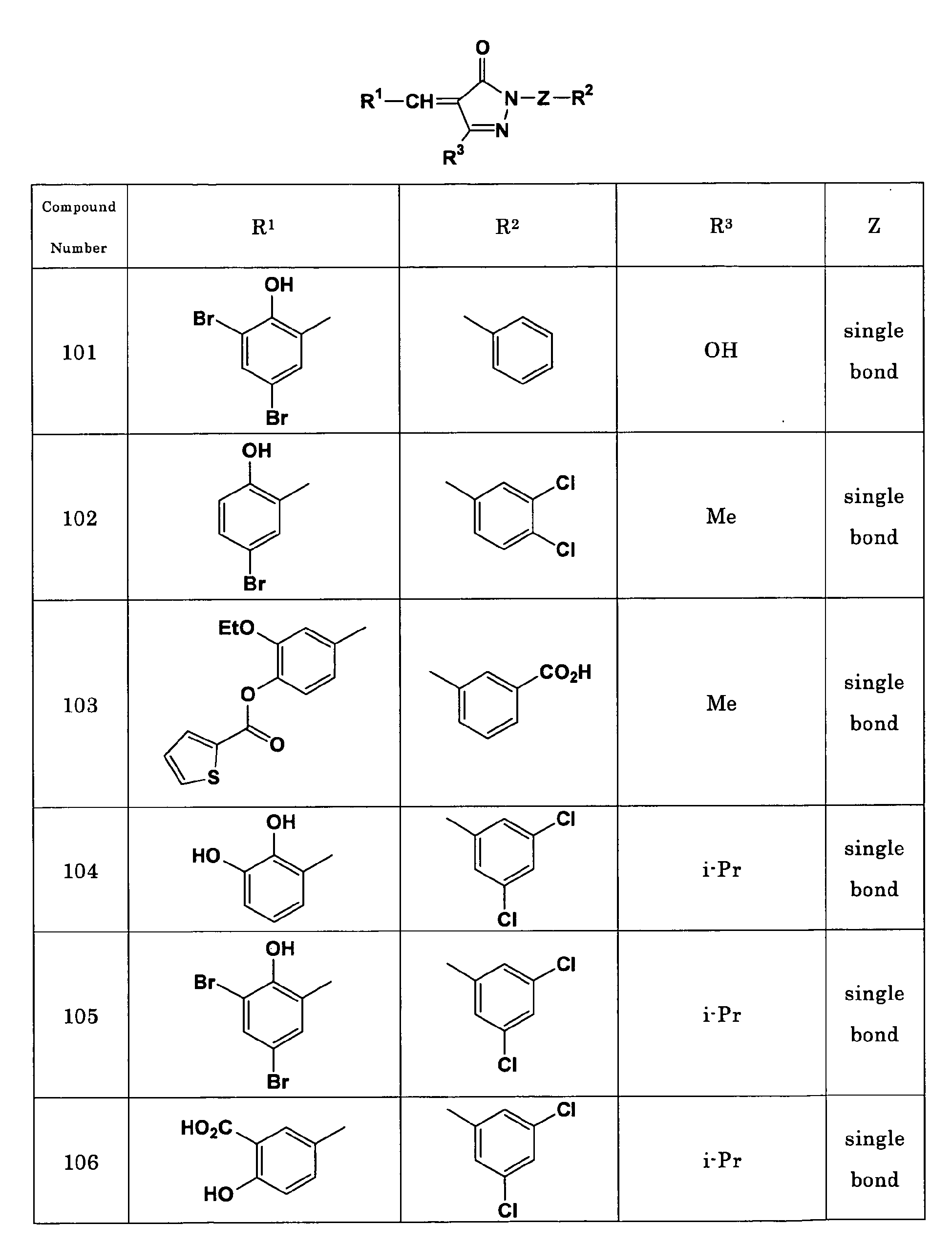

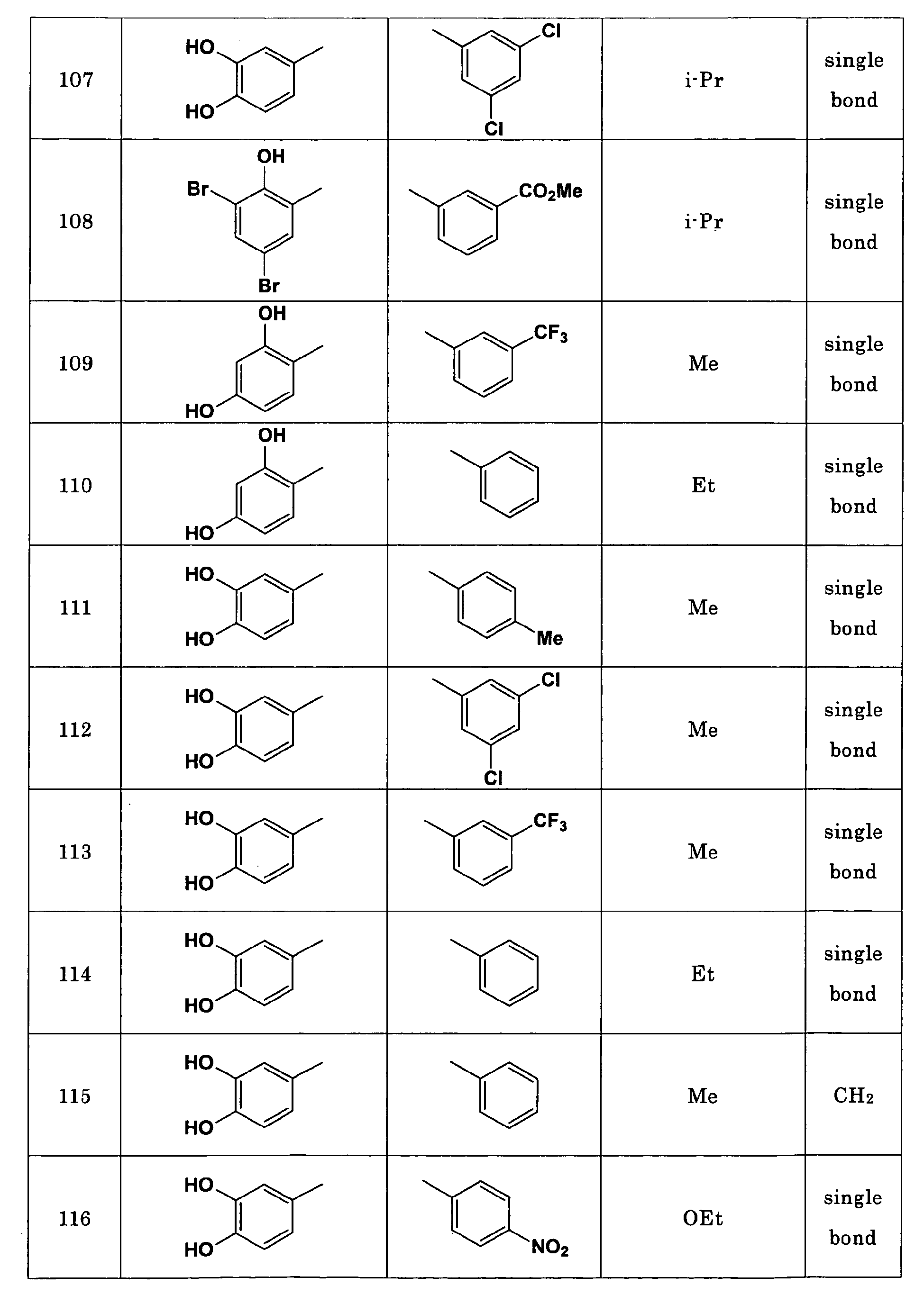

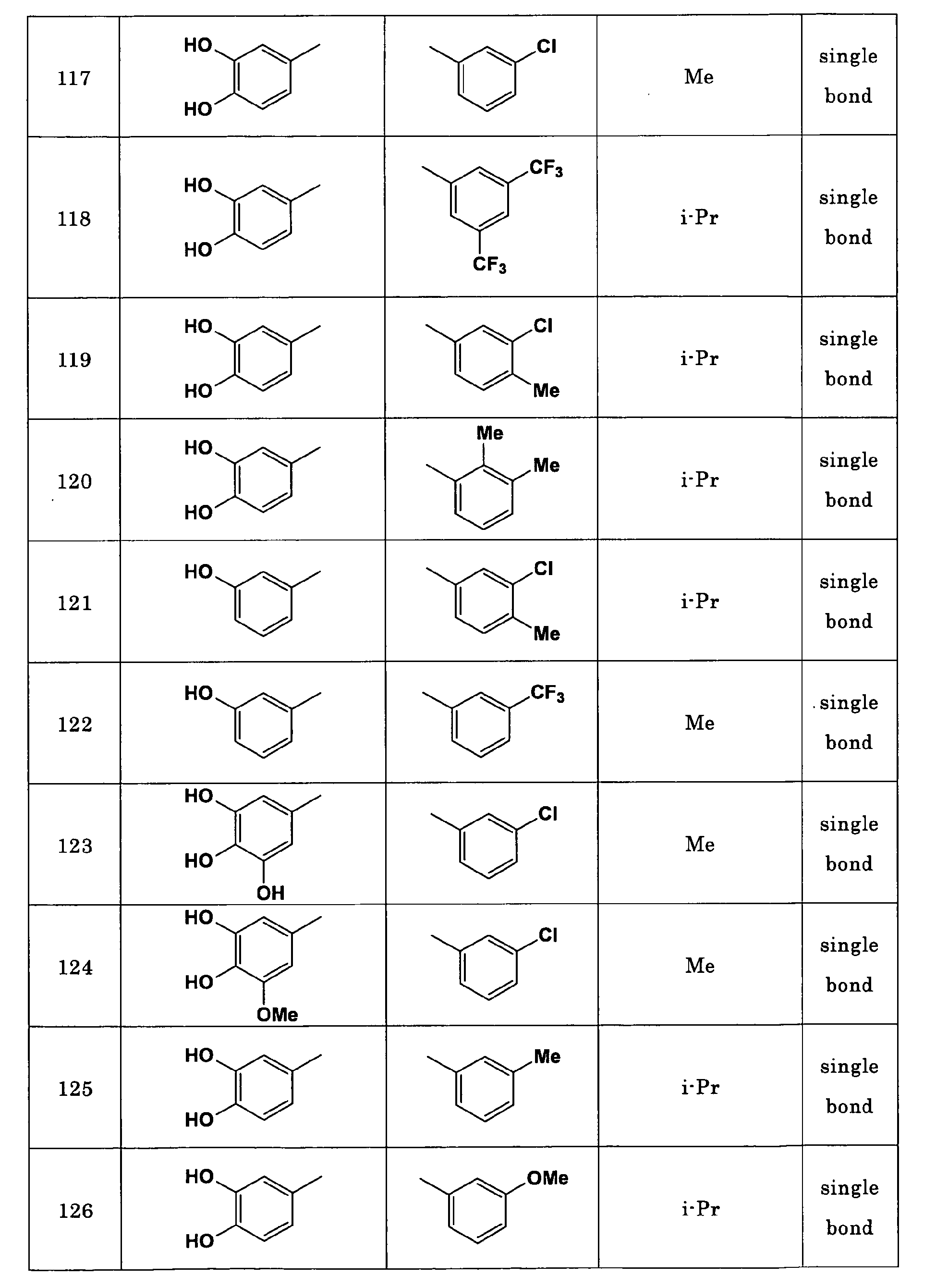

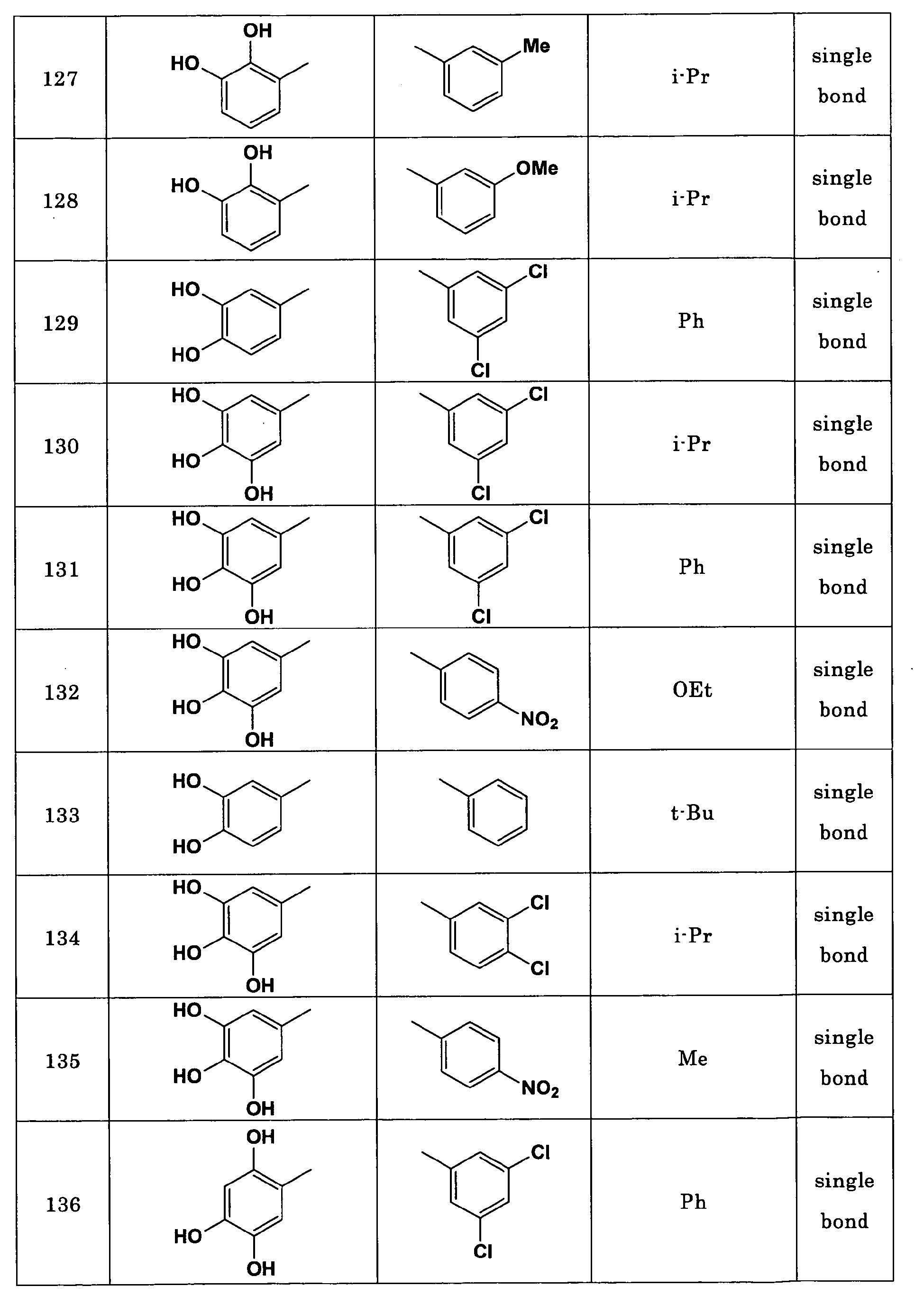

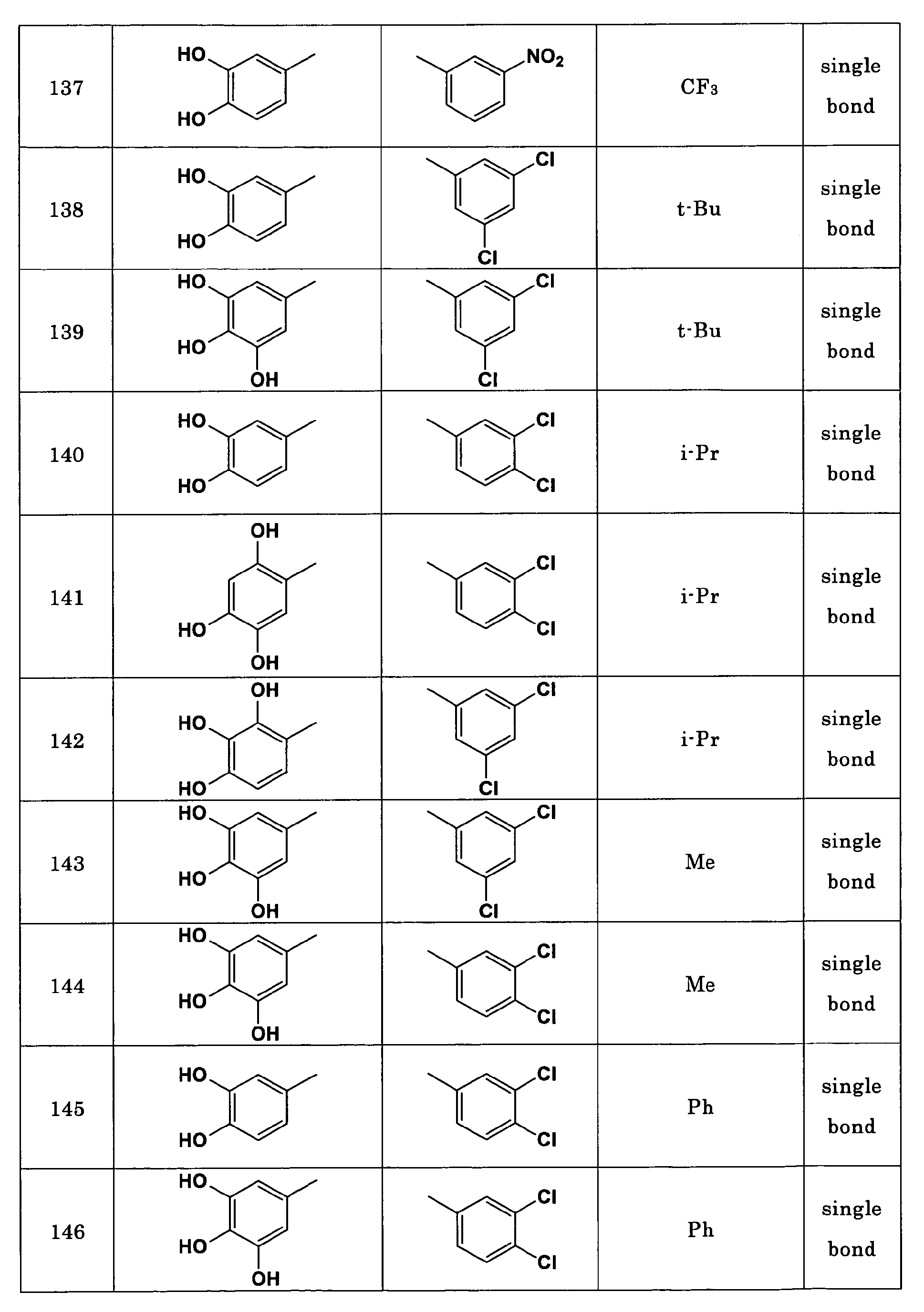

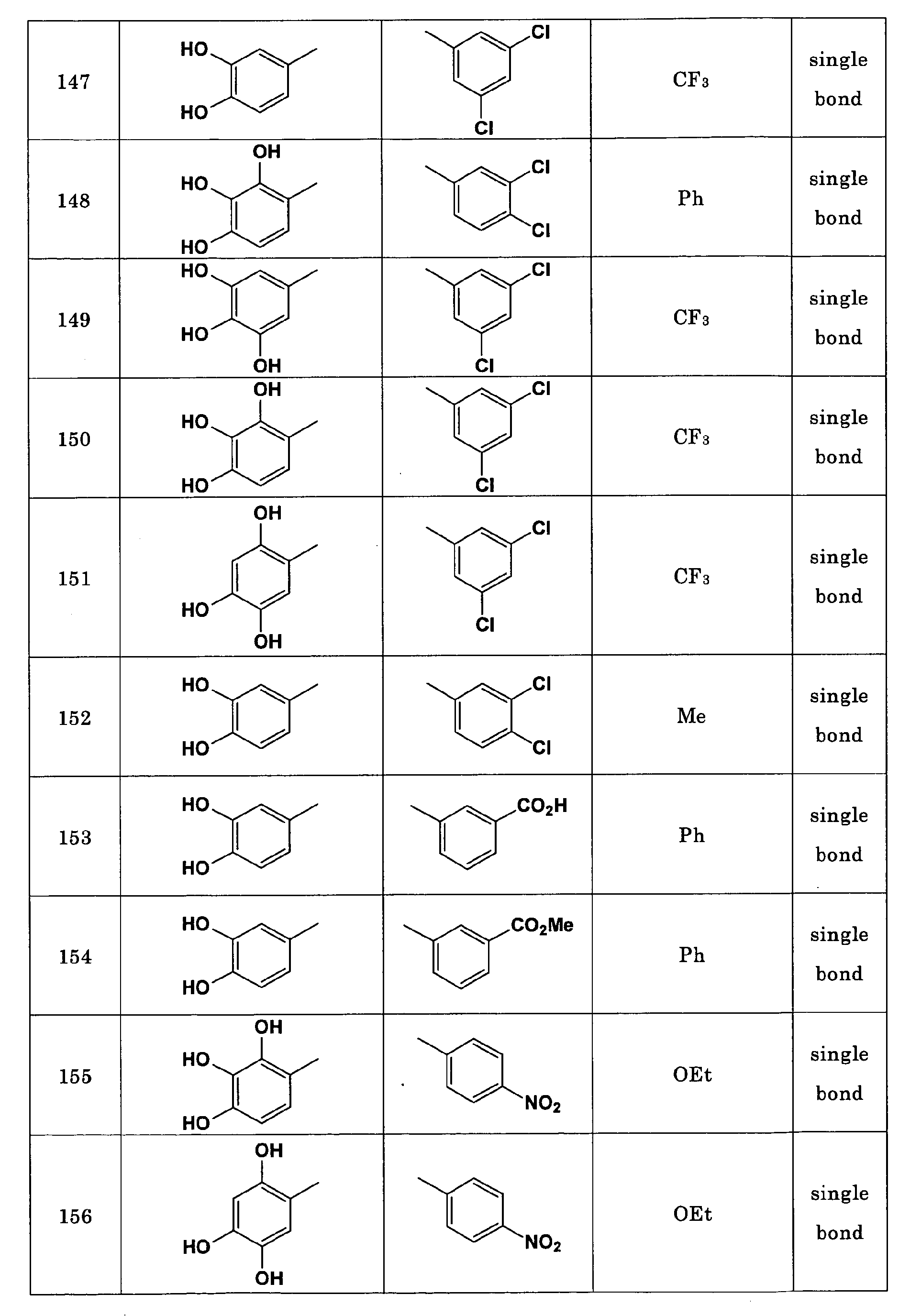

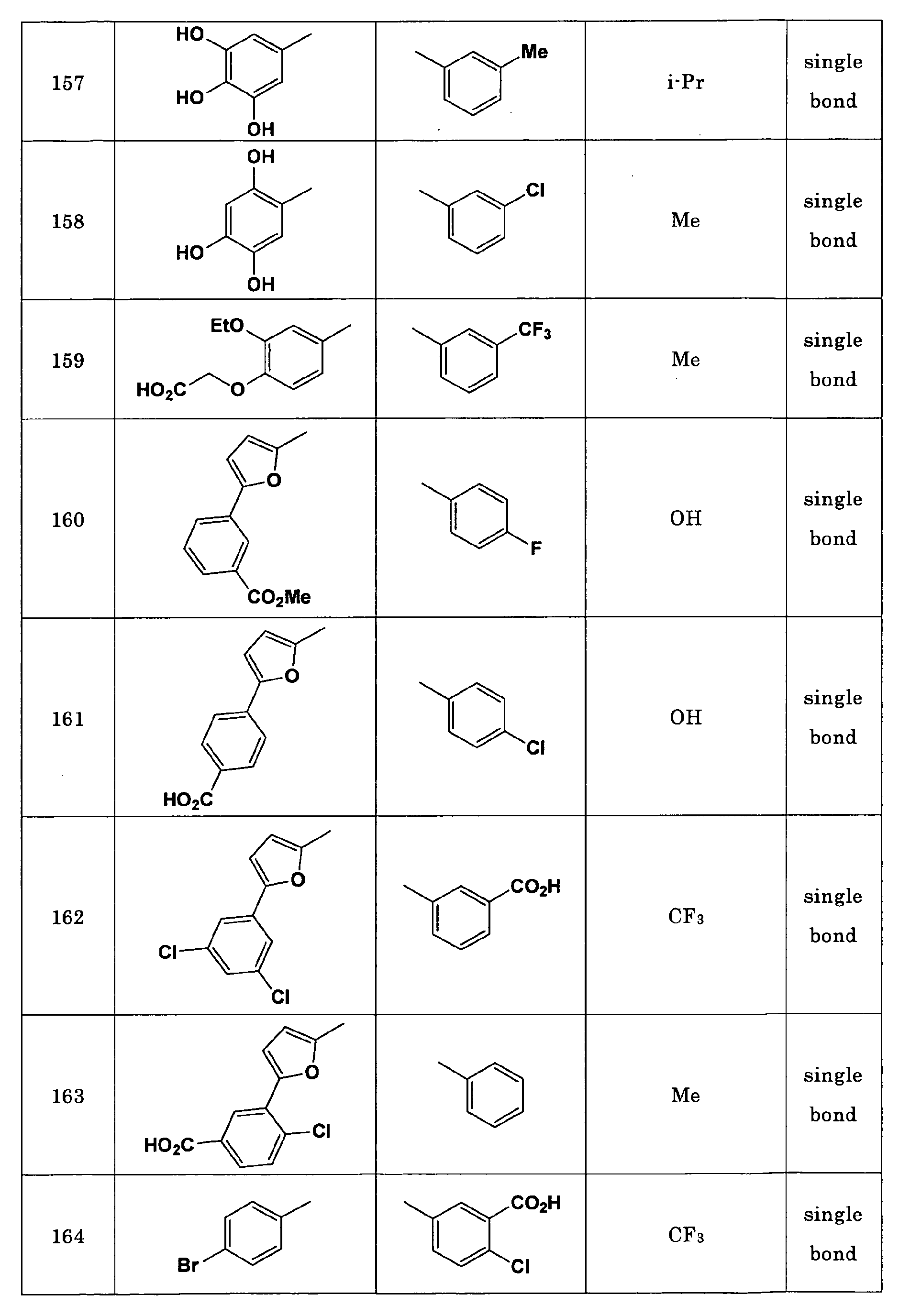

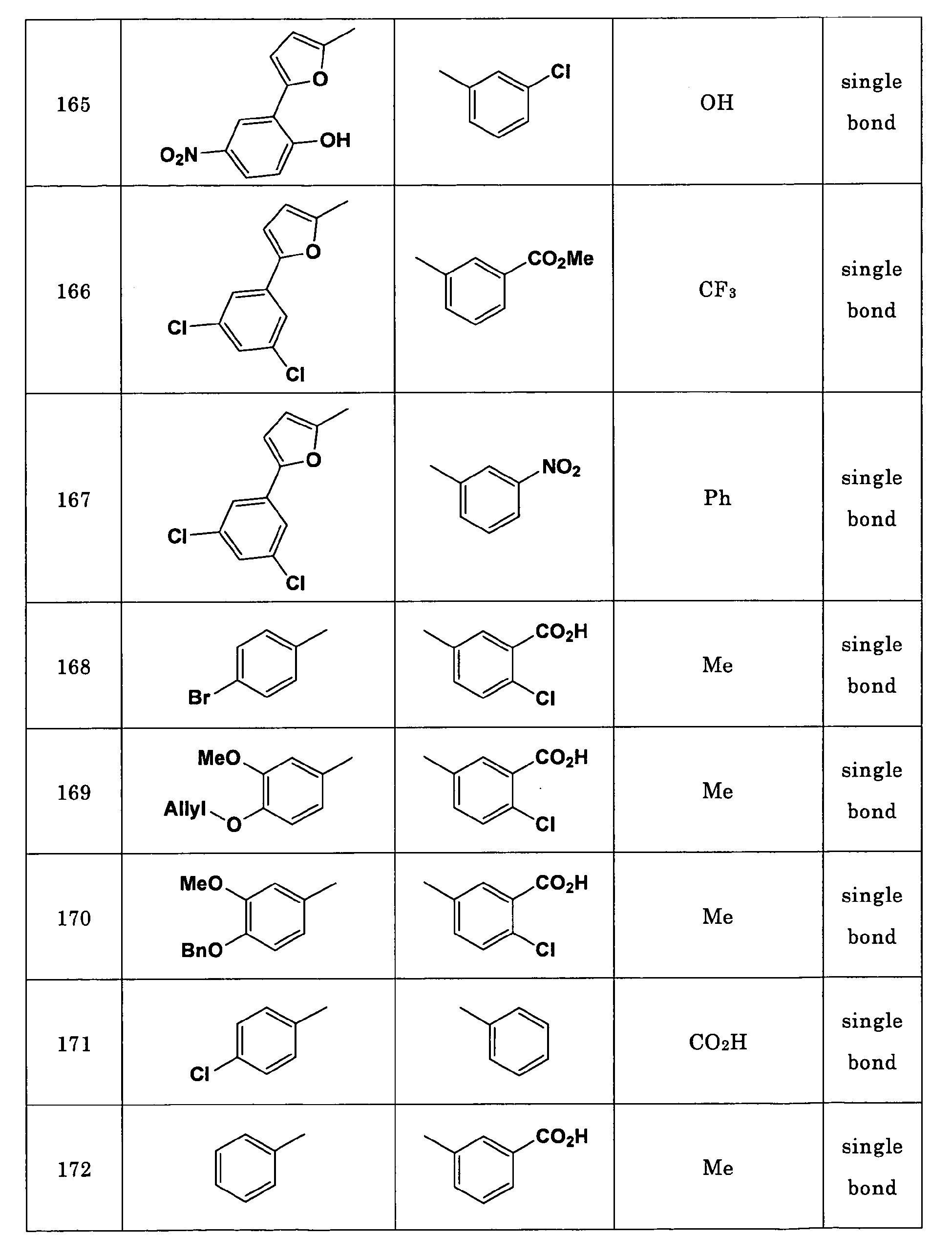

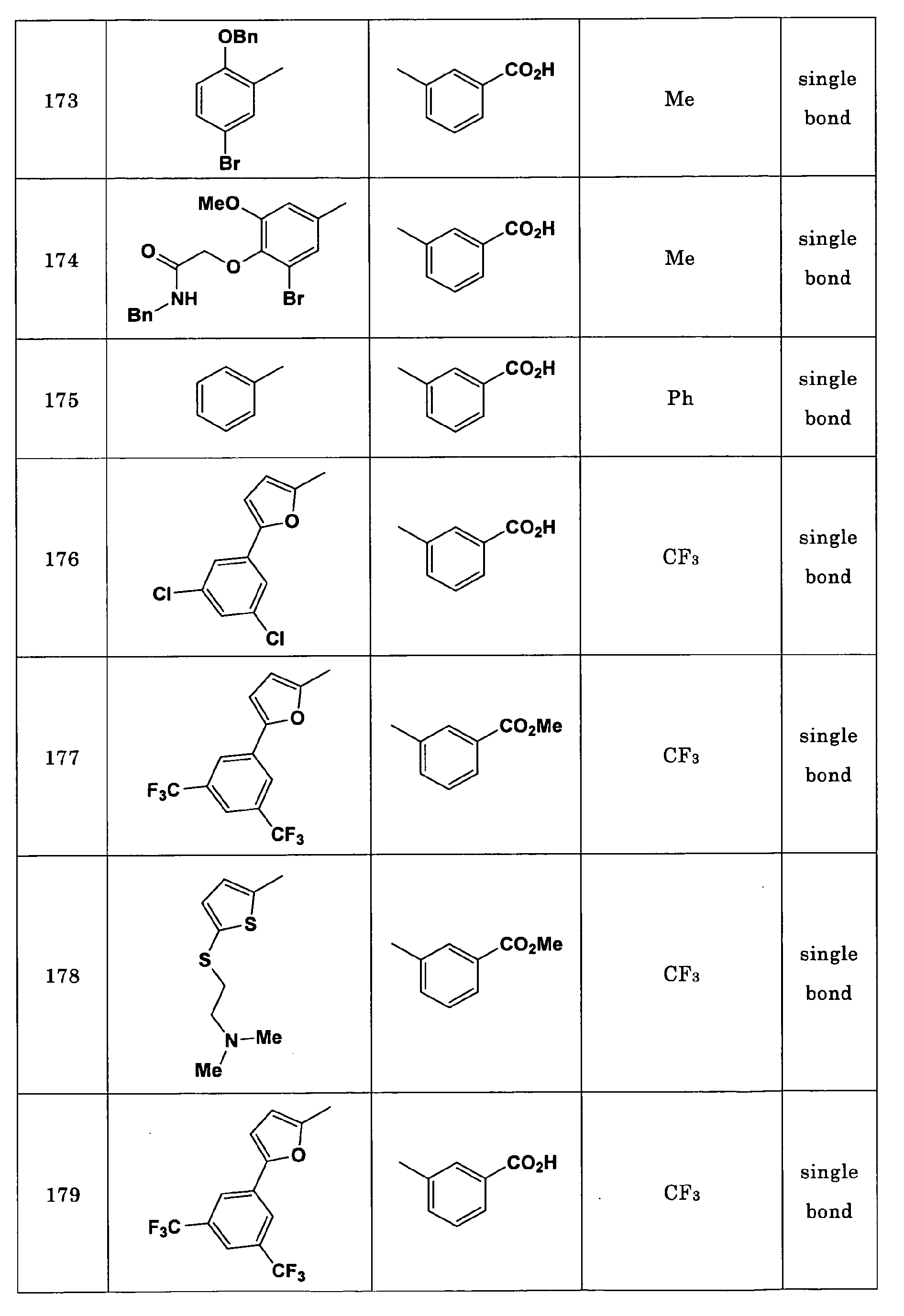

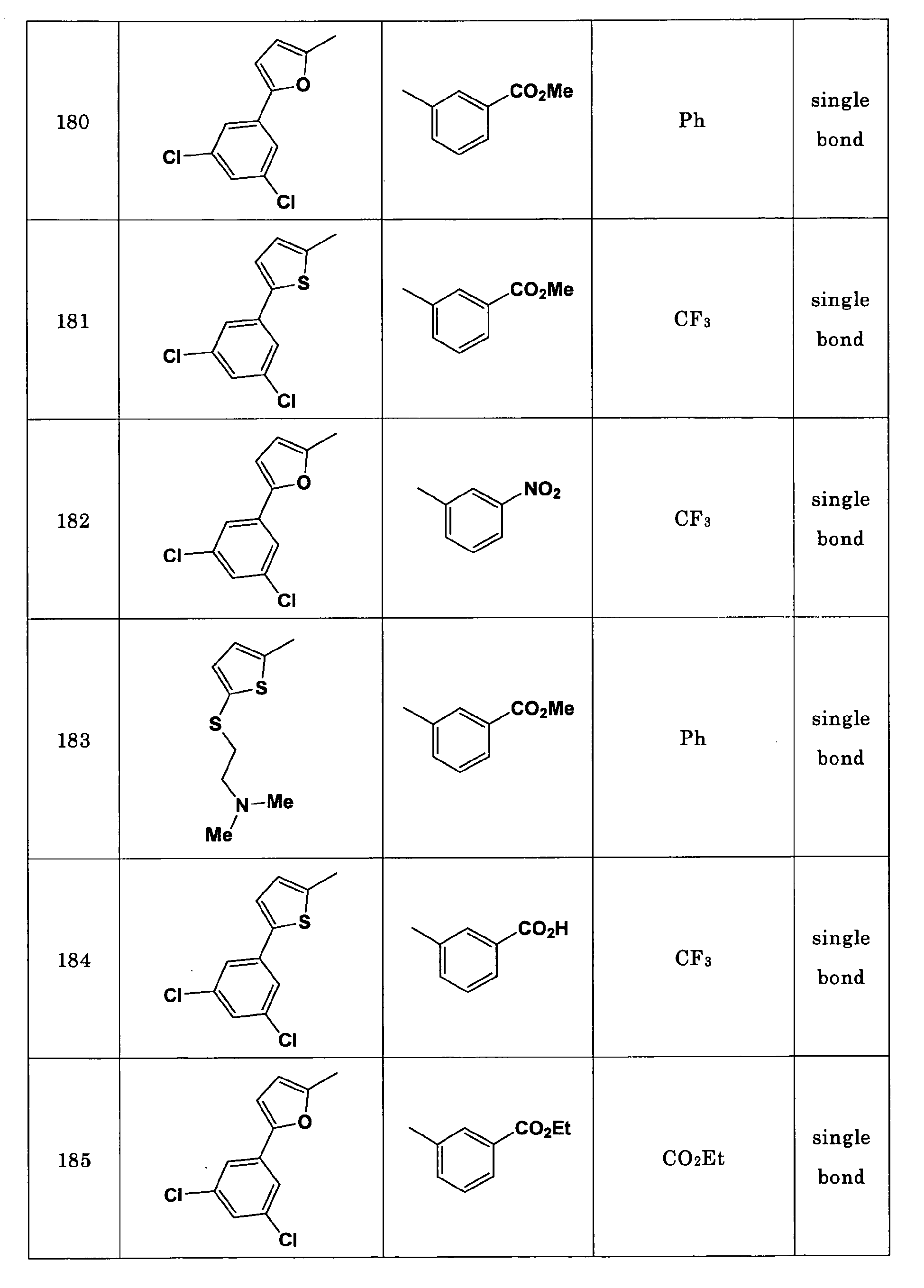

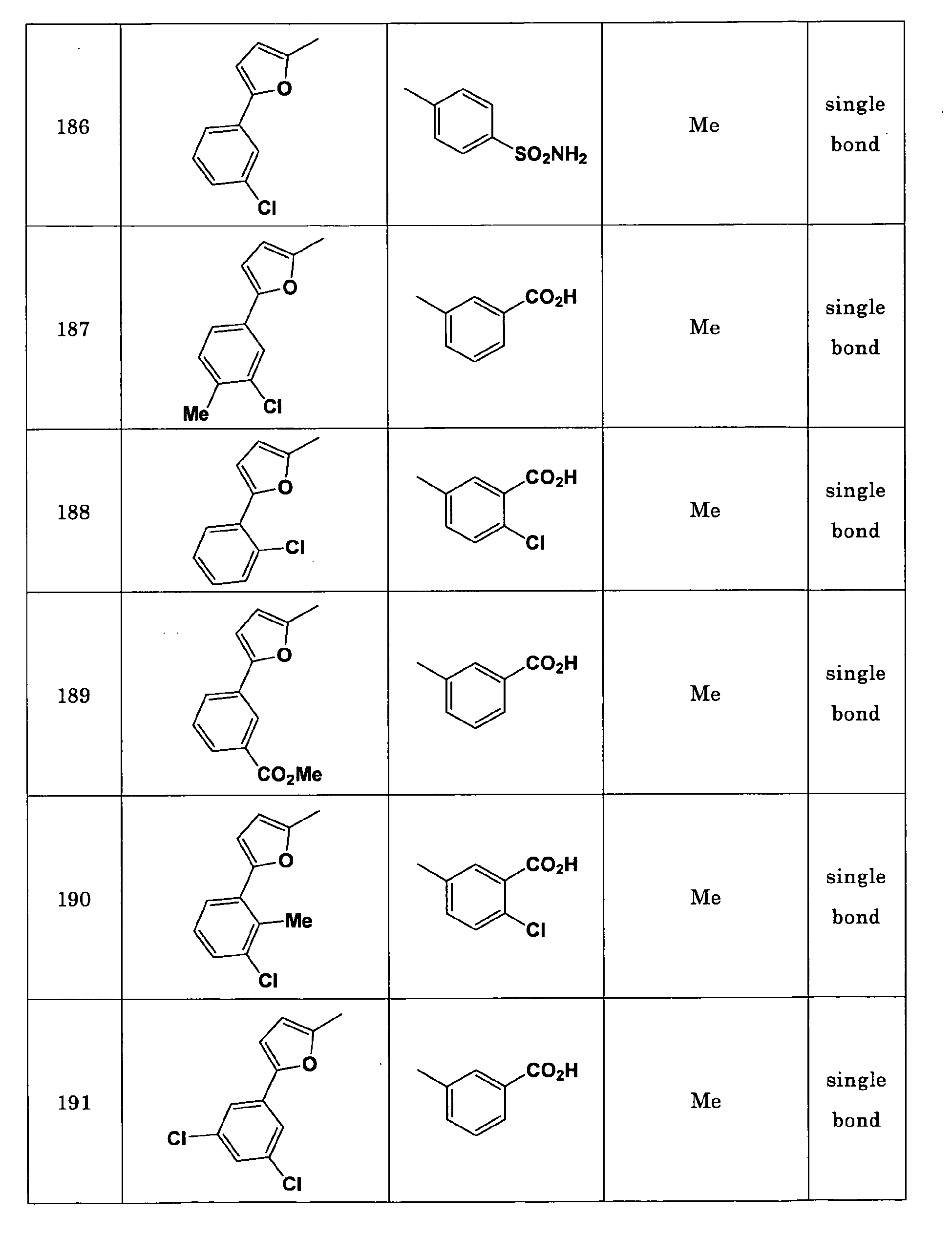

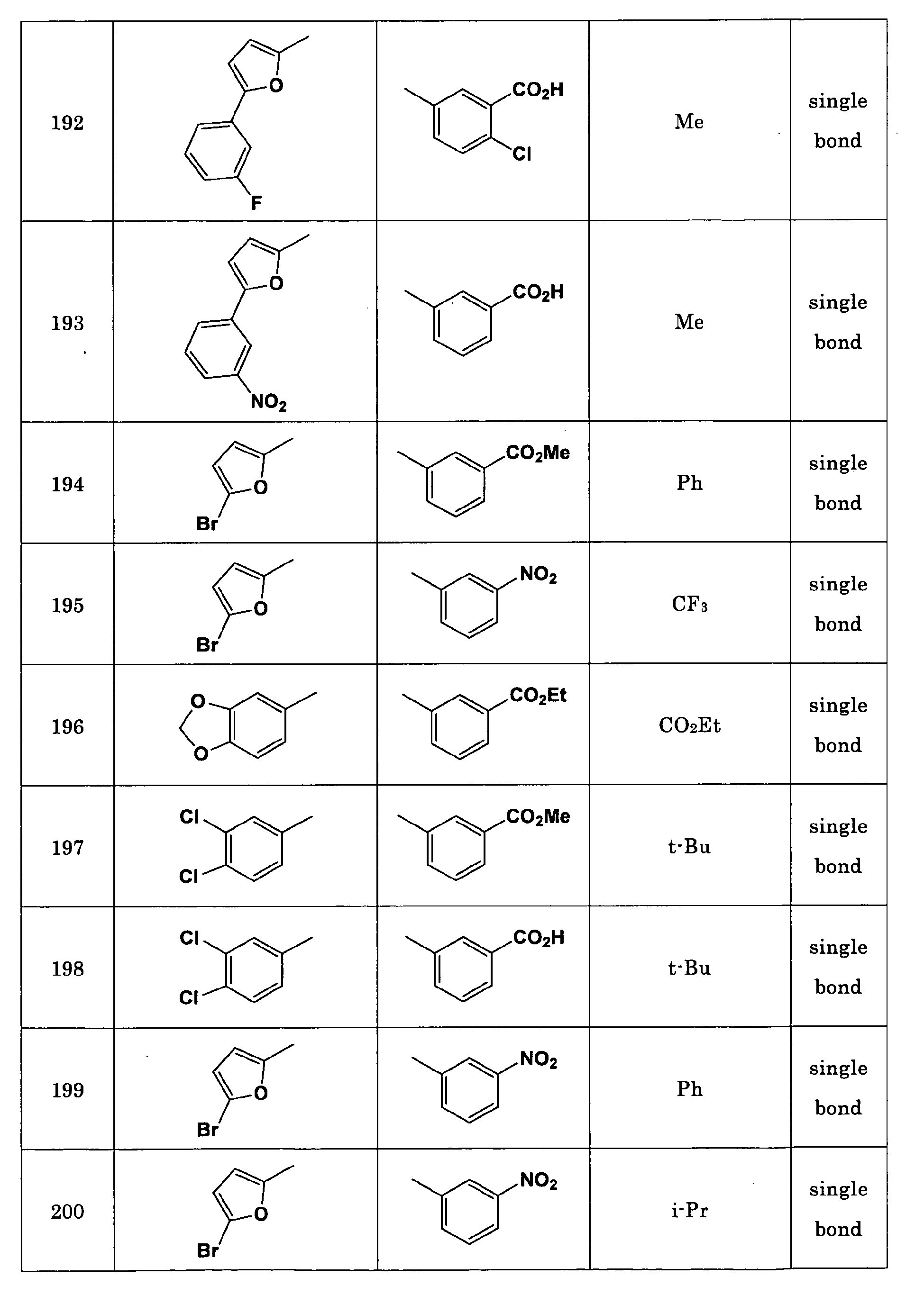

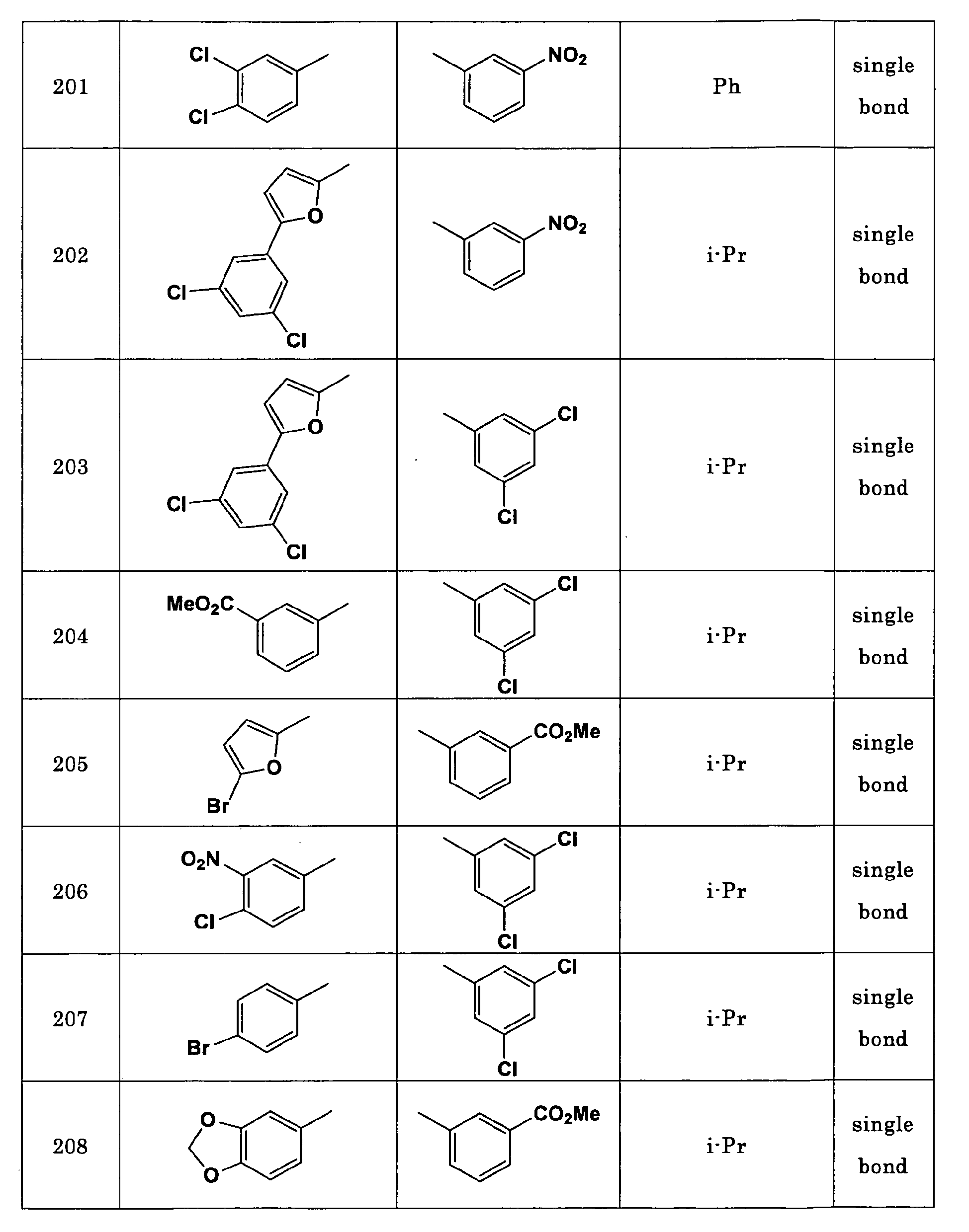

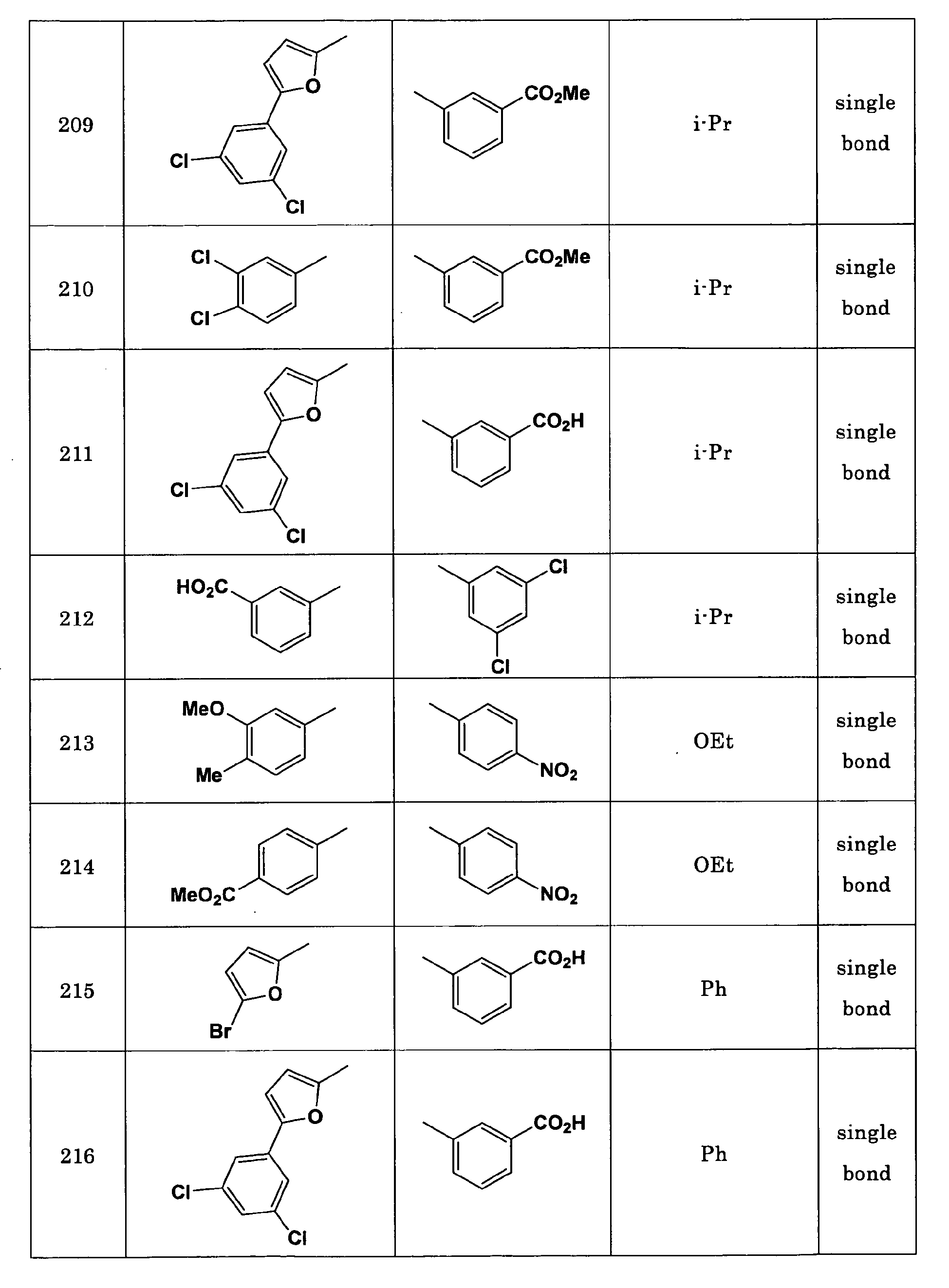

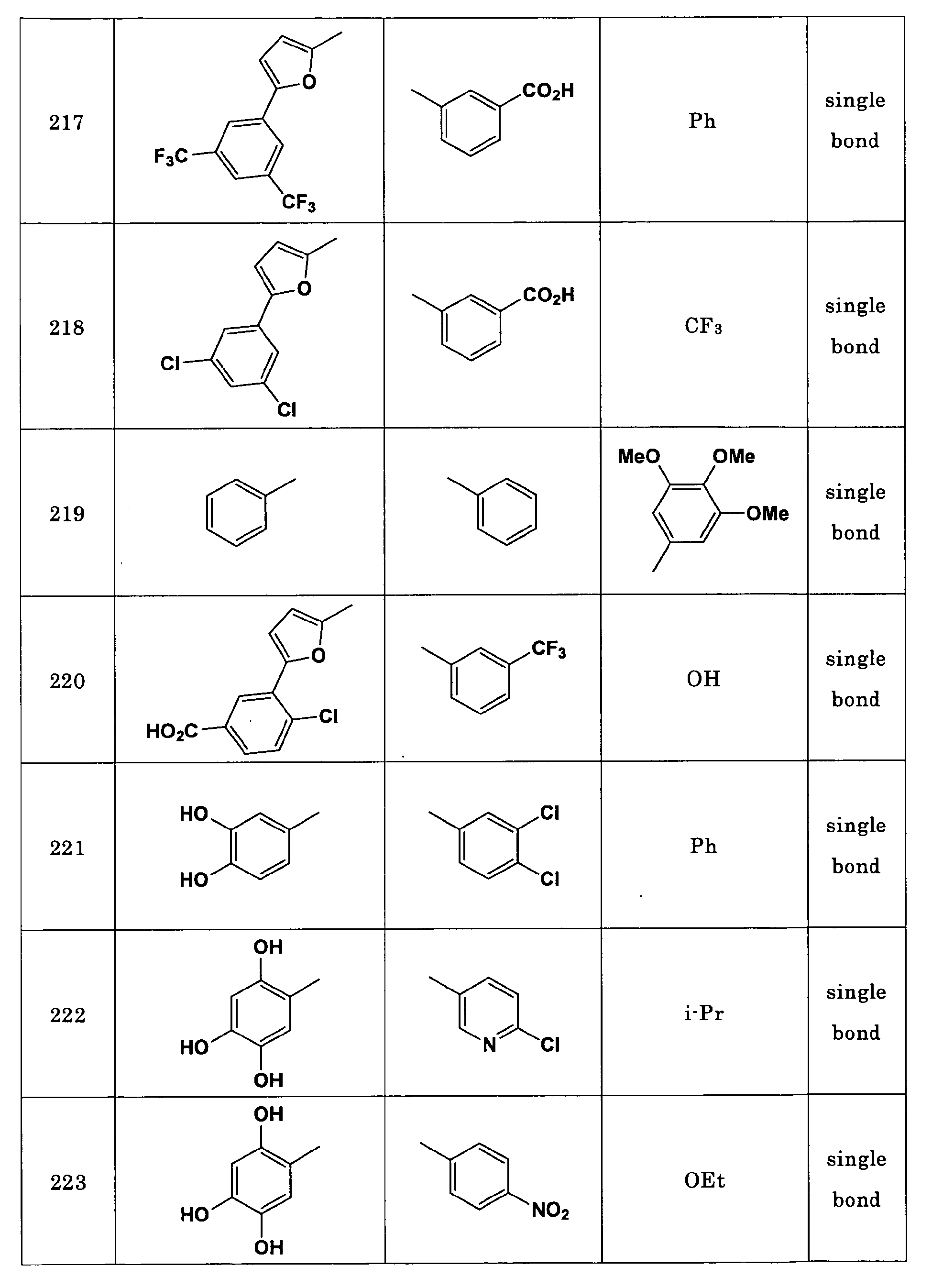

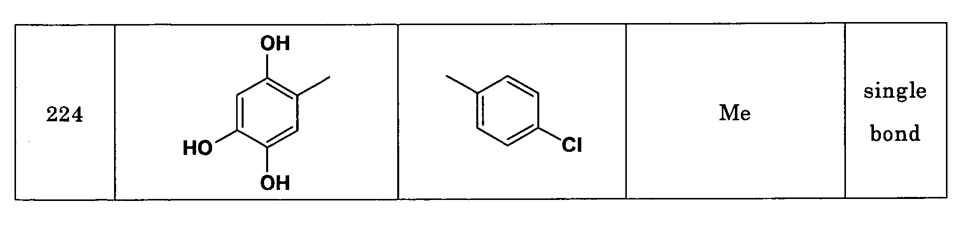

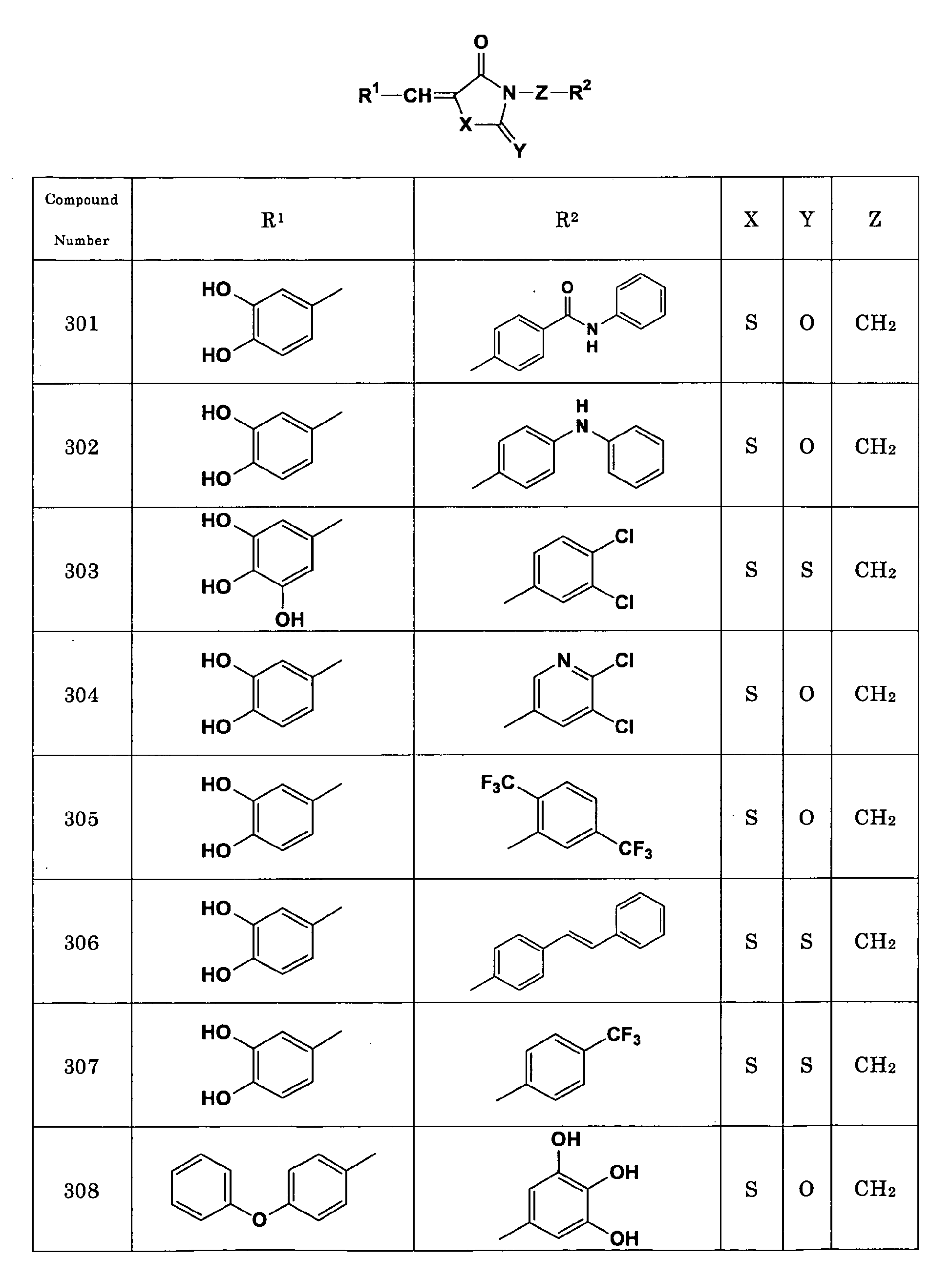

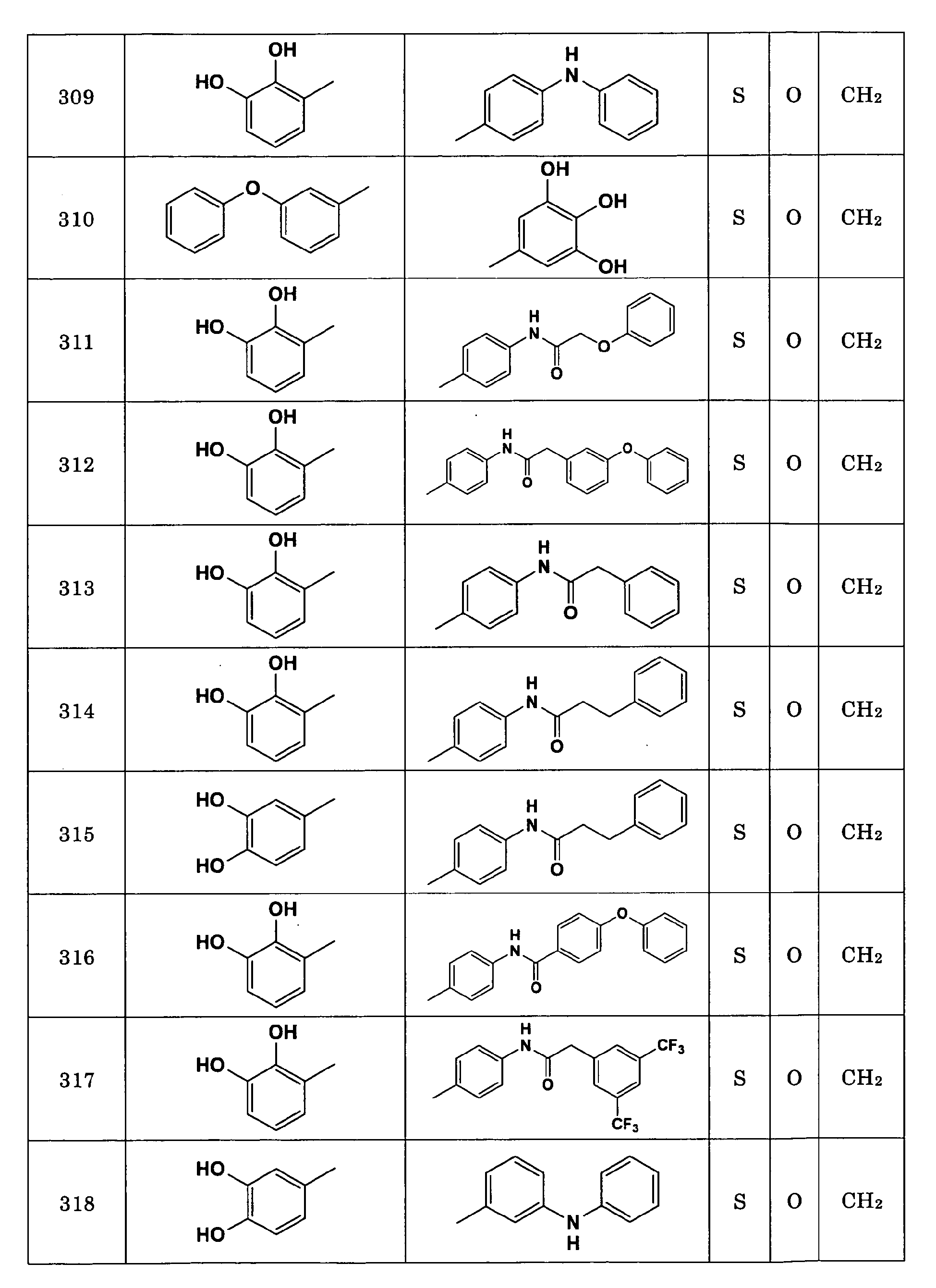

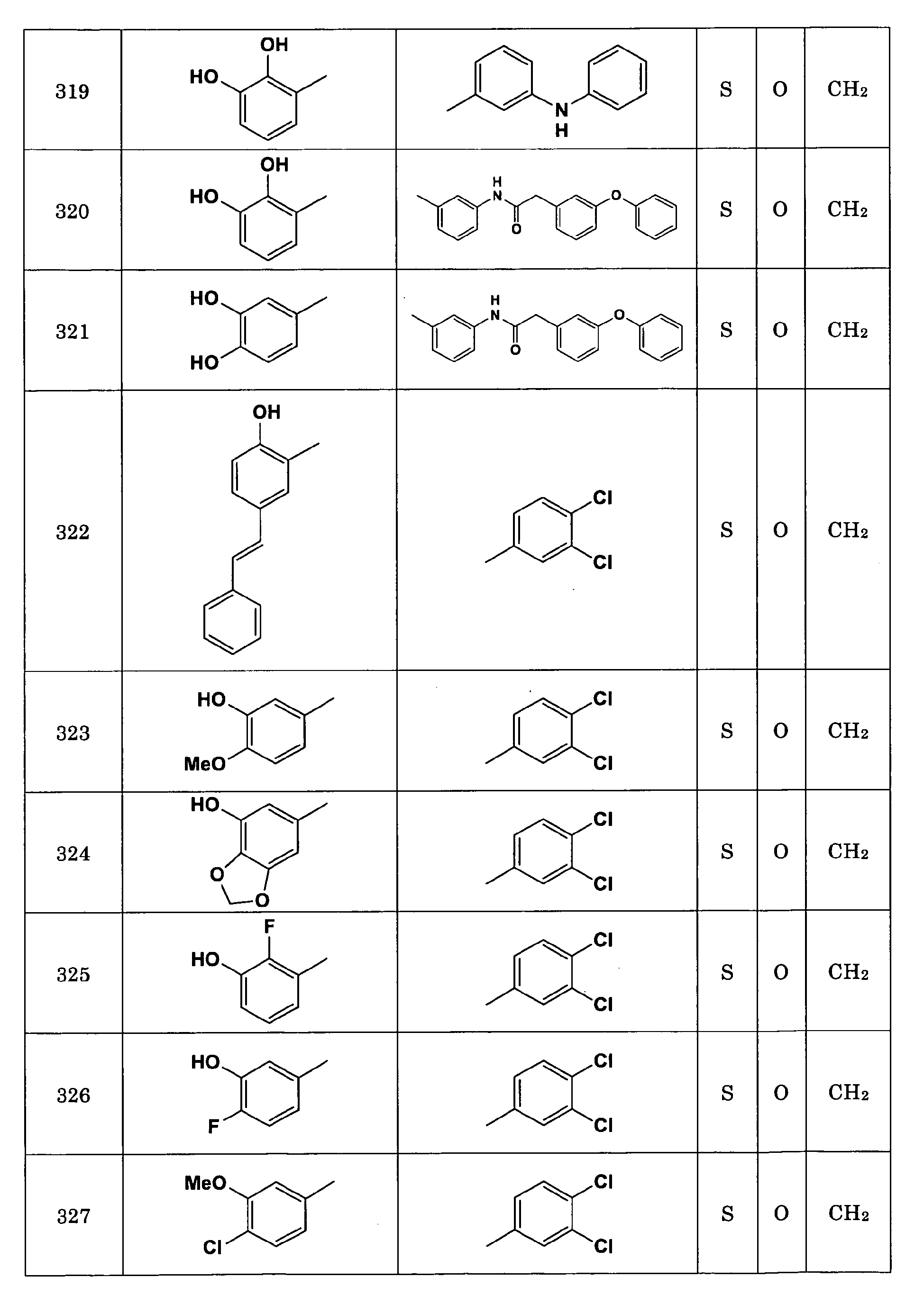

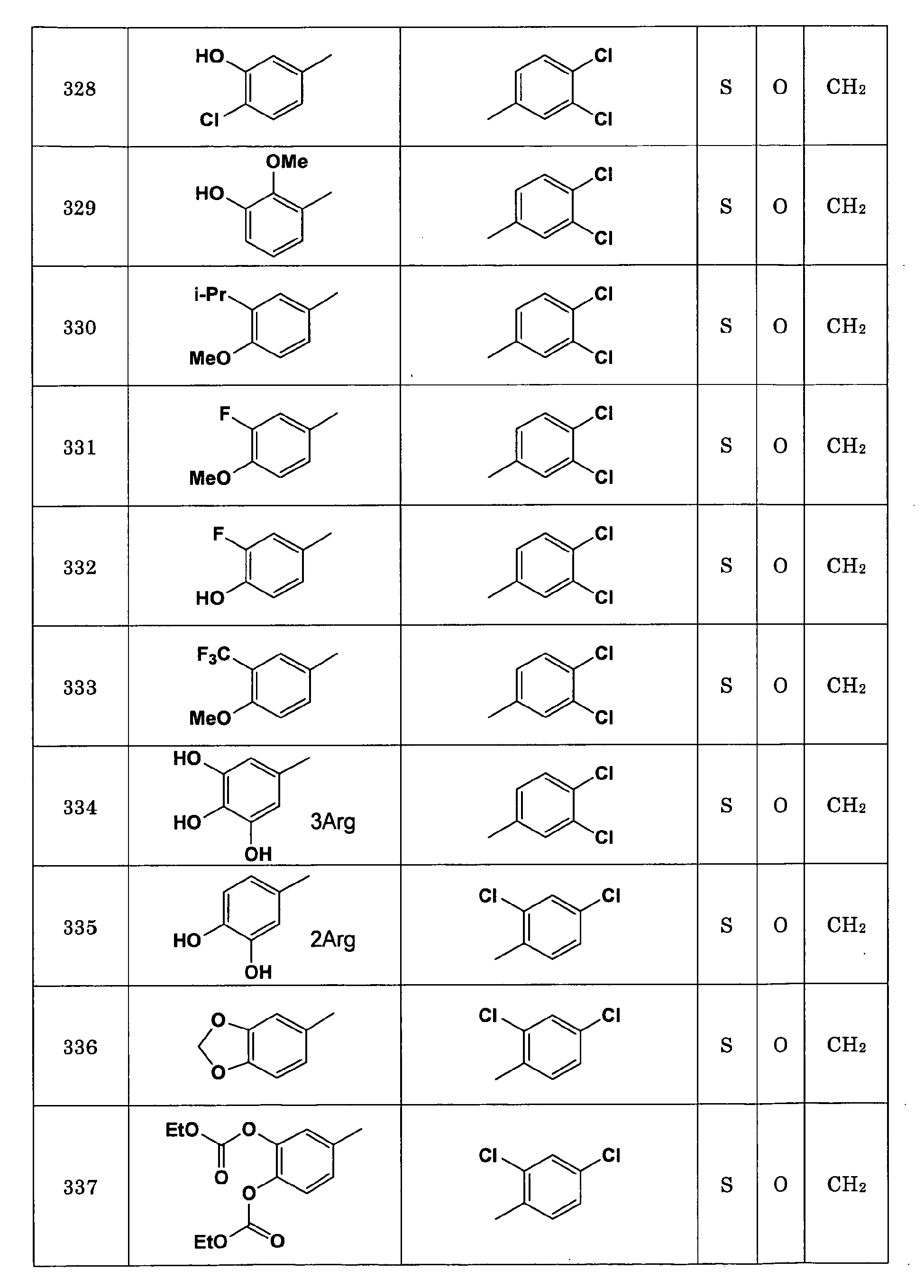

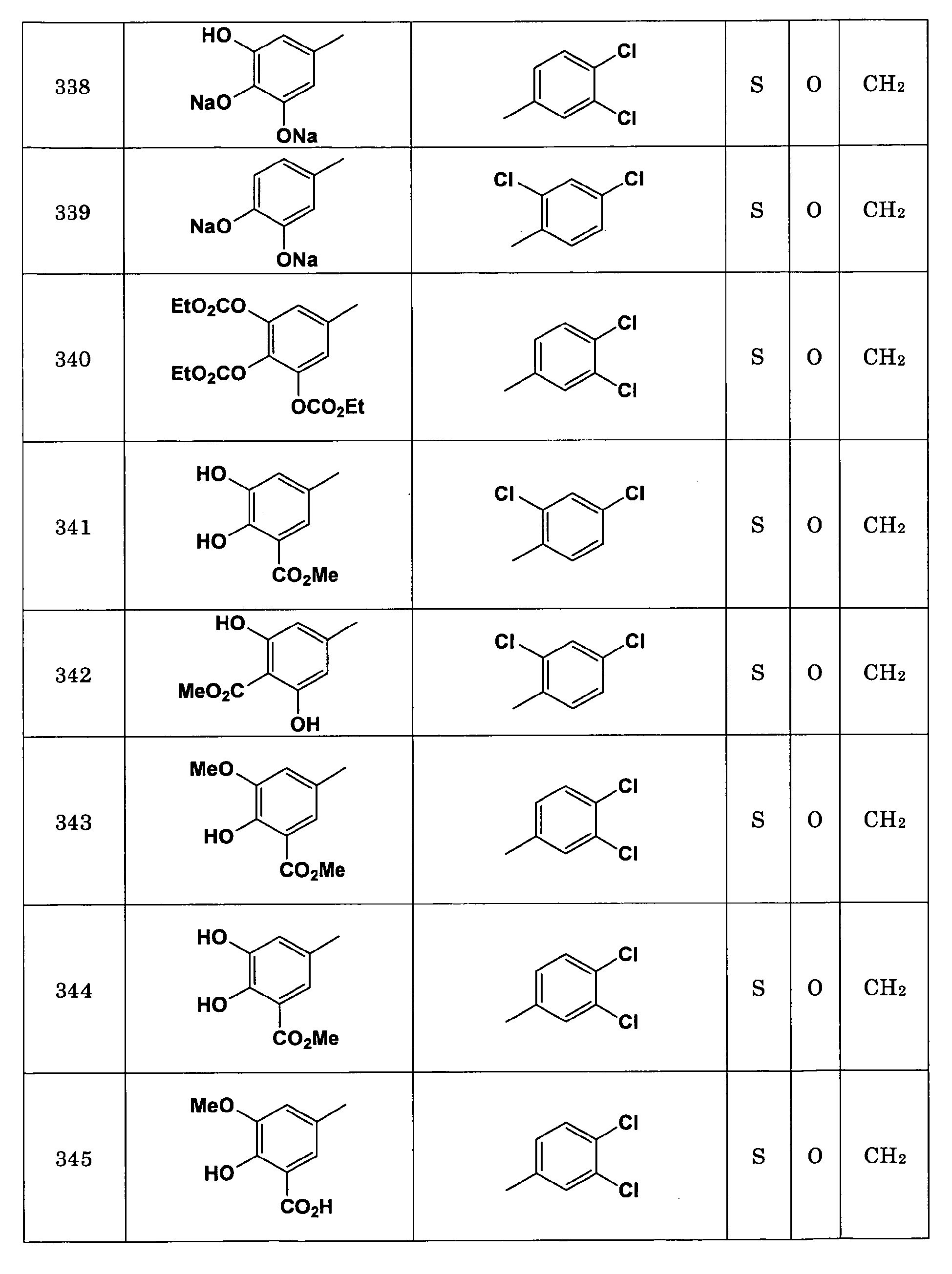

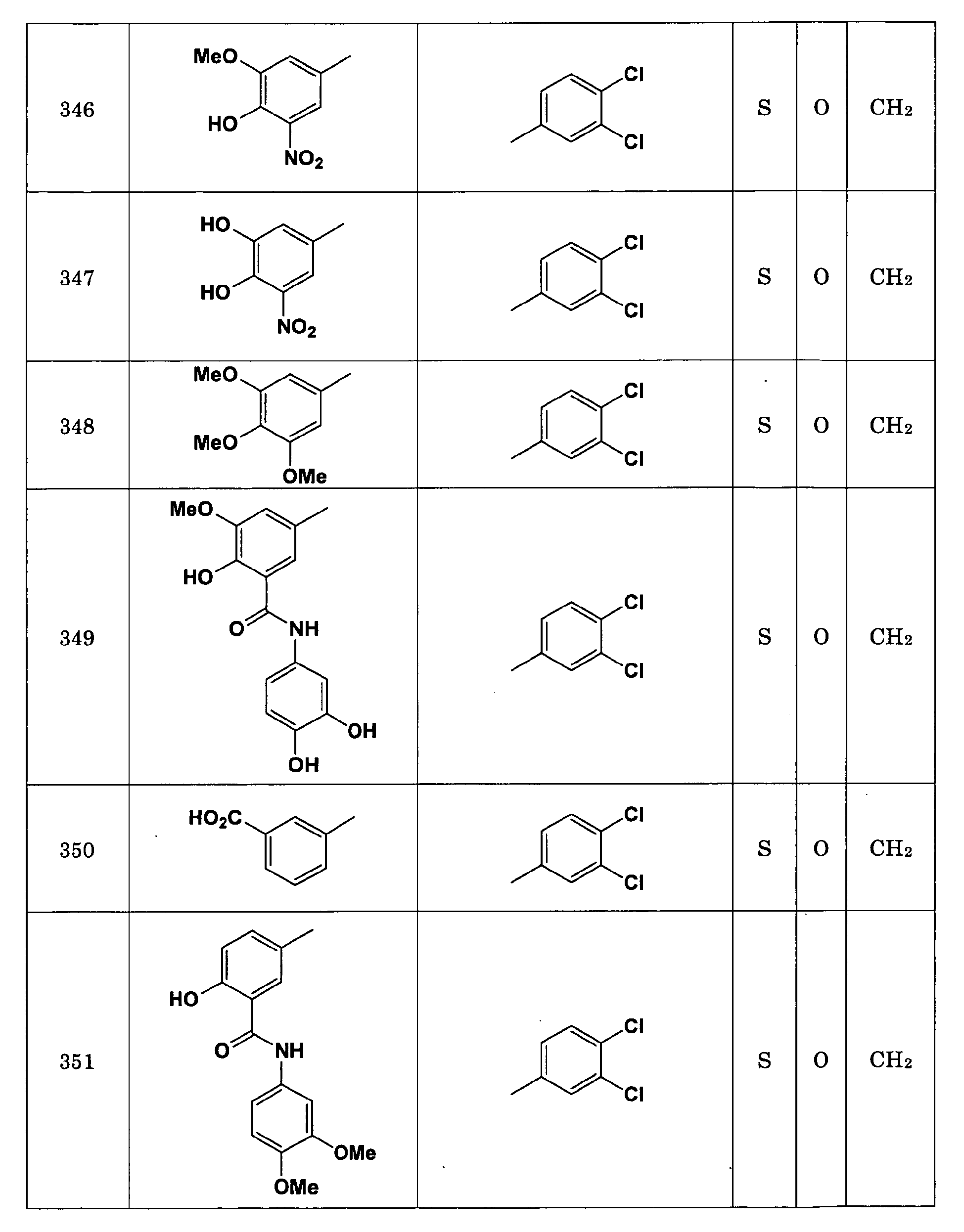

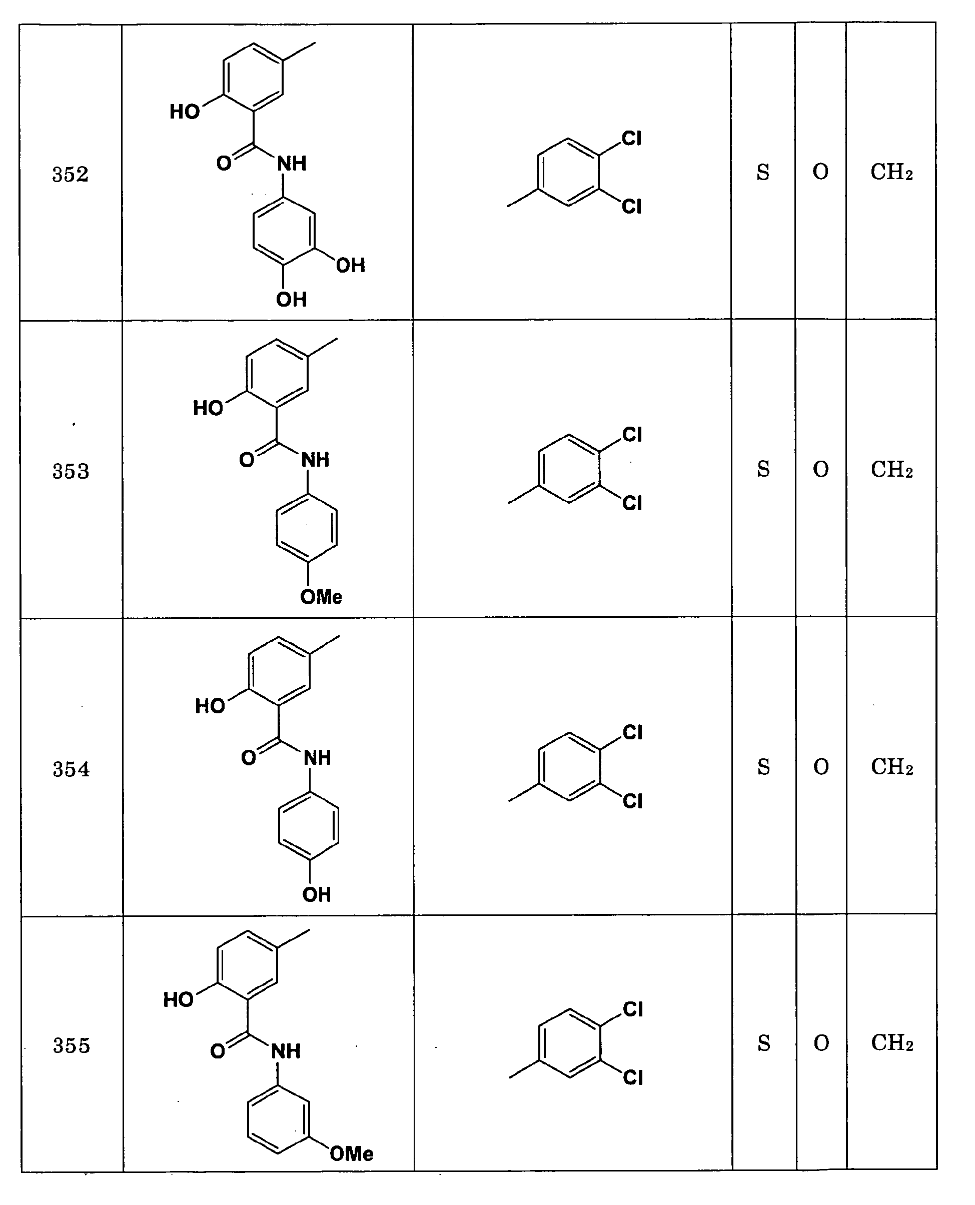

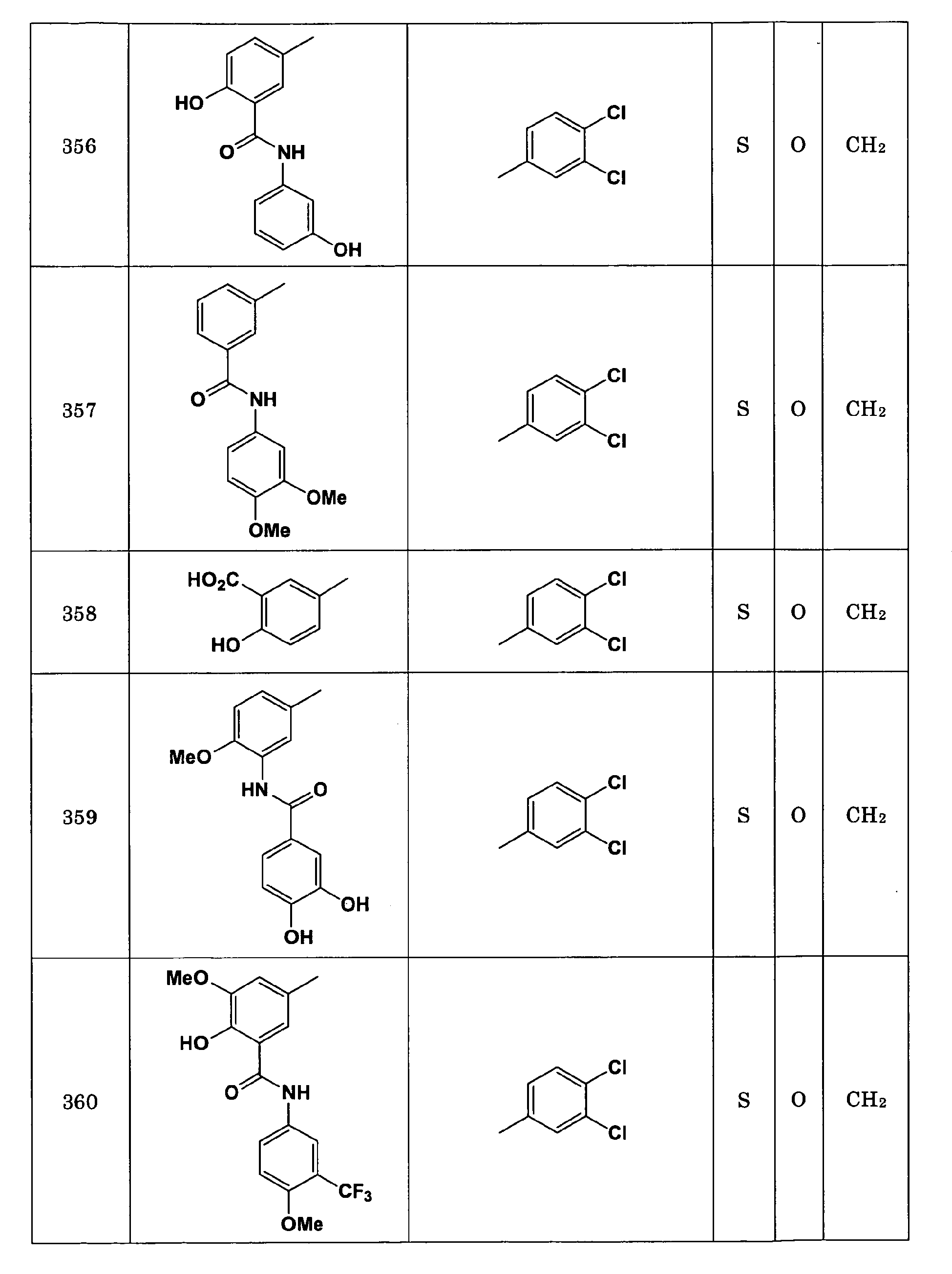

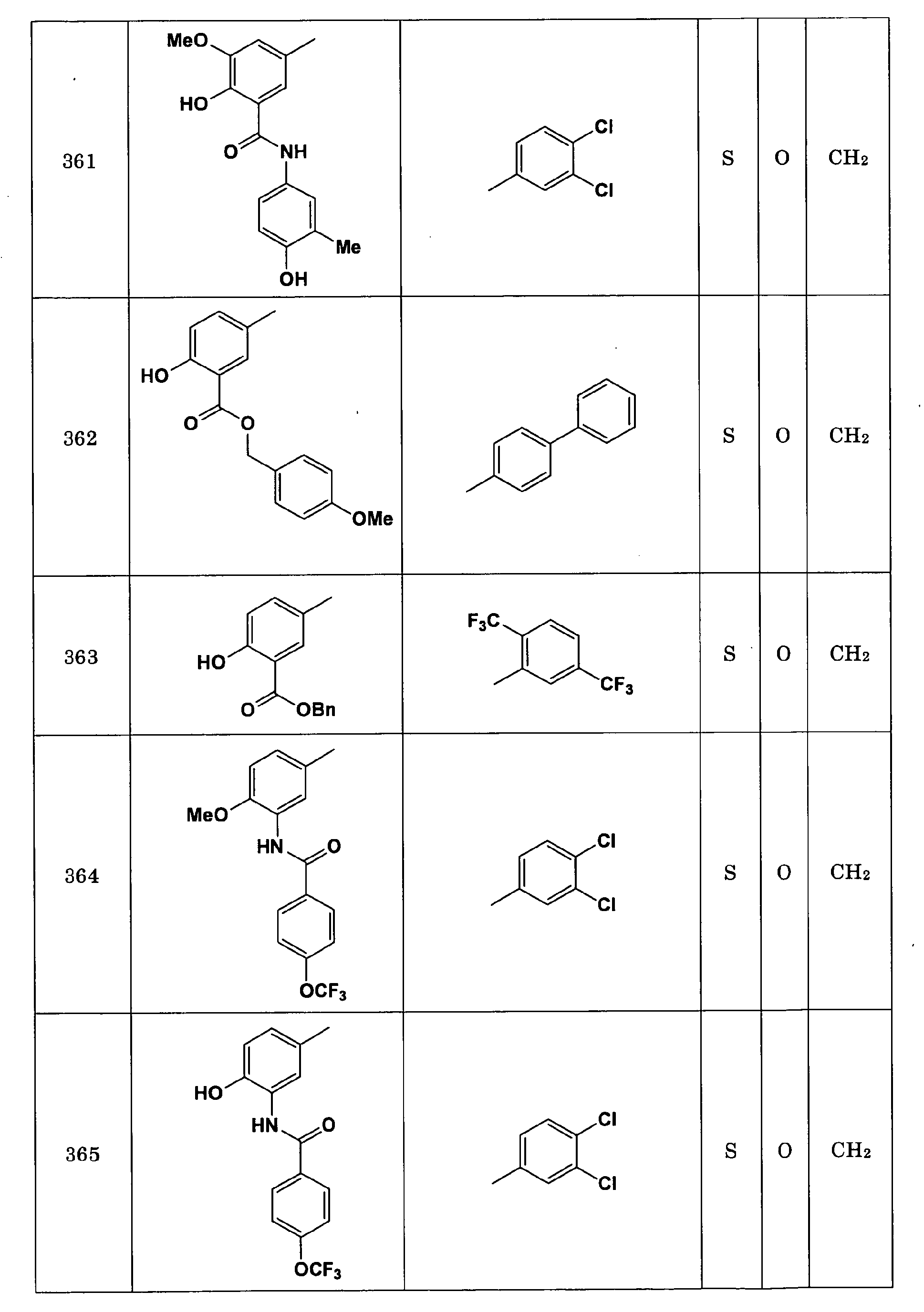

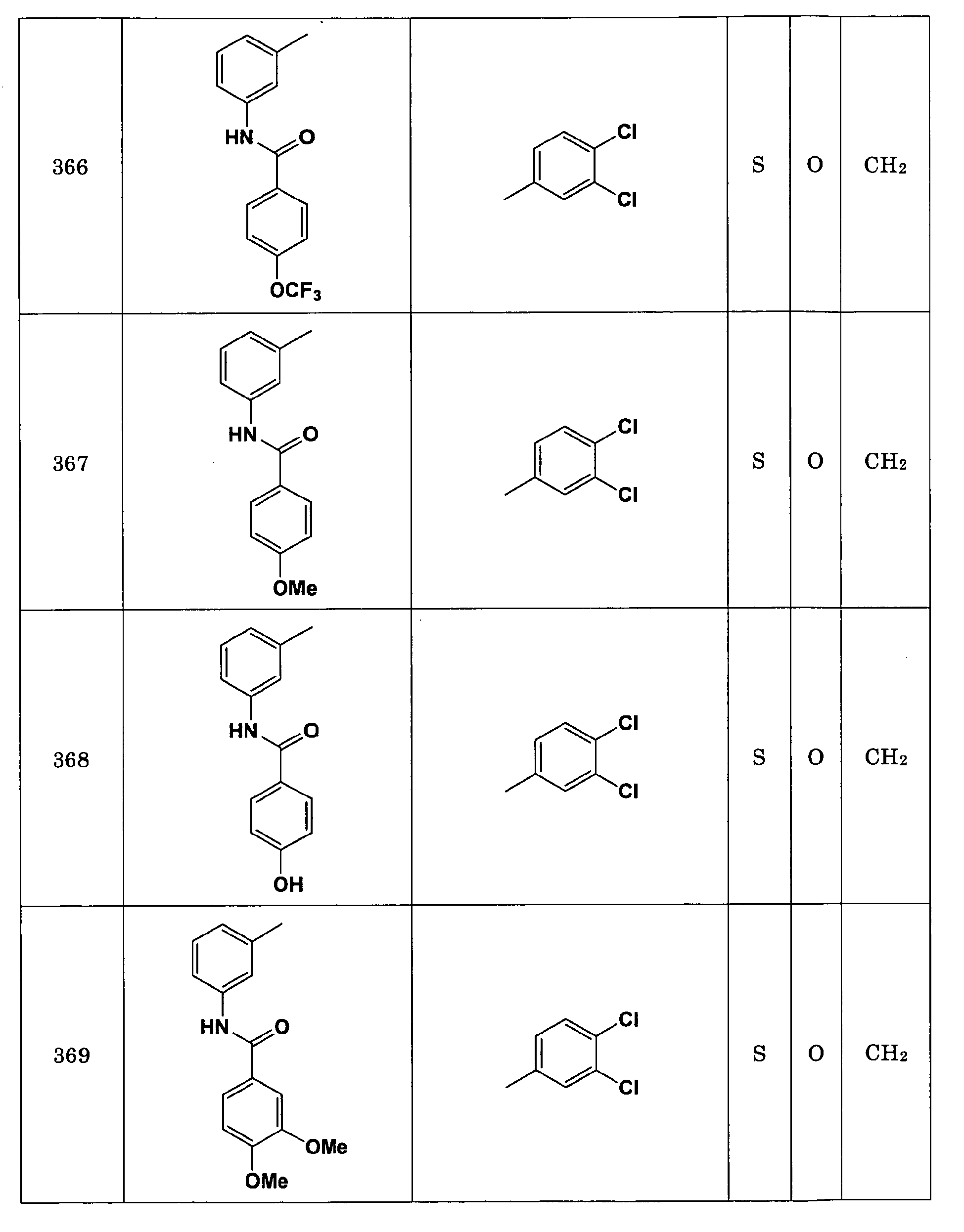

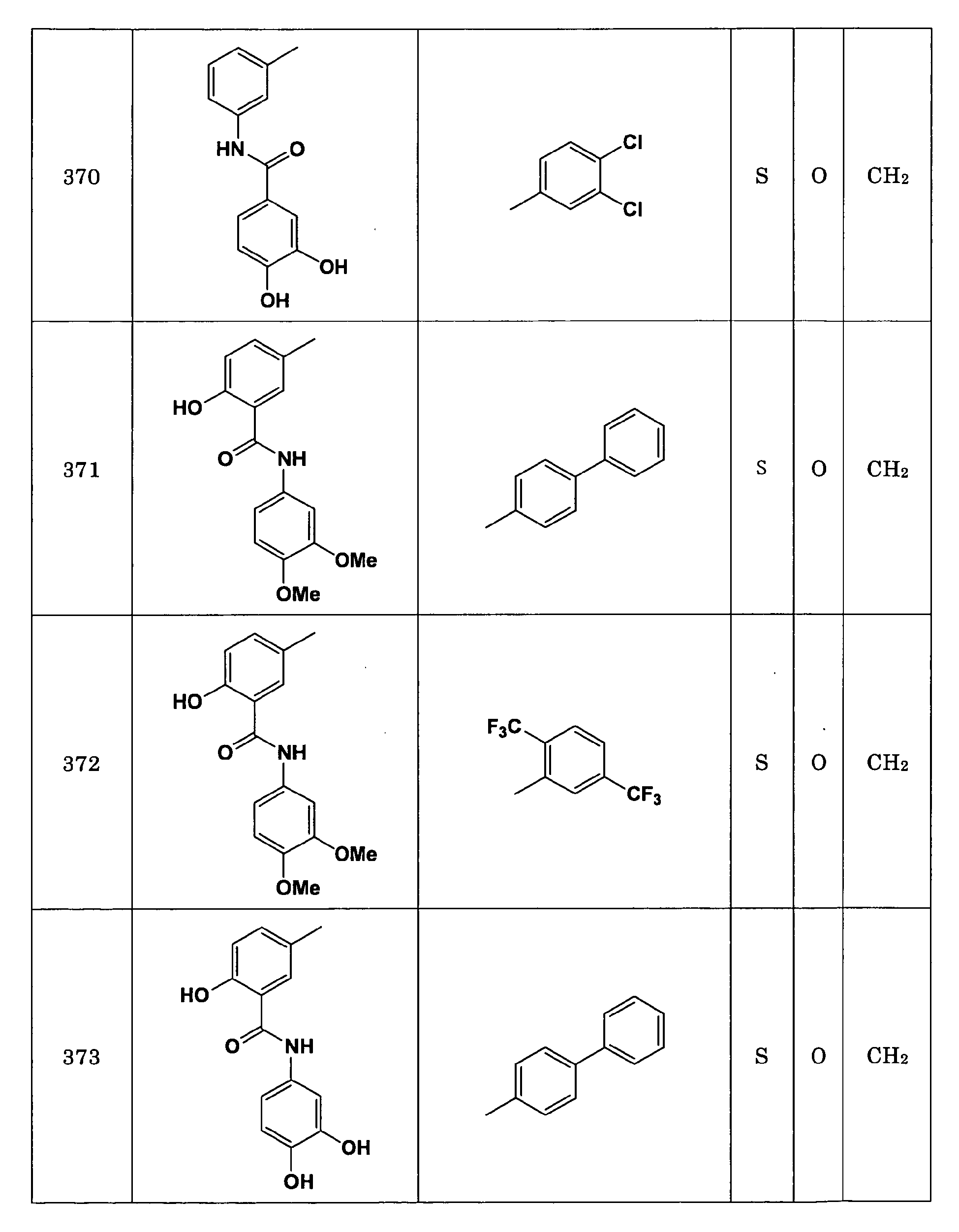

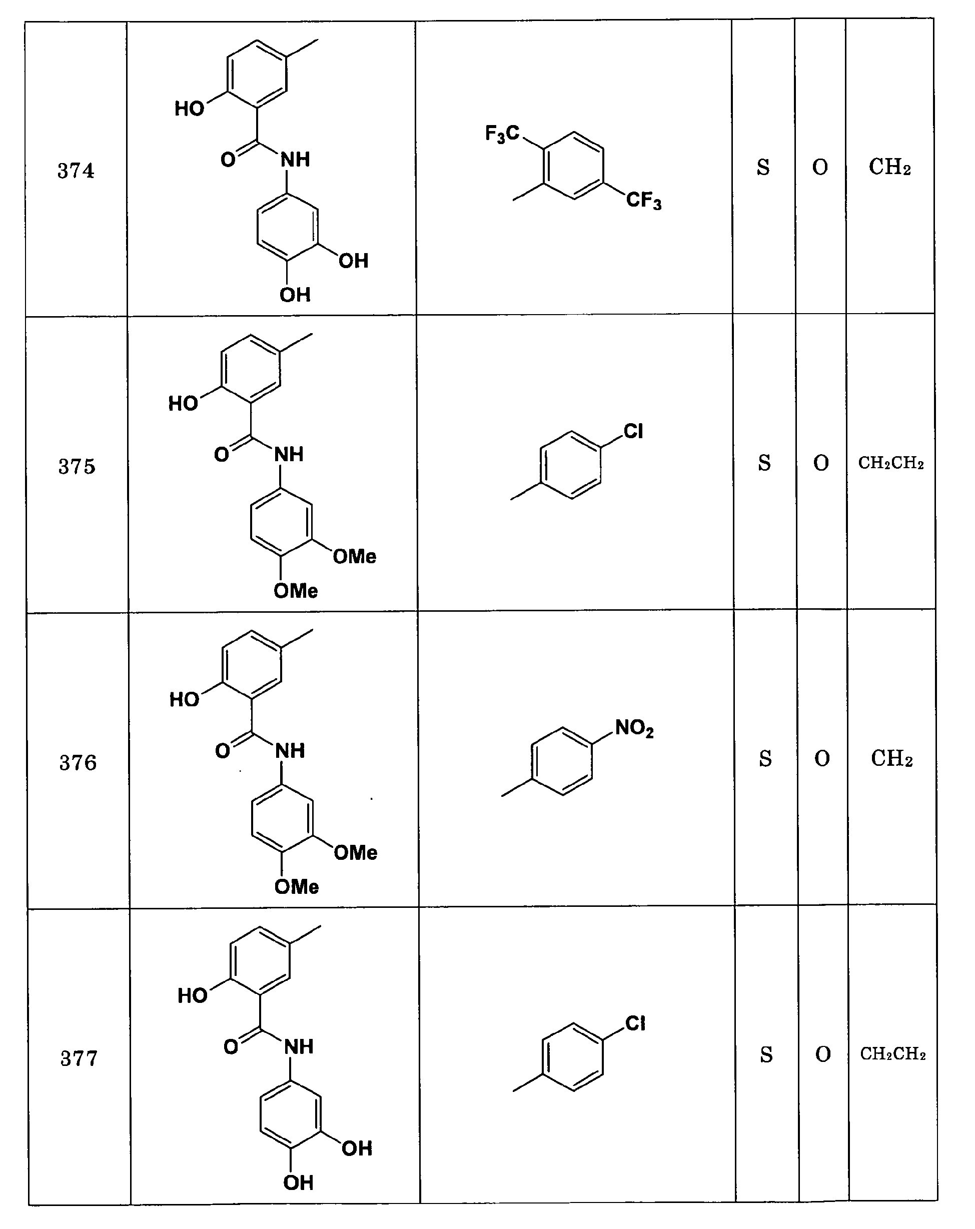

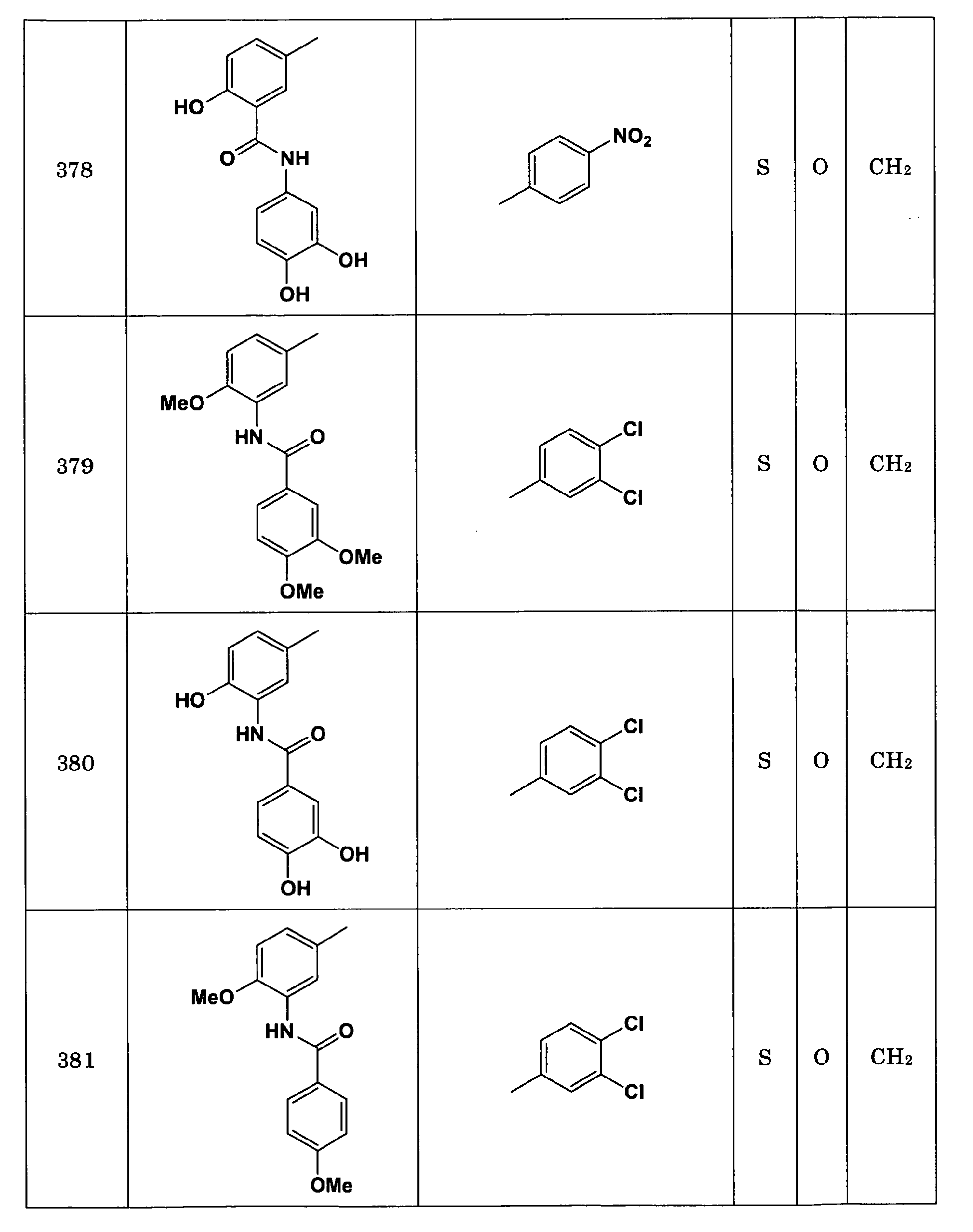

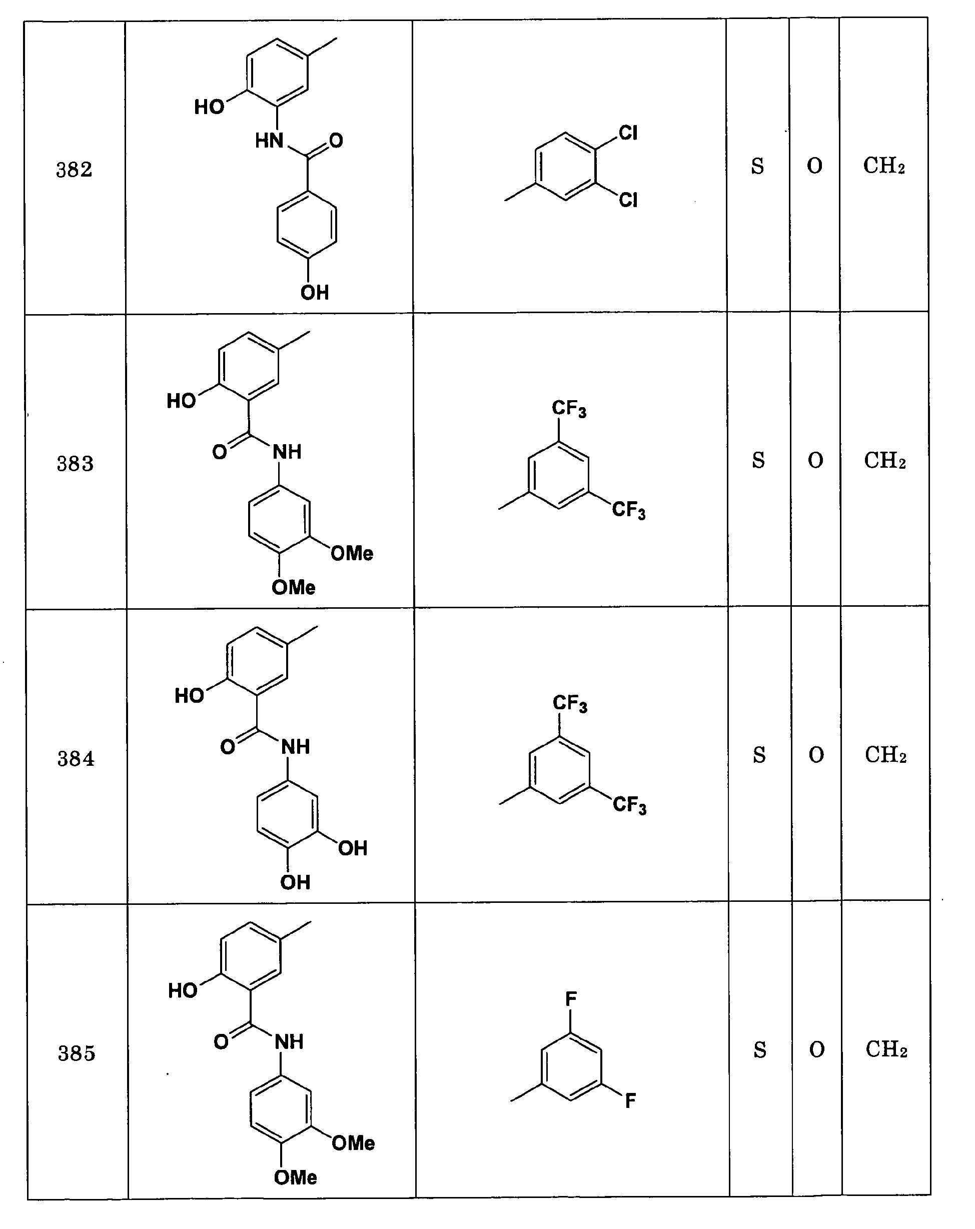

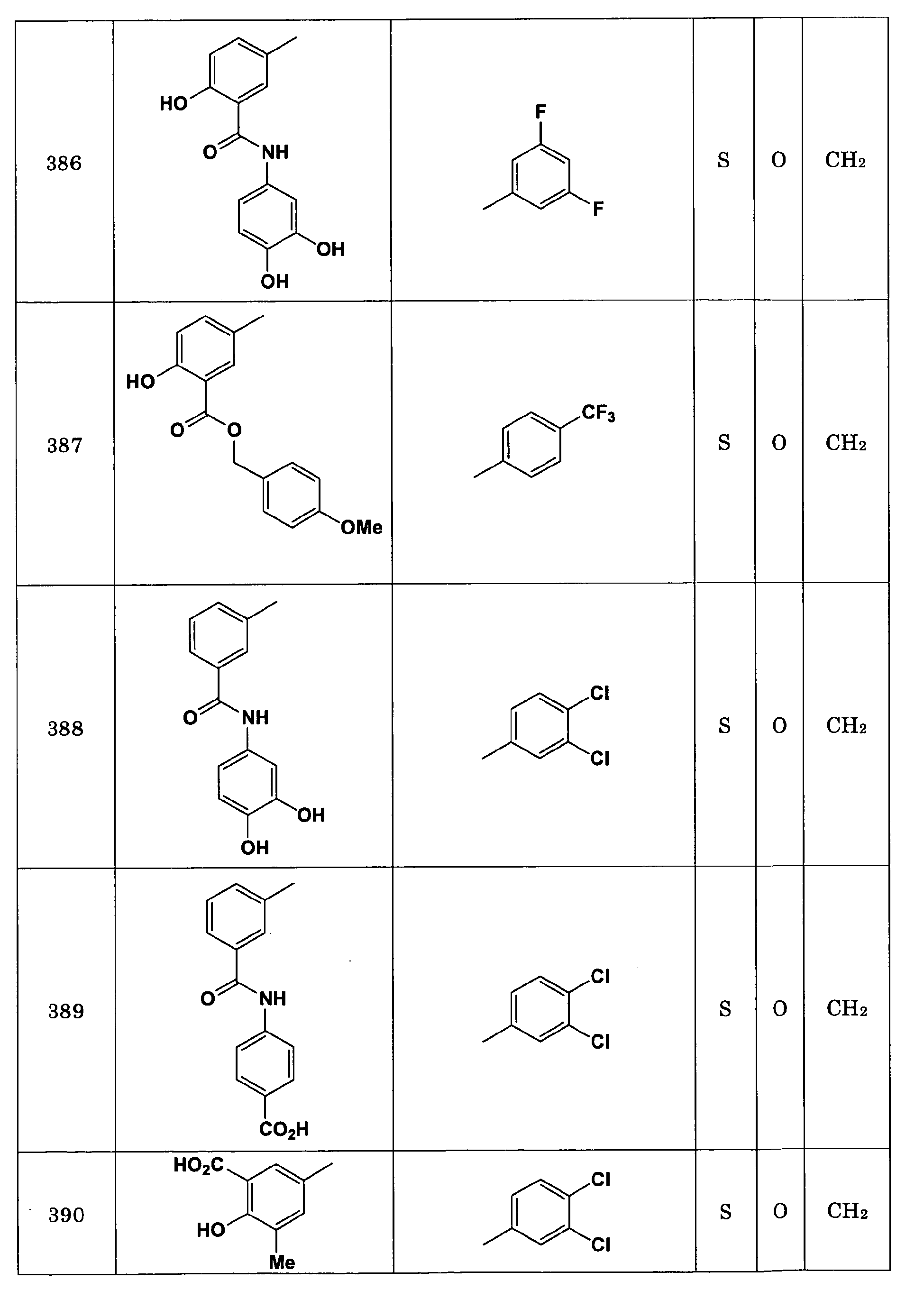

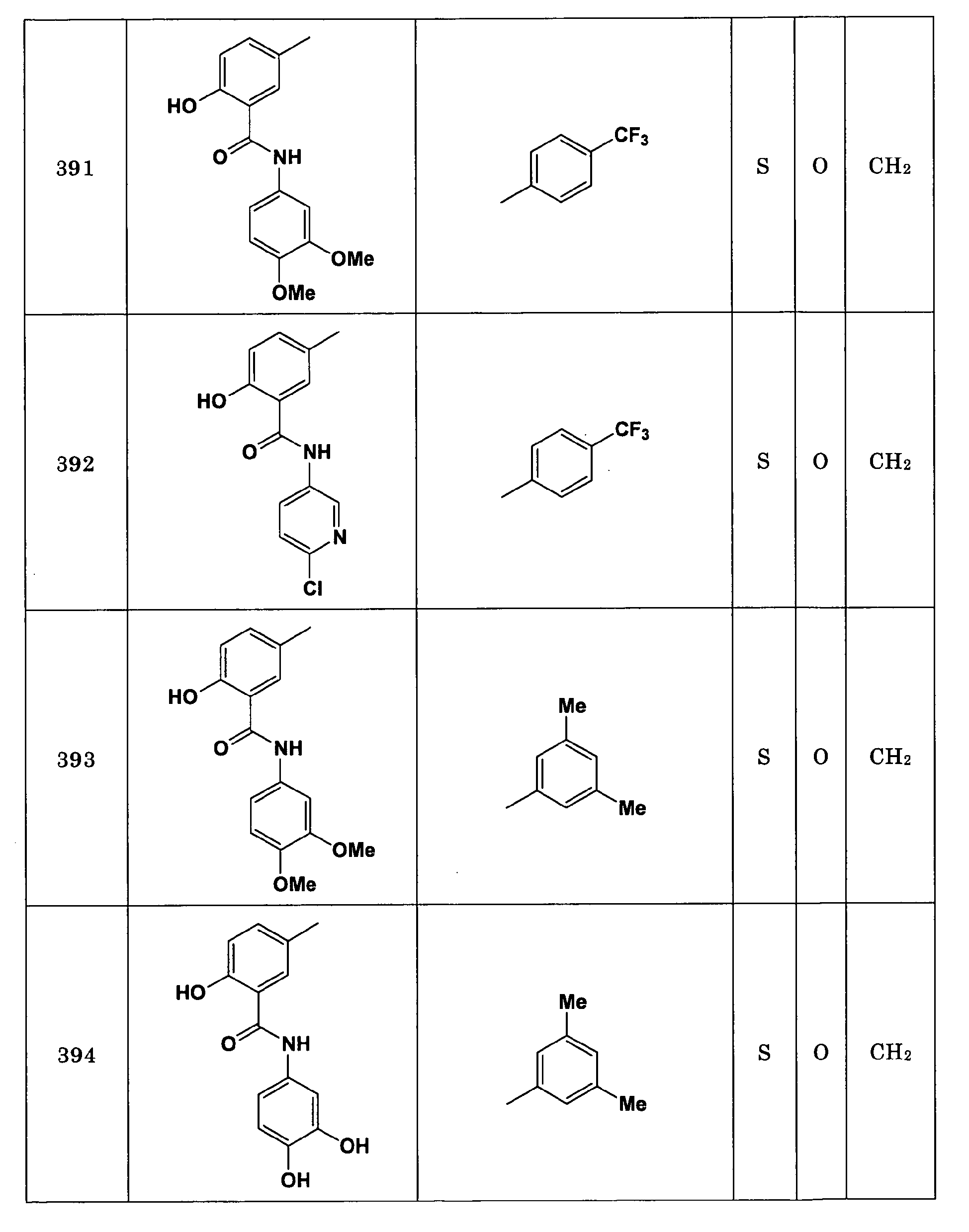

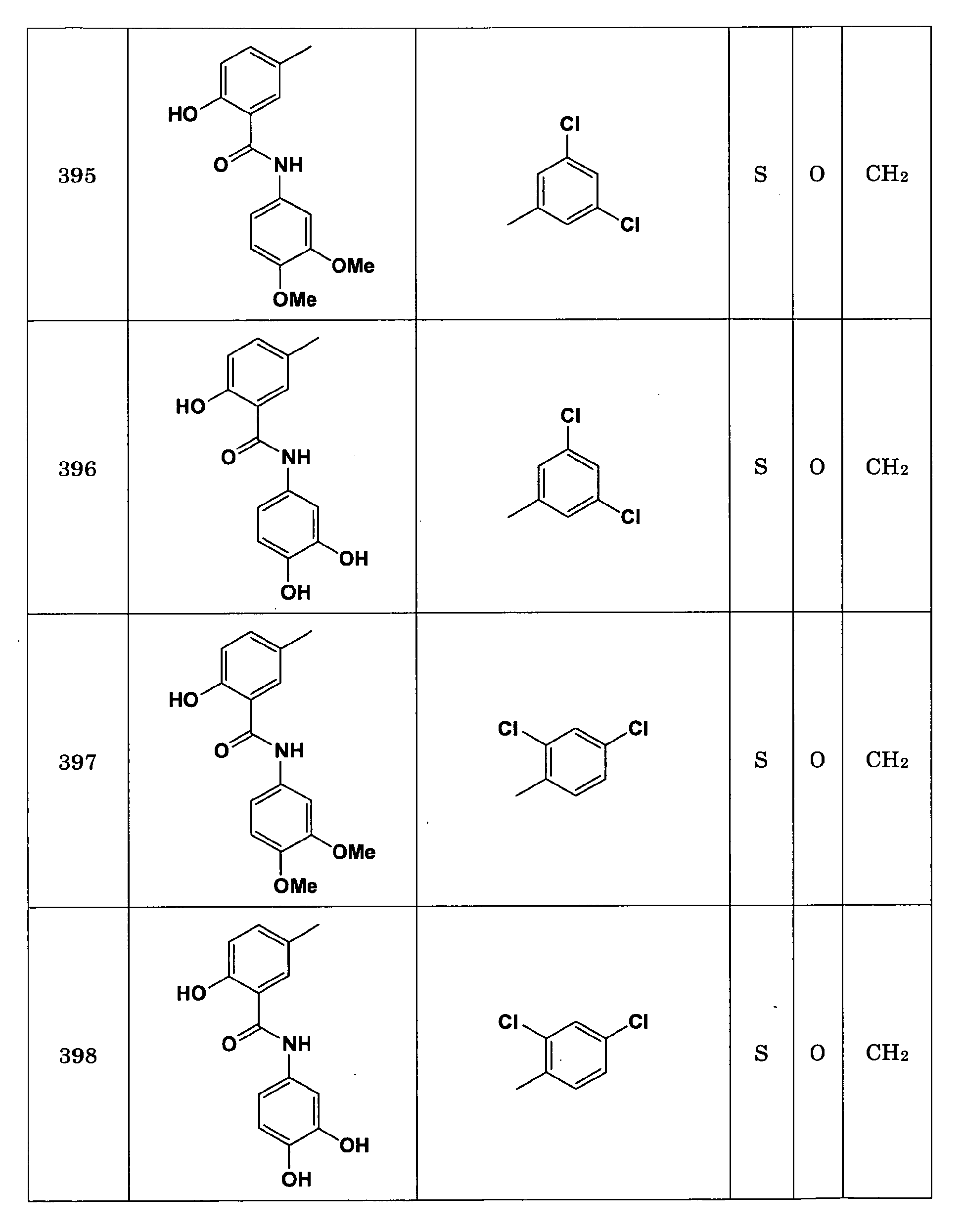

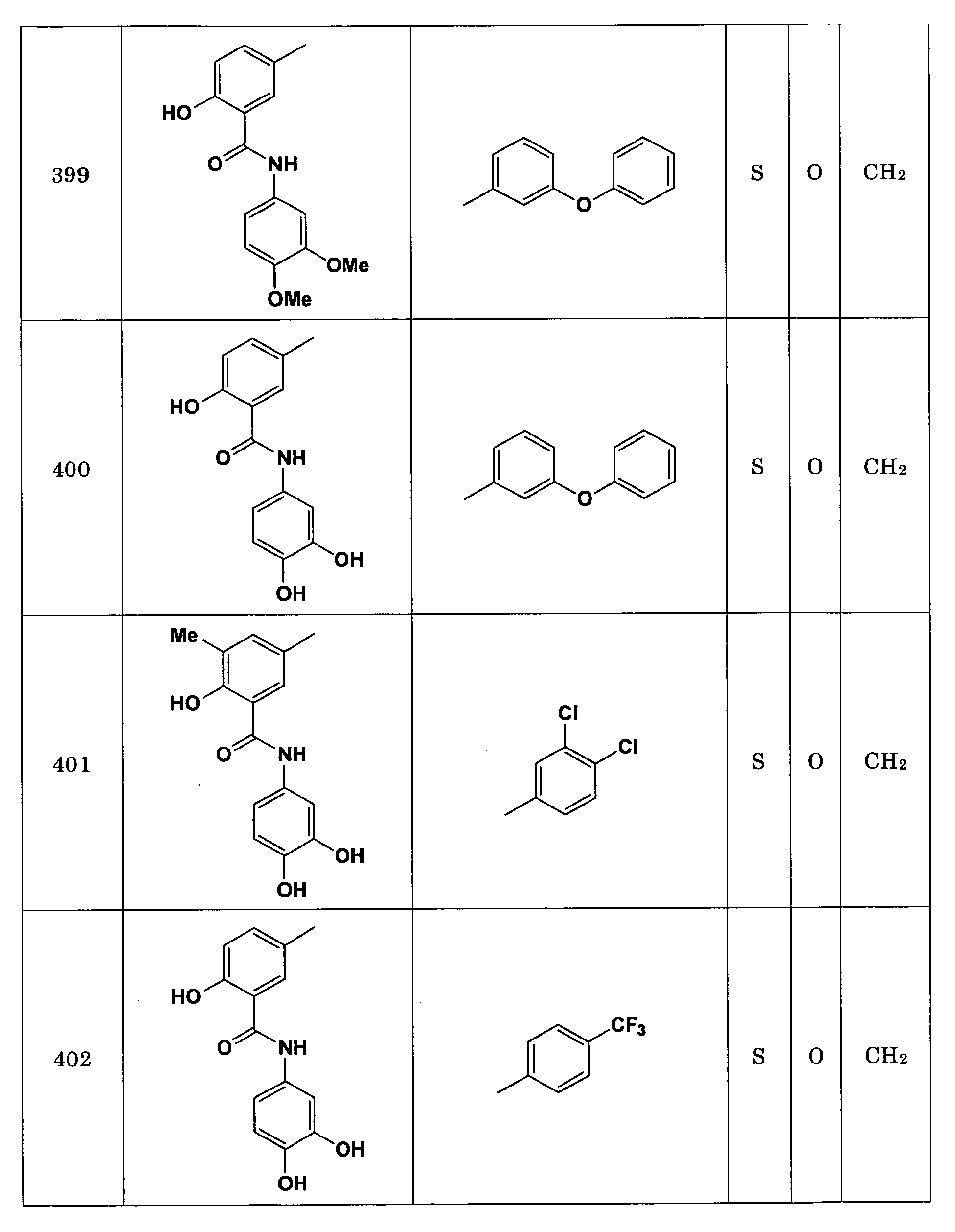

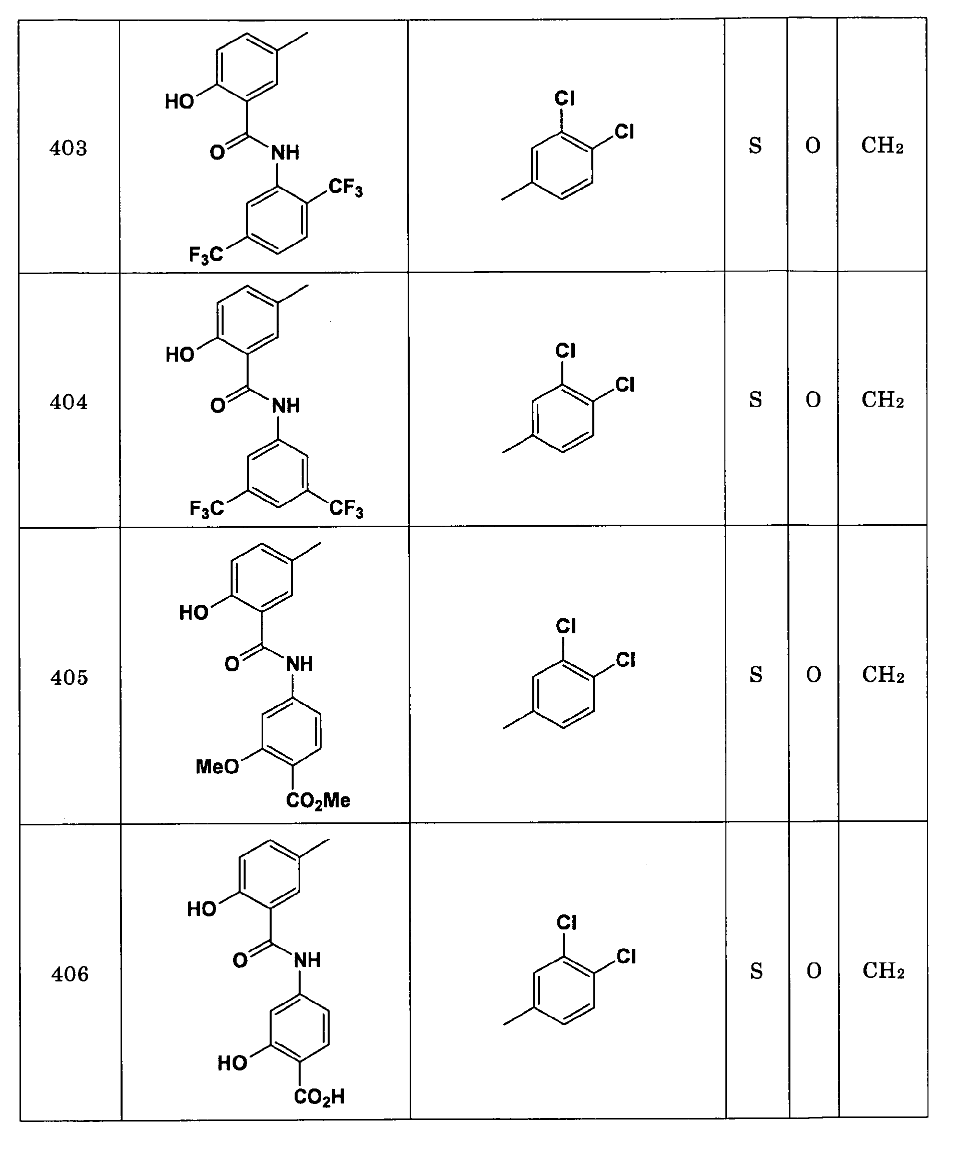

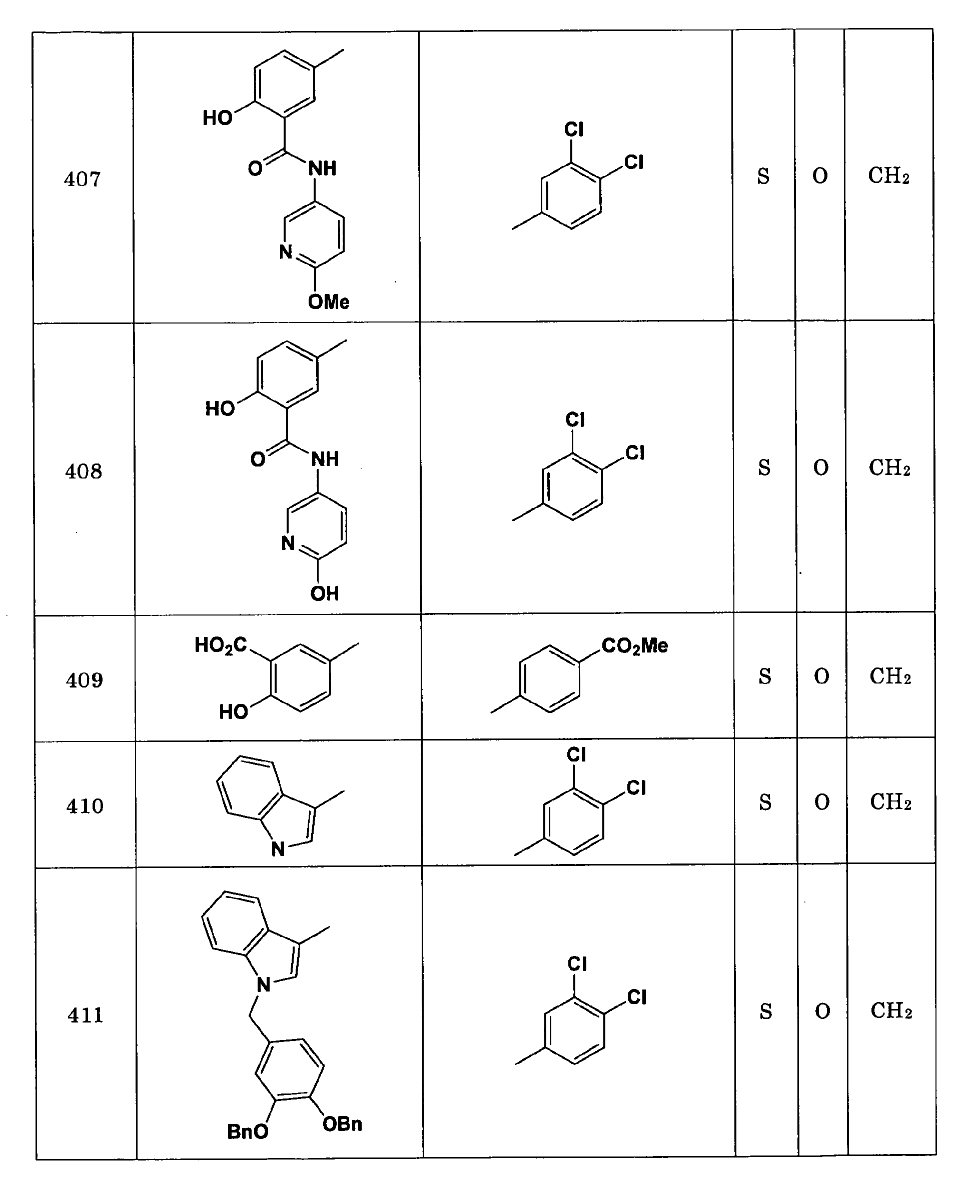

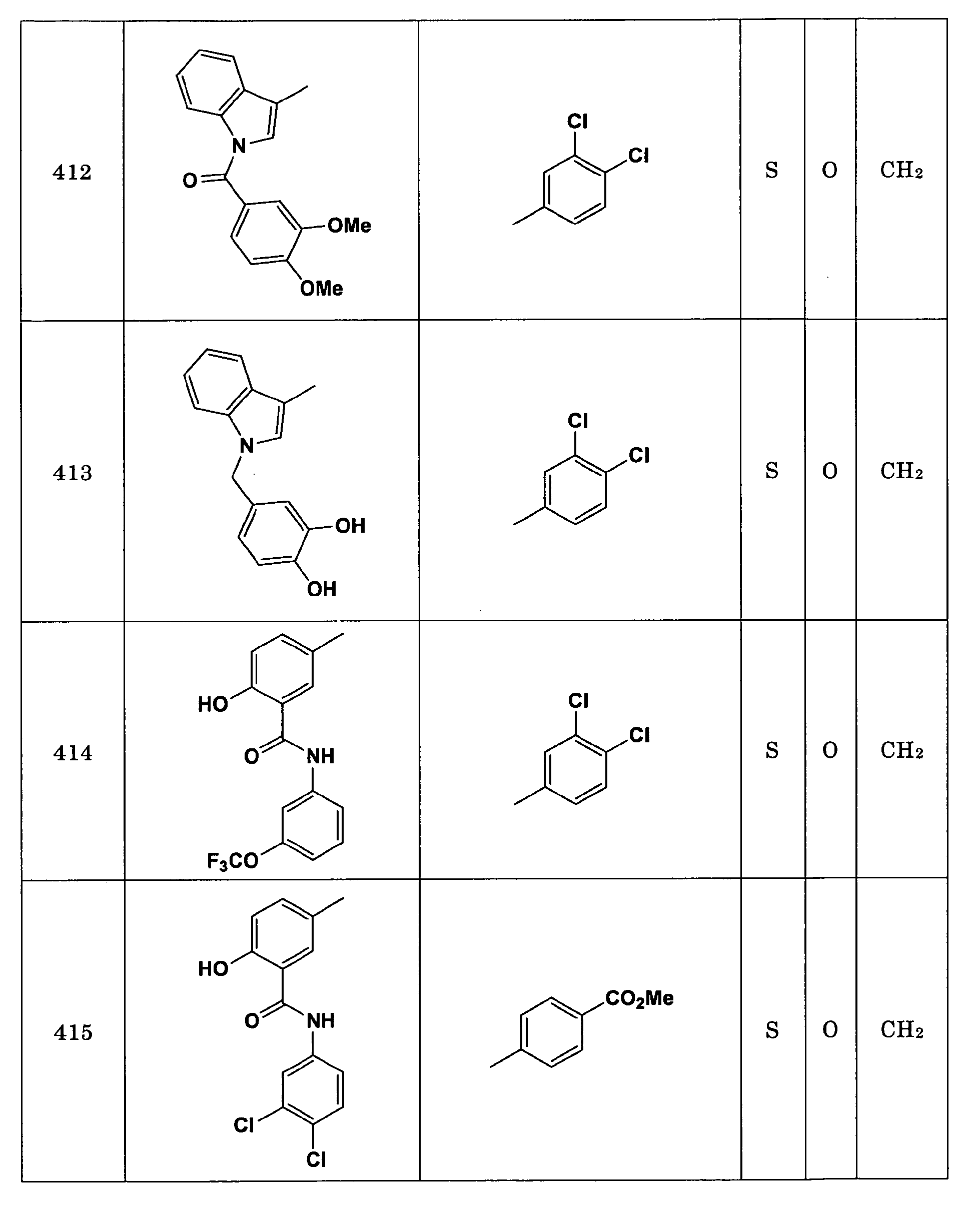

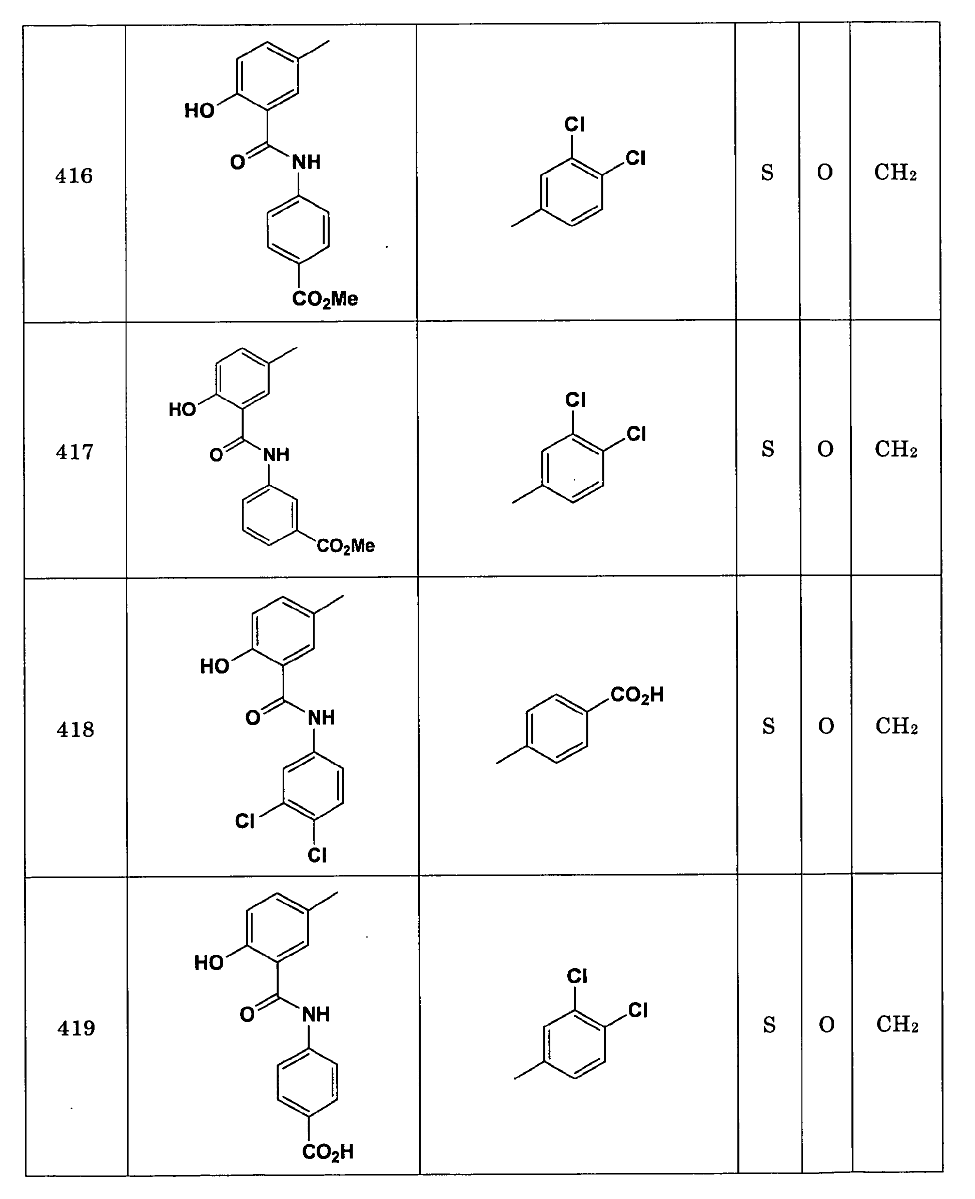

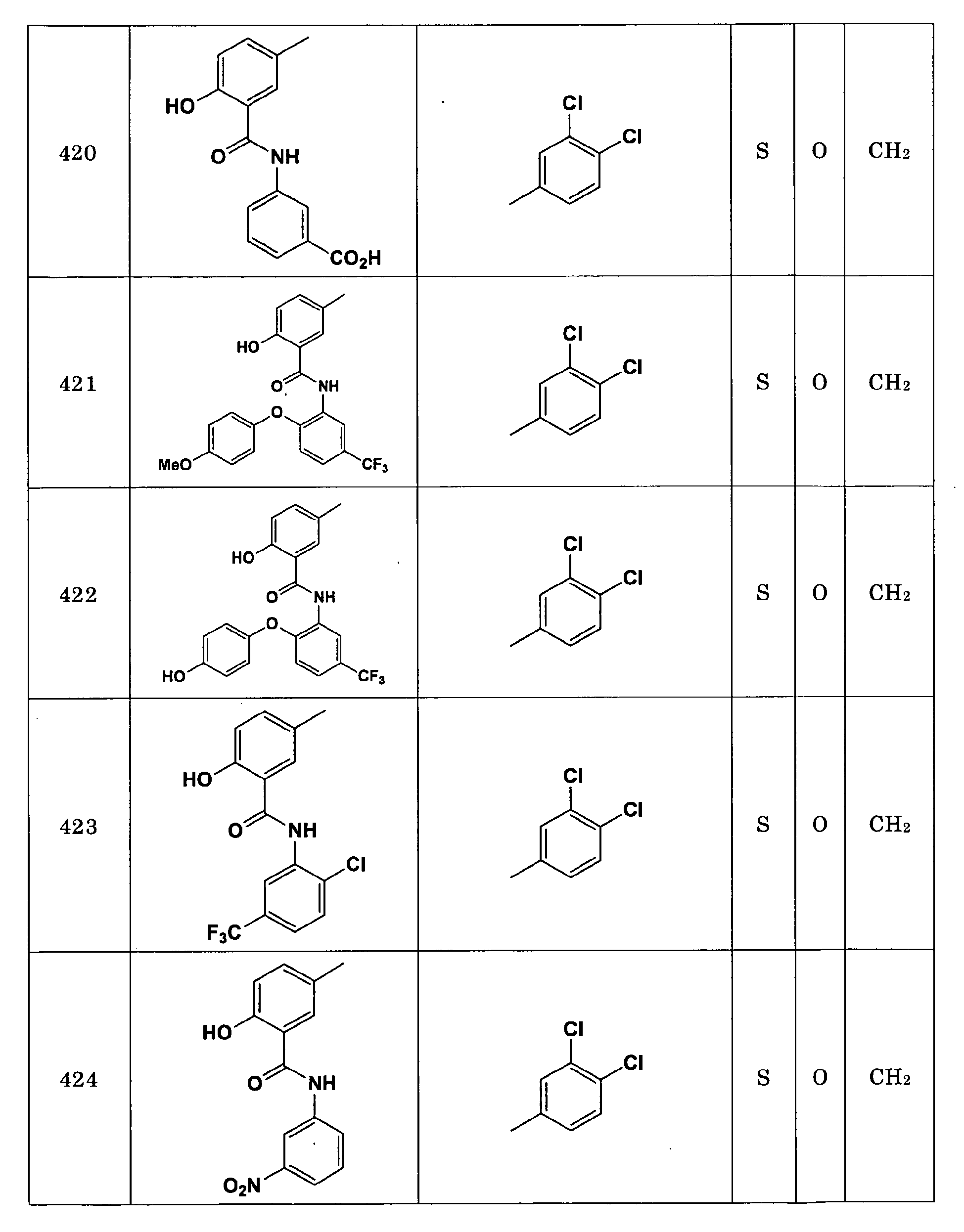

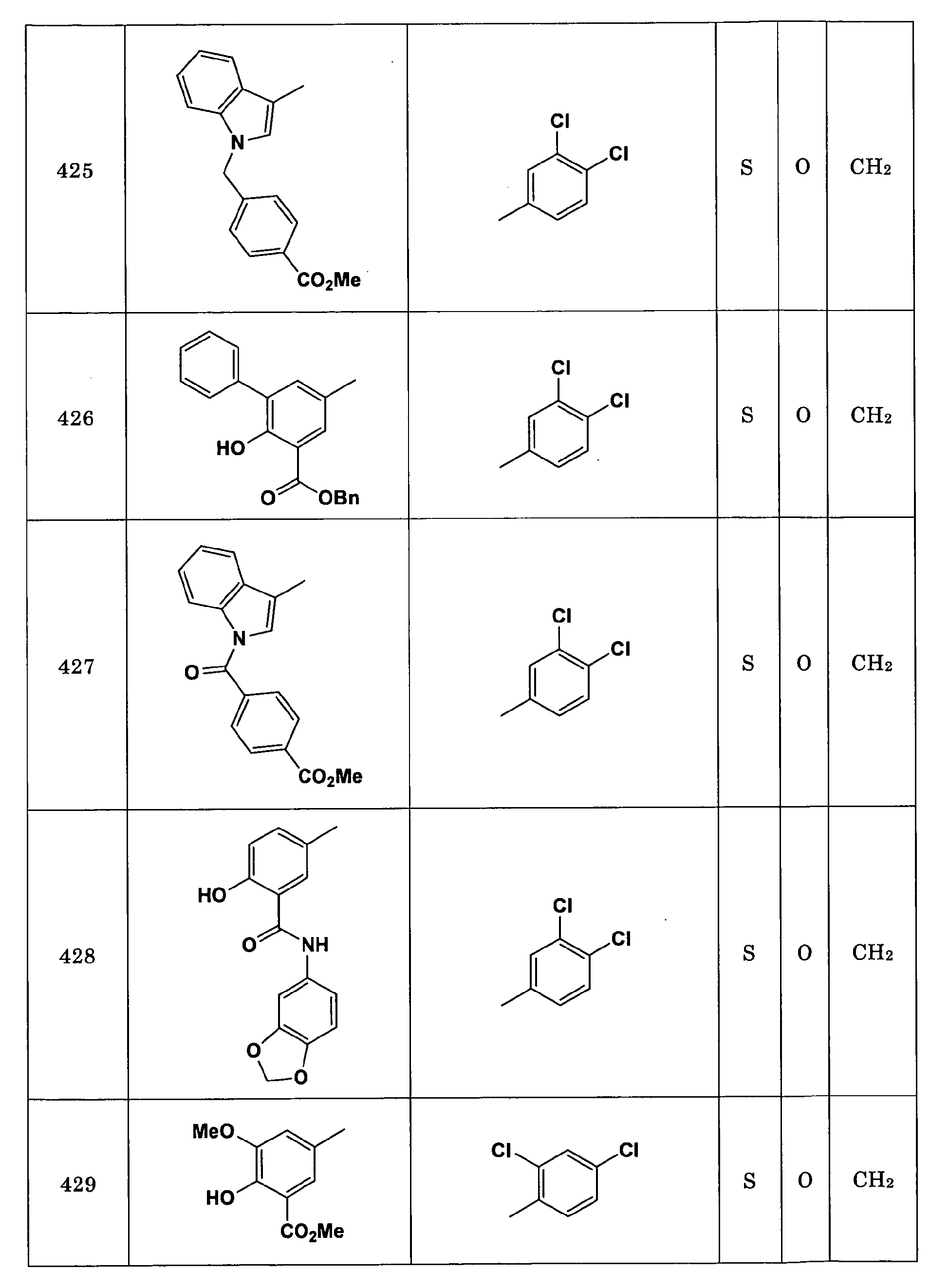

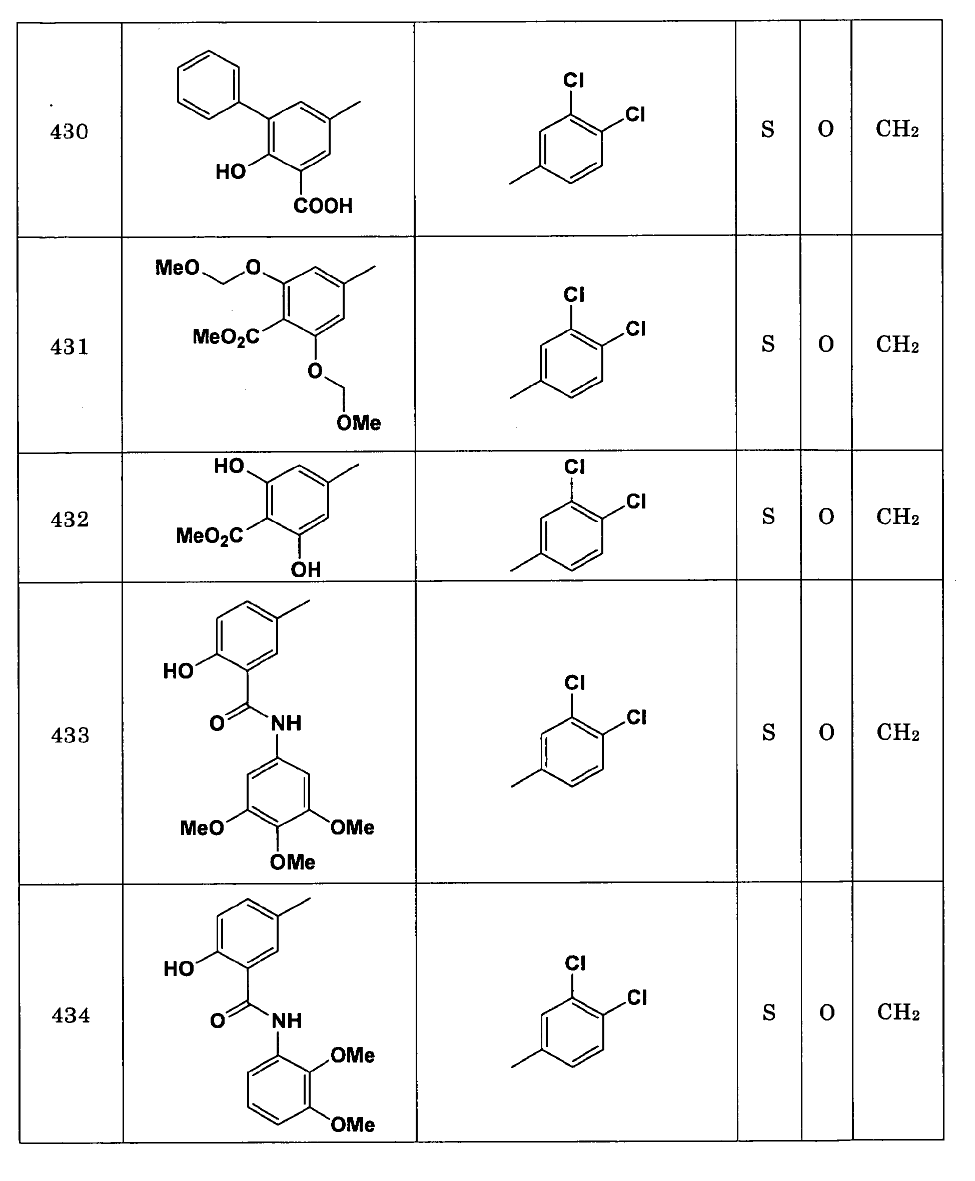

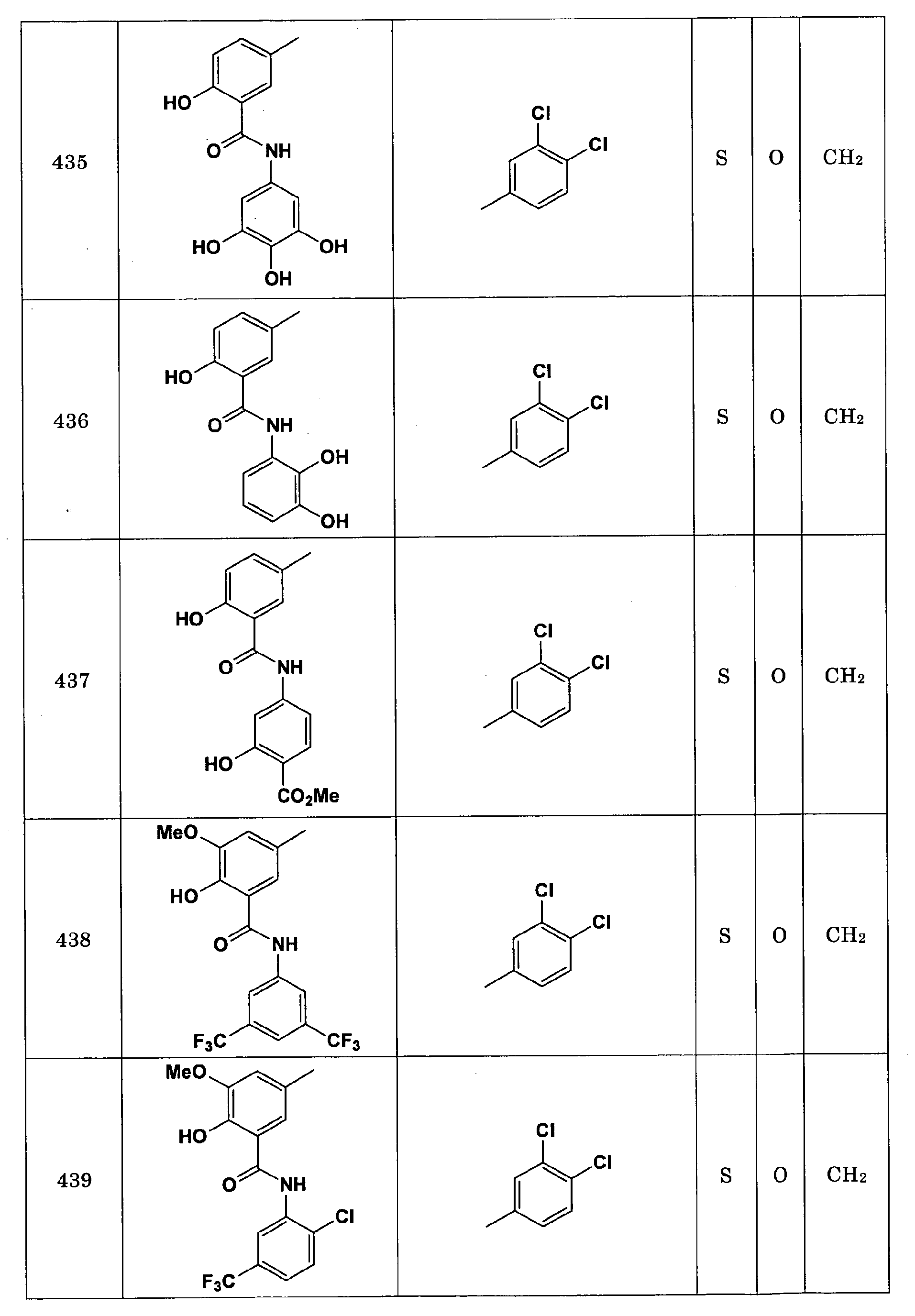

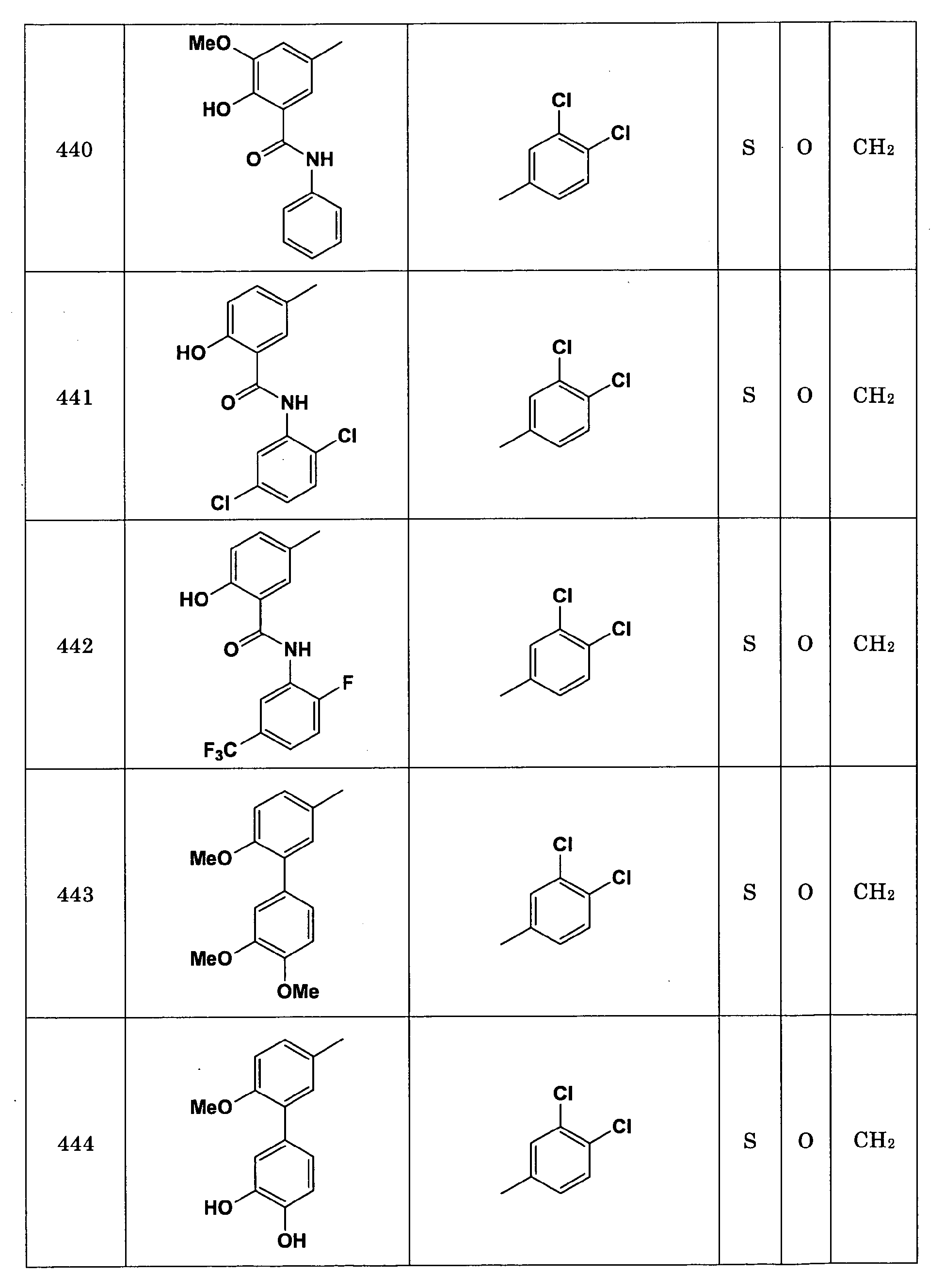

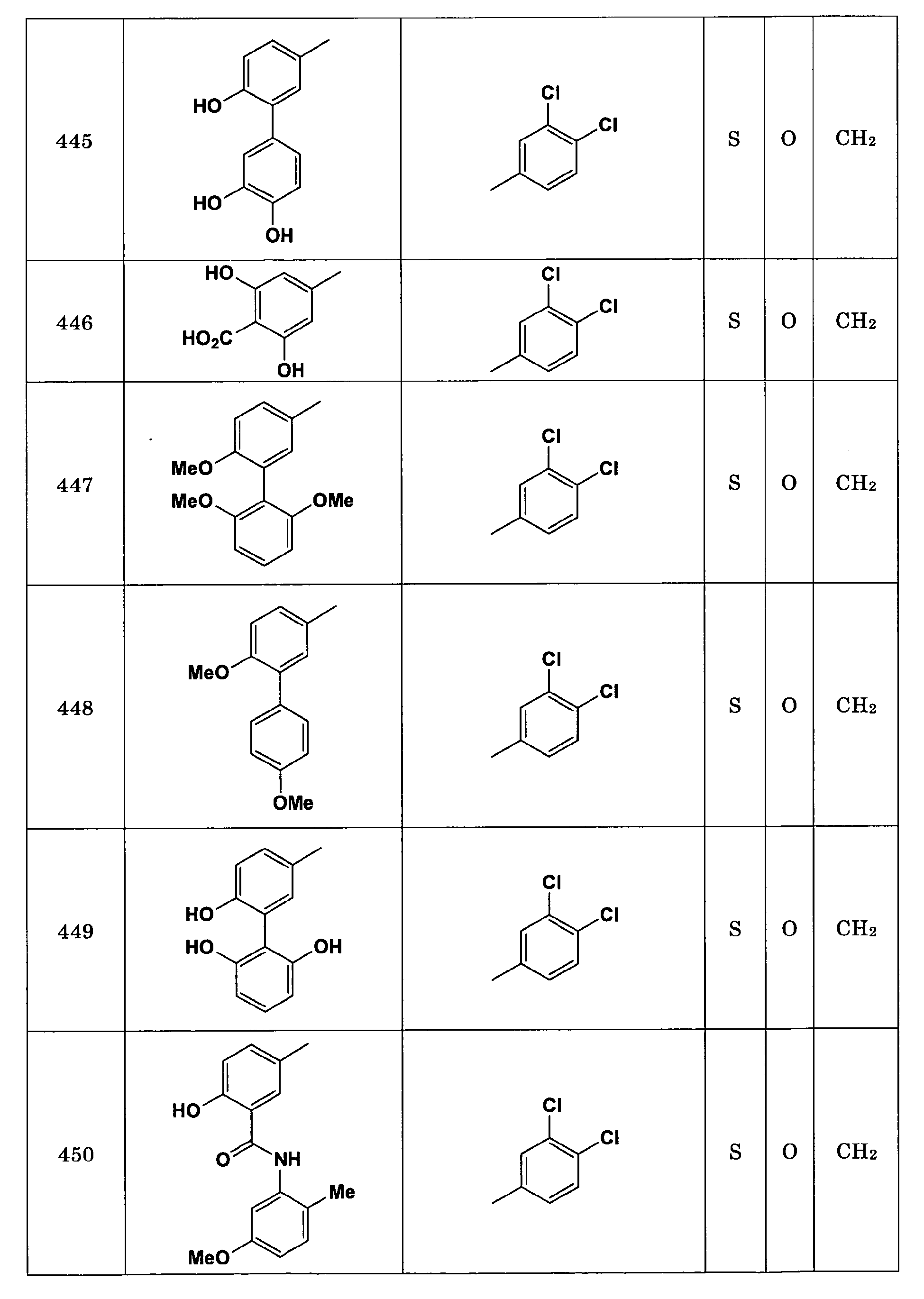

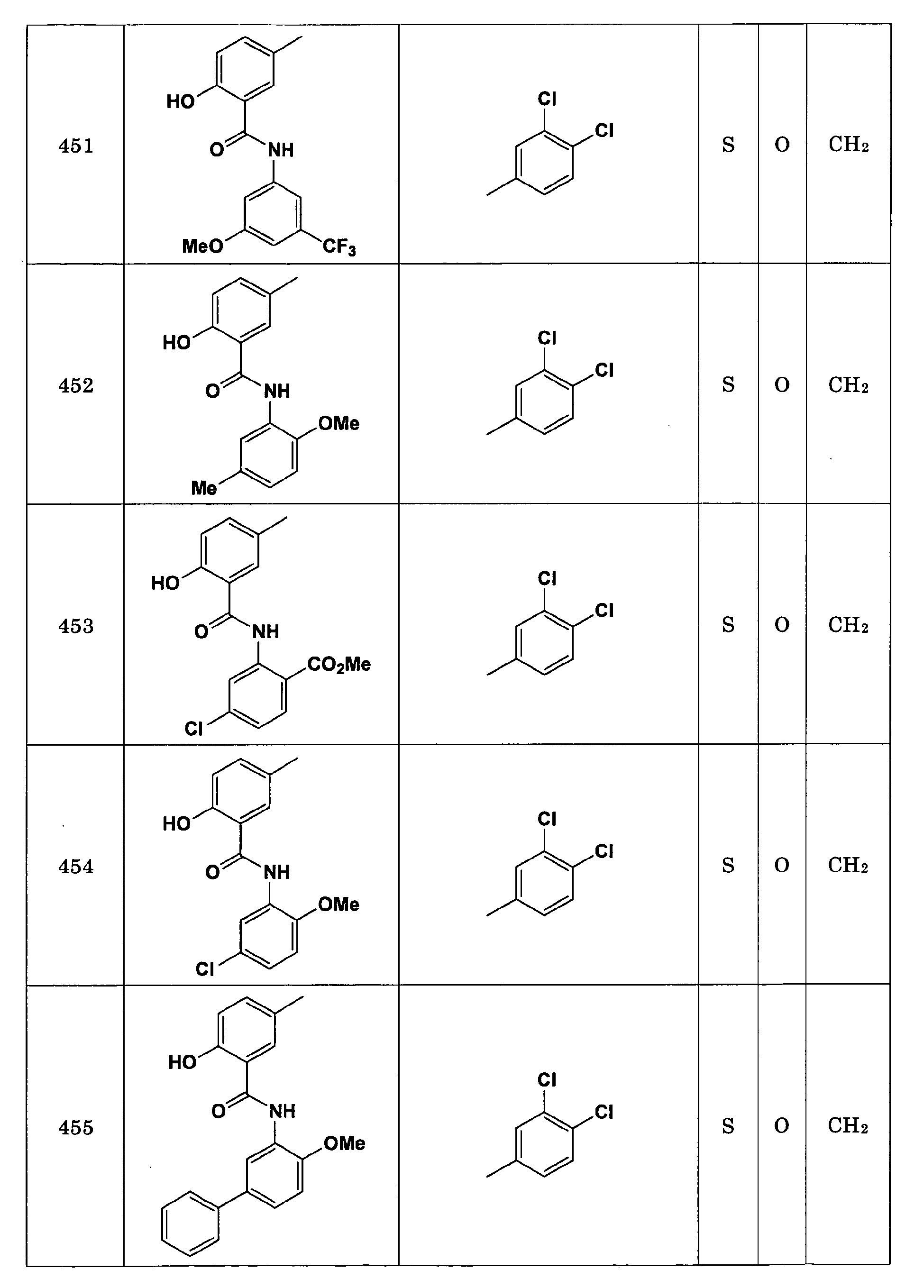

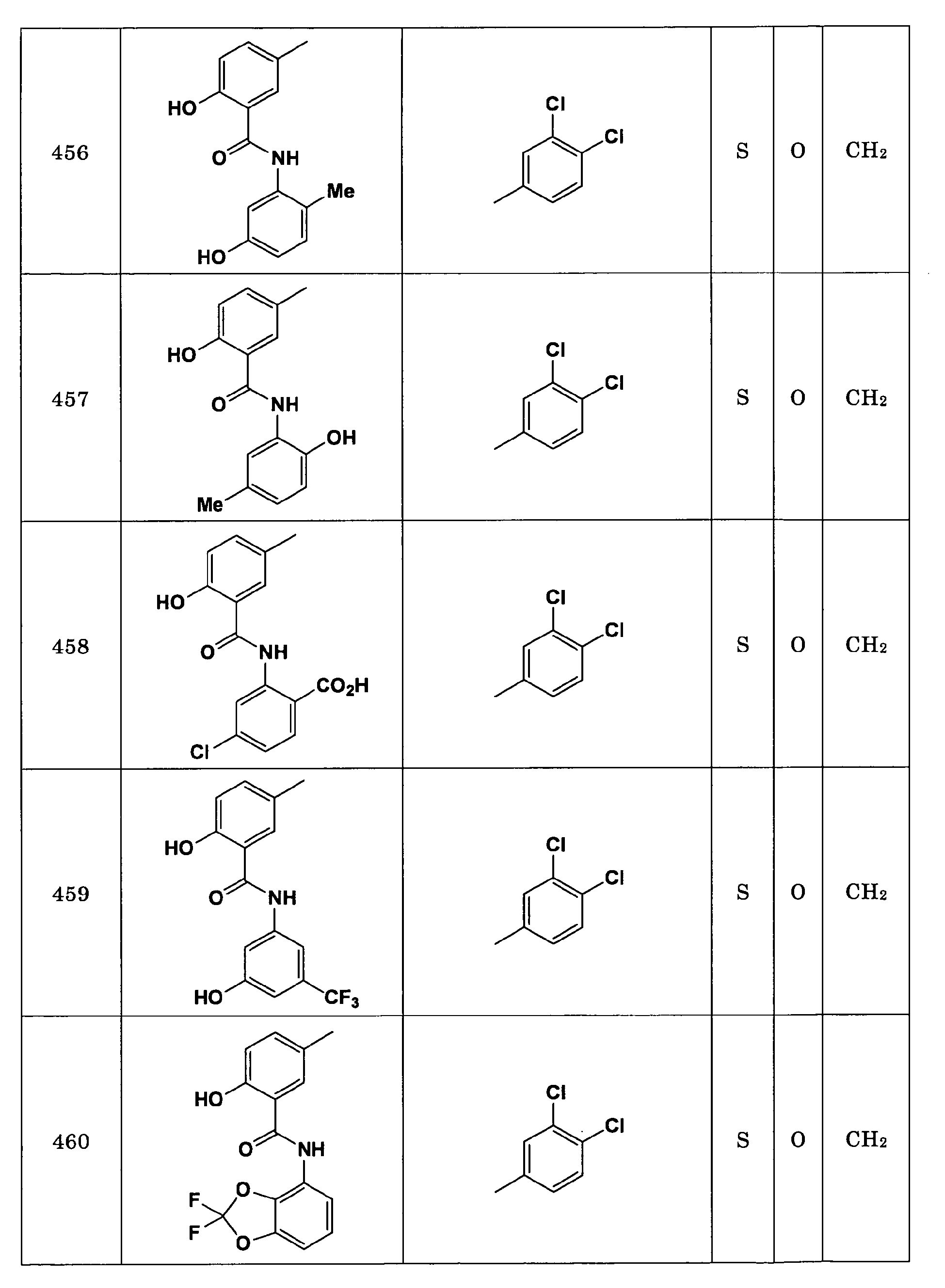

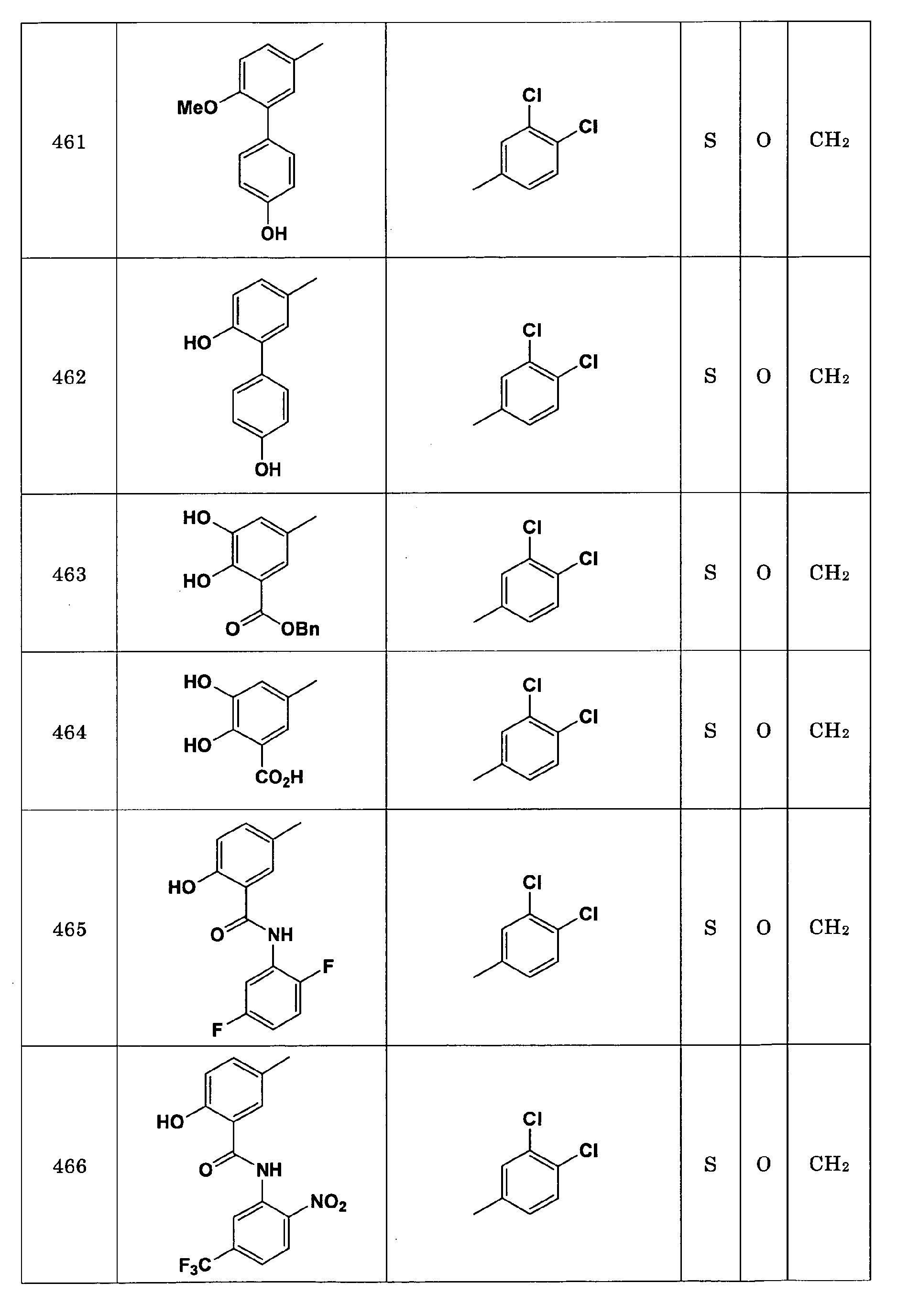

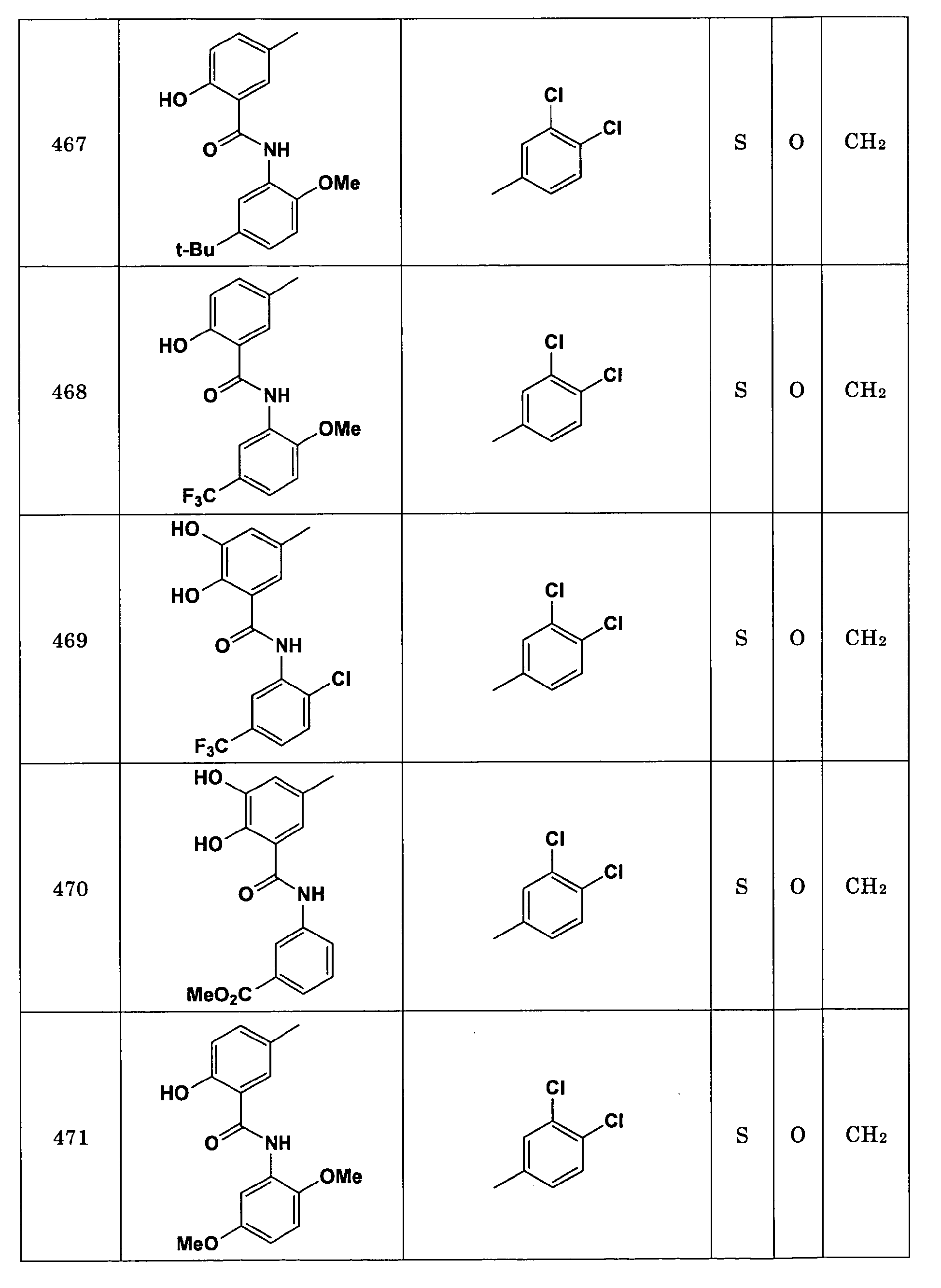

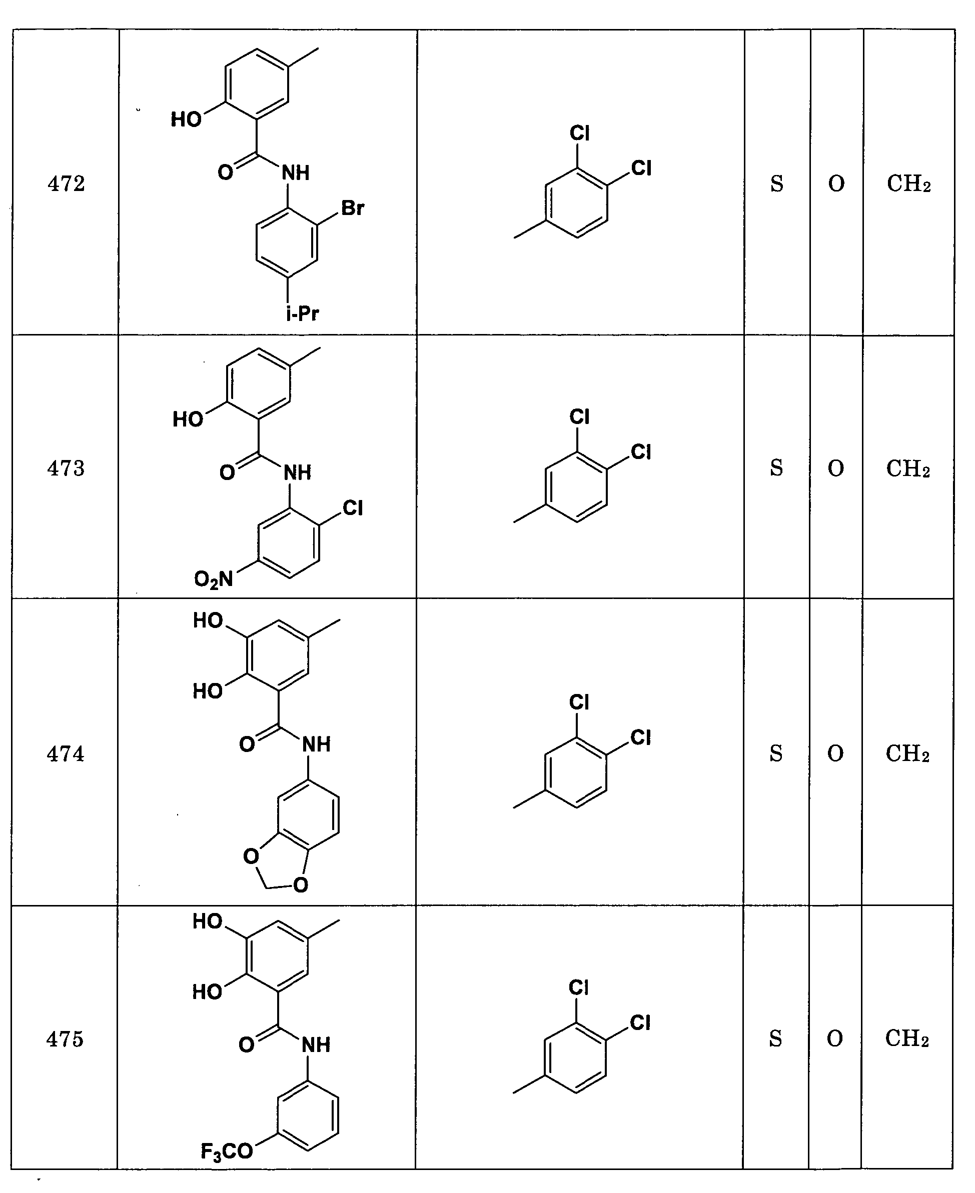

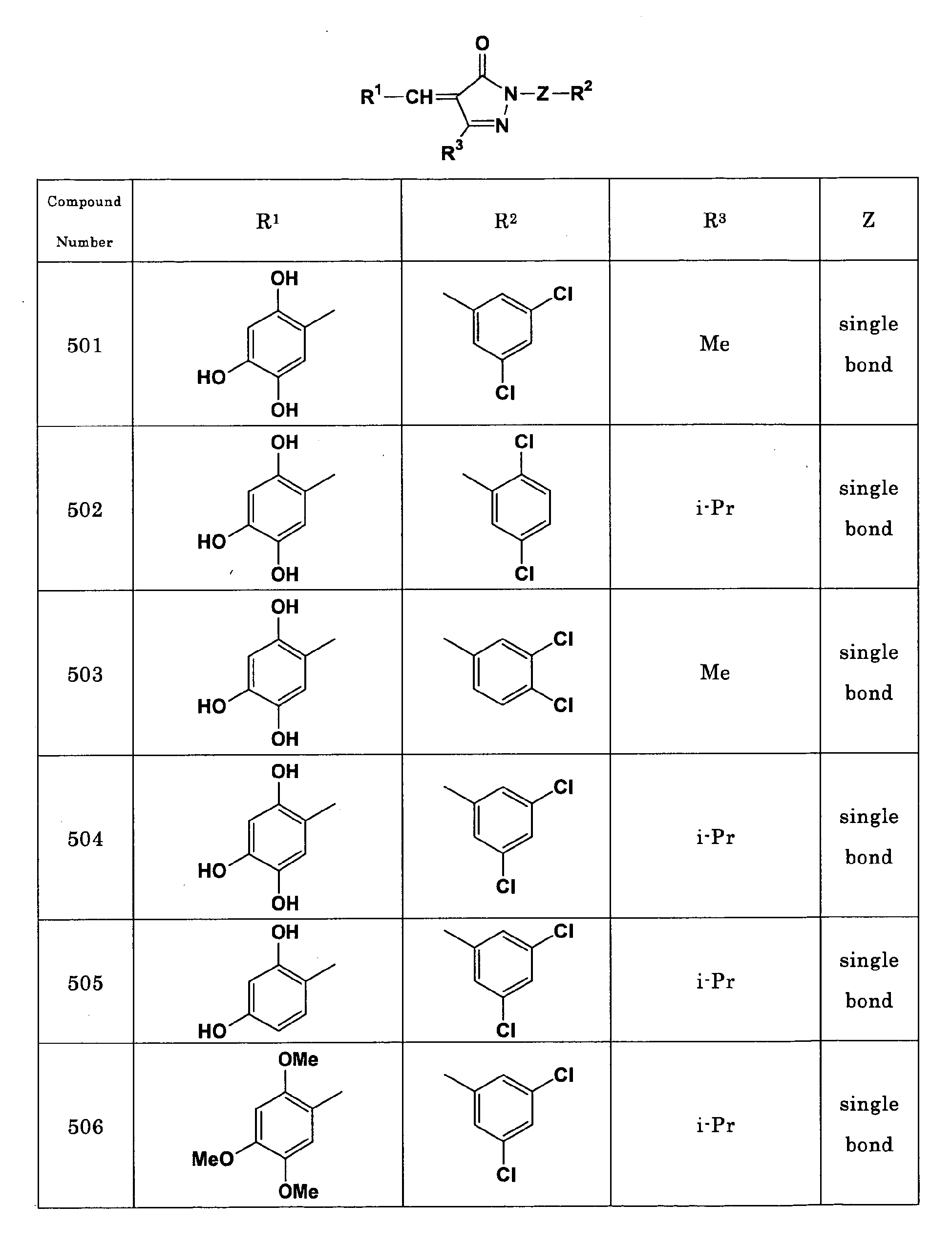

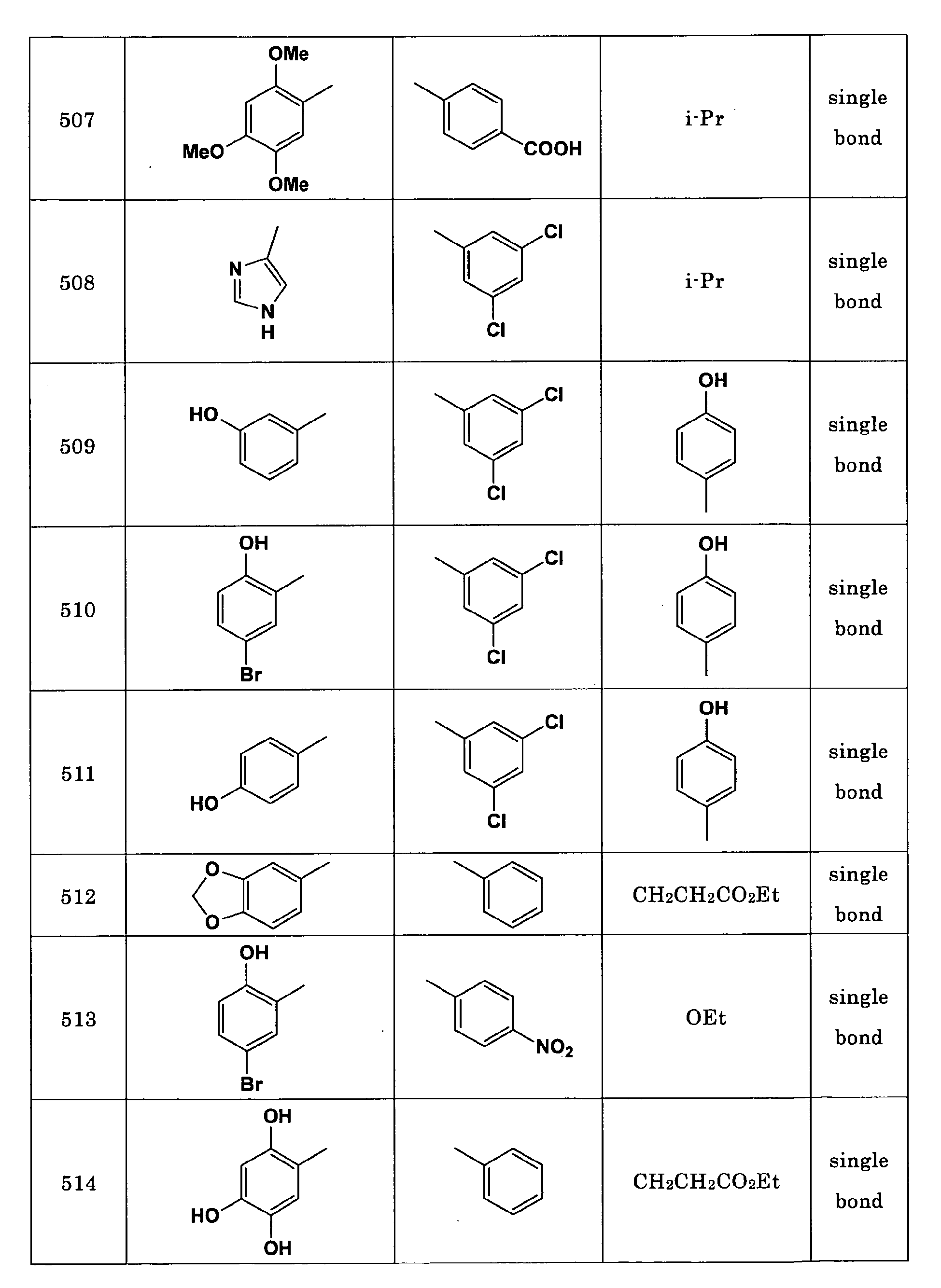

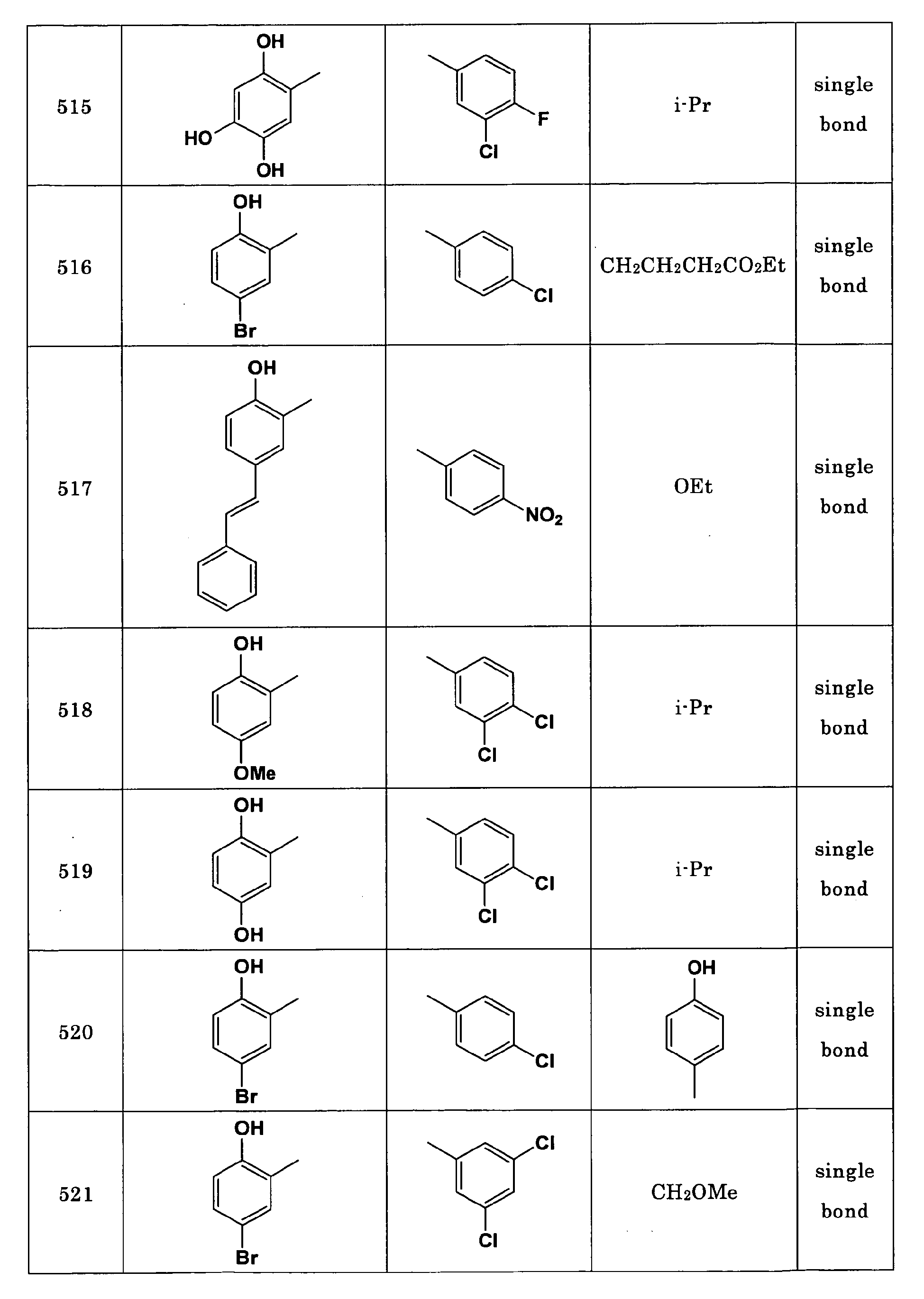

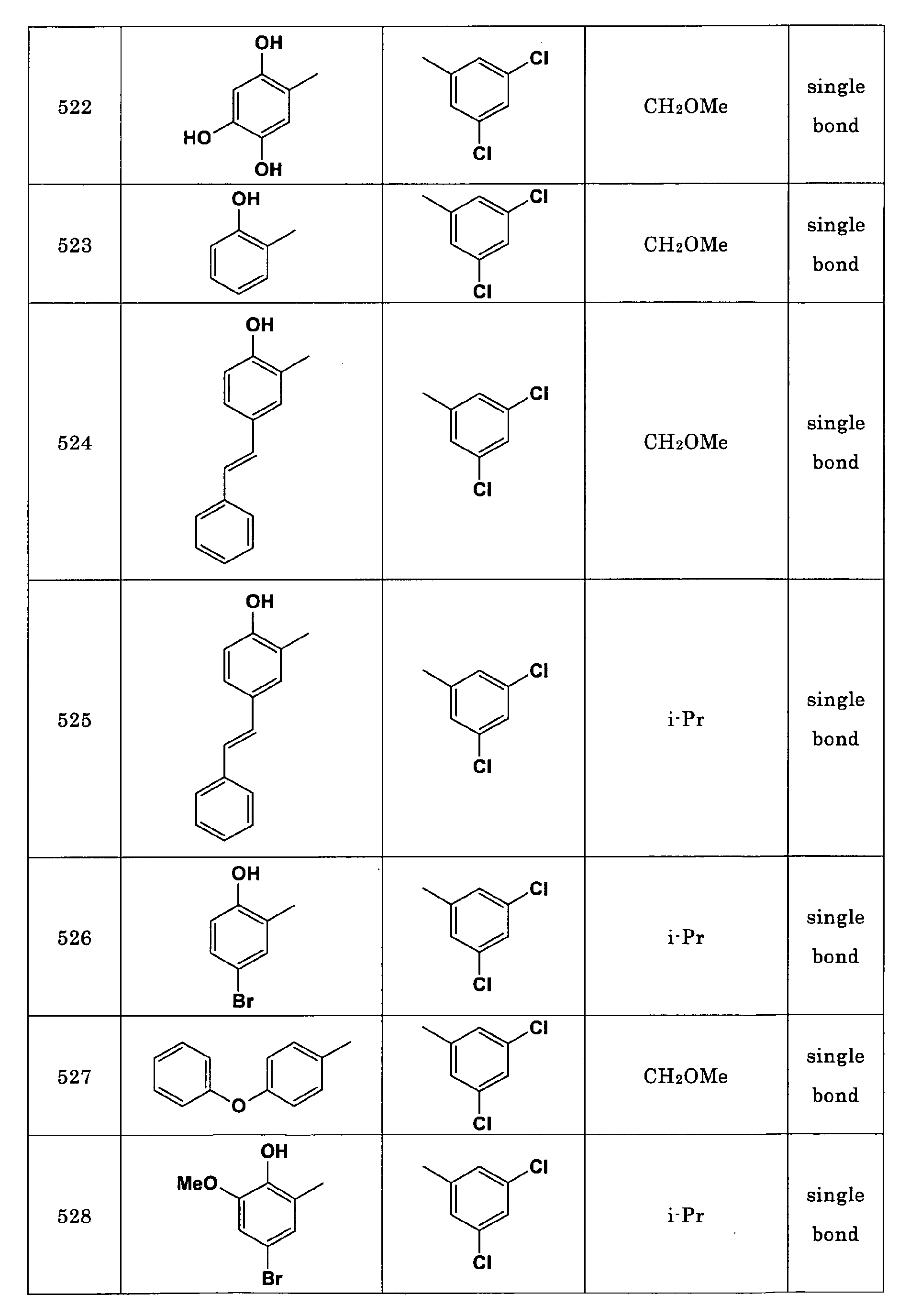

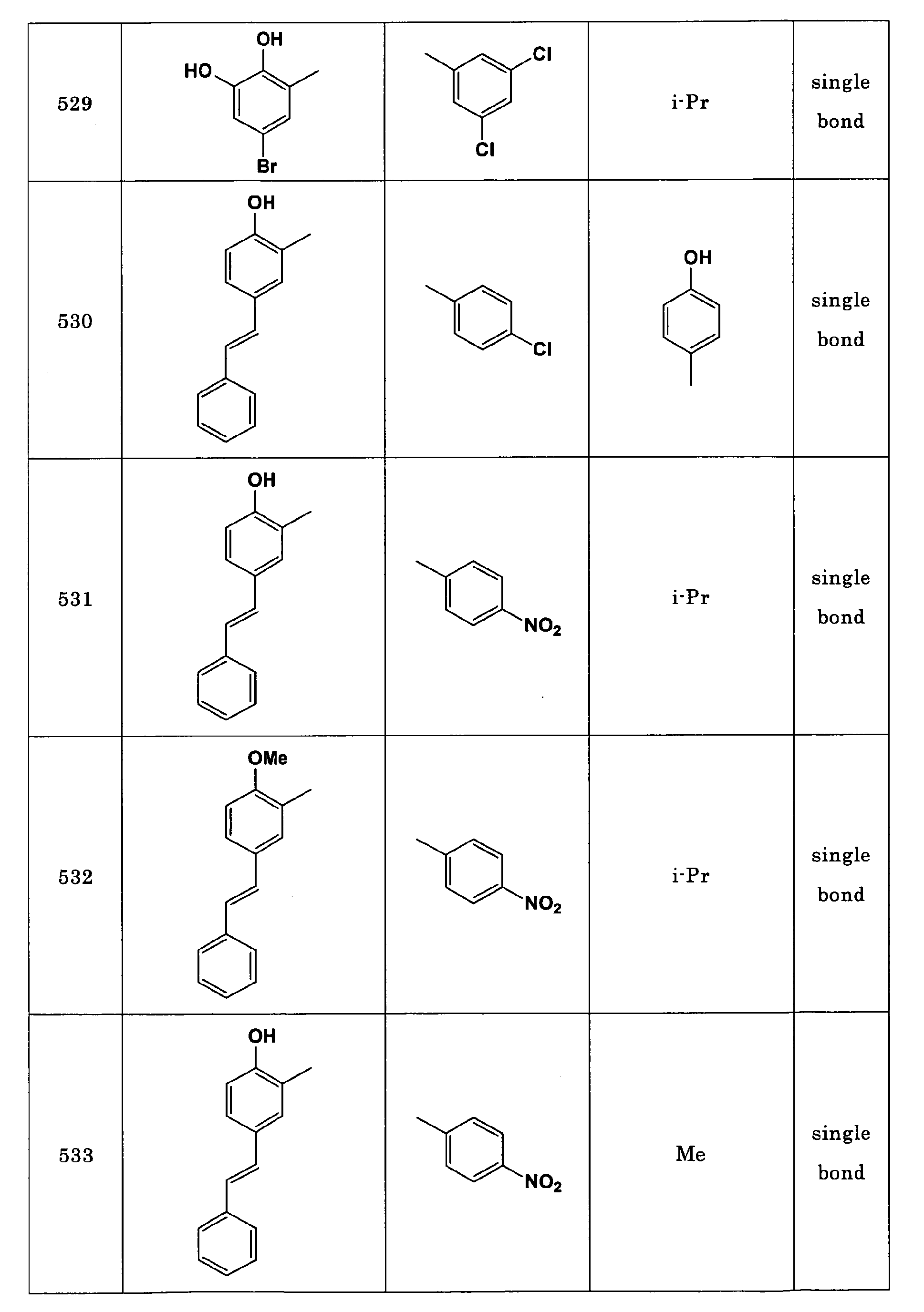

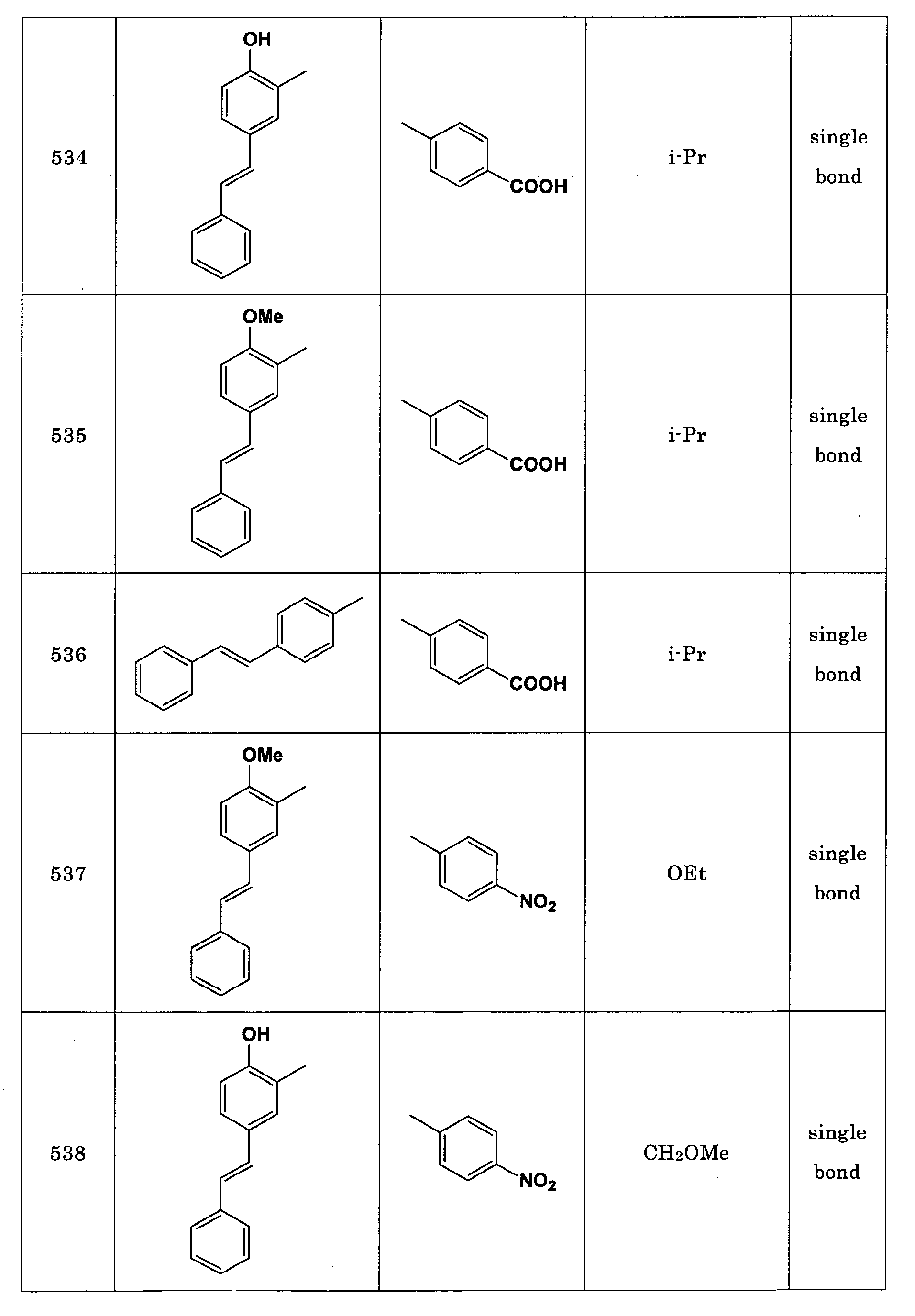

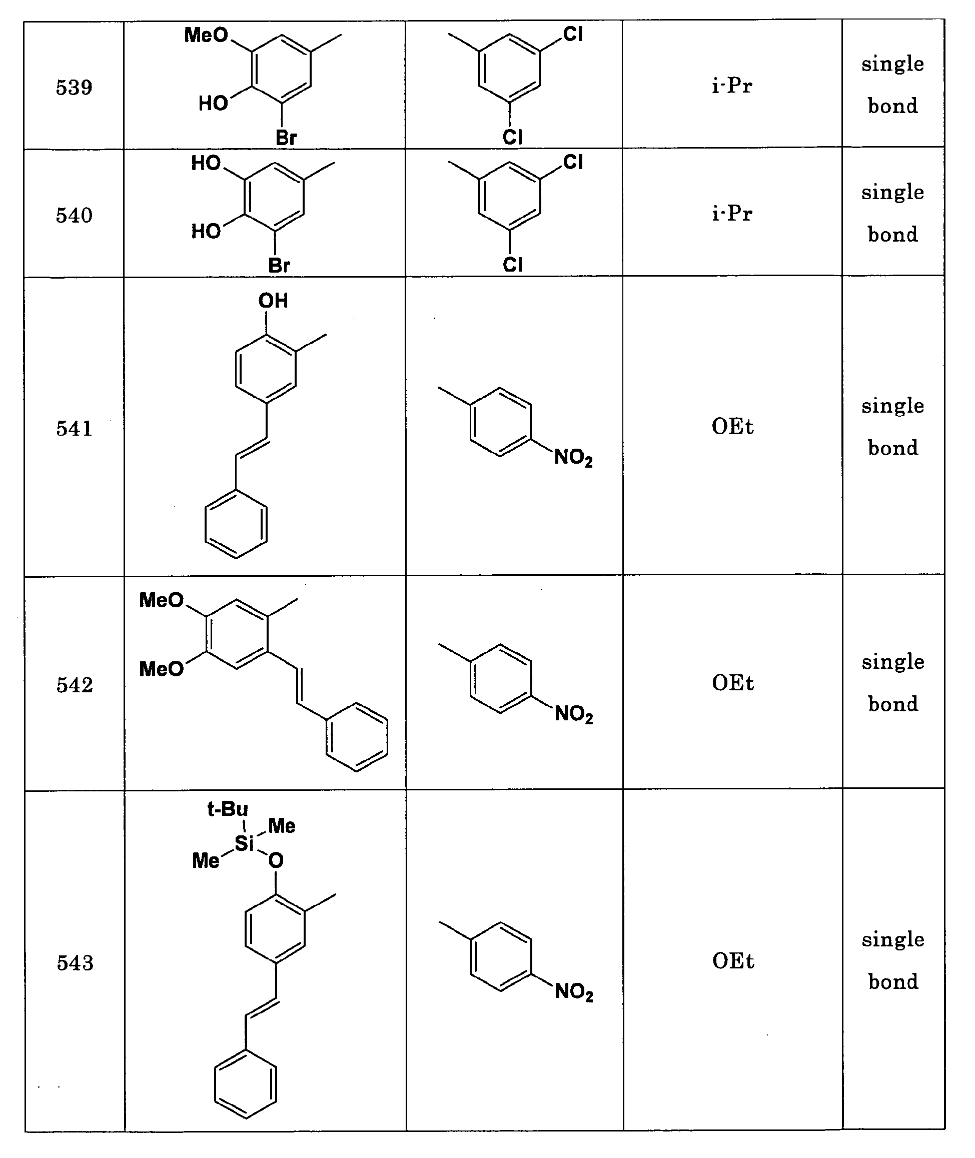

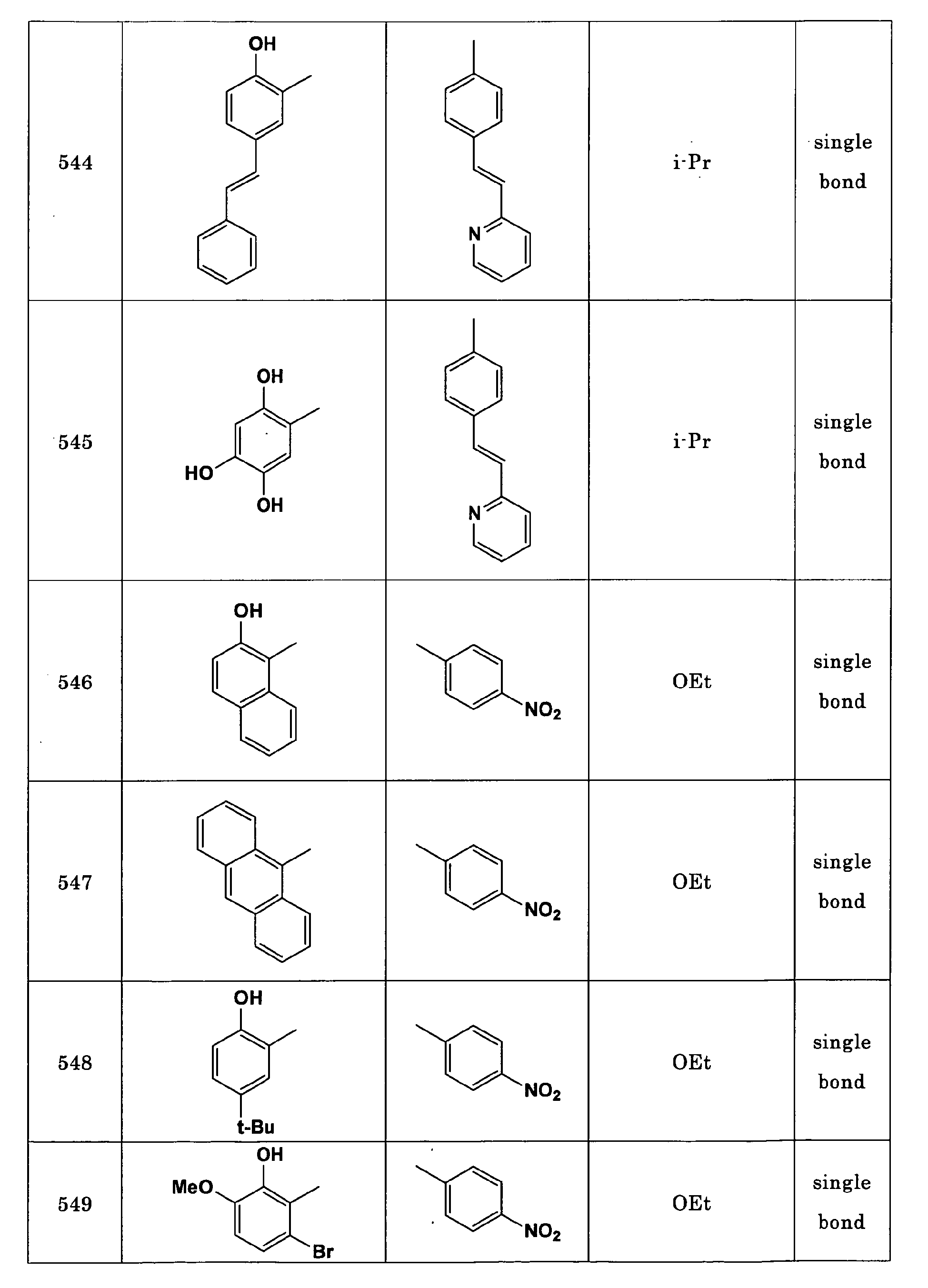

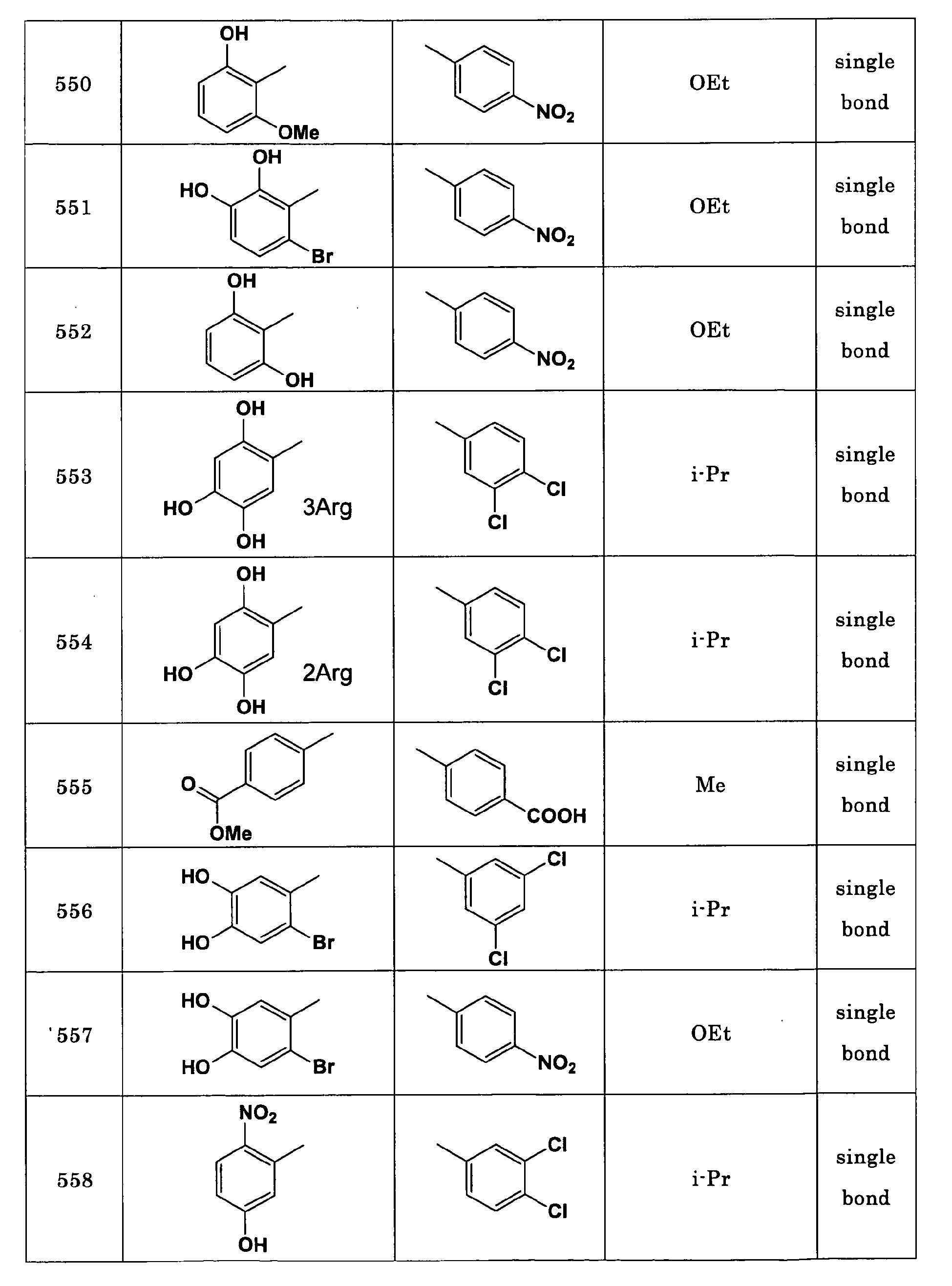

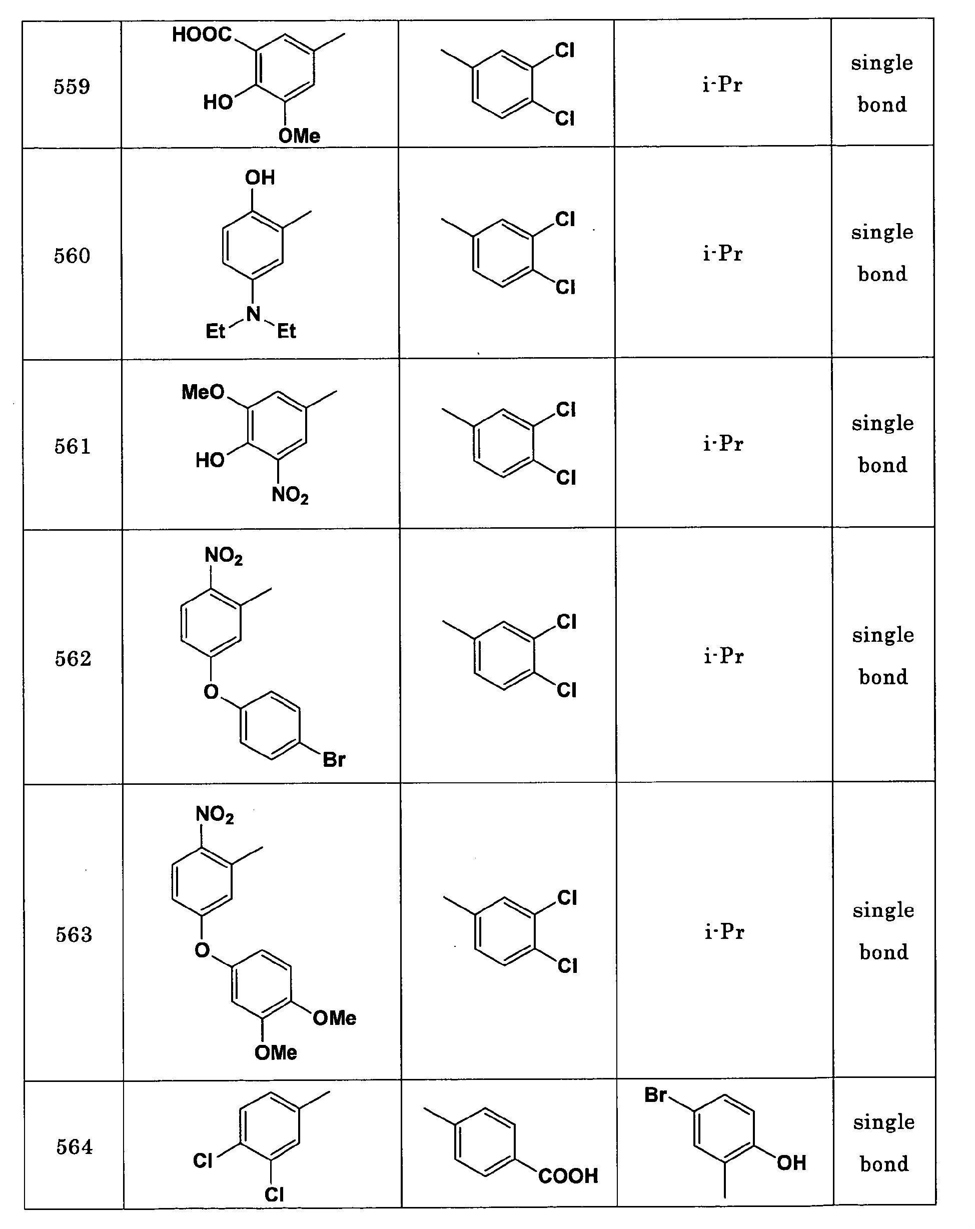

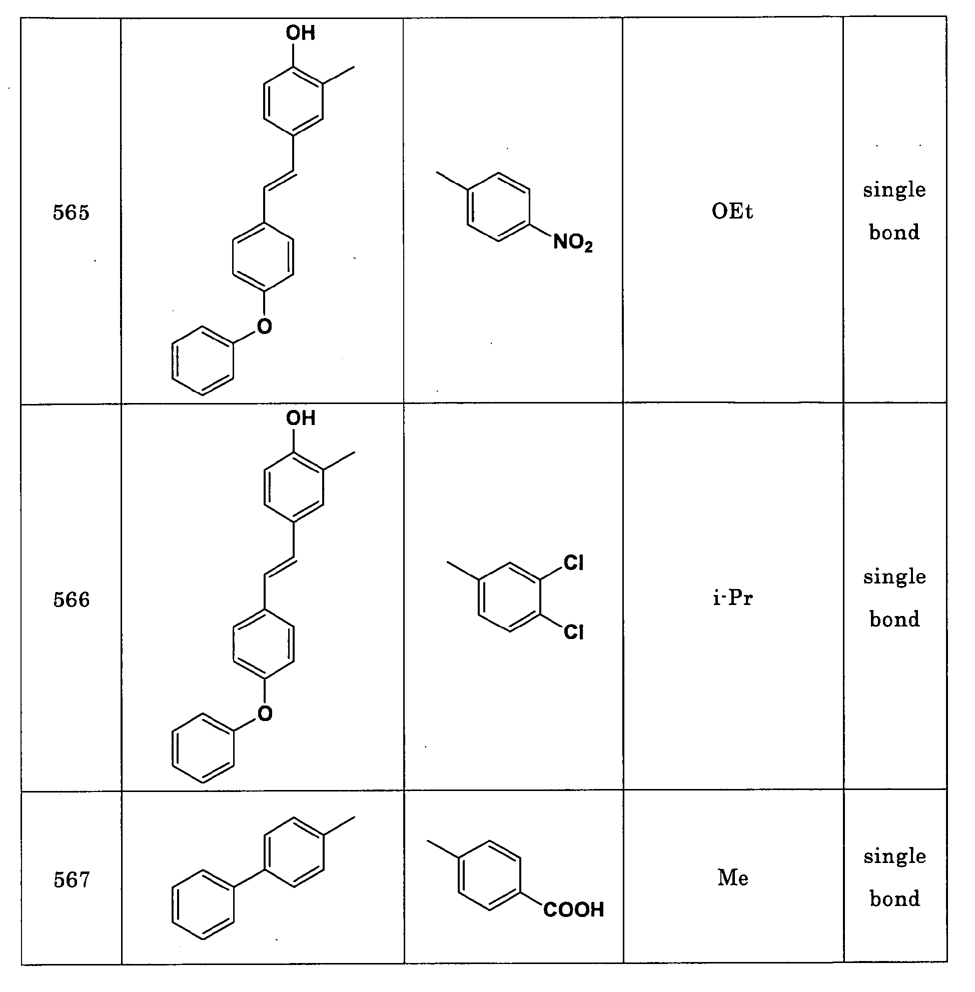

The compounds represented by the general formula (I) or (II) can be prepared, for example, by methods shown bellow. However the method for the preparation of the aforementioned compounds is not limited to the following methods.

The compounds represented by the general formula (I) can be prepared, for example, by a method described in the following reaction scheme 1, wherein each of R¹, R², W and Z has the same meaning as that described above.

### [Reaction Scheme 1]

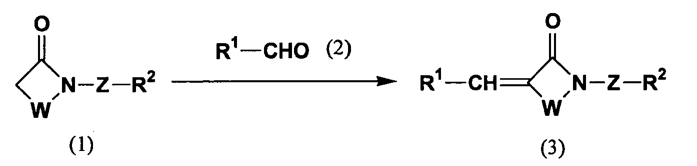

The target compound (3) can be prepared by reacting the 5-membered hetero ring (1) with the aldehyde (2) in the presence of a catalyst. As the catalyst, examples include, for example, salts such as sodium acetate, ammonium acetate and potassium acetate; acids such as acetic acid and propionic acid; bases such as piperidine and pyrrolidine; or a mixture thereof. As the solvent, any solvent can be used as long as it does not have an undesired effect on the reaction, and examples include, for example, alcohols such as methanol, ethanol and propanol; halogenated solvents such as dichloromethane, dichloroethane and chloroform; ethers such as tetrahydrofuran and 1,4-dioxane; aromatic solvents such as benzene, toluene, monochlorobenzene and o-dichlorobenzene; amides such as N,N-dimethylformamide and N-methylpyrrolidone; organic acids such as acetic acid and propionic acid; or a mixed solvent thereof. The reaction temperature is not limited. The reaction is usually carried out at from 0°C to the boiling point of the solvent which is to be used, preferably at from room temperature to 150°C. The reaction time may change depending on the reaction temperature. The reaction is usually carried out for from 10 minutes to 3 days, preferably for from 30 minutes to 6 hours. Some of the target compounds (3) are commercially available and can easily be obtained.

The aforementioned 5-membered hetero ring (1) can be prepared, for example, by methods shown bellow.

In the aforementioned 5-membered hetero ring (1), the compound wherein W is -S-C(=O)-, and Z is a connecting group wherein a number of atoms in a main chain is 1 to 3 (said connecting group may be substituted) can be prepared, for example, by a method described in the following reaction scheme 2, wherein R² has the same meaning as that described above, Z represents a connecting group wherein a number of atoms in a main chain is 1 to 3 (said connecting group may be substituted), V represents a halogen atom or hydroxy group.

### [Reaction Scheme 2]

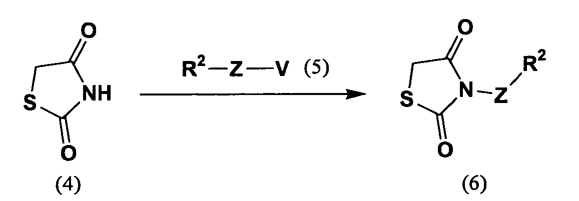

The compound (6) can be prepared by reacting thiazolidine-2,4-dione (4) with the compound (5) wherein V is a halogen atom in a solvent, in the presence of a base. As the base, examples include, for example, sodium hydroxide, potassium carbonate, triethylamine and pyridine. As the solvent, any solvent can be used as long as it does not have an undesired effect on the reaction, and examples include, for example, alcohols such as methanol, ethanol and propanol; halogenated solvents such as dichloromethane, dichloroethane and chloroform; ethers such as tetrahydrofuran and 1,4-dioxane; aromatic solvents such as benzene, toluene, monochlorobenzene and o-dichlorobenzene; amides such as N,N-dimethylformamide and N-methylpyrrolidone; organic acids such as acetic acid and propionic acid; or a mixed solvent thereof; or a mixed solvent thereof with water. The reaction temperature is not limited. The reaction is usually carried out at from 0°C to the boiling point of the solvent which is to be used, preferably at from room temperature to 150°C. The reaction time may change depending on the reaction temperature. The reaction is usually carried out for from 10 minutes to 3 days, preferably for from 30 minutes to 6 hours.

Furthermore, the compound (6) can also be prepared by the "Mitsunobu Reaction" of thiazolidine-2,4-dione (4) with the compound (5) wherein V is hydroxy group. As the reagent for the "Mitsunobu Reaction," examples include a combination of azodicarboxylic acid diesters such as diethyl azodicarboxylate, and tri-aryl-phosphines such as triphenylphosphine. As the solvent, any solvent can be used as long as it does not have an undesired effect on the reaction, and examples include, for example, halogenated solvents such as dichloromethane, dichloroethane and chloroform; ethers such as tetrahydrofuran and 1,4-dioxane; or a mixed solvent thereof. The reaction temperature is not limited. The reaction is usually carried out at from 0°C to 100°C, preferably at from room temperature to 80°C. The reaction time may change depending on the reaction temperature. The reaction is usually carried out for from 10 minutes to 3 days, preferably for from 30 minutes to 24 hours. The "Mitsunobu Reaction" is described in the following literature. "Synthesis," (Germany), 1981, p.1-28.

In the aforementioned 5-membered hetero ring (1), the compound wherein W is -S-C(=S)-, and Z is a single bond can be prepared, for example, by a method described in the following reaction scheme 3, wherein R² has the same meaning as that described above, Z represents a single bond.

### [Reaction Scheme 3]

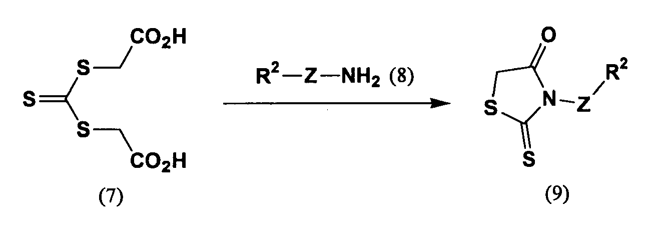

The compound (9) can be prepared by reacting bis(carboxymethyl)trithiocarbonate (7) with the amine (5) in a solvent, in the presence or absence of a base. As the base, examples include, for example, sodium hydroxide, potassium carbonate, sodium hydrogencarbonate, triethylamine and pyridine. As the solvent, any solvent can be used as long as it does not have an undesired effect on the reaction, and examples include, for example, alcohols such as methanol, ethanol and propanol; ethers such as tetrahydrofuran and 1,4-dioxane; or a mixed solvent thereof; or a mixed solvent thereof with water. The reaction temperature is not limited. The reaction is usually carried out at from 0°C to the boiling point of the solvent which is to be used, preferably at from room temperature to 150°C. The reaction time may change depending on the reaction temperature. The reaction is usually carried out for from 10 minutes to 3 days, preferably for from 30 minutes to 6 hours.

In the aforementioned 5-membered hetero ring (1), the compound wherein W is -NH-C(=S)-, and Z is a single bond can be prepared, for example, by a method described in or referred to the following well-known literature. "Synthetic Communications," (USA), 1999, Vol. 29, No. 11, p.1997-2005.

In the aforementioned 5-membered hetero ring (1), the compound wherein W is -C(R³)=N- can be prepared, for example, by a method described in the following reaction scheme 4, wherein each of R², R³ and Z has the same meaning as that described above, E represents carboxy group which is esterified.

### [Reaction Scheme 4]

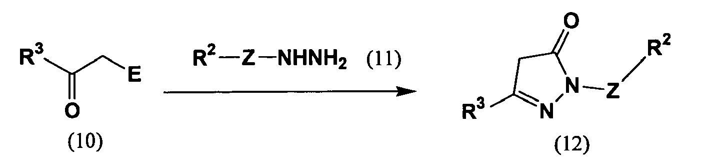

The compound (12) can be prepared by reacting *β* -ketoester (10) with the hydrazine (11) in a solvent, in the presence or absence of a dehydrating agent and/or a catalyst. As the dehydrating agent, examples include, for example, molecular sieves. As the catalyst, examples include, for example, salts such as sodium acetate, ammonium acetate and potassium acetate; acids such as sulfuric acid, acetic acid and propionic acid; bases such as piperidine and pyrrolidine; or a mixture thereof. As the solvent, any solvent can be used as long as it does not have an undesired effect on the reaction, and examples include, for example, alcohols such as methanol, ethanol and propanol; halogenated solvents such as dichloromethane, dichloroethane and chloroform; ethers such as tetrahydrofuran and 1,4-dioxane; aromatic solvents such as benzene, toluene, monochlorobenzene and o-dichlorobenzene; amides such as N,N-dimethylformamide and N-methylpyrrolidone; organic acids such as acetic acid and propionic acid; or a mixed solvent thereof. The reaction temperature is not limited. The reaction is usually carried out at from 0°C to the boiling point of the solvent which is to be used, preferably at from room temperature to 150°C. The reaction time may change depending on the reaction temperature. The reaction is usually carried out for from 10 minutes to 3 days, preferably for from 30 minutes to 6 hours.

In the aforementioned reaction, when the hydrazine (11) forms salt with acids such as hydrochloric acid and sulfuric acid, an equivalent or more amount of the base to the amount of the hydrazine (11) is preferably used. Examples of said base include, for example, sodium hydroxide, potassium carbonate, sodium hydrogencarbonate, triethylamine and pyridine. Some of the 5-membered hetero rings (1) are commercially available and can easily be obtained.

In the aforementioned general formula (I), the compound wherein R¹ is a phenyl group which is substituted with a C₆-C₁₀ aryl-substituted carbamoyl group (said aryl group may be substituted, and said phenyl group may further be substituted with one or more substituents other than C₆-C₁₀ aryl-substituted carbamoyl group), or a phenyl group which is substituted with a heteroaryl-substituted carbamoyl group (said heteroaryl group may be substituted, and said phenyl group may further be substituted with one or more substituents other than heteroaryl-substituted carbamoyl group) can be prepared by condensation of the carboxylic acid, which is obtained by conversion of the corresponding carboxylic acid ester (whose examples include, for example, methyl ester, tert-butyl ester, benzyl ester, and 4-methoxybenzyl ester), and the C₆-C₁₀ arylamine or the heteroarylamine.

The method for conversion of the carboxylic acid ester to the carboxylic acid is not limited. A method using boron tribromide is preferred. As the solvent, any solvent can be used as long as it does not have an undesired effect on the reaction. Halogenated solvents such as dichloromethane are preferably used. The reaction temperature is not limited. The reaction is usually carried out at from -20°C to room temperature, preferably at from 0°C to room temperature. The reaction time may change depending on the reaction temperature. The reaction is usually carried out for from 10 minutes to 3 days, preferably for from 30 minutes to 6 hours. When the tert-butyl ester is used, a method using acids such as hydrochloric acid, sulfuric acid, formic acid and acetic acid is also preferred. As the solvent, any solvent can be used as long as it does not have an undesired effect on the reaction. Alcohols such as methanol, ethanol and propanol; water; or a mixed solvent thereof are preferably used. The aforementioned acids may be used as the solvent. The reaction temperature is not limited. The reaction is usually carried out at from 0°C to the boiling point of the solvent which is to be used, preferably at from room temperature to 80°C. The reaction time may change depending on the reaction temperature. The reaction is usually carried out for from 10 minutes to 3 days, preferably for from 30 minutes to 6 hours.

The method for condensation of the carboxylic acid and the C₆-C₁₀ arylamine or the heteroarylamine is not limited, however,
(1) a method using phosphorus trichloride as a condensing agent;
(2) a method using phosphorus oxychloride as a condensing agent; and
(3) a method using (1-(3-dimethylaminopropyl)-3-ethylcarbodiimide hydrochloride (WSCI·HCl) as a condensing agent are preferred.

As the solvent, when phosphorus trichloride is used as a condensing agent, any solvent can be used as long as it does not have an undesired effect on the reaction. Aromatic solvents such as benzene, toluene, monochlorobenzene and o-dichlorobenzene are preferably used. The reaction temperature is not limited. The reaction is usually carried out at from room temperature to the boiling point of the solvent which is to be used, preferably at from 80°C to 120°C. The reaction time may change depending on the reaction temperature. The reaction is usually carried out for from 10 minutes to 3 days, preferably for from 30 minutes to 6 hours.

When phosphorus oxychloride is used as a condensing agent, it is preferred that the reaction is carried out by addition of a base such as triethylamine and pyridine. As the solvent, any solvent can be used as long as it does not have an undesired effect on the reaction. Halogenated solvents such as dichloromethane, dichloroethane and chloroform; ethers such as tetrahydrofuran and 1,4-dioxane; or a mixed solvent thereof are preferably used. The reaction temperature is not limited. The reaction is usually carried out at from 0°C to the boiling point of the solvent which is to be used, preferably at from 0°C to room temperature. The reaction time may change depending on the reaction temperature. The reaction is usually carried out for from 10 minutes to 3 days, preferably for from 30 minutes to 6 hours.

When (1-(3-dimethylaminopropyl)-3-ethylcarbodiimide hydrochloride is used as a condensing agent, it is preferred that the reaction is carried out by addition of dimethylaminopyridine (DMAP) for promoting the reaction. As the solvent, any solvent can be used as long as it does not have an undesired effect on the reaction. Halogenated solvents such as dichloromethane, dichloroethane and chloroform; ethers such as tetrahydrofuran and 1,4-dioxane; amides such as N,N-dimethylformamide and N-methylpyrrolidone; or a mixed solvent thereof are preferably used. The reaction temperature is not limited. The reaction is usually carried out at from 0°C to the boiling point of the solvent which is to be used, preferably at from 0°C to room temperature. The reaction time may change depending on the reaction temperature. The reaction is usually carried out for from 10 minutes to 3 days, preferably for from 30 minutes to 24 hours.

In the aforementioned reaction, when the functional group protection or deprotection; or the functional group introduction or modification needs to be carried out, methods described in or referred to the following various literature well known to by those skilled in the art can be used.
"Protective Groups in Organic Syntheses," (USA), Third edition, Theodra W. Green, Peter G.M. Wuts, Eds., Apr. in 1999, John Wiley & Sons, Inc.;
"Handbook of Reagents for Organic Synthesis," (USA), 4 Volumes, Jun. in 1999, John Wiley & Sons, Inc.;
"Fiesers' Reagents for Organic Synthesis," (USA), Vol. 1 - Vol. 20, John Wiley & Sons, Inc.;
"Comprehensive Organic Transformations: A Guide to Functional Group Preparations," (USA), Second edition, Richard C. Larock, Ed., Oct. in 1999, John Wiley & Sons, Inc.;
"Compendium of Organic Synthetic Methods," (Canada), Vol. 1 - Vol. 10, John Wiley & Sons, Inc.; and
"Encyclopedia of Reagents for Organic Synthesis," (USA), 8 Volumes, Nov. in 1995, John Wiley & Sons, Inc..

Furthermore, the compounds represented by the general formula (II) can also prepared in the same manner as the aforementioned general formula (I).

The intermediates and the target compounds in the aforementioned preparations can be isolated and purified by using methods for isolation and purification ordinarily used in the field of organic synthetic chemistry, for example, neutralization, filtration, extraction, drying, concentration, recrystallization, various chromatographic techniques and the like. The intermediates may be used in subsequent reactions without purification. When a salt of compound of the general formula (I) or (II) is desired, a product obtained in the form of a salt may be purified without any treatment. When a product is obtained in a free form, a salt can be formed by dissolving or suspending the product in a suitable organic solvent, and then adding an acid or base. It is also possible to convert a compound represented by the general formula (I) or (II) obtained in the form of a salt into a compound in a free form, and then convert the result into an appropriate salt.

In the examples of the present specification, preparation methods of typical compounds included in the general formula (I) or (II) are explained in details. Therefore, those skilled in the art can prepare any compound fall within the general formula (I) or (II) based on explanations in the aforementioned general preparation methods and the examples, by choosing appropriate reaction raw materials, reagents, and reaction conditions, and by applying appropriate modification and alteration of methods disclosed in the examples, if necessary.

The medicament of the present invention can be used for preventive and/or therapeutic treatment of diseases caused by an expression of PAI-1 or an enhancement of PAI-1 activity. The term "therapeutic treatment" used in the present specification includes prevention of progression of diseases and the term "preventive treatment" includes the prevention of reoccurrence. The medicament of the present invention can be used, for example, for preventive and/or therapeutic treatment of diseases caused by thrombogenesis, fibrogenesis, accumulation of visceral fat, cell proliferation, angiogenesis, deposition and remodeling of extracellular matrix, and cell movement and migration.

More specifically, the medicament of the present invention can be used for preventive and/or therapeutic treatment of one or more diseases selected from ischemic cerebro-vascular accidents such as cerebral infarction, transient ischemic attack, cerebral stroke, multiinfarct dementia and the like; acute coronary syndromes such as angina pectoris, myocardial infarction and the like; intraatrial thrombosis caused by atrial fibrillation and the like; embolisms and thromboses such as pulmonary embolism and the like; peripheral vascular embolisms and thromboses such as deep vein thrombosis, thrombotic phlebitis and the like; thrombus after bypass graft surgery; arteriosclerosis; disseminated intracascular coagulation; acute coronary occlusion and restenosis after percutaneous transluminal coronary angioplasty, angiopathy and thromboses caused by immunologic aberrations such as antiphospholipid syndrome and the like; angiopathy and thromboses caused by congenital thrombotic tendency such as genetic aberration and the like; thrombotic renal diseases such as glomerulonephritis and the like; nonbacterial thrombotic endocarditis; severe infectious diseases such as sepsis and the like; fibrin-dependent pain in arthritis; diabetic complications such as retinopathy, nephropathy, neuropathy, peripheral circulation disorder and the like; hypertension; diabetes; hyperinsulinemia; hypercholesterolemia; insulin resistance; hyperlipidemia; obesity; cancers such as lung cancer, pancreatic cancer, colon cancer, gastric cancer, prostatic cancer, breast cancer, cervical cancer, ovarian cancer and the like; invasion of cancer; metastasis of cancer; asthma; fibroses such as pulmonary fibrosis, renal fibrosis and the like; acute rejections and arterial lesions after organ transplantation such as cardiac transplantation, renal transplantation or the like; and wounds.

As the active ingredient of the medicament of the present invention, one or more kinds of substances selected from the group consisting of the compounds represented by the general formula (I) or (II), and a pharmacologically acceptable salt thereof, and a hydrate thereof and a solvate thereof can be used. Although the aforementioned substances, per se, may be administered, it is generally desirable to provide the medicament in a form of a pharmaceutical composition comprising the aforementioned substance as the active ingredient and one or more pharmaceutical additives. The pharmaceutical composition may optionally be added with one or more of other pharmaceutically active ingredients. The medicament of the present invention can be administered to human and other mammals.

The form of the pharmaceutical is not particularly limited, and an appropriate form most suitable for a purpose of the prophylactic or therapeutic treatment can be selected from forms of pharmaceutical preparations for oral or parenteral administration. Examples of pharmaceutical preparations suitable for oral administration include, for example, tablets, granules, subtilized granules, powders, hard capsules, soft capsules, pills, troches, chewable formulations, syrups, emulsions, suspensions, solutions and the like. Examples of pharmaceutical preparations suitable for parenteral administration include, for example, injections (for subcutaneous injection, intramuscular injection, intravenous injection or the like), drip infusions, suppositories, transdermal preparations, transmucosal preparations, eye drops, nasal drops, ear drops, ointments, emplastrums, creams, tapes, patches, inhalants, sprays and the like. However, the preparations are not limited to these examples. It is also possible to provide liquid preparations such as injections and drip infusions as, for example, pharmaceutical compositions in a form of powder and dissolve or suspend the preparations in suitable medium (for example, water, physiological saline, glucose infusion, buffer and the like) upon use.

The pharmaceutical compositions can be prepared according to well-known methods by those skilled in the art, by using one or more kinds of pharmaceutical additives widely used in the art. The pharmaceutical additives can be chosen by those skilled in the art depending on the forms of formulations. Examples of the pharmacologically and pharmaceutically acceptable pharmaceutical additive include, for example, excipients such as lactose, sorbit, corn starch, calcium carbonate, and silicon dioxide; disintegrators such as starch and gelatin; binders such as hydroxymethylcellulose and polyvinyl alcohol; lubricants such as magnesium stearate and polyethylene glycol; coating agents such as hydroxypropylmethylcellulose; colorants such as Brilliant Blue, erythrosine, and Tartazine; base materials such as cacao butter, lauric lipid, white petrolatum; propellants such as compressed gases; tackifiers such as sodium polyacrylate; base fabrics such as cotton cloth; isotonic agents such as glucose; pH modifiers such as inorganic acids, organic acids, inorganic bases, and organic bases. However, pharmaceutical additives are not limited to these examples, and any additives available for those skilled in the art can be used.

Although a dose and administration frequency of the medicament of the present invention is not particularly limited, it is preferred to increase or decrease the dose appropriately depending on various factors such as purpose of administration, type of a disease, the age, body weight and symptoms of a patient. The dose of the medicament of the present invention may be, for example, for oral administration, 0.01 to 5,000 mg per day for an adult as the amount of an active ingredient. When the medicament is used as an injection, the dose may be 0.001 to 500 mg per day for an adult as the amount of an active ingredient. The aforementioned daily dose of the medicament of the present invention may be administered once a day, or alternatively, two or three times a day as divided portions with appropriate intervals. The dose may be administered intermittently.

Oral or parenteral administration of the medicament of the present invention may be carried out preoperatively, when the medicament of the present invention is used for preventive and/or therapeutic treatment of intravascular lesions after vascular transplantation or organ transplantation, or after blood circulation restoration, whose examples include, for example, thrombus after bypass graft surgery, acute coronary occlusion and restenosis after percutaneous transluminal coronary angioplasty, and arterial lesions after organ transplantation such as cardiac transplantation, renal transplantation or the like. Furthermore, oral or parenteral administration of the medicament of the present invention may be carried out intraoperatively and/or postoperatively in addition to the aforementioned preoperative administration, if necessary.

As the form of administration of the medicament of the present invention, a medical device for intravascular treatment, which contains the medicament of the present invention in a form that enables a sustained release of said medicament may also be used. Examples of said medical device include, for example, intravascular stent, intravascular balloon catheter and the like. Examples include surgical devices such as artificial blood vessel, medical tube, and medical clip; artificial valve; part of artificial heart or whole artificial heart; endoscope or the like. The materials of the medical devices are not particularly limited. Any materials generally used for manufacturing medical devices may be used, whose examples include, for example, metals, plastics, polymers, biodegradable plastics, biodegradable polymers, proteins, cellulose, ceramics and the like. Said medical device contains one or more kinds of substances selected from the group consisting of the compounds represented by the general formula (I) or (II), and a pharmacologically acceptable salt thereof, and a hydrate thereof and a solvate thereof in a form that enables a sustained release of said substances, and is useful for preventive and/or therapeutic treatment of intravascular lesions after vascular transplantation or organ transplantation, or after blood circulation restoration. The form that enables a sustained release of one or more kinds of substances selected from the group consisting of the aforementioned compounds and a pharmacologically acceptable salt thereof, and a hydrate thereof and a solvate thereof is not particularly limited. Examples of said form include, for example, a form wherein the medicament prepared as a sustained release preparation is pasted or applied on the surface of the medical device, in addition to a form wherein the surface of the medical device is coated by applying the substances, a form wherein the material of the medical device is impregnated with the substances. However, the forms are not limited to these examples. Preferred examples of said medical device include an indwelling intravascular stent, and the stent contains one or more kinds of substances selected from the group consisting of the aforementioned compounds and a pharmacologically acceptable salt thereof, and a hydrate thereof and a solvate thereof in a form that enables a sustained release of said substances.

Base materials for preparing the stent are not particularly limited. Stainless steel (SUS316, SUS304), Nitinol (Ni-Ti alloy), metallic materials such as tantalum, and polymer materials can be generally used. Biodegradable polymer materials can also be used. As for the polymer materials, types of the materials are not particularly limited so far that they have blood compatibility and are not dissolvable in blood. The method for producing said stent is not particularly limited. For example, when the stent base material consists of a metal, a polymer coating layer containing one or more kinds of substances selected from the group consisting of the aforementioned compound and a pharmacologically acceptable salt thereof, and a hydrate thereof and a solvate thereof can be provided on the surface of the stent base material, or when the base material consists of a polymer material, one or more kinds of substances selected from the group consisting of the aforementioned compound and a pharmacologically acceptable salt thereof, and a hydrate thereof and a solvate thereof may be introduced into the polymer material upon molding thereof, or a polymer coating layer containing one or more kinds of substances selected from the group consisting of the aforementioned compound and a pharmacologically acceptable salt thereof, and a hydrate thereof and a solvate thereof can be provided on the surface of the stent base material.

Types of the polymer materials to form the coating layer are not particularly limited so far that the materials have blood compatibility and are not dissolvable in blood. For example, polyester type elastomers , polyamide type elastomers, polyurethane type elastomers, (meth)acrylate ester type polymers, polyvinyl acetates, poly(ethylene-vinyl alcohol) copolymers and the like can be used. A polymer material having compliance respondable to expansion of the stent is more desirable.

Concentrations of one or more kinds of substances selected from the group consisting of the aforementioned compound and a pharmacologically acceptable salt thereof, and a hydrate thereof and a solvate thereof and the aforementioned polymer in a solution for coating can be suitably chosen depending on conditions including, for example, an amount to be eluted (elution rate) of the aforementioned active ingredient from a surface of the coated layer and a shape of the stent. The stent is desirably designed so as to have a sustained release property in such a degree that the effectiveness of the medicament of the present invention is maintained for at least a given period of time. It is generally desirable to design the stent so that a local concentration of the active ingredient can be 10 *µ* M to 1nM.

Methods for producing a stent and means for sustained release are well known and conventionally used by artisans in the fields of stents, artificial organs and the like. For example, as for drug-releasing stents for prevention of restenosis, a review by Kozuma is published (Kozuma, K., Coronary Intervention, Vol. 1, pp.58-62), and specific drug-releasing stents are described in Catheterization and Cardiovascular Interventions, Vol.55, pp.409-417, 2002; New England Journal of Medicine, Vol.346, 1770-1771 and 1773-1780, 2002; WO02/064065 and the like. By referring to these publications, those skilled in the art can readily produce these stents. The intravascular balloon catheter and other medical devices for intravascular treatment can also be readily produced by suitably applying these known techniques.

The medicament of the present invention and these medical devices may be used in combination. For example, oral or parenteral administration of the medicament of the present invention may be carried out before blood circulation restoration, and then blood circulation restoration is carried out using these medical devices. Furthermore, oral or parenteral administration of the medicament of the present invention may be carried out intraoperatively and/or postoperatively in addition to the aforementioned preoperative administration, if necessary.

The medicaments of the present invention and preventive and/or therapeutic methods of the present invention can be applied to human as well as mammals other than human, whose examples include, for example, monkey, dog, pig, rabbit, guinea pig, hamster, rat, and mouse.

### Examples

The present invention will be explained more specifically with reference to the following examples. However the scope of the present invention is not limited to the following examples. The compound number in the following examples correspond to those in the table shown above. And the commercially available compounds, which were purchased and used for the examinations, are contained in these examples. As for such compounds, the suppliers of the reagents and the catalog code numbers are shown.

### Example 1: The compound of Compound No. 1.

This compound is a commercially available compound.
Supplier: LaboTest (Germany).
Catalog code number: LT00055729.

### Example 2: Preparation of the compound of Compound No. 2.

(1) 3-[3,5-Bis(trifluoromethyl)benzyl]thiazolidine-2,4-dione.
   5N Sodium hydroxide solution(0.5ml) was added to a solution of 2,4-thiazolidinedione(198.7mg, 1.69mmol), 3,5-bis(trifluoromethyl)benzyl bromide(0.50g, 1.63mmol) in ethanol(5ml), and the mixture was refluxed for 4 hours. The reaction mixture was cooled, poured into water, and extracted with ethyl acetate. The organic layer was washed with water and brine, and dried over anhydrous sodium sulfate. The residue obtained by evaporation of the solvent under reduced pressure was purified by chromatography on silica gel(hexane:ethyl acetate=3:1→2:1) and recrystallized from chloroform/hexane to give the title compound(405.6mg, 72.5%) as a white crystal.
   ¹H-NMR(CDCl₃): *δ* 4.01(2H,s), 4.87(2H,s), 7.84(1H,s), 7.86(2H,s).
(2) 5-(3,5-Dibromo-2-hydroxybenzylidene)-3-[3,5-bis(trifluoromethyl)benzyl]-thiazolidine-2,4-dione (Compound No. 2).
   Acetic acid(2 drops) and piperidine(2 drops) were added to a mixture of 3,5-dibromosalicylaldehyde(43.2mg, 0.15mmol),
   3-[3,5-bis(trifluoromethyl)benzyl]thiazolidine-2,4-dione(53.Omg, 0.15mmol) and toluene(4ml), and the mixture was stirred at 90°C for 40 minutes. The residue obtained by concentration of the reaction mixture under reduced pressure was purified by chromatography on silica gel(hexane:ethyl acetate=3:1) to give the title compound(71.5mg, 76.5%) as a yellowish white powder.
   ¹H-NMR(CDCl₃): *δ* 5.00(2H,s), 7.48(1H,d,J=2.4Hz), 7.68(1H,d,J=2.4Hz), 7.85(1H,s), 7.90(2H,s), 8.18(1H,s).

### Example 3: Preparation of the compound of Compound No. 3.

(1) 3-(3,5-Dimethoxybenzyl)thiazolidine-2,4-dione.
   The title compound was obtained in the same manner as the Example 2(1) using the following raw materials.
   Raw materials: 3,5-dimethoxybenzyl bromide and 2,4-thiazolidinedione.
   Yield: 65.2%(white crystal).
   ¹H-NMR(CDCl₃): *δ* 3.77(6H,s), 3.95(2H,s), 4.70(2H,s), 6.39(1H,t,J=2.1Hz), 6.53(2H,d,J=2.4Hz).
(2) 5-(2,3-Dihydroxybenzylidene)-3-(3,5-dimethoxybenzyl)thiazolidine-2,4-dione (Compound No. 3).
   The title compound was obtained in the same manner as the Example 2(2) using the following raw materials.
   Raw materials: 2,3-dihydroxybenzaldehyde and
   3-(3,5-dimethoxybenzyl)thiazolidine-2,4-dione.
   Yield: 29.1%(yellow powder).
   ¹H-NMR(DMSO-d₆): *δ* 3.72(6H,s), 4.75(2H,s), 6.42-6.43(3H,m), 6.79(1H,t, J=7.8Hz), 6.86(1H,dd,J=7.8,1.8Hz), 6.92(1H,dd, J=7.8,1.8Hz), 8.19(1H,s), 9.51(1H,s), 9.88(1H,s).

### Example 4: Preparation of the compound of Compound No. 4.

The title compound was obtained in the same manner as the Example 2(2) using the following raw materials.
Raw materials: 3,4-dihydroxybenzaldehyde and
3-[3,5-bis(trifluoromethyl)benzyl]thiazolidine-2,4-dione(compound of the Example 2(1)).
Yield: 81.8%(light yellow powder).
¹H·NMR(DMSO.d₆): *δ* 5.02(2H,s), 6.89(1H,d,J=8.1Hz), 7.00-7.05(2H,m), 7.79(1H,s), 8.03(2H,s), 8.07(1H,s), 9.51(1H,s), 9.92(1H,s).

### Example 5: Preparation of the compound of Compound No. 5.

The title compound was obtained in the same manner as the Example 2(2) using the following raw materials.
Raw materials: 2,4,5-trimethoxybenzaldehyde and
3-[3,5-bis(trifluoromethyl)benzyl]thiazolidine-2,4-dione(compound of the Example 2(1)).
Yield: 44.9%(yellow powder).
¹H-NMR(DMSO-d₆): *δ* 3.76(3H,s), 3.90(3H,s), 3.92(3H,s), 5.03(2H,s), 6.82(1H,s), 6.96(1H,s), 8.03(2H,s), 8.07(1H,s), 8.08(1H,s).

### Example 6: Preparation of the compound of Compound No. 6.

The title compound was obtained in the same manner as the Example 2(2) using the following raw materials.
Raw materials: 3-methylsalicylaldehyde and
3-[3,5-bis(trifluoromethyI)benzyl]thiazolidine-2,4-dione(compound of the Example 2(1)).
Yield: 37.5%(light yellowish white powder).
¹H-NMR(DMSO-d₆): *δ* 2.22(3H,s), 5.03(2H,s), 6.88-6.94(1H,m), 7.22-7.26(2H,m), 8.04(2H,s), 8.08(1H,s), 8.24(1H,s), 9.48(1H,brs).

### Example 7: Preparation of the compound of Compound No. 7.

The title compound was obtained in the same manner as the Example 2(2) using the following raw materials.
Raw materials: 3-hydroxybenzaldehyde and
3- [3,5-bis(trifluoromethyl)benzyl]thiazolidine-2,4-dione(compound of the Example 2(1)).
Yield: 52.0%(white powder).
¹H-NMR(DMSO-d₆): *δ* 5.03(2H,s), 6.90(1H,ddd,J=8.1,1.8,0.6Hz), 7.02(1H,t,J=1.8Hz), 7.08(1H,dd,J=8.1,0.6Hz), 7.35(1H,t,J=8.1Hz), 7.87(1H,s), 8.06(2H,s), 8.07(1H,s), 9.87(1H,s).

### Example 8: Preparation of the compound of Compound No. 8.

(1) 3-(3,4-Dichlorobenzyl)thiazolidine-2,4-dione.
   The title compound was obtained in the same manner as the Example 2(1) using the following raw materials.
   Raw materials: 3,4-dichlorobenzyl bromide and 2,4-thiazolidinedione.
   Yield: 78.9%(white crystal).
   ¹H-NMR(CDCIs):*δ* 3.95(2H,s), 4.70(2H,s), 7.25(1H,dd,J=8.4,2.1Hz), 7.40(1H,d,J=8.4Hz), 7.50(1H,d,J=2.4Hz).
(2) 5-(2,3-Dihydroxybenzylidene)- 3-(3,4-dichlorobenzyl)thiazolidine-2,4-dione (Compound No. 8).
   The title compound was obtained in the same manner as the Example 2(2) using the following raw materials.
   Raw materials: 2,3-dihydroxybenzaldehyde and 3-(3,4-dichlorobenzyl)thiazolidine-2,4-dione.
   Yield: 80.5%(yellow crystal).
   ¹H-NMR(DMSO-d₆): *δ* 4.83(2H,s), 6.76-6.87(2H,m), 6.92(1H,dd,J=7.5,1.8Hz), 7.30(1H,dd,J=8.1,1.8Hz), 7.61-7.63(2H,m), 8.19(1H,s), 9.58(1H,brs), 9.89(1H,brs).

### Example 9: Preparation of the compound of Compound No. 9.

The title compound was obtained in the same manner as the Example 2(2) using the following raw materials.
Raw materials: 3,4-dihydroxybenzaldehyde and
3-(3,4-dichlorobenzyl)thiazolidine-2,4-dione(compound of the Example 8(1)).
Yield: 87.8%(yellow crystal).
¹H-NMR(DMSO-d₆): *δ* 4.83(2H,s), 6.88(1H,d,J=7.8Hz), 7.00-7.03(2H,m), 7.29(1H,dd,J=8.4,1.8Hz), 7.60-7.63(2H,m), 7.78(1H,s), 9.50(1H,s), 9.92(1H,s).

### Example 10: Preparation of the compound of Compound No. 10.

The title compound was obtained in the same manner as the Example 2(2) using the following raw materials.
Raw materials: 3,4,5-trihydroxybenzaldehyde and
3-(3,4-dichlorobenzyl)thiazolidine-2,4-dione(compound of the Example 8(1)).
Yield: 44.7%(yellow crystal).
¹H-NMR(DMSO-d₆): *δ* 4.82(2H,s), 6.61(2H,s), 7.28(1H,dd,J=8.4,2.1Hz), 7.60-7.63(2H,m), 7.68(1H,s), 9.31(3H,brs).

### Example 11: Preparation of the compound of Compound No. 11.

(1) 3-(3,5-Dichlorobenzyl)thiazolidine-2,4-dione.
   Diethyl azodicarboxylate(40% solution in toluene; 0.34 ml) was added to a solution of 2,4-thiazolidinedione(0.10g, 0.85 mmol), 3,5-dichlorobenzylalcohol (0.15g, 0.85 mmol), triphenylphosphine(0.22g, 0.85 mmol) in tetrahydrofuran(5ml) under ice cooling and argon atmosphere, and the mixture was stirred at room temperature for 16 hours. The residue obtained by concentration of the reaction mixture under reduced pressure was purified by chromatography on silica gel(hexane:ethyl acetate=2:1) to give the title compound(0.225g, 95.5%) as a colourless oil.
   ¹H-NMR(CDCl₃): *δ* 3.99(2H,s), 4.69(2H,s), 7.27-7.28(2H,m), 7.30-7.31(1H,m).
(2) 5-(2,3-Dihydroxybenzylidene)-3-(3,5-dichlorobenzyl)thiazolidine-2,4-dione (Compound No. 11).

The title compound was obtained in the same manner as the Example 2(2) using the following raw materials.
Raw materials: 2,3-dihydroxybenzaldehyde and 3-(3,5-dichlorobenzyl)thiazolidine-2,4-dione.
Yield: 42.0%(yellow powder).
¹H-NMR(DMSO-d₆): *δ* 4.84(2H,s), 6.80(1H,t,J=7.5Hz), 6.86(1H,dd,J=7.5,1.5Hz), 6.92(1H,dd,J=7.5,1.5Hz), 7.39(2H,d,J=1.8Hz), 7.57(1H,t,J=1.8Hz), 8.19(1H,s), 9.51(1H,s), 9.88(1H,s).

### Example 12: Preparation of the compound of Compound No. 12.

The title compound was obtained in the same manner as the Example 2(2) using the following raw materials.
Raw materials: 3,4-dihydroxybenzaldehyde and
3-(3,5-dichlorobenzyl)thiazolidine-2,4-dione(compound of the Example 11(1)).
Yield: 77.8%(yellow powder).
¹H-NMR(DMSO-d₆) :*δ* 4.83(2H,s), 6.89(1H,d,J=8.1Hz), 7.00-7.05(2H,m), 7.38(2H,d,J=1.8Hz), 7.56(1H,t,J=1.8Hz), 7.79(1H,s), 9.51(1H,s), 9.93(1H,s).

### Example 13: Preparation of the compound of Compound No. 13.

(1) 3-[3,5-Di(tert-butyl)-4-hydroxybenzyl]thiazolidine-2,4-dione.
   The title compound was obtained in the same manner as the Example 11(1) using the following raw materials.
   Raw materials: 3,5-di(tert-butyl)-4-hydroxybenzylalcohol and 2,4-thiazolidinedione.
   Yield: 88.1%(white crystal).
   ¹H-NMR(CDCl₃): *δ* 1.43(18H,s), 3.91(2H,s), 4.67(2H,s), 5.25(1H,s), 7.26(2H,s).
(2) 5-(2,3-Dihydroxybenzylidene)-3-[3,5-di(tert-butyl)-4-hydroxybenzyl]-thiazolidine-2,4-dione (Compound No. 13).

The title compound was obtained in the same manner as the Example 2(2) using the following raw materials.
Raw materials: 2,3-dihydroxybenzaldehyde and 3-[3,5-di(tert-butyl)-4-hydroxybenzyl]thiazolidine-2,4-dione.
Yield: 38.9%(light orange powder).
¹H-NMR(DMSO-ds): *δ* 1.35(18H,s), 4.70(2H,s), 6.76-6.86(2H,m), 6.91(1H,dd,J=7.5, 1.8Hz), 7.01(1H,s), 7.10(2H,s), 8.19(1H,s), 9.50(1H,s), 9.87(1H,s).

### Example 14: Preparation of the compound of Compound No. 14.

The title compound was obtained in the same manner as the Example 2(2) using the following raw materials.
Raw materials: 3,4-dihydroxybenzaldehyde and
3-[3,5-di(tert-butyl)-4-hydroxybenzyl]thiazolidine-2,4-dione(compound of the Example 13(1)).
Yield: 51.9%(light yellow powder).
¹H-NMR(DMSO-d₆): *δ* 1.35(18H,s), 4.86(2H,s), 6.88(1H,d,J=8.1Hz), 6.99-7.04(3H,m), 7.10(2H,s), 7.79(1H,s), 9.50(1H,s), 9.91(1H,s).

### Example 15: Preparation of the compound of Compound No. 15.

(1) 3-(2,4,5-trimethoxybenzyl)thiazolidine-2,4-dione.
   The title compound was obtained in the same manner as the Example 11(1) using the following raw materials.
   Raw materials: 2,4,5-trimethoxybenzylalcohol and 2,4-thiazolidinedione.
   Yield: 15.4%(white powder).
   ¹H-NMR(CDCl₃): *δ* 3.75(3H,s), 3.81(3H,s), 3.82(3H,s), 3.94(2H,s), 4.78(2H,s), 6.50(1H,s), 6.54(1H,s).
(2) 5-(2,3-Dihydroxybenzylidene)-3-(2,4,5-trimethoxybenzyl)thiazolidine-2,4-dione (Compound No. 15).

The title compound was obtained in the same manner as the Example 2(2) using the following raw materials.
Raw materials: 2,3-dihydroxybenzaldehyde and 3-(2,4,5-trimethoxybenzyl)thiazolidine-2,4-dione.
Yield: 35.8%(yellow powder).
¹H-NMR(DMSO-d₆): *δ* 3.65(3H,s), 3.77(3H,s), 3.78(3H,s), 4.74(2H,s), 6.70(1H,s), 6.74(1H,s), 6.76-6.87(2H,m), 6.91(1H,dd,J=8.1,1.8Hz), 8.16(1H,s), 9.66(2H,brs).

### Example 16: Preparation of the compound of Compound No. 16.

(1) 3-(3-Phenoxybenzyl)thiazolidine-2,4-dione.
   The title compound was obtained in the same manner as the Example 11(1) using the following raw materials.
   Raw materials: 3-phenoxybenzylalcohol and 2,4-thiazolidinedione.
   Yield: 80.9%(colourless oil).
   ¹H-NMR(CDCl₃): *δ* 3.96(2H,s), 4.74(2H,s), 6.91(1H,ddd,J=8.1,2.7,1.8Hz), 6.97-7.02(2H,m), 7.04-7.06(1H,m), 7.08-7.14(2H,m), 7.26(1H,d,J=8.1Hz), 7.30-7.37(2H,m).
(2) 5-(2,3-Dihydroxybenzylidene)-3-(3-phenoxybenzyl)thiazolidine-2,4-dione (Compound No. 16).

The title compound was obtained in the same manner as the Example 2(2) using the following raw materials.
Raw materials: 2,3-dihydroxybenzaldehyde and 3-(3-phenoxybenzyl)thiazolidine-2,4-dione.
Yield: 48.9%(yellow powder).
¹H-NMR(DMSO-d₆): *δ* 4.82(2H,s), 6.77-6.87(2H,m), 6.89-6.94(2H,m), 6.97-7.06(4H,m), 7.12-7.18(1H,m), 7.33-7.43(3H,m), 8.19(1H,s), 9.53(1H,s), 9.89(1H,s).

### Example 17: Preparation of the compound of Compound No. 17.

The title compound was obtained in the same manner as the Example 2(2) using the following raw materials.
Raw materials: 3,4-dihydroxybenzaldehyde and
3-(3-phenoxybenzyl)thiazolidine-2,4-dione(compound of the Example 16(1)).
Yield: 51.1%(light yellow powder).
¹H-NMR(DMSO-d₆): *δ* 4.81(2H,s), 6.87-6.92(2H,m), 6.96-7.06(6H,m), 7.13-7.18(1H,m), 7.33-7.43(3H,m), 7.78(1H,s), 9.52(1H,s), 9.94(1H,s).

### Example 18: Preparation of the compound of Compound No. 18.

(1) 3-(2,3-Dichlorobenzyl)thiazolidine-2,4-dione.
   The title compound was obtained in the same manner as the Example 11(1) using the following raw materials.
   Raw materials: 2,3-dichlorobenzylalcohol and 2,4-thiazolidinedione..
   Yield: 99%(colorless oil).
   ¹H-NMR(CDCl₃): *δ* 4.05(2H,s), 4.93(2H,s), 7.03-7.06(1H,m), 7.18(1H,t,J=8.1Hz), 7.42(1H,dd,J=8.1,1.8Hz).
(2) 5-(2,3-Dihydroxybenzylidene)-3-(2,3-dichlorobenzyl)thiazolidine-2,4-dione (Compound No. 18).

The title compound was obtained in the same manner as the Example 2(2) using the following raw materials.
Raw materials: 2,3-dihydroxybenzaldehyde and 3-(2, 3-dichlorobenzyl)thiazolidine-2,4-dione.
Yield: 62.6%(yellow powder).
¹H-NMR(DMSO-d₆): *δ* 4.93(2H,s), 6.78-6.89(2H,m), 6.93(1H,dd,J=7.5,1.5Hz), 7.23(1H,dd,J=8.1,1.2Hz), 7.36(1H,t,J=8.1Hz), 7.62(1H,dd,J=8.1,1.2Hz), 8.20(1H,s), 9.54(1H,s), 9.90(1H,s).

### Example 19: Preparation of the compound of Compound No. 19.

The title compound was obtained in the same manner as the Example 2(2) using the following raw materials.
Raw materials: 3,4-dihydroxybenzaldehyde and
3-(2,3-dichlorobenzyl)thiazolidine-2,4-dione(compound of the Example 18(1)).
Yield: 64.0%(yellow powder).
¹H-NMR(DMSO-d₆): *δ* 4.92(2H,s), 6.90(1H,d,J=7.8Hz), 7.02-7.06(2H,m), 7.21(1H,dd,J=7.8,1.2Hz), 7.35(1H,t,J=7.8Hz), 7.62(1H,dd,J=7.8;1.2Hz), 7.80(1H,s), 9.53(1H,s), 9.95(1H,s)..

### Example 20: Preparation of the compound of Compound No. 20.

(1) 3-(4-Phenylbenzyl)thiazolidine-2,4-dione.
   The title compound was obtained in the same manner as the Example 11(1) using the following raw materials.
   Raw materials: 4-phenylbenzylalcohol and 2,4-thiazolidinedione.
   Yield: 86.7%(white crystal).
   ¹H-NMR(CDCl₃): *δ* 3.96(2H,s), 4.81(2H,s), 7.32-7.38(1H,m), 7.40-7.49(4H,m), 7.53-7.58(4H,m).
(2) 5-(2,3-Dihydroxybenzylidene)-3-(4-phenylbenzyl)thiazolidine-2,4-dione (Compound No. 20).

The title compound was obtained in the same manner as the Example 2(2) using the following raw materials.
Raw materials: 2,3-dihydroxybenzaldehyde and 3-(4-phenylbenzyl)thiazolidine-2,4-dione.
Yield: 59.6%(yellow powder).
¹H-NMR(DMSO-d₆): *δ* 4.88(2H,s), 6.77-6.88(2H,m), 6.92(1H,dd,J=7.5,1.5Hz), 7.33-7.49(5H,m), 7.63-7.66(4H,m), 8.21(1H,s), 9.52(1H,s), 9.88(1H,s).

### Example 21: Preparation of the compound of Compound No. 21.

The title compound was obtained in the same manner as the Example 2(2) using the following raw materials.
Raw materials: 3,4-dihydroxybenzaldehyde and
3-(4-phenylbenzyl)thiazolidine-2,4-dione(compound of the Example 20(1)).
Yield: 83.2%(yellow powder).
¹H-NMR(DMSO-d₆): *δ* 4.87(2H,s), 6.89(1H,d,J=8.1Hz), 7.00-7.05(2H,m), 7.34-7.49(5H,m), 7.63-7.66(4H,m), 7.80(1H,s), 9.51(1H,s), 9.93(1H,s).

### Example 22: Preparation of the compound of Compound No. 22.

(1) 3-(2,4-Dichlorobenzyl)thiazolidine-2,4-dione.
   The title compound was obtained in the same manner as the Example 11(1) using the following raw materials.
   Raw materials: 2,4-dichlorobenzylalcohol and 2,4-thiazolidinedione.
   Yield: 87.9%(colorless oil).
   ¹H-NMR(CDCl₃): *δ* 4.03(2H,s), 4.88(2H,s), 7.14(1H,d,J=8.4Hz), 7.22(1H,dd,J=8.4,2.1Hz),7 .40(1H,d,J=2.1Hz).
(2) 5-(2,3-Dihydroxybenzylidene)-3-(2,4-dichlorobenzyl)thiazolidine-2,4-dione (Compound No. 22).

The title compound was obtained in the same manner as the Example 2(2) using the following raw materials.
Raw materials: 2,3-dihydroxybenzaldehyde and 3-(2,4-dichlorobenzyl)thiazolidine-2,4-dione.
Yield: 53.0%(yellow powder).
¹H-NMR(DMSO-ds): *δ* 4.88(2H,s), 6.78-6.88(2H,m), 6.93(1H,dd,J=7.8,1.8Hz), 7.30(1H,d,J=8.1Hz), 7.41(1H,dd,J=8.4,2.1Hz), 7.68(1H,d,J=2.1Hz), 8.19(1H,s), 9.53(1H,s), 9.90(1H,s).

### Example 23: Preparation of the compound of Compound No. 23.

The title compound was obtained in the same manner as the Example 2(2) using the following raw materials.
Raw materials: 3,4-dihydroxybenzaldehyde and
3-(2,4-dichlorobenzyl)thiazolidine-2,4-dione(compound of the Example 22(1)).
Yield: 82.4%(yellow powder).
¹H-NMR(DMSO-d₆): *δ* 4.87(2H,s), 6.89(1H,d,J=7.8Hz), 7.01-7.05(2H,m), 7.29(1H,d,J=8.4Hz), 7.41(1H,dd,J=8.4,2.4Hz), 7.68(1H,d,J=2.4Hz), 7.79(1H,s), 9.52(1H,s), 9.94(1H,s).

### Example 24: Preparation of the compound of Compound No. 24.

(1) 3-(3,5-Difluorobenzyl)thiazolidine-2,4-dione.
   The title compound was obtained in the same manner as the Example 2(1) using the following raw materials.
   Raw materials: 3,5-difluorobenzyl bromide and 2,4-thiazolidinedione.
   Yield: 70.8%(white crystal).
   ¹H-NMR(CDCIa): *δ* 3.99(2H,s), 4.72(2H,s), 6.75(1H,tt,J=8.7,2.1Hz), 6.88-6.96(2H,m).
(2) 5-(2,3-Dihydroxybenzylidene)-3-(3,5-difluorobenzyl)thiazolidine-2,4-dione (Compound No. 24).

The title compound was obtained in the same manner as the Example 2(2) using the following raw materials.
Raw materials: 2,3-dihydroxybenzaldehyde and 3-(3,5-difluorobenzyl)thiazolidine-2,4-dione.
Yield: 88.6%(yellow crystal).
¹H-NMR(DMSO-d₆): *δ* 4.85(2H,s), 6.77-6.88(2H,m), 6.92(1H,dd,J=7.5,2.1Hz), 7.02-7.10(2H,m), 7.19(1H,tt,J=9.3,2.1Hz), 8.20(1H,s), 9.62(2H,brs).

### Example 25: Preparation of the compound of Compound No. 25.

The title compound was obtained in the same manner as the Example 2(2) using the following raw materials.
Raw materials: 3,4-dihydroxybenzaldehyde and
3-(3,5-difluorobenzyl)thiazolidine-2,4-dione(compound of the Example 24(1)).
Yield: 93.9%(yellow powder).
¹H-NMR(DMSO-d₆): *δ* 4.84(2H,s), 6.88(1H,d,J=7.8Hz), 7.00-7.06(4H,m), 7.19(1H,tt,J=9.3,2.1Hz), 7.78(1H,s), 9.59(2H,brs).

### Example 26: Preparation of the compound of Compound No. 26.

(1) 3-(3,5-Dimethylbenzyl)thiazolidine-2,4-dione.
   The title compound was obtained in the same manner as the Example 2(1) using the following raw materials.
   Raw materials: 3,5-dimethylbenzyl bromide and 2,4-thiazolidinedione.
   Yield: 100%(white crystal).
   ¹H-NMR(CDCl₃): *δ* 2.29(6H,s), 3.94(2H,s), 4.69(2H,s), 6.93(1H,s), 7.00(2H,s).
(2) 5-(2,3-Dihydroxybenzylidene)-3-(3,5-dimethylbenzyl)thiazolidine-2,4-dione (Compound No. 26).

The title compound was obtained in the same manner as the Example 2(2) using the following raw materials.
Raw materials: 2,3-dihydroxybenzaldehyde and 3-(3, 5-dimethylbenzyl)thiazolidine-2,4-dione.
Yield: 55.3%(light yellow crystal).
¹H.NMR(DMSO-d₆): *δ* 2.24(6H,s), 4.74(2H,s), 6.76-6.87(2H,m), 6.90-6.94(4H,m), 8.19(1H,s), 9.51(1H,s), 9.88(1H,s).

### Example 27: Preparation of the compound of Compound No. 27.

The title compound was obtained in the same manner as the Example 2(2) using the following raw materials.
Raw materials: 3,4-dihydroxybenzaldehyde and
3-(3,5-dimethylbenzyl)thiazolidine-2,4-dione(compound of the Example 26(1)).
Yield: 77.6%(yellow powder).
¹H-NMR(DMSO-d₆): *δ* 2.24(6H,s), 4.73(2H,s), 6.87-6.89(1H,m), 6.89(2H,s), 6.93(1H,s), 6.99-7.04(2H,m), 7.78(1H,s).

### Example 28: Preparation of the compound of Compound No. 28.

(1) 5-Formylsalicylic acid ethyl ester.
   Concentrated hydrochloric acid(0.3 ml) was added to a solution of 5-formylsalicylic acid(1g, 6.02 mmol) in ethanol(10 ml), and the mixture was refluxed for 12 hours. The reaction mixture was cooled, poured into water, and extracted with ethyl acetate. The organic layer was dried over anhydrous sodium sulfate. The residue obtained by evaporation of the solvent under reduced pressure was purified by chromatography on silica gel(hexane:ethyl acetate=3:1) to give the title compound(301.7 mg, 25.8%) as a white crystal.
   ¹H-NMR(DMSO.d₆): *δ* 1.36(3H,t,J=6.9Hz), 4.39(2H,q,J=6.9Hz), 7.17(1H,d,J=8.4Hz), 8.01(1H,dd,J=8.4,2.1Hz), 8.33(1H,d,J=2.1Hz), 9.90(1H,s), 11.27(1H,s).
(2) 5-(3-Ethoxycarbonyl-4-hydroxybenzylidene)-3-[3,5-bis(trifluoromethyl)-benzyl]thiazolidine-2,4-dione (Compound No. 28).

The title compound was obtained in the same manner as the Example 2(2) using the following raw materials.
Raw materials: 5-formylsalicylic acid ethyl ester and
3-[3,5-bis(trifluoromethyl)benzyl]thiazolidine-2,4-dione(compound of the Example 2(1)).
Yield: 86.0%(white crystal).
¹H-NMR(DMSO-d₆): *δ* 1.36(3H,t,J=6.9Hz), 4.38(2H,q,J=6.9Hz), 5.04(2H,s), 7.16(1H,d,J=8.7Hz), 7.79(1H,dd,J=8.7,2.4Hz), 7.97(1H,s), 8.05-8.09(4H,m), 11.00(1H,s).

### Example 29: Preparation of the compound of Compound No. 29.

The title compound was obtained in the same manner as the Example 2(2) using the following raw materials.
Raw materials: 2,3,4-trihydroxybenzaldehyde and
3-(3,4-dichlorobenzyl)thiazolidine-2,4-dione(compound of the Example 8(1)).
Yield: 32.4%(yellow powder).
¹H-NMR(DMSO-d₆): *δ* 4.82(2H,s), 6.50(1H,d,J=8.4Hz), 6.77(1H,d,J=8.7Hz), 7.28(1H,dd,J=8.4,2.4Hz), 7.60(1H,d,J=1.8Hz), 7.62(1H,d,J=8.4Hz), 8.15(1H,s), 8.80(1H,s), 9.53(1H,s), 10.15(1H,s).

### Example 30: Preparation of the compound of Compound No. 30.

(1) 3-(3-Chlorobenzyl)thiazolidine-2,4-dione.
   The title compound was obtained in the same manner as the Example 11(1) using the following raw materials.
   Raw materials: 3-chlorobenzylalcohol and 2,4-thiazolidinedione.
   Yield: 78.1%(white crystal).
   ¹H-NMR(CDCl₃): *δ* 3.97(2H,s), 4.73(2H,s), 7.27-7.29(3H,m), 7.37-7.38(1H,m).
(2) 5-(2,3-Dihydroxybenzylidene)-3-(3-chlorobenzyl)thiazolidine-2,4-dione (Compound No. 30).

The title compound was obtained in the same manner as the Example 2(2) using the following raw materials.
Raw materials: 2,3-dihydroxybenzaldehyde and 3-(3-chlorobenzyl)thiazolidine-2,4-dione.
Yield: 37.2%(yellow powder).
¹H-NMR(DMSO-d₆): *δ* 4.84(2H,s), 6.77-6.87(2H,m), 6.92(1H,dd,J=7.5,1.5Hz), 7.25-7.28(1H,m), 7.38-7.40(3H,m), 8.20(1H,s), 9.53(1H,s), 9.89(1H,s).

### Example 31: Preparation of the compound of Compound No. 31.

The title compound was obtained in the same manner as the Example 2(2) using the following raw materials.
Raw materials: 3,4-dihydroxybenzaldehyde and
3-(3-chlorobenzyl)thiazolidine-2,4-dione(compound of the Example 30(1)).
Yield: 39.8%(yellow powder).
¹H-NMR(DMSO-ds): *δ* 4.83(2H,s), 6.89(1H,d,J=8.1Hz), 7.00-7.05(2H,m), 7.23-7.29(1H,m), 7.37-7.39(3H,m), 7.79(1H,s), 9.52(1H,s), 9.94(1H,s).

### Example 32: Preparation of the compound of Compound No. 32.

(1) 3-(4-Chlorobenzyl)thiazolidine-2,4-dione.
   The title compound was obtained in the same manner as the Example 11(1) using the following raw materials.
   Raw materials: 4-chlorobenzylalcohol and 2,4-thiazolidinedione.
   Yield: 94.1%(white crystal).
   ¹H-NMR(CDCl₃): *δ* 3.95(2H,s), 4.73(2H,s), 7.29(2H,d,J=8.4Hz), 7.35(2H,d,J=8.4Hz).
(2) 5-(2,3-Dihydroxybenzylidene)-3-(4-chlorobenzyl)thiazolidine-2,4-dione (Compound No. 32).
   The title compound was obtained in the same manner as the Example 2(2) using the following raw materials.
   Raw materials: 2,3-dihydroxybenzaldehyde and
   3-(4-chlorobenzyl)thiazolidine-2,4-dione.
   Yield: 67.6%(yellow solid).
   ¹H-NMR(DMSO-ds): *δ* 4.82(2H,s), 6.76-6.87(2H,m), 6.92(1H,dd,J=7.2,1.8Hz), 7.34(2H,d,J=8.7Hz), 7.42(2H,d,J=8.4Hz), 8.19(1H,s), 9.52(1H,s), 9.89(1H,s)

### Example 33: Preparation of the compound of Compound No. 33.

The title compound was obtained in the same manner as the Example 2(2) using the following raw materials.
Raw materials: 3,4-dihydroxybenzaldehyde and
3-(4-chlorobenzyl)thiazolidine-2,4-dione(compound of the Example 32(1)).
Yield: 66.4%(yellow powder).
¹H-NMR(DMSO-d₆): *δ* 4.82(2H,s), 6.89(1H,d,J=8.1Hz), 7.00-7.04(2H,m), 7.33(2H,d,J=8.7Hz), 7.42(2H,d,J=8.4Hz), 7.79(1H,s), 9.52(1H,s), 9.94(1H,s).

### Example 34: Preparation of the compound of Compound No. 34.

(1) 3-(3-Bromobenzyl)thiazolidine-2,4-dione.
   The title compound was obtained in the same manner as the Example 11(1) using the following raw materials.
   Raw materials: 3-bromobenzylalcohol and 2,4-thiazolidinedione.
   Yield: 45.4%(white crystal).
   ¹H-NMR(CDCl₃): *δ* 3.97(2H,s), 4.72(2H,s), 7.20(1H,t,J=7.8Hz), 7.24(1H,ddd,J=7.8,1.8,1.2Hz), 7.44(1H,ddd,J=7.8,1.8,1.2Hz), 7.54(1H,t,J=1.8Hz).
(2) 5-(2,3-Dihydroxybenzylidene)-3-(3-bromobenzyl)thiazolidine-2,4-dione (Compound No. 34).
   The title compound was obtained in the same manner as the Example 2(2) using the following raw materials.
   Raw materials: 2,3-dihydroxybenzaldehyde and
   3-(3-bromobenzyl)thiazolidine-2,4-dione.
   Yield: 56.3%(yellow powder).
   ¹H-NMR(DMSO-d₆): *δ* 4.83(2H,s), 6.77-6.87(2H,m), 6.92(1H,dd,J=7.5,1.8Hz), 7.29-7.35(2H,m), 7-.50-7.54(2H,m), 8.20(1H,s), 9.51(1H,s), 9.88(1H,s).

### Example 35: Preparation of the compound of Compound No. 35.

The title compound was obtained in the same manner as the Example 2(2) using the following raw materials.
Raw materials: 3,4-dihydroxybenzaldehyde and
3-(3-bromobenzyl)thiazolidine-2,4-dione(compound of the Example 34(1)).
Yield: 21.0%(light yellow powder).
¹H-NMR(DMSO-d₆): *δ* 4.82(2H,s), 6.88(1H,d,J=7.8Hz), 7.00-7.04(2H,m), 7.27-7.35(2H,m), 7.50-7.53(2H,m), 7.79(1H,s), 9.51(1H,s), 9.93(1H,s).

### Example 36: Preparation of the compound of Compound No. 36.

(1) 3-(4-Bromobenzyl)thiazolidine-2,4-dione.
   The title compound was obtained in the same manner as the Example 11(1) using the following raw materials.
   Raw materials: 4-bromobenzylalcohol and 2,4-thiazolidinedione.
   Yield: 90.0%(white crystal).
   ¹H-NMR(CDCl₃): *δ* 3.95(2H,s), 4.71(2H,s), 7.28(2H,d,J=8.4Hz), 7.46(2H,d,J=8.4Hz).
(2) 5-(2,3-Dihydroxybenzylidene)-3-(4-bromobenzyl)thiazolidine-2,4-dione (Compound No. 36).
   The title compound was obtained in the same manner as the Example 2(2) using the following raw materials.
   Raw materials: 2,3-dihydroxybenzaldehyde and 3-(4-bromobenzyl)thiazolidine-2,4-dione.
   Yield: 66.4%(yellow powder).
   ¹H-NMR(DMSO-d₆):*δ* 4.80(2H,s), 6.76-6.87(2H,m), 6.92(1H,dd,J=7.5,1.8Hz), 7.28(2H,d.J=8.7Hz), 7.56(2H,d,J=8.4Hz), 8.19(1H,s), 9.52(1H,s), 9.89(1H,s).

### Example 37: Preparation of the compound of Compound No. 37.

The title compound was obtained in the same manner as the Example 2(2) using the following raw materials.
Raw materials: 3,4-dihydroxybenzaldehyde and
3-(4-bromobenzyl)thiazolidine-2,4-dione(compound of the Example 36(1)).
Yield: 72.2%(light yellow powder).
¹H-NMR(DMSO-d₆): *δ* 4.79(2H,s), 6.88(1H,d,J=8.1Hz), 6.99-7.04(2H,m), 7.27(2H,d,J=8.7Hz), 7.55(2H,d,J=8.1Hz), 7.78(1H,s), 9.51(1H,s), 9.93(1H,s).

### Example 38: Preparation of the compound of Compound No. 38.

The title compound was obtained in the same manner as the Example 2(2) using the following raw materials.
Raw materials: 3,4-dihydroxy-5-methoxybenzaldehyde and 3-(3,4-dichlorobenzyl)thiazolidine-2,4-dione(compound of the Example 8(1)).
Yield: 44.8%(light yellow crystal).
¹H-NMR(DMSO-d₆): *δ* 3.33(3H,s), 4.83(2H,s), 6.75(1H,d,J=2.1Hz), 6.83(1H,d,J=2.1Hz), 7.29(1H,dd,J=8.7,2.4Hz), 7.61(1H,s), 7.62(1H,d,J=8.7Hz), 7.80(1H,s), 9.30(1H,s), 9.50(1H,s).

### Example 39: Preparation of the compound of Compound No. 39.

The title compound was obtained in the same manner as the Example 2(2) using the following raw materials.
Raw materials: 5-hydroxy-2-nitrobenzaldehyde and
3-(3,4-dichlorobenzyl)thiazolidine-2,4-dione(compound of the Example 8(1)).
Yield: 37.0%(ocherous crystal).
¹H-NMR(DMSO-d₆): *δ* 4.84(2H,s), 6.96-7.01(2H,m), 7.29(1H,dd,J=8.1,2.1Hz), 7.42(1H,d,J=8.1Hz), 7.53(H,d,J=2.1Hz), 8.18-8.21(1H,m), 8.32(1H,s).

### Example 40: Preparation of the compound of Compound No. 40.

The title compound was obtained in the same manner as the Example 2(2) using the following raw materials.
Raw materials: 3,5-dihydroxybenzaldehyde and
3-(3,4-dichlorobenzyl)thiazolidine-2,4-dione(compound of the Example 8(1)).
Yield: 28.5%(slightly yellow crystal).
¹H-NMR(DMSO-d₆): *δ* 4.83(2H,s), 6.34(1H,t,J=2.1Hz), 6.48(2H,d,J=2.1Hz), 7.30(1H,dd,J=8.1,2.1Hz), 7.62(1H,d,J=2.1Hz), 7.62(1H,d,J=8.1Hz), 7.74(1H,s), 9.72(2H,s).

### Example 41: Preparation of the compound of Compound No. 41.

(1) 3-[3-(Trifluoromethyl)benzyl]thiazolidine-2,4-dione.
   The title compound was obtained in the same manner as the Example 11(1) using the following raw materials.
   Raw materials: 3-(trifluoromethyl)benzylalcohol and 2,4-thiazolidinedione.
   Yield: 80.4%(colourless oil).
   ¹H-NMR(CDCl₃): *δ* 3.98(2H,s), 4.81(2H,s), 7.46(1H,t,J=7.8Hz), 7.56-7.61(2H,m), 7.65(1H,s).
(2) 5-(2,3-Dihydroxybenzylidene)-3-[3-(trifluoromethyl)benzyl]thiazolidine-2,4-dione (Compound No. 41).
   The title compound was obtained in the same manner as the Example 2(2) using the following raw materials.
   Raw materials: 2,3-dihydroxybenzaldehyde and
   3- [3-(trifluoromethyl)benzyl] thiazolidine-2,4-dione.
   Yield: 63.2%(yellow solid).
   ¹H-NMR(DMSO-d₆): *δ* 4.93(2H,s), 6.77-6.88(2H,m), 6.93(1H,dd,J=7.8,1.8Hz), 7.60-7.62(2H,m), 7.68-7.71(2H,m), 8.21(1H,s), 9.54(1H,s), 9.90(1H,s).

### Example 42: Preparation of the compound of Compound No. 42.

The title compound was obtained in the same manner as the Example 2(2) using the following raw materials.
Raw materials: 3,4-dihydroxybenzaldehyde and
3-[3-(trifluoromethyl)benzyl]thiazolidine-2,4-dione(compound of the Example 41(1)). Yield: 72.4%(light yellow powder).
¹H-NMR(DMSO-d₆): *δ* 4.93(2H,s), 6.89(1H,d,J=7.8Hz), 7.00-7.05(2H,m), 7.60-7.61(2H,m), 7.68-7.71(2H,m), 7.80(1H,s), 9.53(1H,s), 9.95(1H,s).

### Example 43: Preparation of the compound of Compound No. 43.

(1) 3-[4-(Trifluoromethyl)benzyllthiazolicline-2,4-clione.
   The title compound was obtained in the same manner as the Example 11(1) using the following raw materials.
   Raw materials: 4-(trifluoromethyl)benzylalcohol and 2,4-thiazolidinedione.
   Yield: 84.8%(white crystal).
   ¹H-NMR(CDCl₃): *δ* 3.98(2H,s), 4.81(2H,s), 7.51(2H,d,J=7.8Hz), 7.59(2H,d,J=8.1Hz).
(2) 5-(2,3-Dihydroxybenzylidene)-3-[4-(trifluoromethyl)benzyl]thiazolidine-2,4-dione (Compound No. 43).
   The title compound was obtained in the same manner as the Example 2(2) using the following raw materials.
   Raw materials: 2,3-dihydroxybenzaldehyde and 3-[4-(trifluoromethyl)benzyl]thiazolidine-2,4-dione.
   Yield: 52.8%(yellow solid).
   ¹H-NMR(DMSO-ds)^{:} *δ* 4.93(2H,s), 6.80(1H,t,J=7.8Hz), 6.86(1H,dd,J=7.8,1.8Hz), 6.93(1H,dd,J=7.8,1.8Hz), 7.54(2H,d,J=8.1Hz), 7.73(2H,d.J=8.1Hz), 8.21(1H,s), 9.54(1H,s), 9.91(1H,s).

### Example 44: Preparation of the compound of Compound No. 44.

The title compound was obtained in the same manner as the Example 2(2) using the following raw materials.
Raw materials: 3,4-dihydroxybenzaldehyde and
3-[4-(trifluoromethyl)benzyl]thiazolidine-2,4-dione(compound of the Example 43(1)). Yield: 80.3%(light yellow powder).
¹H-NMR(DMSO-d₆): *δ* 4.92(2H,s), 6.89(1H,d,J=8.1Hz), 7.00-7.04(2H,m), 7.53(2H,d,J=8.1Hz), 7.73(2H,d.J=8.1Hz), 7.80(1H,s), 9.52(1H,s), 9.94(1H,s).

### Example 45: Preparation of the compound of Compound No. 45.

The title compound was obtained in the same manner as the Example 2(2) using the following raw materials.
Raw materials: 2,4-dihydroxybenzaldehyde and
3-(3,4-dichlorobenzyl)thiazolidine-2,4-dione(compound of the Example 8(1)).
Yield: 14.1%(yellow crystal).
¹H-NMR(DMSO-d₆): *δ* 4.82(2H,s), 6.41(1H,dd,J=7.2,2.4Hz), 6.43(1H,s), 7.21(1H,d,J=9.3Hz), 7.28(1H,dd,J=8.4,2.1Hz), 7.59(1H,d,J=1.8Hz), 7.62(1H,d,J=8.1Hz), 8.12(1H,s), 10.27(1H,s), 10.57(1H,s).

### Example 46: Preparation of the compound of Compound No. 46.

(1) 3-(3,5-Dibromobenzyl)thiazolidine-2,4-dione.
   The title compound was obtained in the same manner as the Example 2(1) using the following raw materials.
   Raw materials: 3,5-dibromobenzyl bromide and 2,4-thiazolidinedione.
   Yield: 24.7%(white crystal).
   ¹H-NMR(CDCl₃): *δ* 3.99(2H,s), 4.68(2H,s), 7.37(1H,s), 7.47-7.48(2H,m).
(2) 5-(3,4-Dihydroxybenzylidene)-3-(3,5-dibromobenzyl)thiazolidine-2,4-dione (Compound No. 46).
   The title compound was obtained in the same manner as the Example 2(2) using the following raw materials.
   Raw materials: 3,4-dihydroxybenzaldehyde and 3-(3,5-dibromobenzyl)thiazolidine-2,4-dione.
   Yield: 100%(yellow crystal).
   ¹H-NMR(DMSO-d₆): *δ* 4.82(2H,s), 6.88(1H,d,J=8.1Hz), 7.00-7.03(2H,m), 7.55(2H,d,J=1.8Hz), 7.78-7.80(2H,m), 9.52(1H,s), 9.93(1H,s).

### Example 47: Preparation of the compound of Compound No. 47.

The title compound was obtained in the same manner as the Example 2(2) using the following raw materials.
Raw materials: 2,3-dihydroxybenzaldehyde and
3-(3,5-dibromobenzyl)thiazolidine-2,4-dione(compound of the Example 46(1)).
Yield: 66.6%(yellow crystal).
¹H-NMR(DMSO-d₆): Iδ 4.82(2H,s), 6.76-6.86(2H,m), 6.92(1H,dd,J=7.2,1.8Hz), 7.56(2H,d,J=1.8Hz), 7.80(1H,t,J=1.8Hz), 8.19(1H,s), 9.68(1H,brs), 9.93(1H,brs).

### Example 48: Preparation of the compound of Compound No. 48.

(1) 3-(3-Nitrobenzyl)thiazolidine-2,4-dione.
   The title compound was obtained in the same manner as the Example 2(1) using the following raw materials.
   Raw materials: 3-nitrobenzyl chloride and 2,4-thiazolidinedione.
   Yield: 66.5%(white crystal).
   ¹H-NMR(CDCl₃): *δ* 4.01(2H,s), 4.86(2H,s), 7.53(1H,t,J=7.8Hz), 7.74(1H,d,J=7.5Hz), 8.17-8.20(1H,m), 8.24-8.25(1H,m).
(2) 5-(3,4-Dihydroxybenzylidene)-3-(3-nitrobenzyl)thiazolidine-2,4-dione (Compound No. 48).
   The title compound was obtained in the same manner as the Example 2(2) using the following raw materials.
   Raw materials: 3,4-dihydroxybenzaldehyde and 3-(3-nitrobenzyl)thiazolidine-2,4-dione.
   Yield: 62.1%(yellow crystal).
   ¹H-NMR(DMSO-d₆): *δ* 4.95(2H,s), 6.86(1H,d,J=7.8Hz), 6.98-7.01(2H,m), 7.61-7.67(1H,m), 7.74-7.77(1H,m), 7.78(1H,s), 8.13-8.17(2H,m), 9.51(1H,s), 9.93(1H,s).

### Example 49: Preparation of the compound of Compound No. 49.

The title compound was obtained in the same manner as the Example 2(2) using the following raw materials.
Raw materials: 2,3-dihydroxybenzaldehyde and
3-(3-nitrobenzyl)thiazolidine-2,4-dione(compound of the Example 48(1)).
Yield: 40.9%(yellow crystal).
¹H-NMR(DMSO-d₆): *δ* 4.98(2H,s), 6.77-6.87(2H,m), 6.92(1H,dd,J=7.5,1.8Hz), 7.64-7.69(1H,m), 7.70-7.80(1H,m), 8.16-8.20(2H,m), 8.20(1H,s), 9.53(1H,s), 9.89(1H,s).

### Example 50: Preparation of the compound of Compound No. 50.

(1) 3-(4-Nitrobenzyl)thiazolidine-2,4-dione.
   The title compound was obtained in the same manner as the Example 11(1) using the following raw materials.
   Raw materials: 4-nitrobenzylalcohol and 2,4-thiazolidinedione.
   Yield: 100%(light yellow crystal).
   ¹H.NMR(CDCl₃): *δ* 4.00(2H,s), 4.85(2H,s), 7.56(2H,d,J=8.7Hz), 8.19(2H,d,J=8.7Hz).
(2) 5-(3,4-Dihydroxybenzylidene)-3-(4-nitrobenzyl)thiazolidine-2,4-dione (Compound No. 50).
   The title compound was obtained in the same manner as the Example 2(2) using the following raw materials.
   Raw materials: 3,4-dihydroxybenzaldehyde and 3-(4-nitrobenzyl)thiazolidine-2,4-dione.
   Yield: 45.9%(yellow crystal).
   ¹H-NMR(DMSO-d₆): *δ* 4.95(2H,s), 6.73(1H,d,J=7.8Hz), 6.96-7.00(2H,m), 7.57(2H,d,J=8.4Hz), 7.75(1H,s), 8.21(2H,d,J=8.4Hz).

### Example 51: Preparation of the compound of Compound No. 51.

The title compound was obtained in the same manner as the Example 2(2) using the following raw materials.
Raw materials: 2,3-dihydroxybenzaldehyde and
3-(4-nitrobenzyl)thiazolidine-2,4-dione(compound of the Example 50(1)).
Yield: 58.9%(yellow crystal).
¹H-NMR(DMSO-d₆):δ 4.97(2H,s), 6.77-6.88(2H,m), 6.93(1H,dd,J=7.2,1.8Hz), 7.59(2H,d,J=9.0Hz), 8.20(1H,s), 8.22(2H,d,J=8.1Hz), 9.54(1H,s), 9.90(1H,s).

### Example 52: Preparation of the compound of Compound No. 52.

The title compound was obtained in the same manner as the Example 2(2) using the following raw materials.
Raw materials: 3,4,5-trihydroxybenzaldehyde and
3-(3-nitrobenzyl)thiazolidine-2,4-dione(compound of the Example 48(1)).
Yield: 20.3%(brown crystal).
¹H.NMR(DMSO-d₆)" *δ* 4.96(2H,s), 6.62(2H,s), 7.64-7.70(2H,m), 7.77(1H,d,J=7.8Hz), 8.16-8.19(2H,m), 9.16(1H,s), 9.43(2H,s).

### Example 53: Preparation of the compound of Compound No. 53.

The title compound was obtained in the same manner as the Example 2(2) using the following raw materials.
Raw materials: 3,4,5-trihydroxybenzaldehyde and
3-(4-nitrobenzyl)thiazolidine-2,4-dione(compound of the Example 50(1)).
Yield: 11.9%(yellow crystal).
¹H-NMR(DMSO-d₆): *δ* 4.96(2H,s), 6.63(2H,s), 7.58(2H,d,J=9.0Hz), 7.70(1H,s), 8.22(2H,d,J=8.7Hz), 9.16(1H,s), 9.43(2H,s).

### Example 54: Preparation of the compound of Compound No. 54.

The title compound was obtained in the same manner as the Example 2(2) using the following raw materials.
Raw materials: 3,4,5-trihydroxybenzaldehyde and
3-(3,5-dichlorobenzyl)thiazolidine-2,4-dione(compound of the Example 11(1)).
Yield: 20.2%(yellow crystal).
¹H-NMR(DMSO-ds): *δ* 4.82(2H,s), 6.62(2H,s), 7.38(2H,d,J=1.8Hz), 7.57(1H,t,J=1.8Hz), 7.68(1H,s), 9.14(1H,s), 9.43(2H,s).

### Example 55: Preparation of the compound of Compound No. 55.

The title compound was obtained in the same manner as the Example 2(2) using the following raw materials.
Raw materials: 5-nitrosalicylaldehyde and
3-(3,4-dichlorobenzyl)thiazolidine-2,4-dione(compound of the Example 8(1)).
Yield: 50.8%(light yellow crystal).
¹H-NMR(DMSO-d₆): *δ* 4.85(2H,s), 7.13(1H,d,J=8.1Hz), 7.32(1H,dd,J=8.1,1.8Hz), 7.62-7.64(2H,m), 8.05(1H,s), 8.21-8.25(2H,m).

### Example 56: Preparation of the compound of Compound No. 56.

(1) 3-(4-Styrylbenzyl)thiazolidine-2,4-dione.
   The title compound was obtained in the same manner as the Example 11(1) using the following raw materials.
   Raw materials: 4-styrylbenzylalcohol and 2,4-thiazolidinedione.
   Yield: 60.1%(white crystal).
   ¹H-NMR(DMSO-d₆): *δ* 4.29(2H,s), 4.68(2H,s), 7.23-7.30(5H,m), 7.34-7.41(2H,m), 7.55-7.62(4H,m).
(2) 5-(2,3-Dihydroxybenzylidene)-3-(4-styrylbenzyl)thiazolidine-2,4-dione (Compound No. 56).
   The title compound was obtained in the same manner as the Example 2(2) using the following raw materials.
   Raw materials: 2,3-dihydroxybenzaldehyde and 3-(4-styrylbenzyl)thiazolidine-2,4-dione.
   Yield: 45.0%(yellow solid).
   ¹H-NMR(DMSO-d₆): *δ* 4.84(2H,s), 6.76-6.88(2H,m), 6.94(1H,dd,J=7.8,2.1Hz), 7.24-7.41(7H,m), 7.56-7.62(4H,m), 8.21(1H,s), 9.53(1H,brs), 9.89(1H,brs).

### Example 57: Preparation of the compound of Compound No. 57.

The title compound was obtained in the same manner as the Example 2(2) using the following raw materials.
Raw materials: 3,4-dihydroxybenzaldehyde and
3-(4-styrylbenzyl)thiazolidine-2,4-dione(compound of the Example 56(1)).
Yield: 30.8%(yellow solid).
¹H-NMR(DMSO-ds): *δ* 4.83(2H,s), 6.88(1H,d,J=8.1Hz), 7.00-7.05(2H,m), 7.23-7.41(7H,m), 7.56-7.62(4H,m), 7.80(1H,s), 9.52(1H,brs), 9.94(1H,brs).

### Example 58: Preparation of the compound of Compound No. 58.

(1) 4-[3,5-Bis(trifluoromethyl)styryl]benzylalcohol.
   A mixture of 4-bromobenzylalcohol(248mg, 1.326mmol), 3,5-bis(trifluoromethyl)styrene(350 mg, 1.457 mmol), tri-o-tolylphosphine(17 mg, 0.0559 mmol), palladium acetate(5.9 mg, 0.0263 mmol) and triethylamine(1.5 ml) was refluxed for 2 hours. The reaction mixture was cooled, and the insoluble matter was filtered off. The residue obtained by evaporation of the filtrate under reduced pressure was purified by chromatography on silica gel (hexane:ethyl acetate=2:1→1:1) to give the title compound(459 mg, 100%) as a white crystal.
   ¹H-NMR(CDCl₃): *δ* 1.71(1H,t,J=6.0Hz), 4.73(2H,d,J=7.5Hz), 7.14(1H,d,J=16.2Hz), 7.25(1H,d,J=16.2Hz), 7.41(2H,d,J=8.4Hz), 7.55(2H,d,J=8.4Hz), 7.73-7.76(1H,m), 7.90-7.91(2H,m).
(2) 3-{4-[3,5-Bis(trifluoromethyl)styryl]benzyl}thiazolidine-2,4-dione.
   The title compound was obtained in the same manner as the Example 11(1) using the following raw materials.
   Raw materials: 4-[3,5-bis(trifluoromethyl)styryl]benzylalcohol and 2,4-thiazolidinedione.
   Yield: 84.0%(white solid).
   ¹H-NMR(CDCl₃):*δ* 3.97(2H,s), 4.79(2H,s), 7.12(1H,d,J=16.5Hz), 7.22(1H,d,J=16.5Hz), 7.43(2H,d,J=8.7Hz), 8.10(2H,d,J=8.7Hz), 7.73-7.76(1H,m), 7.91-7.92(2H,m).
(3) 5-(3,4-Dihydroxybenzylidene)-3-{4-[3,5-bis(trifluoromethyl)styryl]-benzyl}thiazolidine-2,4-dione (Compound No. 58).
   The title compound was obtained in the same manner as the Example 2(2) using the following raw materials.
   Raw materials: 3,4-dihydroxybenzaldehyde and 3-{4-[3,5-bis(trifluoromethyl)styryl]benzyl}thiazolidine-2,4-dione.
   Yield: 35.8%(yellow solid).
   ¹H-NMR(DMSO-d₆): *δ* 4.85(2H,s), 6.89(1H,d,J=8.1Hz), 7.00-7.06(2H,m), 7.36(2H,d,J=8.1Hz), 7.46(1H,d,J=16.5Hz), 7.63(2H,d,J=8.1Hz), 7.68(1H,d,J=16.5Hz), 7.80(1H,s), 7.93-7.97(1H,m), 8.30-8.34(2H,m), 9.60(1H,brs), 9.89(1H,brs).

### Example 59: Preparation of the compound of Compound No. 59.

The title compound was obtained in the same manner as the Example 2(2) using the following raw materials.
Raw materials: 2,3-dihydroxybenzaldehyde and
3-{4-[3,5-bis(trifluoromethyl)styryl]benzyl}thiazolidine-2,4-dione(compound of the Example 58(2)).
Yield: 51.5%(yellow solid).
¹H-NMR(DMSO-d₆): *δ* 4.86(2H,s), 6.77-6.95(3H,m), 7.37(2H,d,J=8.4Hz), 7.46(1H,d,J=16.8Hz), 7.64(2H,d,J=8.4Hz), 7.68(1H,d,J=16.8Hz), 7.93-7.96(1H,m), 8.21(1H,s), 8.30-8.34(2H,m), 9.54(1H,brs), 9.86(1H,brs).

### Example 60: Preparation of the compound of Compound No. 60.

(1) 4-Phenoxybenzylalcohol.
   Sodium borohydride(75.7 mg, 2 mmol) was added to a solution of 4-phenoxybenzaldehyde (396.4 mg, 2 mmol) in ethanol(3 ml) under ice cooling, and the mixture was stirred at room temperature for 1 hour. 2N Hydrochloric acid was added to the reaction mixture, and the mixture was extracted with ethyl acetate. The organic layer was washed with water and brine, and dried over anhydrous magnesium sulfate. The solvent was evaporated under reduced pressure to give the title compound(385.4mg, 96.2%) as a white crystal.
   ¹H-NMR(CDCl₃): *δ* 1.63(1H,t,J=5.1Hz), 4.67(2H,d,J=5.1Hz), 6.99-7.02(4H,m), 7.10(1H,tt,J=7.2,1.2Hz), 7.31-7.36(4H,m).
(2) 3-(4-Phenoxybenzyl)thiazolidine-2,4-dione.
   The title compound was obtained in the same manner as the Example 11(1) using the following raw materials.
   Raw materials: 4-phenoxybenzylalcohol and 2,4-thiazolidinedione.
   Yield: 53.3%(light yellow crystal).
   ¹H-NMR(CDCl₃): *δ* 3.95(2H,s), 4.74(2H,s), 6.94(2H,d,J=8.4Hz), 6.98-7.02(2H,m), 7.12(1H,tt,J=7.5,1.2Hz), 7.31-7.39(4H,m).
(3) 5-(3,4-Dihydroxybenzylidene)-3-(4-phenoxybenzyl)thiazolidine-2,4-dione (Compound No. 60).
   The title compound was obtained in the same manner as the Example 2(2) using the following raw materials.
   Raw materials: 3,4-dihydroxybenzaldehyde and 3-(4-phenoxybenzyl)thiazolidine-2,4-dione.
   Yield: 49.3%(light yellow powder).
   ¹H-NMR(DMSO-d₆): *δ* 4.87(2H,s), 6.86(1H,d,J=8.1Hz), 6.95-7.01(6H,m), 7.12(1H,t,J=7.5Hz), 7.30-7.39(4H,m), 7.76(1H,s), 9.51(1H,s), 9.92(1H,s).

### Example 61: Preparation of the compound of Compound No. 61.

The title compound was obtained in the same manner as the Example 2(2) using the following raw materials.
Raw materials: 2,3-dihydroxybenzaldehyde and
3-(4-phenoxybenzyl)thiazolidine-2,4-dione(compound of the Example 60(2)).
Yield: 42.9%(light yellow powder).
¹H-NMR(DMSO-d₆): *δ* 4.81(2H,s), 6.76-6.86(2H,m), 6.92(1H,dd,J=7.5,1.8Hz), 6.97-7.02(4H,m), 7.14(1H,t,J=7.5Hz), 7.33-7.41(4H,m), 8.20(1H,s), 9.53(1H,s), 9.90(1H,s).

### Example 62: The compound of Compound No. 62.

This compound is a commercially available compound.
Supplier: Specs (Netherlands).
Catalog code number: AM-879/11689013.

### Example 63: The compound of Compound No. 63.

This compound is a commercially available compound.
Supplier: Specs (Netherlands).
Catalog code number: AN-989/40768666.

### Example 64: The compound of Compound No. 64.

This compound is a commercially available compound.
Supplier: LaboTest (Germany).
Catalog code number: LT00070892.

### Example 65: The compound of Compound No. 65.

This compound is a commercially available compound.
Supplier: LaboTest (Germany).
Catalog code number: LT00145560.

### Example 66: Preparation of the compound of Compound No. 66.

(1) 3-(3-Carboxy-4-chlorophenyl)rhodanine.
   5-Amino-2-chlorobenzoic acid(0.50g, 2.91 mmol) and bis(carboxymethyl)trithiocarbonate(0.66 g, 2.92 mmol) were added to a solution of sodium carbonate(0.155 g, 1.46 mmol) in water(15 ml), and the mixture was stirred at 90°C for 3 hours. The reaction mixture was cooled, poured into 2N hydrochloric acid, and extracted with ethyl acetate. The organic layer was washed with water and brine, and dried over anhydrous sodium sulfate. The residue obtained by evaporation of the solvent under reduced pressure was washed with isopropyl ether under suspension to give the title compound(0.641g, 76.6%) as a light yellowish white powder.
   ¹H-NMR(DMSO-d₆): *δ* 4.36(2H,s), 7.48(1H,ddd,J=8.4,2.7,0.6Hz), 7.73(1H,d,J=8.7Hz), 7.78(1H,d,J=2.4Hz), 13.64(1H,brs).
(2) 3-(3-Methoxycarbonyl-4-chlorophenyl)rhodanine.
   A mixture of 3-(3-carboxy-4-chlorophenyl)rhodanine(0.30 g, 1.04 mmol), concentrated sulfuric acid(0.3 ml) and methanol(10 ml) was refluxed for 5 hours. The reaction mixture was cooled, poured into ice and water, and extracted with ethyl acetate. The organic layer was washed with brine and dried over anhydrous sodium sulfate. The residue obtained by evaporation of the solvent under reduced pressure was purified by chromatography on silica gel(hexane:ethyl acetate=2:1→1:1) to give the title compound(112.9 mg, 35.9%) as a yellowish white crystal.
   ¹H-NMR(CDCl₃): *δ* 3.94(3H,s), 4.21(2H,s), 7.30(1H,dd,J=8.7,2.7Hz), 7.62(1H,d,J=8.7Hz), 7.76(1H,d,J=2.7Hz).
(3) 5-(3,5-Dichlorophenyl)furaldehyde.
   A mixture of 5-bromo-2-furaldehyde(2.63 g, 15.0 mmol), 3,5-dichlorophenylboronic acid(315 mg, 16.5 mmol), tetrabutylammonium bromide(4.84 g, 15 mmol), palladium acetate(67 mg, 0.3 mmol), potassium carbonate(5.18g, 37.5 mmol) and water(20 ml) was stirred at room temperature for 2 hours. The reaction mixture was extracted twice with ethyl acetate. The organic layer was washed with brine and dried over anhydrous sodium sulfate. The residue obtained by evaporation of the solvent under reduced pressure was purified by chromatography on silica gel(hexane:ethyl acetate=4:1) to give the title compound(1.01g, 27.9%) as a white solid.
   ¹H-NMR(CDCl₃):δ 6.89(1H,d,J=3.9Hz), 7.33(1H,d,J=3.9Hz), 7.36(1H,t,J=1.8Hz), 7.68(2H,d,J=1.8Hz), 9.69(1H,s).
(4) 5-{[5-(3,5-Dichlorophenyl)furan-2-yl]methylene}-3-(3-methoxycarbonyl-4-chlorophenyl)rhodanine (Compound No. 66).
   The title compound was obtained in the same manner as the Example 2(2) using the following raw materials.
   Raw materials: 5-(3,5-dichlorophenyl)furaldehyde and 3-(3-methoxycarbonyl-4-chlorophenyl)rhodanine.
   Yield: 55.0%(yellow powder).
   ¹H-NMR(DMSO-d₆): *δ* 3.34(3H,s), 7.43(1H,d,J=3.6Hz), 7.61(1H,d,J=3.6Hz)" 7.68-7.72(2H,m), 7.77(1H,s), 7.83(1H,d,J=9.0Hz), 7.90(2H,d,J=2.1Hz), 7.98(1H,d,J=1.8Hz).

### Example 67: Preparation of the compound of Compound No. 67.

The title compound was obtained in the same manner as the Example 2(2) using the following raw materials.
Raw materials: 3,4-methylenedioxybenzaldehyde and
3-(3-carboxyl-4-chlorophenyl)rhodanine (compound of the Example 66(1)).
Yield: 69.7%(yellow solid).
¹H-NMR(DMSO-d₆): *δ* 6.17(2H,s), 7.14(1H,d,J=8.1Hz), 7.22-7.30(2H,m), 7.50(1H,dd,J=8.7,2.1Hz), 7.67(1-H,d,J=8.7Hz), 7.74(1H,d,J=2.1Hz), 7.78(1H,s).

### Example 68: Preparation of the compound of Compound No. 68.

The title compound was obtained in the same manner as the Example 2(2) using the following raw materials.
Raw materials: 4-bromobenzaldehyde and
3-(3-carboxyl-4-chlorophenyl)rhodanine(compound of the Example 66(1)).
Yield: 33.3%(yellow solid).
¹H-NMR(DMSO-d₆): *δ* 7.42(1H,dd,J=8.1,2.4Hz), 7.57-7.67(4H,m), 7.75-7.82(2H,m), 7.83-7.84(1H,m).

### Example 69: Preparation of the compound of Compound No. 69.

The title compound was obtained in the same manner as the Example 2(2) using the following raw materials.
Raw materials: 5-chlorosalicylaldehyde and 3-[3,5-bis(trifluoromethyl)benzyl]thiazolidine-2,4-dione(compound of the Example 2(1)).
Yield: 62.0%(light yellow powder).
¹H-NMR(DMSO-d₆): *δ* 5.03(2H,s), 7.00(1H,d,J=9.0Hz), 7.33(1H,d,J=2.4Hz), 7.38(1H,dd,J=8.7,2.7Hz), 8.03(1H,s), 8.05(2H,s), 8.07(1H,s), 10.95(1H,s).

### Example 70: Preparation of the compound of Compound No. 70.

The title compound was obtained in the same manner as the Example 2(2) using the following raw materials.
Raw materials: 2,3-dihydroxybenzaldehyde and 3-[3,5-bis(trifluoromethyl)benzyl]thiazolidine-2,4-dione(compound of the Example 2(1)).
Yield: 88.5%(yellow crystal).
¹H-NMR(DMSO·d₆): *δ* 5.02(2H,s), 6.88(1H,d,J=7.8Hz), 7.00-7.04(2H,m), 7.79(1H,s), 8.03(2H,s), 8.07(1H,s), 9.49(1H,s), 9.91(1H,s).

### Example 71: Preparation of the compound of Compound No. 71.

(1) 3-(2-Hydroxyphenyl)rhodanine.
   A mixture of 2-aminophenol(900 mg, 8.25 mmol), bis(carboxymethyl)trithiocarbonate(1.87g, 8.26 mmol) and water(25 ml) was refluxed for 4 hours. The reaction mixture was cooled, and the separated crystal was collected by filtration, washed with water and isopropyl ether to give the title compound(370 mg, 19.9%) as a yellow solid.
   ¹H-NMR(DMSO-ds): *δ* 4.41(2H,ABq,J=18.3Hz, Δ =21.0Hz), 6.89(1H,dt,J=7.8,1.5Hz), 6.95(1H,dd,J=8.4,1.2Hz), 7.07(1H,dd,J=7.8,1.5Hz), 7.26-7.32(1H,m), 9.85(1H,s).
(2) Benzo[2,1,3]oxadiazole-5-carbaldehyde-3-oxide.
   A mixture of 4-chloro-3-witrobenzaldehyde(1.00 g, 5.39 mmol), sodium azide(350 mg, 5.39 mmol), benzyltributylammonium bromide(192 mg, 0.539 mmol) and 1,2-dichloroethane(25 ml) was stirred at 60°C for 6 hours. The reaction mixture was left at room temperature overnight, and the insoluble matter was filtered off.
   The filtrate was washed with diluted hydrochloric acid and water, and dried overanhydrous sodium sulfate. The residue obtained by evaporation of the solvent under reduced pressure was purified by chromatography on silica gel(hexane:dichloromethane=1:1) to give the title compound(633 mg, 71.6%) as a yellow solid.
   ¹H-NMR(CDCl₃): *δ* 7.52-8.13(3H,brm), 10.05(1H,s).
(3) Benzo[2,1,3]oxadiazole-5-carbaldehyde.
   Triphenylphosphine(713 mg, 2.72 mmol) was added to a solution of benzo[2,1,3]oxadiazole-5-carbaldehyde-3-oxide(343 mg, 2.09 mmol) in toluene(4 ml), and the mixture was refluxed for 30 minutes. The residue obtained by concentration to about a half quantity of the reaction mixture under reduced pressure was purified by chromatography on silica gel(hexane:ethyl acetate=4:1) to give the title compound (216 mg, 69.8%) as a light yellow solid.
   ¹H-NMR(CDCl₃):δ 7.92-8.00(2H,m), 8.43(1H,t,J=1.2Hz), 10.14(1H,s).
(4) 5-[(Benzo[2,1,3]oxadiazol-5-yl)methylene]-3-(2-hydroxyphenyl)rhodanine (Compound No. 71).
   A mixture of benzo[2,1,3]oxadiazole -5-carbaldehyde(34.7 mg, 0.234 mmol), 3-(2-hydroxyphenyl)rhodanine(35 mg, 0.155 mmol), sodium acetate(12.7 mg, 0.155 mmol) and acetic acid(0.5 ml) was refluxed for 2 hours. The reaction mixture was cooled, and the separated crystal was collected by filtration, washed with acetic acid and isopropyl ether to give the title compound(35 mg, 63.4%) as a yellow solid.
   ¹H.NMR(DMSO.d₆) " *δ* 6.96(1H,dt,J=7.8,1.5Hz), 7.02(1H,dd,J=8.4,1.2Hz), 7.27(1H,dd,J=7.8,1.5Hz), 7.36(1H,dt,J=8.4,1.2Hz), 7.88(1H,dd,J=9.3,1.5Hz), 7.98(1H,s), 8.23(1H,d,J=9.3Hz), 8.41(1H,s), 10.07(1H,s).

### Example 72: Preparation of the compound of Compound No. 72.

The title compound was obtained in the same manner as the Example 71(4) using the following raw materials.
Raw materials: pyridine-4-carbaldehyde and 3-(2-hydroxyphenyl)rhodanine(compound of the Example 71(1)).
Yield: 54.4%(yellow solid).
¹H-NMR(DMSO-d₆): *δ* 6.95(1H,dt,J=7.8,1.5Hz), 7.00(1H,d,J=8.4Hz), 7.26(1H,dd,J=7.8,1.5Hz), 7.35(1H,dt,J=8.4,1.2Hz), 7.63(2H,d,J=6.0Hz), 7.82(1H,s), 7.77(2H,d,J=6.0Hz), 10.03(1H,s).

### Example 73: Preparation of the compound of Compound No. 73.

The title compound was obtained in the same manner as the Example 71(4) using the following raw materials.
Raw materials: 3,4-dihydroxybenzaldehyde and
3-(3-carboxy-4-hydroxyphenyl)rhodanine(compound of the Example 66(1)).
Yield: 74.1%(orange solid).
¹H-NMR(DMSO-d₆): *δ* 6.91(1H,d,J=9.0Hz), 7.09-7.11(2H,m), 7.62(1H,dd,J=8.4,2.4Hz), 7.67(1H,s), 7.76(1H,d,J=8.4Hz), 7.90(1H,d,J=2.4Hz), 9.60(1H,s), 10.05(1H,s), 13.66(1H,bs).

### Example 74: Preparation of the compound of Compound No. 74.

The title compound was obtained in the same manner as the Example 71(4) using the following raw materials.
Raw materials: pyridine-4-carbaldehyde and
3-(3-carboxy-4-hydroxyphenyl)rhodanine(compound of the Example 66(1)).
Yield: 49.6%(yellowish brown solid).
¹H-NMR(DMSO-d₆): *δ* 7.63-7.67(3H,m), 7.79(1H,d,J=9.0Hz), 7.83(1H,s), 7.9S(1H,d,J=2.4Hz), 8.77(2H,d,J=6.0Hz), l3.67(1H,bs).

### Example 75: Preparation of the compound of Compound No. 75.

(1) 3-(2,5-Dimethoxyphenyl)rhodanine.
   The title compound was obtained in the same manner as the Example 71(1) using the following raw materials.
   Raw materials: 2,5-dimethoxyaniline and bis(carboxymethyl)thiocarbonate.
   Yield: 39.1%(light yellow solid).
   ¹H-NMR(DMSO-d₆): *δ* 3.76(3H,s), 3.78(3H,s), 4.18(2H,ABq,J=18.0Hz, Δ =21.9Hz), 6.69-6.70(1H,m), 7.00-7.01(2H,m).
(2) 5-(3,4-Dihydroxybenzylidene)-3-(2,5-dimethoxyphenyl)rhodanine (Compound No. 75).
   The title compound was obtained in the same manner as the Example 71(4) using the following raw materials.
   Raw materials: 3,4-dihydroxybenzaldehyde and 3-(2,5-dimethoxyphenyl)rhodanine.
   Yield: 80.6%(yellow solid).
   ¹H-NMR(DMSO-d₆): *δ* 3.69(3H,s), 3.73(3H,s), 6.91(1H,d,J=9.0Hz), 7.02(1H,d,J=2.7Hz), 7.05-7.10(3H,m), 7.16(1H,d,J=9.0Hz), 7.67(1H,s), 9.59(1H,bs), 10.04(1H,bs).

### Example 76: Preparation of the compound of Compound No. 76.

(1) 3-(3,4-Dimethoxyphenyl)rhodanine.
   The title compound was obtained in the same manner as the Example 71(1) using the following raw materials.
   Raw materials: 3,4-dimethoxyaniline and bis(carboxymethyl)thiocarbonate.
   Yield: 81.7%(grayish white solid).
   ¹H-NMR(DMSO-d₆): *δ* 3.71(3H,s), 3.80(3H,s), 4.35(2H,s), 6.78(1H,dd,J=8.7,2.1Hz), 6.88(1H,d,J=2.1Hz), 7.06(1H,d,J=8.7Hz).
(2) 5-(3,4-Dihydroxybenzylidene)-3-(3,4-dimethoxyphenyl)rhodanine (Compound No. 76).
   The title compound was obtained in the same manner as the Example 71(4) using the following raw materials.
   Raw materials: 3,4-dihydroxybenzaldehyde and 3-(3,4-dimethoxyphenyl)rhodanine.
   Yield: 79.5%(yellow solid).
   ¹H-NMR(DMSO-d₆): *δ* 3.72(3H,s), 3.82(3H,s), 6.89-6.92(2H,m), 7.04(1H,d,J=2.1Hz), 7.07-7.10(3H,m), 7.65(1H,s), 9.58(1H,s), 10.02(1H,s).

### Example 77: Preparation of the compound of Compound No. 77.

(1) 3-(3,4-Dihydroxyphenyl)rhodanine.
   48% Aqueous hydrobromic acid(1 ml) was added to a solution of 3-(3,4-dimethoxyphenyl)rhodanine(compound of the Example 76(1); 200 mg, 0.743 mmol) in acetic acid(4 ml), and the mixture was refluxed for 8 hours. The reaction mixture was cooled, poured into water, and extracted with ethyl acetate. The reaction mixture was cooled. Water was added to the mixture, and the mixture was extracted with ethyl acetate. The residue obtained by evaporation of the solvent under reduced pressure was purified by chromatography on silica gel(hexane:ethyl acetate=1:2) to give the title compound(90 mg, 50.3%) as a light yellow solid.
   ¹H-NMR(DMSO-d₆): *δ* 4.33(2H,s), 6.47(1H,dd,J=8.4,2.1Hz), 6.58(1H,d,J=2.1Hz), 6.81(1H,d,J=8.4Hz), 9.27(2H,bs).
(2) 5-(3,4-Dihydroxybenzylidene)-3-(3,4-dihydroxyphenyl)rhodanine (Compound No. 77).
   The title compound was obtained in the same manner as the Example 71(4) using the following raw materials.
   Raw materials: 3,4-dihydroxybenzaldehyde and 3-(3,4-dihydroxyphenyl)rhodanine.
   Yield: 69.0%(yellow solid).
   ¹H-NMR(DMSO-d₆): *δ* 6.59(1H,dd,J=8.4,2.4Hz), 6,69(1H,d,J=2.4Hz), 6.83(1H,d,J =8.4Hz), 6.90(1H,d,J=8.7Hz), 7.05-7.08(2H,m), 7.63(1H,s), 9.29(1H,s), 9.35(1H,s), 9.57(1H,s), 10.00(1H,s).

### Example 78: Preparation of the compound of Compound No. 78.

(1) 3-[3,4,5-Tri(benzyloxy)benzyl]thiazolidine-2,4-dione.
   The title compound was obtained in the same manner as the Example 11(1) using the following raw materials.
   Raw materials: 3,4,5-tri(benzyloxy)benzylalcohol and 2,4-thiazolidinedione.
   Yield: 69.0%(yellow solid).
   ¹H.NMR(CDCl₃): *δ* 3.89(2H,s), 4.64(2H,s), 5.02(2H,s), 5.09(4H,s), 6.71(2H,s), 7.25-7.44(15H,m).
(2) 5-(3,4-Dichlorobenzylidene)-3-[3,4,5-tri(benzyloxy)benzyl]thiazolidine-2,4-dione.
   The title compound was obtained in the same manner as the Example 2(2) using the following raw materials.
   Raw materials: 3,4,5-tri(benzyloxy)benzylalcohol and 2,4-thiazolidinedione.
   Yield: 22.0%(yellow powder).
   ¹H-NMR(CDCl₃):δ 4.78(2H,s), 5.01(2H,s), 5.10(4H,s), 6.75(2H,s), 7.24-7.44(16H,m), 7.56(1H,d,J=8.4Hz), 7.58(1H,d,J=2.1Hz), 7.77(1H,s).
(3) 5-(3,4-Dichlorobenzylidene)-3-(3,4,5-trihydroxybenzyl)thiazolidine-2,4-dione (Compound No. 78).
   Boron tribromide(1M solution in dichloromethane; 0.3 ml) was added to a solution of 5-(3,4-dichlorobenzylidene)-3-[3,4,5-tri(benzyloxy)benzyl]-thiazolidine-2,4-dione(70.4mg, 0.10mmol) in dichloromethane(3ml) under ice cooling and argon atmosphere, and the mixture was stirred at room temperature for 1 hour. The reaction mixture was poured into water and extracted with ethyl acetate. The organic layer was washed with brine and dried over anhydrous sodium sulfate. The residue obtained by evaporation of the solvent under reduced pressure was crystallized by ethyl acetate/isopropyl ether to give the title compound(20 mg, 47.2%) as a light yellowish gray powder.
   ¹H-NMR(DMSO-d₆): *δ* 4.58(2H,s), 6.25(2H,s), 7.59(1H,dd,J=8.7,2.4Hz), 7.82(1H,d,J=8.7Hz), 7.96(1H,d,J=2.4Hz), 7.97(1H,s), 8.15(1H,s), 8.93(2H,s).

### Example 79: Preparation of the compound of Compound No. 79.

The title compound was obtained in the same manner as the Example 2(2) using the following raw materials.
Raw materials: 4-(acetamido)benzaldehyde and
3-(3,4-dichlorobenzyl)thiazolidine-2,4-dione(compound of the Example 8(1)).
Yield: 63.0%(white solid).
¹H-NMR(DMSO-d₆): *δ* 2.08(3H,s), 4.84(2H,s), 7.30(1H,dd,J=8.4,2.1Hz), 7.58-7.64(4H,m), 7.76(2H,d,J=8.7Hz), 7.89(1H,s), 10.30(1H,s).

### Example 80: Preparation of the compound of Compound No. 80.

A mixture of 5-[4-(acetamido)benzylidene]-3-(3,4-dichlorobenzyl)-thiazolidine-2,4-dione(Compound No. 79; 50 mg, 0.12 mmol), concentrated hydrochloric acid(0.3 ml) and methanol(2 ml) was refluxed for 4 hours. The reaction mixture was cooled, and the separated crystal was collected by filtration to give the title compound(39.4 mg, 80.4%) as a white solid.
¹H-NMR(DMSO-d₆): *δ* 4.58(3H,brs), 4.82(2H,s), 6.77(2H,d,J=8.7Hz), 7.28(1H,dd,J=8.4,2.1Hz), 7.38(2H,d,J=8.7Hz), 7.59(1H,d,J=2.1Hz), 7.62(1H,d,J=8.1Hz), 7.79(1H,s).

### Example 81: Preparation of the compound of Compound No. 81.

(1) 3-(3,4-Dichlorobenzyl)rhodanine.
   The title compound was obtained in the same manner as the Example 11(1) using the following raw materials.
   Raw materials: 3,4-dichlorobenzylalcohol and rhodanine.
   Yield: 29.1%(light yellow powder).
   ¹H-NMR(CDCl₃): *δ* 4.02(2H,s), 4.52(2H,s), 7.25(1H,dd, J=8.4,2.1Hz), 7.41(1H,d,J=8.4Hz), 7.49(1H,d,J=2.1Hz).
(2) 5-(3,4-Dihydroxybenzylidene)-3-(3,4-dichlorobenzyl)rhodanine (Compound No. 81).
   The title compound was obtained in the same manner as the Example 71(4) using the following raw materials.
   Raw materials: 3,4-dihydroxybenzaldehyde and 3-(3,4-dichlorobenzyl)rhodanine.
   Yield: 80.0%(yellow powder).
   ¹H-NMR(DMSO-d₆): *δ* 5.22(2H,s), 6.90(1H,d,J=7.8Hz), 7.06-7.09(2H,m), 7.29(1H,dd,J=8.4,2.1Hz), 7.61(1H,d,J=8.1Hz), 7.62(1H,d,J=2.1Hz), 7.69(1H,s), 9.61(1H,s), 10.09(1H,s).

### Example 82: Preparation of the compound of Compound No. 82.

The title compound was obtained in the same manner as the Example 2(2) using the following raw materials.
Raw materials: 3,4-dihydroxybenzaldehyde and 3-{[4-(N-benzylcarbamoyl)methyl]benzyl}thiazolidine-2,4-dione.
Yield: 37.4%(yellow solid).
¹H-NMR(DMSO-d₆): *δ* 3.45(2H,s), 4.24(2H,d,J=6.0Hz), 4.79(2H,s), 6.88(1H,d,J=8.1Hz), 6.99-7.04(2H,m), 7.19-7.32(9H,m), 7.78(1H,s), 8.54(1H,t,J=6.0Hz), 9.52(1H,s), 9.93(1H,s).

### Example 83: Preparation of the compound of Compound No. 83.

The title compound was obtained in the same manner as the Example 2(2) using the following raw materials.
Raw materials: 3,4-dihydroxybenzaldehyde and 3-[4-(benzoylamino)benzyl]thiazolidine-2,4-dione.
Yield: 86.3%(yellow solid).
¹H-NMR(DMSO-d₆): *δ* 4.79(2H,s), 6.86(1H,d,J=8.1Hz), 7.01(2H,d,J=7.8Hz), 7.29(2H,d,J=8.4Hz), 7.50-7.61(3H,m), 7.75(2H,d,J=8.4Hz), 7.78(1H,s), 7.94(2H,d,J=6.9Hz), 10.29(1H,s).

### Example 84: Preparation of the compound of Compound No. 84.

Concentrated hydrochloric acid(50 *µ*l, 0.468 mmol) and iron powder(13 mg, 0.234 mmol) were added to a mixture of 5-(2,3-dihydroxybenzylidene)-3-(4-nitrobenzyl)thiazolidine-2,4-dione(Compound No. 51; 29 mg, 0.078 mmol) and methanol, and the mixture was stirred at room temperature for 30 minutes. The reaction mixture was poured into water and extracted with ethyl acetate. The organic layer was washed with water and brine, and dried over anhydrous magnesium sulfate. The residue obtained by evaporation of the solvent under reduced pressure was purified by chromatography on silica gel(hexane:ethyl acetate=1:2). The obtained residue was dissolved in ethyl acetate, and 4N hydrogen chloride/ethyl acetate solution was added to the solution. The residue obtained by evaporation of the solvent under reduced pressure was dried to give the title compound(15.7 mg,58.8%) as a light yellow crystal.
¹H-NMR(DMSO-d₆): *δ* 4.82(2H,s), 6.76-6.86(2H,m), 6.93(1H,dd,J=7.8,2.1Hz), 7.20(1H,d,J=8.7Hz), 7.36(1H,d,J=8.7Hz), 8.19(1H,s), 9.55(1H,brs), 9.95(1H,brs).

### Example 85: Preparation of the compound of Compound No. 85.

The title compound was obtained in the same manner as the Example 2(2) using the following raw materials.
Raw materials: 4-hydroxy-3-nitrobenzaldehyde and
3-(3,4-dichlorobenzyl)thiazolidine-2,4-dione(compound of the Example 8(1)).
Yield: 51.2%(yellow powder).
¹H-NMR(DMSO-d₆):δ 4.84(2H,s), 7.20(1H,d,J=9.0Hz), 7.30(1H,dd,J=8.4,1.8Hz), 7.61-7.64(2H,m), 7.73(1H,dd,J=9.0,2.1Hz), 7.94(1H,s), 8.20(1H,d,J=2.4Hz), 11.91(1H,brs).

### Example 86: Preparation of the compound of Compound No. 86.

A mixture of 5-(4-hydroxy-3-nitrobenzylidene)-3-(3,4-dichlorobenzyl)-thiazolidine-2,4-dione(Compound No. 85; 187 mg, 0.44 mmol), iron powder(0.10g), concentrated hydrochloric acid(0.3 ml) and ethanol(5 ml) was refluxed for 2 hours. The reaction mixture was cooled. Ethyl acetate and saturated sodium hydrogencarbonate solution were added to the mixture, and the insoluble matter was filtered off. The filtrate was extracted with ethyl acetate. The organic layer was washed with brine and dried over anhydrous sodium sulfate. The residue obtained by evaporation of the solvent under reduced pressure was washed with ethyl acetate/hexane under suspension to give the title compound(54.6 mg, 31.5%) as a yellow powder.
¹H-NMR(DMSO-d₆): *δ* 4.82(2H,s), 4.89(2H,brs), 6.78(2H,s), 6.86(1H,s), 7.28(1H,dd,J=8.4,2.1Hz), 7.59-7.63(2H,m), 7.71(1H,s), 9.92(1H,brs).

### Example 87: Preparation of the compound of Compound No. 87.

The title compound was obtained in the same manner as the Example 2(2) using the following raw materials.
Raw materials: 3-hydroxy-4-nitrobenzaldehyde and
3-(3,4-dichlorobenzyl)thiazolidine-2,4-dione(compound of the Example 8(1)).
Yield: 59.3%(yellow powder).
¹H-NMR(DMSO-d₆): *δ* 4.85(2H,s), 7.21(1H,d,J=8.7Hz), 7.32(1H,dd,J=8.7,2.4Hz), 7.34(1H,d,J=1.8Hz), 7.62(1H,d,J=8.4Hz), 7.64(1H,d,J=1.8Hz), 7.92(1H,s), 7.99(1H,d,J=8.4Hz), 11.38(1H,brs).

### Example 88: Preparation of the compound of Compound No. 88.

The title compound was obtained in the same manner as the Example 86 using the following raw material.
Raw material: 5-(3-hydroxy-4-nitrobenzylidene)-3-(3,4-dichlorobenzyl)-thiazolidine-2,4-dione(Compound No. 87).
Yield: 42.4%(yellow powder).
¹H-NMR(DMSO-ds): *δ* 4.81(2H,s), 5.65(2H,s), 6.68(1H,d,J=7.8Hz), 6.92-6.97(2H,m), 7.27(1H,dd,J=8.4,2.4Hz), 7.58(1H,d,J=1.8Hz), 7.61(1H,d,J=8.4Hz), 7.70(1H,s), 9.60(1H,s).

### Example 89: Preparation of the compound of Compound No. 89.

The title compound was obtained in the same manner as the Example 2(2) using the following raw materials.
Raw materials: 3,4-dihydroxybenzaldehyde and 3-{4-[(3,4-dichlorobenzoyl)amino]benzyl}thiazolidine-2,4-dione.
Yield: 27.6%(yellow solid).
¹H-NMR(DMSO-d₆): *δ* 4.80(2H,s), 6.88(1H,d,J=7.8Hz), 7.00-7.03(2H,m), 7.31(2H,d,J=8.4Hz), 7.73(2H,d,J=8.4Hz), 7.79(1H,s), 7.82(1H,d,J=8.1Hz), 7.93(1H,dd,J=8.4,2.1Hz), 8.21(1H,d,J=2.1Hz), 9.52(1H,s), 9.94(1H,s), 10.44(1H,s).

### Example 90: Preparation of the compound of Compound No. 90.

(1) 3-[2-(4-Chlorophenyl)ethyl]thiazolidine-2,4-dione.
   The title compound was obtained in the same manner as the Example 11(1) using the following raw materials.
   Raw materials: 4-chlorophenethylalcohol and 2,4-thiazolidinedione.
   Yield: 79.8%(white crystal).
   ¹H-NMR(CDCl₃): *δ* 2.86-2.91(2H,m), 3.80-3.86(2H,m), 3.90(2H,s), 7.15(2H,d,J=8.7Hz), 7.27(2H,d,J=8.1Hz).
(2) 5-(2,3-Dihydroxybenzylidene)-3-[2-(4-chlorophenyl)ethyl]thiazolidine-2,4-dione (Compound No. 90).
   The title compound was obtained in the same manner as the Example 2(2) using the following raw materials.
   Raw materials: 2,3-dihydroxybenzaldehyde and 3-[2-(4-chlorophenyl)ethyl]thiazolidine-2,4-dione.
   Yield: 51.6%(light yellowish brown powder).
   ¹H-NMR(DMSO-d₆): *δ* 2.91(2H,t,J=7.2Hz), 3.87(2H,t,J=6.9Hz), 6.75-6.84(2H,m), 6.91(1H,dd,J=7.2,2.1Hz), 7.23(2H,d,J=8.4Hz), 7.34(2H,d,J=8.4Hz), 8.13(1H,s), 9.45(1H,s), 9.83(1H,s).

### Example 91: Preparation of the compound of Compound No. 91.

The title compound was obtained in the same manner as the Example 2(2) using the following raw materials.
Raw materials: 3,4-dihydroxybenzaldehyde and
3-[2-(4-chlorophenyl)ethyl]thiazolidine-2,4-dione(compound of the Example 90(1)). Yield: 82.2%(yellowish white powder).
¹H-NMR(DMSO-d₆): *δ* 2.90(2H,t,J=7.2Hz), 3.87(2H,t,J=7.2Hz), 6.87(1H,d,J=8.1Hz), 6.97-7.02(2H,m), 7.22(2H,d,J=8.4Hz), 7.34(2H,d,J=8.4Hz), 7.71(1H,s), 9.45(1H,s), 9.86(1H,s).

### Example 92: Preparation of the compound of Compound No. 92.

(1) 3-[(2,4-Dichlorobenzoyl)methyl]thiazolidine-2,4-dione.
   Potassium carbonate(0.70 g, 5.06 mmol) was added to a solution of 2,4-thiazolidinedione(0.30 g,2.56 mmol), 2,2',4'-trichloroacetophenone(0.57 g, 2.56 mmol) in N,N-dimethylformamide(6 ml), and the mixture was stirred at 80°C for 2 hours. The reaction mixture was cooled, poured into water, and extracted with ethyl acetate. The organic layer was washed with water and brine, and dried over anhydrous sodium sulfate. The residue obtained by evaporation of the solvent under reduced pressure was purified by chromatography on silica gel(hexane:ethyl acetate=2:1) to give the title compound(124.5mg, 16.0%) as a brown oil.
   ¹H-NMR(CDCl₃): *δ* 4.09(2H,s), 4.99(2H,s), 7.38(1H,dd,J=8.4,2.1Hz), 7.51(1H,d,J=2.1Hz), 7.69(1H,d,J8.4Hz).
(2) 5-(3,4-Dihydroxybenzylidene)-3-[(2,4-dichlorobenzoyl)-methyl]thiazolidine-2,4-dione (Compound No. 92).
   The title compound was obtained in the same manner as the Example 2(2) using the following raw materials.
   Raw materials: 3,4-dihydroxybenzaldehyde and 3-[(2,4-dichlorobenzoyl)methyl]thiazolidine-2,4-dione.
   Yield: 60.3%(ocherous powder).
   ¹H-NMR(DMSO-d₆): *δ* 5.15(2H,s), 6.90(1H,d,J=8.4Hz), 7.02-7.07(2H,m), 7.64(1H,dd,J=8.4,2.1Hz), 7.82(1H,s), 7.83(1H,d,J=2.1Hz), 7.97(1H,d,J=8.1Hz), 9.50(1H,s), 9.93(1H,s).

### Example 93: Preparation of the compound of Compound No. 93.

(1) 2-Bromo-N-phenylacetamide.
   Bromoacetyl chloride(330 mg, 2.1 mmol) was added to a solution of aniline(190 mg, 2.04 mmol) and triethylamine(0.4 ml, 2.86 mmol) in tetrahydrofuran(5 ml) under ice cooling, and the mixture was stirred for 30 minutes. The reaction mixture was poured into water and extracted with ethyl acetate. The organic layer was washed with water and brine, and dried over anhydrous sodium sulfate. The residue obtained by evaporation of the solvent under reduced pressure was purified by chromatography on silica gel(hexane:ethyl acetate=2:1) and washed with isopropyl ether/hexane to give the title compound(405.6 mg, 92.9%) as a white powder.
   ¹H-NMR(CDCl₆): *δ* 4.04(2H,s), 7.17(1H,tt,J=7.5,1.2Hz), 7.37(2H,t,J=7.2Hz), 7.52-7.55(2H,m), 8.12(1H,s).
(2) 3-[(N-Phenylcarbamoyl)methyl]thiazolidine-2,4-dione.
   Potassium carbonate(262.6 mg, 1.9 mmol) was added to a solution of 2-bromo-N-phenylacetamide(203.5 mg, 0.95 mmol) and 2,4-thiazolidinedione(111.4 mg, 0.95 mmol) in N,N-dimethylformamide(4 ml), and the mixture was stirred at 45°C for 1 hour. The reaction mixture was cooled, poured into water, and extracted with ethyl acetate. The organic layer was washed with water and brine, and dried over anhydrous sodium sulfate. The residue obtained by evaporation of the solvent under reduced pressure was washed with isopropyl ether/hexane to give the title compound(167.7 mg, 70.6%) as a light pink powder.
   ¹H.NMR(CDCl₃): *δ* 4.07(2H,s), 4.42(2H,s), 7.14(1H,t,J=7.5Hz), 7.29-7.35(2H,m), 7.39-7.49(3H,m).
(3) 5-(3,4-Dihydroxybenzylidene)-3-[(N-phenylcarbamoyl)-methyl]thiazolidine-2,4-dione (Compound No. 93).
   The title compound was obtained in the same manner as the Example 2(2) using the following raw materials.
   Raw materials: 3,4-dihydroxybenzaldehyde and 3-[(N-phenylcarbamoyl)methyl]thiazolidine-2,4-dione.
   Yield: 56.1%(orange powder).
   ¹H-NMR(DMSO-ds): *δ* 4.47(2H,s), 6.70(1H,d,J=8.1Hz), 6.95(1H,d,J=2.1Hz), 6.98(1H,dd, J=8.4,2.7Hz), 7.07(1H,t, J=7.5Hz), 7.32(2H,t, J=8.1Hz), 7.53-7.57(2H,m), 7.73(1H,s).

### Example 101: The compound of Compound No. 101.

This compound is a commercially available compound.
Supplier: Specs (Netherlands).
Catalog code number: AG-690/32536049.

### Example 102: The compound of Compound No. 102.

This compound is a commercially available compound.
Supplier: LaboTest (Germany).
Catalog code number: LT00056066.

### Example 103: The compound of Compound No. 103.

This compound is a commercially available compound.
Supplier: Specs (Netherlands).
Catalog code number: AN-988/40838350.

### Example 104: Preparation of the compound of Compound No. 104.

(1) 1-(3,5-Dichlorophenyl)-3-isopropyl-2-pyrazolin-5-one.
   A mixture of 3,5-dichlorophenylhydrazine hydrochloride (1.00g, 4.684 mmol), ethyl isobutyrylacetate(780 mg, 4.684 mmol) and ethanol(5.0 ml) was refluxed for 4 hours. The reaction mixture was cooled. The separated crystal was collected by filtration and washed with ethyl acetate, hexane and water to give the title compound(732 mg, 58.3%) as a white solid.
   ¹H-NMR(DMSO-d₆): *δ* 1.19-1.22(6H,m), 2.70-2.83(1H,m), 5.43(2H,s), 7.43(1H,t,J=1.7Hz), 7.83(2H,d,J=1.7Hz).
(2) 4-(2,3-Dihydroxybenzylidene)-1-(3,5-dichlorophenyl)-3-isopropyl-2-pyrazolin-5-one (Compound No. 104).
   A mixture of 1-(3,5-dichlorophenyl)-3-isopropyl-2-pyrazolin-5-one(100 mg, 0.373 mmol), 2,3-dihydroxybenzaldehyde(53 mg, 0.373 mmol), ammonium acetate(29 mg, 0.373 mmol) and ethanol(1.0 ml) was stirred at room temperature for 30 minutes. The residue obtained by evaporation of the solvent under reduced pressure was purified by column chromatography on silica gel(hexane:ethyl acetate=4:1) to give the title compound(47 mg, 32.2%) as an orange solid.
   ¹H-NMR(CDCl₃) : *δ* 1.41(6H,d,J=6.9Hz), 3.09-3.23(1H,m), 6.46(1H,s), 6.93-7.03(2H,m), 7.21(1H,dd,J=7.2,2.1Hz), 7.22-7.25(1H,m), 7.69(1H,s), 8.02-8.05(2H,m), 10.86(1H,s).

### Example 105: Preparation of the compound of Compound No. 105.

The title compound was obtained in the same manner as the Example 104(2).
Yield: 78.4%(light orange solid).
¹H-NMR(CDCl₃): *δ* 1.40(6H,d,J=6.9Hz), 3.02-3.09(1H,m), 7.20(1H,t,J=1.5Hz), 7.72(1H,s), 7.84(1H,d,J=2.4Hz), 8.01(2H,d,J=1.5Hz), 8.44(1H,d,J=2.4Hz).

### Example 106: Preparation of the compound of Compound No. 106.

The title compound was obtained in the same manner as the Example 104(2).
Yield: 33.0%(ocherous solid).
¹H-NMR(DMSO-d₆): *δ* 1.20-1.41(6H,m), 3.13-3.33(1H,m), 7.02-7.14(1H,m), 7.41-7.44(1H,m), 7.81-8.15(3H,m), 8.91-8.97(2H,m).

### Example 107: Preparation of the compound of Compound No. 107.

The title compound was obtained in the same manner as the Example 104(2).
Yield: 22.8%(ocherous solid).
¹H.NMR(DMSO.d₆): *δ* 1.29(6H,d,J=6.9Hz), 3.27-3.40(1H,m), 6.92(1H,t,J=8.4Hz), 7.42(1H,t,J=1.8Hz), 7.73(1H,s), 7.90(1H,dd,J=8.4,2.4Hz), 8.02-8.05(2H,m), 8.50(1H,d,J=2.4Hz), 9.58(1H,s), 10.52(1H,s).

### Example 108: Preparation of the compound of Compound No. 108.

(1) 1-(3-Carboxyphenyl)-3-isopropyl-2-pyrazolin-5-one.
   The title compound was obtained in the same manner as the Example 104(1).
   Yield: 56.2%(orange solid).
   ¹H-NMR(DMSO-d₆): *δ* 1.12(6H,d,J=7.0Hz), 2.75-2.85(1H,m), 5.43(1H,s), 7.54(1H,t,J=8.1Hz), 7.75-7.78(1H,m), 7.98-8.03(1H,m), 8.33(1H,s), 11.66(1H,s), 13.11(1H,s).
(2) 1-[3-(Methoxycarbonyl)phenyl]-3-isopropyl-2-pyrazolin-5-one.
   Concentrated sulfuric acid(0.2 ml) was added to a solution of 1-(3-Carboxyphenyl)-3-isopropyl-2-pyrazolin-5-one(900 mg, 3.689 mmol) in methanol(5 ml), and the mixture was refluxed for 8 hours. Ethyl acetate was added to the residue obtained by concentration of the reaction mixture under reduced pressure. The organic layer was washed with water, saturated aqueous sodium hydrogen carbonate and brine, and dried over anhydrous sodium sulfate. The residue obtained by evaporation of the solvent under reduced pressure was purified by column chromatography on silica gel(hexane:ethyl acetate=3:2) to give the title compound(900 mg, 93.8%) as a white solid.
   ¹H.NMR(CDCl₃)" *δ* 1.27(6H,d,J=6.9Hz), 2.75-2.89(1H,m), 3.45(2H,s), 3.93(3H,s), 7.46(1H,t,J=8.1Hz), 7.83-7.88(1H,m), 8.13-8.17(1H,m), 8.51(1H,t,J=1.8Hz).
(3) 4-(3,5-Dibromo-2-hydroxybenzylidene)-1-[3-(Methoxycarbonyl)phenyl]-3-isopropyl-2-pyrazolin-5-one (Compound No. 108).
   The title compound was obtained in the same manner as the Example 104(2).
   Yield: 14.1%(orange solid).
   ¹H-NMR(CDCl₃): *δ* 1.41(6H,d,J=6.9Hz), 3.05-3.20(1H,m), 3.95(3H,s), 7.25-7.30(1H,m), 7.51(1H,t,J=7.5Hz), 7.67(1H,s), 7.86(1H,d,J=6.9Hz), 7.89-7.96(1H,m), 8.20-8.28(2H,s), 8.60-8.66(1H,m).

### Example 109: Preparation of the compound of Compound No. 109.

(1) 1-[3-(Trifluoromethyl)phenyl]-3-methyl-2-pyrazolin-5-one.
   The title compound was obtained in the same manner as the Example 104(1).
   Yield: 78.6%(white solid).
   ¹H-NMR(CDCl₃): *δ* 2.22(3H,s), 3.47(2H,s), 7.41-7.43(1H,m), 7.50(1H,t,J=7.8Hz), 8.14-8.17(2H,m).
(2) 4-(2,4-Dihydroxybenzylidene)-1-[3-(trifluoromethyl)phenyl]-3-methyl-2-pyrazolin-5-one (Compound No. 109).
   The title compound was obtained in the same manner as the Example 104(2).
   Yield: 14.1%(white solid).
   ¹H-NMR(DMSO-d₆): *δ* 2.31(3H,s), 6.42-6.45(2H,m), 7.52(1H,d,J=7.8Hz), 7.67(1H,t,J=7.8Hz), 8.01(1H,s), 8.22-8.25(1H,m), 8.37(1H,s), 9.34(1H,d,J=9.6Hz), 10.86(1H,m), 11.00(1H,s).

### Example 110: Preparation of the compound of Compound No. 110.

(1) 1-Phenyl-3-ethyl-2-pyrazolin-5-one.
   The title compound was obtained in the same manner as the Example 104(1).
   Yield: 96.2%(white solid).
   ¹H-NMR(CDCl₃): *δ* 1.25(3H,t,J=7.6Hz), 2.52(2H,q,J=7.6Hz), 3.42(2H,s), 7.13-7.20(1H,m), 7.35-7.41(2H,m), 7.86-7.89(2H,m).
(2) 4-(2,4-Dihydroxybenzylidene)-1-phenyl-3-ethyl-2-pyrazolin-5-one (Compound No. 110).
   The title compound was obtained in the same manner as the Example 104(2).
   Yield: 13.5%(orange solid).
   ¹H-NMR(DMSO-d₆): *δ* 1.27(3H,t,J=7.5Hz), 2.69(2H,q,J=7.5Hz), 6.38-6.43(2H,m), 7.14-7.19(1H,m), 7.40-7.45(2H,m), 7.93-8.01(3H,m), 9.35(1H,d,J=9.3Hz), 10.76(1H,s), 10.90(1H,s).

### Example 111: Preparation of the compound of Compound No. 111.

The title compound was obtained in the same manner as the Example 104(2).
Yield: 14.7%(orange solid).
¹H-NMR(DMSO-d₆):δ 2.30(3H,s), 2.31(3H,s), 6.90(1H,d,J=8.4Hz), 7.23(2H,d,J=8.1Hz), 7.58(1H,s), 7.81(2H,d,J=8.1Hz), 7.92(1H,dd,J=8.4,2.1Hz), 8.48(1H,d,J=2.1Hz), 9.50(1H,brs), 10.38(1H,brs).

### Example 112: Preparation of the compound of Compound No. 112.

(1) 1-(3,5-Dichlorophenyl)-3-methyl-2-pyrazolin-5-one.
   The title compound was obtained in the same manner as the Example 104(1).
   Yield: 48.8%(white solid).
   ¹H-NMR(CDCl₃): *δ* 2.21(3H,s), 3.45(2H,s), 7.15(1H,t,J=2.0Hz), 7.90(d,2H,J=1.7Hz).
(2) 4-(3,4-Dihydroxybenzylidene)-1-(3,5-dichlorophenyl)-3-methyl-2-pyrazolin-5-one (Compound No. 112).
   The title compound was obtained in the same manner as the Example 104(2).
   Yield: 56.2%(brown solid).
   ¹H-NMR(DMSO·d₆): *δ* 2.30(3H,s), 6.80(1H,d,J=8.4Hz), 7.37(1H,t,J=1.8Hz), 7.56(1H,s), 7.84-7.90(1H,m), 8.04(2H,d,J=1.8Hz), 8.42-8.48(1H,m).

### Example 113: Preparation of the compound of Compound No. 113.

The title compound was obtained in the same manner as the Example 104(2).
Yield: 9.8%(yellow solid).
¹H-NMR(DMSO·d₆): *δ* 2.23(3H,s), 6.33(1H,d,J=8.4Hz), 7.16(1H,s), 7.40(1H,d,J=8.1Hz), 7.60(1H,t,J=8.4Hz), 7.8(1H,brs), 8.29-8.46(2H,m), 8.48(1H,s).

### Example 114: Preparation of the compound of Compound No. 114.

The title compound was obtained in the same manner as the Example 104(2).
Yield: 30.9%(yellowish orange solid).
¹H-NMR(DMSO-d₆): *δ* 1.27(3H,t,J=7.4Hz), 2.74(2H,q,J=7.4Hz), 6.90(1H,d,J=8.4Hz), 7.16-7.21(1H,m), 7.41-7.47(2H,m), 7.62(1H,s), 7.91-7.97(3H,m), 8.49(1H,d,J=2.4Hz).

### Example 115: Preparation of the compound of Compound No. 115.

(1) 1-Benzyl-3-methyl-2-pyrazolin-5-one.
   The title compound was obtained in the same manner as the Example 104(1).
   Yield: 55.3%(white solid).
   ¹H-NMR(CDCl₃): *δ* 2.07(3H,s), 3.22(2H,s), 4.80(2H,s), 7.26-7.35(5H,m).
(2) 4-(3,4-Dihydroxybenzylidene)-1-benzyl-3-methyl-2-pyrazolin-5-one (Compound No. 115).
   The title compound was obtained in the same manner as the Example 104(2).
   Yield: 11.0%(ocherous solid).
   ¹H-NMR(DMSO-ds): *δ* 2.16(3H,s), 4.84(2H,s), 6.85(1H,d,J=8.4Hz), 7.24-7.36(5H,m), 7.49(1H,s), 7.89(1H,dd,J=8.4,2.1Hz), 8.47(1H,d,J=2.1Hz), 9.61(2H,brs).

### Example 116: Preparation of the compound of Compound No. 116.

The title compound was obtained in the same manner as the Example 104(2).
Yield: 80.8%(orange solid).

The compound was obtained as a mixture of the geometrical isomers of the exomethylene in the 4-position of the pyrazolone ring.
¹H-NMR(DMSO-d₆): *δ* 1.44 and 1.49(total 3H,each d,J=6.9Hz), 4.43 and 4.50(total 2H,each t,J=6.9Hz), 6.90 and 6.91(total 1H,each d,J=8.7Hz), 7.54 and 7.65(total 1H,each s), 7.58 and 7.82(total 1H,each dd,J=8.7,2.4Hz), 7.86 and 8.43(total 1H,each d,J=2.4Hz), 8.11-8.34(total 4H,m), 9.43 and 9.60(total 1H,each brs), 10.61(1H,s).

### Example 117: Preparation of the compound of Compound No. 117.

The title compound was obtained in the same manner as the Example 104(2).
Yield: 58.8%(yellow solid).
¹H-NMR(DMSO·d₆): *δ* 2.32(3H,s), 6.92(1H,d,J=8.1Hz), 7.21-7.26(1H,m), 7.46(1H,t,J=7.8Hz), 7.64(1H,s), 7.86-7.94(2H,m), 8.09(1H,t,J=2.1Hz), 8.48(1H,d,J=2.1Hz), 9.56(1H,s), 10.47(1H,s).

### Example 118: Preparation of the compound of Compound No. 118.

(1) 1-[3,5-Bis(trifluoromethyl)phenyl]-3-isopropyl-2-pyrazolin-5-one.
   The title compound was obtained in the same manner as the Example 104(1).
   Yield: 19.7%(white solid).
   ¹H-NMR(CDCl₃): *δ* 1.28(6H,d,J=6.9Hz), 2.80-2.87(1H,m), 3.50(2H,s), 7.66(1H,s), 8.46(2H,s).
(2) 4-(3,4-Dihydroxybenzylidene)-1-[3,5-bis(trifluoromethyl)phenyl]-3-isopropyl-2-pyrazolin-5-one (Compound No. 118).
   The title compound was obtained in the same manner as the Example 104(2).
   Yield: 95.9%(yellow solid).
   ¹H-NMR(DMSO-ds): *δ* 1.31(6H,d,J=6.9Hz), 3.27-3.34(1H,m), 6.94(1H,d,J=8.4Hz), 7.77(1H,s), 7.89(1H,s), 7.93(1H,dd,J=8.1,2.1Hz), 8.49(1H,d,J=2.1Hz), 8.61(2H,s), 9.61(1H,brs), 10.56(1H,brs).

### Example 119: Preparation of the compound of Compound No. 119.

(1) 1-(3-Chloro-4-methylphenyl)-3-isopropyl-2-pyrazolin-5-one.
   The title compound was obtained in the same manner as the Example 104(1).
   Yield: 39.9%(white solid).
   ¹H-NMR(CDCl₃): *δ* 1.25(6H,d,J=7.2Hz), 2.36(3H,s), 2.72-2.86(1H,m), 3.41(2H,m), 7.22(1H,d,J=8.1Hz), 7.73(1H,dd,J=8.1,2.1Hz), 7.91(1H,d,J=2.1Hz).
(2) 4-(3,4-Dihydroxybenzylidene)-1-(3-chloro-4-methylphenyl)-3-isopropyl-2-pyrazolin-5-one (Compound No. 119).
   The title compound was obtained in the same manner as the Example 104(2).
   Yield: 20.2%(orange solid).
   ¹H-NMR(DMSO-d₆): *δ* 1.29(6H,d,J=6.6Hz), 2.33(3H,s), 3.20-3.34(1H,m), 6.92(1H,d,J=8.4Hz), 7.40(1H,d,J=8.7Hz), 7.67(1H,s), 7.82(1H,d,J=8.4Hz), 7.92(1H,d,J=8.7Hz), 8.09(1H,s), 8.54(1H,s), 9.55(1H,brs), 10.44(1H,brs).

### Example 120: Preparation of the compound of Compound No. 120.

(1) 1-(2,3-Dimethylphenyl)-3-isopropyl-2-pyrazolin-5-one.
   The title compound was obtained in the same manner as the Example 104(1).
   Yield: 21.7%(white solid).
   ¹H-NMR(CDCl₃): Iδ 1.23(6H,d,J=7.1Hz), 2.14(3H,s), 2.31(3H,s), 2.70-2.85(1H,m), 3.39(2H,m), 7.15(3H,s).
(2) 4-(3,4-Dihydroxybenzylidene)-1-(2,3-dimethylphenyl)-3-isopropyl-2-pyrazolin-5-one (Compound No. 120).
   The title compound was obtained in the same manner as the Example 104(2).
   Yield: 65.7%(orange solid).
   ¹H-NMR(DMSO-d₆): *δ* 1.25(6H,d,J=6.9Hz), 2.04(3H,s), 2.30(3H,s), 3.16-3.29(1H,m), 6.87(1H,d,J=8.4Hz), 7.09-7.24(3H,m), 7.63(1H,s), 7.92(1H,dd,J=8.4,2.1Hz), 8.46(1H,d,J=2.1Hz), 9.39(1H,brs), 10.29(1H,brs).

### Example 121: Preparation of the compound of Compound No. 121.

The title compound was obtained in the same manner as the Example 104(2).
Yield: 15.6%(red solid).
¹H.NMR(DMSO.d₆): *δ* 1.30(6H,d,J=6.6Hz), 2.04(3H,s), 3.21-3.38(1H,m), 7.02-7.07(1H,m), 7.36(1H,t,J=8.1Hz), 7.42(1H,d,J=8.4Hz), 7.76-7.83(2H,m), 7.87-7.93(1H,m), 8.01-8.03(1H,m), 8.16-8.20(1H,m), 9.80(1H,s).

### Example 122: Preparation of the compound of Compound No. 122.

The title compound was obtained in the same manner as the Example 104(2).
Yield: 12.5%(orange solid).

The compound was obtained as a mixture of the geometrical isomers of the exomethylene in the 4-position of the pyrazolone ring. ¹H-NMR(CDCl₃): *δ* 2.21 and 2.34(total 3H,each s), 6.58-8.46(total 10H,m).

### Example 123: Preparation of the compound of Compound No. 123.

The title compound was obtained in the same manner as the Example 104(2).
Yield: 21.4%(ocherous solid).
¹H-NMR(DMSO-d₆): *δ* 2.32(3H,s), 7.40(1H,t,J=1.8Hz), 7.57(1H,s), 7.82(2H,s), 8.01-8.04(2H,m), 9.52(2H,brs), 9.84(1H,brs).

### Example 124: Preparation of the compound of Compound No. 124.

The title compound was obtained in the same manner as the Example 104(2).
Yield: 1.7%(yellow solid).
¹H-NMR(DMSO-d₆): *δ* 2.33(3H,s), 3.87(3H,s), 7.24(1H,ddd,J=8.4,2.1,0.9Hz), 7.47(1H,t,J=8.4Hz), 7.66(1H,s), 7.90-7.96(2H,m), 8.04-8.08(1H,m), 8.12-8.17(1H,m), 9.53(1H,s), 9.92(1H,s).

### Example 125: Preparation of the compound of Compound No. 125.

(1) 1-(3-Methylphenyl)-3-isopropyl-2-pyrazolin-5-one.
   The title compound was obtained in the same manner as the Example 104(1).
   Yield: 66.0%(white solid).
   ¹H-NMR(CDCl₃): *δ* 1.24(6H,d,J=7.2Hz), 2.39(3H,s), 2.70-2.90(1H,m), 3.40(2H,s), 6.97-7.02(1H,m), 7.24-7.30(1H,m), 7.65-7.71(2H,m).
(2) 4-(3,4-Dihydroxybenzylidene)-1-(3-methylphenyl)-3-isopropyl-2-pyrazolin-5-one (Compound No. 125).
   The title compound was obtained in the same manner as the Example 104(2).
   Yield: 76.5%(orange solid).
   ¹H-NMR(DMSO-d₆): *δ* 1.29(6H,d,J=6.9Hz), 2.36(3H,s), 3.20-3.35(1H,m), 6.90(1H,d,J=8.4Hz), 7.00(1H,d,J=7.5Hz), 7.31(1H,t,J=7.5Hz), 7.65(1H,s), 7.79(1H,d,J=7.5Hz), 7.80-7.82(1H,m), 7.92(1H,dd,J=8.4,1.8Hz), 8.52(1H,d,J=1.8Hz), 9.49(1H,s), 10.37(1H,s).

### Example 126: Preparation of the compound of Compound No. 126.

(1) 1-(3-Methoxyphenyl-3-isopropyl-2-pyrazolin-5-one.
   The title compound was obtained in the same manner as the Example 104(1).
   Yield: 48.0%(white solid).
   ¹H-NMR(CDCl₃): *δ* 1.25(6H,d,J=7.1Hz), 2.72-2.85(1H,m), 3.41(2H,s), 3.82(1H,s), 6.70-6.76(1H,m), 7.29(1H,t,J=8.4Hz), 7.48-7.54(2H,m).
(2) 4-(3,4-Dihydroxybenzylidene)-1-(3-methoxyphenyl-3-isopropyl-2-pyrazolin-5-one (Compound No. 126).
   The title compound was obtained in the same manner as the Example 104(2).
   Yield: 81.1%(red solid).
   ¹H-NMR(DMSO.d₆): *δ* 1.29(6H,d,J=6.6Hz), 3.19-3.35(1H,m), 3.79(3H,s), 6.77(1H,dd,J=8.4,2.4Hz), 6.91(1H,d,J=8.4Hz), 7.34(1H,t,J=8.4Hz), 7.50-7.68(3H,m), 7.93(1H,dd,J=8.4,1.5Hz), 8.49(1H,d,J=2.4Hz), 9.49(1H,s), 10.41(1H,s).

### Example 127: Preparation of the compound of Compound No. 127.

The title compound was obtained in the same manner as the Example 104(2).
Yield: 30.1%(red oil).

The compound was obtained as a mixture of the geometrical isomers of the exomethylene in the 4-position of the pyrazolone ring.
¹H-NMR(DMSO-d₆): *δ* 1.03 and 1.32(total 6H,each d,J=6.6Hz), 2.35 and 3.33(total 3H,each s), 3.09-3.21 and 3.55-3.65(total 1H,each m), 6.71-8.53(total 8H,m), 9.81(1H,brs), 9.84(1H,brs).

### Example 128: Preparation of the compound of Compound No. 128.

The title compound was obtained in the same manner as the Example 104(2).
Yield: 26.4%(red oil).

The compound was obtained as a mixture of the geometrical isomers of the exomethylene in the 4-position of the pyrazolone ring.
¹H-NMR(DMSO-ds): *δ* 1.03 and 1.32(total 6H,each d,J=6.9Hz), 3.10-3.21 and 3.51-3.65(total 1H,each m), 3.32 and 3.79(total 3H,each s), 6.71-8.51(total 8H,m), 9.86(1H,brs), 9.85(1H,brs).

### Example 129: Preparation of the compound of Compound No. 129.

(1) 1-(3,5-Dichlorophenyl)-3-phenyl-2-pyrazolin-5-one.
   The title compound was obtained in the same manner as the Example 104(1).
   Yield: 35.0%(white solid).
   ¹H-NMR(CDCl₃): *δ* 3.89(2H,s), 7.19-7.22(1H,m), 7.46-7.52(3H,m), 7.77-7.82(2H,m), 8.01-8.03(2H,m).
(2) 4-(3,4-Dihydroxybenzylidene)-1-(3,5-dichlorophenyl)-3-phenyl-2-pyrazolin-5-one (Compound No. 129).
   The title compound was obtained in the same manner as the Example 104(2).
   Yield: 71.7%(yellow solid).
   ¹H-NMR(DMSO-d₆): *δ* 6.91(1H,d,J=8.7Hz), 7.45-7.46(1H,m), 7.56-7.86(7H,m), 8.10-8.11(2H,d,J=1.8Hz), 8.42-8.52(1H,m), 9.61(1H,brs), 10.70(1H,brs).

### Example 130: Preparation of the compound of Compound No. 130.

The title compound was obtained in the same manner as the Example 104(2).
Yield: 9.3%(yellowish orange solid).
¹H-NMR(DMSO-d₆): *δ* 1.28(6H,d,J=6.9Hz), 3.24-3.38(1H,m), 7.41(1H,t,J=8.4Hz), 7.26(1H,s), 7.83(2H,s), 8.04(2H,d,J=2.1Hz), 9.49(2H,brs), 9.83(1H,brs).

### Example 131: Preparation of the compound of Compound No. 131.

The title compound was obtained in the same manner as the Example 104(2).
Yield: 39.5%(yellowish orange solid).
¹H-NMR(DMSO-d₆): *δ* 7.45-7.48(1H,m), 7.56-7.62(2H,m), 7.71-7.75(7H,m), 8.11(1H,d,J=1.8Hz), 9.56(2H,brs), 9.98(1H,brs).

### Example 132: Preparation of the compound of Compound No. 132.

The title compound was obtained in the same manner as the Example 104(2).
Yield: 44.0%(yellowish orange solid).

The compound was obtained as a mixture of the geometrical isomers of the exomethylene in the 4-position of the pyrazolone ring.
¹H-NMR(DMSO-d₆): *δ* 1.45 and 1.51(total 3H,each d,J=6.6Hz), 4.45 and 4.53(total 2H,each t,J=6.6Hz), 7.31 and 7.57(total 1H,each s), 7.47 and 7.76(total 2H,each s), 8.13-8.37(total 4H,m), 9.45-9.61(total 2H,m), 9.90(1H,brs).

### Example 133: Preparation of the compound of Compound No. 133.

The title compound was obtained in the same manner as the Example 104(2).
Yield: 43.7%(red solid).
¹H-NMR(DMSO-d₆): *δ* 1.45(9H,s), 6.89(1H,d,J=8.4Hz), 7.16-7.22(1H,m), 7.41-7.47(2H,m), 7.84(1H,s), 7.87(1H,dd,J=8.4,2.1Hz), 7.91-7.96(2H,m), 8.53(1H,d,J=2.1Hz), 9.45(1H,brs), 10.39(1H,brs)ₒ

### Example 134: Preparation of the compound of Compound No. 134.

(1) 1-(3,4-Dichlorophenyl)-3-isopropyl-2-pyrazolin-5-one.
   The title compound was obtained in the same manner as the Example 104(1).
   Yield: 38.3%(white solid).
   ¹H-NMR(CDCl₃): *δ* 1.26(6H,d,J=6.9Hz), 2.73-2.85(1H,m), 3.44(2H,s), 7.43(1H,d,J=9.0Hz), 7.85(1H,dd,J=9.0,2.7Hz), 8.08(1H,d,J=2.7Hz).
(2) 4-(3,4,5-Trihydroxybenzylidene)-1-(3,4-dichlorophenyl)-3-isopropyl-2-pyrazolin-5-one (Compound No. 134).
   The title compound was obtained in the same manner as the Example 104(2).
   Yield: 13.2%(orange solid).
   ¹H-NMR(DMSO-d₆): *δ* 1.28(6H,d,J=6.6Hz), 3.21-3.38(1H,m), 7.61(1H,s), 7.71(1H,d,J=9.0Hz),7.84(2H,s), 7.94(1H,dd,J=9.0,2.4Hz), 8.31(1H,d,J=2.4Hz), 9.49(2H,s), 9.80(1H,s).

### Example 135: Preparation of the compound of Compound No. 135.

The title compound was obtained in the same manner as the Example 104(2).
Yield: 25.1%(orange solid).
¹H-NMR(DMSO-d₆): *δ* 2.35(3H,s), 7.51(1H,s), 7.82(2H,s), 8.23-8.37(4H,m), 9.53(2H,s), 9.86(1H,s).

### Example 136: Preparation of the compound of Compound No. 136.

The title compound was obtained in the same manner as the Example 104(2).
Yield: 20.1%(red solid).
¹H-NMR(DMSO-d₆): *δ* 6.41(1H,d,J=8.4Hz), 7.43(1H,t,J=2.1Hz), 7.54-7.70(1H,m), 8.14(1H,d,J=1.8Hz), 8.17(1H,s), 8.93(1H,s), 9.08(1H,brs), 10.53(1H,s), 10.87(1H,brs).

### Example 137: Preparation of the compound of Compound No. 137.

The title compound was obtained in the same manner as the Example 104(2).
Yield: 2.3%(light brown solid).
¹H-NMR(DMSO-d₆):δ 6.97(1H,d, J=8.1Hz), 7.76(1H,s), 7.80(1H,t, J=8.4Hz), 7.98(1H,dd, J=8.4,1.5Hz), 8.12(1H,dd, J=8.1,2.1Hz), 8.29(1H,dd, J=8.4,2.1Hz), 8.59-8.66(1H,m), 8.84(1H,t, J=2.1Hz), 9.84(2H,brs).

### Example 138: Preparation of the compound of Compound No. 138.

(1) 1-(3,5-Dichlorophenyl)-3-(tert-butyl)-2-pyrazolin-5-one.
   The title compound was obtained in the same manner as the Example 104(1).
   Yield: 45.1%(white solid).
   ¹H-NMR(CDCl₃): *δ* 1.28(9H,s), 3.47(2H,s), 3.82(1H,s), 7.15(1H,t,J=1.9Hz), 7.92(2H,d,J=1.9Hz).
(2) 4-(3,4-Dihydroxybenzylidene)-1-(3,5-dichlorophenyl)-3-(tert-butyl)-2-pyrazolin-5-one (Compound No. 138).

The title compound was obtained in the same manner as the Example 104(2).
Yield: 20.1%(yellowish orange solid).
¹H-NMR(DMSO-d₆): *δ* 1.45(9H,s), 6.90(1H,d,J=8.4Hz), 7.42(1H,t,J=1.8Hz), 7.86(1H,dd,J=8.4,2.1Hz), 7.89(1H,s), 8.02(2H,d,J=1.8Hz), 8.52(1H,d,J=2.1Hz), 9.52(1H,brs), 10.51(1H,brs).

### Example 139: Preparation of the compound of Compound No. 139.

The title compound was obtained in the same manner as the Example 104(2).
Yield: 19.3%(yellow solid).
¹H-NMR(DMSO-d₆): *δ* 1.44(9H,s), 7.40-7.42(1H,m), 7.78(1H,s), 7.81(2H,s), 8.02-8.04(2H,m), 9.45(2H,brs), 9.83(1H,brs).

### Example 140: Preparation of the compound of Compound No. 140.

The title compound was obtained in the same manner as the Example 104(2).
Yield: 38.3%(orange solid).
¹H.NMR(DMSO.d₆): *δ* 1.29(1H,d,J=6.7Hz), 3.21-3.40(1H,m), 6.91(1H,d,J=8.1Hz), 7.69-7.73(2H,m), 7.90-7.97(2H,m), 8.28(1H,d,J=2.4Hz), 8.49(1H,d,J=2.1Hz), 9.56(1H,brs), 10.50(1H,brs).

### Example 141: Preparation of the compound of Compound No. 141.

The title compound was obtained in the same manner as the Example 104(2).
Yield: 2.9%(orange solid).
¹H-NMR(DMSO-d₆): *δ* 1.30(6H,d,J=6.9Hz), 3.04-3.16(1H,m), 6.46(1H,s), 7.69(1H,d,J=8.7Hz), 7.96(1H,dd,J=8.7,2.4Hz), 8.08(1H,s), 8.32(1H,d,J=2.4Hz), 8.89(1H,s), 8.96(1H,brs), 10.50(1H,brs), 10.69(1H,brs)).

### Example 142: Preparation of the compound of Compound No. 142.

The title compound was obtained in the same manner as the Example 104(2).
Yield: 12.7%(reddish brown solid).
¹H-NMR(DMSO-d₆): *δ* 1.30(6H,d,J=6.9Hz), 3.10-21(1H,m), 6.51(1H,d,J=9.0Hz), 7.40(1H,t,J=1.5Hz), 8.04(2H,d,J=1.5Hz), 8.14(1H,s), 8.86(1H,d,J=9.OHz), 9.87(1H,brs), 10.08(1H,brs), 10.80(1H,brs).

### Example 143: Preparation of the compound of Compound No. 143.

The title compound was obtained in the same manner as the Example 104(2).
Yield: 21.4%(ocherous solid).
¹H-NMR(DMSO-d₆): *δ* 2.32(3H,s), 7.40(1H,t,J=1.8Hz), 7.57(1H,s), 7.82(2H,s), 8.01-8.04(2H,m), 9.52(2H,brs), 9.84(1H,brs).

### Example 144: Preparation of the compound of Compound No. 144.

(1) 1-(3,4-Dichlorophenyl)-3-methyl-2-pyrazolin-5-one.
   The title compound was obtained in the same manner as the Example 104(1).
   Yield: 72.4%(white solid).
   ¹H-NMR(CDCl₃): *δ* 2.21(3H,s), 3.45(2H,s), 7.44(1H,d,J=8.4Hz), 7.83(1H,dd,J=8.4,2.4Hz), 8.07(1H,d,J=2.4Hz).
(2) 4-(3,4,5-Trihydroxybenzylidene)-1-(3,4-dichlorophenyl)-3-methyl-2-pyrazolin-5-one (Compound No. 144).
   The title compound was obtained in the same manner as the Example 104(2).
   Yield: 10.7%(orange solid).
   ¹H-NMR(DMSO-d₆): *δ* 2.32(3H,m), 7.55(1H,s), 7.69(1H,d,J=9.0Hz), 7.82(2H,s), 7.92(1H,dd,J=9.0,2.4Hz), 8.28(1H,d,J=2.4Hz), 9.50(2H,s), 9.80(1H,s).

### Example 145: Preparation of the compound of Compound No. 145.

(1) 1-(3,4-Dichlorophenyl)-3-phenyl-2-pyrazolin-5-one.
   The title compound was obtained in the same manner as the Example 104(1).
   Yield: 41.3%(white solid).
   ¹H-NMR(DMSO-d₆): *δ* 6.07(1H,s), 7.33-7.47(3H,m), 7.75(1H,d,J=8.7Hz), 7.83-7.87(2H,m), 7.91(1H,dd,J=8.7,2.4Hz), 8.12(1H,d,J=2.4Hz), 12.20(1H,brs).
(2) 4-(3,4-Dihydroxybenzylidene)-1-(3,4-dichlorophenyl)-3-phenyl-2-pyrazolin-5-one (Compound No. 145).

The title compound was obtained in the same manner as the Example 104(2).
Yield: 43.7%(orange solid).
¹H-NMR(DMSO-ds): *δ* 6.90(1H,d,J=9.0Hz), 7.57-7.60(4H,m), 7.70-7.76(3H,m), 7.81(1H,dd,J=9.0,1.8Hz), 8.02(1H,dd,J=9.0,2.4Hz), 8.34(1H,d,J=2.4Hz), 8.48(1H,d,J=1.8Hz), 9.62(1H,brs), 10.64(1H,brs).

### Example 146: Preparation of the compound of Compound No. 146.

The title compound was obtained in the same manner as the Example 104(2).
Yield: 6.9%(yellow solid).
¹H-NMR(DMSO-d₆): *δ* 7.47(1H,s), 7.56-7.62(3H,m), 7.68-7.78(5H,m), 8.01(1H,dd,J=9.0,2.7Hz), 8.36(1H,d,J=2.7Hz), 9.55(2H,brs), 9.96(1H,brs).

### Example 147: Preparation of the compound of Compound No. 147.

(1) 1-(3,5-Dichlorophenyl)-3-trifluoromethyl-2-pyrazolin-5-one.
   The title compound was obtained in the same manner as the Example 104(1).
   Yield: 77.3%(white solid).
   ¹H-NMR(DMSO-d₆): *δ* 5.98(1H,s), 7.66(1H,t,J=1.8Hz), 7.82(2H,d,J=1.8Hz).
(2) 4-(3,4-Dihydroxybenzylidene)-1-(3,5-dichlorophenyl)-3-trifluoromethyl- . 2-pyrazolin-5-one (Compound No. 147).
   The title compound was obtained in the same manner as the Example 104(2).
   Yield: 1.3%(brown solid).
   ¹H-NMR(CD₃OD): *δ* 6.93(1H,d,J=8.4Hz), 7.27-7.32(1H,m), 7.65-7.73(1H,m), 7.85-7.92(1H,m), 7.99-8.05(2H,m), 8.50-8.61(1H,m).

### Example 148: Preparation of the compound of Compound No. 148.

The title compound was obtained in the same manner as the Example 104(2).
Yield: 2.1%(brown solid).
¹H-NMR(DMSO-d₆): *δ* 6.40-6.52(1H,m), 7.52-7.69(5H,m), 7.72(1H,d,J=9.0Hz), 8.04(1H,dd,J=9.0,2.4Hz), 8.19(1H,s), 8.35(1H,d,J=2.4Hz), 9.00(1H,brs).

### Example 149: Preparation of the compound of Compound No. 149.

The title compound was obtained in the same manner as the Example 104(2).
Yield: 6.8%(ocherous solid).

The compound was obtained as a mixture of the geometrical isomers of the exomethylene in the 4-position of the pyrazolone ring.
¹H-NMR(DMSO-d₆): *δ* 6.84 and 7.58(total 1H,each s), 7.49 and 7.55(total 1H,each t,J=1.8Hz), 7.89(2H,brs), 7.98 and 8.01(total 2H,each d,J=1.8Hz), 9.45 and 9.67(total 3H,each s).

### Example 150: Preparation of the compound of Compound No. 150.

The title compound was obtained in the same manner as the Example 104(2).
Yield: 8.2%(yellow solid).

The compound was obtained as a mixture of the geometrical isomers of the exomethylene in the 4-position of the pyrazolone ring.
¹H-NMR(DMSO-d₆): *δ* 5.49 and 8.50(total 1H,each s), 6.15 and 6.77(total 1H,each d,J=8.7Hz), 6.57 and 9.01(total 1H,each d,J=9.3Hz), 7.46 and 7.54(total 1H,each t,J=2.1Hz,J=1.8Hz), 7.97 and 7.99(total 2H,each d,J=2.1Hz,J=1.8Hz).

### Example 151: Preparation of the compound of Compound No. 151.

The title compound was obtained in the same manner as the Example 104(2).
Yield: 8.2%(ocherous solid).

The compound was obtained as a mixture of the geometrical isomers of the exomethylene in the 4-position of the pyrazolone ring.
¹H-NMR(DMSO-d₆): *δ* 5.44 and 6.47(total 1H, each s), 6.13 and 8.26(total 1H, each s), 6.85 and 8.97(total 1H,each s), 7.46 and 7.53(total 1H,each t,J=1.8Hz), 7.83 and 8.00(total 2H,each d,J=1.8Hz), 11.21(1H,brs).

### Example 152: Preparation of the compound of Compound No. 152.

The title compound was obtained in the same manner as the Example 104(2).
Yield: 60.3%(yellow solid).
¹H-NMR(DMSO-d₆): *δ* 2.32(3H,s), 6.29(1H,d,J=8.1Hz), 7.65(1H,s), 7.69(1H,d,J=9.0Hz), 7.88-7.96(2H,m), 8.27(1H,d,J=2.4Hz), 8.47(1H,d,J=2.1Hz), 9.58(1H,s), 10.52(1H,s).

### Example 153: Preparation of the compound of Compound No. 153.

(1) 1-(3-Carboxyphenyl)-3-phenyl-2-pyrazolin-5-one.
   The title compound was obtained in the same manner as the Example 104(1).
   Yield: 38.1%(white solid).
   ¹H-NMR(DMSO-ds): *δ* 6.05(1H,s), 7.31-7.47(3H,m), 7.61(1H,t,J=8.1Hz), 7.82-7.88(3H,m), 8.08-8.13(1H,m), 8.42-8.44(1H,m), 12.04(1H,s), 13.20(1H,s).
(2) 4-(3,4-Dihydroxybenzylidene)-1-(3-carboxyphenyl)-3-phenyl-2-pyrazolin-5-one (Compound No. 153).
   The title compound was obtained in the same manner as the Example 104(2).
   Yield: 76.3%(orange solid).
   ¹H-NMR(DMSO-d₆) : *δ* 6.90(1H,d,J=9.0Hz), 7.56-7.64(5H,m), 7.70-7.74(2H,m), 7.77-7.83(2H,m), 8.24(1H,ddd,J=8.1,2.4,1.8Hz), 8.53(1H,d,J=1.8Hz), 8.68(1H,t,J=1.8Hz), 9.63(1H,s), 10.56(1H,s), 13.09(1H,brs).

### Example 154: Preparation of the compound of Compound No. 154.

(1) 1-[3-(Methoxycarbonyl)phenyl]-3-phenyl-2-pyrazolin-5-one.
   The title compound was obtained in the same manner as the Example 108(2).
   Yield: 76.1%(white solid).
   ¹H-NMR(DMSO-d₆): *δ* 3.91(3H,s), 6.06(1H,s), 7.31-7.47(3H,m), 7.65(1H,t,J=8.1Hz), 7.82-7.89(3H,m), 8.14(1H,ddd,J=8.1,2.1,0.9Hz), 8.43-8.45(1H,m), 12.09(1H,s).
(2) 4-(3,4-Dihydroxybenzylidene)-1-[3-(methoxycarbonyl)phenyl]-3-phenyl-2-pyrazolin-5-one (Compound No. 154).
   The title compound was obtained in the same manner as the Example 104(2).
   Yield: 2.4%(brown solid).
   ¹H-NMR(DMSO-d₆): *δ* 3.90(1H,s), 6.91(1H,d,J=8.1Hz), 7.58-7.85(9H,m), 8.28(1H,ddd,J=8.1,2.1,1.2Hz), 8.51(1H,d,J=1.5Hz), 8.69(1H,t,J=2.1Hz), 9.63(1H,s), 10.60(1H,s).

### Example 155: Preparation of the compound of Compound No. 155.

The title compound was obtained in the same manner as the Example 104(2).
Yield: 32.4%(yellow solid).

The compound was obtained as a mixture of the geometrical isomers of the exomethylene in the 4-position of the pyrazolone ring.
¹H-NMR(DMSO·d₆): *δ* 2.48-2.53(total 3H,m), 4.39-4.52(total 2H,m), 6.48 and 6.52(total 1H,each d,J=9.0Hz), 7.96 and 8.88(total 1H,each d,J=9.0Hz), 8.13-8.34(total 5H,m), 8.81-9.01(total 1H,m), 10.00-10.35(total 1H,m), 10.69-10.97(total 1H,m).

### Example 156: Preparation of the compound of Compound No. 156.

The title compound was obtained in the same manner as the Example 104(2).
Yield: 9.9%(red solid).

The compound was obtained as a mixture of the geometrical isomers of the exomethylene in the 4-position of the pyrazolone ring.
¹H-NMR(DMSO-d₆): *δ* 1.43 and 1.50(total 3H,each d,J=7.2Hz), 4.43 and 4.51(total 2H,each t,J=7.2Hz), 6.45 and 6.46(total 1H,each s), 7.93 and 8.07(total 1H,each s), 8.15-8.34(total 4H,m), 8.81-9.08(total 2H,m), 10.59-10.88(total 2H,m).

### Example 157: Preparation of the compound of Compound No. 157.

The title compound was obtained in the same manner as the Example 104(2).
Yield: 3.4%(light brown solid).
¹H-NMR(DMSO-d₆): *δ* 1.28(6H,d,J=6.6Hz), 2.35(3H,s), 3.18-3.34(1H,m), 6.99-7.02(1H,m), 7.31(1H,t,J=7.8Hz), 7.54(1H,s), 7.71-7.74(1H,m), 7.80-7.84(3H,m), 9.44(2H,brs), 9.78(1H,brs).

### Example 158: Preparation of the compound of Compound No. 158.

The title compound was obtained in the same manner as the Example 104(2).
Yield: 12.1%(light orange solid).
¹H.NMR(DMSO.d₆): *δ* 2.27(3H,s), 6.44(1H,s), 7.18-7.23(1H,m), 7.42(1H,t,J=8.4Hz), 7.89-7.93(1H,m), 7.96(1H,s), 8.11-8.13(1H,m), 8.92(1H,s), 8.95(1H,brs), 10.51(1H,brs), 10.67(1H,brs).

### Example 159: The compound of Compound No. 159.

This compound is a commercially available compound.
Supplier: Specs (Netherlands).
Catalog code number: AK-968/12383334.

### Example 160: The compound of Compound No. 160.

This compound is a commercially available compound.
Supplier: Specs (Netherlands).
Catalog code number: AN-023/13438049.

### Example 161: The compound of Compound No. 161.

This compound is a commercially available compound.
Supplier: Specs (Netherlands).
Catalog code number: AN-465/14458024.

### Example 162: The compound of Compound No. 162.

This compound is a commercially available compound.
Supplier: Specs (Netherlands).
Catalog code number: AN-648/14238003.

### Example 163: The compound of Compound No. 163.

This compound is a commercially available compound.
Supplier: Specs (Netherlands).
Catalog code number: AN-989/13874013.

### Example 164: The compound of Compound No. 164.

This compound is a commercially available compound.
Supplier: LaboTest (Germany).
Catalog code number: LT00444397.

### Example 165: The compound of Compound No. 165.

This compound is a commercially available compound.
Supplier: LaboTest (Germany).
Catalog code number: LT00431228.

### Example 166: Preparation of the compound of Compound No. 166.

(1) 5-(3,5-Dichlorophenyl)-2-furaldehyde.
   A mixture of 5-bromo-2-furaldehyde(2.63 g, 15.0 mmol), 3,5-dichlorophenylboronic acid(315 mg, 16.5 mmol), tetrabutylammonium bromide(4.84 g, 15 mmol), palladium acetate(67 mg, 0.3 mmol), potassium carbonate(5.18 g, 37.5 mmol) and water(20 ml) was stirred at room temperature for 2 hours. The reaction mixture was extracted with ethyl acetate. The organic layer was washed with brine and dried over anhydrous sodium sulfate. The residue obtained by evaporation of the solvent under reduced pressure was purified by column chromatography on silica gel(hexane:ethyl acetate=4:1) to give the title compound(1.01 g, 27.9%) as a white solid.
   ¹H-NMR(CDCl₃): *δ* 6.89(1H,d,J=3.9Hz), 7.33(1H,d,J=3.9Hz), 7.36(1H,t,J=1.8Hz), 7.68(2H,d,J=1.8Hz), 9.69(1H,s).
(2) 1-(3-Carboxyphenyl)-3-trifluoromethyl-2-pyrazolin-5-one.
   The title compound was obtained in the same manner as the Example 104(1).
   Yield: 62.2%(light orange solid).
   ¹H-NMR(DMSO-d₆):*δ* 5.97(1H,s), 7.65(1H,t,J=8.1Hz), 7.93(1H,dt,J=7.8, 1.2Hz), 8.01(1H,ddd,J=8.1,2.1,1.2Hz), 8.29(1H,t,J=1.8Hz), 12.70(1H,s), 13:24(1H,brs).
(3) 1-[3-(Methoxycarbonyl)phenyl]-3-trifluoromethyl-2-pyrazolin-5-one.
   The title compound was obtained in the same manner as the Example 108(2).
   Yield: 93.1%(light yellowish white crystal).
   ¹H-NMR(CDCl₃): *δ* 3.90(3H,s), 5.97(1H,s), 7.68(1H,t,J=8.1Hz), 7.93-7.97(1H,m), 8.05(1H,ddd,J=8.1,2.4,1.2Hz), 8.30(1H,t,J=2.4Hz), 12.76(1H,s).
(4) 4-{[5-(3,5-Dichlorophenyl)furan-2-yl]methylene}-1-[3-(methoxycarbonyl)phenyl]-3-trifluoromethyl-2-pyrazolin-5-one (Compound No. 166).
   The title compound was obtained in the same manner as the Example 104(2).
   Yield: 90.8%(red solid).
   The compound was obtained as a mixture of the geometrical isomers of the exomethylene in the 4-position of the pyrazolone ring (isomeric ratio=ca. 1:0.7).
   ¹H-NMR(CDCl₃): *δ* 3.96(3H,s), 3.96(2.1H,s), 6.76(1H,dd,J=3.9,0.6Hz), 7.12(0.7H,dd,J=3.9,0.6Hz), 7.37(0.7H,t,J=1.8Hz), 7.54(1H,t,J=8.4Hz), 7.55(0.7H,t,J=8.1Hz), 7.63(1H,s), 7.73(0.7H,s), 7.75(2H,d,J=1.8Hz), 7.95(1H,dd,J=2.7,1.5Hz), 7.97(0.7H,dd,J=3.0,1.8Hz), 8.17(0.7H,td,J=8.4,1.2Hz), 8.17(1H,td,J=8.4,1.2Hz), 8.60(1H,t,1.8Hz), 8.63(0.7H,t,J=1.8Hz), 8.90(1H,d,J=3.9Hz), 9.03(0.7H,d,J=4.2Hz).

### Example 167: Preparation of the compound of Compound No. 167.

(1) 1-(3-Nitrophenyl)-3-phenyl-2-pyrazolin-5-one.
   The title compound was obtained in the same manner as the Example 104(1).
   Yield: 70.5%(white solid).
   ¹H-NMR(DMSO-ds): *δ* 7.33-7.48(3H,m), 7.79(1H,t,J=8.1Hz), 7.84-7.89(2H,m), 8.10-8.15(1H,m), 8.34-8.38(1H,m), 8.71(1H,t,J=2.1Hz).
(2) 4-{[5-(3,5-Dichlorophenyl)furan-2-yl]methylene}-1-(3-nitrophenyl)-3-phenyl-2-pyrazolin-5-one (Compound No. 167).
   The title compound was obtained in the same manner as the Example 104(2).
   Yield: 2.8%(red solid).
   The compound was obtained as a mixture of the geometrical isomers of the exomethylene in the 4-position of the pyrazolone ring.
   ¹H-NMR(CDCl₃): *δ* 5.43 and 5.57 (total 1H,each s), 6.40-6.67(total 1H,m), 6.51 and 6.66(total 1H,each d,J=3.3Hz), 7.09-8.01(total 10H,m), 8.43-8.55(total 1H,m), 8.90-9.07(total 1H,m).

### Example 168: The compound of Compound No. 168.

This compound is a commercially available compound.
Supplier: LaboTest (Germany).
Catalog code number: LT00098018.

### Example 169: The compound of Compound No. 169.

This compound is a commercially available compound.
Supplier: Specs (Netherlands).
Catalog code number: AG-205/11866161.

### Example 170: The compound of Compound No. 170.

This compound is a commercially available compound.
Supplier: Specs (Netherlands).
Catalog code number: AG-205/11866174.

### Example 171: The compound of Compound No. 171.

This compound is a commercially available compound.
Supplier: Specs (Netherlands).
Catalog code number: AG-690/11425020.

### Example 172: The compound of Compound No. 172.

This compound is a commercially available compound.
Supplier: Specs (Netherlands).
Catalog code number: AK-968/12572074.

### Example 173: The compound of Compound No. 173.

This compound is a commercially available compound.
Supplier: Specs (Netherlands).
Catalog code number: AK-968/12971206.

### Example 174: The compound of Compound No. 174.

This compound is a commercially available compound.
Supplier: Specs (Netherlands).
Catalog code number: AK-968/15252993.

### Example 175: The compound of Compound No. 175.

This compound is a commercially available compound.
Supplier: Specs (Netherlands).
Catalog code number: AN-655/40760174.

### Example 176: Preparation of the compound of Compound No. 176.

2N Aqueous sodium hydroxide(0.5 ml) was added to a solution of 4-{[5-(3,5-dichlorophenyl)furan-2-yl]methylene}-1-[3-(methoxycarbonyl)phenyl]-3-trifl uoromethyl-2-pyrazolin-5-one (Compound No. 166; 110.2 mg, 0.21 mmol) in a mixed solvent of tetrahydrofuran/ethanol(4 ml + 2 ml), and the mixture was stirred at room temperature for 2 hours. The reaction mixture was poured into 2N hydrochloric acid and extracted with ethyl acetate. The organic layer was washed with brine and dried over anhydrous sodium sulfate. The residue obtained by evaporation of the solvent under reduced pressure was washed with isopropyl ether/hexane under suspension to give the title compound(89.9 mg, 84.0%) as a dark orange powder.

The compound was obtained as a mixture of the geometrical isomers of the exomethylene in the 4-position of the pyrazolone ring.
¹H.NMR(DMSO.d₆): *δ* 7.21(1H,d,J=3.9Hz), 7.61-7.68(total 3H,m), 7.74-7.75(total 2H,m), 7.79(1H,d, J=3.9Hz), 7.86-7.91(total 4H,m), 8.06-8.13(total 3H,m), 8.16(2H,d,J=1.8Hz), 8.44(1H,m), 8.49(1H,m), 8.71(1H,d, J=3.9Hz), 8.75-8.78(1H,m), 13.14(total_2H,brs).

### Example 177: Preparation of the compound of Compound No. 177.

(1) 5-[3,5-Bis(trifluoromethyl)phenyl]-2-furaldehyde.
   The title compound was obtained in the same manner as the Example 166(1).
   Yield: 58.0%(light brown solid).
   ¹H-NMR(CDCl₃): *δ* 7.04(1H,d,J=3.9Hz), 7.38(1H,d,J=3.9Hz), 7.89(1H,s), 8.23(2H,s), 9.74(1H,s).
(2) 4-({5-[3,5-Bis(trifluoromethyl)phenyl]furan-2-yl}methylene)-1-(3-methoxycarbonylphenyl)-3-trifluoromethyl-2-pyrazolin-5-one (Compound No. 177).
   The title compound was obtained in the same manner as the Example 104(2).
   Yield: 51.5%(red solid).
   ¹H-NMR(CDCl₃): *δ* 3.97(3H,s), 7.26(1H,d,J=3.9Hz), 7.55(1H,t,J=8.1Hz), 7.77(1H,s), 7.92(1H,s), 7.97(1H,dt,J=8.1,1.2Hz), 8.17(1H,ddd,J=8.1,2.4, 1.2Hz), 8.28(2H,s), 8.63(1H,t,J=2.4Hz), 9.03(1H,d,J=3.9Hz).

### Example 178: Preparation of the compound of Compound No. 178.

The title compound was obtained in the same manner as the Example 104(2).
Yield: 6%(red solid).
¹H-NMR(CDCl₃): *δ* 2.33(6H,s), 2.74(2H,t,J=6.6Hz), 3.38(2H,t,J=6.6Hz), 3.96(3H,s), 7.13(1H,d,J=3.9Hz), 7.S3(1H,t,J=8.1Hz), 7.80(1H,s), 7.84(1H,d,J=3.9Hz), 7 .94(1H,dt,J=8.1, 1.2Hz), 8.20(1H,ddd,J=8.1,2.1,1.2Hz), 8.62(1H,t,J=1.8Hz).

### Example 179: Preparation of the compound of Compound No. 179.

The title compound was obtained in the same manner as the Example 176.
Yield: 84.5%(red powder).
¹H-NMR(DMSO-d₆): *δ* 7.64(1H,t,J=8.1Hz), 7.85-7.89(1H,m), 7.96-7.98(1H,m), 7.99(1H,d,J=3.9Hz), 8.11(1H,ddd,J=8.1,2.4,1.2Hz), 8.21(1H,s), 8.50(1H,t,J=1.8Hz), 8.73(2H,s), 8.77(1H,d,J=3.9Hz), 13.18(1H,brs).

### Example 180: Preparation of the compound of Compound No. 180.

The title compound was obtained in the same manner as the Example 104(2).
Yield: 38.4%(brown solid).
¹H-NMR(CDCl₃): *δ* 3.95(3H,s), 7.04-7.06(1H,m), 7.60(1H,t,J=1.8Hz), 7.50-7.63(5H,m), 7.66(2H,d,J=1.8Hz), 7.69-7.74(2H,m), 7.91(1H,dt,J=8.1,1.2Hz), 8.33(1H ddd,J=8.1,2.1,1.2Hz), 8.73(1H,t,J=1.8Hz), 8.94(1H,d,J=3.6Hz).

### Example 181: Preparation of the compound of Compound No. 181.

The title compound was obtained in the same manner as the Example 104(2).
Yield: 31.3%(red powder).
¹H-NMR(CDCl₃): *δ* 3.97(3H,s), 7.41(1H,d,J=2.1Hz), 7.53-7.58(2H,m), 7.67(2H,d,J=1.5Hz), 7.94(1H,s), 7.97(1H,dt,J=7.5,1.2Hz), 8.00(1H,d,J=4.2Hz), 8.21(1H,ddd,J=8.1,2.1,1.2Hz), 8.60(1H,d,J=2.1Hz).

### Example 182: Preparation of the compound of Compound No. 182.

The title compound was obtained in the same manner as the Example 104(2).
Yield: 2.8%(red solid).

The compound was obtained as a mixture of the geometrical isomers of the exomethylene in the 4-position of the pyrazolone ring.
¹H-NMR(CDCl₃): *δ* 6.78-7.43(total 4H,m), 7.38 and 7.44(total 1H,each t,J=2.1Hz), 7.60-7.78(total 5H,m).

### Example 183: Preparation of the compound of Compound No. 183.

The title compound was obtained in the same manner as the Example 104(2).
Yield: 4.1%(red solid).

The compound was obtained as a mixture of the geometrical isomers of the exomethylene in the 4-position of the pyrazolone ring.
¹H-NMR(CDCl₃): *δ* 2.21 and 2.34(total 6H,each s), 2.68 and 2.74(total 2H,each d,J=7.2Hz), 3.14 and 3.31(total 2H,each d,J=7.2Hz), 3.91 and 3.95(total 3H,each s), 6.58-8.46(total 10H,m).

### Example 184: Preparation of the compound of Compound No. 184.

The title compound was obtained in the same manner as the Example 176.
Yield: 43.5%(red powder).

The compound was obtained as a mixture of the geometrical isomers of the exomethylene in the 4-position of the pyrazolone ring.
¹H-NMR(DMSO-d₆): *δ* 7.65-7.68(total 3H,m), 7.75(1H,t,J=1.BHz), 7.87-7.90(total 2H,m), 7.96(2H,d,J=1.8Hz), 8.07(total 3H,m), 8.29(1H,d,J=4.5Hz), 8.37-8.39(total 2H,m), 8.46-8.50(total 3H,m), 13.19(total 2H,brs).

### Example 185: Preparation of the compound of Compound No. 185.

The title compound was obtained in the same manner as the Example 104(2).
Yield: 59.4%(cinnabar red powder).
¹H-NMR(CDCl₃): *δ* 1.43(3H,t,J=7.2Hz), 1.50(3H,t,J=7.2Hz), 4.42(2H,q,J=7.2Hz), 4.52(2H,q,J=7.2Hz), 7.09(1H,dd,J=3.9,0.6Hz), 7.40(1H,t,J=8.1Hz), 7.54(1H,t,J=8.1Hz), 7.73(2H,d,J=2.1Hz), 7.95-7.99(1H,m), 8.18(1H,ddd,J=8.1,2.1,1.2Hz), 8.63(1H,t,J=1.BHz), 8.65(1H,s), 8.96(1H,d,J=3.9Hz).

### Example 186: The compound of Compound No. 186.

This compound is a commercially available compound.
Supplier: LaboTest (Germany).
Catalog code number: LT00624783.

### Example 187: The compound of Compound No. 187.

This compound is a commercially available compound.
Supplier: LaboTest (Germany).
Catalog code number: LT00631797.

### Example 188: The compound of Compound No. 188.

This compound is a commercially available compound.
Supplier: Specs (Netherlands).
Catalog code number: AG-205/37169034.

### Example 189: The compound of Compound No. 189.

This compound is a commercially available compound.
Supplier: Specs (Netherlands).
Catalog code number: AN-023/13436014.

### Example 190: The compound of Compound No. 190.

This compound is a commercially available compound.
Supplier: Specs (Netherlands).
Catalog code number: AN-465/15281231.

### Example 191: The compound of Compound No. 191.

This compound is a commercially available compound.
Supplier: Specs (Netherlands).
Catalog code number: AN-648/14238002.

### Example 192: The compound of Compound No. 192.

This compound is a commercially available compound.
Supplier: Specs (Netherlands).
Catalog code number: AN-648/15102026.

### Example 193: The compound of Compound No. 193.

This compound is a commercially available compound.
Supplier: Specs (Netherlands).
Catalog code number: AN-989/40821683.

### Example 194: Preparation of the compound of Compound No. 194.

The title compound was obtained in the same manner as the Example 104(2).
Yield: 67.1%(yellow solid).
¹H-NMR(DMSO-d₆): *δ* 3.90(3H,s), 7.12-7.14(1H,m), 7.56(1H,s), 7.59-7.68(4H,m), 7.74-7.86(3H,m), 8.28(1H,ddd,J=8.1,2.4,0.9Hz), 8.63(1H,t,J=2.4Hz), 8.66(1H,d,J=3.9Hz).

### Example 195: Preparation of the compound of Compound No. 195.

The title compound was obtained in the same manner as the Example 104(2).
Yield: 16.5%(orange solid).
¹H-NMR(CDCl₃):δ 6.79(1H,d,J=3.9Hz), 7.63(1H,t,J=8.4Hz), 7.66(1H,s), 8.09-8.14(1H,m), 8.33-8.37(1H,m), 8.90(1H,d, J=3.9Hz), 8.94(1H,t, J=2.4Hz).

### Example 196: Preparation of the compound of Compound No. 196.

The title compound was obtained in the same manner as the Example 104(2).
Yield: 18.7%(orange solid).
¹H-NMR(CDCl₃): *δ* 1.42(3H,t,J=7.2Hz), 1.48(3H,t,J=7.2Hz), 4.41(2H,q,J=7.2Hz), 4.48(2H,q,J=7.2Hz), 6.13(2H,s), 6.96(1H,d,J=8.1Hz), 7.52(1H,t,J=8.1Hz), 7.85-7.89(1H,m), 7.94-7.98(1H,m), 8.16(1H,ddd,J=8.1,2.1,1.8Hz), 8.59(1H,t,J=1.8Hz), 8.68-8.70(2H,m).

### Example 197: Preparation of the compound of Compound No. 197.

The title compound was obtained in the same manner as the Example 104(2).
Yield: 76.1%(orange powder).
¹H-NMR(CDCl₃): *δ* 1.50(9H,s), 3.94(3H,s), 7.49(1H,t,J=8.1Hz), 7.56(1H,d,J=8.1Hz), 7.71(1H,s), 7.87(1H,dt,J=8.1,1.2Hz), 8.20(1H,ddd,J=8.1,2.1,1.2Hz), 8.23(1H,dd,J=8.4,2.1Hz), 8.43(1H,d,J=2.4Hz), 8.60(1H,t,J=2.1Hz).

### Example 198: Preparation of the compound of Compound No. 198.

The title compound was obtained in the same manner as the Example 176.
Yield: 62.1%(orange powder).
¹H-NMR(DMSO-d₆): *δ* 1.46(9H,s), 7.58(1H,t,J=8.1Hz), 7.77-7.83(2H,m), 8.10(1H,s), 8.09-8.13(1H,m), 8.43(1H,dd,J=8.7,2.1Hz), 8.52(1H,t,J=2.1Hz), 8.80(1H,d,J=2.1Hz), 13.01(1H,brs).

### Example 199: Preparation of the compound of Compound No. 199.

The title compound was obtained in the same manner as the Example 104(2).
Yield: 62.0%(red solid).
¹H-NMR(DMSO-d₆): *δ* 7.14-7.16(1H,m), 7.60-7.63(4H,m), 7.77-7.83(3H,m), 8.09-8.13(1H,m), 8.44-8.47(1H,m), 8.67(1H,d,J=3.9Hz), 8.90(1H,t,J=2.1Hz).

### Example 200: Preparation of the compound of Compound No. 200.

(1) 1-(3-Nitrophenyl)-3-isopropyl-2-pyrazolin-5-one.
   The title compound was obtained in the same manner as the Example 104(1).
   Yield: 79.8%(white solid).
   ¹H-NMR(DMSO-d₆): *δ* 1.19-1.23(6H,m), 2.77-2.85(1H,m), 5.48(1H,s), 7.71-7.77(1H,m), 8.02-8.08(1H,m), 8.20-8.95(1H,brs), 8.23-8.27(1H,m), 8.61-8.62(1H,m).
(2) 4-[(5-Bromofuran-2-yl)methylene]-1-(3-nitrophenyl)-3-isopropyl-2-pyrazolin-5-one (Compound No. 200).
   The title compound was obtained in the same manner as the Example 104(2).
   Yield: 42.6%(orange solid).
   ¹H-NMR(DMSO-d₆): *δ* 1.31(6H,d,J=6.9Hz), 3.21-3.35(1H,m), 7.11(1H,d,J=3.9Hz), 7.76(1H,t,J=8.1Hz), 7.84(1H,s), 8.02-8.08(1H,m), 8.33-8.39(1H,m), 8.60(1H,d,J=3.9Hz), 8.84(1H,t,J=2.1Hz).

### Example 201: Preparation of the compound of Compound No. 201.

The title compound was obtained in the same manner as the Example 104(2).
Yield: 1.7%(orange solid).
¹H-NMR(CDCl₃): *δ* 7.56(1H,s), 7.57-7.70(7H,m), 8.04-8.10(1H,m), 8.34(1H,dd,J=2.1Hz,J=8.4Hz), 8.47-8.53(1H,m), 8.57(1H,d,J=2.1Hz), 8.96(1H,t,J=2.1Hz).

### Example 202: Preparation of the compound of Compound No. 202.

The title compound was obtained in the same manner as the Example 104(2).
Yield: 35.1%(reddish brown solid).
¹H-NMR(CDCl₃): *δ* 1.43(6H,d,J=6.9Hz), 3.04-3.18(1H,m), 7.05(1H,d,J=4.2Hz), 7.38(1H,t,J=1.8Hz), 7.50(1H,s), 7.57(1H,t,J=8.1Hz), 7.89(2H,d,J=1.8Hz), 7.98-8.05(1H,m), 8.42-8.48(1H,m), 8.86(1H,d,J=4.2Hz), 8.95(1H,t,J=2.1Hz).

### Example 203: Preparation of the compound of Compound No. 203.

The title compound was obtained in the same manner as the Example 104(2).
Yield: 40.1%(orange solid).

The compound was obtained as a mixture of the geometrical isomers of the exomethylene in the 4-position of the pyrazolone ring.
¹H-NMR(CDCl₃): *δ* 1.41 and 1.45(total 6H,each d,J=6.9Hz), 3.01-3.07 and 3.80-3.85(total 1H,each m), 7.10-8.84(total 9H,m).

### Example 204: Preparation of the compound of Compound No. 204.

The title compound was obtained in the same manner as the Example 104(2).
Yield: 35.3%(orange solid).
¹H-NMR(CDCIs)^{:} *δ* 1.40(6H,d,J=6.9Hz), 3.04-3.17(1H,m), 3.99(1H,s), 7.16(1H,t,J=1.5Hz), 7.52(1H,s), 7.63(1H,t,J=7.5Hz), 8.03(2H,d,J=1.5Hz), 8.21(1H,dd,J=1.2Hz,J=7.5Hz), 8.73-8.78(1H,m), 8.90-8.95(1H,m).

### Example 205: Preparation of the compound of Compound No. 205.

The title compound was obtained in the same manner as the Example 104(2).
Yield: 72.7%(light orange solid).

The compound was obtained as a mixture of the geometrical isomers of the exomethylene in the 4-position of the pyrazolone ring.
¹H-NMR(CDCl₃) *δ* 1.38 and 1.40(total 6H,each d,J=6.9Hz), 2.96-3.12 and 3.58-3.68(total 1H,each m), 3.88 and 3.95(total 3H,each s), 6.63 and 6.66(total 1H,each d,J=3.9Hz), 7.07 and 8.73(total 1H,each d,J=3.9Hz), 7.31 and 7.34(total 1H,s), 7.44-8.68(total 4H,m).

### Example 206: Preparation of the compound of Compound No. 206.

The title compound was obtained in the same manner as the Example 104(2).
Yield: 66.7%(light orange solid).
¹H-NMR(CDCl₃): *δ* 1.40(6H,d,J=6.9Hz), 3.03-3.12(1H,m), 7.16-7.20(1H,m), 7.22-7.30(1H,m), 7.38(1H,s), 7.69(1H,d,J=8.7Hz), 7.96-8.03(1H,m), 8.60-8.70(1H,m), 8.93(1H,s).

### Example 207: Preparation of the compound of Compound No. 207.

The title compound was obtained in the same manner as the Example 104(2).
Yield: 49.4%(yellowish orange solid).
¹H-NMR(CDCl₃): *δ* 1.39(6H,d,J=6.9Hz), 3.04-3.47(1H,m), 7.14-7.18(1H,m), 7.26-7.28(2H,m), 7.39(1H,s), 7.65(1H,m,J=8.4Hz), 8.80-8.84(2H,m), 8.31(1H,d,J=8.4Hz).

### Example 208: Preparation of the compound of Compound No. 208.

The title compound was obtained in the same manner as the Example 104(2).
Yield: 9.5%(orange solid).

The compound was obtained as a mixture of the geometrical isomers of the exomethylene in the 4-position of the pyrazolone ring.
¹H-NMR(CDCl₃): *δ* 1.21 and 1.40(total 6H,each d,J=6.9Hz), 3.01-3.12(total 1H, m), 3.97 and 3.99(total 3H,each s), 5.92 and 6.05(total 2H,each s), 6.97-8.65(total 8H,m).

### Example 209: Preparation of the compound of Compound No. 209.

The title compound was obtained in the same manner as the Example 104(2).
Yield: 38.4%(red solid).

The compound was obtained as a mixture of the geometrical isomers of the exomethylene in the 4-position of the pyrazolone ring.
1H-NMR(CDCl₃): *δ* 1.42 and 1.47(total 6H,each d,J=6.9Hz), 3.05-3.16 and 3.80-3.87(total 1H,each m), 3.95(3H,s), 6.98 and 7.03(total 1H, each d,J=3.9Hz), 7.24-7.54(total 3H,m), 7.63 and 7.69(total 2H,each d,J=2.1Hz), 7.84-8.89(total 4H,m).

### Example 210: Preparation of the compound of Compound No. 210.

The title compound was obtained in the same manner as the Example 104(2).
Yield: 11.0%(orange solid).
¹H-NMR(CDCl₃): *δ* 1.40(6H,d,J=6.9Hz), 3.01-3.15(1H,m), 3.95(3H,s), 7.33(1H,s), 7.50(1H,t,J=7.8Hz), 7.58(1H,d,J=8.1Hz), 7.84-7.92(1H,m), 8.18-8.26(1H,m), 8.34-8.42(1H,m), 8.60(2H,s).

### Example 211: Preparation of the compound of Compound No. 211.

The title compound was obtained in the same manner as the Example 104(2).
Yield: 30.1%(reddish brown solid).

The compound was obtained as a mixture of the geometrical isomers of the exomethylene in the 4-position of the pyrazolone ring.
¹H-NMR(DMSO-d₆): *δ* 1.34 and 1.39(total 6H,each d,J=3.3Hz), 3.20-3.41(total 1H, m), 7.55-8.67(total 10H,m), 13.05(1H,brs).

### Example 212: Preparation of the compound of Compound No. 212.

A solution of 4-[3-(methoxycarbonyl)benzylidene]-1-(3,5-dichlorophenyl)-3-isopropyl-2-pyrazolin-5-one(Compound No. 204; 30 mg, 0.0719 mmol) and sodium hydroxide(50 mg, 1.25 mmol) in a mixed solvent of methanol/water(1.5 ml + 0.2 ml) was stirred at 70°C for 1 hour. The reaction mixture was cooled, acidified by addition of 2N hydrochloric acid, and extracted with ethyl acetate. The organic layer was washed with brine and dried over anhydrous sodium sulfate. The residue obtained by evaporation of the solvent under reduced pressure was washed with isopropyl ether and ethyl acetate under suspension to give the title compound(20 mg, 69.0%) as an ocherous solid.
¹H-NMR(DMSO-ds): *δ* 1.32(6H,d,J=7.0Hz), 3.26-3.39(1H,m), 3.99(1H,s), 7.44(1H,t,J=1.8Hz), 7.70(1H,t,J=7.8Hz), 7.97(2H,d,J=2.4Hz), 8.09(1H,s), 8.09-8.16(1H,m), 8.79-8.82(1H,m), 9.01-9.04(1H,m), 13.26(1H,s).

### Example 213: Preparation of the compound of Compound No. 213.

The title compound was obtained in the same manner as the Example 104(2).
Yield: 66.7%(orange solid).
¹H-NMR(CDCl3): *δ* 1.59(3H,t,J=6.9Hz), 2.26(3H,s), 3.94(3H,s), 4.54(2H,q,J=6.9Hz), 6.91(1H,d,J=9.0Hz), 7.78(1H,s), 7.93(1H,dd,J=9.0,2.4Hz), 8.12-8.29(5H,m).

### Example 214: Preparation of the compound of Compound No. 214.

The title compound was obtained in the same manner as the Example 104(2).
Yield: 32.2%(brown solid).

The compound was obtained as a mixture of the geometrical isomers of the exomethylene in the 4-position of the pyrazolone ring.
¹H-NMR(CDCl₃): *δ* 1.53-1.60(total 3H,m), 3.98 and 3.99(total 3H,each s), 4.48-4.59(total 2H,m), 7.27-8.92(total 9H,m).

### Example 215: Preparation of the compound of Compound No. 215.

The title compound was obtained in the same manner as the Example 104(2).
Yield: 19.2%(red solid).
¹H-NMR(DMSO-d₆): *δ* 7.10-7.12(1H,m), 7.56-7.64(5H,m), 7.76-7.84(3H,m), 8.24(1H,ddd,J=9.3,1.8,1.2Hz), 8.61(1H,t,J=1.8Hz), 8.66(1H,d,J=3.6Hz), 13.18(1H,brs).

### Example 216: Preparation of the compound of Compound No. 216.

The title compound was obtained in the same manner as the Example 104(2).
Yield: 9.3%(brown solid).

The compound was obtained as a mixture of the geometrical isomers of the exomethylene in the 4-position of the pyrazolone ring.
¹H-NMR(DMSO-d₆): *δ* 6.92-8.76(total 15H,m), 13.12(1H,s).

### Example 217: Preparation of the compound of Compound No. 217.

The title compound was obtained in the same manner as the Example 104(2).
Yield: 2.8%(brown solid).
¹H-NMR(DMSO-d₆): *δ* 7.56-7.68(4H,m), 7.74-7.84(4H,m), 7.92(1H,d,J=4.2Hz), 8.13-8.18(1H,m), 8.22-8.28(1H,m), 8.60-8.68(3H,m), 8.74(1H,d,J=4.2Hz), 13.13(1H,brs).

### Example 218: Preparation of the compound of Compound No. 218.

The title compound was obtained in the same manner as the Example 104(2).
Yield: 2.7%(red solid).

The compound was obtained as a mixture of the geometrical isomers of the exomethylene in the 4-position of the pyrazolone ring.
¹H-NMR(DMSO-d₆): *δ* 7.62-8.18(total 8H,m), 8.44 and 8.49(total 1H,each t,J=2.1Hz), 8.73-8.80(total 1H,m), 13.21(1H,s).

### Example 219: Preparation of the compound of Compound No. 219.

(1) 1-Phenyl-3-(3,4,5-tromethoxyphenyl)-2-pyrazolin-5-one.
   The title compound was obtained in the same manner as the Example 104(1).
   Yield: 67.9%(white solid).
   ¹H-NMR(CDC1₃):5 *δ* 3.83(2H,s), 3.91(3H,s), 3.94(6H,s), 6.98(2H,s), 7.19-7.26(1H,m), 7.40-7.47(2H,m), 7.94-7.99(2H,m).
(2) 4-Benzylidene-1-phenyl-3-(3,4,5-trimethoxyphenyl)-2-pyrazolin-5-one (Compound No. 219).
   The title compound was obtained in the same manner as the Example 104(2).
   Yield: 20.4%(orange solid).
   ¹H-NMR(DMSO-d₆): *δ* 3.77(3H,s), 3.88(6H,s), 7.02(2H,s), 7.23-7.30(1H,m), 7.46-7.68(5H,m), 7.91(1H,s), 7.95-7.99(2H,m), 8.55-8.59(2H,s).

### Example 220: The compound of Compound No. 220.

This compound is a commercially available compound.
Supplier: LaboTest (Germany).
Catalog code number: LT-246X499.

### Example 221: Preparation of the compound of Compound No. 221.

The title compound was obtained in the same manner as the Example 104(2).
Yield: 8.6%(reddish brown solid).
¹H-NMR(DMSO-d₆):δ 6.79(1H,t,J=8.1Hz), 7.07(1H,dd,J=8.1,1.2Hz), 7.59-7.75(5H,m), 7.74(1H,d,J=9.0Hz), 8.01(1H,dd,J=9.0,2.7Hz), 8.26(1H,s), 8.29(1H,d,J=2.7Hz), 8.57(1H,dd,J=8.1,1.2Hz), 9.87(1H,brs), 9.94(1H,brs).

### Example 222: Preparation of the compound of Compound No. 222.

(1) 2-Chloro-5-hydrazinopyridine.
   A solution of sodium sulfite(180 mg, 2.567 mmol) in water(3 ml) was added slowly to a solution of 5-amino-2-chloropyridine(300mg, 2.334mmol) in concentrated hydrochloric acid(3 ml) at -10°C, and the mixture was stirred for 30 minutes. A solution of stannous chloride[Tin(II) chloride; 2483 mg, 12.837 mmol] in concentrated hydrochloric acid(3 ml) was added slowly to the reaction mixture at -5°C, and the mixture was stirred for 1 hour. The reaction mixture was adjusted to pH 13 by addition of 2N NaOH and extracted twice with ethyl acetate. The organic layer was dried over anhydrous sodium sulfate. The residue obtained by evaporation of the solvent under reduced pressure was washed with ethyl acetate/hexane under suspension to give the title compound(160 mg, 47.7%) as a light red solid.
   ¹H-NMR(DMSO-d₆): *δ* 4.17(2H,s), 7.11-7.18(3H,m), 7.87(1H,t,J=1.5Hz).
(2) 1-(2-Chloropyridin-5-yl)-3-isopropyl-2-pyrazolin-5-one.
   A solution of 5-amino-2-chloropyridine(160 mg, 1.114 mmol) and ethyl isobutyrylacetate(204 mg, 1.226 mmol) in ethanol(2 ml) was refluxed for 1 hour. The residue obtained by evaporation of the solvent under reduced pressure was purified by column chromatography on silica gel(hexane:ethyl acetate=3:1) to give the title compound(184 mg, 69.5%) as a white solid.
   ¹H-NMR(CDCl₃): *δ* 1.26(6H,d,J=6.9Hz), 2.74-2.88(1H,m), 3.45(2H,s), 7.33(1H,d,J=8.7Hz), 8.29(1H,dd,J=8.7,2.7Hz), 8.96(1H,d,J=2.7Hz).
(3) 4-(2,4,5-Trihydroxybenzylidene)-1-(2-chloropyridin-5-yl)-3-isopropyl-2-pyrazolin-5-one (Compound No. 222).
   The title compound was obtained in the same manner as the Example 104(2).
   Yield: 20.0%(red solid).
   ¹H-NMR(DMSO-d₆): *δ* 1.30(6H,d,J=6.9Hz), 3.06-3.18(1H,m), 6.45(1H,s), 7.59(1H,d,J=8.7Hz), 8.09(1H,s), 8.37(1H,dd,J=8.7,2.7Hz), 8.89(1H,s), 8.95(1H,brs), 9.06(1H,d,J=2.7Hz), 10.53(1H,brs), 10.74(1H,brs).

### Example 223: Preparation of the compound of Compound No. 223.

The title compound was obtained in the same manner as the Example 104(2).
Yield: 4.4%(red solid).
¹H-NMR(DMSO-d₆) : *δ* 1.45(3H,d,J=6.9Hz), 4.39-4.52(2H,m), 6.34(1H,s), 8.01(1H,brs), 8.18-8.34(4H,m), 8.79(1H,brs).

### Example 224: Preparation of the compound of Compound No. 224.

The title compound was obtained in the same manner as the Example 104(2).
Yield: 40.3%(orange solid).
¹H-NMR(DMSO-ds)^{:} *δ* 2.26(3H,s), 6.45(1H,s), 7.48(2H,d,J=8.7Hz), 7.95(1H,s), 8.00(2H,J=8.7Hz), 8.91(1H,brs), 8.93(1H,s), 10.48(1H,s), 10.65(1H,s).

### Example 301: Preparation of the compound of Compound No. 301.

(1) 3-(4-Methoxycarbonylbenzyl)thiazolidine-2,4-dione.
   A mixture of thiazolidine-2,4-dione(586 mg, 5.00 mmol), methyl 4-(bromomethyl)benzoate(1.15 g, 5.02 mmol), potassium carbonate(700mg, 5.06mmol) and N,N-dimethylformamide(10 ml) was stirred at 60°C for 30 minutes. The reaction mixture was cooled, poured into ice and water, and extracted with ethyl acetate. The organic layer was washed with water and brine, and dried over anhydrous sodium sulfate. The residue obtained by evaporation of the solvent under reduced pressure was washed with diisopropyl ether to give the title compound(1.169 mg, 88.1%) as a light yellow solid.
   ¹H-NMR(DMSO-d₆): *δ* 3.91(3H,s), 3.98(2H,s), 4.81(2H,s), 7.45(2H,d,J=8.1Hz), 8.00(2H,d,J=8.1Hz).
(2) 3-(4-Carboxybenzyl)thiazolidine-2,4-dione.
   A mixture of 3-(4-methoxycarbonylbenzyl)thiazolidine-2,4-dione(975 mg, 3.68 mmol), acetic acid(10 ml), concentrated hydrochloric acid(3 ml) was refluxed for 3 hours. The reaction mixture was cooled to room temperature, and water was added to the mixture. The separated solid was collected by filtration, washed with water and hexane, and dried under reduced pressure to give the title compound(840 mg, 91.0%) as a white solid.
   ¹H-NMR(DMSO-d₆): *δ* 4.30(2H,s), 4.74(2H,s), 7.38(2H,d,J=8.4Hz), 7.91(2H,d,J=8.4Hz), 12.94(1H,s).
(3) 3-[4-(Phenylcarbamoyl)benzyl]thiazolidine-2,4-dione.
   A mixture of 3-(4-carboxybenzyl)thiazolidine-2,4-dione(69 mg, 0.28 mmol), aniline(26 mg, 0.28 mmol), phosphorus trichloride(0.024 ml, 0.28 mmol) and toluene(1.5 ml) was refluxed for 1.2 hours. The reaction mixture was cooled to room temperature, and water was added to the mixture. The separated solid was collected by filtration, washed with water and diisopropyl ether, and dried under reduced pressure to give the title compound(56 mg, 62.5%) as a light yellow solid.
   ¹H-NMR(DMSO-d₆): *δ* 4.31(2H,s), 4.76(2H,s), 7.10(1H,t,J=7.5Hz), 7.35(2H,t, J=7.8Hz), 7.41(2H,d,J=8.4Hz), 7.76(2H,d,J=7.5Hz), 7.90(2H,d,J=8.1Hz), 10.23(1H,s).
(4) 5-(3,4-Dihydroxybenzylidene)-3-[4-(phenylcarbamoyl)benzyl]thiazolidine-2,4-dione (Compound No. 301).
   The title compound was obtained in the same manner as the Example 2(2) using the following raw materials.
   Raw materials: 3,4-dihydroxybenzaldehyde and 3-[4-(phenylcarbamoyl)benzyl]thiazolidine-2,4-dione.
   Yield: 14.3%(yellow solid).
   ¹H-NMR(DMSO-d₆): *δ* 4.91(2H,s), 6.89(1H,d,J=8.1Hz), 7.01-7.05(2H,m), 7.10(1H,t,J=7.5Hz), 7.32-7.37(2H,m), 7.44(2H,d,J=8.1Hz), 7.75(2H,d,J=7.8Hz), 7.81(1H,s), 7.91(2H,d,J=8.4Hz), 9.53(1H,s), 9.95(1H,s), 10.24(1H,s).

### Example 302: Preparation of the compound of Compound No. 302.

(1) 3-(4-Nitrobenzyl)thiazolidine-2,4-dione.
   The title compound was obtained in the same manner as the Example 11(1) using the following raw materials.
   Raw materials: 2,4-thiazolidinedione and 4-nitrobenzylalcohol.
   Yield: 90.9%(white solid).
   ¹H-NMR(DMSO-ds): *δ* 4.29(2H,s), 4.81(2H,s), 7.55(2H,d,J=9.0Hz), 8.20(2H,d,J=8.7Hz).
(2) 3-(4-Aminobenzyl)thiazolidine-2,4-dione.
   Concentrated hydrochloric acid(2ml) and iron powder(0.6 g, 10.7 mmol) were added to a mixture of 3-(4-nitrobenzyl)thiazolidine-2,4-dione(1.147 g, 4.54 mmol) and ethanol(20 ml), and the mixture was stirred at 80°C for 10 minutes. Then concentrated hydrochloric acid(1ml) and iron powder(0.3g) were added, and the mixture was stirred at 80°C for 1 hour. The reaction mixture was poured into saturated aqueous sodium hydrogencarbonate, filtered through celite, and the filtrate was extracted with ethyl acetate. The organic layer was washed with water and brine, and dried over anhydrous magnesium sulfate. The residue obtained by evaporation of the solvent under reduced pressure was washed with diisopropyl ether to give the title compound(0.767 g, 75.9%) as a yellowish white powder.
   ¹H-NMR(DMSO-d₆): *δ* 4.22(2H,s), 4.47(2H,s), 5.06(2H,s), 6.48(2H,d,J=8.4Hz), 6.94(2H,d,J=8.4Hz).
(3) 3-[4-(Phenylamino)benzyl]thiazolidine-2,4-dione.
   A mixture of 3-(4-aminobenzyl)thiazolidine-2,4-dione(150 mg, 0.68 mmol), phenylboronic acid(165 mg, 1.35 mmol), copper(II) acetate(123 mg, 0.68 mmol), triethylamine(0.19 ml, 1.36 mmol) and dichloromethane(4 ml) was stirred at room temperature for 6 hours. The reaction mixture was filtered through celite. The residue obtained by concentration of the filtrate under reduced pressure was purified by chromatography on silica gel(hexane:ethyl acetate=1:1) to give the title compound(114 mg, 56.7%) as an orange oil.
   ¹H-NMR(CDCl₃): *δ* 3.94(2H,s), 4.70(2H,s), 5.74(1H,s), 6.93-6.95(1H,m), 7.00(2H,d,J=8.7Hz), 7.05-7.09(2H,m), 7.24-7.28(2H,m), 7.31(2H,d,J=8.7Hz).
(4) 5-(3,4-Dihydroxybenzylidene)-3-[4-(phenylamino)benzyl]thiazolidine-2,4-dione (Compound No. 302).
   The title compound was obtained in the same manner as the Example 2(2) using the following raw materials.
   Raw materials: 3,4-dihydroxybenzaldehyde and 3-[4-(phenylamino)benzyl]thiazolidine-2,4-dione.
   Yield: 35.7%(yellow solid).
   ¹H-NMR(DMSO-d₆): *δ* 4.72(2H,s), 6.82(1H,t,J=7.5Hz), 6.88(1H,d,J=7.8Hz), 6.99-7.07(6H,m), 7.17-7.24(4H,m), 7.78(1H,s), 8.23(1H,s), 9.52(1H,s), 9.93(1H,s).

### Example 303: Preparation of the compound of Compound No. 303.

The title compound was obtained in the same manner as the Example 71(4) using the following raw materials.
Raw materials: 3,4,5-trihydroxybenzaldehyde and
3-(3,4-dichlorobenzyl)rhodanine(compound of the Example 81(1)).
Yield: 68.2%(yellowish brown powder).
¹H-NMR(DMSO-d₆): *δ* 5.22(2H,s), 6.66(2H,s), 7.29(1H,dd,J=8.7,2.4Hz), 7.59-7.62(3H,m), 9.43(3H,br).

### Example 304: Preparation of the compound of Compound No. 304.

(1) 3-[(5,6-Dichloropyridin-3-yl)methyl]thiazolidine-2,4-dione.
   The title compound was obtained in the same manner as the Example 11(1) using the following raw materials.
   Raw materials: 2,4-thiazolidinedione and 5,6-dichloro-3-pyridinemethanol.
   Yield: 94.8%(white powder).
   ¹H-NMR(CDCl₃): *δ* 4.00(2H,s), 4.75(2H,s), 7.85(1H,d,J=2.4Hz), 8.37(1H,d,J=2.1Hz).
(2) 5-(3,4-Dihydroxybenzylidene)-3-[(5,6-dichloropyridin-3-yl)-methyl]thiazolidine-2,4-dione (Compound No. 304).
   The title compound was obtained in the same manner as the Example 2(2) using the following raw materials.
   Raw materials: 3,4-dihydroxybenzaldehyde and 3-[(5,6-dichloropyridin-3-yl)methyl]thiazolidine-2,4-dione.
   Yield: 78.4%(yellowish white powder).
   ¹H-NMR(DMSO-d₆):*δ* 4.88(2H,s), 6.88(1H,d,J=7.8Hz), 6.99-7.04(2H,m), 7.77(1H,s), 8.09(1H,d,J=2.1Hz), 8.39(1H,d,J=2.1Hz), 9.47(1H,s), 9.88(1H,s).

### Example 305: Preparation of the compound of Compound No. 305.

(1) 3-[2,5-Bis(trifluoromethyl)benzyl]thiazolidine-2,4-dione.
   The title compound was obtained in the same manner as the Example 11(1) using the following raw materials.
   Raw materials: 2,4-thiazolidinedione and 2,5-bis(trifluoromethyl)benzylalcohol.
   Yield: 88.4%(white crystal).
   ¹H-NMR(CDCl₃): *δ* 4.12(2H,s), 5.07(2H,s), 7.32(1H,s), 7.68(1H,d,J=7.8Hz), 7.84(1H,d, J=8.1Hz).
(2) 5-(3,4-Dihydroxybenzylidene)-3-[2,5-bis(trifluoromethyl)benzyl]-thiazolidine-2,4-dione (Compound No. 305).
   The title compound was obtained in the same manner as the Example 2(2) using the following raw materials.
   Raw materials: 3,4-dihydroxybenzaldehyde and
   3- [2,5-bis(trifluoromethyl)benzyl]thiazolidine-2,4-dione.
   Yield: 81.4%(yellowish white powder).
   ¹H-NMR(DMSO-d₆):*δ* 5.06(2H,s), 6.89(1H,d,J=7.8Hz), 7.02-7.07(2H,m), 7.71(1H,s), 7.80(1H,s), 7.93(1H,d,J=8.1Hz), 8.04(1H,d,J=8.4Hz), 9.69(2H,br).

### Example 306: Preparation of the compound of Compound No. 306.

(1) 3-(4-Styrylbenzyl)rhodanine.
   The title compound was obtained in the same manner as the Example 11(1) using the following raw materials.
   Raw materials: 4-styrylbenzylalchohol and rhodanine.
   Yield: 55.3%(light yellowish white powder).
   ¹H-NMR(DMSO-d₆): *δ* 4.25(2H,s), 4.61(2H,s), 7.25-7.30(3H,m), 7.38(2H,t,J=7.2Hz), 7.45(2H,d,J=8.1Hz), 7.58-7.61(4H,m).
(2) 5-(3,4-Dihydroxybenzylidene)-3-(4-styrylbenzyl)rhodanine (Compound No. 306).
   The title compound was obtained in the same manner as the Example 71(4) using the following raw materials.
   Raw materials: 3,4-dihydroxybenzaldehyde and 3-(4-styrylbenzyl)rhodanine.
   Yield: 84.6%(yellow powder).
   ¹H-NMR(DMSO-d₆): *δ* 5.24(2H,s), 6.90(1H,d, J=8.7Hz), 7.05-7.09(2H,m), 7.23-7.40(7H,m), 7.55-7.61(4H,m), 7.70(1H,s), 9.56(1H,s), 10.03(1H,s).

### Example 307: Preparation of the compound of Compound No. 307.

(1) 3-[4-(Trifluoromethyl)benzyl]rhodanine.
   The title compound was obtained in the same manner as the Example 11(1) using the following raw materials.
   Raw materials: 4-(trifluoromethyl)benzylalchohol and rhodanine.
   Yield: 32.4%(light yellowish white powder).
   ¹H-NMR(DMSO-d₆): δ 4.03(2H,s), 5.23(2H,s), 7.54(2H,d,J=8.4Hz), 7.58(2H,d,J=8.7Hz).
(2) 5-(3,4-Dihydroxybenzylidene)-3-[4-(trifluoromethyl)benzyl]rhodanine (Compound No. 307).
   The title compound was obtained in the same manner as the Example 71(4) using the following raw materials.
   Raw materials: 3,4-dihydroxybenzaldehyde and 3-[4-(trifluoromethyl)benzyl]rhodanine.
   Yield: 73.4%(yellow crystal powder).
   ¹H-NMR(DMSO-ds): *δ* 5.32(2H,s), 6.91(1H,d,J=9.0Hz), 7.06-7.09(2H,m), 7.52(2H,d,J=8.1Hz), 7.70-7.72(3H,m), 9.56(1H,s), 10.04(1H,s).

### Example 308: Preparation of the compound of Compound No. 308.

(1) 3-[3,4,5-Tris(benzyloxy)benzyl]thiazolidine-2,4-dione.
   The title compound was obtained in the same manner as the Example 11(1) using the following raw materials.
   Raw materials: 3,4,5-tris(benzyloxy)benzylalcohol and 2,4-thiazolidinedione.
   Yield: 77.3%(white crystal).
   ¹H-NMR(CDCl₃): *δ* 3.89(2H,s), 4.64(2H,s), 5.02(2H,2), 5.09(4H,s), 6.71(2H,s), 7.25-7.44(15H,m).
(2) 5-(4-Phenoxybenzylidene)-3-[3,4,5-tris(benzyloxy)benzyl]thiazolidine-2,4-dione.
   The title compound was obtained in the same manner as the Example 2(2) using the following raw materials.
   Raw materials: 4-phenoxybenzaldehyde and 3-[3,4,5-tris(benzyloxy)benzyl]thiazolidine-2,4-dione.
   Yield: 62.1%(yellowish white solid).
   ¹H-NMR(DMSO-d₆): *δ* 4.74(2H,s), 4.91(2H,s), 5.09(4H,s), 6.71(2H,s), 7.12-7.15(4H,m), 7.24-7.49(18H,m), 7.69(2H,d,J=9.0Hz), 7.95(1H,s).
(3) 5-(4-Phenoxybenzylidene)-3-(3,4,5-trihydroxybenzyl)thiazolidine-2,4-dione (Compound No. 308).
   Boron tribromide(1.0M solution in dichloromethane; 0.3ml, 0.3mmol) was added to a solution of 5-(4-phenoxybenzylidene)-3-[3,4,5-tris(benzyloxy)benzyl]thiazolidine-2,4-dione(83.4m g, 0.12mmol) in dichloromethane(3ml) under ice cooling, and the mixture was stirred at room temperature for 1.5 hours. The reaction mixture was poured into ice and water, and extracted with ethyl acetate. The organic layer was washed with brine and dried over anhydrous sodium sulfate. The residue obtained by evaporation of the solvent under reduced pressure was purified by chromatography on silica gel(ethyl acetate:hexane=1:1→2:1) and recrystallized from chloroform/hexane to give the title compound(18.8mg, 36.6%) as a yellowish white powder.
   ¹H-NMR(DMSO-ds): *δ* 4.58(2H,s), 6.25(2H,s), 7.10-7.14(4H,m), 7.24(1H,t,J=7.5Hz), 7.46(2H,t,J=8.1Hz), 7.66(2H,d,J=8.7Hz), 7.94(1H,s), 8.07(1H,s), 8.86(2H,s).

### Example 309: Preparation of the compound of Compound No. 309.

The title compound was obtained in the same manner as the Example 2(2) using the following raw materials.
Raw materials: 2,3-dihydroxybenzaldehyde and
3-[4-(phenylamino)benzyl]thiazolidine-2,4-dione(compound of the Example 302(3)). Yield: 43.1%(yellow foamy solid).
¹H-NMR(DMSO-d₆): *δ* 4.73(2H,s), 6.76-6.86(3H,m), 6.92(1H,dd,J =7.5,1.8Hz), 7.01-7.07(4H,m), 7.18-7.24(4H,m), 8.20(1H,s), 8.23(1H,s), 9.51(1H,s), 9.89(1H,s).

### Example 310: Preparation of the compound of Compound No. 310.

(1) 5-(3-Phenoxybenzylidene)-3-[3,4,5-tris(benzyloxy)benzyl]thiazolidine-2,4-dione.
   The title compound was obtained in the same manner as the Example 2(2) using the following raw materials.
   Raw materials: 3-phenoxybenzaldehyde and
   3-[3,4,5-tris(benzyloxy)benzyl]thiazolidine-2,4-dione(compound of the Example 308(1)).
   Yield: 56.1%(yellowish white powder).
   ¹H-NMR(CDCl₃): *δ* 4.76(2H,s), 5.01(2H,s), 5.09(4H,s), 6.74(2H,s), 7.04-7.10(4H,m), 7.15-7.46(20H,m), 7.81(1H,s).
(2) 5-(3-Phenoxybenzylidene)-3-(3,4,5-trihydroxybenzyl) thiazolidine-2,4-dione (Compound No. 310).
   The title compound was obtained in the same manner as the Example 308(3) using the following raw materials.
   Raw materials:
   5-(3-phenoxybenzylidene)-3-[3,4,5-tris(benzyloxy)benzyl]thiazolidine-2,4-dione.
   Yield: 49.9%(light orange powder).
   ¹H-NMR(DMSO-d₆): *δ* 4.56(2H,s), 6.24(2H,s), 7.07-7.23(5H,m), 7.37-7.47(3H,m), 7.55(1H,t,J=8.1Hz), 7.94(1H,s), 8.07(1H,s), 8.98(2H,s).

### Example 311: Preparation of the compound of Compound No. 311.

(1) 3-[4-(Phenoxyacetylamino)benzyl]thiazolidine-2,4-dione.
   Phenoxyacetyl chloride(43 *µ* 1, 0.31 mmol) and triethylamine(0.1 ml, 0.71 mmol) were added to a solution of 3-(4-aminobenzyl)thiazolidine-2,4-dione(compound of the Example 302(2); 66.6 mg, 0.3 mmol) in tetrahydrofuran(3 ml), and the mixture was stirred at room temperature for 30 minutes. The reaction mixture was poured into diluted hydrochloric acid and extracted with ethyl acetate. The organic layer was washed with water and brine, and dried over anhydrous sodium sulfate. The solvent was evaporated under reduced pressure to give the title compound(107.2 mg, 99.7%) as a white solid.
   ¹H-NMR(CDCl₃): *δ* 3.94(2H,s), 4.61(2H,s), 4.74(2H,s), 6.97-7.01(2H,m), 7.07(1H,t,J=7.5Hz), 7.33-7.42(4H,m), 7.55(2H,d,J=8.7Hz), 8.27(1H,s).
(2) 5-(2,3-Dihydroxybenzylidene)-3-[4-(phenoxyacetylamino)benzyl]-thiazolidine-2,4-dione (Compound No. 311).
   The title compound was obtained in the same manner as the Example 2(2) using the following raw materials.
   Raw materials: 2,3-dihydroxybenzaldehyde and 3-[4-(phenoxyacetylamino)benzyl]thiazolidine-2,4-dione.
   Yield: 53.0%(ocherous powder).
   ¹H-NMR(DMSO-d₆): *δ* 4.67(2H,s), 4.76(2H,s), 6.41(1H,t,J=7.5Hz), 6.67-6.70(2H,m), 6.94-7.00(3H,m), 7.24-7.33(4H,m), 7.60(2H,d,J=8.7Hz), 8.18(1H,s), 10.10(1H,brs).

### Example 312: Preparation of the compound of Compound No. 312.

(1) 3-{4-[(3-Phenoxyphenyl)acetylamino]benzyl}thiazolidine-2,4-dione.
   (1-(3-Dimethylaminopropyl)-3-ethylcarbodiimide hydrochloride(WSCI· HCl; 110.1 mg, 0.56 mmol) and 4-dimethylaminopyridine(DMAP; 2 mg) were added to a solution of 3-(4-aminobenzyl)thiazolidine-2,4-dione(compound of the Example 302(2); 63.5 mg, 0.29 mmol) and 3-phenoxyphenylacetic acid(65.2 mg, 0.28 mmol) in tetrahydrofuran(2 ml), and the mixture was stirred at room temperature for 2 hours. The reaction mixture was poured into water and extracted with ethyl acetate. The organic layer was washed with water and brine, and dried over anhydrous sodium sulfate. The residue obtained by evaporation of the solvent under reduced pressure was purified by chromatography on silica gel(hexane:ethyl acetate= 1: 1-2: 1) to give the title compound(95mg, 76.9%) as a clear and light yellow oil.
   ¹H-NMR(CDCl₃): *δ* 3.69(2H,s), 3.92(2H,s), 4.70(2H,s), 6.90-7.16(8H,m), 7.29-7.39(6H,m).
(2) 5-(2,3-Dihydroxybenzylidene)-3-{4-[(3-phenoxyphenyl)acetylamino]-benzyl}thiazolidine-2,4-dione (Compound No. 312).
   The title compound was obtained in the same manner as the Example 2(2) using the following raw materials.
   Raw materials: 2,3-dihydroxybenzaldehyde and 3-{4-[(3-phenoxyphenyl)acetylaminolbenzyl)thiazolidine-2,4-dione.
   Yield: 38.3%(orange powder).
   ¹H-NMR(DMSO-d₆): *δ* 3.61(2H,s), 4.76(2H,s), 6.76-6.93(4H,m), 6.99-7.02(3H,m), 7.07-7.16(3H,m), 7.07-7.16(2H,m), 7.24(2H,d,J=8.7Hz), 7.32(1H,t,J=8.1Hz), 7.35-7.41(2H,m), 7.54(2H,d,J=8.7Hz), 8.19(1H,s), 9.49(1H,brs), 9.78(1H,brs), 10.16(1H,s).

### Example 313: Preparation of the compound of Compound No. 313.

(1) 3-[4-(Phenylacetylamino)benzyl]thiazolidine-2,4-dione.
   Phenylacetyl chloride(43 *µ* 1, 0.32 mmol) and triethylamine(0.1 ml, 0.72 mmol) were added to a solution of 3-(4-aminobenzyl)thiazolidine-2,4-dione(compound of the Example 302(2); 66.7 mg, 0.3 mmol) in tetrahydrofuran(2 ml), and the mixture was stirred at room temperature for 1 hour. The reaction mixture was poured into diluted hydrochloric acid and extracted with ethyl acetate. The organic layer was washed with water and brine, and dried over anhydrous sodium sulfate. The residue obtained by evaporation of the solvent under reduced pressure was washed with ethyl acetate/diisopropyl ether under suspension to give the title compound(101.6mg, 99.5%) as a light yellowish white powder.
   ¹H-NMR(DMSO-d₆): *δ* 3.62(2H,s), 4.25(2H,s), 4.60(2H,s), 7.19(2H,d,J=8.7Hz), 7.23-7.26(1H,m), 7.31-7.32(4H,m), 7.53(2H,d,J=8.7Hz), 10.16(1H,s).
(2) 5-(2,3-Dihydroxybenzylidene)-3-[4-(phenylacetylamino)benzyl]-thiazolidine-2,4-dione (Compound No. 313)
   The title compound was obtained in the same manner as the Example 2(2) using the following raw materials.
   Raw materials: 2,3-dihydroxybenzaldehyde and 3-[4-(phenylacetylamino)benzyl]thiazolidine-2,4-dione.
   Yield: 13.5%(yellow powder).
   ¹H-NMR(DMSO-d₆): *δ* 3.62(2H,s), 4.76(2H,s), 6.76-6.86(2H,m), 6.92(1H,dd,J=7.5,1.5Hz), 7.22-7.26(1H,m), 7.24(2H,d,J=8.7Hz), 7.31-7.32(4H,m), 7.56(1H,d,J=8.7Hz), 8.18(1H,s), 9.46(1H,s), 9.83(1H,s), 10.18(1H,s).

### Example 314: Preparation of the compound of Compound No. 314.

(1) 3-[4-(3-Phenylpropionylamino)benzyl]thiazolidine-2,4-dione.
   Hydrocinnamoyl chloride(67g 1, 0.45 mmol) and triethylamine(0.13 ml, 0.93 mmol) were added to a solution of 3-(4-aminobenzyl)thiazolidine-2,4-dione(compound of the Example 302(2); 0.10 g, 0.45 mmol) in tetrahydrofuran(3 ml), and the mixture was stirred at room temperature for 1 hour. The reaction mixture was poured into diluted hydrochloric acid and extracted with ethyl acetate. The organic layer was washed with water and brine, and dried over anhydrous sodium sulfate. The residue obtained by evaporation of the solvent under reduced pressure was crystallized by ethyl acetate/diisopropyl ether/hexane to give the title compound(157.5mg, 98.8%) as a white powder.
   ¹H-NMR(CDCl₃): *δ* 2.65(2H,t,J=7.8Hz), 3.05(2H,t,J=7.8Hz), 3.93(2H,s), 4.71(2H,s), 6.96(1H,brs), 7.22-7.39(9H,m).
(2) 5-(2,3-Dihydroxybenzylidene)-3-[4-(3-phenylpropionylamino)benzyl]-thiazolidine-2,4-dione (Compound No. 314).
   The title compound was obtained in the same manner as the Example 2(2) using the following raw materials.
   Raw materials: 2,3-dihydroxybenzaldehyde and 3-[4-(3-phenylpropionylamino)benzyl]thiazolidine-2,4-dione.
   Yield: 59.0%(yellow powder).
   ¹H-NMR(DMSO-d₆): *δ* 2.60(2H,t,J=7.8Hz), 2.90(2H,t,J=7.8Hz), 4.76(2H,s), 6.76-6.86(2H,m), 6.92(1H,dd,J=7.5,1.8Hz), 7.14-7.30(7H,m), 7.54(2H,d,J=9.0Hz), 8.19(1H,s), 9.46(1H,s), 9.83(1H,s), 9.92(1H,s).

### Example 315: Preparation of the compound of Compound No. 315.

The title compound was obtained in the same manner as the Example 2(2) using the following raw materials.
Raw materials: 3,4-dihydroxybenzaldehyde and
3-[4-(3-phenylpropionylamino)benzyl]thiazolidine-2,4-dione(compound of the Example 314(1)).
Yield: 66.5%(light yellow powder).
¹H-NMR(DMSO-ds): *δ* 2.60(2H,t,J=7.8Hz), 2.90(2H,t,J=7.8Hz), 4.75(2H,s), 6.88(1H,d,J=8.4Hz), 6.98-7.03(2H,m), 7.14-7.30(7H,m), 7.53(2H,d,J=8.4Hz), 7.77(1H,s), 9.47(1H,brs), 9.85(1H,brs), 9.95(1H,s).

### Example 316: Preparation of the compound of Compound No. 316.

(1) 3-[4-(4-Phenoxybenzoylamino)benzyl]thiazolidine-2,4-dione.
   Phosphorus oxychloride(45 *µ* 1, 0.49 mmol) was added to a solution of 3-(4-aminobenzyl)thiazolidine-2,4-dione(compound of the Example 302(2); 0.10 g, 0.45 mmol), 4-phenoxybenzoic acid(96.4 mg, 0.45 mmol) and pyridine(80 *µ* l, 0.99 mmol) in tetrahydrofuran(3 ml) under ice cooling, and the mixture was stirred at room temperature for 1 hour. The reaction mixture was poured into diluted hydrochloric acid and extracted with ethyl acetate. The organic layer was washed with brine, and dried over anhydrous sodium sulfate. The residue obtained by evaporation of the solvent under reduced pressure was purified by chromatography on silica gel(hexane:ethyl acetate=1:1) to give the title compound(95.1mg, 50.5%) as a white powder.
   ¹H-NMR(DMSO-d₆): *δ* 4.28(2H,s), 4.64(2H,s), 7.08-7.12(4H,m), 7.20-7.26(3H,m), 7.46(2H,t,J=7.8Hz), 7.71(2H,d,J=8.4Hz), 7.99(2H,d,J=8.7Hz), 10.22(1H,s).
(2) 5-(2,3-Dihydroxybenzylidene)-3-[4-(4-phenoxybenzoylamino)benzyl]-thiazolidine-2,4-dione (Compound No. 316).
   The title compound was obtained in the same manner as the Example 2(2) using the following raw materials.
   Raw materials: 2,3-dihydroxybenzaldehyde and 3-[4-(4-phenoxybenzoylamino)benzyl]thiazolidine-2,4-dione.
   Yield: 49.7%(light orange powder).
   ¹H-NMR(DMSO-d₆): *δ* 4.80(2H,s), 6.76-6.87(2H,m), 6.92(1H,dd,J=7.8,1.8Hz), 7.06-7.12(4H,m), 7.19-7.25(1H,m), 7.30(2H,d,J=8.7Hz), 7.40-7.49(2H,m), 7.74(2H,d,J=8.7Hz), 7.99(2H,d,J=8.7Hz), 8.20(1H,s), 9.46(1H,s), 9.83(1H,s), 10.20(1H,s).

### Example 317: Preparation of the compound of Compound No. 317.

(1) 3-{4-[3,5-Bis(trifluoromethyl)phenylacetylamino]benzyl}thiazolidine-2,4-dione.
   The title compound was obtained in the same manner as the Example 316(1) using the following raw materials.
   Raw materials: 3-(4-aminobenzyl)thiazolidine-2,4-dione(compound of the Example 302(2)) and 3,5-bis(trifluoromethyl)phenylacetic acid.
   Yield: 61.1%(yellowish white powder).
   ¹H.NMR(DMSO·d₆): *δ* 3.93(2H,s), 4.26(2H,s), 4.61(2H,s), 7.21(2H,d,J=8.7Hz), 7.54(2H,d,J=8.7Hz), 8.01(1H,s), 8.04(2H,s), 10.30(1H,s).
(2) 5-(2,3-Dihydroxybenzylidene)-3-{4-[3,5-Bis(trifluoromethyl)-phenylacetylamino]benzyl}thiazolidine-2,4-dione (Compound No. 317).
   The title compound was obtained in the same manner as the Example 2(2) using the following raw materials.
   Raw materials: 2,3-dihydroxybenzaldehyde and 3-{4-[3,5-Bis(trifluoromethyl)phenylacetylamino]benzyl}thiazolidine-2,4-dione.
   Yield: 54.5%(yellow powder).
   ¹H-NMR(DMSO.d₆): *δ* 3.93(2H,s), 4.77(2H,s), 6.76-6.85(2H,m), 6.91(1H,dd,J=7.5,1.8Hz), 7.25(2H,d,J=8.4Hz), 7.56(2H,d,J=9.0Hz), 8.00(1H,s), 8.03(2H,s), 8.18(1H,s), 9.50(1H,s), 9.88(1H,s), 10.32(1H,s).

### Example 318: Preparation of the compound of Compound No. 318.

(1) 3-(3-Nitrobenzyl)thiazolidine-2,4-dione.
   The title compound was obtained in the same manner as the Example 301(1) using the following raw materials.
   Raw materials: thiazolidine-2,4-dione and 3-nitrobenzyl chloride.
   Yield: 92.4%(light yellowish white powder).
   ¹H-NMR(DMSO-d₆): *δ* 4.28(2H,s), 4.82(2H,s), 7.65(1H,t,J=7.8Hz), 7.73-7.75(1H,m), 8.14-8.18(2H,m).
(2) 3-(3-Aminobenzyl)thiazolidine-2,4-dione hydrochloride.
   Concentrated hydrochloric acid(3ml) and iron powder(1.24 g, 22.2 mmol) were added to a mixture of 3-(3-nitrobenzyl)thiazolidine-2,4-dione(1.866 g, 7.40 mmol) and ethanol(30 ml), and the mixture was refluxed for 2.5 hours. The reaction mixture was poured into saturated aqueous sodium hydrogencarbonate, filtered through celite, and the filtrate was extracted with ethyl acetate. The organic layer was washed with water and brine, and dried over anhydrous magnesium sulfate. The residue obtained by evaporation of the solvent under reduced pressure was purified by chromatography on silica gel(hexane:ethyl acetate=1:1 → 1:2). The obtained crude product was dissolved in diisopropyl ether, and 4N hydrochloric acid in ethyl acetate and hexane were successively added to the solution. The separated solid was collected by filtration and dried under reduced pressure to give the title compound(1.771g, 92.5%) as a white powder.
   ¹H-NMR(DMSO-d₆): δ 3.68(2H,brs), 3.93(2H,s), 4.67(2H,s),
   6.61(1H,ddd,J=7.8,2.4,0.9Hz), 6.70-6.71(1H,m), 6.76-6.79(1H,m), 7.10(1H,t,J=7.8Hz).
(3) 3-[3-(Phenylamino)benzyl]thiazolidine-2,4-dione.
   The title compound was obtained in the same manner as the Example 302(3) using the following raw materials.
   Raw materials: 3-(3-aminobenzyl)thiazolidine-2,4-dione hydrochloride and phenylboronic acid.
   Yield: 62.3%(yellow oil).
   ¹H-NMR(CDCl₃): *δ* 3.94(2H,s), 4.71(2H,s), 5.76(1H,brs), 6.90-6.97(2H,m), 7.01-7.07(4H,m), 7.20(1H,t,J=7.8Hz), 7.25-7.30(2H,m).
(4) 5-(3,4-Dihydroxybenzylidene)-3-[3-(phenylamino)benzyl]thiazolidine-2,4-dione (Compound No. 318).
   The title compound was obtained in the same manner as the Example 2(2) using the following raw materials.
   Raw materials: 3,4-dihydroxybenzaldehyde and 3-[3-(phenylamino)benzyl]thiazolidine-2,4-dione.
   Yield: 35.3%(yellow solid).
   ¹H-NMR(DMSO-ds): *δ* 4.76(2H,s), 6.74(1H,d,J=7.5Hz), 6.82(1H,t,J=7.2Hz), 6.88(1H,d,J=7.8Hz), 6.97-7.06(6H,m), 7.16-7.24(3H,m), 7.79(1H,s), 8.24(1H,s), 9.51(1H,s), 9.93(1H,s).

### Example 319: Preparation of the compound of Compound No. 319.

The title compound was obtained in the same manner as the Example 2(2) using the following raw materials.
Raw materials: 2,3-dihydroxybenzaldehyde and
3-[3-(phenylamino)benzyl]thiazolidine-2,4-dione(compound of the Example 318(3)).
Yield: 9.5%(yellow solid).
¹H-NMR(DMSO-d₆): *δ* 4.77(2H,s), 6.75(1H,d,J=7.5Hz), 6.79-6.87(3H,m), 6.92(1H,dd,J=7.5,1.8Hz), 6.97-7.07(4H,m), 7.16-7.24(3H,m), 8.20(1H,s), 8.25(1H,s), 9.52(1H,s), 9.89(1H,s).

### Example 320: Preparation of the compound of Compound No. 320.

(1) 3-{3-[(3-Phenoxyphenyl)acetylamino]benzyl}thiazolidine-2,4-dione.
   Phosphorus oxychloride(120 *µ*1, 1.31 mmol) was added to a solution of 3-(3-aminobenzyl)thiazolidine-2,4-dione hydrochloride(compound of the Example 318(2); 325.5 mg, 1.26 mmol), 3-phenoxyphenylacetic acid(287.1 mg, 1.26 mmol) and pyridine(0.3 ml, 3.73 mmol) in tetrahydrofuran(5 ml) under ice cooling, and the mixture was stirred at room temperature for 40 minutes. The reaction mixture was poured into diluted hydrochloric acid and extracted with ethyl acetate. The organic layer was washed with water and brine, and dried over anhydrous sodium sulfate. The residue obtained by evaporation of the solvent under reduced pressure was purified by chromatography on silica gel(hexane:ethyl acetate=1:1) to give the title compound(343.4mg, 63.1%) as a white powder.
   ¹H-NMR(DMSO-ds):*δ* 3.61(2H,s), 4.26(2H,s), 4.62(2H,s), 6.87(1H,dd,J=7.8,1.8Hz), 6.95(1H,d,J=7.8Hz), 6.99-7.04(3H,m), 7.08-7.17(2H,m), 7.25(1H,t,J=8.1Hz), 7.33(1H,t,J=8.1Hz), 7.37-7.42(2H,m), 7.46(1H,m), 7.54-7.57(1H,m), 10.19(1H,s).
(2) 5-(2,3-Dihydroxybenzylidene)-3-{3-[(3-phenoxyphenyl)acetylamino]-benzyl}thiazolidine-2,4-dione (Compound No. 320).
   The title compound was obtained in the same manner as the Example 2(2) using the following raw materials.
   Raw materials: 2,3-dihydroxybenzaldehyde and 3-{3-[(3-phenoxyphenyl)acetylamino]benzyl}thiazolidine-2,4-dione.
   Yield: 39.4%(yellowish brown powder).
   ¹H-NMR(DMSO-ds): *δ* 3.61(2H,s), 4.78(2H,s), 6.79(1H,t,J=7.5Hz), 6.84-6.89(2H,m), 6.92(1H,dd,J=7.5,1.8Hz), 6.99-7.03(4H,m), 7.07-7.16(2H,m), 7.27(1H,t,J=7.8Hz), 7.30(1H,t,J=7.8Hz), 7.35-7.41(2H,m), 7.44(1H,m), 7.59-7.62(1H,m), 8.20(1H,s), 9.57(1H,brs), 9.83(1H,brs), 10.19(1H,s).

### Example 321: Preparation of the compound of Compound No. 321.

The title compound was obtained in the same manner as the Example 2(2) using the following raw materials.
Raw materials: 3,4-dihydroxybenzaldehyde and
3-{3-[(3-phenoxyphenyl)acetylamino]benzyl}thiazolidine-2,4-dione (compound of the Example 320(1)).
Yield: 66.1%(light yellowish brown powder).
¹H-NMR(DMSO-d₆): *δ* 3.60(2H,s), 4.78(2H,s), 6.85-6.90(2H,m), 6.98-7.16(8H,m), 7.27(1H,t,J=8.1Hz), 7.30(1H,t,J=8.1Hz), 7.35-7.41(2H,m), 7.46(1H,m), 7.56-7.59(1H,m), 7.79(1H,s), 9.52(1H,brs), 9.93(1H,brs), 10.19(1H,s).

### Example 322: Preparation of the compound of Compound No. 322.

(1) 5-Styrylsalicylaldehyde.
   A mixture of 5-bromosalicylaldehyde(1.5g, 7.462mmol), tri-o-tolylphosphine(116mg, 0.373mmol), palladium acetate(42mg, 0.187mmol) and triethylamine(7mL) was stirred at room temperature for 10 minutes. Styrene(855mg, 8.208mmol) was added to the mixture and refluxed for 2 hours. The reaction mixture was cooled and acidified by addition of 2N hydrochloric acid. The separated crystal was collected by filtration and washed with ethyl acetate under suspension to give the title compound(457mg, 27.3%) as a yellow solid.
   ¹H-NMR(CDCl₃): *δ* 7.00-7.06(3H,m), 7.24-7.41(3H,m), 7.48-7.53(2H,m), 7.68(1H,d,J=2.4Hz), 7.73(1H,dd,J=2.1,9.0Hz), 9.94(1H,s), 11.02(1H,s).
(2) 5-(2-Hydroxy-5-styrylbenzylidene)-3-(3,4-dichlorobenzyl)thiazolidine-2,4-dione (Compound No. 322).
   A mixture of 5-styrylsalicylaldehyde(65 mg, 0.290 mmol), 3-(3,4-dichlorobenzyl)thiazolidine-2,4-dione(compound of the Example 8(1); 80 mg, 0.290 mmol), ammonium acetate(67 mg, 0.869 mmol) and ethanol(1 ml) was stirred at 80°C for 1 hour. The residue obtained by evaporation of the solvent under reduced pressure was washed with methanol under suspension to give the title compound(35mg, 25.0%) as a yellow solid.
   ¹H-NMR(DMSO-d₆):δ 4.85(2H,s), 7.01(1H,d,J=8.7Hz), 7.07(1H,d,J=16.5Hz), 7.22-2.41(5H,m), 7.53-7.68(6H,m), 8.15(1H,s), 10.84(1H,s).

### Example 323: Preparation of the compound of Compound No. 323.

The title compound was obtained in the same manner as the Example 2(2) using the following raw materials.
Raw materials: 3-hydroxy-4-methoxybenzaldehyde and
3-(3,4-dichlorobenzyl)thiazolidine-2,4-dione(compound of the Example 8(1)).
Yield: 76.7%(light yellow crystal).
¹ H-NMR(CDCIs): *δ* 3.96(3H,s), 4.83(2H,s), 5.70(1H,s), 6.93(1H,d,J=8.1Hz), 7.04-7.09(2H,m), 7.28(1H,dd,J=8.1,1.8Hz), 7.40(1H,d,J=8.1Hz), 7.54(1H,d,J=2.1Hz), 7.82(1H,s).

### Example 324: Preparation of the compound of Compound No. 324.

The title compound was obtained in the same manner as the Example 2(2) using the following raw materials.
Raw materials: 3-hydroxy-4,5-methylenedioxybenzaldehyde and 3-(3,4-dichlorobenzyl)thiazolidine-2,4-dione(compound of the Example 8(1)).
Yield: 4.9%(light yellow crystal).
¹H-NMR(CDCl₃): *δ* 4.81(2H,s), 5.83(1H,s), 6.09(2H,s), 6.77(1H,d,J=1.5Hz), 6.96(1H,d,J=1.8Hz), 7.26-7.28(1H,m), 7.40(1H,d,J=8.1Hz), 7.52(1H,d,J=2.1Hz), 7.77(1H,s).

### Example 325: Preparation of the compound of Compound No. 325.

The title compound was obtained in the same manner as the Example 2(2) using the following raw materials.
Raw materials: 2-fluoro-3-hydroxybenzaldehyde and
3-(3,4-dichlorobenzyl)thiazolidine-2,4-dione(compound of the Example 8(1)).
Yield: 44.9%(white crystal).
¹H-NMR(DMSO-d₆): δ 4.84(2H,s), 6.98(1H,td,J=7.5,2.1Hz), 7.08-7.21(2H,m), 7.32(1H,dd,J=8.1,2.1Hz), 7.62(1H,d,J=8.4Hz), 7.63(1H,d,J=1.8Hz), 7.93(1H,s), 10.30(1H,s).

### Example 326: Preparation of the compound of Compound No. 326.

The title compound was obtained in the same manner as the Example 2(2) using the following raw materials.
Raw materials: 3-hydroxy-4-fluorobenzaldehyde and
3-(3,4-dichlorobenzyl)thiazolidine-2,4-dione(compound of the Example 8(1)).
Yield: 76.9%(white crystal).
¹H-NMR(DMSO-d₆): δ 4.84(2H,s), 7.10-7.15(1H,m), 7.23(1H,dd,J=8.7,2.4Hz), 7.29-7.36(2H,m), 7.62(1H,d,J=2.1Hz), 7.62(1H,d,J=8.1Hz), 7.87(1H,s), 10.38(1H,s).

### Example 327: Preparation of the compound of Compound No. 327.

The title compound was obtained in the same manner as the Example 2(2) using the following raw materials.
Raw materials: 3-methoxy-4-chlorobenzaldehyde and
3-(3,4-dichlorobenzyl)thiazolidine-2,4-dione(compound of the Example 8(1)).
Yield: 60.7%(light yellow crystal).
¹H-NMR(DMSO-d₆): δ 3.92(3H,s), 4.85(2H,s), 7.20(1H,dd,J=8.4,2.4Hz), 7.31(1H,dd,J=8.4,2.4Hz), 7.43(1H,d,J=1.8Hz), 7.60-7.64(3H,m), 7.98(1H,s).

### Example 328: Preparation of the compound of Compound No. 328.

The title compound was obtained in the same manner as the Example 308(3) using the following raw material.
Raw material: 5-(3-methoxy-4-chlorobenzylidene)-3-(3,4-dichlorobenzyl)-thiazolidine-2,4-dione(Compound No. 327).
Yield: 87.7%(yellow crystal).
¹H-NMR(DMSO-d₆):δ 4.83(2H,s), 7.11(1H,dd,J=8.4,1.8Hz), 7.21(1H,d,J=2.1Hz), 7.31(1H,dd,J=8.4,2.1Hz), 7.51(1H,d,J=8.1Hz), 7.62(1H,d,J=8.1Hz), 7.63(1H,d,J=2.1Hz), 7.86(1H,s), 10.68(1H,s).

### Example 329: Preparation of the compound of Compound No. 329.

The title compound was obtained in the same manner as the Example 2(2) using the following raw materials.
Raw materials: 2-methoxy-3-hydroxybenzaldehyde and
3-(3,4-dichlorobenzyl)thiazolidine-2,4-dione(compound of the Example 8(1)).
Yield: 82.9%(yellow crystal).
¹H-NMR(DMSO·d₆): *δ* 3.80(3H,s), 4.84(2H,s), 6.92-6.97(1H,m), 7.01-7.13(2H,m), 7.31(1H,dd,J=8.4,2.1Hz), 7.62(1H,d,J=8.4Hz), 7.63(1H,d,J=2.1Hz), 8.05(1H,s), 9.86(1H,s).

### Example 330: Preparation of the compound of Compound No. 330.

The title compound was obtained in the same manner as the Example 2(2) using the following raw materials.
Raw materials: 3-isopropyl-4-methoxybenzaldehyde and
3-(3,4-dichlorobenzyl)thiazolidine-2,4-dione(compound of the Example 8(1)).
Yield: 97.9%(light yellow crystal).
¹H-NMR(DMSO-d₆):δ 1.19(6H,d,J=6.9Hz), 3.26(1H,m), 3.88(3H,s), 4.84(2H,s), 7.15(1H,d,J=8.4Hz), 7.30(1H,dd,J=8.4,2.1Hz), 7.49-7.52(2H,m), 7.61(1H,d,J=2.4Hz), 7.62(1H,d,J=8.1Hz), 7.94(1H,s).

### Example 331: Preparation of the compound of Compound No. 331.

The title compound was obtained in the same manner as the Example 2(2) using the following raw materials.
Raw materials: 3-fluoro-4-methoxybenzaldehyde and
3-(3,4-dichlorobenzyl)thiazolidine-2,4-dione(compound of the Example 8(1)).
Yield: 77.3%(ocherous crystal).
¹H-NMR(CDCl₃): *δ* 3.95(3H,s), 4.83(2H,s), 7.05(1H,t, J=8.1Hz), 7.22-7.30(3H,m), 7.41(1H,d,J=8.4Hz), 7.54(1H,d,J=2.1Hz), 7.81(1H,s).

### Example 332: Preparation of the compound of Compound No. 332.

The title compound was obtained in the same manner as the Example 308(3) using the following raw material.
Raw material: 5-(3-fluoro-4-methoxybenzylidene)-3-(3,4-dichlorobenzyl)-thiazolidine-2,4-dione(Compound No. 331).
Yield: 50.3%(white crystal).
¹H-NMR(CDCl₆): *δ* 4.83(2H,s), 5.68(1H,s), 7.10(1H,t,J=8.4Hz), 7.21-7.30(3H,m), 7.41(1H,d,J=8.4Hz), 7.54(1H,d,J=2.1Hz), 7.80(1H,s).

### Example 333: Preparation of the compound of Compound No. 333.

The title compound was obtained in the same manner as the Example 2(2) using the following raw materials.
Raw materials: 3-trifluoromethyl-4-methoxybenzaldehyde and 3-(3,4-dichlorobenzyl)thiazolidine-2,4-dione(compound of the Example 8(1)).
Yield: 35.3%(light yellow crystal).
¹H-NMR(CDCl₃): *δ* 3.98(3H,s), 4.84(2H,s), 7.12(1H,d,J=9.0Hz), 7.28(1H,dd,J=8.4,2.1Hz), 7.41(1H,d,J=8.1Hz), 7.54(1H,d,J=2.4Hz), 7.65(1H,dd,J=8.7,2.4Hz), 7.72(1H,d,J=2.1Hz), 7.86(1H,s).

### Example 334: Preparation of the compound of Compound No. 334.

L-(+)-Arginine(89 mg, 0.510 mmol) was added portionwise to 5-(3,4,5-trihydroxybenzylidene)-3-(3,4-dichlorobenzyl)thiazolidine-2,4-dione(Compoun d No. 10; 70mg, 0.170mmol) and the compounds were mixed in the morter. The mixture was washed with ethyl acetate under suspension to give the title compound(114mg, 71.8%) as a reddish orange solid.
¹H-NMR(D₂O): *δ* 1.65-1.84(12H,m), 3.25-3.30(6H,m), 3.42-3.47(3H,m), 4.87(2H,s), 6.69(2H,s), 7.34(1H,dd,J=1.8,8.1Hz), 7.51-7.54(2H,m), 7.73(1H,s)

### Example 335: Preparation of the compound of Compound No. 335.

L-(+)-Arginine(97 mg, 0.555 mmol) was added portionwise to 5-(3,4-dihydroxybenzylidene)-3-(2,4-dichlorobenzyl)thiazolidine-2,4-dione(Compound No. 23; 110mg, 0.278mmol) and the compounds were mixed in the morter. The mixture was washed with dichloromethane under suspension to give the title compound(140mg, 67.7%) as a red solid.
¹H-NMR(CD₃OD): *δ* 1.60-1.79(8H,m), 3.14-3.21(4H,m), 3.29-3.34(2H,m), 4.95(2H,s), 6.75(1H, d, J=8.1Hz), 6.93-6.99(2H,m), 7.17(1H,d,J=8.7Hz), 7.30(1H,dd,J=8.1,2.1Hz), 7.48(1H,d,J=2.1Hz), 7.77(1H,s).

### Example 336: Preparation of the compound of Compound No. 336.

The title compound was obtained in the same manner as the Example 2(2) using the following raw materials.
Raw materials: 3,4-methylenedioxybenzaldehyde and
3-(2,4-dichlorobenzyl)thiazolidine-2,4-dione(compound of the Example 22(1)).
Yield: 50.2%(light yellowish white powder).
¹H-NMR(DMSO-d₆):δ 4.88(2H,s), 6.15(2H,s), 7.12(1H,d,J=8.1Hz), 7.20-7.24(2H,m), 7.31(1H,d,J=8.4Hz), 7.41(1H,dd,J=8.4,1.8Hz), 7.68(1H,d,J=2.4Hz), 7.91(1H,s).

### Example 337: Preparation of the compound of Compound No. 337.

Ethyl chloroformate(80 *µ* 1, 0.84mmol) and triethylamine(160 *µ* 1, 1.15mmol) were added to a solution of 5-(3,4-dihydroxybenzylidene)-3-(2,4-dichlorobenzyl)thiazolidine-2,4-dione(Compound No. 23; 153.1mg, 0.89mmol) in tetrahydrofuran(3ml) under ice cooling, and the mixture was stirred at room temperature for 2 hours. The reaction mixture was poured into diluted hydrochloric acid and extracted with ethyl acetate. The organic layer was washed with water and brine, and dried over anhydrous sodium sulfate. The residue obtained by evaporation of the solvent under reduced pressure was purified by chromatography on silica gel(hexane:ethyl acetate=2:1) to give the title compound(186mg, 89.1%) as a light yellowish white powder.
¹H-NMR(CDCl₃):*δ* 1.40(3H,t,J=7.2Hz), 1.40(3H,t,J=7,2Hz), 4.35(2H,q,J=7.2Hz), 4.35(2H,q,J=7.2Hz), 5.01(2H,s), 7 .16(1H,d,J =8. 7Hz), 7:23(1H,dd,J=8.7,2.4Hz), 7.42-7.47(4H,m), 7.86(1H,s).

### Example 338: Preparation of the compound of Compound No. 338.

5-(3,4,5-Trihydroxybenzylidene)-3-(3,4-dichlorobenzyl)thiazolidine-2,4-dione( Compound No. 10; 50mg, 0.12mmol) was dissolved in a solution of sodium hydroxide(9.7mg, 0.24mmol) in methanol(0.5ml). The residue obtained by evaporation of the solvent under reduced pressure was crystallized by addition of isopropyl ether. The crystal obtained by evaporation of isopropyl ether under reduced pressure was dried under reduced pressure to give the title compound(57mg) as a blackish brown crystal.
mp 241°C(decomp.).

### Example 339: Preparation of the compound of Compound No. 339.

The title compound was obtained in the same manner as the Example 338 using the following raw material.
Raw material: 5-(3,4-dihydroxybenzylidene)-3-(2,4-dichlorobenzyl)thiazolidine-2,4-dione(Compound No. 23).
mp 202°C(decomp.).

### Example 340: Preparation of the compound of Compound No. 340.

The title compound was obtained in the same manner as the Example 337 using the following raw materials.
Raw materials: 5-(3,4,5-trihydroxybenzylidene)-3-(3,4-dichlorobenzyl)thiazolidine-2,4-dione(Compound No. 10) and ethyl chloroformate.
Yield: 94.4%(white crystal).
¹H-NMR(DMSO-d₆): *δ* 1.28(3H,t,J=7.2Hz), 1.30(6H,t,J=7.2Hz), 4.30(2H,q,J=7.2Hz), 4.30(6H,q,J=7.2Hz), 4.85(2H,s), 7.32(1H,dd,J=8.4,2.1Hz), 7.62(1H,d,J=8.1Hz), 7.63(1H,d,J=2.4Hz), 7.70(2H,s), 7.94(1H,s).

### Example 341: Preparation of the compound of Compound No. 341.

(1) 3-Methoxy-4-hydroxy-5-carboxybenzaldehyde.
   A mixture of 3-methoxysalicylic acid(1.00 g, 5.95 mmol), hexamethylenetetramine(4.3 g, 30.7 mmol) and trifluoroacetic acid(20ml) was stirred at 90°C for 8 hours. The reaction mixture was cooled, poured into ice and water, and extracted with ethyl acetate. The organic layer was washed with water and brine, and dried over anhydrous sodium sulfate. The residue obtained by evaporation of the solvent under reduced pressure was washed with isopropyl ether/hexane under suspension to give the title compound(777.5 mg, 66.6%) as an orangish white powder.
   ¹H-NMR(DMSO-d₆):δ 3.88(3H,s), 7.55(1H,d,J=1.8Hz), 8.00(1H,d,J=2.1Hz), 9.85(1H,s).
(2) 3-Methoxy-4-hydroxy-5-(methoxycarbonyl)benzaldehyde.
   A mixture of 3-methoxy-4-hydroxy-5-carboxybenzaldehyde(0.70 g, 3.56 mmol), methanol(10 ml), concentrated sulfuric acid(0.3 ml), hexamethylenetetramine(4.3g, 30.7 mmol) and trifluoroacetic acid(20 ml) was refluxed for 24 hours. The reaction mixture was cooled, poured into ice and water, and extracted with ethyl acetate. The organic layer was washed with water and brine, and dried over anhydrous sodium sulfate. The residue obtained by evaporation of the solvent under reduced pressure was washed with ethyl acetate/isopropyl ether to give the title compound(699.6mg, 93.3%) as an orangish white powder.
   ¹H-NMR(DMSO-d₆): *δ* 3.90(3H,s), 3.93(3H,s), 7.58(1H,d,J=1.8Hz), 7.98(1H,d,J=1.8Hz), 9.87(1H,s), 11.12(1H,s).
(3) 5-[3-Methoxy-4-hydroxy-5-(methoxycarbonyl)benzylidene]-3-(2,4-dichlorobenzyl)thiazolidine-2,4-dione.
   The title compound was obtained in the same manner as the Example 2(2) using the following raw materials.
   Raw materials: 3-methoxy-4-hydroxy-5-(methoxycarbonyl)benzaldehyde and 3-(2,4-dichlorobenzyl)thiazolidine-2,4-dione(compound of the Example 22(1)).
   Yield: 66.7%(yellow powder).
   ¹H-NMR(CDCl₃): *δ* 3.96(3H,s), 4.02(3H,s), 5.01(2H,s), 7.14-7.16(2H,m), 7.23(1H,d,J=2.1Hz), 7.42(1H,d,J=1.8Hz), 7.68(1H,d,J=1.8Hz), 7.84(1H,s), 11.43(1H,s).
(4) 5-[3,4-Dihydroxy-5-(methoxycarbonyl)benzylidene]-3-(2,4-dichlorobenzyl)thiazolidine-2,4-dione (Compound No. 341).
   Boron tribromide(1M solution in dichloromethane; 1ml, 1mmol) was added to a solution of 5-[3-methoxy-4-hydroxy-5-(methoxycarbonyl)-benzylidenel-3-(2,4-dichlorobenzyl)thiazolidine-2,4-dione(145.5 mg, 0.31 mmol) in dichloromethane(3 ml) under ice cooling, and the mixture was stirred at room temperature for 5 hours. The reaction mixture was poured into ice and water, and extracted with ethyl acetate. The organic layer was washed with brine and dried over anhydrous sodium sulfate. The residue obtained by evaporation of the solvent under reduced pressure was crystallized by ethyl acetate/isopropyl ether/hexane to give the title compound(65.7mg, 46.5%) as a yellowish white powder.
   ¹H-NMR(DMSO-d₆): δ 3.92(3H,s), 4.87(2H,s), 7.30(1H,d,J=2.4Hz), 7.32(1H,d,J=9.0Hz), 7.41(1H,dd,J=8.4,2.1Hz), 7.60(1H,d,J=2.4Hz), 7.68(1H,d,J=2.4Hz), 7.87(1H,s), 10.12(1H,s), 10.74(1H,s).

### Example 342: Preparation of the compound of Compound No. 342.

(1) Methyl 3,5-bis(methoxymethoxy)benzoate.
   Chloromethyl methyl ether(6.3 ml, 34 mmol) was added to a solution of methyl 3,5-dihydroxybenzoate(6.42 g, 38 mmol), and N,N-diisopropylethylamine(15.5 ml, 91 mmol) in dichloromethane(20 ml) under ice cooling, and the mixture was stirred at room temperature for 18 hours. The reaction mixture was poured into diluted hydrochloric acid and extracted with ethyl acetate. The organic layer was washed with water and brine, and dried over anhydrous sodium sulfate. The residue obtained by evaporation of the solvent under reduced pressure was purified by chromatography on silica gel(hexane:ethyl acetate=2:1) to give the title compound(6.35 g, 64.9%) as a colorless clear oil.
   ¹H-NMR(CDCl₃): *δ* 3.48(6H,s), 3.90(3H,s), 5.19(4H,s), 6.92(1H,t,J=2.1Hz), 7.37(2H,d,J=2.1Hz).
(2) 3,5-Bis(methoxymethoxy)benzylalcohol.
   Lithium aluminium hydride(0.97 g, 25 mmol) was added to a solution of methyl 3,5-bis(methoxymethoxy)benzoate(6.35 g, 25 mmol) in tetrahydrofuran(40 ml) under ice cooling and argon atmosphere, and the mixture was stirred for 15 minutes. Water was added portionwise to the reaction mixture. The mixture was filtered through celite and the filtrate was extracted with ethyl acetate. The organic layer was washed with water and brine, and dried over anhydrous sodium sulfate. The solvent was evaporated under reduced pressure to give the title compound(5.59g, 98.9%) as a colorless clear oil.
   ¹H.NMR(CDCl₃): *δ* 3.48(6H,s), 4.64(2H,s), 5.16(4H,s), 6.65(1H,t,J=2.4Hz), 6.72(2H,d,J=2.4Hz).
(3) 3,5-Bis(methoxymethoxy)benzyl tert-butyldimethylsilyl ether.
   Tert-butyldimethylsilyl chloride(4.06 g, 27 mmol) was added to a solution of 3,5-bis(methoxymethoxy)benzylalcohol(5.59 g, 25 mmol) and imidazole(3.34 g, 49 mmol) in tetrahydrofuran(40 ml) under ice cooling and argon atmosphere, and the mixture was stirred at room temperature for 1 hour. The reaction mixture was poured into diluted hydrochloric acid and extracted with ethyl acetate. The organic layer was washed with water and brine, and dried over anhydrous sodium sulfate. The residue obtained by evaporation of the solvent under reduced pressure was purified by chromatography on silica gel(hexane:ethyl acetate=4:1) to give the title compound(8.16g, 97.2%) as a colorless clear oil.
   ¹H-NMR(CDCl₃):δ 0.10(6H,s), 0.94(9H,s), 3.47(6H,s), 4.68(2H,d,J=0.6Hz), 5.15(4H,s), 6.61(1H,t,J=2.4Hz), 6.69(2H,dt,J=2.4,0.6Hz).
(4) 3,5-Bis(methoxymethoxy)-4-(methoxycarbonyl)benzyl tert-butyldimethylsilyl ether.
   n-Butyllithium(1.6M solution in hexane; 16.4 ml, 26 mmol) was added slowly to a solution of 3,5-bis(methoxymethoxy)benzyl tert-butyldimethylsilyl ether(8.16g, 24mmol) in tetrahydrofuran(60 ml) under ice cooling and argon atmosphere, and the mixture was stirred for 20 minutes. Methyl chloroformate(2.0ml, 26mmol) was added to the mixture, and the mixture was stirred at room temperature for 1 hour. The reaction mixture was poured into ice and water, and extracted with ethyl acetate. The organic layer was washed with water and brine, and dried over anhydrous sodium sulfate. The residue obtained by evaporation of the solvent under reduced pressure was purified by chromatography on silica gel(hexane:ethyl acetate=4:1→3:1) to give the title compound(4,30g, 45.0%) as a light yellow clear oil.
   ¹H-NMR(CDCl₃): *δ* 0.09(6H,s), 0.94(9H,s), 3.46(6H,s), 3.91(3H,s), 4.70(2H,t,J=0.6Hz), 5.16(4H,s), 6.81(2H,t,J=0.6Hz).
(5) 3,5-Bis(methoxymethoxy)-4-(methoxycarbonyl)benzylalcohol.
   Tetrabutylammonium fluoride(1.0M solution in tetrahydrofuran; 11 ml, 11 mmol) was added to a solution of 3,5-bis(methoxymethoxy)-4-(methoxycarbonyl)benzyl tert-butyldimethylsilyl ether(4.29 g, 10 mmol) in tetrahydrofuran(15ml), and the mixture was stirred at room temperature for 30 minutes. The reaction mixture was poured into water and extracted with ethyl acetate. The organic layer was washed with water and brine, and dried over anhydrous sodium sulfate. The residue obtained by evaporation of the solvent under reduced pressure was purified by chromatography on silica gel(hexane:ethyl acetate=1:1 → 1:2) to give the title compound(2.40 g, 78.0%) as a white crystal.
   ¹H-NMR(CDCl₃): *δ* 1.83(1H,brs), 3.47(6H,s), 3.91(3H,s), 4.65(2H,m), 5.20(4H,s), 6.82(2H,d,J=2.4Hz).
(6) 3,5-Bis(methoxymethoxy)-4-(methoxycarbonyl)benzylaldehyde.
   A solution of 3,5-bis(methoxymethoxy)-4-(methoxycarbonyl)benzylalcohol(2.40 g, 8.4 mmol) in dichloromethane(13 ml) was added to a suspension of pyridinium dichromate(4.1g, 11mmol) in dichloromethane(30ml), and the mixture was stirred at room temperature for 16 hours. The reaction mixture was filtered through Celite, and the residue obtained by concentration of the filtrate under reduced pressure was purified by chromatography on silica gel(hexane:ethyl acetate=1:1) to give the title compound(1.99g, 83.5%) as a yellow crystal.
   ¹H-NMR(CDCl₃): *δ* 3.48(6H,s), 3.95(3H,s), 5.25(4H,s), 7.34(2H,s), 9.93(1H,s).
(7) 5-[3,5-Bis(methoxymethoxy)-4-(methoxycarbonyl)benzylidene]-3-(2,4-dichlorobenzyl)thiazolidine-2,4-dione.
   The title compound was obtained in the same manner as the Example 2(2) using the following raw materials.
   Raw materials: 3,5-bis(methoxymethoxy)-4-(methoxycarbonyl)benzylaldehyde and 3-(2,4-dichlorobenzyl)thiazolidine-2,4-dione(compound of the Example 22(1)).
   Yield: 67.8%(light yellow powder).
   ¹H-NMR(CDCl₃): *δ* 3.49(6H,s), 3.94(3H,s), 5.00(2H,s), 5.21(4H,s), 7.02(2H,s), 7.14(1H,d,J=8.4Hz), 7.22(1H,dd,J=8.4,2.1Hz), 7.42(1H,d,J=1.8Hz), 7.84(1H,s).
(8) 5-[3,5-Dihydroxy-4-(methoxycarbonyl)benzylidene]-3-(2,4-dichlorobenzyl)thiazolidine-2,4-dione (Compound No. 342).
   A mixture of 5-[3,5-bis(methoxymethoxy)-4-(methoxycarbonyl)benzylidene]-3-(2,4-dichlorobenzyl)thiazolidine-2,4-dione(129.4 mg, 0.24 mmol), methanol(2 ml) and concentrated hydrochloric acid(1 ml) was refluxed for 2 hours under argon atmosphere. The reaction mixture was cooled. Isopropyl ether was added to the mixture and the separated solid was washed under suspension to give the title compound(78.4 mg, 72.5%) as a yellow powder.
   ¹H-NMR(DMSO-d₆): *δ* 3.77(3H,s), 4.87(2H,s), 6.63(2H,s), 7.35(1H,d,J=8.4Hz), 7.41(1H,dd,J=8.4,2.1Hz), 7.68(1H,d,J=1.8Hz), 7.75(1H,s), 10.29(2H,s).

### Example 343: Preparation of the compound of Compound No. 343.

The title compound was obtained in the same manner as the Example 2(2) using the following raw materials.
Raw materials: 3-methoxy-4-hydroxy-5-(methoxycarbonyl)benzaldehyde(compound of the Example 341(2)) and 3-(3,4-dichlorobenzyl)thiazolidine-2,4-dione(compound of the Example 8(1)).
Yield: 7.5%(yellowish white powder).
¹H-NMR(CDCl₃): *δ* 3.96(3H,s), 4.01(3H,s), 4.84(2H,s), 7.13(1H,d,J=2.1Hz), 7.29(1H,dd,J=8.4,2.1Hz), 7.4l(1H,d,J=8.1Hz), 7.54(1H,d,J=2.1Hz), 7.66(1H,d,J=1.8Hz), 7.83(1H,s), 11.42(1H,s).

### Example 344: Preparation of the compound of Compound No. 344.

The title compound was obtained in the same manner as the Example 341(4) using the following raw material.
Raw material: 5-[3-methoxy-4-hydroxy-5-(methoxycarbonyl)benzylidene]-3-(3,4-dichlorobenzyl)thiazolidine-2,4-dione(Compound No. 343).
Yield: 71.8%(yellowish white powder).
¹H-NMR(DMSO-d₆): *δ* 3.91(3H,s), 4.83(2H,s), 7.28-7.32(2H,m), 7.58-7.63(3H,m), 7.86(1H,s), 1O.10(1H,s), 10.73(1H,s).

### Example 345: Preparation of the compound of Compound No. 345.

(1) 3-Methoxy-4-hydroxy-5-(tert-butoxycarbonyl)benzaldehyde.
   Tert-butanol(0.76g, 10.2 mmol), WSCI·HCl(1.95g, 10.2 mmol) and DMAP(20 mg) were added to a suspension of 3-methoxy-4-hydroxy-5-carboxybenzaldehyde (compound of the Example 341(1); 1.0g, 5.1 mmol) in tetrahydrofuran(20 ml), and the mixture was stirred at room temperature for 18 hours. The reaction mixture was poured into water and extracted with ethyl acetate. The organic layer was washed with water and brine, and dried over anhydrous sodium sulfate. The residue obtained by evaporation of the solvent under reduced pressure was purified by chromatography on silica gel(hexane:ethyl acetate=2:1) to give the title compound(77.5 mg, 6.0%) as a white crystal.
   ¹H-NMR(CDCl₃): *δ* 1.67(9H,s), 3.97(3H,s), 7.52(1H,d,J=1.8Hz), 7.89(1H,d,J=1.8Hz), 9.84(1H,s), 12.03(1H,s).
(2) 5-[3-Methoxy-4-hydroxy-5-(tert-butoxycarbonyl)benzylidene]-3-(3,4-dichlorobenzyl)thiazolidine-2,4-dione.
   The title compound was obtained in the same manner as the Example 2(2) using the following raw materials.
   Raw materials: 3-methoxy-4-hydroxy-5-(tert-butoxycarbonyl)benzaldehyde and 3-(3,4-dichlorobenzyl)thiazolidine-2,4-dione(compound of the Example 8(1)).
   Yield: 70.7%(yellow oil).
   ¹H-NMR(CDCl₃): *δ* 1.64(9H,s), 3.94(3H,s), 4.84(2H,s), 7.09(1H,d,J=1.8Hz), 7.28(1H,dd,J=7.8,1.8Hz), 7.41(1H,d,J=8.1Hz), 7.54(1H,d,J=2.1Hz), 7.60(1H,d,J=2.1Hz), 7.83(1H,s), 11.71(1H,s).
(3) 5-(3-Methoxy-4-hydroxy-5-carboxybenzlidene)-3-(3,4-dichlorobenzyl)thiazolidine-2,4-dione (Compound No. 345).
   Boron tribromide(1M solution in dichloromethane; 0.3 ml, 0.3 mmol) was added to a solution of 5-[3-methoxy-4-hydroxy-5-(tert-butoxycarbonyl)benzylidenel-3-(3,4-dichlorobenzyl)thiazolidine-2,4-dione(110.9 mg, 0.22 mmol) in dichloromethane(2 ml) under ice cooling, and the mixture was stirred at room temperature for 1 hour. The reaction mixture was poured into ice and water, and extracted with ethyl acetate. The organic layer was washed with brine and dried over anhydrous sodium sulfate. The residue obtained by evaporation of the solvent under reduced pressure was washed with isopropyl ether/hexane under suspension to give the title compound(83.7 mg, 85.0%) as a light yellowish green powder.
   ¹H-NMR(DMSO-d₆): *δ* 3.86(3H,s), 4.84(2H,s), 7.30(1H,dd,J=8.4,2.1Hz), 7.47(1H,d,J=1.8Hz), 7.61(1H,d,J=2.1Hz), 7.62(1H,d,J=8.1Hz), 7.66(1H,d,J=1.8Hz), 7.95(1H,s).

### Example 346: Preparation of the compound of Compound No. 346.

The title compound was obtained in the same manner as the Example 2(2) using the following raw materials.
Raw materials: 3-methoxy-4-hydroxy-5-nitrobenzaldehyde and 3-(3,4-dichlorobenzyl)thiazolidine-2,4-dione(compound of the Example 8(1)).
Yield: 82.4%(yellowish brown powder).
¹H.NMR(DMSO.d₆): *δ* 3.94(3H,s), 4.85(2H,s), 7.31(1H,dd,J=8.4,2.4Hz), 7.48(1H,d,J=2.1Hz), 7.62(1H,d,J=2.4Hz), 7.62(1H,d,J=8.4Hz), 7.75(1H,d,J=1.8Hz), 7.95(1H,s), 11.28(1H,br).

### Example 347: Preparation of the compound of Compound No. 347.

The title compound was obtained in the same manner as the Example 341(4) using the following raw material.
Raw material: 5-(3-methoxy-4-hydroxy-5-nitrobenzylidene)-3-(3,4-dichlorobenzyl)thiazolidine-2,4-dione(Compound No. 346).
Yield: 48.1%(yellowish brown powder).
¹H-NMR(DMSO-ds): δ 4.83(2H,s), 7.29-7.32(2H,m), 7.62(1H,d,J=2.1Hz), 7.62(1H,d,J=8.1Hz), 7.72(1H,d,J=2.1Hz), 7.89(1H,s), 10.85(2H,brs).

### Example 348: Preparation of the compound of Compound No. 348.

The title compound was obtained in the same manner as the Example 2(2) using the following raw materials.
Raw materials: 3,4,5-trimethoxybenzaldehyde and
3-(3,4-dichlorobenzyl)thiazolidine-2,4-dione(compound of the Example 8(1)).
Yield: 45.3%(yellowish white powder).
¹H-NMR(DMSO-ds): *δ* 3.74(3H,s), 3.84(6H,s), 4.85(2H,s), 6.96(2H,s), 7.31(1H,dd,J=8.4,1.8Hz), 7.62(1H,d,J=1.8Hz), 7.63(1H,d,J=8.1Hz), 7.92(1H,s).

### Example 349: Preparation of the compound of Compound No. 349.

(1) 5-{3-Methoxy-4-hydroxy-5-[3,4-bis(methoxymethoxy)phenylcarbamoyl]-benzylidene}-3-(3,4-dichlorobenzyl)thiazolidine-2,4-dione.
   WSCI(43.9 mg, 0.23 mmol) and DMAP(10 mg) were added to a solution of 5-(3-methoxy-4-hydroxy-5-carboxybenzlidene)-3-(3,4-dichlorobenzyl)thiazolidine-2,4-d ione(Compound No. 345; 51.6 mg, 0.11 mmol) and 3,4-bis(methoxymethoxy)aniline (24.8 mg, 0.12 mmol) in N-methylpyrrolidone(2 ml), and the mixture was stirred at room temperature for 18 hours. The reaction mixture was poured into diluted hydrochloric acid and extracted with ethyl acetate. The organic layer was washed with water and brine, and dried over anhydrous sodium sulfate. The residue obtained by evaporation of the solvent under reduced pressure was purified by chromatography on silica gel(hexane:ethyl acetate= 1:2- 1:3) to give the title compound(15.5 mg, 21.0%) as a yellow oil.
   ¹H-NMR(CDCl₃): *δ* 3.53(3H,s), 3.54(3H,s), 3.99(3H,s), 4.84(2H,s), 5.23(2H,s), 5.28(2H,s), 7.12(1H,d,J=1.8Hz), 7.19-7.20(2H,m), 7.28(1H,dd,J=8.4,1.BHz), 7.41(1H,d,J=8.1Hz), 7.49(1H,d,J=1.8Hz), 7.53-7.54(2H,m), 7.86(1H,s), 8.41(1H,s), 10.96(1H,brs).
(2) 5-[3-Methoxy-4-hydroxy-5-(3,4-dihydroxyphenylcarbamoyl)-benzylidene]-3-(3,4-dichlorobenzyl)thiazolidine-2,4-dione (Compound No. 349).
   A mixture of 5-{3-methoxy-4-hydroxy-5-[3,4-bis(methoxymethoxy)-phenylcarbamoyl]benzylidene}-3-(3,4-dichlorobenzyl)thiazolidine-2,4-dione(15.5 mg, 0.024 mmol), methanol(1 ml) and concentrated hydrochloric acid(0.05 ml) was refluxed for 1 hour. The reaction mixture was cooled. Isopropyl ether was added to the mixture and the separated solid was washed under suspension to give the title compound(7.6 mg, 57.1%) as a yellow solid.
   ¹H-NMR(DMSO-d₆): *δ* 3.90(3H,s), 4.85(2H,s), 6.71(1H,d,J=8.4Hz), 6.90(1H,dd,J=8.7,2.4Hz), 7.20(2H,d,J=2.1Hz), 7.29-7.34(2H,m), 7.62-7.64(2H,m), 7.81(1H,d,J=1.8Hz), 7.91(1H,s), 8.80(1H,brs), 9.05(1H,brs), 10.15(1H,s), 12.23(1H,brs).

### Example 350: Preparation of the compound of Compound No. 350.

(1) 3-(Tert-butoxycarbonyl)benzaldehyde.
   Di-tert-butyl dicarbonate(1134 mg, 5.195 mmol) was added to a solution of 3-formylbenzoic acid(600 mg, 3.996 mmol) and DMAP(98 mg, 0.799 mmol) in tetrahydrofuran(5 ml), and the mixture was stirred at room temperature overnight. Water was added to the reaction mixture, and the mixture was extracted with ethyl acetate. The organic layer was washed with brine and dried over anhydrous sodium sulfate. The residue obtained by evaporation of the solvent under reduced pressure was purified by chromatography on silica gel(hexane:ethyl acetate=3:1) to give the title compound(330 mg, 40.0%) as a colorless clear oil.
   ¹H-NMR(CDCl₃):*δ* 1.63(9H,s), 7.60(1H,t,J=7.5Hz), 8.04-8.47(3H,m), 10.08(1H,s).
(2) 5-[3-(Tert-butoxycarbonyl)benzylidene]-3-(3,4-dichlorobenzyl)-thiazolidine-2,4-dione.
   Acetic acid(2 drops) and piperidine(2 drops) were added to a mixture of 3-(tert-butoxycarbonyl)benzaldehyde(323 mg, 1.566 mmol), 3-(3,4-dichlorobenzyl)thiazolidine-2,4-dione (compound of the Example 8(1); 432 mg, 1.566 mmol) and toluene(5 ml), and the mixture was refluxed for 1 hour. The residue obtained by concentration of the reaction mixture under reduced pressure was purified by chromatography on silica gel(hexane:ethyl acetate=3:1) to give the title compound(500 mg, 78.2%) as a white solid.
   ¹H-NMR(DMSO-d₆): *δ* 1.58(9H,s), 4.86(2H,s), 7.32(1H,dd,J=1.8,8.1Hz), 7.61-7.71(3H,m), 7.90(1H,d,J=8.1Hz), 7.99(1H,d,J=7.8Hz), 8.06(1H,s), 8.16(1H,s).
(3) 5-(3-Carboxybenzylidene)-3-(3,4-dichlorobenzyl)thiazolidine-2,4-dione (Compound No. 350).
   A mixture of 5-[3-(tert-butoxycarbonyl)benzylidene]-3-(3,4-dichlorobenzyl)-thiazolidine-2,4-dione(500 mg, 1.077 mmol) and formic acid(3 ml) was refluxed for 1 hour. The reaction mixture was cooled. Water was added to the mixture and the separated solid was collected by filtration to give the title compound(424 mg, 96.5%) as a yellow solid.
   ¹H-NMR(DMSO-d₆):δ 4.85(2H,s), 7.32(1H,dd,J=2.1,8.4Hz), 7.62(1H,d,J=7.4Hz), 7.63(1H,s), 7.68(1H,t,J=7.5Hz), 7.87-7.91(1H,m), 8.02-8.06(2H,m), 8.17-8.19(1H,m), 13.32(1H,brs).

### Example 351: Preparation of the compound of Compound No. 351.

(1) Methyl 2-methoxy-5-formylbenzoate.
   A mixture of 5-formylsalicylic acid(2.391 g, 14.39 mmol), methyl iodide(8.17g, 57.57 mmol), potassium carbonate(7.96 g, 57.57 mmol) and N,N-dimethylformamide(20 ml) was stirred at 45°C for 1 week. The reaction mixture was poured into water and the separated solid was collected by filtration to give the title compound(1.760g, 63.0%) as a white solid.
   ¹H-NMR(CDCl₃):δ 3.93(3H,s), 4.01(3H,s), 7.12(1H,d,J=8.7Hz), . 8.03(1H,dd,J=8.7,2.1Hz), 8.34(1H,d,J=2.1Hz), 9.93(1H,s).
(2) 5-(3-Methoxycarbonyl-4-methoxybenzylidene)-3-(3,4-dichlorobenzyl)thiazolidine-2,4-dione.
   The title compound was obtained in the same manner as the Example 2(2) using the following raw materials.
   Raw materials: methyl 2-methoxy-5-formylbenzoate and
   3-(3,4-dichlorobenzyl)thiazolidine-2,4-dione(compound of the Example 8(1)).
   Yield: 90.7%.
   ¹H-NMR(DMSO-d₆): *δ* 3.83(3H,s), 3.91(3H,s), 4.84(2H,s), 7.31(1H,dd,J=8.7,2.4Hz), 7.36(1H,d,J=9.0Hz), 7.62(1H,d,J=2.4Hz), 7.62(1H,d,J=8.4Hz), 7.84(1H,dd,J=9.0,2.4Hz), 7.95(1H,d,J=2.7Hz), 7.97(1H,s).
(3) 5-(3-Carboxy-4-hydroxybenzylidene)-3-(3,4-dichlorobenzyl)thiazolidine-2,4-dione.
   The title compound was obtained in the same manner as the Example 341(4) using the following raw material.
   Raw material: 5-(3-methoXycarbonyl-4-methoxybenzylidene)-3-(3,4-dichlorobenzyl)thiazolidine-2,4-dione.
   Yield: 100%(white crystal).
   ¹H-NMR(DMSO-d₆): δ 4.84(2H,s), 7.13(1H,d,J=8.7Hz), 7.30(1H,dd,J=8.4,2.1Hz), 7.62(1H,d,J=1.8Hz), 7.62(1H,d,J=8.1Hz), 7.79(1H,dd,J=8.7,2.4Hz), 7.96(1H,s), 8.09(1H,d,J=2.1Hz).
(4) 5-[4-Hydroxy-3-(3,4-dimethoxyphenylcarbamoyl)benzylidene]-3-(3,4-dichlorobenzyl)thiazolidine-2,4-dione (Compound No. 351).
   The title compound was obtained in the same manner as the following Example 357 using the following raw materials.
   Raw materials: 5-(3-carboxy-4-hydroxybenzylidene)-3-(3,4-dichlorobenzyl)-thiazolidine-2,4-dione and 3,4-dimethoxyaniline.
   Yield: 95.8%(light yellow crystal).
   ¹H-NMR(DMSO-d₆): *δ* 3.74(3H,s), 3.77(3H,s), 4.84(2H,s), 6.90-6.95(2H,m), 7.19(1H,dd,J=8.7,2.1Hz), 7.30(1H,dd,J=8.7,2.4Hz), 7.46(1H,d,J=2.1Hz), 7.54(1H,dd,J=8.7,2.1Hz), 7.61(1H,s), 7.62(1H,d,J=8.4Hz), 7.86(1H,s), 8.16(1H,d,J=2.1Hz), 11.60(1H,brs).

### Example 352: Preparation of the compound of Compound No. 352.

The title compound was obtained in the same manner as the Example 341(4) using the following raw material.
Raw material: 5-[4-hydroxy-3-(3,4-dimethoxyphenylcarbamoyl)benzylidene]-3-(3,4-dichlorobenzyl)thiazolidine-2,4-dione(Compound No. 351).
Yield: 31.6%(yellow crystal).
¹H-NMR(DMSO-d₆): *δ* 4.85(2H,s), 6.71(1H,d,J=8.4Hz), 6.90(1H,dd,J=8.4,2.7Hz), 7.14(1H,d,J=8.4Hz), 7.24(1H,d,J=2.1Hz), 7.31(1H,dd,J=8.4,1.8Hz), 7.61-7.68(3H,m), 7.91(1H,s), 8.20(1H,d,J=2.1Hz), 8.78(1H,s), 9.05(1H,s), 10.13(1H,s), 12.37(1H,brs).

### Example 353: Preparation of the compound of Compound No. 353.

The title compound was obtained in the same manner as the following Example 357 using the following raw materials.
Raw materials: 5-(3-carboxy-4-hydroxybenzylidene)-3-(3,4-dichlorobenzyl)-thiazolidine-2,4-dione(compound of the Example 351(3)) and 4-methoxyaniline.
Yield: 60.2%(light yellow crystal).
¹H-NMR(DMSO-d₆): *δ* 3.76(3H,s), 4.85(2H,s), 6.96(1H,d, J=9.0Hz), 7.16(1H,d,J=8.7Hz), 7.31(1H,dd, J=8.4,2.1Hz), 7.61-7.64(4H,m), 7.68(1H,dd, J=8.4,2.1Hz), 7.92(1H,s), 8.20(1H,d, J=2.1Hz), 10.30(1H,s), 12.22(1H,brs).

### Example 354: Preparation of the compound of Compound No. 354.

The title compound was obtained in the same manner as the Example 341(4) using the following raw material.
Raw material: 5-[4-hydroxy-3-(4-methoxyphenylcarbamoyl)benzylidene]-3-(3,4-dichlorobenzyl)thiazolidine-2,4-dione(Compound No. 353).
Yield: 36.0%(yellow crystal).
¹H-NMR(DMSO-d₆): δ 4.84(2H,s), 6.76(2H,d,J=8.4Hz), 7.12(1H,d,J=8.4Hz), 7.28-7.32(1H,m), 7.48(2H,d,J=8.7Hz), 7.61-7.68(3H,m), 7.90(1H,s), 8.20(1H,d,J=1.5Hz), 9.34(1H,s), 10.33(1H,brs), 12.38(1H,brs).

### Example 355: Preparation of the compound of Compound No. 355.

The title compound was obtained in the same manner as the following Example 357 using the following raw materials.
Raw materials: 5-(3-carboxy-4-hydroxybenzylidene)-3-(3,4-dichlorobenzyl)-thiazolidine-2,4-dione(compound of the Example 351(3)) and 3-methoxyaniline.
Yield: 56.5%(light yellow crystal).
¹H-NMR(DMSO-d₆): *δ* 3.77(3H,s), 4.85(2H,s), 6.73(1H,td,J=6.6,2.4Hz), 7.17(1H,d,J=8.7Hz), 7.26-7.33(3H,m), 7.43(1H,s), 7.62(1H,d,J=2.1Hz), 7.63(1H,d,J=8.1Hz), 7.69(1H,dd,J=8.4,2.1Hz), 7.93(1H,s), 8.15(1H,d,J=2.1Hz), 10.35(1H,s), 12.00(1H,br).

### Example 356: Preparation of the compound of Compound No. 356.

The title compound was obtained in the same manner as the Example 341(4) using the following raw material.
Raw material: 5-[4-hydroxy-3-(3-methoxyphenylcarbamoyl)benzylidene]-3-(3,4-dichlorobenzyl)thiazolidine-2,4-dione(Compound No. 355).
Yield: 51.4%(yellow crystal).
¹H-NMR(DMSO-d₆):δ 4.84(2H,s), 6.52-6.55(1H,m), 7.05-7.17(3H,m), 7.28-7.32(2H,m), 7.61-7.64(2H,m), 7.66(1H,dd,J=8.4,2.1Hz), 7.92(1H,s), 8.15(1H,d,J=2.1Hz), 9.47(1H,s), 10.41(1H,brs), 12.14(1H,brs).

### Example 357: Preparation of the compound of Compound No. 357.

Phosphorus trichloride(11 mg, 0.082 mmol) was added to a mixture of 5-(3-carboxybenzylidene)-3-(3,4-dichlorobenzyl)thiazolidine-2,4-dione(Compound No. 350; 67 mg, 0.164 mmol), 3,4-dimethoxyaniline(25 mg, 0.164 mmol) and toluene(1.5 ml), and the mixture was refluxed at 150°C for 2 hours. The reaction mixture was cooled and water was added to the mixture. The separated solid was collected by filtration, washed with methanol under suspension to give the title compound(45 mg, 50.5%) as a light yellow solid.
¹H-NMR(DMSO-d₆):δ 3.75(3H,s), 3.77(3H,s), 4.83(2H,s), 6.95(1H,d,J=9.0Hz), 7.36(2H,dt,J=8.7,1.5Hz), 7.47(1H,d,J=2.1Hz), 7.61-7.65(2H,m), 7.71(1H,t,J=7.8Hz), 7.81-7.84(1H,m), 8.01-8.07(1H,m), 8.18(1H,s), 10.23(1H,s).

### Example 358: Preparation of the compound of Compound No. 358.

This compound is identical with the compound prepared in the Example 351(3).

### Example 359: Preparation of the compound of Compound No. 359.

(1) 5-(3-Nitro-4-methoxybenzylidene)-3-(3,4-dichlorobenzyl)thiazolidine-2,4-dione.
   Piperidine(0.1 ml), acetic acid(0.1 ml) and 3-nitro-4-methoxybenzaldehyde(0.988g, 5.45 mmol) were added to a solution of 3-(3,4-dichlorobenzyl)thiazolidine-2,4-dione (compound of the Example 8(1); 1.506g, 5.45 mmol) in toluene(7.0 ml), and the mixture was refluxed for 7 hours. The reaction mixture was cooled and poured into ethyl acetate. The separated solid was collected by filtration and washed with hexane to give the title compound(1.6157g, 66.6%) as a light yellow crystal.
   ¹ H-NMR(DMSO-d₆): *δ* 4.01(3H,s), 4.85(2H,s), 7.30-7.33(1H,m), 7.55(1H,d,J=8.7Hz), 7.61-7.64(2H,m), 7.91(1H,dd,J=8.7,2.1Hz), 8.00(1H,s), 8.21(1H,d,J=2.1Hz).
(2) 5-(3-Amino-4-methoxybenzylidene)-3-(3,4-dichlorobenzyl)thiazolidine-2,4-dione.
   Iron powder(76.3 mg, 1.366 mmol) and concentrated hydrochloric acid(285 mg, 2.732 mmol) were added to a solution of 5-(3-nitro-4-methoxybenzylidene)-3-(3,4-dichlorobenzyl)thiazolidine-2,4-dione(200 mg, 0.455 mmol) in ethanol(3.0 ml). The mixture was heated gradually and refluxed for 3 hours. The reaction mixture was extracted with ethyl acetate. The organic layer was washed with water and filtered. The residue obtained by evaporation of the solvent under reduced pressure was washed with ethyl acetate/isopropyl ether under suspension to give the title compound(50 mg, 26.9%) as a light yellow crystal.
   ¹H-NMR(DMSO-d₆): *δ* 3.84(3H,s), 4.82(2H,s), 5.10(2H,s), 6.88-6.97(3H,m), 7.29(1H,dd,J=8.1,1.8Hz), 7.60-7.64(2H,m), 7.75(1H,s).
(3) 5-[4-Methoxy-3-(3,4-dihydroxybenzoylamino)benzylidene]-3-(3,4-dichlorobenzyl)thiazolidine-2,4-dione (Compound No. 359).
   The title compound was obtained in the same manner as the Example 357 using the following raw materials.
   Raw materials: 5-(3-amino-4-methoxybenzylidene)-3-(3,4-dichlorobenzyl)-thiazolidine-2,4-dione and 3,4-dihydroxybenzoic acid.
   Yield: 60.0%(yellow crystal).
   ¹H-NMR(DMSO-d₆): *δ* 3.94(3H,s), 4.84(2H,s), 6.84(1H,d,J=7.8Hz), 7.25-7.38(4H,m), 7.50(1H,dd,J=8.4,2.4Hz), 7.62(1H,d,J=8.4Hz), 7.63(1H,d,J=2.1Hz), 7.92(1H,s), 8.31(1H,d,J=2.1Hz), 9.15(1H,s), 9.30(1H,s), 9.65(1H,s).

### Example 360: Preparation of the compound of Compound No. 360.

The title compound was obtained in the same manner as the Example 357 using the following raw materials.
Raw materials: 5-(3-methoxy-4-hydroxy-5-carboxybenzlidene)-3-(3,4-dichlorobenzyl)thiazolidine-2,4-dione(Compound No. 345) and 3-trifluoromethyl-4-methoxyaniline.
Yield: 4.9%(yellow solid).
¹H-NMR(DMSO-d₆): *δ* 3.89(3H,s), 3.92(3H,s), 4.86(2H,s), 7.28-7.39(3H,m), 7.62(1H,s), 7.63(1H,d,J=8.1Hz), 7.71-7.76(1H,m), 7.86-8.08(3H,m), 10.49(1H,s).

### Example 361: Preparation of the compound of Compound No. 361.

The title compound was obtained in the same manner as the Example 357 using the following raw materials.
Raw materials: 5-(3-methoxy-4-hydroxy-5-carboxybenzlidene)-3-(3,4-dichlorobenzyl)thiazolidine-2,4-dione(Compound No. 345) and 3-methyl-4-hydroxyaniline.
Yield: 99.0%(yellow solid).
¹H-NMR(DMSO-d₆): *δ* 2.14(3H,s), 3.90(3H,s), 4.85(2H,s), 6.77(1H,d,J=8.4Hz), 7.27-7.37(4H,m), 7.61(1H,s), 7.62(1H,d,J=8.4Hz), 7.83(1H,d,J=1.8Hz), 7.89(1H,s), 9.25(1H,s), 1O.22(1H,s), 12.34(1H,brs).

### Example 362: Preparation of the compound of Compound No. 362.

(1) 5-Formylsalicylic acid 4-methoxybenzyl ester.
   Diethyl azodicarboxylate(40% solution in toluene, d=1.113; 2.8 ml, 7.223 mmol) was added slowly to a mixture of 5-formylsalicylic acid(1.2 g, 7.223 mmol), 4-methoxybenzylalcohol(998 mg, 7.223 mmol), triphenylphosphine(1.895g, 7.223 mmol) and tetrahydrofuran(15 ml) at 0°C, and the mixture was stirred at room temperature for 1 hour. The residue obtained by concentration of the reaction mixture under reduced pressure was purified by chromatography on silica gel(hexane:ethyl acetate=3:1) to give the title compound(604 mg, 29.2%) as a white solid.
   ¹H-NMR(CDCl₆): *δ* 3.82(3H,s), 5.37(2H,s), 6.92-6.99(2H,m), 7.10(1H,d,J=8.7Hz), 7.38-7.43(2H,m), 7.99(1H,dd,J=8.7Hz,J=2.1Hz), 8.36(1H,d,J=2.1Hz), 9.86(1H,s), 11.42(1H,s).
(2) 5-[3-(4-Methoxybenzyloxycarbonyl)-4-hydroxybenzylidene]-3-(4-phenylbenzyl)thiazolidine-2,4-dione (Compound No. 362).

The title compound was obtained in the same manner as the Example 350(2) using the following raw materials.
Raw materials: 5-formylsalicylic acid 4-methoxybenzyl ester and 3-(4-phenylbenzyl)thiazolidine-2,4-dione(compound of the Example 20(1)).
Yield: 70.7%(light yellow solid).
¹H-NMR(CDCl₃): *δ* 3.84(3H,s), 4.93(2H,s), 5.36(2H,s), 6.91-6.97(2H,m), 7.07(1H,d,J=8.7Hz), 7.31-7.62(12H,m), 7.82(1H,s), 8.03(1H,d,J=2.7Hz), 11.12(1H,s).

### Example 363: Preparation of the compound of Compound No. 363.

(1) 5-Formylsalicylic acid benzyl ester.
   A mixture of benzyl bromide(342 mg, 2.0 mmol), potassium fluoride(256 mg, 4.4 mmol) and N,N-dimethylformamide2mL) was stirred at room temperature for 5 minutes. 5-Formylsalicylic acid(332 mg, 2.0 mmol) was added to the mixture and it was stirred at 100°C for 1 hour. The reaction mixture was cooled. Water was added to the mixture, and the mixture was extracted with ethyl acetate. The organic layer was washed with saturated aqueous sodium hydrogencarbonate and brine, and dried over anhydrous sodium sulfate. The residue obtained by evaporation of the solvent under reduced pressure was purified by chromatography on silica gel(ethyl acetate) to give the title compound(353 mg, 68.9%) as a white solid.
   ¹H-NMR(CDCl₃): δ 5.43(2H,s), 7.01(1H,d,J=8.7Hz), 7.32-7.49(5H,m), 8.00(1H,dd,J=8.7,2.1Hz), 8.39(1H,d,J=2.1Hz), 9.87(1H,s), 11.38(1H,s).
(2) 5-(3-Benzyloxycarbonyl-4-hydroxybenzylidene)-3-[2,5-bis(trifluoromethyl)benzyl]thiazolidine-2,4-dione (Compound No. 363).
   The title compound was obtained in the same manner as the Example 350(2) using the following raw materials.
   Raw materials: 5-formylsalicylic acid benzyl ester and 3-[2,5-bis(trifluoromethyl)benzyl]thiazolidine-2,4-dione(compound of the Example 305(1)).
   Yield: 79.7%(light yellow solid).
   ¹H-NMR(CDCl₃):δ 5.19(2H,s), 5.44(2H,s), 7.12(1H,d,J=8.7Hz), 7.34-7.47(6H,m), 7.65(1H,dd,J=8.7,2.7Hz), 7.66-7.69(1H,m), 7.85(1H,d,J=8.4Hz), 7.90(1H,s), 8.12(1H,d,J=2.7Hz), 11.14(1H,s).

### Example 364: Preparation of the compound of Compound No. 364.

4-(Trifluoromethoxy)benzoyl chloride(31.3 mg, 0.136 mmol) was added to a mixture of 5-(3-amino-4-methoxybenzylidene)-3-(3,4-dichlorobenzyl)-thiazolidine-2,4-dione(compound of the Example 359(2); 100 mg, 0.136 mmol), pyridine(11 *µ*1, 0.136 mmol), 4-dimethylaminopyridine(catalytic amount) and tetrahydrofuran(3 ml) under ice cooling, and the mixture was stirred at room temperature for 2 hours. The reaction mixture was poured into ice and water, and extracted with ethyl acetate. The organic layer was washed with brine, and dried over anhydrous magnesium sulfate. The residue obtained by evaporation of the solvent under reduced pressure was purified by chromatography on silica gel(ethyl acetate:hexane=1:2 → 1:1) to give the title compound(14.7 mg, 18.0%) as a white crystal.
¹H-NMR(DMSO-d₆): *δ* 3.93(3H,s), 4.84(2H,s), 7.28-7.32(2H,m), 7.52-7.57(3H,m), 7.62(1H,d,J=8.4Hz), 7.63(1H,d,J=2.1Hz), 7.94(1H,s), 8.11(2H,d,J=8.7Hz), 8.18(1H,d,J=2.1Hz), 9.77(1H,s).

### Example 365: Preparation of the compound of Compound No. 365.

The title compound was obtained in the same manner as the Example 341(4) using the following raw material.
Raw material: 5-{4-methoxy-3-[4-(trifluoromethoxy)benzoylamino]benzylidene}-3-(3,4-dichlorobenzyl)thiazolidine-2,4-dione(Compound No. 364).
Yield: 59.8%(white crystal).
¹H-NMR(DMSO-d₆): δ 4.84(2H,s), 7.08(1H,d, J=8.4Hz), 7.30(1H,dd,J=8.4,1.8Hz), 7.41(1H,dd,J=8.4,2.1Hz), 7.54(2H,d,J=8.7Hz), 7.62(1H,d,J=1.8Hz),7.62(1H,d,J=8.1Hz), 7.89(1H,s), 8.10-8.13(3H,m), 9.72(1H,s), 10.86(1H,s).

### Example 366: Preparation of the compound of Compound No. 366.

(1) 5-(3-Nitrobenzylidene)-3-(3,4-dichlorobenzyl)thiazolidine-2,4-dione.
   The title compound was obtained in the same manner as the Example 359(1) using the following raw materials.
   Raw materials: 3-nitrobenzaldehyde and
   3-(3,4-dichlorobenzyl)thiazolidine-2,4-dione(compound of the Example 8(1)).
   Yield: 87.4%(light yellow crystal).
   ¹H-NMR(DMSO-d₆): δ 4.86(2H,s), 7.33(1H,d,J=8.1,1.8Hz), 7.63(1H,d,J=8.1Hz), 7.64(1H,s), 7.85(1H,t,J=8.1Hz), 8.05(1H,d,J=7.8Hz), 8.14(1H,s), 8.32(1H,dd,J=8.1,1.5Hz), 8.50(1H,s).
(2) 5-(3-Aminobenzylidene)-3-(3,4-dichlorobenzyl)thiazolidine-2,4-dione.
   10% Palladium on carbon(100 mg) was added to a solution of 5-(3-nitrobenzylidene)-3-(3,4-dichlorobenzyl)thiazolidine-2,4-dione(60 mg, 1.411 mmol) in a mixed solution of ethanol, ethyl acetate and tetrahydrofuran(total 20 ml), and the mixture was stirred at room temperature over night under hydrogen atmosphere. The reaction mixture was filtered and the residue obtained by concentration of the filtrate under reduced pressure was purified by chromatography on silica gel(hexane:ethyl acetate=2:1) to give the title compound(136.7 mg, 24.5%) as a yellow crystal.
   ¹H-NMR(DMSO-d₆): δ 4.83(2H,s), 5.43(2H,s), 6.67-6.71(1H,m), 6.75-6.78(2H,m), 7.17(1H,t,J=8.4Hz), 7.30(1H,dd,J=8.1,1.8Hz), 7.62(1H,d,J=8.1Hz), 7.62(1H,d,J=1.8Hz), 7.76(1H,s).
(3) 5-{3-[4-(Trifluoromethoxy)benzoylamino]benzylidene}-3-(3,4-dichlorobenzyl)thiazolidine-2,4-dione (Compound No. 366).

The title compound was obtained in the same manner as the Example 364 using the following raw materials.
Raw materials: 5-(3-aminobenzylidene)-3-(3,4-dichlorobenzyl)thiazolidine-2,4-dione and 4-(trifluoromethoxy)benzoyl chloride.
Yield: 72.1%(light yellow crystal).
¹H-NMR(DMSO-d₆): δ 4.85(2H,s), 7.31-7.34(1H,m), 7.41-7.44(1H,m), 7.51-7.57(3H,m), 7.26-7.64(2H,m), 7.83-7.86(1H,m), 7.94(1H,s), 8.10(2H,d,J=8.4Hz), 8.18(1H,s), 10.58(1H,s).

### Example 367: Preparation of the compound of Compound No. 367.

The title compound was obtained in the same manner as the Example 364 using the following raw materials, provided that triethylamine was used instead of pyridine as the base.
Raw materials: 5-(3-aminobenzylidene)-3-(3,4-dichlorobenzyl)thiazolidine-2,4-dione(compound of the Example 366(2)) and 4-methoxybenzoyl chloride.
Yield: 15.1%(light yellow crystal).
¹H.NMR(DMSO-d₆): *δ* 3.85(3H,s), 4.85(2H,s), 7.09(2H,d,J=9.0Hz), 7.32(1H,dd, J=8.4,2.1Hz), 7.39(1H,d,J=7.8Hz), 7.52(1H,t, J=7.8Hz), 7.63(1H,d, J=8.4Hz), 7.64(1H,d, J=1.8Hz), 7.83-7.86(1H,m), 7.93(1H,s), 7.98(2H,d,J=8.7Hz), 8.20(1H,s), 10.33(1H,s).

### Example 368: Preparation of the compound of Compound No. 368.

The title compound was obtained in the same manner as the Example 341(4) using the following raw material.
Raw material: 5-[3-(4-methoxybenzoylamino)benzylidene]-3-(3,4-dichlorobenzyl)-thiazolidine-2,4-dione(Compound No. 367).
Yield: 26.0%(white crystal).
¹H-NMR(DMSO.d₆): *δ* 4.85(2H,s), 6.88(2H,d,J=8.4Hz), 7.31-7.39(2H,m), 7.51(1H,t,J=8.4Hz), 7.63(1H,d,J=8.1Hz), 7.64(1H,s), 7.83-7.92(4H,m), 8.19(1H,s), 10.15(1H,s), 10.23(1H,s).

### Example 369: Preparation of the compound of Compound No. 369.

The title compound was obtained in the same manner as the Example 364 using the following raw materials, provided that triethylamine was used instead of pyridine as the base.
Raw materials: 5-(3-aminobenzylidene)-3-(3,4-dichlorobenzyl)thiazolidine-2,4-dione(compound of the Example 366(2)) and 3,4-dimethoxybenzoyl chloride.
Yield: 6.7%(light yellow crystal).
¹H-NMR(DMSO-d₆): *δ* 3.86(6H,s), 4.85(2H,s), 7.11(1H,d,J=8.4Hz), 7.31-7.34(1H,m), 7.39-7.41(1H,m), 7.50-7.55(2H,m), 7.62-7.67(3H,m), 7.80-7.83(1H,m), 7.93(1H,s), 8.23(1H,s), 10.31(1H,s).

### Example 370: Preparation of the compound of Compound No. 370.

The title compound was obtained in the same manner as the Example 341(4) using the following raw material.
Raw material: 5-[3-(3,4-dimethoxybenzoylamino)benzylidene]-3-(3,4-dichlorobenzyl)thiazolidine-2,4-dione(Compound No. 369).
Yield: 35.9%(grayish white crystal).
¹H-NMR(DMSO-d₆): δ 4.85(2H,s), 6.84(1H,d,J=8.4Hz), 7.31-7.41(3H,m), 7.47-7.53(2H,m), 7.63(1H,d,J=8.4Hz), 7.64(1H,s), 7.82-7.86(1H,m), 7.92(1H,s), 8.17(1H,s), 9.26(1H,s), 9.66(1H,s), 10.18(1H,s).

### Example 371: Preparation of the compound of Compound No. 371.

(1) 5-(3-Carboxy-4-hydroxybenzylidene)-3-(4-phenylbenzyl)thiazolidine-2,4-dione.
   The title compound was obtained in the same manner as the following Example 372(1) using the following raw material.
   Raw material: 5-[3-(4-methoxybenzyloxycarbonyl)-4-hydroxybenzylidene]-3-(4-phenylbenzyl)thiazolidine-2,4-dione (Compound No. 362).
   Yield: 100%(white solid).
   ¹H-NMR(DMSO-d₆):δ 4.89(2H,s), 7.13(1H,d,J=8.4Hz), 7.34-7.49(5H,m), 7.63-7.67(4H,m), 7.80(1H,dd,J=8.4,2.1Hz), 7.98(1H,s), 8.09(1H,d,J=2.1Hz).
(2) 5-[4-Hydroxy-3-(3,4-dimethoxyphenylcarbamoyl)benzylidene]-3-(4-phenylbenzyl)thiazolidine-2,4-dione (Compound No. 371).
   The title compound was obtained in the same manner as the Example 357 using the following raw materials.
   Raw materials:
   5-(3-carboxy-4-hydroxybenzylidene)-3-(4-phenylbenzyl)thiazolidine-2,4-dione and 3,4-dimethoxyaniline.
   Yield: 31.5%(light yellow solid).
   ¹H-NMR(DMSO-d₆): *δ* 3.75(3H,s), 3.77(3H,s), 4.89(2H,s), 6.95(1H,d,J=8.7Hz), 7.06(1H,d,J=8.7Hz), 7.21(1H,dd,J=1.5,8.1Hz), 7.34-7.49(6H,m), 7.63-7.67(5H,m), 7.92(1H,s), 8.18(1H,d,J=2.1Hz), 10.77(1H,brs).

### Example 372: Preparation of the compound of Compound No. 372.

(1) 5-(3-Carboxy-4-hydroxybenzylidene)-3-[2,5-bis(trifluoromethyl)-benzyl]thiazolidine-2,4-dione.
   Boron tribromide(1.0M solution in dichloromethane; 0.93 ml, 0.93 mmol) was added slowly to a solution of 5-(3-benzyloxycarbonyl-4-hydroxybenzylidene)-3-[2,5-bis(trifluoromethyl)benzyl]thiazolidine-2,4-dione(Compound No. 363; 270 mg, 0.464 mmol) in dichloromethane(3 ml) at 0°C, and the mixture was stirred at room temperature for 1 hour. Water was added to the reaction mixture. The separated solid was collected by filtration and washed with dichloromethane to give the title compound(170 mg, 75.0%) as a light green solid.
   ¹H-NMR(DMSO-d₆): δ 5.07(2H,s), 7.15(1H,d,J=9.0Hz), 7.76-7.85(2H,m), 7.92-8.07(3H,m), 8.11(1H,d,J=2.4Hz).
(2) 5-[4-Hydroxy-3-(3,4-dimethoxyphenylcarbamoyl)benzylidene]-3-[2,5-bis(trifluoromethyl)benzyl]thiazolidine-2,4-dione (Compound No. 372).
   The title compound was obtained in the same manner as the Example 357 using the following raw materials.
   Raw materials: 5-(3-carboxy-4-hydroxybenzylidene)-3-[2,5-bis(trifluoromethyl)-benzyl]thiazolidine-2,4-dione and 3,4-dimethoxyaniline.
   Yield: 55.4%(yellow solid).
   ¹H-NMR(DMSO-d₆): δ 3.75(3H,s), 3.77(3H,s), 5.08(2H,s), 6.95(1H,d,J=8.7Hz), 7.13(1H,d,J=8.7Hz), 7.22(1H,dd,J=2.4,8.7Hz), 7.43(1H,d,J=2.4Hz), 7.69(1H,dd,J=2.1,8.4Hz), 7.75(1H,s), 7.93-7.96(2H,m), 8.02-8.07(1H,m), 8.21(1H,d,J=2.1Hz), 10.52(1H,brs).

### Example 373: Preparation of the compound of Compound No. 373.

Boron tribromide(1.0M solution in dichloromethane; 0.78 ml, 0.78 mmol) was added slowly to a solution of 5-[4-hydroxy-3-(3,4-dimethoxyphenylearbamoyl)-benzylidene]-3-(4-phenylbenzyl)thiazolidine-2,4-dione(Compound No. 371; 110 mg, 0.194 mmol) in dichloromethane(2 ml) at 0°C, and the mixture was stirred at room temperature for 1 hour. Water was added to the reaction mixture, and the mixture was extracted with ethyl acetate. The organic layer was washed with brine and dried over anhydrous sodium sulfate. The residue obtained by evaporation of the solvent under reduced pressure was purified by chromatography on silica gel(hexane:ethyl acetate=1:1) to give the title compound(12 mg, 11.5%) as a yellow solid.
¹H-NMR(DMSO-d₆): *δ* 4.89(2H,s), 6.70(1H,d,J=8.4Hz), 6.90(1H,dd,J=8.4,2.7Hz), 7.14(1H,d,J=9.0Hz), 7.24(1H,d,J=2.7Hz), 7.33-7.51(5H,m), 7.62-7.70(5H,m), 7.93(1H,s), 8.20(1H,d,J=2.1Hz), 8.78(1H,brs), 9.05(1H,brs), 10.12(1H,s), 12.38(1H,s).

### Example 374: Preparation of the compound of Compound No. 374.

The title compound was obtained in the same manner as the Example 373 using the following raw material.
Raw material: 5-[4-hydroxy-3-(3,4-dimethoxyphenylcarbamoyl)benzylidene]-3-[2,5-bis(trifluoromethyl)benzyl]thiazolidine-2,4-dione(Compound No. 372).
Yield: 38.0%(yellow solid).
¹H-NMR(DMSO-d₆): *δ* 5.08(2H,s), 6.71(1H,d,J=8.4Hz), 6.91(1H,dd,J=2.4,8.7Hz), 7.15(1H,d,J=8.7Hz), 7.25(1H,d,J=2.1Hz), 7.69(1H,dd,J=2.1,8.4Hz), 7.76(1H,s), 7.92-8.07(3H,m), 8.22(1H,d,J=2.4Hz), 8.79(1H,s), 9.06(1H,s), 10.13(1H,s), 12.39(1H,s).

### Example 375: Preparation of the compound of Compound No. 375.

(1) 5-(3-Benzyloxycarbonyl-4-hydroxybenzylidene)-3-[2-(4-chlorophenyl)ethyl]thiazolidine-2,4-dione.
   The title compound was obtained in the same manner as the Example 350(2) using the following raw materials. Raw materials: 5-formylsalicylic acid benzyl ester(compound of the Example 363(1)) and 3-[2-(4-chlorophenyl)ethyl]thiazolidine-2,4-dione(compound of the Example 90(1)). Yield: 98.4%(light orange solid).
   ¹H-NMR(CDCl₃): *δ* 2.94(2H,t,J=7.8Hz), 3.93-3.98(2H,m), 5.43(2H,s), 7.09(1H,d,J=8.7Hz), 7.15-7.50(9H,m), 7.60(1H,dd,J=2.4,8.7Hz), 7.78(1H,s), 8.07(1H,d,J=2.4Hz).
(2) 5-(3-Carboxy-4-hydroxybenzylidene)-3-[2-(4-chlorophenyl)-ethyl]thiazolidine-2,4-dione.
   The title compound was obtained in the same manner as the Example 372(1) using the following raw material.
   Raw material: 5-(3-benzyloxycarbonyl-4-hydroxybenzylidene)-3-[2-(4-chlorophenyl)ethyl]thiazolidine-2,4-dione.
   Yield: 51.6%(white solid).
   ¹H-NMR(DMSO·d₆): *δ* 2.91(2H,t,J=7.2Hz), 3.88(2H,t,J=7.2Hz), 7.12(1H,d,J=8.7Hz), 7.22(2H,d,J=8.1Hz), 7.34(2H,d,J=8.1Hz), 7.77(1H,dd,J=8.7,2.1Hz), 7.89(1H,s), 8.06(1H,d,J=2.1Hz).
(3) 5-[4-Hydroxy-3-(3,4-dimethoxyphenylcarbamoyl)benzylidene]-3-[2-(4-chlorophenyl)ethyl]thiazolidine-2,4-dione (Compound No. 375).
   The title compound was obtained in the same manner as the Example 357 using the following raw materials.
   Raw materials: 5-(3-carboxy-4-hydroxybenzylidene)-3-[2-(4-chlorophenyl)ethyl]-thiazolidine-2,4-dione and 3,4-dimethoxyaniline.
   Yield: 66.8%(light yellow solid).
   ¹H-NMR(DMSO-d₆): *δ* 2.92(2H,t,J=6.9Hz), 3.75(3H,s), 3.77(3H,s), 3.89(2H,t,J=6.9Hz), 6.95(1H,d,J=8.7Hz), 7.15(1H,d,J=8.7Hz), 7.21-7.36(5H,m), 7.42(1H,d,J=2.4Hz), 7.66(1H,dd,J=2.4,8.7Hz), 7.85(1H,s), 8.17(1H,d,J=2.1Hz), 10.26(1H,s).

### Example 376: Preparation of the compound of Compound No. 376.

(1) 5-(3-Benzyloxycarbonyl-4-hydroxybenzylidene)-3-(4-nitrobenzyl)-thiazolidine-2, 4-dione.
   The title compound was obtained in the same manner as the Example 350(2) using the following raw materials.
   Raw materials: 5-formylsalicylic acid benzyl ester(compound of the Example 363(1)) and 3-(4-nitrobenzyl)thiazolidine-2,4-dione(compound of the Example 50(1)).
   Yield: 49.0%(white solid).
   ¹H.NMR(CDCl₃-d₆): *δ* 4.97(2H,s), 5.43(2H,s), 7.09(2H,d,J=9.0Hz), 7.37-7.62(7H,m), 7.85(1H,s), 8.07(1H,d,J=2.1Hz), 8.20(2H,d,J=9.0Hz), 11.12(1H,s).
(2) 5-(3-Carboxy-4-hydroxybenzylidene)-3-(4-nitrobenzyl)thiazolidine-2,4-dione.
   The title compound was obtained in the same manner as the Example 372(1) using the following raw material.
   Raw material: 5-(3-benzyloxycarbonyl-4-hydroxybenzylidene)-3-(4-nitrobenzyl)-thiazolidine-2,4-dione.
   Yield: 61.7%(white solid).
   ¹H-NMR(DMSO-d₆): *δ* 4.98(2H,s), 7.13(1H,d,J=8.7Hz), 7.60(2H,d,J=8.7Hz), 7.78-7.83(1H,m), 7.98(1H,s), 8.10-8.11(1H,m), 8.22(2H,d,J=8.7Hz), 11.00(1H,brs). (3) 5-[4-Hydroxy-3-(3,4-dimethoxyphenylcarbamoyl)benzylidene]-3-(4-nitrobenzyl)thiazolidine-2,4-dione (Compound No. 376)
   The title compound was obtained in the same manner as the Example 357 using the following raw materials.
   Raw materials: 5-(3-carboxy-4-hydroxybenzylidene)-3-(4-nitrobenzyl)thiazolidine-2,4-dione and 3,4-dimethoxyaniline.
   Yield: 72.8%(light yellow solid).
   ¹H-NMR(DMSO-d₆): *δ* 3.75(3H,s), 3.77(3H,s), 4.99(2H,s), 6.96(1H,d,J=8.7Hz), 7.16(1H,d,J=8.7Hz), 7.23(1H,dd,J=8.7,2.1Hz), 7.42(1H,d,J=2.1Hz), 7.60(2H,d,J=8.7Hz), 7.69(1H,dd,J=2.1,8.7Hz), 7.94(1H,s), 8.19-8.26(3H,m), 10.27(1H,s).

### Example 377: Preparation of the compound of Compound No. 377.

The title compound was obtained in the same manner as the Example 373 using the following raw material.
Raw material: 5-[4-hydroxy-3-(3,4-dimethoxyphenylcarbamoyl)benzylidene]-3-[2-(4-chlorophenyl)ethyl]thiazolidine-2,4-dione(Compound No. 375).
Yield: 23.5%(light yellow solid).
¹H.NMR(DMSO.d₆):*δ* 2.92(2H,d,J=7.2Hz), 3.89(2H,d,J=7.2Hz), 6.71(1H,d,J=8.4Hz), 6.90(2H,dd,J=2.4,8.4Hz), 7.12(1H,d,J=8.4Hz), 7.23(2H,d,J=8.4Hz), 7.24(1H,s), 7.34(2H,d,J=8.4Hz), 7.64(1H,dd,J=2.4,8.4Hz), 7.84(1H,s), 8.17(1H,d,J=2.4Hz), 8.80(1H,brs), 9.06(1H,brs), 10.11(1H,s), 12.37(1H,s).

### Example 378: Preparation of the compound of Compound No. 378.

The title compound was obtained in the same manner as the Example 373 using the following raw material.
Raw material: 5-[4-hydroxy-3-(3,4-dimethoxyphenylcarbamoyl)benzylidene]-3-(4-nitrobenzyl)thiazolidine-2,4-dione(Compound No. 376).
Yield: 22.4%(yellow solid).
¹H·NMR(DMSO.d₆): *δ* 4.99(2H,s), 6.71(1H,d,J=8.7Hz), 6.90(1H,dd,J=8.4,2.1Hz), 7.12(2H,d,J=8.4Hz), 7.24(1H,d,J=2.4Hz), 7.58-7.70(3H,m), 7.93(1H,s), 8.17-8.26(3H,m), 8.79(1H,s), 9.05(1H,s), 10.13(1H,s), 12.39(1H,brs).

### Example 379: Preparation of the compound of Compound No. 379.

The title compound was obtained in the same manner as the Example 364 using the following raw materials.
Raw materials: 5-(3-amino-4-methoxybenzylidene)-3-(3,4-dichlorobenzyl)thiazolidine-2,4-dione(compound of the Example 359(2)) and 3,4-dimethoxybenzoyl chloride. Yield: 11.3%(light yellow crystal).
¹H-NMR(DMSO-d₆): *δ* 3.85(6H,s), 3.94(3H,s), 4.85(2H,s), 7.09(1H,d,J=8.4Hz), 7.27-7.33(2H,m), 7.52-7.56(2H,m), 7.60-7.64(3H,m), 7.94(1H,s), 8.21(1H,d,J=1.8Hz), 9.44(1H,s).

### Example 380: Preparation of the compound of Compound No. 380.

The title compound was obtained in the same manner as the Example 373 using the following raw material.
Raw material: 5-[4-Methoxy-3-(3,4-dimethoxybenzoylamino)benzylidene]-3-(3,4-dichlorobenzyl)thiazolidine-2,4-dione(Compound No. 379).
Yield: 65.1%(yellow crystal).
¹H-NMR(DMSO-d₆): δ 4.84(2H,s), 6.85(1H,d,J=8.4Hz), 7.06(1H,d,J=8.4Hz), 7.29-7.38(4H,m), 7.62(1H,d,J=1.8Hz), 7.62(1H,d,J=8.4Hz), 7.87(1H,s), 8.25(1H,d,J=1.8Hz), 9.18(1H,s), 9.34(1H,s), 9.67(1H,s), 10.93(1H,s).

### Example 381: Preparation of the compound of Compound No. 381.

The title compound was obtained in the same manner as the Example 364 using the following raw materials.
Raw materials: 5-(3-amino-4-methoxybenzylidene)-3-(3,4-dichlorobenzyl)-thiazolidine-2,4-dione(compound of the Example 359(2)) and 4-methoxybenzoyl chloride.
Yield: 81.3%(light yellow crystal).
¹H-NMR(DMSO-d₆): *δ* 3.85(3H,s), 3.94(3H,s), 4.85(2H,s), 7.07(2H,d,J=9.3Hz), 7.28(1H,d,J=8.4Hz), 7.31(1H,dd,J=8.4,2.1Hz), 7.53(1H,dd,J=8.4,2.1Hz), 7.62(1H,d,J=8.4Hz), 7.63(1H,d,J=1.8Hz), 7.93(1H,s), 7.97(2H,d,J=9.0Hz), 8.25(1H,d,J=2.4Hz), 9.41(1H,s).

### Example 382: Preparation of the compound of Compound No. 382.

The title compound was obtained in the same manner as the Example 373 using the following raw material.
Raw material: 5-[4-methoxy-3-(4-methoxybenzoylamino)benzylidene]-3-(3,4-dichlorobenzyl)thiazolidine-2,4-dione (Compound No. 381).
Yield: 94.3%(light yellow crystal).
¹H-NMR(DMSO-d₆): δ 4.84(2H,s), 6.88(2H,d,J=8.7Hz), 7.06(1H,d,J=8.4Hz), 7.30(1H,dd,J=8.7,2.4Hz), 7.37(1H,dd,J=8.7,2.4Hz), 7.62(1H,d,J=8.4Hz), 7.62(1H,d,J=1.8Hz), 7.86(2H,d,J=8.1Hz), 7.87(1H,s), 8.21(1H,d,J=2.4Hz), 9.30(1H,s), 10.17(1H,s), 10.89(1H,s).

### Example 383: Preparation of the compound of Compound No. 383.

(1) 5-(3-Benzyloxycarbonyl-4-hydroxybenzylidene)-3-[3,5-bis-(trifluoromethyl)benzyl]thiazolidine-2,4-dione.
   The title compound was obtained in the same manner as the Example 2(2) using the following raw materials.
   Raw materials: 5-formylsalicylic acid benzyl ester(compound of the Example 363(1)) and 3-[3,5-bis(trifluoromethyl)benzyl]thiazolidine-2,4-dione(compound of the Example 2(1)).
   Yield: 70.4%(light yellow crystal).
   ¹H-NMR(DMSO-d₆): *δ* 5.03(2H,s), 5.41(2H,s), 7.17(1H,d,J=8.4Hz), 7.37-7.45(3H,m), 7.51-7.54(2H,m), 7.79(1H,dd,J=8.7,2.1Hz), 7.98(1H,s), 8.05(2H,s), 8.07(1H,s), 8.11(1H,d,J=2.4Hz), 10.99(1H,s).
(2) 5-(3-Carboxy-4-hydroxybenzylidene)-3-[3,5-bis(trifluoromethyl)-benzyl]thiazolidine-2,4-dione.
   The title compound was obtained in the same manner as the Example 372(1) using the following raw material.
   Raw material: 5-(3-benzyloxycarbonyl-4-hydroxybenzylidene)-3-[3,5-bis(trifluoromethyl)benzyl]thiazolidine-2,4-dione.
   Yield: 94.3%(white crystal).
   ¹H-NMR(DMSO-d₆):δ 5.04(2H,s), 7.13(1H,d,J=8.7Hz), 7.80(1H,dd,J=8.7,2.4Hz), 7.97(1H,s), 8.05(2H,s), 8.07(1H,s), 8.09(1H,d,J=2.4Hz).
(3) 5-[4-Hydroxy-3-(3,4-dimethoxyphenylcarbamoyl)benzylidene]-3-[3,5-bis(trifluoromethyl)benzyl]thiazolidine-2,4-dione (Compound No. 383).
   The title compound was obtained in the same manner as the Example 357 using the following raw materials.
   Raw materials:
   5-(3-carboxy-4-hydroxybenzylidene)-3-[3,5-bis(trifluoromethyl)benzyl]thiazolidine-2,4-dione and 3,4-dimethoxyaniline.
   Yield: 81.4%(light yellow crystal).
   ¹H-NMR(DMSO-d₆):δ 3.75(3H,s), 3.77(3H,s), 5.05(2H,s), 6.96(1H,d,J=8.7H), 7.16(1H,d,J=8.4Hz), 7.23(1H,dd,J=8.4,2.1Hz), 7.42(1H,d,J=2.1Hz), 7.69(1H,dd,J=8.7,2.1Hz), 7.93(1H,s), 8.05(2H,s), 8.07(1H,s), 8.20(1H,d,J=2.1Hz), 10.28(1H,s), 12.27(1H,brs).

### Example 384: Preparation of the compound of Compound No. 384.

The title compound was obtained in the same manner as the Example 373 using the following raw material.
Raw material: 5-[4-hydroxy-3-(3,4-dimethoxyphenylcarbamoyl)benzylidene]-3-[3,5-bis(trifluoromethyl)benzyl]thiazolidine-2,4-dione (Compound No. 383).
Yield: 97.8%(light yellow crystal).
¹H-NMR(DMSO-d₆): *δ* 5.05(2H,s), 6.71(1H,d,J=8.1Hz), 6.90(1H,dd,J=8.4,2.1Hz), 7.14(1H,d,J=8.1Hz), 7.25(1H,d,J=2.1Hz), 7.67(1H,dd,J=8.4,2.1Hz), 7.92(1H,s), 8.05(2H,s), 8.07(1H,s), 8.21(1H,d,J=1.8Hz), 10.12(1H,s), 12.37(1H,s).

### Example 385: Preparation of the compound of Compound No. 385.

(1) 5-(3-Benzyloxycarbonyl-4-hydroxybenzylidene)-3-(3,5-difluorobenzyl)-thiazolidine-2,4-dione.
   The title compound was obtained in the same manner as the Example 2(2) using the following raw materials.
   Raw materials: 5-formylsalicylic acid benzyl ester(compound of the Example 363(1)) and 3-(3,5-difluorobenzyl)thiazolidine-2,4-dione(compound of the Example 24(1)). Yield: 56.0%(grayish white crystal).
(2) 5-(3-Carboxy-4-hydroxybenzylidene)-3-(3,5-difluorobenzyl)thiazolidine-2,4-dione.
   The title compound was obtained in the same manner as the Example 372(1) using the following raw material.
   Raw material: 5-(3-benzyloxycarbonyl-4-hydroxybenzylidene)-3-(3,5-difluorobenzyl)thiazolidine-2,4-dione.
   Yield: 91.4%(light yellow crystal).
   ¹H-NMR(DMSO-d₆): δ 4.86(2H,s), 7.05-7.07(2H,m), 7.12(1H,d,J=8.4Hz), 7.14-7.22(1H,m), 7.79(1H,dd,J=8.4,2.1Hz), 7.96(1H,s), 8.09(1H,d,J=2.1Hz).
(3) 5-[4-Hydroxy-3-(3,4-dimethoxyphenylcarbamoyl)benzylidene]-3-(3,5-difluorobenzyl)thiazolidine-2,4-dione (Compound No. 385).
   The title compound was obtained in the same manner as the Example 357 using the following raw materials.
   Raw materials: 5-(3-carboxy-4-hydroxybenzylidene)-3-(3,5-difluorobenzyl)-thiazolidine-2,4-dione and 3,4-dimethoxyaniline.
   Yield: 74.5%(yellow crystal).
   ¹H-NMR(DMSO-d₆): δ 3.75(3H,s), 3.77(3H,s), 4.87(2H,s), 6.96(1H,d,J=8.7Hz), 7.05-7.08(1H,m), 7.16(1H,d,J=8.7Hz), 7.16-7.20(1H,m), 7.23(1H,dd,J=8.7,2.1Hz), 7.43(1H,d,J=2.1Hz), 7.69(1H,dd,J=8.7,2.1Hz), 7.92(1H,s), 8.20(1H,d,J=2.1Hz), 10.28(1H,s), 12.28(1H,s).

### Example 386: Preparation of the compound of Compound No. 386.

The title compound was obtained in the same manner as the Example 373 using the following raw material.
Raw material: 5-[4-hydroxy-3-(3,4-dimethoxyphenylcarbamoyl)benzylidene]-3-(3,5-difluorobenzyl)thiazolidine-2,4-dione(Compound No. 385).
Yield: 75.5%(yellowish brown crystal).
¹H-NMR(DMSO-d₆): δ 4.86(2H,s), 6.71(1H,d,J=8.4Hz), 6.91(1H,d,J=8.1Hz), 7.05-7.07(2H,m), 7.13-7.22(2H,m), 7.25(1H,s), 7.67(1H,d,J=8.1Hz), 7.91(1H,s), 8.21(1H,s), 10.12(1H,s), 12.39(1H,s).

### Example 387: Preparation of the compound of Compound No. 387.

The title compound was obtained in the same manner as the Example 350(2) using the following raw materials.
Raw materials: 5-formylsalicylic acid 4-methoxybenzyl ester(compound of the Example 362(1)) and 3-[4-(trifluoromethyl)benzyl]thiazolidine-2,4-dione(compound of the Example 43(1)).
Yield: 67.0%(white solid).
¹H-NMR(CDCl₃): *δ* 3.83(3H,s), 4.94(2H,s), 5.36(2H,s), 6.90-6.97(2H,m), 7.07(1H,d,J=8.7Hz), 7.26(1H,s), 7.38-7.42(2H,m), 7.52-7.62(5H,m), 7.82(1H,s), 8.03(1H,d,J=2.1Hz), 11.14(1H,s).

### Example 388: Preparation of the compound of Compound No. 388.

(1) 5-{3-[3,4-Bis(methoxymethoxy)phenylcarbamoyl]benzylidene}-3-(3,4-dichlorobenzyl)thiazolidine-2,4-dione.
   Phosphorus oxychloride(49 *µ* 1, 0.53 mmol) and pyridine(41 *µ* l*,* 0.51 mmol) were added to a solution of 5-(3-carboxybenzylidene)-3-(3,4-dichlorobenzyl)thiazolidine-2,4-dione(Compound No. 350; 207.1 mg, 0.51 mmol) and
   3,4-bis(methoxymethoxy)aniline (108.2 mg, 0.51 mmol) in tetrahydrofuran(5 ml) under ice cooling and argon atmosphere, and the mixture was stirred at room temperature for 30 minutes. The reaction mixture was poured into water and extracted with ethyl acetate. The organic layer was washed with water and brine, and dried over anhydrous sodium sulfate. The residue obtained by evaporation of the solvent under reduced pressure was purified by chromatography on silica gel(hexane: ethyl acetate=1:1→1:2) to give the title compound(99.5 mg, 32.5%) as a yellowish white solid.
   ¹H-NMR(CDCl₃): *δ* 3.53(3H,s), 3.54(3H,s), 4.84(2H,s), 5.22(2H,s), 5.26(2H,s), 7.16(1H,d,J=8.4Hz), 7.23-7.30(2H,m), 7.42(1H,d,J=8.1Hz), 7.50-7.67(4H,m), 7.77(1H,s), 7.87(1H,d,J=7.5Hz), 7.94(1H,s), 8.00(1H,s).
(2) 5-[3-(3,4-Dihydroxyphenylcarbamoyl)benzylidene]-3-(3,4-dichlorobenzyl)thiazolidine-2,4-dione (Compound No. 388).
   The title compound was obtained in the same manner as the Example 349(2) using the following raw material.
   Raw material: 5-{3-[3,4-bis(methoxymethoxy)phenylcarbamoyl]benzylidene}-3-(3,4-dichlorobenzyl)thiazolidine-2,4-dione.
   Yield: 84.6%(yellow powder).
   ¹H-NMR(DMSO-ds): *δ* 4.86(2H,s), 6.69(1H,d,J=8.4Hz), 6.98(1H,dd,J=8.4,2.4Hz), 7.30(1H,d,J=2.7Hz), 7.32(1H,dd,J=8.4, 1.8Hz), 7.62-7.71(3H,m), 7.80(1H,d,J=8.1Hz), 8.00-8.03(2H,m), 8.14(1H,s), 8.70(1H,s), 9.00(1H,s), 10.05(1H,s).

### Example 389: Preparation of the compound of Compound No. 389.

(1) 5-{3-[4-(tert-Butoxycarbonyl)phenylcarbamoyl]benzylidene}-3-(3,4-dichlorobenzyl)thiazolidine-2,4-dione.
   The title compound was obtained in the same manner as the Example 388(1) using the following raw materials.
   Raw materials:
   5-(3-carboxybenzylidene)-3-(3,4-dichlorobenzyl)thiazolidine-2,4-dione(Compound No. 350) and 4-aminobenzoic acid tert-butyl ester.
   Yield: 50.3%(yellowish white solid).
   ¹H-NMR(CDCl₃):δ 1.60(9H,s), 4.84(2H,s), 7.28(1H,dd,J=8.4,2.1Hz), 7.41(1H,d,J=8.1Hz), 7.54(1H,d,J=2.1Hz), 7.60(1H,t,J=7.5Hz), 7.65(1H,t,J=8.1Hz), 7.73(2H,d,J=8.4Hz), 7.78-7.91(1H,m), 7.93(1H,s), 8.00-8.02(3H,m), 8.10(1H,s).
(2) 5-[3-(4-Carboxyphenylcarbamoyl)benzylidene]-3-(3,4-dichlorobenzyl)thiazolidine-2,4-dione (Compound No. 389).
   The title compound was obtained in the same manner as the Example 345(3) using the following raw material.
   Raw material: 5-{3-[4-(tert-butoxycarbonyl)phenylcarbamoyl]benzylidene}-3-(3,4-dichlorobenzyl)thiazolidine-2,4-dione.
   Yield: 39.0%(light brownish white powder).
   ¹H-NMR(DMSO-d₆): δ 4.87(2H,s), 7.31-7.34(1H,m), 7.62.7.65(2H,m), 7.74(1H,t,J=7.5Hz), 7.86(1H,d,J=7.5Hz), 7.92(2H,d,J=9.0Hz), 7.96(2H,d,J=8.7Hz), 8.06-8.09(2H,m), 8.19(1H,s), 10.66(1H,s), 12.69(1H,brs).

### Example 390: Preparation of the compound of Compound No. 390.

(1) 3-Methyl-4-hydroxy-5-carboxybenzaldehyde.
   A mixture of 3-methylsalicylic acid(1.00 g, 6.57 mmol), hexamethylenetetramine(1.84 g, 13.1 mmol) and trifluoroacetic acid(8 ml) was stirred at 90°C for 2 hours. The reaction mixture was cooled, poured into ice and water, and extracted with ethyl acetate. The organic layer was washed with water and brine, and dried over anhydrous sodium sulfate. The residue obtained by evaporation of the solvent under reduced pressure was washed with hexane under suspension to give the title compound(0.85 g) as a crude product which was a light yellow solid.
   ¹H-NMR(DMSO-d₆): *δ* 2.52(3H,s), 7.90(1H,s), 8.23(1H,d,J=1.5Hz), 9.85(1H,s), 11.41(1H,brs), 13.82(1H,br).
(2) 3-Methyl-4-hydroxy-5-(benzyloxycarbonyl)benzaldehyde.
   Benzylalcohol(0.49 ml, 4.73 mmol) was added to a solution of 3-methyl-4-hydroxy-5-carboxybenzaldehyde(0.85 g, 4.73 mmol) and triphenylphosphine(1.24 g, 4.73 mmol) in tetrahydrofuran(10 ml) under argon atmosphere. Diethyl azodicarboxylate(40% solution in toluene; 1.85 ml) was added slowly to the mixture under ice cooling, and the mixture was stirred at room temperature for 2 hours. The residue obtained by concentration of the reaction mixture under reduced pressure was purified by chromatography on silica gel(hexane:ethyl acetate=7:1) to give the title compound(0.87 g, 48.9%; 2 steps) as a white crystal.
   ¹H-NMR(CDCl₃): *δ* 2.32(3H,s), 5.42(2H,s), 7.38-7.47(5H,m), 7.87(1H,m), 8.25(1H,d,J=2.1Hz), 9.83(1H,s), 11.65(1H,s).
(3) 5-[3-Methyl-4-hydroxy-5-(benzyloxycarbonyl)benzylidene]-3-(3,4-dichlorobenzyl)thiazolidine-2,4-dione.
   The title compound was obtained in the same manner as the Example 2(2) using the following raw materials.
   Raw materials: 3-methyl-4-hydroxy-5-(benzyloxycarbonyl)benzaldehyde and 3-(3,4-dichlorobenzyl)thiazolidine-2,4-dione(compound of the Example 8(1)).
   Yield: 64.5%(light yellowish white solid).
   ¹H-NMR(DMSO-d₆): *δ* 2.26(3H,s), 4.83(2H,s), 5.46(2H,s), 7.30(1H,dd,J=8.1,2.1Hz), 7.38-7.46(3H,m), 7.52-7.55(2H,m), 7.61(1H,d,J=2.4Hz), 7.62(1H,d,J=8.1Hz), 7.73(1H,d,J=2.4Hz), 7.93(1H,s), 8.02(1H,s), 11.16(1H,s).
(4) 5-(3-Methyl-4-hydroxy-5-carboxybenzylidene)-3-(3,4-dichlorobenzyl)thiazolidine-2,4-dione (Compound No. 390).
   Boron tribromide(1M solution in dichloromethane; 1.3 ml, 1.3 mmol) was added to a mixture of 5-[3-methyl-4-hydroxy-5-(benzyloxycarbonyl)benzylidene]-3-(3,4-dichlorobenzyl)thiazolidine-2,4-dione(552 mg, 1.04 mmol) and dichloromethane(5 ml) under ice cooling, and the mixture was stirred at room temperature for 1 hour. Water was added to the reaction mixture, and the mixture was extracted with a mixed solution of tetrahydrofuran/ethyl acetate. The organic layer was washed with brine and dried over anhydrous sodium sulfate. The residue obtained by evaporation of the solvent under reduced pressure was washed with isopropyl ether/hexane under suspension to give the title compound(433.7mg, 94.7%) as a yellowish white powder.
   ¹H-NMR(DMSO-d₆):δ 2.24(3H,s), 4.83(2H,s), 7.30(1H,dd,J=8.4,2.1Hz), 7.61(1H,d,J=2.4Hz), 7.62(1H,d,J=8.1Hz), 7.68(1H,s), 7.89(1H,s), 7.94(1H,s).

### Example 391: Preparation of the compound of Compound No. 391.

(1) 5-(4-Hydroxy-3-carboxybenzylidene)-3-[4-(trifluoromethyl)benzyl)-thiazolidine-2,4-dione.
   The title compound was obtained in the same manner as the following Example 372(1) using the following raw material.
   Raw material: 5-[4-hydroxy-3-(4-methoxybenzyloxycarbonyl)benzylidene]-3-[4-(trifluoromethyl)benzyl]thiazolidine-2,4-dione(Compound No. 387).
   Yield: 99.7%(white solid).
   ¹H-NMR(DMSO-d₆): *δ* 4.94(2H,s), 7.13(1H,d,J=8.4Hz), 7.54(2H,d,J=8.1Hz), 7.73(2H,d,J=8.1Hz), 7.80(1H,dd,J=2.1,8.4Hz), 7.97(1H,s), 8.09(1H,d,J=2.1Hz).
(2) 5-[4-Hydroxy-3-(3,4-dimethoxyphenylcarbamoyl)benzylidene]-3-[4-(trifluoromethyl)benzyl]thiazolidine-2,4-dione (Compound No. 391).
   The title compound was obtained in the same manner as the Example 357 using the following raw materials.
   Raw materials: 5-(4-hydroxy-3-carboxybenzylidene)-3-[4-(trifluoromethyl)benzyl]-thiazolidine-2,4-dione and 3,4-dimethoxyaniline.
   Yield: 42.1%(light yellow solid).
   ¹H-NMR(DMSO-d₆): *δ* 3.75(3H,s), 3.77(3H,s), 4.94(2H,s), 6.96(1H,d,J=8.7Hz), 7.16(1H,d,J=8.7Hz), 7.23(1H,dd,J=2.4,8.7Hz), 7.54(2H,d,J=8.1Hz), 7.67-7.75(3H,m), 7.93(1H,s), 8.19(1H,d,J=2.1Hz), 10.27(1H,s), 12.28(1H,brs).

### Example 392: Preparation of the compound of Compound No. 392.

The title compound was obtained in the same manner as the Example 357 using the following raw materials.
Raw materials: 5-(4-hydroxy-3-carboxybenzylidene)-3-[4-(trifluoromethyl)benzyl-]thiazolidine-2,4-dione(compound of the Example 391(1)) and
5-amino-2-chloropyridine.
Yield: 5.0%(yellow solid).
¹H-NMR(DMSO-d₆): *δ* 4.49(2H,s), 7.18(1H,d,J=8.7Hz), 7.52-7.56(3H,m), 7.69-7.75(3H,m), 7.95(1H,s), 8.10(1H,d,J=2.4Hz), 8.23(1H,dd,J=8.7,2.4Hz), 8.75(1H,d,J=2.7Hz).

### Example 393: Preparation of the compound of Compound No. 393.

(1) Methyl 5-formylsalicylate.
   A mixture of 5-formylsalicylic acid(1.39 g, 8.637 mmol), concentrated sulfuric acid(catalytic amount) and methanol(10 ml) was refluxed for 39 hours. The reaction mixture was concentrated under reduced pressure and poured into water. The separated solid was collected by filtration and dried under reduced pressure to give the title compound(1.308 g, 86.8%).
   ¹H-NMR(CDCl₃):δ 4.02(3H,s), 7.12(1H,d,J=9.0Hz), 8.01(1H,dd,J=8.7,2.1Hz), 8.40(1H,d,J=1.8Hz), 9.89(1H,s), 11.38(1H,s).
(2) 5-(3-Methoxycarbonyl-4-hydroxybenzylidene)-3-(3,5-dimethylbenzyl)-thiazolidine-2,4-dione.
   The title compound was obtained in the same manner as the Example 2(2) using the following raw materials.
   Raw materials: methyl 5-formylsalicylate and
   3-(3,5-dimethylbenzyl)thiazolidine-2,4-dione(compound of the Example 26(1)).
   Yield: 79.7%(white crystal).
   ¹H-NMR(DMSO-d₆): *δ* 2.24(6H,s), 3.91(3H,s), 4.75(2H,s), 6.90(2H,s), 6.93(1H,s), 7.16(1H,d,J=8.7Hz), 7.78(1H,dd,J=8. 7,2.1Hz), 7.95(1H,s), 8.05(1H,d,J=2.1Hz), 10.98(1H,s).
(3) 5-(3-Carboxy-4-hydroxybenzylidene)-3-(3,5-dimethylbenzyl)thiazolidine-2,4-dione.
   The title compound was obtained in the same manner as the Example 341(4) using the following raw material.
   Raw material: 5-(3-methoxycarbonyl-4-hydroxybenzylidene)-3-(3,5-dimethylbenzyl)-thiazolidine-2,4-dione.
   Yield: 75.4%(yellow crystal).
   ¹H-NMR(DMSO-d₆): δ 2.24(6H,s), 4.75(2H,s), 6.90(2H,s), 6.92(1H,s), 7.12(1H,d,J=8.7Hz), 7.79(1H,d,J=9.0Hz), 7.95(1H,s), 8.08(1H,s).
(4) 5-[4-Hydroxy-3-(3,4-dimethoxyphenylcarbamoyl)benzylidene]-3-(3,5-dimethylbenzyl)thiazolidine-2,4-dione (Compound No. 393).
   The title compound was obtained in the same manner as the following Example 357 using the following raw materials.
   Raw materials: 5-(3-carboxy-4-hydroxybenzylidene)-3-(3,5-dimethylbenzyl)-thiazolidine-2,4-dione and 3,4-dimethoxyaniline.
   Yield: 69.5%(grayish yellow crystal).
   ¹H-NMR(DMSO-d₆): Iδ 3.74(3H,s), 3.77(3H,s), 4.74(2H,s), 6.80-6.83(1H,m), 6.90-6.94(4H,m), 7.17(1H,dd,J=8.7,2.4Hz), 7.46-7.51(2H,m), 7.83(1H,s), 8.15(1H,d,J=2.4Hz), 9.72(1H,br), 12.09(1H,br).

### Example 394: Preparation of the compound of Compound No. 394.

The title compound was obtained in the same manner as the Example 373 using the following raw material.
Raw material: 5-[4-hydroxy-3-(3,4-dimethoxyphenylcarbamoyl)benzylidene]-3-(3,5-dimethylbenzyl)thiazolidine-2,4-dione (Compound No. 393).
Yield: 79.3%(yellow crystal).
¹H-NMR(DMSO-d₆): *δ* 2.24(6H,s), 4.76(2H,s), 6.71(1H,d,J=8.7Hz), 6.89-6.93(4H,m), 7.13(1H,d,J=8.7Hz), 7.25(1H,d,J=2.1Hz), 7.66(1H,dd,J=8.7,2.1Hz), 7.91(1H,s), 8.20(1H,d,J=2.1Hz), 8.78(1H,s), 9.06(1H,s), 1O.18(1H,s), 12.38(1H,brs).

### Example 395: Preparation of the compound of Compound No. 395.

(1) 5-(3-Methoxycarbonyl-4-hydroxybenzylidene)-3-(3,5-dichlorobenzyl)-thiazolidine-2,4-dione.
   The title compound was obtained in the same manner as the Example 2(2) using the following raw materials.
   Raw materials: methyl 5-formylsalicylate(compound of the Example 393(1)) and 3-(3,5-dichlorobenzyl)thiazolidine-2,4-dione(compound of the Example 11(1)).
   Yield: 54.3%.
   ¹H-NMR(DMSO-d₆): *δ* 3.92(3H,s), 4.85(2H,s), 7.16(lH,d,J=8.4Hz), 7.40(2H,d,J=1.8Hz), 7.57(1H,t,J=1.5Hz), 7.79(1H,dd,J=8.4,2.1Hz), 7.96(1H,s), 8.07(1H,d,J=2.4Hz), 10.96(1H,s).
(2) 5-(3-Carboxy-4-hydroxybenzylidene)-3-(3,5-dichlorobenzyl)thiazolidine-2,4-dione.
   The title compound was obtained in the same manner as the Example 341(4) using the following raw material.
   Raw material: 5-(3-methoxycarbonyl-4-hydroxybenzylidene)-3-(3,5-dichlorobenzyl)-thiazolidine-2,4-dione.
   Yield: 99.3%(light yellow crystal).
   ¹H-NMR(DMSO-d₆):δ 4.85(2H,s), 7.14(1H,d,J=8.7Hz), 7.40(2H,d,J=2.1Hz), 7.56(1H,t,J=2.1Hz), 7.80(1H,dd,J=8.7,2.4Hz), 7.96(1H,s), 8.09(1H,d,J=2.1Hz).
(3) 5-[4-Hydroxy-3-(3,4-dimethoxyphenylcarbamoyl)benzylidene]-3-(3,5-dichlorobenzyl)thiazolidine-2,4-dione (Compound No. 395).
   The title compound was obtained in the same manner as the following Example 357 using the following raw materials.
   Raw materials: 5-(3-carboxy-4-hydroxybenzylidene)-3-(3,5-dichlorobenzyl)-thiazolidine-2,4-dione and 3,4-dimethoxyaniline.
   Yield: 54.6%(yellow crystal).
   ¹H-NMR(DMSO-d₆): *δ* 3.75(3H,s), 3.77(3H,s), 4.85(2H,s), 6.96(1H,d,J=9.0Hz), 7.16(1H,d,J=8.4Hz), 7.23(1H,dd,J=8.7,2.4Hz), 7.40(2H,d,J=1.8Hz), 7.43(1H,d,J=2.1Hz), 7.57(1H,t,J=1.8Hz), 7.69(1H,dd,J=8.7,2.1Hz), 7.92(1H,s), 8.20(1H,d,J=2.4Hz), 10.29(1H,s), 12.27(1H,brs).

### Example 396: Preparation of the compound of Compound No. 396.

The title compound was obtained in the same manner as the Example 373 using the following raw material.
Raw material: 5-[4-hydroxy-3-(3,4-dimethoxyphenylcarbamoyl)benzylidene]-3-(3,5-dichlorobenzyl)thiazolidine-2,4-dione(Compound No. 395).
Yield: 73.7%(yellow crystal).
¹H-NMR(DMSO-d₆):δ 4.85(2H,s), 6.71(1H,d, J=8.4Hz), 6.91(1H,dd, J=8.4,2.1Hz), 7.14(1H,d, J=8.7Hz), 7.25(1H,d, J=2.1Hz), 7.40(2H,d, J=2.1Hz), 7.56-7.58(1H,m), 7.67(1H,dd,J=8.7,2.1Hz), 7.91(1H,s), 8.21(1H,d,J=2.1Hz), 8.79(1H,brs), 9.06(1H,brs), 10.12(1H,s), 12.39(1H,s).

### Example 397: Preparation of the compound of Compound No. 397.

(1) 5-(3-Methoxycarbonyl-4-hydroxybenzylidene)-3-(2,4-dichlorobenzyl)-thiazolidine-2,4-dione.
   The title compound was obtained in the same manner as the Example 2(2) using the following raw materials.
   Raw materials: methyl 5-formylsalicylate(compound of the Example 393(1)) and 3-(2,4-dichlorobenzyl)thiazolidine-2,4-dione(compound of the Example 22(1)).
   Yield: 61.9%.
(2) 5-(3-Carboxy-4-hydroxybenzylidene)-3-(2,4-dichlorobenzyl)thiazolidine-2,4-dione.
   The title compound was obtained in the same manner as the Example 341(4) using the following raw material.
   Raw material: 5-(3-methoxycarbonyl-4-hydroxybenzylidene)-3-(2,4-dichlorobenzyl)-thiazolidine-2,4-dione.
   Yield: 83.8%(light yellow crystal).
   ¹H-NMR(DMSO-d₆): *δ* 4.88(2H,s), 7.12(1H,d,J=9.0Hz), 7.32(1H,d,J=8.4Hz), 7.41(1H,dd,J=8.4,1.5Hz), 7.68(1H,d,J=2.4Hz), 7.80(1H,dd,J=8.7,1.8Hz), 7.97(1H,s), 8.10(1H,d,J=1.8Hz).
(3) 5-[4-Hydroxy-3-(3,4-dimethoxyphenylcarbamoyl)benzylidene]-3-(2,4-dichlorobenzyl)thiazolidine-2,4-dione (Compound No. 397).
   The title compound was obtained in the same manner as the following Example 357 using the following raw materials.
   Raw materials: 5-(3-carboxy-4-hydroxybenzylidene)-3-(2,4-dichlorobenzyl)-thiazolidine-2,4-dione and 3,4-dimethoxyaniline.
   Yield: 24.1%(light ocherous crystal).
   ¹H-NMR(DMSO-d₆): δ 3.75(3H,s), 3.77(3H,s), 4.89(2H,s), 6.96(1H,d,J=8.7Hz), 7.16(1H,d,J=8.7Hz), 7.23(1H,dd,J=8.4,2.4Hz), 7.32(1H,d,J=8.4Hz), 7.40-7.44(2H,m), 7.69(1H,d,J=1.8Hz), 7.70(1H,dd,J=8.4,2.4Hz), 7.93(1H,s), 8.21(1H,d,J=2.4Hz), 10.31(1H,s), 12.28(1H,brs).

### Example 398: Preparation of the compound of Compound No. 398.

The title compound was obtained in the same manner as the Example 373 using the following raw material.
Raw material: 5-[4-hydroxy-3-(3,4-dimethoxyphenylcarbamoyl)benzylidene]-3-(2,4-dichlorobenzyl)thiazolidine-2,4-dione(Compound No. 397).
Yield: 75.2%(yellow crystal).
¹H-NMR(DMSO-d₆): *δ* 4.89(2H,s), 6.71(1H,d,J=8.1Hz), 6.91(1H,dd,J=8.7,2.4Hz), 7.15(1H,d,J=8.7Hz), 7.25(1H,d,J=2.4Hz), 7.32(1H,d,J=8.7Hz), 7.42(1H,dd,J=8.4,2.1Hz), 7.67-7.70(2H,m), 7.92(1H,s), 8.22(1H,d,J=2.1Hz), 8.78(1H,brs), 9.04(1H,brs), 10.13(1H,s), 12.40(1H,s).

### Example 399: Preparation of the compound of Compound No. 399.

(1) 5-(3-Methoxycarbonyl-4-hydroxybenzylidene)-3-(3-phenoxybenzyl)-thiazolidine-2,4-dione.
   The title compound was obtained in the same manner as the Example 2(2) using the following raw materials.
   Raw materials: methyl 5-formylsalicylate(compound of the Example 393(1)) and 3-(3-phenoxybenzyl)thiazolidine-2,4-dione(compound of the Example 16(1)).
   Yield: 72.5%(white crystal).
   ¹H-NMR(DMSO-ds): *δ* 3.91(3H,s), 4.82(2H,s), 6.89-6.93(1H,m), 6.98-7.07(4H,m), 7.13-7.17(2H,m), 7.33-7.42(3H,m), 7.78(1H,dd,J=8.7,2.4Hz), 7.95(1H,s), 8.06(1H,d,J=2.7Hz), 10.97(1H,brs).
(2) 5-(3-Carboxy-4-hydroxybenzylidene)-3-(3-phenoxybenzyl)thiazolidine-2,4-dione.
   The title compound was obtained in the same manner as the Example 341(4) using the following raw material.
   Raw material: 5-(3-methoxycarbonyl-4-hydroxybenzylidene)-3-(3-phenoxybenzyl)-thiazolidine-2,4-dione.
   Yield: 97.6%(orangish white crystal).
   ¹H-NMR(DMSO-d₆): *δ* 4.82(2H,s), 6.89-6.92(1H,m), 6.98-7.07(4H,m), 7.12-7.17(2H,m), 7.36-7.42(3H,m), 7.78-7.81(1H,m), 7.96(1H,s), 8.09(1H,d,J=0.9Hz).
(3) 5-[4-Hydroxy-3-(3,4-dimethoxyphenylcarbamoyl)benzylidene]-3-(3-phenoxybenzyl)thiazolidine-2,4-dione (Compound No. 399).
   The title compound was obtained in the same manner as the following Example 357 using the following raw materials.
   Raw materials: 5-(3-carboxy-4-hydroxybenzylidene)-3-(3-phenoxybenzyl)-thiazolidine-2,4-dione and 3,4-dimethoxyaniline.
   Yield: 33.0%(white crystal).
   ¹H-NMR(DMSO-d₆): δ 3.75(3H,s), 3.77(3H,s), 4.83(2H,s), 6.90-7.07(6H,m), 7.13-7.17(2H,m), 7.23(1H,dd,J=8.4,2.1Hz), 7.34-7.42(4H,m), 7.68(1H,dd,J=8.7,2.1Hz), 7.92(1H,s), 8.20(1H,d,J=2.1Hz), 10.28(1H,s), 12.28(1H,s).

### Example 400: Preparation of the compound of Compound No. 400.

The title compound was obtained in the same manner as the Example 373 using the following raw material.
Raw material: 5-[4-hydroxy-3-(3,4-dimethoxyphenylcarbamoyl)benzylidene]-3-(3-phenoxybenzyl)thiazolidine-2,4-dione(Compound No. 399).
Yield: 88.7%(light yellow crystal).
¹H-NMR(DMSO-d₆): δ 4.83(2H,s), 6.71(1H,d,J=8.4Hz), 6.90-6.93(2H,m), 6.98-7.07(4H,m), 7.13-7.18(2H,m), 7.25(1H,d,J=2.1Hz), 7.34-7.43(3H,m), 7.67(1H,dd,J=8.7,2.1Hz), 7.91(1H,s), 8.20(1H,d,J=2.1Hz), 9.07(2H,br), 10.12(1H,s), 12.39(1H,s).

### Example 401: Preparation of the compound of Compound No. 401.

(1) 5-[3-Methyl-4-hydroxy-5-(3,4-dimethoxyphenylcarbamoyl)-benzylidene]-3-(3,4-dichlorobenzyl)thiazolidine-2,4-dione.
   The title compound was obtained in the same manner as the Example 388(1) using the following raw materials.
   Raw materials: 5-(3-methyl-4-hydroxy-5-carboxybenzylidene)-3-(3,4-dichlorobenzyl)-thiazolidine-2,4-dione(Compound No. 390) and 3,4-dimethoxyaniline.
   Yield: 22.4%(yellow solid).
   ¹H-NMR(CDCl₃):δ 2.33(3H,s), 3.91(3H,s), 3.93(3H,s), 4.84(2H,s), 6.89(1H,d,J=8.4Hz), 7.01(1H,dd,J=8.4,2.1Hz), 7.27-7.30(2H,m), 7.41(1H,d,J=8.4Hz), 7.46(1H,s), 7.52(1H,dd,J=8.1,1.8Hz), 7.83(1H,s), 7.87(1H,s), 12.77(1H,s).
(2) 5-[3-Methyl-4-hydroxy-5-(3,4-dihydroxyphenylcarbamoyl)-benzylidene]-3-(3,4-dichlorobenzyl)thiazolidine-2,4-dione.
   The title compound was obtained in the same manner as the Example 373 using the following raw material.
   Raw material: 5-[3-methyl-4-hydroxy-5-(3,4-dimethoxyphenylcarbamoyl)-benzylidene]-3-(3,4-dichlorobenzyl)thiazolidine-2,4-dione.
   Yield: 28.5%(yellowish white powder).
   ¹H-NMR(DMSO-d₆): *δ* 2.27(3H,s), 4.85(2H,s), 6.73(1H,d,J=8.7Hz), 6.92(1H,dd,J=8.4,2.1Hz), 7.17(1H,d,J=2.1Hz), 7.30(1H,dd,J=8.4,2.4Hz), 7.55(1H,s), 7.62(1H,s), 7.63(1H,d,J=8.4Hz), 7.84(1H,s), 8.23(1H,d,J=2.1Hz), 8.88(1H,s), 9.11(1H,s), 10.27(1H,s), 13.26(1H,s).

### Example 402: Preparation of the compound of Compound No. 402.

The title compound was obtained in the same manner as the Example 373 using the following raw material.
Raw material: 5-[4-hydroxy-3-(3,4-dimethoxyphenylcarbamoyl)benzylidene]-3-[4-(trifluoromethyl)benzyl]thiazolidine-2,4-dione(Compound No. 391).
Yield: 84.3%(brown solid).
¹H-NMR(DMSO-d₆): *δ* 4.87(2H,s), 6.71(1H,d,J=8.7Hz), 6.91(1H,dd,J=8.7,2.4Hz), 7.15(1H,d,J=8.7Hz), 7.25(1H,d,J=2.4Hz), 7.52(2H,d,J=8.1Hz), 7.64-7.76(3H,m), 7.93(1H,s), 8.21(1H,d,J=2.4Hz), 8.80(1H,brs), 9.05(1H,brs), 10.13(1H,s), 12.39(1H,s).

### Example 403: Preparation of the compound of Compound No. 403.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and 2,5-bis(trifluoromethyl)aniline.
Yield: 18.4%(yellow crystal).
¹H-NMR(DMSO-d₆):δ 4.84(1H,s), 7.20(1H,d,J=8.4Hz), 7.31(1H,dd,J=8.4,1.8Hz), 7.62(1H,d,J=7.8Hz), 7.62(1H,d,J=2.1Hz), 7.75-7.81(2H,m), 7.95(1H,s), 8.04(1H,d,J=8.1Hz), 8.32(1H,d,J=2.1Hz), 8.80(1H,s), 11.08(1H,s), 12.89(1H,brs).

### Example 404: Preparation of the compound of Compound No. 404.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and 3,5-bis(trifluoromethyl)aniline.
Yield: 52.9%(yellow crystal).
¹H.NMR(DMSO.d₆): δ 4.84(2H,s), 7.19(1H,d,J=8.7Hz), 7.29-7.32(1H,m), 7.61-7.64(2H,m), 7.70-7.73(1H,m), 7.84(1H,s), 7.93(1H,s), 8.07(1H,d,J=1.5Hz), 8.46(2H,s), 10.89(1H,s), 11.84(1H,brs).

### Example 405: Preparation of the compound of Compound No. 405.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and methyl 4-amino-2-methoxybenzoate.
Yield: 93.4%(yellow crystal).
¹H-NMR(DMSO-d₆): *δ* 3.77(3H,s), 3.83(3H,s), 4.84(2H,s), 7.17(1H,d,J=8.7Hz), 7.30(1H,dd,J=8.4,1.8Hz), 7.35(1H,dd,J=8.4,1.8Hz), 7.61-7.74(5H,m), 7.93(1H,s), 8.10(1H,d,J=2.1Hz), 10.74(1H,s), 12.03(1H,br).

### Example 407: Preparation of the compound of Compound No. 407.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and 5-amino-2-methoxypyridine.
Yield: 98.8%(light brown crystal).
¹H-NMR(DMSO-d₆): δ 4.85(2H,s), 6.88(1H,d,J=9.0Hz), 7.19(1H,d,J=8.7Hz), 7.31(1H,dd,J=8.7,2.1Hz), 7.61-7.64(2H,m), 7.70(1H,dd,J=9.0,2.1Hz), 7.92(1H,s), 8.04(1H,dd,J=8.7,2.4Hz), 8.19(1H,d,J=2.1Hz), 8.49(1H,d,J=2.4Hz), 10.40(1H,s), 12.22(1H,brs).

### Example 408: Preparation of the compound of Compound No. 408.

A mixture of 5-{4-hydroxy-3-[(2-methoxypyridin-5-yl)carbamoyl]benzylidene}-3-(3,4-dichlorobenzyl)thiazolidine-2,4-dione(Compound No. 407; 53 mg, 0.1 mmol), potassium iodide(33 mg, 0.2 mmol) and acetic acid(2 ml) was refluxed for 3 hours. The reaction mixture was poured into water and extracted with ethyl acetate. The organic layer was washed with water and saturated aqueous sodium hydrogencarbonate, and dried over anhydrous magnesium sulfate. The residue obtained by evaporation of the solvent under reduced pressure was washed with ethyl acetate under suspension to give the title compound(36 mg, 69.8%) as a yellowish brown solid.
¹H-NMR(DMSO·d₆): δ 4.84(2H,s), 6.40(1H,d,J=9.6Hz), 7.14(1H,d,J=9.0Hz), 7.31(1H,dd,J=8.4,2.1Hz), 7.59-7.64(3H,m), 7.67(1H,dd,J=8.4,2.7Hz), 7.90(1H,s), 7.94(1H,d,J=2.7Hz), 8.12(1H,d,J=2.4Hz), 10.20(1H,brs), 11.65(1H,brs), 12.15(1H,brs).

### Example 409: Preparation of the compound of Compound No. 409.

(1) 5-(3-Benzyloxycarbonyl-4-hydroxybenzylidene)thiazolidine-2,4-dione.
   The title compound was obtained in the same manner as the Example 350(2) using the following raw materials.
   Raw materials: 5-formylsalicylic acid benzyl ester(compound of the Example 363(1)) and thiazolidine-2,4-dione.
   Yield: 62.7%(white crystal):
   ¹H·NMR(DMSO·d₆): *δ* b 5.41(2H,s), 7.14(1H,d,J=8.7Hz), 7.37-7.44(3H,m), 7.51-7.53(2H,m), 7.75(1H,dd,J=8.7,2.1Hz), 7.78(1H,s), 8.06(1H,d,J=2.1Hz), 10.93(1H,s), 12.56(1H,brs).
(2) 5-(3-Benzyloxycarbonyl-4-hydroxybenzylidene)-3-(4-methoxycarbonylbenzyl)-thiazolidine-2,4-dione.
   A mixture of 5-(3-benzyloxycarbonyl-4-hydroxybenzylidene)thiazolidine-2,4-dione (355 mg, 1 mmol), methyl 4-(bromomethyl)benzoate(229 mg, 1 mmol), potassium carbonate(276 mg, 2 mmol) and N,N-dimethylformamide(5 ml) was stirred at 55°C for 5 hours. The reaction mixture was cooled. Water was added to the mixture, and the mixture was extracted with ethyl acetate. The organic layer was washed with water and brine, and dried over anhydrous magnesium sulfate. The residue obtained by evaporation of the solvent under reduced pressure was washed with ethyl acetate under suspension to give the title compound(364.6 mg, 72.5%) as a yellow solid.
   ¹H-NMR(DMSO-d₆): *δ* 3.84(3H,s), 4.91(2H,s), 5.41(2H,s), 7.16(1H,d,J=8.7Hz), 7.36-7.46(5H,m), 7.51-7.53(2H,m), 7.79(1H,dd,J=8.7,2.1Hz), 7.92(1H,s), 7.96(2H,d,J=8.4Hz), 8.11(1H,d,J=2.1Hz), 10.97(1H,brs).
(3) 5-(3-Carboxy-4-hydroxybenzylidene)-3-(4-methoxycarbonylbenzyl)-thiazolidine-2,4-dione (Compound No. 409).
   The title compound was obtained in the same manner as the Example 372(1) using the following raw material.
   Raw material: 5-(3-benzyloxycarbonyl-4-hydroxybenzylidene)-3-(4-methoxycarbonylbenzyl)thiazolidine-2,4-dione.
   Yield: 86.1%(white crystal).
   ¹H-NMR(DMSO-d₆): *δ* 3.84(3H,s), 4.92(2H,s), 7.13(1H,d,J=8.7Hz), 7.45(2H,d,J=8.1Hz), 7.80(1H,dd,J=8.7,2.4Hz), 7.94(2H,d,J=8.1Hz), 7.97(1H,s), 8.10(1H,d,J=2.1Hz).

### Example 410: Preparation of the compound of Compound No. 410.

The title compound was obtained in the same manner as the Example 2(2) using the following raw materials.
Raw materials: 3-formylindole and 3-(3,4-dichlorobenzyl)thiazolidine-2,4-dione(compound of the Example 8(1)).
Yield: 91.1%(yellow solid).
¹H-NMR(DMSO-d₆):δ 4.85(2H,s), 7.19-7.27(2H,m), 7.31(1H,dd,J=8.4,2.4Hz), 7.52(1H,d,J=7.8Hz), 7.6l(1H,s), 7.63(1H,d,J=8.4Hz), 7.83(1H,d,J=2.7Hz), 7 .92(1H,d,J =7 .2Hz), 8.23(1H,s), 12.21 (1H,s).

### Example 411: Preparation of the compound of Compound No. 411.

Sodium hydride(20 mg, 0.5 mmol) was added to a solution of 5-(indol-3-ylmethylene)-3-(3,4-dichlorobenzyl)thiazolidine-2,4-dione(Compound No. 410; 200 mg, 0.49 mmol) in DMF(5 ml) under ice cooling, and the mixture was stirred for 5 minutes. 3,4-Dibenzyloxybenzyl chloride(168 mg, 0.49 mmol) was added to the mixture, and the mixture was stirred at room temperature for 3 hours. The reaction mixture was poured into diluted hydrochloric acid and extracted with ethyl acetate. The organic layer was washed with water and brine, and dried over anhydrous sodium sulfate. The residue obtained by evaporation of the solvent under reduced pressure was washed with ethyl acetate/isopropyl ether under suspension to give the title compound(186.7 mg, 53.3%) as a yellow solid.
¹H-NMR(DMSO-d₆): δ 4.86(2H,s), 5.06(2H,s), 5.08(2H,s), 5.46(2H,s), 6.82(1H,dd,J=8.4,1.8Hz), 6.99(1H,d,J=8.1Hz), 7.17(1H,d,J=1.8Hz), 7.22-7.41(13H,m), 7.57(1H,dd,J=6.9,1.8Hz), 7.62(1H,s), 7.63(1H,d,J=8.4Hz), 7.94(1H,dd,J=6.9,1.8Hz), 8.05(1H,s), 8.21(1H,s).

### Example 412: Preparation of the compound of Compound No. 412.

3,4-Dimethoxybenzoyl chloride(100 mg, 0.49 mmol) and triethylamine(0.1 ml, 0.72 mmol) were added to a solution of 5-(indol-3-ylmethylene)-3-(3,4-dichlorobenzyl)-thiazolidine-2,4-dione(Compound No. 410; 200 mg, 0.49 mmol) in DMF(5 ml), and the mixture was stirred at room temperature for 18 hours. The reaction mixture was poured into diluted hydrochloric acid. The separated solid was collected by filtration and washed with ethyl acetate under suspension to give the title compound(38.1 mg, 13.5%) as a light yellowish white solid.
1H-NMR(DMSO-ds): δ 3.85(3H,s), 3.91(3H,s), 4.85(2H,s), 7.22(1H,d,J=9.0Hz), 7.31(1H,dd,J=8.4,2.1Hz), 7.43-7.53(4H,m), 7.61-7.64(2H,m), 7.76(1H,s), 8.06-8.08(1H,m), 8.19-8.24(2H,m).

### Example 413: Preparation of the compound of Compound No. 413.

The title compound was obtained in the same manner as the Example 373 using the following raw material.
Raw material: 5-[1-(3,4-dibenzyloxybenzyl)indol-3-ylmethylene]-3-(3,4-dichlorobenzyl)thiazolidine-2,4-dione(Compound No. 411).
Yield: 78.7%(yellow powder).
¹H-NMR(DMSO-d₆): *δ* 4.85(2H,s), 5.40(2H,s), 6.62-6.69(3H,m), 7.20-7.28(2H,m), 7.31(1H,dd,J=8.4,2.1Hz), 7.55-7.57(1H,m), 7.62-7.64(2H,m), 7.94(1H,dd,J=7.2, 1.2Hz), 8.02(1H,s), 8.21(1H,s), 8.90(2H,s).

### Example 414: Preparation of the compound of Compound No. 414.

The title compound was obtained in the same manner as the Example 357 using the following raw materials.
Raw materials: 5-(4-hydroxy-3-carboxybenzylidene)-3-(3,4-dichlorobenzyl)-thiazolidine-2,4-dione(Compound No. 358) and 3-(trifluoromethoxy)aniline.
Yield: 60.6%(light yellow solid).
1H-NMR(DMSO-ds): δ 4.85(2H,s), 7.10-7.19(2H,s), 7.31(1H,dd,J=1.8,8.4Hz), 7.51(1H,t,J=8.1Hz), 7.61-7.72(4H,m), 7.94(2H,s), 8.08(1H,d,J=2.7Hz), 10.59(1H,s), 11.93(1H,brs).

### Example 415: Preparation of the compound of Compound No. 415.

The title compound was obtained in the same manner as the Example 357 using the following raw materials.
Raw materials: 5-(3-carboxy-4-hydroxybenzylidene)-3-[4-(methoxycarbonyl)-benzyl]thiazolidine-2,4-dione(Compound No. 409) and 3,4-dichloroaniline.
Yield: 66.6%(light yellow crystal).
¹H-NMR(DMSO-d₆): *δ* 3.84(3H,s), 4.92(2H,s), 7.17(1H,d,J=8.7Hz), 7.45(1H,d,J=8.4Hz), 7.61-7.72(3H,m), 7.93-7.96(3H,m), 8.07-8.08(1H,m), 8.14-8.20(1H,m), 10.57(1H,s), 11.92(1H,brs).

### Example 416: Preparation of the compound of Compound No. 416.

The title compound was obtained in the same manner as the Example 357 using the following raw materials.
Raw materials: 5-(4-hydroxy-3-carboxybenzylidene)-3-(3,4-dichlorobenzyl)-thiazolidine-2,4-dione(Compound No. 358) and methyl 4-aminobenzoate.
Yield: 47.6%(yellow crystal).
¹H-NMR(DMSO-d₆): δ 3.85(3H,s), 4.85(2H,S), 7.18(1H,d,J=8.7Hz), 7.31(1H,dd,J=8.4,2.1Hz), 7.62(1H,d,J=2.1Hz), 7.63(1H,d,J=8.1Hz), 7.70(1H,dd,J=8.7,1.8Hz), 7.89(2H,d,J=8.7Hz), 7.94(1H,s), 7.98(2H,d,J=9.0Hz), 8.10(1H,d,J=2.1Hz), 10.65(1H,s), 11.98(1H,brs).

### Example 417: Preparation of the compound of Compound No. 417.

The title compound was obtained in the same manner as the Example 357 using the following raw materials.
Raw materials: 5-(4-hydroxy-3-carboxybenzylidene)-3-(3,4-dichlorobenzyl)-thiazolidine-2,4-dione(Compound No. 358) and methyl 3-aminobenzoate.
Yield: 39.2%(light yellow crystal).
¹H-NMR(DMSO-d₆): δ 3.88(3H,s), 4.85(2H,s), 7.18(1H,d,J=8.4Hz), 7.31(1H,dd,J=8.4,1.8Hz), 7 .54(1H,t,J=7 .8Hz), 7 .62(1H,d,J=2. 7Hz), 7.63(1H,d,J=8.1Hz), 7.68-7.75(2H,m), 7.94-7.97(2H,m), 8.15(1H,d,J=2.1Hz), 8.44(1H,s), 10.55(1H,s), 12.02(1H,brs).

### Example 418: Preparation of the compound of Compound No. 418.

The title compound was obtained in the same manner as the Example 373 using the following raw material.
Raw material: 5-[4-hydroxy-3-(3,4-dichlorophenylcarbamoyl)benzylidene]-3-(4-methoxycarbonylbenzyl)thiazolidine-2,4-dione(Compound No. 415).
Yield: 91.9%(light yellow crystal).
¹H-NMR(DMSO-d₆): *δ* 4.91(2H,s), 7.18(1H,d,J=8.7Hz), 7.43(2H,d,J=8.1Hz), 7.62-7.72(3H,m), 7.93(2H,d,J=8.7Hz), 7.94(1H,s), 8.08(1H,d,J=2.1Hz), 8.14(1H,d,J=2.1Hz), 10.55(1H,s), 11.91(1H,brs).

### Example 419: Preparation of the compound of Compound No. 419.

The title compound was obtained in the same manner as the Example 373 using the following raw material.
Raw material: 5-{4-hydroxy-3-[4-(methoxycarbonyl)phenylcarbamoyl]benzylidene}-3-(3,4-dichlorobenzyl)thiazolidine-2,4-dione(Compound No. 416).
Yield: 68.4%(light yellowish brown crystal).
¹H-NMR(DMSO-d₆): δ 4.85(2H,s), 7.18(1H,d,J=8.7Hz), 7.31(1H,dd,J=8.4,1.8Hz), 7.62(1H,d,J=1.8Hz), 7.63(1H,d,J=8.4Hz), 7.70(1H,dd,J=8.4,2.1Hz), 7.86(2H,d,J=8.7Hz), 7.94(1H,s), 7.96(2H,d,J=8.7Hz), 8.11(1H,d,J=2.1Hz), 10.60(1H,s), 11.98(1H,brs).

### Example 420: Preparation of the compound of Compound No. 420.

The title compound was obtained in the same manner as the Example 373 using the following raw material.
Raw material: 5-{4-hydroxy-3-[3-(methoxycarbonyl)phenylcarbamoyl]benzylidene}-3-(3,4-dichlorobenzyl)thiazolidine-2,4-dione(Compound No. 417).
Yield: 57.4%(grayish white crystal).
¹H-NMR(DMSO-d₆): *δ* 4.85(2H,s), 7.18(1H,d,J=8.7Hz), 7.29-7.33(1H,m), 7.51(1H,t,J=8.4Hz), 7.61-7.64(2H,m), 7.69-7.73(2H,m), 7.93-7.95(2H,m), 8.16(1H,m), 8.38(1H,s), 10.54(1H,s), 12.05(1H,brs).

### Example 421: Preparation of the compound of Compound No. 421.

The title compound was obtained in the same manner as the Example 357 using the following raw materials.
Raw materials: 5-(4-hydroxy-3-carboxybenzylidene)-3-(3,4-dichlorobenzyl)-thiazolidine-2,4-dione(Compound No. 358) and 2-(4-methoxyphenoxy)-5-(trifluoromethyl)aniline.
Yield: 41.6%(light yellow crystal).
¹H-NMR(DMSO-d₆): *δ* 3.78(3H,s), 4.85(2H,s), 6.90(1H,d,J=8.4Hz), 7.04(2H,d,J=8.4Hz), 7.16(1H,d,J=9.0Hz), 7.21(2H,d,J=8:7Hz), 7.32(1H,d,J=8.7H), 7.43(1H,d,J=8.7Hz), 7.61-7.64(2H,m), 7.74-7.77(1H,m), 7.95(1H,s), 8.35(1H,d,J=1.5Hz), 8.98(1H,s), 11.31(1H,s), 12.75(1H,brs).

### Example 422: Preparation of the compound of Compound No. 422.

The title compound was obtained in the same manner as the Example 373 using the following raw material.
Raw material: 5-(4-hydroxy-3-{[2-(4-methoxyphenoxy)-5-(trifluoromethyl)-phenyl]carbamoyl}benzylidene)-3-(3-phenoxybenzyl)thiazolidine-2,4-dione(Compound No. 421).
Yield: 100.0%(grayish black crystal).
Yield: 100.0%(gray crystal)
¹H-NMR(DMSO-ds): *δ* 4.85(2H,s), 6.85(2H,d,J=9.0Hz), 6.87(1H,d,J=8.4Hz), 7.08(2H,d,J=8.7Hz), 7.16(1H,d,J=8.7Hz), 7.31(1H,dd,J=8.7,1.8Hz), 7.39-7.43(1H,m), 7.63(1H,d,J=2.1Hz), 7.63(1H,d,J=8.1Hz), 7.76(1H,dd,J=8.4,2.4Hz), 7.95(1H,s), 8.35(1H,d,J=2.1Hz), 8.97(1H,s), 9.55(1H,brs), 11.30(1H,s).

### Example 423: Preparation of the compound of Compound No. 423.

The title compound was obtained in the same manner as the Example 357 using the following raw materials.
Raw materials: 5-(4-hydroxy-3-carboxybenzylidene)-3-(3,4-dichlorobenzyl)-thiazolidine-2,4-dione(Compound No. 358) and 2-chloro-5-(trifluoromethyl)aniline.
Yield: 35.3%(light yellow crystal).
¹H-NMR(DMSO-d₆): δ 4.85(2H,s), 7.21(1H,d,J=9.0Hz), 7.32(1H,dd,J=8.7,2.1Hz), 7.55(1H,dd,J=8.4,2.1Hz), 7.63(1H,d,J=1.8Hz), 7.63(1H,d,J=8.1Hz), 7.79(1H,dd,J=8.4,2.1Hz), 7.84(1H,d,J=8.4Hz), 7.96(1H,s), 8.33(1H,d,J=2.1Hz), 8.94(1H,d,J=1.8Hz), 11.18(1H,s).

### Example 424: Preparation of the compound of Compound No. 424.

The title compound was obtained in the same manner as the Example 357 using the following raw materials.
Raw materials: 5-(4-hydroxy-3-carboxybenzylidene)-3-(3,4-dichlorobenzyl)-thiazolidine-2,4-dione(Compound No. 358) and 3-nitroaniline.
Yield: 65.6%(light yellowish brown crystal).
¹H-NMR(DMSO-d₆): δ 4.85(2H,s), 7.19(1H,d,J=8.7Hz), 7.31(1H,dd,J=8.1,1.8Hz), 7.62-7.64(2H,m), 7.69(1H,d,J=7.8Hz), 7.71(1H,dd,J=7.8,2.1Hz), 7.94(1H,s), 7.98-8.02(1H,m), 8.05-8.10(2H,m), 8.80(1H,t,J=2.1Hz), 10.76(1H,s).

### Example 425: Preparation of the compound of Compound No. 425.

The title compound was obtained in the same manner as the Example 411 using the following raw materials.
Raw materials: 5-(indol-3-ylmethylene)-3-(3,4-dichlorobenzyl)thiazolidine-2,4-dione(Compound No. 410) and methyl 4-(bromomethyl)benzoate.
Yield: 87.9%(orange powder).
¹H-NMR(DMSO-d₆): *δ* 3.82(3H,s), 4.85(2H,s), 5.72(2H,s), 7.23-7.27(2H,m), 7.31(1H,dd,J=8.4,2.1Hz), 7.37(2H,d,J=8.4Hz), 7.50-7.53(1H,m), 7.62-7.64(2H,m), 7.92(2H,d,J=8.1Hz), 7.96-7 .99(1H,m), 8.14(1H,s), 8.23(1H,s).

### Example 426: Preparation of the compound of Compound No. 426.

(1) 3-Phenyl-4-hydroxy-5-carboxybenzaldehyde.
   The title compound was obtained in the same manner as the Example 390(1) using the following raw materials.
   Raw materials: 3-phenylsalicylic acid and hexamethylenetetramine.
   Yield: 66.7%(slight yellowish white powder).
   ¹H-NMR(DMSO-d₆): *δ* 7.37-7.50(3H,m), 7.59-7.63(2H,m), 8.04(1H,d,J=2.1Hz), 8.39(1H,d,J=1.8Hz), 9.94(1H,s).
(2) 3-Phenyl-4-hydroxy-5-(benzyloxycarbonyl)benzaldehyde.
   Benzyl bromide(0.4 ml, 3.36 mmol) was added to a mixture of potassium fluoride(0.4g, 6.88 mmol) and dimethylformamide(5 ml) under argon atmosphere, and the mixture was stirred at room temperature for 10 minutes.
   3-Phenyl-4-hydroxy-5-carboxybenzaldehyde(0.75g, 3.11 mmol) was added to the mixture, and the mixture was stirred at 100°Cfor 1.5 hours. The reaction mixture was poured into water and extracted with ethyl acetate. The organic layer was washed with water and brine, and dried over anhydrous sodium sulfate. The residue obtained by evaporation of the solvent under reduced pressure was purified by chromatography on silica gel(ethyl acetate:hexane=3:1) to give the title compound (0.82g, 78.8%) as a yellow clear oil.
   ¹H-NMR(CDCl₃): *δ* 5.45(2H,s), 7.37-7.50(8H,m), 7.57-7.60(2H,m), 8.06(1H,d,J=2.4Hz), 8.4l(1H,d,J=2.1Hz), 9.91(1H,s), 11.93(1H,s).
(3) 5-[3-Phenyl-4-hydroxy-5-(benzyloxycarbonyl)benzylidene]-3-(3,4-dichlorobenzyl)thiazolidine-2,4-dione (Compound No. 426).
   The title compound was obtained in the same manner as the Example 2(2) using the following raw materials.
   Raw materials: 3-phenyl-4-hydroxy-5-(benzyloxycarbonyl)benzaldehyde and 3-(3,4-dichlorobenzyl)thiazolidine-2,4-dione(compound of the Example 8(1)).
   Yield: 55.4%(light yellowish white solid).
   ¹H-NMR(DMSO-d₆) : δ 4.84(2H,s), 5.49(2H,s), 7.30(1H,dd,J=8.4,2.1Hz), 7.39-7.51(6H,m), 7.54-7.63(6H,m), 7.90(1H,d,J=2.1Hz), 8.05(1H,s), 8.18(1H,d,J=2.1Hz), 11.43(1H,s).

### Example 427: Preparation of the compound of Compound No. 427.

Sodium hydride(30 mg, 0.75 mmol) was added to a solution of 5-(indol-3-ylmethylene)-3-(3,4-dichlorobenzyl)thiazolidine-2,4-dione(Compound No. 410; 200 mg, 0.49 mmol) in DMF(5 ml) under ice cooling, and the mixture was stirred for 15 minutes. Terephthalic acid monomethyl ester chloride(100 mg, 0.50 mmol) was added to the mixture, and the mixture was stirred at room temperature for 2 hours. The reaction mixture was poured into water and extracted with ethyl acetate. The organic layer was washed with water and brine, and dried over anhydrous sodium sulfate. The residue obtained by evaporation of the solvent under reduced pressure was washed with ethyl acetate/isopropyl ether under suspension to give the title compound(203.3 mg, 72.5%) as a yellowish white powder.
¹H-NMR(DMSO-d₆): *δ* 3.94(3H,s), 4.84(2H,s), 7.30(1H,dd,J=8.7,2.4Hz), 7.45-7.63(5H,m), 8.00(2H,d,J=8.1Hz), 8.07-8.10(1H,m), 8.17-8.22(3H,m), 8.28.8.30(1H,m).

### Example 428: Preparation of the compound of Compound No. 428.

The title compound was obtained in the same manner as the Example 357 using the following raw materials.
Raw materials: 5-(4-hydroxy-3-carboxybenzylidene)-3-(3,4-dichlorobenzyl)-thiazolidine-2,4-dione(Compound No. 358) and 3,4-methylenedioxyaniline.
Yield: 58.1%(light yellow solid).
¹H-NMR(DMSO-d₆): δ 4.84(2H,s), 6.03(2H,s), 6.92(1H,d,J=8.1Hz), 7.07-7.14(2H,m), 7.30(1H,dd,J=2.1,8.4Hz), 7.41(1H,d,J=2.1Hz), 7.60-7.68(3H,m), 7.91(1H,s), 8.15(1H,d,J=2.1Hz), 10.48(1H,brs).

### Example 429: Preparation of the compound of Compound No. 429.

This compound is identical with the compound prepared in the Example 341(3).

### Example 430: Preparation of the compound of Compound No. 430.

The title compound was obtained in the same manner as the Example 372(1) using the following raw material.
Raw material: 5-[3-phenyl-4-hydroxy-5-(benzyloxycarbonyl)benzylidene]-3-(3,4-dichlorobenzyl)thiazolidine-2,4-dione(Compound No. 426).
Yield: 32.0%(yellowish white powder).
¹H-NMR(DMSO-d₆):δ 4.84(2H,s), 7.31(1H,dd,J=8.1,2.1Hz), 7.37-7.50(3H,m), 7.60-7.64(4H,m), 7.86(1H,d,J=2.1Hz), 8.02(1H,s), 8.11(1H,d,J=2.7Hz).

### Example 431: Preparation of the compound of Compound No. 431.

The title compound was obtained in the same manner as the Example 2(2) using the following raw materials.
Raw materials:
3,5-bis(methoxymethoxy)-4-(methoxycarbonyl)benzylaldehyde(compound of the Example 342(7)) and 3-(3,4-dichlorobenzyl)thiazolidine-2,4-dione(compound of the Example 8(1)).
Yield: 85.1%(yellowish white powder).
¹H-NMR(CDCl₃): *δ* 3.48(6H,s), 3.93(3H,s), 4.83(2H,s), 5.20(4H,s), 7.00(2H,s), 7.27(1H,dd,J=8.4,2.1Hz), 7.41(1H,d,J=8.4Hz), 7.53(1H,d,J=2.1Hz), 7.83(1H,s).

### Example 432: Preparation of the compound of Compound No. 432.

The title compound was obtained in the same manner as the Example 342(8) using the following raw material.
Raw material: 5-[3,5-bis(methoxymethoxy)-4-(methoxycarbonyl)benzylidene]-3-(3,4-dichlorobenzyl)thiazolidine-2,4-dione(Compound No. 431).
Yield: 85.4% (light yellow powder).
¹H-NMR(DMSO·d₆): *δ* 3.77(3H,s), 4.83(2H,s), 6.62(2H,s), 7.31(1H,dd,J=8.1,1.8Hz), 7.62(1H,d,J=8.1Hz), 7.63(1H,d,J=1.5Hz), 7.74(1H,s), 10.30(2H,s).

### Example 433: Preparation of the compound of Compound No. 433.

The title compound was obtained in the same manner as the Example 357 using the following raw materials.
Raw materials: 5-(4-hydroxy-3-carboxybenzylidene)-3-(3,4-dichlorobenzyl)-thiazolidine-2,4-dione(Compound No. 358) and 3,4,5-trimethoxyaniline.
Yield: 99.8%(grayish white crystal).
¹H-NMR(DMSO-_{d}): *δ* 3.64(3H,s), 3.78(6H,s), 4.84(2H,s), 7.01(1H,d,J=9.0Hz), 7.12(2H,s), 7.28-7.32(1H,m), 7.58-7.64(3H,m), 7.89(1H,s), 8.14(1H,d,J=2.1Hz), 11.14(1H,brs).

### Example 434: Preparation of the compound of Compound No. 434.

The title compound was obtained in the same manner as the Example 357 using the following raw materials.
Raw materials: 5-(4-hydroxy-3-carboxybenzylidene)-3-(3,4-dichlorobenzyl)-thiazolidine-2,4-dione(Compound No. 358) and 2,3-dimethoxyaniline.
Yield: 76.0%(light yellow crystal).
¹H-NMR(DMSO-ds): *δ* 3.82(3H,s), 3.84(3H,s), 4.85(2H,s), 6.85(1H,d,J=8.4Hz), 7.09(1H,t,J=8.4Hz), 7.20(1H,d,J=8.4Hz), 7.31(1H,dd,J=8.4,2.1Hz), 7.62(1H,d,J=8.1Hz), 7.63(1H,d,J=1.8Hz), 7.75(1H,dd,J=8.4,2.1Hz), 7.96(1H,s), 8.10(1H,d,J=8.1Hz), 8.33(1H,d,J=2.1Hz), 10.97(1H,brs).

### Example 435: Preparation of the compound of Compound No. 435.

The title compound was obtained in the same manner as the Example 373 using the following raw material.
Raw material: 5-[4-hydroxy-3-(3,4,5-trimethoxyphenylcarbamoyl)benzylidene]-3-(3,4-dichlorobenzyl)thiazolidine-2,4-dione(Compound No. 433).
Yield: 21.5%(light yellow crystal).
¹H-NMR(DMSO-d₆): *δ* 4.85(2H,s), 6.72(2H,s), 7.13(1H,d,J=8.4Hz), 7.29-7.32(1H,m), 7.62-7.68(3H,m), 7.91(1H,s), 7.99(1H,s), 8.18-8.19(1H,m), 8.96(2H,s), 10.04(1H,s), 12.37(1H,brs).

### Example 436: Preparation of the compound of Compound No. 436.

The title compound was obtained in the same manner as the Example 373 using the following raw material.
Raw material: 5-[4-hydroxy-3-(2,3-dimethoxyphenylcarbamoyl)benzylidene]-3-(3,4-dichlorobenzyl)thiazolidine-2,4-dione(Compound No. 434).
Yield: 68.4%(light yellow crystal).
¹H-NMR(DMSO-d₆):δ 4.84(2H,s), 6.59(1H,dd,J=8.1, 1.8Hz), 6.65(1H,t, J=8.1Hz), 7.08(1H,d,J=8.7Hz), 7.31(1H,dd, J=8.4,2.1Hz), 7.62(1H,d, J=2.1Hz), 7.63(1H,d, J=8.4Hz), 7.68(1H,dd, J=8.4,2.1Hz), 7.76-7.78(1H,m), 7.93(1H,s), 8.31(1H,d, J=2.4Hz), 9.23(1H,s), 9.37(1H,s), 11.20(1H,brs), 12.46(1H,brs).

### Example 437: Preparation of the compound of Compound No. 437.

The title compound was obtained in the same manner as the Example 341(4) using the following raw material.
Raw material: 5-{4-hydroxy-3-[3-methoxy-4-(methoxycarbonyl)phenylcarbamoyl]-benzylidene}-3-(3,4-dichlorobenzyl)thiazolidine-2,4-dione(Compound No. 405).
Yield: 25.6%(light yellow crystal).
¹H-NMR(DMSO-d₆): *δ* 3.89(3H,s), 4.84(2H,s), 7.17(1H,d,J=8.7Hz), 7.23-7.27(1H,m), 7.29-7.33(1H,m), 7.54(1H,m), 7.62(1H,d,J=2.1Hz), 7.63(1H,d,J=8.1Hz), 7.68-7.72(1H,m), 7.79(1H,d,J=8.7Hz), 7.94(1H,s), 8.06(1H,d,J=2.1Hz), 10.56(1H,s),10.65(1H,s), 11.94(1H,brs).

### Example 438: Preparation of the compound of Compound No. 438.

The title compound was obtained in the same manner as the Example 357 using the following raw materials.
Raw materials: 5-(3-methoxy-4-hydroxy-5-carboxybenzlidene)-3-(3,4-dichlorobenzyl)thiazolidine-2,4-dione (Compound No. 345) and 3,5-bis(trifluoromethyl)aniline.
Yield: 39.3%(yellow crystal).
¹H-NMR(DMSO-d₆): *δ* 3.93(3H,s), 4.85(2H,s), 7.31(1H,dd,J=8.4,1.8Hz), 7.40(1H,d,J=1.8Hz), 7.62-7.64(3H,m), 7.85(1H,s), 7.93(1H,s), 8.42(2H,s), 10.99(1H,s).

### Example 439: Preparation of the compound of Compound No. 439.

The title compound was obtained in the same manner as the Example 357 using the following raw materials.
Raw materials: 5-(3-methoxy-4-hydroxy-5-carboxybenzlidene)-3-(3,4-dichlorobenzyl)thiazolidine-2,4-dione (Compound No. 345) and 2-chloro-5-(trifluoromethyl)aniline.
Yield: 7.2%(light yellow crystal).
¹H-NMR(DMSO·d₆): *δ* 3.94(3H,s), 4.85(2H,s), 7.29-7.33(1H,m), 7.52-7.56(2H,m), 7.62(1H,d,J=2.1Hz), 7.63(1H,d,J=8.1Hz), 7.83(1H,d,J=8.7Hz), 7.92(1H,d,J=2.1Hz), 7.96(1H,s), 8.91(1H,d,J=2.1Hz), 11.41(1H,brs).

### Example 440: Preparation of the compound of Compound No. 440.

The title compound was obtained in the same manner as the Example 357 using the following raw materials.
Raw materials: 5-(3-methoxy-4-hydroxy-5-carboxybenzlidene)-3-(3,4-dichlorobenzyl)thiazolidine-2,4-dione (Compound No. 345) and aniline.
Yield: 4.5%(yellow crystal).
¹H-NMR(DMSO-d₆): *δ* 3.92(3H,s), 4.85(2H,s), 7.15(1H,t,J=8.1Hz), 7.31(1H,dd,J=8.1,1.8Hz), 7.36-7.41(3H,m), 7.62-7.65(2H,m), 7.68-7.71(2H,m), 7.76(1H,d,J=2.1Hz), 7.93(1H,s), 10.48(1H,brs), 11.70(1H,brs).

### Example 441: Preparation of the compound of Compound No. 441.

The title compound was obtained in the same manner as the Example 357 using the following raw materials.
Raw materials: 5-(4-hydroxy-3-carboxybenzylidene)-3-(3,4-dichlorobenzyl)-thiazolidine-2,4-dione(Compound No. 358) and 2,5-dichloroaniline.
Yield: 64.1%(light yellow crystal).
¹H-NMR(DMSO-d₆): *δ* 4.85(2H,s), 7.19(1H,d,J=8.4Hz), 7.26(1H,dd,J=8.4,2.1Hz), 7.31(1H,dd,J=8.4,1.2Hz), 7.60-7.64(3H,m), 7.77(1H,dd,J=8.4,1.8Hz), 7.95(1H,s), 8.31(1H,d,J=2.4Hz), 8.64(1H,d,J=2.4Hz), 11.09(1H,s), 12.88(1H,brs).

### Example 442: Preparation of the compound of Compound No. 442.

The title compound was obtained in the same manner as the Example 357 using the following raw materials.
Raw materials: 5-(4-hydroxy-3-carboxybenzylidene)-3-(3,4-dichlorobenzyl)-thiazolidine-2,4-dione(Compound No. 358) and 2-fluoro-5-(trifluoromethyl)aniline. Yield: 63.1%(light yellow crystal).
¹H-NMR(DMSO-d₆): *δ* 4.85(2H,s), 7.17(1H,d,J=8.4Hz), 7.31(1H,d,J=8.4Hz), 7.58-7.64(4H,m), 7.76(1H,d,J=9.0Hz), 7.94(1H,s), 8.28(1H,s), 8.79(1H,d,J=6.9Hz), 11.06(1H,s), 12.73(1H,br).

### Example 443: Preparation of the compound of Compound No. 443.

(1) 4-Methoxy-3-(3,4-dimethoxyphenyl)benzaldehyde.
   3,4-Dimethoxyphenylboronic acid(457 mg, 2.512 mmol) and a solution of sodium carbonate(266 mg, 2.512 mmol) in water(1.3 ml) were added to a solution of 3-bromo-4-methoxybenzaldehyde(450 mg, 2.093 mmol) and
   tetrakis(triphenylphosphine)palladium(0)(125 mg, 0.105 mmol) in dimethoxyethane(3 ml), and the mixture was stirred at 100°C for 3 hours. The reaction mixture was cooled and diluted with ethyl acetate. The organic layer was washed with brine, and dried over anhydrous sodium sulfate. The residue obtained by evaporation of the solvent under reduced pressure was purified by chromatography on silica gel(hexane:ethyl acetate=3:2) to give the title compound(558 mg, 97.9%) as a white solid.
   ¹H-NMR(CDCl₃): δ 3.92(3H,s), 3.93(3H,s), 3.94(3H,s), 6.94(1H,d,J=8.1Hz), 7.05-7.12(3H,m), 7.82-7.87(2H,m).
(2) 5-[4-Methoxy-3-(3,4-dimethoxyphenyl)benzylidene]-3-(3,4-dichlorobenzyl)thiazolidine-2,4-dione (Compound No. 443).
   The title compound was obtained in the same manner as the Example 350(2) using the following raw materials.
   Raw materials: 4-methoxy-3-(3,4-dimethoxyphenyl)benzaldehyde and 3-(3,4-dichlorobenzyl)thiazolidine-2,4-dione(compound of the Example 8(1)).
   Yield: 79.8%(yellow solid).
   ¹H-NMR(DMSO-d₆) : *δ* 3.78(3H,s), 3.79(3H,s), 3.86(3H,s), 4.84(2H,s), 7.01-7.10(3H,m), 7.28(1H,d,J=9.3Hz), 7.30(1H,dd,J=1.8,8.4Hz), 7.58-7.64(4H,m), 7.80(1H,s).

### Example 444: Preparation of the compound of Compound No. 444.

Boron tribromide(1.0M solution in dichloromethane; 1.4 ml, 1.4 mmol) was added slowly to a mixture of 5-[4-methoxy-3-(3,4-dimethoxyphenyl)-benzylidene]-3-(3,4-dichlorobenzyl)thiazolidine-2,4-dione(Compound No. 443; 150 mg, 0.283 mmol) and dichloromethane(3.5 m.) at 0°C, and the mixture was stirred at room temperature for 30 minutes. Water was added to the reaction mixture, and the mixture was extracted with ethyl acetate. The organic layer was washed with brine and dried over anhydrous sodium sulfate. The residue obtained by evaporation of the solvent under reduced pressure was washed with hexane/ethyl acetate under suspension to give the title compound(131 mg, 92.1%) as a yellow solid.
¹H-NMR(DMSO-d₆): δ 3.85(3H,s), 4.84(2H,s), 6.79(2H,s), 6.95(1H,s), 7.25(1H,d,J=8.7Hz), 7.30(1H,dd,J=2.1,8.4Hz), 7.49-7.64(4H,m), 7.97(1H,s).

### Example 445: Preparation of the compound of Compound No. 445.

Boron tribromide(1.0M solution in dichloromethane; 1.2 ml, 1.2 mmol) was added slowly to a mixture of 5-[4-methoxy-3-(3,4-dihydroxyphenyl)benzylidene]-3-(3,4-dichlorobenzyl)thiazolidine-2,4-dione(Compound No. 444; 100 mg, 0.200 mmol) and dichloromethane(1.5 ml) at 0°C, and the mixture was stirred at room temperature for 2 days. Water was added to the reaction mixture, and the mixture was extracted with ethyl acetate. The organic layer was washed with brine and dried over anhydrous sodium sulfate. The residue obtained by evaporation of the solvent under reduced pressure was purified by chromatography on silica gel(hexane:ethyl acetate=1:2) to give the title compound (45 mg, 46.1%) as a light yellow solid.
¹H-NMR(DMSO-d₆): δ 4.84(2H,s), 6.78(1H,d,J=8.1Hz), 6.85(1H,dd,J=1.8,8.1Hz), 7.03(1H,d,J=1.8Hz), 7.06(1H,d,J=8.7Hz), 7.27-7.49(3H,m), 7.61(1H,s), 7.62(1H,d,J=8.4Hz), 7.92(1H,s), 8.96(1H,brs), 10.40(1H,s).

### Example 446: Preparation of the compound of Compound No. 446.

The title compound was obtained in the same manner as the Example 373 using the following raw material.
Raw material: 5-[4-hydroxy-3-(3,4-dimethoxyphenylcarbamoyl)benzylidene]-3-(3,4-dichlorobenzyl)thiazolidine-2,4-dione(Compound No. 432).
Yield: 77.7%(yellow powder).
¹H-NMR(DMSO-d₆):δ 4.82(2H,s), 6.39(2H,s), 7.30(1H,dd,J=8.7,2.4Hz), 7.60-7.63(2H,m), 7.73(1H,s).

### Example 447: Preparation of the compound of Compound No. 447.

(1) 4-Methoxy-3-(2,6-dimethoxyphenyl)benzaldehyde.
   Tetrakis(triphenylphosphine)palladium(0)(231.1 mg, 0.2 mmol) was added to a solution of 3-bromo-4-methoxybenzaldehyde(0.43 g, 2 mmol) in 1,2-dimethoxyethane(5 ml), and the mixture was stirred at room temperature for 10 minutes. A solution of 2,6-dimethoxyphenylboronic acid(545.9 mg, 3 mmol) in ethanol(4 ml) was added to the mixture, and the mixture was stirred for 10 minutes. A solution of sodium carbonate(1.8 g, 17 mmol) in water(5 ml) was added to the mixture, and the mixture was stirred for 10 minutes, then refluxed for 1 hour. The reaction mixture was cooled, poured into diluted hydrochloric acid, and extracted with ethyl acetate. The organic layer was washed with brine, and dried over anhydrous sodium sulfate. The residue obtained by evaporation of the solvent under reduced pressure was purified by chromatography on silica gel(hexane:ethyl acetate=2:1) and crystallized by ethyl acetate/diisopropyl ether/hexane to give the title compound(419.2 mg, 77.0%) as a yellowish white powder.
   ¹H-NMR(CDCl₃): δ 3.92(3H,s), 3.93(3H,s), 3.94(3H,s), 6.94(1H,d,J=8.1Hz), 7.05-7.12(3H,m), 7.82-7.87(2H,m).
   ¹H-NMR(CDCl₃): *δ* 3.73(6H,s), 3.84(3H,s), 6.66(2H,d,J=8.4Hz), 7.08(1H,d,J=9.0Hz), 7.32(1H,t,J=8.1Hz), 7.72(1H,d,J=2.1Hz), 7.89(1H,dd,J=8.7,2.4Hz), 9.90(1H,s).
(2) 5-[4-Methoxy-3-(2,6-dimethoxyphenyl)benzylidene]-3-(3,4-dichlorobenzyl)thiazolidine-2,4-dione (Compound No. 447).
   The title compound was obtained in the same manner as the Example 2(2) using the following raw materials.
   Raw materials: 4-methoxy-3-(2,6-dimethoxyphenyl)benzaldehyde and 3-(3,4-dichlorobenzyl)thiazolidine-2,4-dione(compound of the Example 8(1)).
   Yield: 82.4% (yellowish white powder).
   ¹H-NMR(CDCl₆): *δ* 3.76(6H,s), 3.82(3H,s), 4.82(2H,s), 6.66(2H,d,J=8.1Hz), 7.06(1H,d,J=8.7Hz), 7.26-7.30(1H,m), 7.30(1H,t,J=8.4Hz), 7.36(1H,d,J=2.1Hz), 7.40(1H,d,J=8.1Hz), 7.50(1H,dd,J=8.7,2.1Hz), 7.54(1H,d,J=1.8Hz), 7.91(1H,s).

### Example 448: Preparation of the compound of Compound No. 448.

(1) 4-Methoxy-3-(4-methoxyphenyl)benzaldehyde.
   The title compound was obtained in the same manner as the Example 447(1) using the following raw materials. Raw materials: 3-bromo-4-methoxybenzaldehyde and 4-methoxyphenylboronic acid. Yield: 99.7%(light yellowish brown powder).
   ¹H-NMR(CDCl₆): *δ* 3.86(3H,s), 3.91(3H,s), 6.97(2H,d,J=8.7Hz), 7.08(1H,d,J=9.0Hz), 7.47(2H,d,J=9.0Hz), 7.82-7.86(2H,m), 9.93(1H,s).
(2) 5-[4-Methoxy-3-(4-methoxyphenyl)benzylidene]-3-(3,4-dichlorobenzyl)thiazolidine-2,4-dione (Compound No. 448).
   The title compound was obtained in the same manner as the Example 2(2) using the following raw materials.
   Raw materials: 4-methoxy-3-(4-methoxyphenyl)benzaldehyde and 3-(3,4-dichlorobenzyl)thiazolidine-2,4-dione(compound of the Example 8(1)).
   Yield: 89.9%(light yellow powder).
   ¹H-NMR(CDCl₃): *δ* 3.86(3H,s), 3.88(3H,s), 4.84(2H,s), 6.98(2H,d,J=8.7Hz), 7.05(1H,d,J=9.3Hz), 7.29(1H,dd,J=8.4,2.1Hz), 7.41(2H,d,J=8.4Hz), 7.44-7.48(4H,m), 7.54(1H,d,J=1.8Hz), 7.91(1H,s).

### Example 449: Preparation of the compound of Compound No. 449.

The title compound was obtained in the same manner as the Example 373 using the following raw material.
Raw material: 5-[4-methoxy-3-(2,6-dimethoxyphenyl)benzylidene]-3-(3,4-dichlorobenzyl)thiazolidine-2,4-dione(Compound No. 447).
Yield: 85.9%(light yellow powder).
¹H.NMR(CDCl₃): *δ* 4.82(2H,s), 5.31(2H,brs), 5.96(1H,brs), 6.64(2H,d,J=7.8Hz), 7.20(1H,d,J=8.7Hz), 7.22(1H,d,J=8.1Hz), 7.26-7.29(1H,m), 7.40(1H,d,J=8.1Hz), 7.44(1H,d,J=2.1Hz), 7.53(1H,d,J=2.4Hz), 7.53(1H,dd,J=8.4,2.4Hz), 7.87(1H,s).

### Example 450: Preparation of the compound of Compound No. 450.

The title compound was obtained in the same manner as the following Example 357 using the following raw materials.
Raw materials:
5-(3-carboxy-4-hydroxybenzylidene)-3-(3,4-dichlorobenzyl)thiazolidine-2,4-dione(Comp ound No. 358) and 2-methyl-5-methoxyaniline.
Yield: 62.3%(light yellow solid).
¹H-NMR(DMSO-d₆): *δ* 2.24(3H,s), 3.75(3H,s), 4.85(2H,s), 6.68(1H,dd,J=2.7,8.4Hz), 7.14-7.19(3H,m), 7.31(1H,dd,J=1.5,8.1Hz), 7.62(1H,d,J=1.8Hz), 7.63(1H,d,J=8.1Hz),7.72(1H,dd,J=2.1,8.4Hz), 7.76(1H,d,J=2.1Hz), 7.94(1H,s), 8.307(1H,d,J=2.4Hz), 10.46(1H,brs).

### Example 451: Preparation of the compound of Compound No. 451.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and 3-methoxy-5-(trifluoromethyl)aniline. Yield: 45.3%(light yellow solid).
¹H-NMR(DMSO-d₆): δ 3.85(3H,s), 4.84(2H,s), 7.01(1H,s), 7.16(1H,d,J=8.7Hz), 7.29-7.33(1H,m), 7.60-7.71(4H,m), 7.78(1H,s), 7.93(1H,s), 8.08(1H,d,J=1.8Hz), 10.71(1H,brs).

### Example 452: Preparation of the compound of Compound No. 452.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and 2-methoxy-5-methylaniline.
Yield: 87.5%(light yellow solid).
¹H-NMR(DMSO-d₆):*δ* 2.28(3H,s), 3.87(3H,s), 4.85(2H,s), 6.89-6.92(1H,m), 6.98(1H,d,J=8.4Hz), 7.18(1H,d,J=8.4Hz), 7.31(1H,dd,J=1.8,8.4Hz), 7.61-7.64(1H,m), 7.75(1H,dd,J =2.1,8.4Hz), 7.95(1H,s), 8.31-8.34(2H,m), 10.78(1H,s), 12.59(1H,brs).

### Example 453: Preparation of the compound of Compound No. 453.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and methyl 2-amino-4-chlorobenzoate.
Yield: 83.1%(white solid).
¹H-NMR(DMSO-d₆): *δ* 3.89(3H,s), 4.85(2H,s), 7.18(1H,d,J=8.7Hz), 7.29-7.35(2H,m), 7.60-7.64(2H,m), 7.75(1H,dd,J=2.4,8.7Hz), 7.95(1H,s), 8.07(1H,d,J=8.7Hz), 8.24(1H,d,J=2.7Hz), 8.87(1H,d,J=1.8Hz), 12.23(1H,s), 12.48(1H,s).

### Example 454: Preparation of the compound of Compound No. 454.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and 2-methoxy-5-chloroaniline.
Yield: 46.2%(white solid).
¹H-NMR(DMSO-d₆): δ 3.92(3H,s), 4.85(2H,s), 7.13-7.21(3H,m), 7.31(1H,dd,J=1.8,8.4Hz), 7.60-7.64(2H,m), 7.75(1H,dd,J=2.7,8.7Hz), 7.95(1H,s), 8.31(1H,d,J=2.1Hz), 8.56(1H,d,J=1.8Hz), 10.94(1H,s), 12.70(1H,brs).

### Example 455: Preparation of the compound of Compound No. 455.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and 2-methoxy-5-phenylaniline.
Yield: 65.1%(white solid).
¹H-NMR(DMSO-d₆): δ 3.96(3H,s), 4.851(2H,s), 7.21(2H,d,J=9.0Hz), 7.29-7.50(5H,m), 7.61-7.64(4H,m), 7.75(1H,dd,J=2.4,8.7Hz), 7.95(1H,s), 8.35(1H,d,J=2.7Hz), 8.83(1H,d,J=2.4Hz), 10.91(1H,s), 12.66(1H,brs).

### Example 456: Preparation of the compound of Compound No. 456.

The title compound was obtained in the same manner as the Example 373 using the following raw material.
Raw material: 5-[4-hydroxy-3-(2-methyl-5-methoxyphenylcarbamoyl)-benzylidene]-3-(3,4-dichlorobenzyl)thiazolidine-2,4-dione(Compound No. 450).
Yield: 65.5%(yellow solid).
¹H-NMR(DMSO-d₆): *δ* 2.19(3H,s), 4.85(2H,s), 6.50(1H,dd,J=2.4,8.1Hz), 7.04(1H,d,J=8.4Hz), 7.18(1H,d,J=8.4Hz), 7.31(1H,dd,J=1.8,8.4Hz), 7.59-7.64(3H,m), 7.72(1H,dd,J=2.4,8.7Hz), 7.94(1H,s), 8.30(1H,d,J=2.4Hz), 9.28(1H,s), 10.22(1H,s), 12.67(1H,brs).

### Example 457: Preparation of the compound of Compound No. 457.

The title compound was obtained in the same manner as the Example 373 using the following raw material.
Raw material: 5-[4-hydroxy-3-(2-methoxy-5-methylphenylcarbamoyl)-benzylidene]-3-(3,4-dichlorobenzyl)thiazolidine-2,4-dione(Compound No. 452).
Yield: 68.0%(light yellow solid).
¹H-NMR(DMSO-d₆): *δ* 2.24(3H,s), 4.85(2H,s), 6.71-6.82(2H,m), 7.15(1H,d,J=8.4Hz), 7.31(1H,dd,J=2.1,8.1Hz), 7.62(1H,d,J=2.1Hz), 7.63(1H,d,J=8.1Hz), 7.72(1H,dd,J=2.7,8.4Hz), 7.941(1H,s), 8.241(1H,s), 8.33(1H,d,J=2.4Hz), 9.88(1H,s), 10.74(1H,s), 12.56(1H,brs).

### Example 458: Preparation of the compound of Compound No. 458.

The title compound was obtained in the same manner as the Example 373 using the following raw material.
Raw material: 5-[4-hydroxy-3-(2-methoxycarbonyl-5-chlorophenylcarbamoyl)-benzylidene]-3-(3,4-dichlorobenzyl)thiazolidine-2,4-dione(Compound No. 453).
Yield: 28.4%(white solid).
¹H-NMR(DMSO-d₆):δ 4.85(2H,s), 6.91(1H,d,J=8.4Hz), 6.98(1H,dd,J=2.4,8.4Hz), 7.16(1H,d,J=9.0Hz), 7.31(1H,dd,J=2.1,8.1Hz), 7.62(1H,d,J=2.1Hz), 7.63(1H,d,J=8.1Hz), 7.34(1H,dd,J=7.2,9.0Hz), 7.95(1H,s), 8.32(1H,d,J=2.1Hz), 8.50(1H,d,J=2.7Hz), 10.53(1H,s), 10.90(1H,s), 12.67(1H,brs).

### Example 459: Preparation of the compound of Compound No. 459.

The title compound was obtained in the same manner as the Example 373 using the following raw material.
Raw material: 5-{4-hydroxy-3-[3-methoxy-5-(trifluoromethyl)phenylcarbamoyl]-benzylidene}-3-(3,4-dichlorobenzyl)thiazolidine-2,4-dione(Compound No. 451).
Yield: 42.2%(light yellow solid).
¹H-NMR(DMSO-d₆): δ 4.85(2H,s), 6.81(1H,s), 7.16(1H,d,J=8.7Hz), 7.31(1H,dd,J=8.4,2.1Hz), 7.50(1H,s), 7.58-7.65(3H,m), 7.69(1H,dd,J=2.1,8.4Hz), 7.93(1H,s), 8.08(1H,d,J=2.1Hz), 10.22(1H,s), 10.50(1H,s), 11.91(1H,brs).

### Example 460: Preparation of the compound of Compound No. 460.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and 4-amino-2,2-difluorobenzo-1,3-dioxole.
Yield: 26.8%(white solid).
¹H-NMR(DMSO-d₆): δ 4.84(2H,s), 7.17(1H,d,J=8.7Hz), 7.24(2H,d,J=4.2Hz), 7.31(1H,dd,J=1.8,8.4Hz), 7.62(1H,d,J=1.5Hz), 7.62(1H,d,J=7.8Hz), 7.72-7.80(2H,m), 7.94(1H,s), 8.23(1H,d,J=2.4Hz), 10.65(1H,s), 12.51(1H,brs).

### Example 461: Preparation of the compound of Compound No. 461.

Boron tribromide(1.0M solution in dichloromethane; 1.5 ml, 1.5 mmol) was added to a mixture of 5-[4-methoxy-3-(4-methoxyphenyl)benzylidene]-3-(3,4-dichlorobenzyl)thiazolidine-2,4-dione(Compound No. 448; 345.1 mg, 0.69 mmol) and dichloromethane(5 ml) under ice cooling, and the mixture was stirred at room temperature for overnight. The reaction mixture was poured into ice and water, and extracted with ethyl acetate. The organic layer was washed with brine and dried over anhydrous sodium sulfate. The residue obtained by evaporation of the solvent under reduced pressure was purified by chromatography on silica gel(hexane:ethyl acetate=4:3→1:1) and crystallized by diisopropyl ether/hexane to give the title compound (98.2 mg, 29.3%) as a yellowish white powder.
¹H-NMR(CDCl₃): δ 3.88(3H,s), 4.84(2H,s), 5.14(1H,s), 6.91(1H,d,J=8.7Hz), 7.05(1H,d,J=8.4Hz), 7.29(1H,dd,J=8.4,1.8Hz), 7.41(2H,d,J=8.7Hz), 7.43-7.48(3H,m), 7.54(1H,d,J=1.8Hz), 7.91(1H,s).

The compound of the following Compound No. 462 was also obtained as a by-product.

### Example 462: Preparation of the compound of Compound No. 462.

This compound was obtained in the aforementioned Example 461 as a by-product.
Yield: 39.6%(128.9 mg, light yellow powder).
¹H-NMR(CDCl₃): δ 4.83(2H,s), 5.29(1H,s), 5.68(1H,s), 6.99(2H,d,J=8.7Hz), 7.07(1H,d,J=8.4Hz), 7.26-7.42(6H,m), 7.54(1H,d,J=2.1Hz), 7.89(1H,s).

### Example 463: Preparation of the compound of Compound No. 463.

(1) 3,4-Dihydroxy-5-carboxybenzaldehyde.
   The title compound was obtained in the same manner as the Example 341(1) using the following raw materials.
   Raw materials: 2,3-dihydroxybenzoic acid and hexamethylenetetramine.
   Yield: 28.6%(yellow crystal).
   ¹H-NMR(DMSO-d₆): *δ* 7.42(1H,d,J=2.1Hz), 7.88(1H,d,J=1.8Hz), 7.79(1H,s), 9.98(1H,brs).
(2) 3,4-Dihydroxy-5-(benzyloxycarbonyl)benzaldehyde.
   The title compound was obtained in the same manner as the Example 362(1) using the following raw materials.
   Raw materials: 3,4-dihydroxy-5-carboxybenzaldehyde and benzylalcohol.
   Yield: 81.8%(yellowish brown oil).
   ¹H-NMR(DMSO-ds): *δ* 5.42(2H,s), 7.31-7.32(1H,m), 7.40-7.44(3H,m), 7.49-7.52(2H,m), 7.86-7.87(1H,m), 9.80(1H,s), 10.22(1H,brs), 10.96(1H,brs).
(3) 5-[3,4-Dihydroxy-5-(benzyloxycarbonyl)benzylidene]-3-(3,4-dichlorobenzyl)thiazolidine-2,4-dione (Compound No. 463).
   The title compound was obtained in the same manner as the Example 2(2) using the following raw materials.
   Raw materials: 3,4-dihydroxy-5-(benzyloxycarbonyl)benzaldehyde and 3-(3,4-dichlorobenzyl)thiazolidine-2,4-dione(compound of the Example 8(1)).
   Yield: 81.5%(yellow crystal).
   ¹H-NMR(DMSO-d₆): δ 4.83(2H,s), 5.42(2H,s), 7.28-7.31(2H,m), 7.37-7.45(3H,m), 7.51-7.53(2H,m), 7.60-7.63(3H,m), 7.88(1H,s), 10.37(2H,brs).

### Example 464: Preparation of the compound of Compound No. 464.

The title compound was obtained in the same manner as the Example 372(1) using the following raw material.
Raw material: 5-[3,4-dihydroxy-5-(benzyloxycarbonyl)benzylidene]-3-(3,4-dichlorobenzyl)thiazolidine-2,4-dione (Compound No. 463).
Yield: 88.8%(yellow crystal).
¹H-NMR(DMSO-d₆):δ 4.83(2H,s), 7.26(1H,d,J=2.1Hz), 7.30(1H,dd,J=8.1,1.5Hz), 7.59-7.63(3H,m), 7.85(1H,s), 9.89(1H,brs).

### Example 465: Preparation of the compound of Compound No. 465.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and 2,5-difluoroaniline.
Yield: 73.7%(whitish orange crystal).
¹H-NMR(DMSO-d₆): δ 4.85(2H,s), 6.99-7.07(1H,m), 7.19(1H,d,J=8.7Hz), 7.30-7.33(1H,m), 7.37-7.45(1H,m), 7.62-7.64(2H,m), 7.77(1H,dd,J=8.7,2.1Hz), 7.96(1H,s), 8.22-8.29(1H,m), 8.29(1H,d,J=2.1Hz), 10.84(1H,s), 12.72(1H,brs).

### Example 466: Preparation of the compound of Compound No. 466.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and 2-nitro-5-(trifluoromethyl)aniline.
Yield: 11.1%(yellow crystal).
¹H-NMR(DMSO-d₆): δ 4.84(2H,s), 7.17(1H,d,J=8.1Hz), 7.31(1H,d,J=8.1Hz), 7.62-7.77(4H,m), 7.94(1H,s), 8.28(1H,s), 8.36(1H,d,J=8.4Hz), 9.08(1H,s), 12.23(1H,brs).

### Example 467: Preparation of the compound of Compound No. 467.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and 2-methoxy-5-(tert-butyl)aniline. Yield: 10.9%(yellowish white crystal).
¹H-NMR(DMSO-d₆): *δ* 1.29(9H,s), 3.87(3H,s), 4.85(2H,s), 7.01(1H,d,J=8.4Hz), 7.10(1H,dd,J=8.4,2.4Hz), 7.17(1H,d,J=8.4Hz), 7.31(1H,dd,J=8.4,1.8Hz), 7.63(1H,d,J=1.5Hz), 7.63(1H,d,J=8.4Hz), 7.72(1H,dd,J=8.7,2.1Hz), 7.94(1H,s), 8.34(1H,d,J=1.8Hz), 8.57(1H,d,J=2.1Hz), 10.88(1H,s), 12.60(1H,brs).

### Example 468: Preparation of the compound of Compound No. 468.

The title compound was obtained in the same manner as the Example 357 using the following raw materials. Raw materials: 5-(3-carboxy-4-hydroxybenzylidene)-3-(3,4-dichlorobenzyl)-thiazolidine-2,4-dione(Compound No. 358) and 2-methoxy-5-(trifluoromethyl)aniline. Yield: 24.9%(yellowish white crystal).
¹H-NMR(DMSO-d₆): *δ* 4.00(3H,s), 4.85(2H,s), 7.20(1H,d,J=8.4Hz), 7.30-7.32(2H,m), 7.47-7.51(1H,m), 7.63(1H,d,J=8.4Hz), 7.63(1H,d,J=2.1Hz), 7.76(1H,dd,J=8.7,2.1Hz), 7.95(1H,s), 8.33(1H,d,J=2.1Hz), 8.88(1H,d,J=1.BHz), 11.05(1H,s), 12.74(1H,brs).

### Example 469: Preparation of the compound of Compound No. 469.

The title compound was obtained in the same manner as the Example 357 using the following raw materials.
Raw materials: 5-(3,4-dihydroxy-5-carboxybenzylidene)-3-(3,4-dichlorobenzyl)-thiazolidine-2,4-dione(Compound No. 464) and 2-chloro-5-(trifluoromethyl)aniline.
Yield: 28.5%(yellowish orange crystal).
¹H-NMR(DMSO-ds):*δ* 4.84(2H,s), 7.27(1H,d,J=2.1Hz), 7.31(1H,dd,J=8.4,1.5Hz), 7.52-7.56(1H,m), 7.63(1H,d,J=1.8Hz), 7.63(1H,d,J=8.1Hz), 7.81-7.86(3H,m), 8.90(1H,m), 10.43(1H,brs), 11.48(1H,brs).

### Example 470: Preparation of the compound of Compound No. 470.

The title compound was obtained in the same manner as the Example 357 using the following raw materials.
Raw materials: 5-(3,4-dihydroxy-5-carboxybenzylidene)-3-(3,4-dichlorobenzyl)-thiazolidine-2,4-dione(Compound No. 464) and methyl 3-aminobenzoate.
Yield: 30.7%(yellow crystal).
¹H-NMR(DMSO-d₆): *δ* 3.88(3H,s), 4.84(2H,s), 7.21(1H,d,J=1.8Hz), 7.31(1H,dd,J=8.4,1.8Hz), 7.54(1H,t,J=7.8Hz), 7.61-7.64(2H,m), 7.72-7.75(2H,m), 7.83(1H,s), 7.96-7.99(1H,m), 8.39(1H,m), 10.16(1H,brs), 10.69(1H,brs), 11.67(1H,brs).

### Example 471: Preparation of the compound of Compound No. 471.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and 2,5-dimethoxyaniline.
Yield: 3.0%(light yellow solid).
¹H-NMR(DMSO-ds): *δ* 3.74(3H,s), 3.85(3H,s), 4.85(2H,s), 6.65(1H,dd,J=3.0,9.0Hz), 7.02(1H,d,J=9.0Hz), 7.16(1H,d,J=8.4Hz), 7.31(1H,dd,J=1.8,8.1Hz), 7.63(1H,d,J=1.8Hz), 7.63(1H,d,J=8.1Hz), 7.72(1H,dd,J=2.4,8.4Hz), 7.94(1H,s), 8.19(1H,d,J=3.0Hz), 8.32(1H,d,J=2.4Hz), 11.01(1H,s), 12.69(1H,brs).

### Example 472: Preparation of the compound of Compound No. 472.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and 2-bromo-4-isopropylaniline.
Yield: 8.2%(white solid).
¹H-NMR(DMSO-d₆):δ 1.21(6H,d,J=6.9Hz), 2.75-2.97(1H,m), 4.85(2H,s), 7.19(1H,d,J=8.7Hz), 7.28-7.36(2H,m), 7.55-7.66(3H,m), 7.75(1H,dd,J=2.1,8.4Hz), 7.95(1H,s), 8.25(1H,d,J=8.4Hz), 8.32(1H,d,J=1.8Hz), 10.69(1H,s), 12.74(1H,brs).

### Example 473: Preparation of the compound of Compound No. 473.

The title compound was obtained in the same manner as the Example 357 using the following raw materials.
Raw materials: 5-(3-carboxy-4-hydroxybenzylidene)-3-(3,4-dichlorobenzyl)-thiazolidine-2,4-dione(Compound No. 358) and 2-chloro-5-nitroaniline.
Yield: 9.5%(yellow crystal).
¹H-NMR(DMSO-d₆): *δ* 4.85(2H,s), 7.17(1H,d,J=8.4Hz), 7.30-7.33(1H,m), 7.63(1H,d,J=1.5Hz), 7.63(1H,d,J=8.4Hz), 7. 76(1H,dd,J=8. 7,2.4Hz), 7.88(1H,d,J=8.7Hz), 7.95(1H,s), 8.01(1H,dd,J=8.7,2.4Hz), 8.32(1H,d,J=2.1Hz), 9.47(1H,d,J=3.0Hz), 11.53(1H,brs), 12.84(1H,br).

### Example 474: Preparation of the compound of Compound No. 474.

The title compound was obtained in the same manner as the Example 357 using the following raw materials.
Raw materials: 5-(3,4-dihydroxy-5-carboxybenzylidene)-3-(3,4-dichlorobenzyl)-thiazolidine-2,4-dione(Compound No. 464) and methyl 3,4-nethylenedioxyaniline. Yield: 43.3%(yellowish brown crystal).
¹H-NMR(DMSO-d₆): δ 4.84(2H,s), 6.03(2H,s), 6.92(1H,d,J=8.4Hz), 7.08(1H,dd,J=8.1,1.5Hz), 7.20(1H,d,J=1.5Hz), 7.30(1H,dd,J=8.1,1.8Hz), 7.37(1H,d,J=1.5Hz), 7.62(1H,d,J=3.0Hz), 7.62(1H,d,J=8.1Hz), 7.72(1H,m), 7.81(1H,s), 10.11(1H,brs), 10.35(1H,brs), 11.95(1H,brs).

### Example 475: Preparation of the compound of Compound No. 475.

The title compound was obtained in the same manner as the Example 357 using the following raw materials.
Raw materials: 5-(3,4-dihydroxy-5-carboxybenzylidene)-3-(3,4-dichlorobenzyl)-thiazolidine-2,4-dione(Compound No. 464) and methyl 3-(trifluoromethoxy)aniline.
Yield: 29.4%(yellow crystal).
¹H-NMR(DMSO-d₆):δ 4.84(2H,s), 7.11-7.14(1H,m), 7.21(1H,d,J=1.5Hz), 7.31(1H,dd,J=8.4,1.8Hz), 7.50(1H,t,J=8.4Hz), 7.61-7.64(4H,m), 7.83(1H,s), 7.89(1H,s), 10.19(1H,brs), 10.68(1H,brs), 11.36(1H,br).

### Example 501: Preparation of the compound of Compound No. 501.

A mixture of 1-(3,5-dichlorophenyl)-3-methyl-2-pyrazolin-5-one(compound of the Example 112(1); 120 mg, 0.494 mmol), 2,4,5-trihydroxybenzaldehyde(79 mg, 0.494 mmol), sodium acetate(122 mg, 1.482 mmol) and acetic acid(1.5 ml) was stirred at 80°C for 1 hour. The reaction mixture was cooled, and the separated solid was collected by filtration and purified by chromatography on silica gel(hexane:ethyl acetate=1:1) to give the title compound(40 mg, 21.3%) as an orange solid.
¹H-NMR(DMSO-d₆): *δ* 2.27(3H,s), 6.45(1H,s), 7.38(1H,t,J=1.8Hz), 7.98(1H,s), 8.05(2H,d,J=1.8Hz), 8.89(1H,s), 9.02(1H,brs), 10.58(1H,s), 10.74(1H,brs).

### Example 502: Preparation of the compound of Compound No. 502.

(1) 1-(2,5-Dichlorophenyl)-3-isopropyl-2-pyrazolin-5-one.
   A mixture of 2,5-dichlorophenylhydrazine(500 mg, 2.824 mmol), ethyl isobutyrylacetate(517 mg, 3.107 mmol) and ethanol(3 ml) was refluxed for 2 hours. The reaction mixture was cooled, and the residue obtained by evaporation of the solvent under reduced pressure was dried under reduced pressure. Sodium ethoxide(211 mg, 3.107 mmol) and ethanol(5ml) were added to the residue, and the mixture was refluxed for 3 hours. The reaction mixture was cooled, and the residue obtained by evaporation of the solvent under reduced pressure was dissolved in ethyl acetate. The ethyl acetate solution was washed with brine and dried over anhydrous sodium sulfate. The residue obtained by evaporation of the solvent under reduced pressure was purified by chromatography on silica gel(hexane:ethyl acetate=2:1) to give the title compound(164 mg, 21.4%) as a white solid.
   ¹H-NMR(DMSO-d₆): *δ* 1.17(6H,d,J=6.9Hz), 2.70-2.83(1H,m), 5.35(1H,s), 7.52-7.67(3H,m), 11.24(1H,s).
(2) 4-(2,4,5-Trihydroxybenzylidene)-1-(2,5-dichlorophenyl)-3-isopropyl-2-pyrazolin-5-one (Compound No. 502).
   The title compound was obtained in the same manner as the Example 501 using the following raw materials.
   Raw materials: 2,4,5-trihydroxybenzaldehyde and 1-(2, 5-dichlorophenyl)-3-isopropyl-2-pyrazolin-5-one.
   Yield: 4.1%(red solid).
   ¹H-NMR(CD₃OD): *δ* 1.34(6H,d,J=6.1Hz), 3.11-3.22(1H,m), 6.39(1H,s), 7.43-7.59(3H,m), 8.29(1H,s), 8.84(1H,brs).

### Example 503: Preparation of the compound of Compound No. 503.

The title compound was obtained in the same manner as the Example 501 using the following raw materials.
Raw materials: 2,4,5-trihydroxybenzaldehyde and 1-(3,4-dichlorophenyl)-3-methyl-2-pyrazolin-5-one(compound of the Example 144(1)).
Yield: 37.4%(orange solid).
¹H-NMR(DMSO-d₆): *δ* 2.27(3H,s), 6.45(1H,s), 7.68(1H,d,J=8.7Hz), 7.94(1H,dd,J=8.7,2.7Hz), 7.96(1H,s), 8.30(1H,d,J=2.7Hz), 8.91(1H,s), 9.00(1H,brs), 10.55(1H,s), 10.71(1H,brs).

### Example 504: Preparation of the compound of Compound No. 504.

The title compound was obtained in the same manner as the Example 501 using the following raw materials.
Raw materials: 2,4,5-trihydroxybenzaldehyde and 1-(3,5-dichlorophenyl)-3-isopropyl-2-pyrazolin-5-one(compound of the Example 104(1)).
Yield: 39.9%(reddish brown solid).
¹H-NMR(DMSO-d₆):δ 1.30(6H,d,J=6.9Hz), 3.06-3.18(1H,m), 6.4S(1H,s), 7.39(1H,t,J=1.8Hz), 8.06(2H,d,J=1.8Hz), 8.09(1H,s), 8.89(1H,s), 9.02(1H,brs), 1O.SS(1H,s), 10.74(1H,brs).

### Example 505: Preparation of the compound of Compound No. 505.

The title compound was obtained in the same manner as the Example 501 using the following raw materials.
Raw materials: 2,4-dihydroxybenzaldehyde and 1-(3,5-dichlorophenyl)-3-isopropyl-2-pyrazolin-5-one(compound of the Example 104(1)).
Yield: 31.1%(red solid).
¹H-NMR(DMSO-d₆): *δ* 1.27(6H,d,J=6.9Hz), 2.75-2.89(1H,m), 2.75-2.89(2H,m), 7.46(1H,t,J=8.1Hz), 1.27(2H,d,J=6.9Hz), 8.51(1H,s), 1.27(1H,d,J=6.9Hz), 8.51(1H,s), 8.51(1H,s).

### Example 506: Preparation of the compound of Compound No. 506.

The title compound was obtained in the same manner as the Example 501 using the following raw materials.
Raw materials: 2,4,5-trimethoxybenzaldehyde and 1-(3,5-dichlorophenyl)-3-isopropyl-2-pyrazolin-5-one(compound of the Example 104(1)).
Yield: 80.8%(orange solid).
¹H-NMR(CDCl₃): *δ* 1.38(6H,d,J=6.9Hz), 3.05-3.20(1H,m), 3.96(3H,s), 4.00(3H,s), 4.03(3H,s), 6.47(1H,s), 7.12(1H,t,J=2.1Hz), 8.05(2H,d,J=2.1Hz), 8.08(1H,s), 9.32(1H,s).

### Example 507: Preparation of the compound of Compound No. 507.

(1) 1-(4-Carboxyphenyl)-3-isopropyl-2-pyrazolin-5-one.
   The title compound was obtained in the same manner as the following Example 509(1) using the following raw materials.
   Raw materials: 4-hydrazinobenzoic acid and ethyl isobutyrylacetate.
   Yield: 58.0%(white solid).
   ¹H-NMR(DMSO-ds): *δ* 1.19(6H,d,J=6.9Hz), 2.73-2.85(1H,m), 5.43(1H,s), 7.87-8.01(4H,m), 11.81(1H,brs), 12.86(1H,s).
(2) 4-(2,4,5-Trimethoxybenzylidene)-1-(4-carboxyphenyl)-3-isopropyl-2-pyrazolin-5-one(Compound No. 507).
   The title compound was obtained in the same manner as the Example 501 using the following raw materials.
   Raw materials: 2,4,5-trimethoxybenzaldehyde and 1-(4-carboxyphenyl)-3-isopropyl-2-pyrazolin-5-one.
   Yield: 42.9%(orange solid).
   ¹H-NMR(DMSO-d₆): *δ* 1.32(6H,d,J=6.9Hz), 3.01-3.20(1H,m), 3.83(3H,s), 3.97(3H,s), 3.98(3H,s), 6.80(1H,s), 7.97-8.12(5H,m), 9.21(1H,s), 12.65(1H,brs).

### Example 508: Preparation of the compound of Compound No. 508.

The title compound was obtained in the same manner as the Example 501 using the following raw materials.
Raw materials: 4-formylimidazole and 1-(3,5-dichlorophenyl)-3-isopropyl-2-pyrazolin-5-one(compound of the Example 104(1)).
Yield: 89.9%(light orange solid).
¹H-NMR(CDCl₃): *δ* 1.39(6H,d,J=6.9Hz), 3.01-3.15(1H,m), 7.20(1H,t,J=1.5Hz), 7.45(1H,s), 7.88(1H,s), 8.02(1H,s), 8.03(2H,d,J=1.5Hz).

### Example 509: Preparation of the compound of Compound No. 509.

(1) 1-(3,5-Dichlorophenyl)-3-(4-hydroxyphenyl)-2-pyrazolin-5-one.
   A mixture of 3,5-dichlorophenylhydrazine(551 mg, 2.582 mmol), methyl 4-hydroxybenzoylacetate(700 mg, 2.582 mmol) and ethanol(5 ml) was refluxed for 1 hour. The reaction mixture was cooled, and the solid obtained by evaporation of the solvent under reduced pressure was washed with ethyl acetate under suspension to give the title compound(450 mg, 54.3%) as a white solid.
   ¹H-NMR(DMSO-d₆): *δ* 5.93(1H,s), 6.79-6.85(2H,m), 7.47(1H,t,J=1.8Hz), 7.64-7.69(2H,m), 7.92(2H,d,J=1.8Hz).
(2) 4-(3-Hydroxybenzylidene)-1-(3,5-dichlorophenyl)-3-(4-hydroxyphenyl)-2-pyrazolin-5-one (Compound No. 509).
   The title compound was obtained in the same manner as the Example 501 using the following raw materials.
   Raw materials: 3-hydroxybenzaldehyde and 1-(3,5-dichlorophenyl)-3-(4-hydroxyphenyl)-2-pyrazolin-5-one.
   Yield: 29.7%(red solid).
   ¹H-NMR(DMSO-ds): *δ* 6.97(2H,d,J=4.2Hz), 7.04-7.09(1H,m), 7.35(1H,t,J=8.1Hz), 7.46(1H,t,J=1.8Hz), 7.59(2H,d,J=4.2Hz), 7.72(1H,s), 7.76-7.78(1H,m), 8.04(2H,d,J=1.8Hz), 8.05-8.08(1H,m), 9.80-10.15(2H,m).

### Example 510: Preparation of the compound of Compound No. 510.

The title compound was obtained in the same manner as the Example 501 using the following raw materials.
Raw materials: 5-bromosalicylaldehyde and 1-(3,5-dichlorophenyl)-3-(4-hydroxyphenyl)-2-pyrazolin-5-one(compound of the Example 509(1)).
Yield: 10.9%(orange solid).
¹H-NMR(DMSO-ds): *δ* 6.95(1H,d,J=8.7Hz), 6.96(2H,d,J=8.7Hz), 7.48(1H,t,J=2.1Hz), 7.57(2H,d,J=8.7Hz), 7.63(1H,dd,J=3.0,8.7Hz), 8.04(2H,d,J=2.1Hz), 8.08(1H,s), 9.16(1H,d,J=3.0Hz), 10.05(1H,s), 10.17(1H,s).

### Example 511: Preparation of the compound of Compound No. 511.

The title compound was obtained in the same manner as the Example 501 using the following raw materials.
Raw materials: 4-hydroxybenzaldehyde and 1-(3,5-dichlorophenyl)-3-(4-hydroxyphenyl)-2-pyrazolin-5-one(compound of the Example 509(1)).
Yield: 40.5%(orange solid).
¹H-NMR(DMSO-d₆): *δ* 6.94(2H,d,J=8.7Hz), 6.95(2H,d,J=8.7Hz), 7.42(1H,t,J=2.1Hz), 7.54(2H,d,J=8.7Hz), 7.68(1H,s), 8.07(2H,d,J=2.1Hz), 8.55(2H,d,J=8.7Hz), 9.98(1H,s), 10.99(1H,s).

### Example 512: Preparation of the compound of Compound No. 512.

(1) 1-Phenyl-3-[2-(ethoxycarbonyl)ethyl]-2-pyrazolin-5-one.
   The title compound was obtained in the same manner as the Example 509(1) using the following raw materials.
   Raw materials: phenylhydrazine and diethyl *β*-ketoadipate.
   Yield: 92.1%(white solid).
   ¹H-NMR(CDCl₃):*δ* 1.28(3H,t,J=7.3Hz), 2.72-2.84(4H,m), 3.46(2H,s), 4.18(2H,q,J=7.3Hz), 7.15-7.20(1H,m), 7.35-7.41(2H,m), 7.85(2H,d,J=7.6Hz).
(2) 4-(3,4-Methylenedioxybenzylidene)-1-phenyl-3-[2-(ethoxycarbonyl)ethyl]-2-pyrazolin-5-one (Compound No. 512).
   The title compound was obtained in the same manner as the Example 501 using the following raw materials.
   Raw materials: 3,4-methylenedioxybenzaldehyde and 1-phenyl-3-[2-(ethoxycarbonyl)ethyl]-2-pyrazolin-5-one.
   Yield: 37.9%(orange solid).
   ¹H-NMR(CDCl₃): *δ* 1.29(3H,t,J=7.2Hz), 2.85-3.01(4H,m), 4.19(2H,q,J=7.2Hz), 6.09(2H,s), 6.93(1H,d,J=8.1Hz), 7.15-7.21(1H,m), 7.35(1H,s), 7.38-7.43(2H,m), 7.77(1H,dd,J=1.5,8.1Hz), 7.92-7.99(2H,m), 8.66(1H,d,J=1.5Hz).

### Example 513: Preparation of the compound of Compound No. 513.

The title compound was obtained in the same manner as the Example 501 using the following raw materials.
Raw materials: 5-bromosalicylaldehyde and 1-(4-nitrophenyl)-3-ethoxy-2-pyrazolin-5-one.
Yield: 13.7%(orange solid).
¹H-NMR(DMSO-d₆):δ 1.47(3H,t,J=7.2Hz), 4.45(2H,q,J=7.2Hz), 6.92-6.99(1H,m), 7.56-7.65(1H,m), 7.99-8.19(3H,m), 8.27-8.33(2H,m), 8.44(1H,d,J=2.7Hz), 11.25-11.35(1H,m).

### Example 514: Preparation of the compound of Compound No. 514.

The title compound was obtained in the same manner as the Example 501 using the following raw materials.
Raw materials: 2,4,5-trihydroxybenzaldehyde and
1-phenyl-3-[2-(ethoxycarbonyl)ethyl]-2-pyrazolin-5-one(compound of the Example 512(1)).
Yield: 4.7%(orange solid).
¹H-NMR(DMSO-d₆): *δ* 1.19(3H,t,J=7.2Hz), 2.75-2.95(4H,m), 4.09(2H,q,J=7.2Hz), 6.45(1H,s), 7.13-7.19(1H,m), 7.39-7.45(1H,m), 7.91-7.98(3H,m), 8.91(1H,brs), 8.94(1H,s), 10.46(1H,s), 10.62(1H,brs).

### Example 515: Preparation of the compound of Compound No. 515.

(1) 1-(3-Chloro-4-fluorophenyl)-3-isopropyl-2-pyrazolin-5-one.
   The title compound was obtained in the same manner as the Example 509(1) using the following raw materials.
   Raw materials: 3-chloro-4-fluorophenylhydrazine and ethyl isobutyrylacetate.
   Yield: 53.6%(brown solid).
   ¹H-NMR(DMSO-d₆): *δ* 1.25(6H,d,J=6.9Hz), 2.75-2.86(1H,m), 3.43(2H,s), 7.15(1H,t,J=9.0Hz), 7.77(1H,ddd,J=2.4,9.0,4.5Hz), 8.00(1H,dd,J=2.4,6.6Hz).
(2) 4-(2,4,5-Trihydroxybenzylidne)-1-(3-chloro-4-fluorophenyl)-3-isopropyl-2-pyrazolin-5-one (Compound No. 515).
   The title compound was obtained in the same manner as the Example 501 using the following raw materials.
   Raw materials: 2,4,5-trihydroxybenzaldehyde and 1-(3-chloro-4-fluorophenyl)-3-isopropyl-2-pyrazolin-5-one.
   Yield: 7.0%(orange solid).
   ¹H-NMR(DMSO-d₆): *δ* 1.29(6H,d,J=6.9Hz), 3.02-3.16(1H,m), 6.43(1H,s), 7.49(1H,t,J=9.3Hz), 7.93(1H,ddd,J=9.3,3.0,6.9Hz), 8.23(1H,dd,J=3.0,6.9Hz), 8.06(1H,s), 8.99(1H,brs).

### Example 516: Preparation of the compound of Compound No. 516.

(1) 1-(4-Chlorophenyl)-3-[3-(ethoxycarbonyl)propyl]-2-pyrazolin-5-one.
   The title compound was obtained in the same manner as the Example 509(1) using the following raw materials.
   Raw materials: 4-chlorophenylhydrazine hydrochloride and diethyl 3-oxopimelate.
   Yield: 37.9%(orange solid).
   ¹H-NMR(CDCl₃): 1.29(3H,t,J=7.2Hz), 2.85-3.01(4H,m), 4.19(2H,q,J=7.2Hz), 6.09(2H,s), 6.93(1H,d,J=8.1Hz), 7.15-7.21(1H,m), 7.35(1H,s), 7.38-7.43(2H,m), 7.77(1H,dd,J=1.5Hz,J=8.1Hz), 7.92-7.99(2H,m), 8.66(1H,d,J=1.5Hz).
(2) 4-(2-Hydroxy-5-bromobenzylidene)-1-(4-chlorophenyl)-3-[3-(ethoxycarbonyl)propyl]-2-pyrazolin-5-one (Compound No. 516).
   The title compound was obtained in the same manner as the Example 501 using the following raw materials.
   Raw materials: 5-bromosalicylaldehyde and 1-(4-chlorophenyl)-3-[3-(ethoxycarbonyl)propyl]-2-pyrazolin-5-one.
   Yield: 53.8%(orange solid).
   ¹H-NMR(DMSO-d₆):*δ* 1.16(3H,t,J=7.2Hz), 1.58-2.02(2H,m), 2.48(2H,d,J=7.2Hz), 2.73(2H,d,J=7.5Hz), 4.04(2H,q,J=7.2Hz), 6.99(1H,d,J=9.0Hz), 7.40(2H,d,J=9.3Hz), 7.61(1H,dd,J=9.0,2.7Hz), 7.95(2H,d,J=9.3Hz), 7.96(1H,s), 9.27(1H,d,J=2.7Hz), 11.23(1H,s), 11.16-11.23(1H,m).

### Example 517: Preparation of the compound of Compound No. 517.

A mixture of 1-(4-nitrophenyl)-3-ethoxy-2-pyrazolin-5-one(56 mg, 0.223 mmol), 5-styrylsalicylaldehyde(compound of the Example 322(1); 50 mg, 0.223 mmol), ammonium acetate(52 mg, 0.669 mmol) and ethanol(1 ml) was stirred at room temperature for 40 minutes. The separated solid was collected by filtration, and washed successively with water and ethanol under suspension to give the title compound(60 mg, 59.1%) as a red solid.
¹H-NMR(DMSO-d₆): *δ* 1.43-1.49(3H,m), 4.48-4.53(2H,m), 7.00-7.84(9H,m), 8.13-9.29(6H,m), 11.16-11.23(1H,m).

### Example 518: Preparation of the compound of Compound No. 518.

The title compound was obtained in the same manner as the Example 501 using the following raw materials.
Raw materials: 5-methoxysalicylaldehyde and
1-(3,4-dichlorophenyl)-3-isopropyl-2-pyrazolin-5-one(compound of the Example 134(1)).
Yield: 51.5%(orange solid).
¹H-NMR(CDCl₃): *δ* 1.41(6H,d,J=6.9Hz), 3.10-3.21(1H,m), 3.84(3H,s), 7.08-7.18(3H,m), 7.48(1H,d,J=8.7Hz), 7.67(1H,s), 7.98(1H,dd,J=2.4,8.7Hz), 8.22(1H,d,J=2.4Hz), 9.08(1H,s).

### Example 519: Preparation of the compound of Compound No. 519.

Boron tribromide(1.0M solution in dichloromethane; 0.7 ml, 0.7 mmol) was added slowly to a solution of 4-(2-hydroxy-5-methoxybenzylidene)-1-(3,4-dichlorophenyl)-3-isopropyl-2-pyrazolin-5-one(Compound No. 518; 30 mg, 0.074 mmol) in dichloromethane(1.5 mL) at 0°C, and the mixture was stirred at room temperature for 1 hour. Water was added to the reaction mixture, and the separated solid was collected by filtration and purified by chromatography on silica gel(dichloromethane:methanol=20:1) to give the title compound(22 mg, 76.0%) as a red solid.
¹H-NMR(DMSO-d₆): *δ* 1.31(6H,d,J=6.9Hz), 3.09-3.21(1H,m), 6.84(1H,d,J=8.7Hz), 6.96(1H,dd,J=8.7,3.0Hz), 7.72(1H,d,J=8.7Hz), 7 .93(1H,dd,J=8. 7,2.4Hz), 8.12(1H,s), 8.25(1H,d,J=2.4Hz), 8.51(1H,d,J=3.0Hz).

### Example 520: Preparation of the compound of Compound No. 520.

(1) 1-(4-Chlorophenyl)-3-(4-hydroxyphenyl)-2-pyrazolin-5-one.
   The title compound was obtained in the same manner as the Example 509(1) using the following raw materials.
   Raw materials: 4-chlorophenylhydrazine hydrochloride and methyl
   4-hydroxybenzoylacetate.
   Yield: 96.7%(white solid).
   ¹H-NMR(DMSO-d₆): *δ* 4.01(1H,s), 5.91(1H,s), 6.79-6.84(2H,m), 7.49-7.87(6H,m), 10.34(1H,brs).
(2) 4-(2-Hydroxy-5-bromobenzylidene)-1-(4-chlorophenyl)-3-(4-hydroxyphenyl)-2-pyrazolin-5-one (Compound No. 520).
   The title compound was obtained in the same manner as the Example 501 using the following raw materials.
   Raw materials: 5-bromosalicylbenzaldehyde and
   1-(4-chlorophenyl)-3-(4-hydroxyphenyl)-2-pyrazolin-5-one.
   Yield: 18.3%(red solid).
   ¹H-NMR(DMSO-d₆): *δ* 6.95(1H,d,J=8.7Hz), 7.57-7.78(8H,m), 8.02(1H,dd,J=8.7,2.7Hz), 8.09(1H,s), 8.24(2H,d,J=2.7Hz), 9.25(2H,d,J=2.4Hz), 11.18(1H,brs).

### Example 521: Preparation of the compound of Compound No. 521.

(1) 1-(3,5-Dichlorophenyl)-3-methoxymethyl-2-pyrazolin-5-one.
   The title compound was obtained in the same manner as the Example 509(1) using the following raw materials.
   Raw materials: 3,5-dichlorophenylhydrazine hydrochloride and methyl 4-methoxyacetoacetate.
   Yield: 60.7%(light yellow solid).
   ¹H-NMR(DMSO-d₆): *δ* 3.28(3H,s), 4.27(2H,s), 5.58(1H,s), 7.49(1H,t,J=1.8Hz), 7.82(2H,d,J=1.8Hz)
(2) 4-(2-Hydroxy-5-bromobenzylidene)-1-(3,5-dichlorophenyl)-3-methoxymethyl-2-pyrazolin-5-one (Compound No. 521).
   The title compound was obtained in the same manner as the Example 501 using the following raw materials.
   Raw materials: 5-bromosalicylbenzaldehyde and 1-(3,5-dichlorophenyl)-3-methoxymethyl-2-pyrazolin-5-one.
   Yield: 56.9%(yellowish orange solid).
   ¹H-NMR(DMSO-d₆): *δ* 3.36(3H,s), 4.54(2H,s), 6.98(1H,d,J=8.7Hz), 7.46(1H,d,J=1.8Hz), 7.64(1H,dd,J=2.4,8.7Hz), 7.95(2H,d,J=1.8Hz), 8.25(1H,s), 9.26(2H,d,J=2.4Hz), 11.29(1H,s).

### Example 522: Preparation of the compound of Compound No. 522.

The title compound was obtained in the same manner as the Example 501 using the following raw materials.
Raw materials: 2,4,5-trihydroxybenzaldehyde and
1-(3,5-dichlorophenyl)-3-methoxymethyl-2-pyrazolin-5-one(compound of the Example 521(1)).
Yield: 32.4%(yellowish orange solid).
¹H-NMR(DMSO.d₆):*δ* 3.33(3H,s), 4.48(2H,s), 6.44(1H,s), 7.42(1H,t,J=2.1Hz), 8.05(2H,d,J=2.1Hz), 8.27(1H,s), 8.91(1H,s), 9.05(1H,brs), 10.62(1H,s), 10.87(1H,brs).

### Example 523: Preparation of the compound of Compound No. 523.

The title compound was obtained in the same manner as the Example 501 using the following raw materials.
Raw materials: 2-hydroxybenzaldehyde and
1-(3,5-dichlorophenyl)-3-methoxymethyl-2-pyrazolin-5-one(compound of the Example 521(1)).
Yield: 49.3%(orange solid).
¹H-NMR(DMSO-d₆) : *δ* 3.47(3H,s), 4.61(2H,s), 7.02-7.16(2H,m), 7.23(1H,t,J=1.8Hz), 7.51-7.70(2H,s), 8.03(2H,d,J=1.8Hz), 8.10(1H,s), 9.81(1H,brs).

### Example 524: Preparation of the compound of Compound No. 524.

The title compound was obtained in the same manner as the Example 501 using the following raw materials.
Raw materials: 5-styrylsalicylaldehyde(compound of the Example 322(1)) and 1-(3,5-dichlorophenyl)-3-methoxymethyl-2-pyrazolin-5-one(compound of the Example 521(1)).
Yield: 63.3%(orange solid).
¹H-NMR(CDCl₃) : *δ* 3.49(3H,s), 4.62(2H,s), 7.05(2H,s), 7.23(1H,t,J=8.1Hz), 7.24-7.30(1H,m), 7.34-7.39(2H,m), 7.49-7.53(2H,m), 7.71-7.77(2H,m), 8.03(2H,d,J=1.8Hz), 8.09(1H,s), 10.09(1H,s).

### Example 525: Preparation of the compound of Compound No. 525.

The title compound was obtained in the same manner as the Example 501 using the following raw materials.
Raw materials: 5-styrylsalicylaldehyde(compound of the Example 322(1)) and 1-(3,5-dichlorophenyl)-3-isopropyl-2-pyrazolin-5-one(compound of the Example 104(1)).
Yield: 26.9%(orange solid).
¹H-NMR(CDCl₃): *δ* 1.43(6H,d,J=6.9Hz), 3.06-3.24(1H,m), 7.05(2H,s), 7.17(1H,d,J=8.4Hz), 7.22(1H,t,J=2.1Hz), 7.26-7.30(1H,m), 7.34-7.40(2H,m), 7.48-7.53(2H,m), 7.65(1H,d,J=2.4Hz), 7.70(1H,s), 7.74(1H,dd,J=8.4,2.4Hz), 8.06(2H,d,J=2.1Hz), 10.11(1H,brs).

### Example 526: Preparation of the compound of Compound No. 526.

The title compound was obtained in the same manner as the Example 501 using the following raw materials.
Raw materials: 5-bromosalicylaldehyde and
1-(3,5-dichlorophenyl)-3-isopropyl-2-pyrazolin-5-one(compound of the Example 104(1)).
Yield: 26.9%(orange solid).
¹H.NMR(CDCl₃): *δ* 1.40(6H,d,J=6.9Hz), 3.08-3.18(1H,m), 7.05(1H,d,J=8.7Hz), 7.21-7.23(1H,m), 7.56-7.70(1H,m), 7.57(1H,dd,J=8.7,2.4Hz), 7.73(1H,d,J=2.4Hz), 8.03(2H,d,J=1.8Hz), 9.81(1H,s).

### Example 527: Preparation of the compound of Compound No. 527.

The title compound was obtained in the same manner as the Example 501 using the following raw materials.
Raw materials: 4-phenoxybenzaldehyde and
1-(3,5-dichlorophenyl)-3-methoxymethyl-2-pyrazolin-5-one(compound of the Example 521(1)).
Yield: 29.3%(orange solid).
¹H-NMR(CDCl₃): *δ* 3.45(3H,s), 4.55(2H,m), 7.04-7.27(6H,m), 7.39-7.46(2H,m), 7.78(1H,s), 8.02(1H,d,J=2.1Hz), 8.54(2H,d,J=9.0Hz).

### Example 528: Preparation of the compound of Compound No. 528:

The title compound was obtained in the same manner as the Example 501 using the following raw materials.
Raw materials: 3-methoxy-5-bromosalicylaldehyde and
1-(3,5-dichlorophenyl)-3-isopropyl-2-pyrazolin-5-one(compound of the Example 104(1)).
Yield: 76.6%(orange solid).
¹H-NMR(CDCl₃): *δ* 1.38(6H,d,J=6.9Hz), 3.08-3.18(1H,m), 3.94(3H,s), 7.13(1H,d,J=1.8Hz), 7.15(1H,t,J=2.1Hz), 8.03(2H,d,J=2.1Hz), 8.67(1H,d,J=1.8Hz).

### Example 529: Preparation of the compound of Compound No. 529.

The title compound was obtained in the same manner as the Example 519 using the following raw material.
Raw material: 4-(2-hydroxy-3-methoxy-5-bromobenzylidene-1-(3,5-dichlorophenyl)-3-isopropyl-2-pyrazolin-5-one(Compound No. 528).
Yield: 41.2%(red solid).
¹H.NMR(CDCl₃): *δ* 1.43(2H,d,J=6.9Hz), 3.06-3.20(1H,m), 6.48(1H,s), 7.13(1H,d,J=2.1Hz), 7.25(1H,t,J=2.1Hz), 7.30(1H,d,J=2.1Hz), 7.55(1H,s), 8.02(2H,d,J=2.1Hz), 10.86(1H,s).

### Example 530: Preparation of the compound of Compound No. 530.

The title compound was obtained in the same manner as the Example 501 using the following raw materials.
Raw materials: 5-styrylsalicylaldehyde(compound of the Example 322(1)) and
1-(4-chlorophenyl)-3-(4-hydroxyphenyl)-2-pyrazolin-5-one(compound of the Example 520(1)).
Yield: 29.3%(orange solid).
¹H-NMR(DMSO-ds): *δ* 6.91-7.59(3H,m), 7.16(2H,d,J=7.8Hz), 7.23-7.41(4H,m), 7.50-7.61(5H,m), 7.76(1H,dd,J=2.1,8.7Hz), 8.03-8.07(2H,m), 8.18(1H,s), 9.20(1H,s), 10.99(1H,s).

### Example 531: Preparation of the compound of Compound No. 531.

(1) 1-(4-Nitrophenyl)-3-isopropyl-2-pyrazolin-5-one.
   The title compound was obtained in the same manner as the Example 502(1) using the following raw materials.
   Raw materials: 4-nitrophenylhydrazine and ethyl isobutyrylacetate.
   Yield: 65.4%(yellow solid).
   ¹H-NMR(DMSO-ds): *δ* 1.20(6H,d,J=6.9Hz), 2.76-2.87(1H,m), 5.48(1H,s), 8.07(2H,d,J=9.3Hz), 8.31(2H,d,J=9.3Hz), 12.18(1H,s).
(2) 4-(2-Hydroxy-5-styrylbenzylidene)-1-(4-nitrophenyl)-3-isopropyl-2-pyrazolin-5-one (Compound No. 531).
   The title compound was obtained in the same manner as the Example 501 using the following raw materials.
   Raw materials: 5-styrylsalicylaldehyde(compound of the Example 322(1)) and 1-(4-nitrophenyl)-3-isopropyl-2-pyrazolin-5-one.
   Yield: 72.7%(orange solid).
   ¹H.NMR(DMSO-d₆): *δ* 1.35(6H,d,J=6.9Hz), 3.16- 3.27(1H,m), 7.04(1H,d,J =8.4Hz), 7.14-7.41(5H,m), 7.58-7.60(2H,m), 7.77(1H,dd,J=8.4,2.1Hz), 8.22(1H,s), 8.23-8.40(4H,m), 9.12(1H,d,J=2.1Hz), 11.10(1H,s).

### Example 532: Preparation of the compound of Compound No. 532.

(1) 2-Methoxy-5-styrylbenzaldehyde.
   The title compound was obtained in the same manner as the Example 322(1) using the following raw materials.
   Raw materials: 2-methoxy-5-bromobenzaldehyde and styrene.
   Yield: 41.3%(light yellow solid).
   ¹H-NMR(CDCl₃-d₆): *δ* 3.95(3H,s), 6.98(1H,d,J=8.7Hz), 7.23-7.28(1H,m), 7.31-7.39(1H,m), 7.47-7.51(1H,m), 7.68(1H,dd,J=8.7,2.4Hz), 7.99(1H,d,J=2.4Hz), 10.48(1H,s).
(2) 4-(2-Methoxy-5-styrylbenzylidene)-1-(4-nitrophenyl)-3-isopropyl-2-pyrazolin-5-one (Compound No. 532).

The title compound was obtained in the same manner as the Example 501 using the following raw materials.
Raw materials: 2-methoxy5-styrylbenzaldehyde and
1-(4-nitrophenyl)-3-isopropyl-2-pyrazolin-5-one(compound of the Example 531(1)).
Yield: 82.3%(orange solid).
¹H-NMR(DMSO-d₆): *δ* 1.35(6H,d,J=6.9Hz), 3.16-3.29(1H,m), 3.97(3H,s), 7.15-7.30(4H,m), 7.37-7.42(2H,m), 7.59-7.61(2H,m), 7.87(1H,dd,J=8.4,2.1Hz), 8.12(1H,s), 8.20-8.40(4H,m), 8.98(1H,d,J=2.1Hz).

### Example 533: Preparation of the compound of Compound No. 533.

The title compound was obtained in the same manner as the Example 501 using the following raw materials.
Raw materials: 5-styrylsalicylaldehyde(compound of the Example 322(1)) and 1-(4-nitrophenyl)-3-methyl-2-pyrazolin-5-one.
Yield: 85.8%(red solid).
¹H-NMR(DMSO-d₆): *δ* 2.36(3H,s), 7.02-7.41(6H,m), 7.57-7.60(2H,m), 7.77(1H dd,J=8.4,2.1Hz), 8.08(1H,s), 8.23-8.35(4H,m), 9.19(1H,d,J=2.1Hz), 11.14(1H,s).

### Example 534: Preparation of the compound of Compound No. 534.

The title compound was obtained in the same manner as the Example 501 using the following raw materials.
Raw materials: 5-styrylsalicylaldehyde(compound of the Example 322(1)) and
1-(4-carboxyphenyl)-3-isopropyl-2-pyrazolin-5-one(compound of the Example 507(1)).
Yield: 30.9%(brown solid).
¹H-NMR(DMSO-d₆):δ 1.34(6H,d,J=6.9Hz), 3.14-3.27(1H,m), 7.04(1H,d,J=6.9Hz), 7.15(2H,d,J=7.8Hz), 7.22-7.28(1H,m), 7.35-7.40(2H,m), 7.58-7.61(2H,m), 7.76(1H,dd,J=8.4,2.1Hz), 8.01-8.14(5H,m), 9.14(1H,d,J=2.1Hz), 11.05(1H,brs), 12.85(1H,brs).

### Example 535: Preparation of the compound of Compound No. 535.

The title compound was obtained in the same manner as the Example 501 using the following raw materials.
Raw materials: 2-methoxy-5-styrylbenzaldehyde(compound of the Example 532(1)) and 1-(4-carboxyphenyl)-3-isopropyl-2-pyrazolin-5-one(compound of the Example 507(1)).
Yield: 79.9%(orange solid).
¹H-NMR(DMSO-ds)^{:} δ 1.34(6H,d,J=6.9Hz), 3.13-3.25(1H,m), 3.96(3H,s), 7.20-7.41(6H,m), 7.59(2H,d,J=7.2Hz), 7.86(1H,dd,J=2.4,9.0Hz), 8.00-8.11(SH,m), 8.99(1H,d,J=2.4Hz), 12.83(1H,brs).

### Example 536: Preparation of the compound of Compound No. 536.

The title compound was obtained in the same manner as the Example 501 using the following raw materials.
Raw materials: 4-formyl-trans-stilbene and 1-(4-carboxyphenyl)-3-isopropyl-2-pyrazolin-5-one(compound of the Example 507(1)).
Yield: 71.8%(red solid).
¹H-NMR(DMSO-d₆): δ 1.33(6H,d,J=7.2Hz), 3.26-3.37(1H,m), 7.30-7.45(4H,m), 7.53(1H,d,J=16.5Hz), 7.68(2H,d,J=7.2Hz), 7.81(2H,d,J=8.4Hz), 7.91(1H,s), 8.01-8.13(4H,m), 8.65(2H,d,J=8.4Hz), 12.85(1H,brs).

### Example 537: Preparation of the compound of Compound No. 537.

The title compound was obtained in the same manner as the Example 501 using the following raw materials.
Raw materials: 2-methoxy-5-styrylbenzaldehyde(compound of the Example 532(1)) and 1-(4-nitrophenyl)-3-ethoxy-2-pyrazolin-5-one.
Yield: 63.4%(red solid).
¹H-NMR(CDCl₃): δ 1.52(3H,t,J=7.2Hz), 3.96(3H,s), 4.53(2H,q,J=7.2Hz), 6.94-7.14(3H,m), 7.27-7.51(5H,m), 7.65(1H,dd,J=9.0,2.4Hz), 8.18-8.30(4H,m), 8.40(1H,s), 8.42(1H,d,J=2.4Hz).

### Example 538: Preparation of the compound of Compound No. 538.

(1) 1-(4-Nitrophenyl)-3-methoxymethyl-2-pyrazolin-5-one.
   The title compound was obtained in the same manner as the Example 509(1) using the following raw materials.
   Raw materials: 4-nitrophenylhydrazine and methyl 4-methoxyacetoacetate.
   Yield: 15.4%(light yellow solid).
   ¹H-NMR(CDCl₃): *δ* 3.46(3H,s), 3.61(2H,s), 4.32(2H,s), 8.13(2H,d,J=9.6Hz), 8.27(2H,d,J=9.6Hz).
(2) 4-(2-Hydroxy-5-styrylbenzylidene)-1-(4-nitrophenyl)-3-methoxymethyl-2-pyrazolin-5-one (Compound No. 538).
   The title compound was obtained in the same manner as the Example 501 using the following raw materials.
   Raw materials: 5-styrylsalicylaldehyde(compound of the Example 322(1)) and 1-(4-nitrophenyl)-3-methoxymethyl-2-pyrazolin-5-one.
   Yield: 73.6%(red solid).
   ¹H-NMR(DMSO-ds): *δ* 3.39(3H,s), 4.58(2H,s), 7.04(1H,d,J=8.7Hz), 7.15-7.41(5H,m), 7.58-7.61(2H,m), 7.80(1H,dd,J=2.1,8.7Hz), 8.21-8.41(5H,m), 9.24(1H,d,J=2.1Hz), 10.99(1H,s), 11.18(1H,s).

### Example 539: Preparation of the compound of Compound No. 539.

The title compound was obtained in the same manner as the Example 501 using the following raw materials.
Raw materials: 5-bromovaniline and 1-(3,5-dichlorophenyl)-3-isopropyl-2-pyrazolin-5-one(compound of the Example 104(1)).
Yield: 70.0%(orange solid).
¹H-NMR(DMSO-ds): *δ* 1.31(6H,d, J=6.6Hz), 3.17-3.35(1H,m), 3.95(3H,s), 7.38(1H,t,J=2.1Hz), 7.81(1H,s), 7.97(2H,d,J=2.1Hz), 8.55(1H,d,J=1.5Hz), 8.59(1H,d,J=1.5Hz), 11.06(1H,brs).

### Example 540: Preparation of the compound of Compound No. 540.

The title compound was obtained in the same manner as the Example 519 using the following raw materials.
Raw materials: 4-(3-Methoxy-4-hydroxy-5-bromobenzylidene)-1-(3,5-dichlorophenyl)-3-isopropyl-2-pyrazolin-5-one(Compound No. 539).
Yield: 84.3%(orange solid).
¹H-NMR(DMSO-d₆): *δ* 1.31(6H,d,J=6.6Hz), 3.23-3.33(1H,m), 7.42(1H,t,J=1.8Hz), 7.75(1H,s), 8.01(2H,d, J=1.8Hz), 8.29(1H,d,J=2.1Hz), 8.50(1H,d,J=2.1Hz), 10.51(1H,brs), 10.75(1H,brs).

### Example 541: Preparation of the compound of Compound No. 541.

The title compound was obtained in the same manner as the Example 501 using the following raw materials.
Raw materials: 5-styrylsalicylaldehyde(compound of the Example 322(1)) and 1-(4-nitrophenyl)-3-ethoxy-2-pyrazolin-5-one.
Yield: 55.8%(red solid).
¹H-NMR(DMSO-d₆): *δ* 1.41-1.47(3H,m), 4.39-4.50(2H,m), 6.98-7.78(9H,m), 8.08-9.27(6H,m), 11.13-11.19(1H,m).

### Example 542: Preparation of the compound of Compound No. 542.

(1) 2-Styryl-4,5-dimethoxybenzaldehyde.
   The title compound was obtained in the same manner as the Example 322(1) using the following raw materials.
   Raw materials: 2-bromo-4,5-dimethoxybenzaldehyde and styrene.
   Yield: 35.7%(light yellow solid).
   ¹H-NMR(CDCl₃): *δ* 3.96(3H,s), 4.03(3H,s), 6.97(1H,d,J=16.2Hz), 7.10(1H,s), 7.26-7.57(6H,m), 7.88(1H,d,J=16.2Hz), 10.33(1H,s).
(2) 4-(2-Styryl-4,5-dimethoxybenzylidene)-1-(4-nitrophenyl)-3-ethoxy-2-pyrazolin-5-one (Compound No. 542).

The title compound was obtained in the same manner as the Example 501 using the following raw materials.
Raw materials: 2-styryl-4,5-dimethoxybenzaldehyde and 1-(4-nitrophenyl)-3-ethoxy-2-pyrazolin-5-one.
Yield: 55.8%(red solid).
¹H-NMR(CDCl₃): *δ* 1.47(3H,t,J=6.9Hz), 4.04(3H,s), 4.08(3H,s), 4.48(2H,q,J=6.9Hz), 6.87(1H,d,J=15.9Hz), 7.07(1H,s), 7.33-7.57(6H,m), 8.08(1H,s), 8.21-8.29(4H,m), 8.99(1H,s).

### Example 543: Preparation of the compound of Compound No. 543.

A mixture of 4-(2-hydroxy-5-styrylbenzylidene)-1-(4-nitrophenyl)-3-ethoxy-2-pyrazolin-5-one(Compound No. 541; 100 mg, 0.220 mmol), tert-butyldimethylsilyl chloride(76 mg, 0.505 mmol), imidazole(21 mg, 0.307 mmol) and dimethylformamide(1.0 mL) was stirred at room temperature for 1 hour. Water was added to the reaction mixture, and the separated solid was collected by filtration. The solid was washed successively with methanol and ethyl acetate, and purified by chromatography on silica gel(dichloromethane) to give the title compound(48 mg, 38.4%) as a red solid.
¹H-NMR(CDCl₃):δ 0.28-0.31(6H,m), 1.07-1.09(9H,m), 1.47-1.58(3H,m), 4.43-4.59(2H,m), 6.87-7.67(15H,m).

### Example 544: Preparation of the compound of Compound No. 544.

(1) 1-{4-[2-(Pyridin-2-yl)vinyl]phenyl}-3-isopropyl-2-pyrazolin-5-one.
   The title compound was obtained in the same manner as the Example 509(1) using the following raw materials.
   Raw materials: 4'-hydrazino-2-stilbazole dihydrochloride and ethyl isobutyrylacetate.
   Yield: 33.3%(yellow solid).
   ¹H-NMR(CDCl₃): *δ* 1.26(6H,d,J=6.9Hz), 2.74-2.86(1H,m), 3.44(2H,s), 7.11-7.17(2H,m), 7.39(1H,d,J=8.1Hz), 7.58-7.69(4H,m), 7.93(2H,d,J=9.0Hz), 8.59-8.61(1H,m).
(2) 4-(2-Hydroxy-5-styrylbenzylidene)-1-{4-[2-(pyridin-2-yl)vinyl]phenyl}-3-isopropyl-2-pyrazolin-5-one (Compound No. 544).
   The title compound was obtained in the same manner as the Example 501 using the following raw materials.
   Raw materials: 5-styrylsalicylaldehyde(compound of the Example 322(1)) and 1-{4-[2-(pyridin-2-yl)vinyl]phenyl}-3-isopropyl-2-pyrazolin-5-one.
   Yield: 39.9%(orange solid).
   ¹H-NMR(DMSO-d₆): *δ* 2.50(6H,d,J=6.9Hz), 3.15.3.25(1H,m), 7.00-8.06(18H,m), 8.13(1H,s), 8.56-8.58(1H,m), 9.19(1H,d,J=2.4Hz), 11.02(1H,s).

### Example 545: Preparation of the compound of Compound No. 545.

The title compound was obtained in the same manner as the Example 501 using the following raw materials.
Raw materials: 2,4,5-trihydroxybenzaldehyde and 1-{4-[2-(pyridin-2-yl)viny1]phenyI}-3-isopropyl-2-pyrazolin -5-one(compound of the Example 544(1)).
Yield: 0.8%(red solid).
¹H-NMR(CDaOD):*δ* 1.28-1.39(6H,m), 3.12-3.22(1H,m), 6.39(1H,s), 7.19-7.94(6H,m), 8.02(2H,d,J=8.7Hz), 8.20(1H,s), 8.49-8.51(2H,m), 8.85(1H,brs).

### Example 546: Preparation of the compound of Compound No. 546.

The title compound was obtained in the same manner as the Example 501 using the following raw materials.
Raw materials: 2-hydroxy-1-naphthaldehyde and 1-(4-nitrophenyl)-3-ethoxy-2-pyrazolin-5-one.
Yield: 32.9%(reddish brown solid).
¹H-NMR(CDCl₃): *δ* 1.57(3H,t,J=7.2Hz), 4.58(2H,q,J=7.2Hz), 7.33(1H,d,J=9.0Hz), 7.47-7.69(2H,m), 7.84(1H,d,J=7.8Hz), 7.93(1H,d,J=8.4Hz), 8.01(1H,d,J=9.0Hz), 8.26-8.33(4H,m), 8.54(1H,s), 10.69(1H,brs).

### Example 547: Preparation of the compound of Compound No. 547.

The title compound was obtained in the same manner as the Example 501 using the following raw materials.
Raw materials: 9-anthraldehyde and 1-(4-nitrophenyl)-3-ethoxy-2-pyrazolin-5-one.
Yield: 31.0%(red solid).
¹H-NMR(CDCl₃):δ 0.59(3H,t,J=6.6Hz), 3.97(2H,q,J=6.6Hz), 7.51-7.57(4H,m), 8.02-8.33(8H,m), 8.58(1H,s), 8.93(1H,s).

### Example 548: Preparation of the compound of Compound No. 548.

The title compound was obtained in the same manner as the Example 501 using the following raw materials.
Raw materials: 5-(tert-butyl)salicylaldehyde and 1-(4-nitrophenyl)-3-ethoxy-2-pyrazolin-5-one.
Yield: 49.7%(orange solid).
¹H-NMR(DMSO-d₆):δ 1.29(9H,s), 1.46(3H,t,J=6.9Hz), 4.52(2H,q,J=6.9Hz), 6.95(1H,d,J=8.7Hz), 7.54(1H,dd,J=8.7,2.4Hz), 8.11-8.33(6H,m), 10.78(1H,s).

### Example 549: Preparation of the compound of Compound No. 549.

The title compound was obtained in the same manner as the Example 501 using the following raw materials.
Raw materials: 3-methoxy-6-bromosalicylaldehyde and 1-(4-nitrophenyl)-3-ethoxy-2-pyrazolin-5-one.
Yield: 37.5%(reddish brown solid).
¹H-NMR(DMSO-d₆): *δ* 1.15-1.21(3H,m), 3.84-3.85(3H,m), 4.24-4.55(2H,m), 7.00-8.37(7H,m), 9.84-9.88(1H,m).

### Example 550: Preparation of the compound of Compound No. 550.

The title compound was obtained in the same manner as the Example 501 using the following raw materials.
Raw materials: 6-methoxysalicylaldehyde and 1-(4-nitrophenyl)-3-ethoxy-2-pyrazolin-5-one.
Yield: 45.6%(yellow solid).
¹H-NMR(DMSO-d₆): *δ* 1.46(3H,t,J=6.9Hz), 3.79(3H,s), 4.47(2H,d,J=6.9Hz), 6.56(2H,d,J=8.4Hz), 7.33(1H,t,J=8.1Hz), 7.75(1H,s), 7.75-8.35(4H,m), 10.47(1H,s).

### Example 551: Preparation of the compound of Compound No. 551.

The title compound was obtained in the same manner as the Example 519 using the following raw material.
Raw material: 4-(2-hydroxy-3-methoxy-6-bromobenzylidene)-1-(4-nitrophenyl)-3-ethoxy-2-pyrazolin-5-one(Compound No. 549).
Yield: 8.6%(ocherous solid).
¹H-NMR(DMSO-d₆): *δ* 1.19-1.49(3H,m), 4.25-4.55(2H,m), 6.81-7.01(2H,m), 7.65-7.77(1H,m), 7.76-8.37(4H,m), 9.52-9.98(2H,m).

### Example 552: Preparation of the compound of Compound No. 552.

The title compound was obtained in the same manner as the Example 519 using the following raw material.
Raw material: 4-(2-hydroxy-6-methoxybenzylidene)-1-(4-nitrophenyl)-3-ethoxy-2-pyrazolin-5-one(Compound No. 550).
Yield: 38.7%(ocherous solid).
¹H-NMR(DMSO-d₆): *δ* 1.25-1.48(3H,m), 4.27-4.53(2H,m), 6.36-6.42(2H,m), 7.09-7.22(1H,m), 7.85-7.22(1H,m), 7.85-7.86(4H,m), 10.18-10.38(2H,m).

### Example 553: Preparation of the compound of Compound No. 553.

L-(+)-Arginine(141 mg, 0.810 mmol) was added portionwise to 4-(2,4,5-trihydroxybenzylidene)-1-(3,4-dichlorophenyl)-3-isopropyl-2-pyrazolin-5-one( Compound No. 141; 100mg, 0.2701mmol) and the compounds were mixed in the morter. The mixture was washed with ethyl acetate under suspension to give the title compound(170 mg, 67.7%) as a red solid.
¹H-NMR(D₂O): *δ* 1.32(6H,d,J=6.9Hz), 1.60-1.86(12H,m), 3.17-3.30(7H,m), 3.54(3H,t,J=6.0Hz), 7.62(1H,d,J=9.0Hz), 7.75(1H,dd,J=9.0,2.4Hz), 7.89(1H,s), 7.99(1H,d,J=2.4Hz).

### Example 554: Preparation of the compound of Compound No. 554.

L-(+)-Arginine(94 mg, 0.540 mmol) was added portionwise to 4-(2,4,5-trihydroxybenzylidene)-1-(3,4-dichlorophenyl)-3-isopropyl-2-pyrazolin-5-one( Compound No. 141; 100 mg, 0.2701 mmol) and the compounds were mixed in the morter. The mixture was washed with dichloromethane under suspension to give the title compound(100 mg, 48.9%) as a red solid.
¹H-NMR(D₂O): *δ* 1.29(6H,d,J=6.9Hz), 1.60-1.92(8H,m), 3.14-3.26(5H,m), 3.69(3H,t,J=6.3Hz), 7.59(1H,d,J=8.4Hz), 7.71(1H,dd,J=8.8,2.4Hz), 7.90(1H,s), 7.25(1H,d,J=2.4Hz).

### Example 555: Preparation of the compound of Compound No. 555.

The title compound was obtained in the same manner as the Example 501 using the following raw materials.
Raw materials: methyl terephthalaldehydate and 1-(4-carboxyphenyl)-3-methyl-2-pyrazolin-5-one.
Yield: 80.7%(red solid).
¹H.NMR(DMSO·d₆): *δ* 2.34(3H,s), 3.89(3H,s), 7.87(1H,s), 7.98-8.07(6H,m), 8.57-8.80(2H,d,J=8.4Hz).

### Example 556: Preparation of the compound of Compound No. 556.

(1) 4-(2-Bromo-4,5-dimethoxybenzylidene)-1-(3,5-dichlorophenyl)-3-isopropyl-2-pyrazolin-5-one.
   The title compound was obtained in the same manner as the Example 501 using the following raw materials.
   Raw materials: 2-bromo-4,5-dimethoxybenzaldehyde and 1-(3,5-dichlorophenyl)-3-isopropyl-2-pyrazolin-5-one(compound of the Example 104(1)).
   Yield: 77.0%(red solid).
   ¹H-NMR(CDCl₃):δ 1.41(6H,d,J=6.9Hz), 3.06-3.02(1H,m), 3.97(3H,s), 4.05(3H,s), 7.14-7.16(2H,m), 7.94(1H,s), 8.01(2H,d,J=1.8Hz), 9.18(1H,s).
(2) 4-(2-Bromo-4,5-dihydroxybenzylidene)-1-(3,5-dichlorophenyl)-3-isopropyl-2-pyrazolin-5-one (Compound No. 556).
   Boron tribromide(1.0M solution in dichloromethane; 0.7 mL, 0.7 mmol) was added slowly to a solution of 4-(2-bromo-4,5-dimethoxybenzylidene)-1-(3,5-dichlorophenyl)-3-isopropyl-2-pyrazolin-5-one(50 mg, 0.1 mmol) in dichloromethane(1.5 mL) at 0°C, and the mixture was stirred at room temperature for 1 hour. Water was added to the reaction mixture, and the separated solid was collected by filtration and washed with methanol under suspension to give the title compound(35 mg, 74.4%) as a reddish orange solid.
   ¹H-NMR(DMSO-d₆): *δ* 1.33(6H,d,J=6.9Hz), 3.10-3.24(1H,m), 7.17(1H,s), 7.42(1H,t,J=1.8Hz), 7.88(1H,s), 7.99(2H,d,J=1.8Hz), 8.78(1H,s), 9.83(1H,brs), 10.84(1H,brs).

### Example 557: Preparation of the compound of Compound No. 557.

(1) 4-(2-Bromo-4,5-dimethoxybenzylidene)-1-(4-nitrophenyl)-3-ethoxy-2-pyrazolin-5-one.
   The title compound was obtained in the same manner as the Example 501 using the following raw materials.
   Raw materials: 2-bromo-4,5-dimethoxybenzaldehyde and 1-(4-nitrophenyl)-3-ethoxy-2-pyrazolin-5-one.
   Yield: 94.8%(brown solid).
   ¹H-NMR(CDCl₃): *δ* 1.54(3H,t,J=6.6Hz), 3.97(3H,s), 4.50(2H,q,J=6.6Hz), 4.11(3H,s), 7.18(1H,s), 8.07(1H,s), 8.17-8.29(4H,m), 9.26(1H,s).
(2) 4-(2-Bromo-4,5-dihydroxybenzylidene)-1-(4-nitrophenyl)-3-ethoxy-2-pyrazolin-5-one (Compound No. 557).
   The title compound was obtained in the same manner as the Example 556(2) using the following raw material.
   Raw material: 4-(2-bromo-4,5-dimethoxybenzylidene)-1-(4-nitrophenyl)-3-ethoxy-2-pyrazolin-5-one.
   Yield: 22.3%(orange solid).
   ¹H-NMR(DMSO-d₆):δ 1.42-1.47(3H,m), 4.43-4.52(2H,m), 7.14-7.17(1H,m), 7.88-8.91(6H,m), 9.66-9.90(1H,m), 10.95(1H,brs).

### Example 558: Preparation of the compound of Compound No. 558.

The title compound was obtained in the same manner as the Example 501 using the following raw materials.
Raw materials: 2-nitro-5-hydroxybenzaldehyde and
1-(3,4-dichlorophenyl)-3-isopropyl-2-pyrazolin-5-one(compound of the Example 134(1)).
Yield: 52.4%(yellow solid).
¹H-NMR(DMSO-ds): *δ* 1.35(6H,d,J=6.9Hz), 3.10-3.25(1H,m), 7.05(1H,dd,J=2.4,9.3Hz), 7.17(1H,d,J=2.7Hz), 7.68(1H,d,J=9.0Hz), 7.82(1H,dd,J=2.4,9.0Hz), 8.09(1H,d,J=2.4Hz), 8.21(1H,d,J=9.3Hz), 8.38(1H,s), 11.23(1H,s).

### Example 559: Preparation of the compound of Compound No. 559.

The title compound was obtained in the same manner as the Example 501 using the following raw materials.
Raw materials: 3-methoxy-4-hydroxy-5-carboxybenzaldehyde(compound of the Example 341(1)) and 1-(3,4-dichlorophenyl)-3-isopropyl-2-pyrazolin-5-one(compound of the Example 134(1)).
Yield: 53.7%(orange solid).
¹H-NMR(DMSO-d₆): *δ* 1.30(6H,d,J=6.9Hz), 3.21-3.35(1H,m), 3.92(1H,s), 7.69(1H,d,J=9.3Hz), 7.91(1H,s), 7.97(1H,dd,J=2.4,9.3Hz), 8.18(1H,d,J=2.4Hz), 8.62(1H,d,J=1.8Hz), 8.95(1H,d,J=1.8Hz), 10.50(1H,s).

### Example 560: Preparation of the compound of Compound No. 560.

The title compound was obtained in the same manner as the Example 501 using the following raw materials.
Raw materials: 5-(diethylamino)salicylaldehyde and
1-(3,4-dichlorophenyl)-3-isopropyl-2-pyrazolin-5-one(compound of the Example 134(1)).
Yield: 5.7%(red solid).
¹H-NMR(DMSO-d₆):δ 1.17(6H,t,J=6.9Hz), 1.29(6H,d,J=6.9Hz), 3.03-3.14(1H,m), 3.46(1H,d,J=6.9Hz), 6.20(1H,d,J=2.4Hz), 6.44(1H,dd,J=2.4,9.6Hz), 7.65(1H,d,J=9.6Hz), 7.97-8.01(2H,m), 8.32(1H,d,J=2.4Hz), 9.41(1H,d,J=9.3Hz), 10.74(1H,s).

### Example 561: Preparation of the compound of Compound No. 561.

The title compound was obtained in the same manner as the Example 501 using the following raw materials.
Raw materials: 5-nitrovaniline and
1-(3,4-dichlorophenyl)-3-isopropyl-2-pyrazolin-5-one(compound of the Example 134(1)).
¹H-NMR(DMSO-d₆): *δ* 1.31(6H,d,J=6.9Hz), 3.20-3.33(1H,m), 3.98(3H,s), 7.69(1H,d,J=9.0Hz), 7.93(1H,s), 7.95(1H,dd,J=2.4,9.0Hz), 8.17(1H,d,J=2.4Hz), 8.81(1H,d,J=1.8Hz), 8.90(1H,d,J=1.8Hz).

### Example 562: Preparation of the compound of Compound No. 562.

(1) 2-Nitro-5-(4-bromophenoxy)benzaldehyde.
   Sodium hydride(173 mg, 4.347 mmol) was added to a solution of 4-bromophenol(501 mg, 2.898 mmol) in dimethylsulfoxide(7mL) at 0°C, and the mixture was stirred at room temperature for 10 minutes.
   5-Fluoro-2-nitrobenzaldehyde(500 mg, 2.898 mmol) was added to the mixture, and the mixture was stirred at 50°C for 2 hours. Brine was added to the reaction mixture, and the mixture was extracted with ethyl acetate. The organic layer was dried over anhydrous sodium sulfate. The residue obtained by evaporation of the solvent under reduced pressure was purified by chromatography on silica gel(hexane:ethyl acetate=3:1) to give the title compound(395 mg, 42.3%) as a yellow solid.
   ¹H-NMR(CDCl₃): *δ* 6.98(2H,d,J=9.0Hz), 7.22(1H,dd,J=2.7,9.0Hz), 7.36(1H,d,J=2.7Hz), 7.58(2H,d,J=9.0Hz), 8.17(1H,d,J=9.0Hz), 10.44(1H,s).
(2) 4-[2-Nitro-5-(4-bromophenoxy)benzylidene]-1-(3,4-dichlorophenyl)-3-isopropyl-2-pyrazolin-5-one (Compound No. 562).
   The title compound was obtained in the same manner as the Example 501 using the following raw materials.
   Raw materials: 2-nitro-5-(4-bromophenoxy)benzaldehyde and 1-(3,4-dichlorophenyl)-3-isopropyl-2-pyrazolin-5-one(compound of the Example 134(1)).
   Yield: 11.2%(orange solid).
   ¹H-NMR(DMSO-d₆): *δ* 1.33(6H,d,J=6.6Hz), 3.08-3.22(1H,m), 7.17-7.23(2H,m), 7 .30(1H,dd,J=9.3,2. 7Hz), 7.59-7.72(4H,m), 7.81(1H,dd,J=9.0,2.4Hz), 8.08(1H,d,J=2.4Hz), 8.32(1H,d,J=9.OHz), 8.33(1H,d,J=2.7Hz).

### Example 563: Preparation of the compound of Compound No. 563.

(1) 2-Nitro-5-(3,4-dimethoxyphenoxy)benzaldehyde.
   The title compound was obtained in the same manner as the Example 562(1) using the following raw materials.
   Raw materials: 5-fluoro-2-nitrobenzaldehyde and 3,4-dimethoxyphenol.
   Yield: 32.2%(light yellow solid).
   ¹H-NMR(CDCl₃-d₆): *δ* 3.86(1H,s), 3.92(1H,s), 6.63-6.70(2H,m), 6.88-6.92(1H,m), 7.18(1H,dd,J=2.7,9.0Hz), 7.33(1H,d,J=3.0Hz), 8.15(1H,d,J=9.3Hz).
(2) 4-[2-Nitro-5-(3,4-dimethoxyphenoxy)benzylidene]-1-(3,4-dichlorophenyl)-3-isopropyl-2-pyrazolin-5-one (Compound No. 563).
   The title compound was obtained in the same manner as the Example 501 using the following raw materials.
   Raw materials: 2-nitro-5-(3,4-dimethoxyphenoxy)benzaldehyde and 1-(3,4-dichlorophenyl)-3-isopropyl-2-pyrazolin-5-one(compound of the Example 134(1)).
   Yield: 52.2%(orange solid).
   ¹H-NMR(CDCl₃): *δ* 1.41(6H,d,J=6.9Hz), 3.00-3.11(1H,m), 3.86(3H,s), 3.89(3H,s), 6.69-6.93(3H,m), 7.14-7.22(2H,m), 7.42(1H,d,J=9.0Hz), 7.77(1H,dd,J=6.3,2.7Hz), 7.95(1H,s), 8.10(1H,d,J=2.4Hz), 8.31(1H,d,J=3.9Hz).

### Example 564: Preparation of the compound of Compound No. 564.

(1) Methyl (2-hydroxy-5-bromobenzoyl)acetate.
   A solution of 5'-bromo-2'-hydroxyacetophenone(1.482 g, 6.892 mmol) in tetrahydrofuran(4mL) was added slowly to lithium bis(trimethylsilyl)amide(29% solution intetrahydrofuran; 11.93g, 20.676 mmol) at -78°C, and the mixture was stirred at -10°C for 30 minutes. A solution of dimethyl carbonate(683 mg, 7.581 mmol) in tetrahydrofuran(4 ml) was added to the mixture at -78°C, and the mixture was stirred at room temperature for 2 hours. The reaction mixture was neutralized by addition of 2N hydrochloric acid at 0°C and extracted with ethyl acetate. The organic layer was washed with brine and dried over anhydrous sodium sulfate. The residue obtained by evaporation of the solvent under reduced pressure was recrystallized from ethyl acetate-hexane to give the title compound(990 mg, 52.6%) as a white solid.
   ¹H-NMR(CDCl₃): *δ* 3.79(3H,s), 4.00(2H,s), 6.93(1H,d,J=9.0Hz), 7.58(1H,dd,J=9.0,2.7Hz), 7.76(1H,d,J=2.7Hz), 11.77(1H,s).
(2) 1-(4-Carboxyphenyl)-3-(2-hydroxy-5-bromophenyl)-2-pyrazolin-5-one.
   The title compound was obtained in the same manner as the Example 509(1) using the following raw materials.
   Raw materials: 4-hydrazinobenzoic acid and methyl (2-hydroxy-5-bromobenzoyl)acetate.
   Yield: 77.3%(light brown solid).
   ¹H-NMR(DMSO-d₆): *δ* 6.29(lH,s), 6.91(1H,d,J=9.OHz), 7.35(1H,dd,J=9.0,2.1Hz), 7.95-8.09(5H,m), 10.74(1H,brs), 12.62(1H,brs).
(3) 4-(3,4-Dichlorobenzylidene)-1-(4-carboxyphenyl)-3-(2-hydroxy-5-bromophenyl)-2-pyrazolin-5-one (Compound No. 564).
   The title compound was obtained in the same manner as the Example 501 using the following raw materials.
   Raw materials: 3,4-dichlorobenzaldehyde and 1-(4-carboxyphenyl)-3-(2-hydroxy-5-bromophenyl)-2-pyrazolin-5-one.
   Yield: 4.8%(red solid).
   ¹H-NMR(DMSO-d₆): *δ* 7.87-8.06(6H,m), 8.23-8.28(3H,m), 8.57(1H,dd,J=1.8,8.4Hz), 9.01(1H,d,J=1.8Hz), 12.90(1H,brs).

### Example 565: Preparation of the compound of Compound No. 565.

(1) 5-(4-Phenoxystyryl)salicylaldehyde.
   The title compound was obtained in the same manner as the Example 322(1) using the following raw materials.
   Raw materials: 5-bromosalicylaldehyde and 4-phenoxystyrene.
   Yield: 47.4%(light yellow solid).
   ¹H-NMR(CDCl₃-d₆): *δ* 6.99-7.16(8H,m), 7.33-7.39(2H,m), 7.47(2H,d,J=8.7Hz), 7.65(1H,d,J=2.1Hz), 7.71(1H,dd,J=2.1,8.7Hz), 9.94(1H,s), 11.01(1H,s).
(2) 4-[2-Hydroxy-5-(4-phenoxystyryl)benzylidene]-1-(4-nitrophenyl)-3-ethoxy-2-pyrazolin-5-one (Compound No. 565).
   The title compound was obtained in the same manner as the Example 501 using the following raw materials.
   Raw materials: 5-(4-phenoxystyryl)salicylaldehyde and 1-(4-nitrophenyl)-3-ethoxy-2-pyrazolin-5-one.
   Yield: 67.4%(red solid).
   ¹H-NMR(DMSO-d₆):*δ* 1.45(3H,t,J=7.2Hz), 4.42-4.53(2H,m), 6.99-7.19(8H,m), 7.39-7.77(5H,m), 8.11-9.26(6H,m), 11.12-11.19(1H,m).

### Example 566: Preparation of the compound of Compound No. 566.

The title compound was obtained in the same manner as the Example 501 using the following raw materials.
Raw materials: 5-(4-phenoxystyryl)salicylaldehyde(compound of the Example 565(1)) and 1-(3,4-dichlorophenyl)-3-isopropyl-2-pyrazolin-5-one(compound of the Example 134(1)).
Yield: 22.2%(red solid).
¹H-NMR(CDCl₃): *δ* 1.42(6H,d,J=6.9Hz), 3.11-3.23(1H,m), 6.99-7.50(13H,m), 7.61(1H,d,J=2.4Hz), 7.69(1H,s), 7.72(1H,dd,J=8.4,2.4Hz), 7.98(1H,dd,J=2.4,9.0Hz), 8.22(1H,d,J=2.4Hz), 10.21(1H,s).

### Example 567: Preparation of the compound of Compound No. 567.

The title compound was obtained in the same manner as the Example 501 using the following raw materials.
Raw materials: 4-phenylbenzaldehyde and 1-(4-carboxyphenyl)-3-methyl-2-pyrazolin-5-one.
Yield: 31.4%(orange solid).
¹H-NMR(DMSO-d₆): *δ* 2.39(3H,s), 7.42-7.56(3H,m), 7.81-7.94(5H,m), 7.99-8.11(4H,m), 12.80(1H,brs).

### Test Example 1: PAI-1 Inhibitory Activity

### Test Method A

To a 50 *µ* L solution obtained by diluting a DMSO solution of the test compound at fourfold concentration of the test concentration (=final concentration when fluorogenic substrate was added) with a Tris buffer (pH8.0), 50 *µ* L of 24 nM recombinant human PAI-1 solution adjusted with the same buffer was added, and incubated for 10 minutes at 37°C. 50 *µ* L of 800IU/ml two-chain tPA (activity standard) solution adjusted with the same buffer was added, and incubated for 10 minutes at 37°C. To this solution, 50 *µ* L solution of 400 *µ* M fluogenic substrate for tPA (Pyr-Gly-Arg-MCA, Peptide Institute, Inc.) adjusted with the same buffer was added, and reacted for 30 minutes at 37°C. The reaction was stopped by adding 50 *µ*L of 2% acetic acid. Fluorescence was measured at the excitation wavelength=360 nm, emission wavelength=460 or 465 nm using a microplate reader (SPECTRAFLUOR or GENios) by TECAN. The fluorescence intensities in the presence of the PAI-1 as the control (DMSO) or in the absence of the PAI-1 (tPA alone) were taken as 100% and 0% of PAI-1 activities, respectively, and inhibitory ratios of PAI-1 activities in the presence of the test compound were obtained.

### <Composition of the Tris Buffer (pH8.0)>

50 mM Tris
150 mM NaCl
1 mM CaCl₂
0.1% PEG6000

### Test Method B

To a 90 *µ* L solution obtained by diluting a DMSO solution of the test compound at 2.2-fold concentration of the test concentration (=final concentration when fluorogenic substrate was added) with a Tris buffer (pH7.5), 10*µ* L of 120nM recombinant human PAI-1 solution adjusted with pH6.6 phosphate buffer was added, and incubated for 15 minutes at room temperature. 50 *µ* L of 800IU/ml two-chain tPA (activity standard) solution adjusted with Tris buffer (pH7.5) was added, and incubated for 30 minutes at room temperature. To this solution, 50 *µ* L solution of 400 *µ* M fluorogenic substrate for tPA (Pyr-Gly-Arg-MCA, Peptide Institute, Inc.) adjusted with a Tris buffer (pH7.5) was added, and reacted for 30 minutes at room temperature. The reaction was stopped by adding 50 *µ* L of 2% acetic acid. Fluorescence was measured at the excitation wavelength=360 nm, emission wavelength=460 or 465 nm using a microplete reader (SPECTRAFLUOR or GENios) by TECAN. The fluorescence intensities in the presence of the PAI-1 as the control (DMSO) or in the absence of the PAI-1 (tPA alone) were taken as 100% and 0% of PAI-1 activities, respectively, and inhibitory ratios of PAI-1 activities in the presence of the test compound were obtained.

### <Composition of the Tris Buffer (pH7.5)>

50 mM Tris
150 mM NaCl
10 *µ* g/ml BSA
0.01% Tween80

### <Composition of the Phosphate Buffer (pH6.6)>

50 mM phosphoric acid
100 mM NaCl
1 mM EDTA

In either method of Test Method A or B, fluorescence of tPA activation by the test compound in the absence of PAI-1 or that of the test compound was not observed. The results are shown in the following table.

In the following table, "method B" described in the column of the inhibitory ratio means that the results were measured by the above Test Method B. The other results indicated were measured by the above Test Method A.

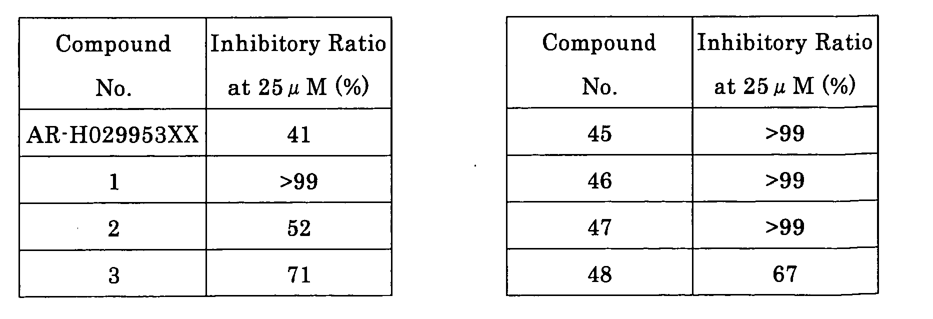

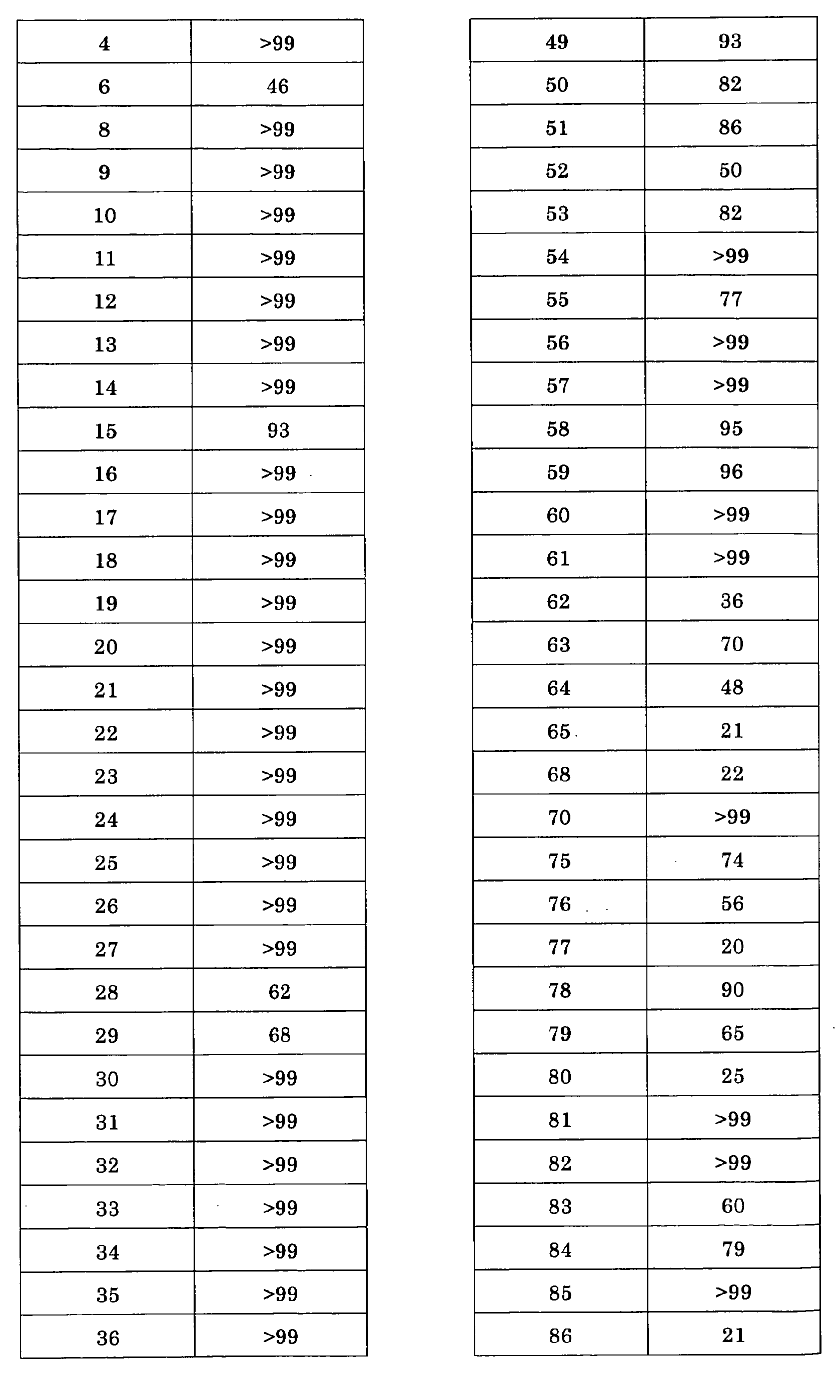

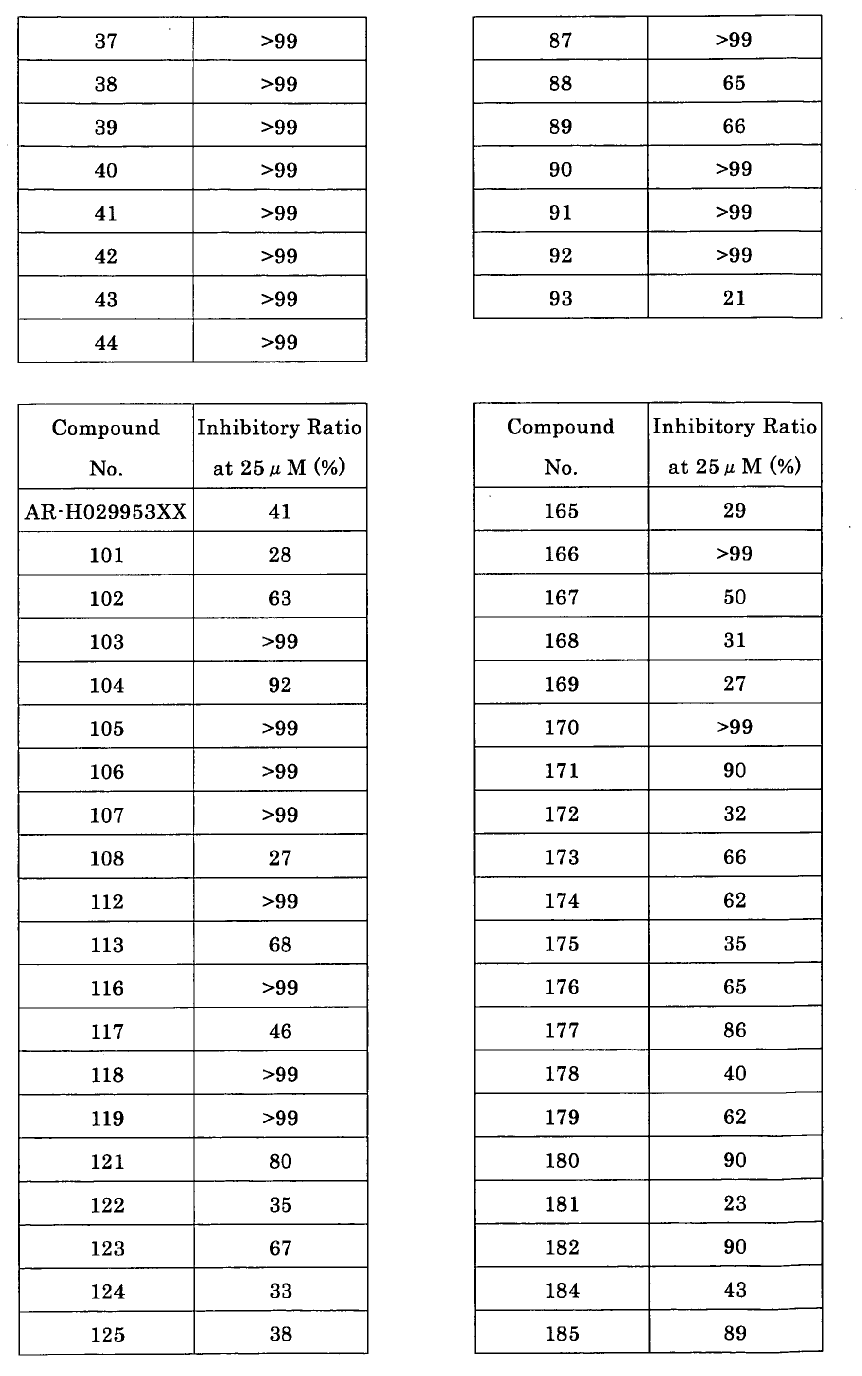

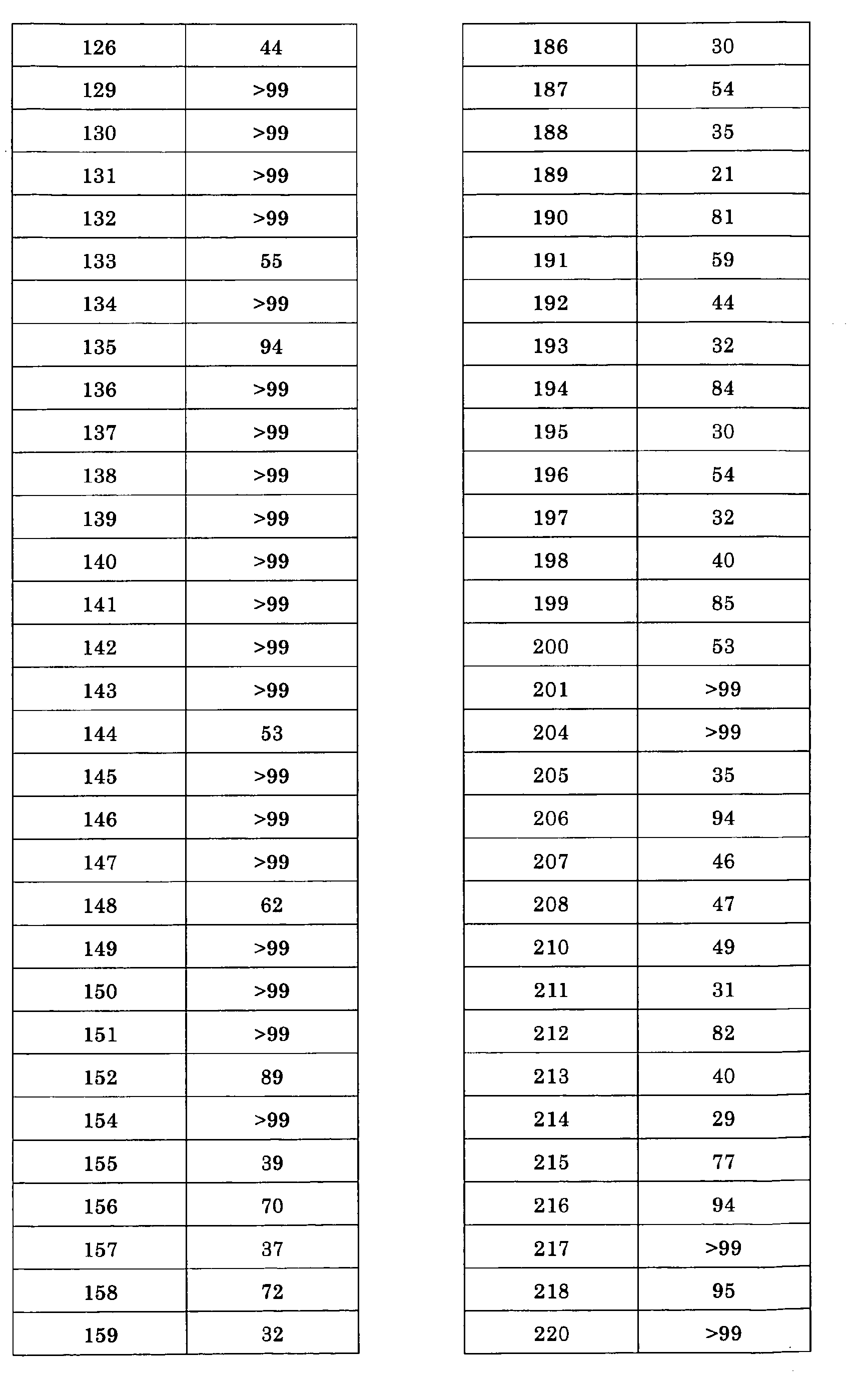

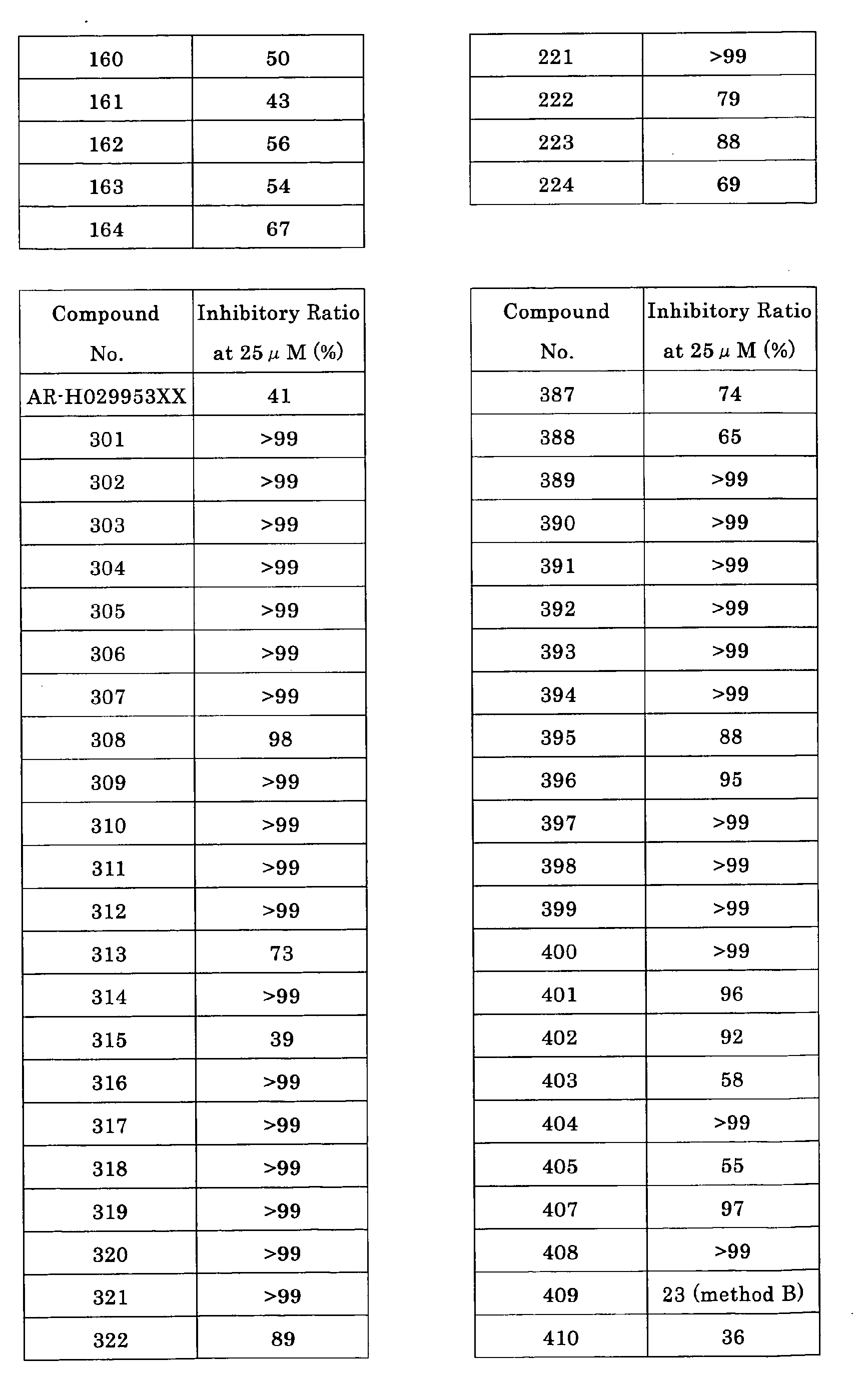

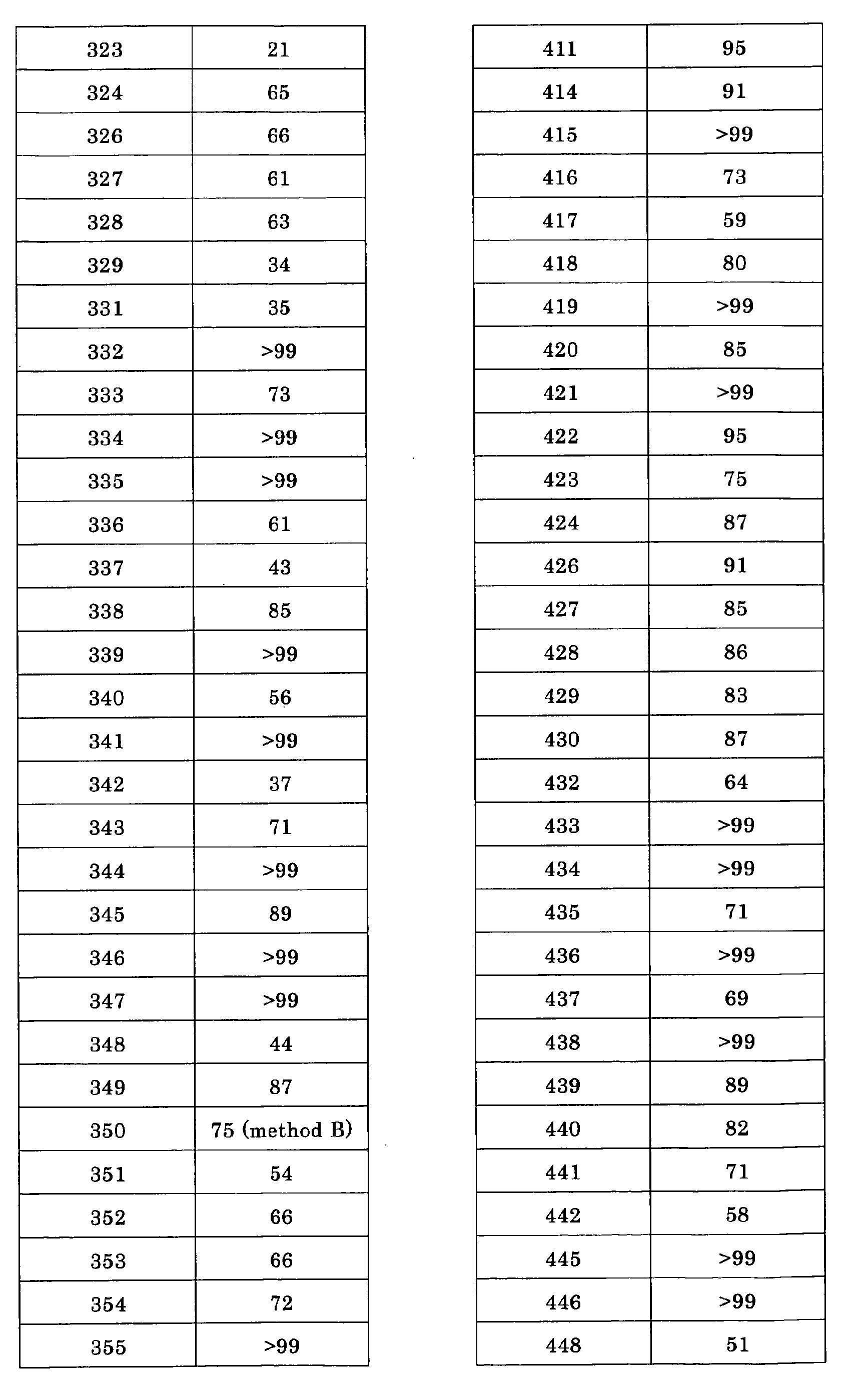

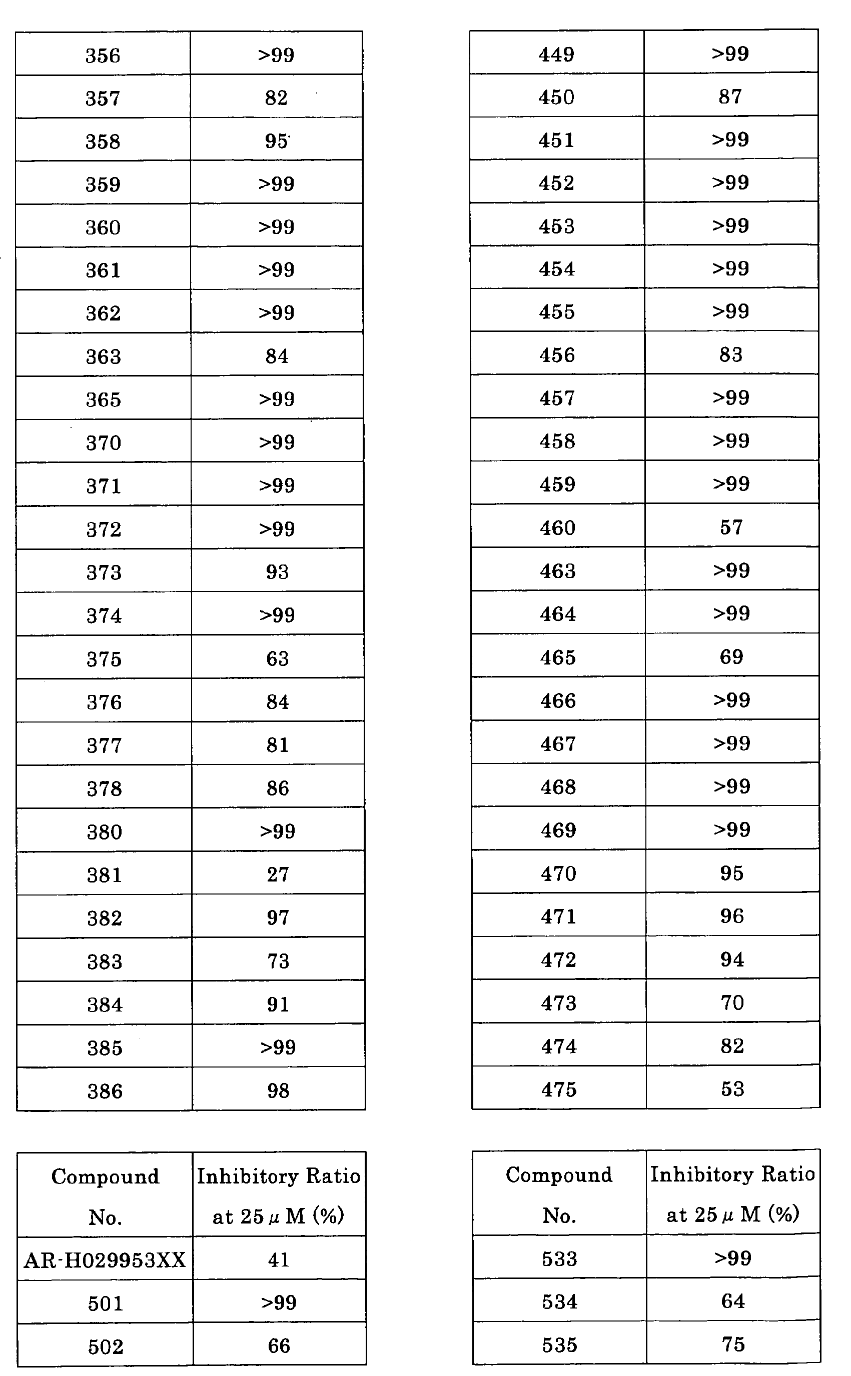

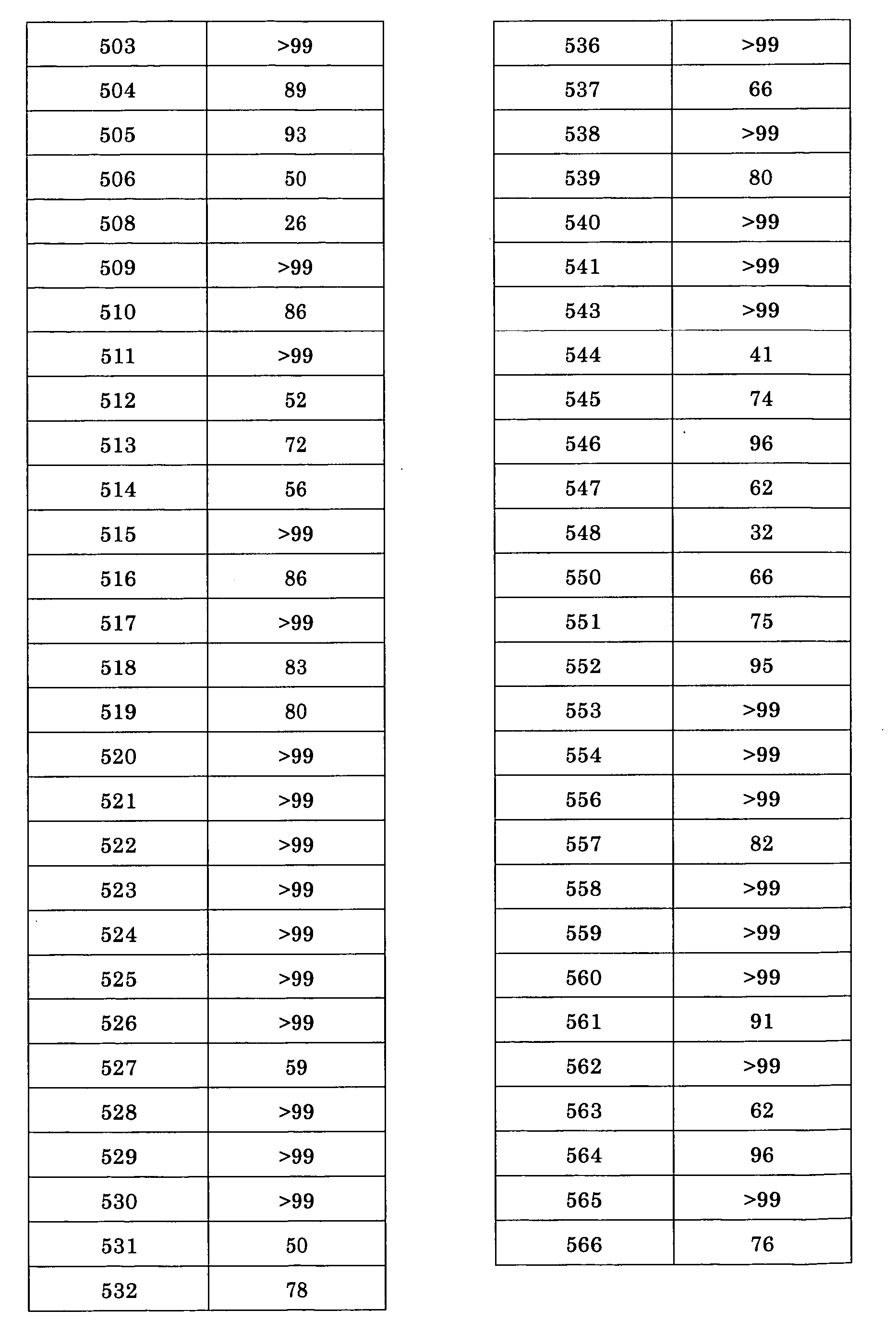

50% Inhibitory concentrations were measured according to the aforementioned method. The results are shown in the following table.

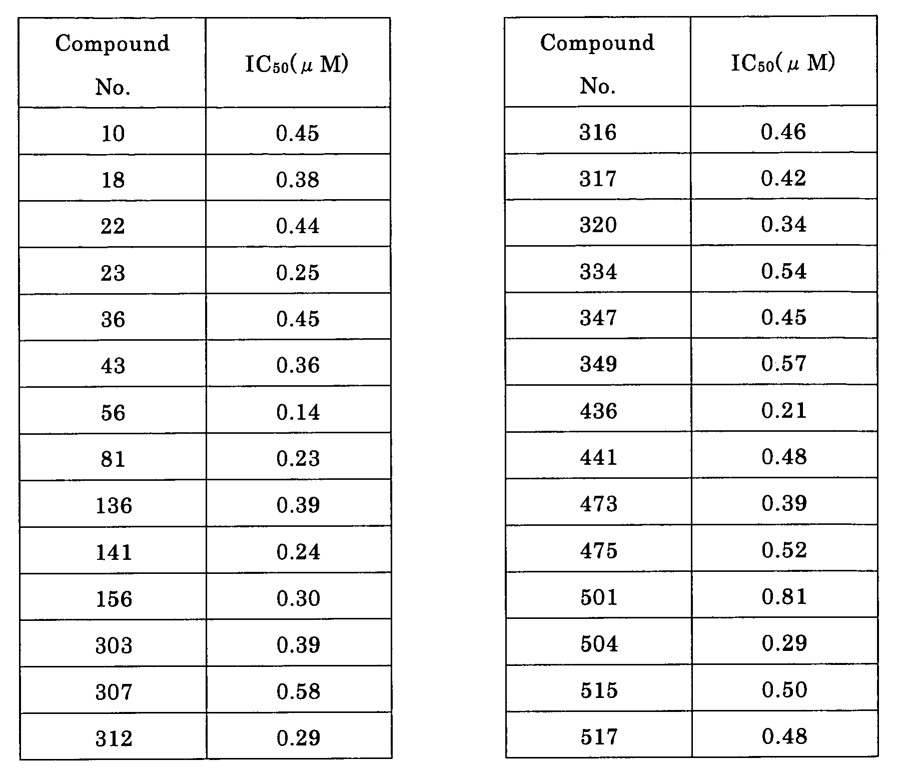

### Test Example 2: Inhibition of Complex Formation of PAI-1 and tPA

To a 2.5 *µ* L solution obtained by diluting a DMSO solution of the test compound at 4-fold concentration of the test concentration with PBS(-), 5 *µ* L of 150 nM recombinant human PAI-1 solution adjusted with a phosphate buffer (pH6.6, 0.05M Sodium Phosphate, 0.1 M NaCl, 1 mM EDTA) was added, and reacted for 5 minutes at 37°C. 2.5 *µ* L of 600 nM recombinant human one-chain tPA solution adjusted with PBS(-) was added, and further reacted for 5 minutes at 37°C. 10 *µ* L of stop buffer (1.25 mM Tris-HCl pH6.8, 4% SDS, 0.01% BPB, 14% Glycerol) was added, heated for 30 seconds at 100°C, and then the total amount of 20 *µ* L was applied to 10% SDS-PAGE gel electrophoresis. When the test compound was not added as being a control, a band of PAI-1-tPA complex was observed. Compounds of Nos. 10, 18, 22, 23, 36, 43, 56, 81, 136, 141, 156, 312, 316, 317, 320, 334, 347, 349, 352, 384, 401, 402, 423, 435, 436, 441, 473, 475, 503, 504, 515, and 517 completely inhibited the complex formation of PAI-1 and tPA observed with SDS-PAGE at the 100 *µ* M concentration.

### Test Example 3: Fibrin Clot Lysis Assay

To a 50 *µ*L solution obtained by diluting a DMSO solution of the test compound at 5-fold concentration of the test concentration with assay buffer (1/15M phosphate buffer, pH7.4, 0.01% Tween80), 50*µ* L of 25 nM recombinant human PAI-1 solution was added, and reacted for 10 minutes at room temperature. 25 *µ*L of 10 nM recombinant human two-chain tPA solution was added and reacted for 10 minutes at room temperature, and then 50 *µ*L of 5 *µ*M recombinant human fibrinogen was added, and reacted for 5 minutes at room temperature. 25 *µ* L of 0.1 *µ*M Glu-plasminogen was added and mixed, and incubated for 5 minutes at room temperature. Finally, 50 *µ*L of 25U/ml recombinant human a -thrombin was added, immediately transferred to a microplate reader (SPECTRAFLUOR or GENios) by TECAN warmed beforehand at 37°C, and absorbance at 405 nm was measured with passage of time. A time required to give an absorbance that is equal to an absorbance obtained by the test compound diluted only with the buffer was determined as a fibrin lysis time. As a result of measurements of the fibrin lysis time in the presence or absence of the test compound, compounds of Nos. 10, 23, 81, 141, 347, 349, 352, 423, 436, 441, 473, 475, and 536 gave 50% or more reduction of the fibrin lysis time at the concentration of 10 *µ* M or 20 *µ* M.

### Test Example 4: Tests in Rat Thrombosis Model

Catheters for shunt were inserted into the right carotid artery and the left carotid artery of a rat, blood was circulated for 30 minutes, and weights of thromboses adhered to threads placed inside the catheters were measured. The weight of the thrombus of the control group was 94.9±3.3 mg(n=4), whilst when the compound No. 10 was administered beforehand intraperitoneally at 30 mg/kg/day for continuous 8 days, the weight of thrombus formed was found to be 67.2±6.3 mg(n=4) and significant reduction of the weight as compared with the control was observed (p<0.01).

### Test Example 5: Tests in Mouse (murine) Heart Transplantation Model

In the major mismatch group allograft test where the heart of a Balb/c mouse is transplanted to a C57BL/6 mouse, the compound No. 10 was continuously administered intraperitoneally at 20 mg/kg/day, and as a result, significant prolongation of a graft survival was observed as compared with the control group [control group(n=12): 7.2±0.2 days; test compound- administered group(n=4): 9.0±0.7 days (p<0.05)]. Also in the class II mismatch group allograft test, a ratio of fibrous lesion 60 days after the transplantation was significantly lowered [control group (n=6): 44.5±2.7%; test compound-administered group(n=5): 18.8±2.2%(p<0.05)]. Vascular endothelial hyperplasia (a ratio of thickened endothelium to the internal elastic lamina) was significantly improved [control group; 64.5±5.8%; test compound-administered group: 21.7±4.9%(p<0.05)].

### Industrial Applicability

The medicaments of the present invention have inhibitory activity against plasminogen activator inhibitor-1 (PAI-1). Therefore, the medicaments of the present invention are useful for preventive and/or therapeutic treatment of diseases with stenosis or occlusion caused by thrombus; other diseases caused by an expression of PAI-1, an enhancement of PAI-1 activity, or a lowering of plasmin activity, for example, diseases caused by fibrogenesis, accumulation of visceral fat, cell proliferation, angiogenesis, deposition or remodeling of extracellular matrix, and cell movement and migration.

## Claims

1. A medicament having inhibitory activity against plasminogen activator inhibitor-1, which comprises as an active ingredient a substance selected from the group consisting of a compound represented by the following general formula (I) and a pharmacologically acceptable salt thereof, and a hydrate thereof and a solvate thereof: 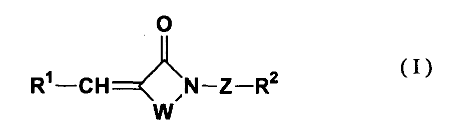 wherein R¹ represents an aromatic group which may be substituted,
R² represents an aromatic group which may be substituted,
W represents a group selected from the following connecting group W-1: 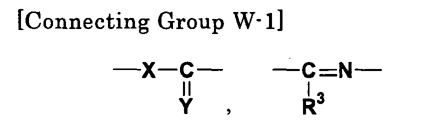 (wherein a bond at the left end binds to the carbon atom and a bond at the right end binds to the nitrogen atom,
X represents sulfur atom or NH,
Y represents oxygen atom or sulfur atom,
R³ represents a hydrocarbon group which may be substituted, hydroxy group which may be substituted, or carboxy group which may be esterified),
Z represents a single bond or a connecting group wherein a number of atoms in a main chain is 1 to 3 (said connecting group may be substituted).

2. The medicament according to claim 1, wherein R¹ is an aromatic group which may be substituted,
R² is an aromatic group which may be substituted,
W is a group represented by the following formula: 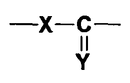 (wherein a bond at the left end binds to the carbon atom and a bond at the right end binds to the nitrogen atom,
X represents sulfur atom or NH,
Y represents oxygen atom or sulfur atom),
Z is a single bond, methylene group, ethylene group, -CH₂CO- group, or -CH₂CONH-group.

3. The medicament according to claim 2, wherein R¹ is an aromatic group which may be substituted,
R² is an aromatic group which may be substituted,
X is sulfur atom,
Y is oxygen atom or sulfur atom,
Z is methylene group.

4. The medicament according to claim 3, wherein R¹ is a phenyl group which is substituted with one to three hydroxy groups (said phenyl group may further be substituted with one or more substituents other than the hydroxy group(s)),
R² is an aromatic group which may be substituted,
X is sulfur atom,
Y is oxygen atom or sulfur atom,
Z is methylene group.

5. The medicament according to claim 4, wherein R¹ is a phenyl group which is substituted with two or three hydroxy groups (said phenyl group may further be substituted with one or more substituents other than the hydroxy group(s)),
R² is a phenyl group which may be substituted,
X is sulfur atom,
Y is oxygen atom or sulfur atom,
Z is methylene group.

6. The medicament according to claim 5, wherein the compound represented by the general formula (I) is a compound selected from the following 14 compounds:
• the compound wherein R¹ is 3,4,5-trihydroxyphenyl group, R² is 3,4-dichlorophenyl group, X is sulfur atom, Y is oxygen atom, Z is methylene group;
• the compound wherein R¹ is 2,3-dihydroxyphenyl group, R² is 2,3-dichlorophenyl group, X is sulfur atom, Y is oxygen atom, Z is methylene group;
• the compound wherein R¹ is 2,3-dihydroxyphenyl group, R² is 2,4-dichlorophenyl group, X is sulfur atom, Y is oxygen atom, Z is methylene group;
• the compound wherein R¹ is 3,4-dihydroxyphenyl group, R² is 2,4-dichlorophenyl group, X is sulfur atom, Y is oxygen atom, Z is methylene group;
• the compound wherein R¹ is 2,3-dihydroxyphenyl group, R² is 4-bromophenyl group, X is sulfur atom, Y is oxygen atom, Z is methylene group;
• the compound wherein R¹ is 2,3-dihydroxyphenyl group, R² is 4-(trifluoromethyl)phenyl group, X is sulfur atom, Y is oxygen atom, Z is methylene group;
• the compound wherein R¹ is 2,3-dihydroxyphenyl group, R² is 4-styrylphenyl group, X is sulfur atom, Y is oxygen atom, Z is methylene group;
• the compound wherein R¹ is 3,4-dihydroxyphenyl group, R² is 3,4-dichlorophenyl group, X is sulfur atom, Y is sulfur atom, Z is methylene group;
• the compound wherein R¹ is 2,3-dihydroxyphenyl group, R² is 4-[(3-phenoxyphenyl)acetylamino]phenyl group, X is sulfur atom, Y is oxygen atom, Z is methylene group;
• the compound wherein R¹ is 2,3-dihydroxyphenyl group, R² is 4-(4-phenoxybenzoylamino)phenyl group, X is sulfur atom, Y is oxygen atom, Z is methylene group;
• the compound wherein R¹ is 2,3-dihydroxyphenyl group, R² is 4-[3,5-bis(trifluoromethyl)phenylacetylamino]phenyl group, X is sulfur atom, Y is oxygen atom, Z is methylene group;
• the compound wherein R¹ is 2,3-dihydroxyphenyl group, R² is 3-[(3-phenoxyphenyl)acetylamino]phenyl group, X is sulfur atom, Y is oxygen atom, Z is methylene group;
• the compound wherein R¹ is 3,4-dihydroxy-5-nitrophenyl group, R² is 3,4-dichlorophenyl group, X is sulfur atom, Y is sulfur atom, Z is methylene group; and
• the compound wherein R¹ is 3,4-dihydroxy-5-[3-(trifluoromethoxy)-phenylcarbamoyl]phenyl group, R² is 3,4-dichlorophenyl group, X is sulfur atom, Y is sulfur atom, Z is methylene group.

7. The medicament according to claim 3, wherein R¹ is a phenyl group which is substituted with a C₆-C₁₀ aryl-substituted carbamoyl group (said aryl group may be substituted, and said phenyl group may further be substituted with one or more substituents other than the C₆-C₁₀ aryl-substituted carbamoyl group), or a phenyl group which is substituted with a heteroaryl-substituted carbamoyl group (said heteroaryl group may be substituted, and said phenyl group may further be substituted with one or more substituents other than the heteroaryl-substituted carbamoyl group),
R² is an aromatic group which may be substituted,
X is sulfur atom,
Y is oxygen atom or sulfur atom,
Z is methylene group.

8. The medicament according to claim 7, wherein R¹ is a phenyl group which is substituted with a C₆-C₁₀ aryl-substituted carbamoyl group in the 3-position (said aryl group may be substituted, and said phenyl group may further be substituted with one or more substituents other than the C₆-C₁₀ aryl-substituted carbamoyl group) or a phenyl group which is substituted with a heteroaryl-substituted carbamoyl group (said heteroaryl group may be substituted, and said phenyl group may further be substituted with one or more substituents other than the heteroaryl-substituted carbamoyl group), and a phenyl group which is substituted with hydroxy group in the 4-position (said phenyl group may be substituted in the 5-position),
R² is a phenyl group which may be substituted,
X is sulfur atom,
Y is oxygen atom,
Z is methylene group.

9. The medicament according to claim 8, wherein the compound represented by the general formula (I) is a compound selected from the following 11 compounds:
• the compound wherein R¹ is 3-methoxy-4-hydroxy-5-(3,4-dihydroxyphenylcarbamoyl)phenyl group, R² is 3,4-dichlorophenyl group, X is sulfur atom, Y is oxygen atom, Z is methylene group;
• the compound wherein R¹ is 4-hydroxy-3-(3,4-dihydroxyphenylcarbamoyl)phenyl group, R² is 3,4-dichlorophenyl group, X is sulfur atom, Y is oxygen atom, Z is methylene group;
• the compound wherein R¹ is 4-hydroxy-3-(3,4-dihydroxyphenylcarbamoyl)phenyl group, R² is 3,5-bis(trifluoromethyl)phenyl group, X is sulfur atom, Y is oxygen atom, Z is methylene group ;
• the compound wherein R¹ is 3-methyl-4-hydroxy-5-(3,4-dihydroxyphenylcarbamoyl)phenyl group, R² is 3,4-dichlorophenyl group, X is sulfur atom, Y is oxygen atom, Z is methylene group;
• the compound wherein R¹ is 4-hydroxy-3-(3,4-dihydroxyphenylcarbamoyl)phenyl group, R² is 4-(trifluoromethyl)phenyl group, X is sulfur atom, Y is oxygen atom, Z is methylene group;
• the compound wherein R¹ is 4-hydroxy-3-[2-chloro-5-(trifluoromethyl)phenylcarbamoyl]phenyl group, R² is 3,4-dichlorophenyl group, X is sulfur atom, Y is oxygen atom, Z is methylene group;
• the compound wherein R¹ is 4-hydroxy-3-(3,4,5-trihydroxyphenylcarbamoyl)phenyl group, R² is 3,4-dichlorophenyl group, X is sulfur atom, Y is oxygen atom, Z is methylene group;
• the compound wherein R¹ is 4-hydroxy-3-(2,3-dihydroxyphenylcarbamoyl)phenyl group, R² is 3,4-dichlorophenyl group, X is sulfur atom, Y is oxygen atom, Z is methylene group;
• the compound wherein R¹ is 4-hydroxy-3-(2,5-dichlorophenylcarbamoyl)phenyl group, R² is 3,4-dichlorophenyl group, X is sulfur atom, Y is oxygen atom, Z is methylene group;
• the compound wherein R¹ is 4-hydroxy-3-(2-chloro-5-nitrophenylcarbamoyl)phenyl group, R² is 3,4-dichlorophenyl group, X is sulfur atom, Y is oxygen atom, Z is methylene group; and
• the compound wherein R¹ is 3,4-dihydroxy-5-[3-(trifluoromethyl)-phenylcarbamoyl]phenyl group, R² is 3,4-dichlorophenyl group, X is sulfur atom, Y is oxygen atom, Z is methylene group.

10. The medicament according to claim 3, wherein R¹ and R² are either the following (i) or (ii),
X is sulfur atom,
Y is oxygen atom or sulfur atom,
Z is methylene group:
(i) R¹ is a phenyl group which is substituted with carboxy group (said phenyl group may further be substituted with one or more substituents other than the carboxy group), R² is an aromatic group which may be substituted;
(ii) R¹ is an aromatic group which may be substituted,
R² is a phenyl group which is substituted with carboxy group (said phenyl group may further be substituted with one or more substituents other than the carboxy group).

11. The medicament according to claim 10, wherein the compound represented by the general formula (I) is a compound selected from the following 2 compounds:
• the compound wherein R¹ is 3-carboxyphenyl group, R² is 3,4-dichlorophenyl group, X is sulfur atom, Y is oxygen atom, Z is methylene group; and
• the compound wherein R¹ is 3-carboxy-4-hydroxyphenyl group, R² is 4-methoxycarbonylphenyl group, X is sulfur atom, Y is oxygen atom, Z is methylene group.

12. The medicament according to claim 2, wherein the compound represented by the general formula (I) is a compound selected from the group consisting of the compounds described in the specification as Compound Nos. 1 to 93 and Compound Nos. 301 to 475.

13. The medicament according to claim 1, wherein R¹ is an aromatic group which may be substituted,
R² is an aromatic group which may be substituted,
W is a group represented by the following formula: 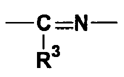 (wherein a bond at the left end binds to the carbon atom and a bond at the right end binds to the nitrogen atom,
R³ represents a hydrocarbon group which may be substituted, hydroxy group which may be substituted, or carboxy group which may be esterified),
Z is a single bond or methylene group.

14. The medicament according to claim 13, wherein R¹ is an aromatic group which may be substituted,
R² is an aromatic group which may be substituted,
R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
Z is a single bond.

15. The medicament according to claim 14, wherein R¹ is a phenyl group which is substituted with one to three hydroxy groups (said phenyl group may further be substituted with one or more substituents other than the hydroxy group),
R² is an aromatic group which may be substituted,
R³ is a C₁-C₆ alkyl group, a C₁-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
Z is a single bond.

16. The medicament according to claim 15, wherein R¹ is a phenyl group which is substituted with two or three hydroxy groups (said phenyl group may further be substituted with one or more substituents other than the hydroxy group),
R² is a phenyl group which may be substituted,
R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
Z is a single bond.

17. The medicament according to claim 16, wherein the compound represented by the general formula (I) is a compound selected from the following 6 compounds:
• the compound wherein R¹ is 2,4,5-trihydroxyphenyl group, R² is 3,5-dichlorophenyl group, R³ is phenyl group, Z is a single bond;
• the compound wherein R¹ is 2,4,5-trihydroxyphenyl group, R² is 3,4-dichlorophenyl group, R³ is isopropyl group, Z is a single bond;
• the compound wherein R¹ is 2,4,5-trihydroxyphenyl group, R² is 4-nitrophenyl group, R³ is ethoxy group, Z is a single bond;
• the compound wherein R¹ is 2,4,5-trihydroxyphenyl group, R² is 3,4-dichlorophenyl group, R³ is methyl group, Z is a single bond;
• the compound wherein R¹ is 2,4,5-trihydroxyphenyl group, R² is 3,5-dichlorophenyl group, R³ is isopropyl group, Z is a single bond; and
• the compound wherein R¹ is 2,4,5-trihydroxyphenyl group, R² is 3-chloro-4-fluorophenyl group, R³ is isopropyl group, Z is a single bond.

18. The medicament according to claim 14, wherein R¹ is a phenyl group which is substituted with C₂-C₆ alkenyl group which may be substituted (said phenyl group may further be substituted with one or more substituents other than the C₂-C₆ alkenyl group),
R² is an aromatic group which may be substituted,
R³ is a C₁-C₆ alkyl group, a C₆-C₁₀ aryl group which may be substituted, a C₁-C₆ halogenated alkyl group, a C₂-C₇ alkoxycarbonyl-substituted C₁- C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
Z is a single bond.

19. The medicament according to claim 18, wherein the compound represented by the general formula (I) is a compound selected from the following 2 compounds:
• the compound wherein R¹ is 2-hydroxy-5-styrylphenyl group, R² is 4-nitrophenyl group, R³ is ethoxy group, Z is a single bond; and
• the compound wherein R¹ is 4-styrylphenyl group, R² is 4-carboxyphenyl group, R³ is isopropyl group, Z is a single bond.

20. The medicament according to claim 14, wherein R¹, R² and R³ are any one of the following (i) to (iii), Z is a single bond:
(i) R¹ is a phenyl group which is substituted with carboxy group (said phenyl group may further be substituted with one or more substituents other than the carboxy group), R² is an aromatic group which may be substituted,
R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group;
(ii) R¹ is an aromatic group which may be substituted,
R² is a phenyl group which is substituted with carboxy group (said phenyl group may further be substituted with one or more substituents other than the carboxy group), R³ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group;
(iii) R¹ is an aromatic group which may be substituted,
R² is an aromatic group which may be substituted,
R³ is carboxy group.

21. The medicament according to claim 20, wherein the compound represented by the general formula (I) is a following compound:
• the compound wherein R¹ is 4-styrylphenyl group, R² is 4-carboxyphenyl group, R³ is isopropyl group, Z is a single bond.

22. The medicament according to claim 13, wherein the compound represented by the general formula (I) is a compound selected from the group consisting of the compounds described in the specification as Compound Nos. 101 to 224 and Compound Nos. 501 to 567.

23. The medicament according to any one of claims 1 to 22, for therapeutic and/or preventive treatment of diseases caused by an expression of PAI-1 or an enhancement of PAI-1 activity.

24. The medicament according to any one of claims 1 to 22, for preventive and/or therapeutic treatment of a disease caused by one or more abnormal conditions selected from the group consisting of thrombogenesis, fibrogenesis, accumulation of visceral fat, cell proliferation, angiogenesis, deposition or remodeling of extracellular matrix, and cell movement or migration.

25. A compound represented by the general formula (II) or a salt thereof, or a hydrate thereof or a solvate thereof: 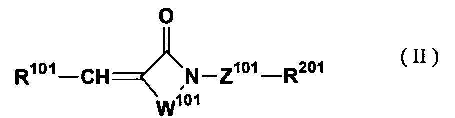 wherein R¹⁰¹ represents an aromatic group which may be substituted,
R²⁰¹ represents an aromatic group which may be substituted,
W¹⁰¹ represents a group selected from the following connecting group W-101-1: 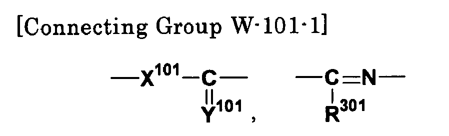 (wherein a bond at the left end binds to the carbon atom and a bond at the right end binds to the nitrogen atom,
X¹⁰¹ represents sulfur atom or NH,
Y¹⁰¹ represents oxygen atom or sulfur atom,
R³⁰¹ represents a hydrocarbon group which may be substituted, hydroxy group which may be substituted, or carboxy group which may be esterified),
Z¹⁰¹ represents a single bond or a connecting group wherein a number of atoms in a main chain is 1 to 3 (said connecting group may be substituted), provided that the following compounds are excluded:
• the compound wherein R¹⁰¹ is 3,5-dibromo-2-hydroxyphenyl group, R²⁰¹ is phenyl group, W¹⁰¹ is -X¹⁰¹-C(=Y¹⁰¹)-, X¹⁰¹ is sulfur atom, Y¹⁰¹ is oxygen atom, Z¹⁰¹ is a single bond;
• the compound wherein R¹⁰¹ is 5-(3-carboxyphenyl)furan-2-yl group, R²⁰¹ is 3-fluorophenyl group, W¹⁰¹ is -X¹⁰¹-C(=Y¹⁰¹)-, X¹⁰¹ is sulfur atom, Y¹⁰¹ is sulfur atom, Z¹⁰¹ is a single bond;
• the compound wherein R¹⁰¹ is 3,5-dibromo-2-hydroxyphenyl group, R²⁰¹ is 4-chlorophenyl group, W¹⁰¹ is X¹⁰¹-C(=Y¹⁰¹)-, X¹⁰¹ is NH, Y¹⁰¹ is sulfur atom, Z¹⁰¹ is a single bond;
• the compound wherein R¹⁰¹ is 5-(3-carboxyphenyl)furan-2-yl group, R²⁰¹ is 4-methoxyphenyl group, W¹⁰¹ is -X¹⁰¹-C(=Y¹⁰¹)-, X¹⁰¹ is sulfur atom, Y¹⁰¹ is sulfur atom, Z¹⁰¹ is methylene group;
• the compound wherein R¹⁰¹ is 5-chloro-2-hydroxyphenyl group, R²⁰¹ is phenyl group, W¹⁰¹ is -X¹⁰¹⁻C(=Y¹⁰¹)-, X¹⁰¹ is sulfur atom, Y¹⁰¹ is sulfur atom, Z¹⁰¹ is a single bond;
• the compound wherein R¹⁰¹ is 3,5-dibromo-2-hydroxyphenyl group, R²⁰¹ is phenyl group, W¹⁰¹ is -C(R³⁰¹)=N-, R³⁰¹ is hydroxy group, Z¹⁰¹ is a single bond;
• the compound wherein R¹⁰¹ is 5-bromo-2-hydroxyphenyl group, R²⁰¹ is 3,4-dichlorophenyl group, W¹⁰¹ is -C(R³⁰¹)=N-, R³⁰¹ is methyl group, Z¹⁰¹ is a single bond;
• the compound wherein R¹⁰¹ is 3-ethoxy-4-{[(thiophen-2-yl)carbonyl]oxy}phenyl group, R²⁰¹ is 3-carboxyphenyl group, W¹⁰¹ is -C(R³⁰¹)=N-, R³⁰¹ is methyl group, Z¹⁰¹ is a single bond;
• the compound wherein R¹⁰¹ is 4-carboxymethoxy-3-ethoxyphenyl group, R²⁰¹ is 3-(trifluoromethyl)phenyl group, W¹⁰¹ is -C(R³⁰¹)=N-, R³⁰¹ is methyl group, Z¹⁰¹ is a single bond;
• the compound wherein R¹⁰¹ is 5-[3-(methoxycarbonyl)phenyl]furan-2-yl group, R²⁰¹ is 4-fluorophenyl group, W¹⁰¹ is -C(R³⁰¹)=N-, R³⁰¹ is hydroxy group, Z¹⁰¹ is a single bond;
• the compound wherein R¹⁰¹ is 5-(4-carboxyphenyl)furan-2-yl group, R²⁰¹ is 4-chlorophenyl group, W¹⁰¹ is -C(R³⁰¹)=N-, R³⁰¹ is hydroxy group, Z¹⁰¹ is a single bond;
• the compound wherein R¹⁰¹ is 5-(3,5-dichlorophenyl)furan-2-yl group, R²⁰¹ is 3-carboxyphenyl group, W¹⁰¹ is -C(R³⁰¹)=N-, R³⁰¹ is trifluoromethyl group, Z¹⁰¹ is a single bond;
• the compound wherein R¹⁰¹ is 5-(5-carboxy-2-chlorophenyl)furan-2-yl group, R²⁰¹ is phenyl group, W¹⁰¹ is -C(R³⁰¹)=N-, R³⁰¹ is methyl group, Z¹⁰¹ is a single bond;
• the compound wherein R¹⁰¹ is 4-bromophenyl group, R²⁰¹ is 3-carboxy-4-chlorophenyl group, W¹⁰¹ is -C(R³⁰¹)=N-, R³⁰¹ is trifluoromethyl group, Z¹⁰¹ is a single bond;
• the compound wherein R¹⁰¹ is 5-(2-hydroxy-5-nitrophenyl)furan-2-yl group, R²⁰¹ is 3-chlorophenyl group, W¹⁰¹ is -C(R³⁰¹)=N-, R³⁰¹ is hydroxy group, Z¹⁰¹ is a single bond;
• the compound wherein R¹⁰¹ is 4-bromophenyl group, R²⁰¹ is 3-carboxy-4-chlorophenyl group, W¹⁰¹ is -C(R³⁰¹)=N-, R³⁰¹ is methyl group, Z¹⁰¹ is a single bond;
• the compound wherein R¹⁰¹ is 4-allyloxy-3-methoxyphenyl group, R²⁰¹ is 3-carboxy-4-chlorophenyl group, W¹⁰¹ is -C(R³⁰¹)=N-, R³⁰¹ is methyl group, Z¹⁰¹ is a single bond;
• the compound wherein R¹⁰¹ is 4-benzyloxy-3-methoxyphenyl group, R²⁰¹ is 3-carboxy-4-chlorophenyl group, W¹⁰¹ is -C(R³⁰¹)=N-, R³⁰¹ is methyl group, Z¹⁰¹ is a single bond;
• the compound wherein R¹⁰¹ is 4-chlorophenyl group, R²⁰¹ is phenyl group, W¹⁰¹ is -C(R³⁰¹)=N-, R³⁰¹ is carboxy group, Z¹⁰¹ is a single bond;
• the compound wherein R¹⁰¹ is phenyl group, R²⁰¹ is 3-carboxyphenyl group, W¹⁰¹ is -C(R³⁰¹)=N-, R³⁰¹ is methyl group, Z¹⁰¹ is a single bond;
• the compound wherein R¹⁰¹ is 2-benzyloxy-5-bromophenyl group, R²⁰¹ is 3-carboxyphenyl group, W¹⁰¹ is -C(R³⁰¹)=N-, R³⁰¹ is methyl group, Z¹⁰¹ is a single bond;
• the compound wherein R¹⁰¹ is 4-(benzylcarbamoyl)methoxy-3-bromo-5-methoxyphenyl group, R²⁰¹ is 3-carboxyphenyl group, W¹⁰¹ is -C(R³⁰¹)=N-, R³⁰¹ is methyl group, Z¹⁰¹ is a single bond;
• the compound wherein R¹⁰¹ is phenyl group, R²⁰¹ is 3-carboxyphenyl group, W¹⁰¹ is -C(R³⁰¹)=N-, R³⁰¹ is phenyl group, Z¹⁰¹ is a single bond;
• the compound wherein R¹⁰¹ is 5-(3-chlorophenyl)furan-2-yl group, R²⁰¹ is 4-sulfamoylphenyl group, W¹⁰¹ is -C(R³⁰¹)=N-, R³⁰¹ is methyl group, Z¹⁰¹ is a single bond;
• the compound wherein R¹⁰¹ is 5-(3-chloro-4-methylphenyl)furan-2-yl group, R²⁰¹ is 3-carboxyphenyl group, W¹⁰¹ is -C(R³⁰¹)=N-, R³⁰¹ is methyl group, Z¹⁰¹ is a single bond;
• the compound wherein R¹⁰¹ is 5-(2-chlorophenyl)furan-2-yl group, R²⁰¹ is 3-carboxy-4-chlorophenyl group, W¹⁰¹ is -C(R³⁰¹)=N-, R³⁰¹ is methyl group, Z¹⁰¹ is a single bond;
• the compound wherein R¹⁰¹ is 5-[3-(methoxycarbonyl)phenyl]furan-2-yl group, R²⁰¹ is 3-carboxyphenyl group, W¹⁰¹ is -C(R³⁰¹)=N-, R³⁰¹ is methyl group, Z¹⁰¹ is a single bond;
• the compound wherein R¹⁰¹ is 5-(3-chloro-2-methylphenyl)furan-2-yl group, R²⁰¹ is 3-carboxy-4-chlorophenyl group, W¹⁰¹ is -C(R³⁰¹)=N-, R³⁰¹ is methyl group, Z¹⁰¹ is a single bond;
• the compound wherein R¹⁰¹ is 5-(3,5-dichlorophenyl)furan-2-yl group, R²⁰¹ is 3-carboxyphenyl group, W¹⁰¹ is -C(R³⁰¹)=N-, R³⁰¹ is methyl group, Z¹⁰¹ is a single bond;
• the compound wherein R¹⁰¹ is 5-(3-fluorophenyl)furan-2-yl group, R²⁰¹ is 3-carboxy-4-chlorophenyl group, W¹⁰¹ is -C(R³⁰¹)=N-, R³⁰¹ is methyl group, Z¹⁰¹ is a single bond;
• the compound wherein R¹⁰¹ is 5-(3-nitrophenyl)furan-2-yl group, R²⁰¹ is 3-carboxyphenyl group, W¹⁰¹ is -C(R³⁰¹)=N-, R³⁰¹ is methyl group, Z¹⁰¹ is a single bond; and
• the compound wherein R¹⁰¹ is 5-(5-carboxy-2-chlorophenyl)furan-2-yl group, R²⁰¹ is 3-(trifluoromethyl)phenyl group, W¹⁰¹ is -C(R³⁰¹)=N-, R³⁰¹ is hydroxy group, Z¹⁰¹ is a single bond.

26. The compound according to claim 25 or a salt thereof, or a hydrate thereof or a solvate thereof, wherein R¹⁰¹ is a phenyl group which is substituted with one to three hydroxy groups (said phenyl group may further be substituted with one or more substituents other than the hydroxy group),
R²⁰¹ is an aromatic group which may be substituted,
W¹⁰¹ is a group represented by the following formula: 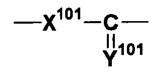 (wherein a bond at the left end binds to the carbon atom and a bond at the right end binds to the nitrogen atom,
X¹⁰¹ represents sulfur atom,
Y¹⁰¹ represents oxygen atom or sulfur atom),
Z¹⁰¹ is methylene group.

27. The compound according to claim 26 or a salt thereof, or a hydrate thereof or a solvate thereof, wherein R¹⁰¹ is a phenyl group which is substituted with two or three hydroxy groups (said phenyl group may further be substituted with one or more substituents other than the hydroxy group),
R²⁰¹ is a phenyl group which may be substituted,
X¹⁰¹ is sulfur atom,
Y¹⁰¹ is oxygen atom or sulfur atom),
Z¹⁰¹ is methylene group.

28. The compound according to claim 27 or a salt thereof, or a hydrate thereof or a solvate thereof, wherein the compound represented by the general formula (II) is a compound selected from the following 14 compounds:
• the compound wherein R¹⁰¹ is 3,4,5-trihydroxyphenyl group, R²⁰¹ is 3,4-dichlorophenyl group, X¹⁰¹ is sulfur atom, Y¹⁰¹ is oxygen atom, Z¹⁰¹ is methylene group;
• the compound wherein R¹⁰¹ is 2,3-dihydroxyphenyl group, R²⁰¹ is 2,3-dichlorophenyl group, X¹⁰¹ is sulfur atom, Y¹⁰¹ is oxygen atom, Z¹⁰¹ is methylene group;
• the compound wherein R¹⁰¹ is 2,3-dihydroxyphenyl group, R²⁰¹ is 2,4-dichlorophenyl group, X¹⁰¹ is sulfur atom, Y¹⁰¹ is oxygen atom, Z¹⁰¹ is methylene group;
• the compound wherein R¹⁰¹ is 3,4-dihydroxyphenyl group, R²⁰¹ is 2,4-dichlorophenyl group, X¹⁰¹ is sulfur atom, Y¹⁰¹ is oxygen atom, Z¹⁰¹ is methylene group;
• the compound wherein R¹⁰¹ is 2,3-dihydroxyphenyl group, R²⁰¹ is 4-bromophenyl group, X¹⁰¹ is sulfur atom, Y¹⁰¹ is oxygen atom, Z¹⁰¹ is methylene group;
• the compound wherein R¹⁰¹ is 2,3-dihydroxyphenyl group, R²⁰¹ is 4-(trifluoromethyl)phenyl group, X¹⁰¹ is sulfur atom, Y¹⁰¹ is oxygen atom, Z¹⁰¹ is methylene group;
• the compound wherein R¹⁰¹ is 2,3-dihydroxyphenyl group, R²⁰¹ is 4-styrylphenyl group, X¹⁰¹ is sulfur atom, Y¹⁰¹ is oxygen atom, Z¹⁰¹ is methylene group;
• the compound wherein R¹⁰¹ is 3,4-dihydroxyphenyl group, R²⁰¹ is 3,4-dichlorophenyl group, X¹⁰¹ is sulfur atom, Y¹⁰¹ is sulfur atom, Z¹⁰¹ is methylene group;
• the compound wherein R¹⁰¹ is 2,3-dihydroxyphenyl group, R²⁰¹ is 4-[(3-phenoxyphenyl)acetylamino]phenyl group, X¹⁰¹ is sulfur atom, Y¹⁰¹ is oxygen atom, Z¹⁰¹ is methylene group;
• the compound wherein R¹⁰¹ is 2,3-dihydroxyphenyl group, R²⁰¹ is 4-(4-phenoxybenzoylamino)phenyl group, X¹⁰¹ is sulfur atom, Y¹⁰¹ is oxygen atom, Z¹⁰¹ is methylene group;
• the compound wherein R¹⁰¹ is 2,3-dihydroxyphenyl group, R²⁰¹ is 4-[3,5-bis(trifluoromethyl)phenylacetylamino]phenyl group, X¹⁰¹ is sulfur atom, Y¹⁰¹ is oxygen atom, Z¹⁰¹ is methylene group;
• the compound wherein R¹⁰¹ is 2,3-dihydroxyphenyl group, R²⁰¹ is 3-[(3-phenoxyphenyl)acetylamino]phenyl group, X¹⁰¹ is sulfur atom, Y¹⁰¹ is oxygen atom, Z¹⁰¹ is methylene group;
• the compound wherein R¹⁰¹ is 3,4-dihydroxy-5-nitrophenyl group, R²⁰¹ is 3,4-dichlorophenyl group, X¹⁰¹ is sulfur atom, Y¹⁰¹ is sulfur atom, Z¹⁰¹ is methylene group; and
• the compound wherein R¹⁰¹ is 3,4-dihydroxy-5-[3-(trifluoromethoxy)phenylcarbamoyl]phenyl group, R²⁰¹ is 3,4-dichlorophenyl group, X¹⁰¹ is sulfur atom, Y¹⁰¹ is sulfur atom, Z¹⁰¹ is methylene group.

29. The compound according to claim 25 or a salt thereof, or a hydrate thereof or a solvate thereof, wherein R¹⁰¹ is a phenyl group which is substituted with a C₆-C₁₀ aryl-substituted carbamoyl group (said aryl group may be substituted, and said phenyl group may further be substituted with one or more substituents other than the C₆-C₁₀ aryl-substituted carbamoyl group), or a phenyl group which is substituted with a heteroaryl-substituted carbamoyl group (said heteroaryl group may be substituted, and said phenyl group may further be substituted with one or more substituents other than the heteroaryl-substituted carbamoyl group),
R²⁰¹ is an aromatic group which may be substituted,
W¹⁰¹ is a group represented by the following formula: 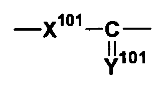 (wherein a bond at the left end binds to the carbon atom and a bond at the right end binds to the nitrogen atom,
X¹⁰¹ represents sulfur atom,
Y¹⁰¹ represents oxygen atom or sulfur atom),
Z¹⁰¹ is methylene group.

30. The compound according to claim 29 or a salt thereof, or a hydrate thereof or a solvate thereof, wherein R¹ is a phenyl group which is substituted with a C₆-C₁₀ aryl-substituted carbamoyl group in the 3-position (said aryl group may be substituted, and said phenyl group may further be substituted with one or more substituents other than the C₆-C₁₀ aryl-substituted carbamoyl group) or a phenyl group which is substituted with a heteroaryl-substituted carbamoyl group (said heteroaryl group may be substituted, and said phenyl group may further be substituted with one or more substituents other than the heteroaryl-substituted carbamoyl group), and a phenyl group which is substituted with hydroxy group in the 4-position (said phenyl group may be substituted in the 5-position),
R²⁰¹ is a phenyl group which may be substituted,
X¹⁰¹ is sulfur atom,
Y¹⁰¹ is oxygen atom,
Z¹⁰¹ is methylene group.

31. The compound according to claim 30 or a salt thereof, or a hydrate thereof or a solvate thereof, wherein the compound represented by the general formula (II) is a compound selected from the following 11 compounds:
• the compound wherein R¹⁰¹ is 3-methoxy-4-hydroxy-5-(3,4-dihydroxyphenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
• the compound wherein R¹⁰¹ is 4-hydroxy-3-(3,4-dihydroxyphenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
• the compound wherein R¹⁰¹ is 4-hydroxy-3-(3,4-dihydroxyphenylcarbamoyl)phenyl group, R²⁰¹ is 3,5-bis(trifluoromethyl)phenyl group, X¹⁰¹ is sulfur atom, Y¹⁰¹ is oxygen atom, Z¹⁰¹ is methylene group;
• the compound wherein R¹⁰¹ is 3-methyl-4-hydroxy-5-(3,4-dihydroxyphenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
• the compound wherein R¹⁰¹ is 4-hydroxy-3-(3,4-dihydroxyphenylcarbamoyl)phenyl group, R²⁰¹ is 4-(trifluoromethyl)phenyl group, X¹⁰¹ is sulfur atom, Y¹⁰¹ is oxygen atom, Z¹⁰¹ is methylene group;
• the compound wherein R¹⁰¹ is 4-hydroxy-3-[2-chloro-5-(trifluoromethyl)phenylcarbamoyl]phenyl group, R²⁰¹ is 3,4-dichlorophenyl group, X¹⁰¹ is sulfur atom, Y¹⁰¹ is oxygen atom, Z¹⁰¹ is methylene group;
• the compound wherein R¹⁰¹ is 4-hydroxy-3-(3,4,5-trihydroxyphenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
• the compound wherein R¹⁰¹ is 4-hydroxy-3-(2,3-dihydroxyphenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
• the compound wherein R¹⁰¹ is 4-hydroxy-3-(2,5-dichlorophenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group;
• the compound wherein R¹⁰¹ is 4-hydroxy-3-(2-chloro-5-nitrophenylcarbamoyl)phenyl group, R²⁰¹ is 3,4-dichlorophenyl group, X¹⁰¹ is sulfur atom, Y¹⁰¹ is oxygen atom, Z¹⁰¹ is methylene group; and
• the compound wherein R¹⁰¹ is 3,4-dihydroxy-5-[3-(trifluoromethyl)phenylcarbamoyl]phenyl group, R²⁰¹ is 3,4-dichlorophenyl group, X¹⁰¹ is sulfur atom, Y¹⁰¹ is oxygen atom, Z¹⁰¹ is methylene group.

32. The compound according to claim 25 or a salt thereof, or a hydrate thereof or a solvate thereof, wherein R¹⁰¹ and R²⁰¹ are either the following (i) or (ii),
X¹⁰¹ is sulfur atom,
Y¹⁰¹ is oxygen atom or sulfur atom,
Z¹⁰¹ is methylene group: 1
(i) R¹⁰¹ is a phenyl group which is substituted with carboxy group (said phenyl group may further be substituted with one or more substituents other than the carboxy group), R²⁰¹ is an aromatic group which may be substituted;
(ii) R¹⁰¹ is an aromatic group which may be substituted,
R²⁰¹ is a phenyl group which is substituted with carboxy group (said phenyl group may further be substituted with one or more substituents other than the carboxy group).

33. The compound according to claim 32 or a salt thereof, or a hydrate thereof or a solvate thereof, wherein the compound represented by the general formula (II) is a compound selected from the following 2 compounds:
• the compound wherein R¹⁰¹ is 3-carboxyphenyl group, R²⁰¹ is 3,4-dichlorophenyl group, X¹⁰¹ is sulfur atom, Y¹⁰¹ is oxygen atom, Z¹⁰¹ is methylene group; and
• the compound wherein R¹⁰¹ is 3-carboxy-4-hydroxyphenyl group, R²⁰¹ is 4-methoxycarbonylphenyl group, X¹⁰¹ is sulfur atom, Y¹⁰¹ is oxygen atom, Z¹⁰¹ is methylene group.

34. The compound according to claim 25 or a salt thereof, or a hydrate thereof or a solvate thereof, wherein the compound represented by the general formula (II) is a compound selected from the group consisting of the compounds described in the specification as Compound Nos. 2 to 61, Compound Nos. 66 to 93, and Compound Nos. 301 to 475.

35. The compound according to claim 25 or a salt thereof, or a hydrate thereof or a solvate thereof, wherein R¹⁰¹ is a phenyl group which is substituted with one to three hydroxy groups (said phenyl group may further be substituted with one or more substituents other than the hydroxy group),
R²⁰¹ is an aromatic group which may be substituted,
W¹⁰¹ is a group represented by the following formula: 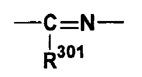 (wherein a bond at the left end binds to the carbon atom and a bond at the right end binds to the nitrogen atom,
R³⁰¹ represents a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group),
Z¹⁰¹ is a single bond.

36. The compound according to claim 35 or a salt thereof, or a hydrate thereof or a solvate thereof, wherein R¹⁰¹ is a phenyl group which is substituted with two or three hydroxy groups (said phenyl group may further be substituted with one or more substituents other than the hydroxy group),
R²⁰¹ is a phenyl group which may be substituted,
R³⁰¹ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group,
Z¹⁰¹ is a single bond.

37. The compound according to claim 36 or a salt thereof, or a hydrate thereof or a solvate thereof, wherein the compound represented by the general formula (II) is a compound selected from the following 6 compounds:
• the compound wherein R¹⁰¹ is 2,4,5-trihydroxyphenyl group, R²⁰¹ is 3,5-dichlorophenyl group, R³⁰¹ is phenyl group, Z¹⁰¹ is a single bond;
• the compound wherein R¹⁰¹ is 2,4,5-trihydroxyphenyl group, R²⁰¹ is 3,4-dichlorophenyl group, R³⁰¹ is isopropyl group, Z¹⁰¹ is a single bond;
• the compound wherein R¹⁰¹ is 2,4,5-trihydroxyphenyl group, R²⁰¹ is 4-nitrophenyl group, R³⁰¹ is ethoxy group, Z¹⁰¹ is a single bond;
• the compound wherein R¹⁰¹ is 2,4,5-trihydroxyphenyl group, R²⁰¹ is 3,4-dichlorophenyl group, R³⁰¹ is methyl group, Z¹⁰¹ is a single bond;
• the compound wherein R¹⁰¹ is 2,4,5-trihydroxyphenyl group, R²⁰¹ is 3,5-dichlorophenyl group, R³⁰¹ is isopropyl group, Z¹⁰¹ is a single bond; and
• the compound wherein R¹⁰¹ is 2,4,5-trihydroxyphenyl group, R²⁰¹ is 3-chloro-4-fluorophenyl group, R³⁰¹ is isopropyl group, Z¹⁰¹ is a single bond.

38. The compound according to claim 25 or a salt thereof, or a hydrate thereof or a solvate thereof, wherein R¹⁰¹ is a phenyl group which is substituted with C₂-C₆ alkenyl group which may be substituted (said phenyl group may further be substituted with one or more substituents other than the C₂-C₆ alkenyl group),
R²⁰¹ is an aromatic group which may be substituted,
W¹⁰¹ is a group represented by the following formula: 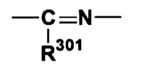 (wherein a bond at the left end binds to the carbon atom and a bond at the right end binds to the nitrogen atom,
R³⁰¹ represents a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group),
Z¹⁰¹ is a single bond.

39. The compound according to claim 38 or a salt thereof, or a hydrate thereof or a solvate thereof, wherein the compound represented by the general formula (II) is a compound selected from the following 2 compounds:
• the compound wherein R¹⁰¹ is 2-hydroxy-5-styrylphenyl group, R²⁰¹ is 4-nitrophenyl group, R³⁰¹ is ethoxy group, Z¹⁰¹ is a single bond; and
• the compound wherein R¹⁰¹ is 4-styrylphenyl group, R²⁰¹ is 4-carboxyphenyl group, R³⁰¹ is isopropyl group, Z¹⁰¹ is a single bond.

40. The compound according to claim 25 or a salt thereof, or a hydrate thereof or a solvate thereof, wherein R¹⁰¹ and R²⁰¹ are any one of the following (i) to (iii),
W¹⁰¹ is a group represented by the following formula: 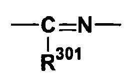 (wherein a bond at the left end binds to the carbon atom and a bond at the right end binds to the nitrogen atom),
R³⁰¹ is any one of the following (i) to (iii),
Z is a single bond:
(i) R¹⁰¹ is a phenyl group which is substituted with carboxy group (said phenyl group may further be substituted with one or more substituents other than the carboxy group), R²⁰¹ is an aromatic group which may be substituted,
R³⁰¹ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group;
(ii) R¹⁰¹ is an aromatic group which may be substituted,
R²⁰¹ is a phenyl group which is substituted with carboxy group (said phenyl group may further be substituted with one or more substituents other than the carboxy group), R³⁰¹ is a C₁-C₆ alkyl group, a C₆-C₁₀ aryl group which may be substituted, a C₁-C₆ halogenated alkyl group, a C₂-C₇ alkoxycarbonyl-substituted C₁-C₆ alkyl group, a C₁-C₆ alkoxy-substituted C₁-C₆ alkyl group, hydroxy group, a C₁-C₆ alkoxy group, carboxy group, or a C₂-C₇ alkoxycarbonyl group;
(iii) R¹⁰¹ is an aromatic group which may be substituted,
R²⁰¹ is an aromatic group which may be substituted,
R³⁰¹ is carboxy group.

41. The compound according to claim 40 or a salt thereof, or a hydrate thereof or a solvate thereof, wherein the compound represented by the general formula (II) is a following compound:
• the compound wherein R¹⁰¹ is 4-styrylphenyl group, R²⁰¹ is 4-carboxyphenyl group, R³⁰¹ is isopropyl group, Z¹⁰¹ is a single bond.

42. The compound according to claim 25 or a salt thereof, or a hydrate thereof or a solvate thereof, wherein the compound represented by the general formula (II) is a compound selected from the group consisting of the compounds described in the specification as Compound Nos. 104 to 158, Compound Nos. 166 to 167, Compound Nos. 176 to 185, Compound Nos. 194 to 219, Compound Nos. 221 to 224, and Compound Nos. 501 to 567.
